(19) Europäisches Patentamt / European Patent Office / Office européen des brevets

(11) **EP 3 617 231 A1**

(12) **EUROPEAN PATENT APPLICATION**
published in accordance with Art. 153(4) EPC


(43) Date of publication:
**04.03.2020 Bulletin 2020/10**

(21) Application number: **18792237.2**

(22) Date of filing: **27.04.2018**

(51) Int Cl.:
***C07K 16/28*** *(2006.01)* ***A01H 1/00*** *(2006.01)*
***A61K 39/395*** *(2006.01)* ***A61K 49/00*** *(2006.01)*
***A61P 35/00*** *(2006.01)* ***C07K 16/46*** *(2006.01)*
***C12N 5/10*** *(2006.01)* ***G01N 33/574*** *(2006.01)*
***C12N 15/13*** *(2006.01)* ***C12P 21/08*** *(2006.01)*

(86) International application number:
**PCT/JP2018/017291**

(87) International publication number:
**WO 2018/199318 (01.11.2018 Gazette 2018/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.04.2017 JP 2017090054**



(71) Applicant: **National University Corporation Kochi University**
**Kochi-shi, Kochi 780-8520 (JP)**

(72) Inventors:
- **NAKA Tetsuji**
  **Kochi-shi**
  **Kochi 780-8520 (JP)**
- **SERADA Satoshi**
  **Kochi-shi**
  **Kochi 780-8520 (JP)**
- **FUJIMOTO Minoru**
  **Kochi-shi**
  **Kochi 780-8520 (JP)**

(74) Representative: **Uexküll & Stolberg Partnerschaft von Patent- und Rechtsanwälten mbB**
**Beselerstraße 4**
**22607 Hamburg (DE)**


(54) **ANTI-GPC-1 ANTIBODY**


(57) Provided is a novel anti-Glypican-1 antibody and a method for using the same. Provided is an anti-Glypican-1 antibody having an intracellular invasion activity which has never been observed in conventional anti-Glypican-1 antibodies. By taking advantage of the intracellular invasion activity of the antibody according to the present invention, the present invention is usable for various therapeutic purposes beyond the scope of the conventional assumption. Also provided is a composition for preventing or treating Glypican-1 positive cancer, said composition comprising a complex of a substance capable of binding to Glypican-1 (for example, an anti-Glypican-1 antibody) with a drug having a cytotoxic activity.


FIG.6

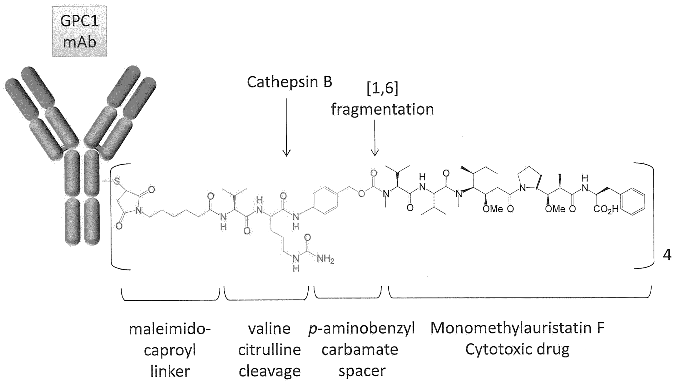


EP 3 617 231 A1

SEQUENCE LISTING

<110>  Kochi University

<120>  anti-GPC-1 antibody

<130>  KOU001PCT

<150>  JP 2017-90054
<151>  2017-04-28

<160>  204

<170>  PatentIn version 3.5

<210>  1
<211>  1677
<212>  DNA
<213>  Homo sapiens

<400>  1
atggagctcc gggcccgagg ctggtggctg ctatgtgcgg ccgcagcgct ggtcgcctgc        60

gcccgcgggg acccggccag caagagccgg agctgcggcg aggtccgcca gatctacgga       120

gccaagggct tcagcctgag cgacgtgccc caggcggaga tctcgggtga gcacctgcgg       180

atctgtcccc agggctacac ctgctgcacc agcgagatgg aggagaacct ggccaaccgc       240

agccatgccg agctggagac cgcgctccgg gacagcagcc gcgtcctgca ggccatgctt       300

gccacccagc tgcgcagctt cgatgaccac ttccagcacc tgctgaacga ctcggagcgg       360

acgctgcagg ccaccttccc cggcgccttc ggagagctgt acacgcagaa cgcgagggcc       420

ttccgggacc tgtactcaga gctgcgcctg tactaccgcg gtgccaacct gcacctggag       480

gagacgctgg ccgagttctg ggcccgcctg ctcgagcgcc tcttcaagca gctgcacccc       540

cagctgctgc tgcctgatga ctacctggac tgcctgggca agcaggccga ggcgctgcgg       600

cccttcgggg aggccccgag agagctgcgc ctgcgggcca cccgtgcctt cgtggctgct       660

cgctcctttg tgcagggcct gggcgtggcc agcgacgtgg tccggaaagt ggctcaggtc       720

cccctgggcc cggagtgctc gagagctgtc atgaagctgg tctactgtgc tcactgcctg       780

ggagtccccg gcgccaggcc ctgccctgac tattgccgaa atgtgctcaa gggctgcctt       840

gccaaccagg ccgacctgga cgccgagtgg aggaacctcc tggactccat ggtgctcatc       900

accgacaagt tctggggtac atcgggtgtg gagagtgtca tcggcagcgt gcacacgtgg       960

ctggcggagg ccatcaacgc cctccaggac aacagggaca cgctcacggc caaggtcatc      1020

cagggctgcg ggaaccccaa ggtcaacccc cagggccccg ggcctgagga gaagcggcgc      1080

cggggcaagc tggccccgcg ggagaggcca ccttcaggca cgctggagaa gctggtctcc      1140

gaagccaagg cccagctccg cgacgtccag gacttctgga tcagcctccc agggacactg      1200

tgcagtgaga agatggccct gagcactgcc agtgatgacc gctgctggaa cgggatggcc      1260

agaggccggt acctccccga ggtcatgggt gacggcctgg ccaaccagat caacaacccc 1320

gaggtggagg tggacatcac caagccggac atgaccatcc ggcagcagat catgcagctg 1380

aagatcatga ccaaccggct gcgcagcgcc tacaacggca acgacgtgga cttccaggac 1440

gccagtgacg acggcagcgg ctcgggcagc ggtgatggct gtctggatga cctctgcagc 1500

cggaaggtca gcaggaagag ctccagctcc cggacgccct tgacccatgc cctcccaggc 1560

ctgtcagagc aggaaggaca gaagacctcg gctgccagct gcccccagcc cccgaccttc 1620

ctcctgcccc tcctcctctt cctggccctt acagtagcca ggccccggtg gcggtaa 1677

<210> 2
<211> 558
<212> PRT
<213> Homo sapiens

<400> 2

Met Glu Leu Arg Ala Arg Gly Trp Trp Leu Leu Cys Ala Ala Ala Ala
1 5 10 15

Leu Val Ala Cys Ala Arg Gly Asp Pro Ala Ser Lys Ser Arg Ser Cys
20 25 30

Gly Glu Val Arg Gln Ile Tyr Gly Ala Lys Gly Phe Ser Leu Ser Asp
35 40 45

Val Pro Gln Ala Glu Ile Ser Gly Glu His Leu Arg Ile Cys Pro Gln
50 55 60

Gly Tyr Thr Cys Cys Thr Ser Glu Met Glu Glu Asn Leu Ala Asn Arg
65 70 75 80

Ser His Ala Glu Leu Glu Thr Ala Leu Arg Asp Ser Ser Arg Val Leu
85 90 95

Gln Ala Met Leu Ala Thr Gln Leu Arg Ser Phe Asp Asp His Phe Gln
100 105 110

His Leu Leu Asn Asp Ser Glu Arg Thr Leu Gln Ala Thr Phe Pro Gly
115 120 125

Ala Phe Gly Glu Leu Tyr Thr Gln Asn Ala Arg Ala Phe Arg Asp Leu
130 135 140

Tyr Ser Glu Leu Arg Leu Tyr Tyr Arg Gly Ala Asn Leu His Leu Glu
145 150 155 160

Glu Thr Leu Ala Glu Phe Trp Ala Arg Leu Leu Glu Arg Leu Phe Lys

```
                165                 170                 175

Gln Leu His Pro Gln Leu Leu Leu Pro Asp Asp Tyr Leu Asp Cys Leu
            180                 185                 190

Gly Lys Gln Ala Glu Ala Leu Arg Pro Phe Gly Glu Ala Pro Arg Glu
        195                 200                 205

Leu Arg Leu Arg Ala Thr Arg Ala Phe Val Ala Ala Arg Ser Phe Val
    210                 215                 220

Gln Gly Leu Gly Val Ala Ser Asp Val Val Arg Lys Val Ala Gln Val
225                 230                 235                 240

Pro Leu Gly Pro Glu Cys Ser Arg Ala Val Met Lys Leu Val Tyr Cys
                245                 250                 255

Ala His Cys Leu Gly Val Pro Gly Ala Arg Pro Cys Pro Asp Tyr Cys
            260                 265                 270

Arg Asn Val Leu Lys Gly Cys Leu Ala Asn Gln Ala Asp Leu Asp Ala
        275                 280                 285

Glu Trp Arg Asn Leu Leu Asp Ser Met Val Leu Ile Thr Asp Lys Phe
    290                 295                 300

Trp Gly Thr Ser Gly Val Glu Ser Val Ile Gly Ser Val His Thr Trp
305                 310                 315                 320

Leu Ala Glu Ala Ile Asn Ala Leu Gln Asp Asn Arg Asp Thr Leu Thr
                325                 330                 335

Ala Lys Val Ile Gln Gly Cys Gly Asn Pro Lys Val Asn Pro Gln Gly
            340                 345                 350

Pro Gly Pro Glu Glu Lys Arg Arg Arg Gly Lys Leu Ala Pro Arg Glu
        355                 360                 365

Arg Pro Pro Ser Gly Thr Leu Glu Lys Leu Val Ser Glu Ala Lys Ala
    370                 375                 380

Gln Leu Arg Asp Val Gln Asp Phe Trp Ile Ser Leu Pro Gly Thr Leu
385                 390                 395                 400

Cys Ser Glu Lys Met Ala Leu Ser Thr Ala Ser Asp Asp Arg Cys Trp
                405                 410                 415
```

Asn Gly Met Ala Arg Gly Arg Tyr Leu Pro Glu Val Met Gly Asp Gly
420 425 430

Leu Ala Asn Gln Ile Asn Asn Pro Glu Val Glu Val Asp Ile Thr Lys
435 440 445

Pro Asp Met Thr Ile Arg Gln Gln Ile Met Gln Leu Lys Ile Met Thr
450 455 460

Asn Arg Leu Arg Ser Ala Tyr Asn Gly Asn Asp Val Asp Phe Gln Asp
465 470 475 480

Ala Ser Asp Asp Gly Ser Gly Ser Gly Ser Gly Asp Gly Cys Leu Asp
485 490 495

Asp Leu Cys Ser Arg Lys Val Ser Arg Lys Ser Ser Ser Ser Arg Thr
500 505 510

Pro Leu Thr His Ala Leu Pro Gly Leu Ser Glu Gln Glu Gly Gln Lys
515 520 525

Thr Ser Ala Ala Ser Cys Pro Gln Pro Pro Thr Phe Leu Leu Pro Leu
530 535 540

Leu Leu Phe Leu Ala Leu Thr Val Ala Arg Pro Arg Trp Arg
545 550 555

<210> 3
<211> 1673
<212> DNA
<213> Mus musculus

<400> 3
tggaactccg gacccgaggc tggtggctgc tgtgcgcggc cgccgcgctg gtcgtctgcg 60

cccgcgggga ccccgccagc aagagccgga gctgcagcga agtccgccag atctacgggg 120

ctaagggctt tagcctgagc gatgtgcctc aggcagagat ctcgggtgag cacctgcgga 180

tctgccccca gggctacact tgctgtacta gtgagatgga ggagaatttg gccaaccaca 240

gccgaatgga gctggagagc gcactccatg acagcagccg cgccctgcag gccacactgg 300

ccacccagct gcatggcatc gatgaccact tccagcgcct gctgaatgac tcggagcgca 360

cactgcagga ggctttccct ggggcctttg ggacctgta tacgcagaac actcgtgcct 420

tccgggacct atatgctgag ctgcgcctct actaccgtgg ggccaacctg caccttgagg 480

agacgctggc cgagttctgg gcacggctgc tggagcgcct cttcaagcag ctgcaccccc 540

agctgctgcc tgatgactac ctggactgcc tgggcaagca ggcggaggca ctgcggccgt 600

ttggagatgc ccctcgagaa ctgcgcctgc gggccacccg tgcctttgtg gctgcacgtt 660

```
cctttgtgca gggcctgggt gtggccagtg atgtagtccg gaaggtggcc caggtacctc      720
tggccccaga atgttctcgg gccatcatga agttggtcta ctgtgctcat tgccggggag      780
tcccgggcgc ccggccctgc cccgactatt gccgaaatgt gctcaaaggc tgccttgcca      840
accaggccga cctggatgcc gagtggagga acctcctgga ctccatggtg ctcatcactg      900
acaagttctg ggcccgtcg ggtgcggaga gtgtcattgg cggtgtgcac gtgtggctgg       960
cggaggccat caacgccctc caggacaaca aggacacact cacagctaag gtcatccagg     1020
cctgtggaaa ccccaaggtc aatccccacg gctctgggcc cgaggagaag cgtcgccgtg     1080
gcaaattggc actgcaggag aagccctcca caggtactct ggaaaaactg gtctctgagg     1140
ccaaggccca gctccgagac attcaggact tctggatcag cctcccaggg acactgtgca     1200
gtgagaagat ggccatgagt cctgccagtg acgaccgctg ctggaatgga atttccaagg     1260
gccggtacct accagaggtg atgggtgacg ggctggccaa ccagatcaac aaccctgagg     1320
tggaagtgga catcaccaag ccagacatga ccatccgcca gcagattatg cagctcaaga     1380
tcatgaccaa ccgtttacgt ggcgcctatg gcggcaacga cgtggacttc caggatgcta     1440
gtgatgacgg cagtggctcc ggcagcggtg gcggatgccc agatgacacc tgtggccgga     1500
gggtcagcaa gaagagttcc agctcccgga ccccccttgac ccatgccctc cccggcctgt    1560
cagaacagga gggacagaag acctcagctg ccacctgccc agagccccac agcttcttcc     1620
tgctcttcct cgtcaccttg gtccttgcgg cagccaggcc caggtggcgg taa            1673
```

<210> 4
<211> 557
<212> PRT
<213> Mus musculus

<400> 4

```
Met Glu Leu Arg Thr Arg Gly Trp Trp Leu Leu Cys Ala Ala Ala Ala
1               5                   10                  15

Leu Val Val Cys Ala Arg Gly Asp Pro Ala Ser Lys Ser Arg Ser Cys
            20                  25                  30

Ser Glu Val Arg Gln Ile Tyr Gly Ala Lys Gly Phe Ser Leu Ser Asp
        35                  40                  45

Val Pro Gln Ala Glu Ile Ser Gly Glu His Leu Arg Ile Cys Pro Gln
    50                  55                  60

Gly Tyr Thr Cys Cys Thr Ser Glu Met Glu Glu Asn Leu Ala Asn His
65                  70                  75                  80
```

```
Ser Arg Met Glu Leu Glu Ser Ala Leu His Asp Ser Ser Arg Ala Leu
                85                  90                  95

Gln Ala Thr Leu Ala Thr Gln Leu His Gly Ile Asp Asp His Phe Gln
            100                 105                 110

Arg Leu Leu Asn Asp Ser Glu Arg Thr Leu Gln Glu Ala Phe Pro Gly
        115                 120                 125

Ala Phe Gly Asp Leu Tyr Thr Gln Asn Thr Arg Ala Phe Arg Asp Leu
    130                 135                 140

Tyr Ala Glu Leu Arg Leu Tyr Tyr Arg Gly Ala Asn Leu His Leu Glu
145                 150                 155                 160

Glu Thr Leu Ala Glu Phe Trp Ala Arg Leu Leu Glu Arg Leu Phe Lys
                165                 170                 175

Gln Leu His Pro Gln Leu Leu Pro Asp Asp Tyr Leu Asp Cys Leu Gly
            180                 185                 190

Lys Gln Ala Glu Ala Leu Arg Pro Phe Gly Asp Ala Pro Arg Glu Leu
        195                 200                 205

Arg Leu Arg Ala Thr Arg Ala Phe Val Ala Ala Arg Ser Phe Val Gln
    210                 215                 220

Gly Leu Gly Val Ala Ser Asp Val Val Arg Lys Val Ala Gln Val Pro
225                 230                 235                 240

Leu Ala Pro Glu Cys Ser Arg Ala Ile Met Lys Leu Val Tyr Cys Ala
                245                 250                 255

His Cys Arg Gly Val Pro Gly Ala Arg Pro Cys Pro Asp Tyr Cys Arg
            260                 265                 270

Asn Val Leu Lys Gly Cys Leu Ala Asn Gln Ala Asp Leu Asp Ala Glu
        275                 280                 285

Trp Arg Asn Leu Leu Asp Ser Met Val Leu Ile Thr Asp Lys Phe Trp
    290                 295                 300

Gly Pro Ser Gly Ala Glu Ser Val Ile Gly Gly Val His Val Trp Leu
305                 310                 315                 320

Ala Glu Ala Ile Asn Ala Leu Gln Asp Asn Lys Asp Thr Leu Thr Ala
                325                 330                 335
```

Lys Val Ile Gln Ala Cys Gly Asn Pro Lys Val Asn Pro His Gly Ser
340 345 350

Gly Pro Glu Glu Lys Arg Arg Arg Gly Lys Leu Ala Leu Gln Glu Lys
355 360 365

Pro Ser Thr Gly Thr Leu Glu Lys Leu Val Ser Glu Ala Lys Ala Gln
370 375 380

Leu Arg Asp Ile Gln Asp Phe Trp Ile Ser Leu Pro Gly Thr Leu Cys
385 390 395 400

Ser Glu Lys Met Ala Met Ser Pro Ala Ser Asp Asp Arg Cys Trp Asn
405 410 415

Gly Ile Ser Lys Gly Arg Tyr Leu Pro Glu Val Met Gly Asp Gly Leu
420 425 430

Ala Asn Gln Ile Asn Asn Pro Glu Val Glu Val Asp Ile Thr Lys Pro
435 440 445

Asp Met Thr Ile Arg Gln Gln Ile Met Gln Leu Lys Ile Met Thr Asn
450 455 460

Arg Leu Arg Gly Ala Tyr Gly Gly Asn Asp Val Asp Phe Gln Asp Ala
465 470 475 480

Ser Asp Asp Gly Ser Gly Ser Gly Ser Gly Gly Gly Cys Pro Asp Asp
485 490 495

Thr Cys Gly Arg Arg Val Ser Lys Lys Ser Ser Ser Ser Arg Thr Pro
500 505 510

Leu Thr His Ala Leu Pro Gly Leu Ser Glu Gln Glu Gly Gln Lys Thr
515 520 525

Ser Ala Ala Thr Cys Pro Glu Pro His Ser Phe Phe Leu Leu Phe Leu
530 535 540

Val Thr Leu Val Leu Ala Ala Ala Arg Pro Arg Trp Arg
545 550 555

<210> 5
<211> 5
<212> PRT
<213> artificial sequence

<220>

<223>  Amino Acid Sequence of Heavy Chain CDR1 of K090-01a002

<400>  5

Arg Tyr Tyr Met His
1               5

<210>  6
<211>  17
<212>  PRT
<213>  artificial sequence

<220>
<223>  Amino Acid Sequence of Heavy Chain CDR2 of K090-01a002

<400>  6

Glu Ile Asn Pro Ser Thr Gly Asp Thr Thr Tyr Asn Gln Lys Phe Lys
1               5                   10                  15

Ala

<210>  7
<211>  10
<212>  PRT
<213>  artificial sequence

<220>
<223>  Amino Acid Sequence of Heavy Chain CDR3 of K090-01a002

<400>  7

Glu Lys Arg Asp Asp Gly Val Leu Ala Tyr
1               5                   10

<210>  8
<211>  11
<212>  PRT
<213>  artificial sequence

<220>
<223>  Amino Acid Sequence of Light Chain CDR1 of K090-01a002

<400>  8

Lys Ala Ser Gln Ser Val Gly Asn Asn Val Ala
1               5                   10

<210>  9
<211>  7
<212>  PRT
<213>  artificial sequence

<220>
<223>  Amino Acid Sequence of Light Chain CDR2 of K090-01a002

<400>  9

Tyr Ala Ser Asn Arg Tyr Thr
1               5

<210>  10
<211>  9
<212>  PRT
<213>  artificial sequence

<220>
<223>  Amino Acid Sequence of Light Chain CDR3 of K090-01a002

<400>  10

Gln Gln His Tyr Ser Ser Pro Leu Thr
1               5

<210>  11
<211>  5
<212>  PRT
<213>  artificial sequence

<220>
<223>  Amino Acid Sequence of Heavy Chain CDR1 of K090-01a007

<400>  11

Ser Tyr Trp Met His
1               5

<210>  12
<211>  17
<212>  PRT
<213>  artificial sequence

<220>
<223>  Amino Acid Sequence of Heavy Chain CDR2 of K090-01a007

<400>  12

Asn Ile Asp Pro Tyr Asp Ser Glu Thr His Tyr Asn Gln Lys Phe Lys
1               5                   10                  15

Asp

<210>  13
<211>  9
<212>  PRT
<213>  artificial sequence

<220>
<223>  Amino Acid Sequence of Heavy Chain CDR3 of K090-01a007

<400>  13

Asp Ser Asn Trp Gly Tyr Phe Asp Tyr
1               5

<210> 14
<211> 16
<212> PRT
<213> artificial sequence

<220>
<223> Amino Acid Sequence of Light Chain CDR1 of K090-01a007

<400> 14

Arg Ser Ser Gln Ser Leu Val His Ser Asn Gly Asn Thr Tyr Leu His
1 5 10 15

<210> 15
<211> 7
<212> PRT
<213> artificial sequence

<220>
<223> Amino Acid Sequence of Light Chain CDR2 of K090-01a007

<400> 15

Lys Val Ser Asn Arg Phe Ser
1 5

<210> 16
<211> 9
<212> PRT
<213> artificial sequence

<220>
<223> Amino Acid Sequence of Light Chain CDR3 of K090-01a007

<400> 16

Ser Gln Ser Thr His Val Pro Trp Thr
1 5

<210> 17
<211> 5
<212> PRT
<213> artificial sequence

<220>
<223> Amino Acid Sequence of Heavy Chain CDR1 of K090-01a016

<400> 17

Asp Tyr Tyr Ile Asn
1 5

<210> 18
<211> 17
<212> PRT
<213> artificial sequence

<220>
<223>  Amino Acid Sequence of Heavy Chain CDR2 of K090-01a016

<400>  18

Tyr Ile Asp Pro Tyr Asn Asp Gly Ser Lys Tyr Asn Glu Lys Phe Lys
1               5                   10                  15

Gly

<210>  19
<211>  15
<212>  PRT
<213>  artificial sequence

<220>
<223>  Amino Acid Sequence of Heavy Chain CDR3 of K090-01a016

<400>  19

Thr Pro Tyr Tyr Ser Phe Tyr Ser Tyr Gly Phe Tyr Phe Asp Tyr
1               5                   10                  15

<210>  20
<211>  15
<212>  PRT
<213>  artificial sequence

<220>
<223>  Amino Acid Sequence of Light Chain CDR1 of K090-01a016

<400>  20

Arg Ala Ser Lys Ser Val Ser Thr Ser Ser Tyr Ser Tyr Met His
1               5                   10                  15

<210>  21
<211>  7
<212>  PRT
<213>  artificial sequence

<220>
<223>  Amino Acid Sequence of Light Chain CDR2 of K090-01a016

<400>  21

Tyr Ala Ser Tyr Leu Glu Ser
1               5

<210>  22
<211>  9
<212>  PRT
<213>  artificial sequence

<220>
<223>  Amino Acid Sequence of Light Chain CDR3 of K090-01a016

<400> 22

Gln His Ser Arg Glu Phe Pro Leu Thr
1 5

<210> 23
<211> 5
<212> PRT
<213> artificial sequence

<220>
<223> Amino Acid Sequence of Heavy Chain CDR1 of K090-01a017

<400> 23

Asn Tyr Trp Ile Gly
1 5

<210> 24
<211> 17
<212> PRT
<213> artificial sequence

<220>
<223> Amino Acid Sequence of Heavy Chain CDR2 of K090-01a017

<400> 24

Asp Leu Tyr Pro Gly Gly Gly Tyr Thr His Tyr Asn Glu Lys Phe Lys
1 5 10 15

Gly

<210> 25
<211> 13
<212> PRT
<213> artificial sequence

<220>
<223> Amino Acid Sequence of Heavy Chain CDR3 of K090-01a017

<400> 25

Glu Arg Val Tyr Phe Asp Gly Ser Arg Tyr Phe Asp Val
1 5 10

<210> 26
<211> 11
<212> PRT
<213> artificial sequence

<220>
<223> Amino Acid Sequence of Light Chain CDR1 of K090-01a017

<400> 26

Lys Ala Ser Gln Ser Val Gly Asn Asn Val Ala

1 5 10

<210> 27
<211> 7
<212> PRT
<213> artificial sequence

<220>
<223> Amino Acid Sequence of Light Chain CDR2 of K090-01a017

<400> 27

Tyr Ala Ser Asn Arg Tyr Thr
1 5

<210> 28
<211> 9
<212> PRT
<213> artificial sequence

<220>
<223> Amino Acid Sequence of Light Chain CDR3 of K090-01a017

<400> 28

Gln Gln His Tyr Ser Ser Pro Trp Thr
1 5

<210> 29
<211> 5
<212> PRT
<213> artificial sequence

<220>
<223> Amino Acid Sequence of Heavy Chain CDR1 of K090-01a021

<400> 29

Gly Tyr Tyr Met His
1 5

<210> 30
<211> 17
<212> PRT
<213> artificial sequence

<220>
<223> Amino Acid Sequence of Heavy Chain CDR2 of K090-01a021

<400> 30

Glu Ile Asn Pro Ser Thr Gly Gly Thr Thr Tyr Asn Gln Lys Phe Lys
1 5 10 15

Ala

<210> 31
<211> 10
<212> PRT
<213> artificial sequence

<220>
<223> Amino Acid Sequence of Heavy Chain CDR3 of K090-01a021

<400> 31

Glu Lys Arg Asp Asp Gly Val Phe Ala Tyr
1 5 10

<210> 32
<211> 11
<212> PRT
<213> artificial sequence

<220>
<223> Amino Acid Sequence of Light Chain CDR1 of K090-01a021

<400> 32

Lys Ala Ser Gln Ser Val Gly Asn Asn Val Ala
1 5 10

<210> 33
<211> 7
<212> PRT
<213> artificial sequence

<220>
<223> Amino Acid Sequence of Light Chain CDR2 of K090-01a021

<400> 33

Tyr Ala Ser Asn Arg Tyr Thr
1 5

<210> 34
<211> 9
<212> PRT
<213> artificial sequence

<220>
<223> Amino Acid Sequence of Light Chain CDR3 of K090-01a021

<400> 34

Gln Gln His Tyr Ser Ser Pro Leu Thr
1 5

<210> 35
<211> 5
<212> PRT
<213> artificial sequence

<220>
<223> Amino Acid Sequence of Heavy Chain CDR1 of K090-01a026

<400> 35

Asn Tyr Trp Ile Gly
1 5

<210> 36
<211> 17
<212> PRT
<213> artificial sequence

<220>
<223> Amino Acid Sequence of Heavy Chain CDR2 of K090-01a026

<400> 36

Asp Ile Tyr Pro Gly Gly Gly Tyr Thr His Tyr Asn Glu Lys Phe Lys
1 5 10 15

Gly

<210> 37
<211> 13
<212> PRT
<213> artificial sequence

<220>
<223> Amino Acid Sequence of Heavy Chain CDR3 of K090-01a026

<400> 37

Glu Arg Val Tyr Tyr Asp Gly Ser Arg Phe Phe Asp Val
1 5 10

<210> 38
<211> 11
<212> PRT
<213> artificial sequence

<220>
<223> Amino Acid Sequence of Light Chain CDR1 of K090-01a026

<400> 38

Lys Ala Ser Gln Ser Val Gly Asn Asn Val Ala
1 5 10

<210> 39
<211> 7
<212> PRT
<213> artificial sequence

<220>
<223> Amino Acid Sequence of Light Chain CDR2 of K090-01a026

<400> 39

```
Tyr Ala Ser Asn Arg Tyr Thr
1               5


<210>  40
<211>  9
<212>  PRT
<213>  artificial sequence

<220>
<223>  Amino Acid Sequence of Light Chain CDR3 of K090-01a026

<400>  40

Gln Gln His Tyr Ser Ser Pro Trp Thr
1               5


<210>  41
<211>  5
<212>  PRT
<213>  artificial sequence

<220>
<223>  Amino Acid Sequence of Heavy Chain CDR1 of K090-01a009

<400>  41

Gly Tyr Phe Met Asn
1               5


<210>  42
<211>  17
<212>  PRT
<213>  artificial sequence

<220>
<223>  Amino Acid Sequence of Heavy Chain CDR2 of K090-01a009

<400>  42

Arg Ile Asn Pro Asp Asn Gly Asp Ile Phe Tyr Asn Gln Lys Phe Lys
1               5                   10                  15


Gly



<210>  43
<211>  8
<212>  PRT
<213>  artificial sequence

<220>
<223>  Amino Acid Sequence of Heavy Chain CDR3 of K090-01a009

<400>  43

Ser Trp Asn Trp Tyr Phe Asp Val
1               5
```

<210> 44
<211> 11
<212> PRT
<213> artificial sequence

<220>
<223> Amino Acid Sequence of Light Chain CDR1 of K090-01a009

<400> 44

Arg Ala Ser Glu Asn Ile Tyr Ser Tyr Leu Ala
1 5 10

<210> 45
<211> 7
<212> PRT
<213> artificial sequence

<220>
<223> Amino Acid Sequence of Light Chain CDR2 of K090-01a009

<400> 45

Asn Ala Lys Thr Leu Ala Glu
1 5

<210> 46
<211> 9
<212> PRT
<213> artificial sequence

<220>
<223> Amino Acid Sequence of Light Chain CDR3 of K090-01a009

<400> 46

Gln His His Tyr Gly Thr Pro Tyr Thr
1 5

<210> 47
<211> 5
<212> PRT
<213> artificial sequence

<220>
<223> Amino Acid Sequence of Heavy Chain CDR1 of K090-01a030

<400> 47

Asn Tyr Trp Ile Gly
1 5

<210> 48
<211> 17
<212> PRT
<213> artificial sequence

<220>

<223>  Amino Acid Sequence of Heavy Chain CDR2 of K090-01a030

<400>  48

Asp Ile Tyr Pro Gly Gly Gly Tyr Thr His Tyr Asn Glu Lys Phe Lys
1               5                   10                  15

Gly

<210>  49
<211>  13
<212>  PRT
<213>  artificial sequence

<220>
<223>  Amino Acid Sequence of Heavy Chain CDR3 of K090-01a030

<400>  49

Glu Arg Val Tyr Tyr Asp Gly Ser Arg Tyr Phe Asp Val
1               5                   10

<210>  50
<211>  11
<212>  PRT
<213>  artificial sequence

<220>
<223>  Amino Acid Sequence of Light Chain CDR1 of K090-01a030

<400>  50

Lys Ala Ser Gln Ser Val Gly Asn Asn Val Ala
1               5                   10

<210>  51
<211>  7
<212>  PRT
<213>  artificial sequence

<220>
<223>  Amino Acid Sequence of Light Chain CDR2 of K090-01a030

<400>  51

Tyr Ala Ser Asn Arg Tyr Thr
1               5

<210>  52
<211>  9
<212>  PRT
<213>  artificial sequence

<220>
<223>  Amino Acid Sequence of Light Chain CDR3 of K090-01a030

<400>  52

Gln Gln His Tyr Ser Ser Pro Trp Thr
1               5

<210>  53
<211>  5
<212>  PRT
<213>  artificial sequence

<220>
<223>  Amino Acid Sequence of Heavy Chain CDR1 of K090-01a033

<400>  53

Gly Tyr Tyr Met His
1               5

<210>  54
<211>  17
<212>  PRT
<213>  artificial sequence

<220>
<223>  Amino Acid Sequence of Heavy Chain CDR2 of K090-01a033

<400>  54

Glu Ile Asn Pro Ser Thr Gly Asp Thr Thr Tyr Asn Gln Lys Phe Lys
1               5                   10                  15

Ala

<210>  55
<211>  10
<212>  PRT
<213>  artificial sequence

<220>
<223>  Amino Acid Sequence of Heavy Chain CDR3 of K090-01a033

<400>  55

Glu Lys Arg Asp Asp Gly Val Phe Ala Tyr
1               5                   10

<210>  56
<211>  11
<212>  PRT
<213>  artificial sequence

<220>
<223>  Amino Acid Sequence of Light Chain CDR1 of K090-01a033

<400>  56

Lys Ala Ser Glu Asn Val Gly Thr Tyr Val Ser
1               5                   10

<210> 57
<211> 7
<212> PRT
<213> artificial sequence

<220>
<223> Amino Acid Sequence of Light Chain CDR2 of K090-01a033

<400> 57

Gly Ala Ser Asn Arg Tyr Thr
1               5

<210> 58
<211> 9
<212> PRT
<213> artificial sequence

<220>
<223> Amino Acid Sequence of Light Chain CDR3 of K090-01a033

<400> 58

Gly Gln Ser Tyr Ser Tyr Pro Leu Thr
1               5

<210> 59
<211> 5
<212> PRT
<213> artificial sequence

<220>
<223> Amino Acid Sequence of Heavy Chain CDR1 of K090-01a042

<400> 59

Arg Tyr Trp Met His
1               5

<210> 60
<211> 17
<212> PRT
<213> artificial sequence

<220>
<223> Amino Acid Sequence of Heavy Chain CDR2 of K090-01a042

<400> 60

Tyr Ile Asn Pro Ser Ser Gly Tyr Thr Glu Tyr Asn Gln Lys Phe Lys
1               5                   10                  15

Asp

<210> 61

<211> 10
<212> PRT
<213> artificial sequence

<220>
<223> Amino Acid Sequence of Heavy Chain CDR3 of K090-01a042

<400> 61

Leu Gly Asp Tyr Ser Tyr Tyr Phe Asp Tyr
1 5 10

<210> 62
<211> 16
<212> PRT
<213> artificial sequence

<220>
<223> Amino Acid Sequence of Light Chain CDR1 of K090-01a042

<400> 62

Arg Ser Ser Gln Ser Ile Val His Ser Asn Gly Asn Thr Tyr Leu Glu
1 5 10 15

<210> 63
<211> 7
<212> PRT
<213> artificial sequence

<220>
<223> Amino Acid Sequence of Light Chain CDR2 of K090-01a042

<400> 63

Lys Val Ser Asn Arg Phe Ser
1 5

<210> 64
<211> 9
<212> PRT
<213> artificial sequence

<220>
<223> Amino Acid Sequence of Light Chain CDR3 of K090-01a042

<400> 64

Phe Gln Gly Ser His Val Pro Trp Thr
1 5

<210> 65
<211> 5
<212> PRT
<213> artificial sequence

<220>
<223> Amino Acid Sequence of Heavy Chain CDR1 of K090-02a002

<400> 65

Asp Tyr Gly Met His
1 5

<210> 66
<211> 17
<212> PRT
<213> artificial sequence

<220>
<223> Amino Acid Sequence of Heavy Chain CDR2 of K090-02a002

<400> 66

Tyr Ile Ser Ser Gly Ser Ser Thr Ile Tyr Tyr Ala Asp Thr Val Lys
1 5 10 15

Gly

<210> 67
<211> 16
<212> PRT
<213> artificial sequence

<220>
<223> Amino Acid Sequence of Heavy Chain CDR3 of K090-02a002

<400> 67

His Ser Tyr Tyr Ser Tyr Asp Val Gly Gly Asp Tyr Val Met Asp Tyr
1 5 10 15

<210> 68
<211> 11
<212> PRT
<213> artificial sequence

<220>
<223> Amino Acid Sequence of Light Chain CDR1 of K090-02a002

<400> 68

Lys Ala Ser Gln Asp Val Gly Thr Ala Val Ala
1 5 10

<210> 69
<211> 7
<212> PRT
<213> artificial sequence

<220>
<223> Amino Acid Sequence of Light Chain CDR2 of K090-02a002

<400> 69

Trp Ala Ser Thr Arg His Thr

1 5

<210> 70
<211> 9
<212> PRT
<213> artificial sequence

<220>
<223> Amino Acid Sequence of Light Chain CDR3 of K090-02a002

<400> 70

Gln Gln Tyr Ser Ser Tyr Pro Leu Thr
1 5

<210> 71
<211> 5
<212> PRT
<213> artificial sequence

<220>
<223> Amino Acid Sequence of Heavy Chain CDR1 of K090-02a006

<400> 71

Gly Tyr Asn Met Asn
1 5

<210> 72
<211> 17
<212> PRT
<213> artificial sequence

<220>
<223> Amino Acid Sequence of Heavy Chain CDR2 of K090-02a006

<400> 72

Asn Ile Asn Pro Tyr Tyr Gly Thr Thr Asn Tyr Asn Gln Lys Phe Lys
1 5 10 15

Gly

<210> 73
<211> 9
<212> PRT
<213> artificial sequence

<220>
<223> Amino Acid Sequence of Heavy Chain CDR3 of K090-02a006

<400> 73

Asp Gly Tyr Tyr Gly Ala Met Asp Tyr
1 5

<210> 74
<211> 11
<212> PRT
<213> artificial sequence

<220>
<223> Amino Acid Sequence of Light Chain CDR1 of K090-02a006

<400> 74

Lys Ala Ser Gln Asn Val Gly Thr Tyr Val Ala
1               5                   10

<210> 75
<211> 7
<212> PRT
<213> artificial sequence

<220>
<223> Amino Acid Sequence of Light Chain CDR2 of K090-02a006

<400> 75

Ser Ala Ser Tyr Arg Tyr Ser
1               5

<210> 76
<211> 9
<212> PRT
<213> artificial sequence

<220>
<223> Amino Acid Sequence of Light Chain CDR3 of K090-02a006

<400> 76

Gln Gln Tyr Tyr Asn Tyr Pro Leu Thr
1               5

<210> 77
<211> 5
<212> PRT
<213> artificial sequence

<220>
<223> Amino Acid Sequence of Heavy Chain CDR1 of K090-02a010

<400> 77

Asp Tyr Gly Met His
1               5

<210> 78
<211> 17
<212> PRT
<213> artificial sequence

<220>
<223> Amino Acid Sequence of Heavy Chain CDR2 of K090-02a010

<400> 78

Tyr Ile Asn Ser Gly Ser Ser Thr Ile Tyr Tyr Ala Asp Thr Val Lys
1 5 10 15

Gly

<210> 79
<211> 16
<212> PRT
<213> artificial sequence

<220>
<223> Amino Acid Sequence of Heavy Chain CDR3 of K090-02a010

<400> 79

His Ser Tyr Tyr Ser Tyr Asp Val Gly Gly Asp Tyr Val Met Asp Tyr
1 5 10 15

<210> 80
<211> 11
<212> PRT
<213> artificial sequence

<220>
<223> Amino Acid Sequence of Light Chain CDR1 of K090-02a010

<400> 80

Lys Ala Ser Gln Asp Val Gly Thr Ala Val Ala
1 5 10

<210> 81
<211> 7
<212> PRT
<213> artificial sequence

<220>
<223> Amino Acid Sequence of Light Chain CDR2 of K090-02a010

<400> 81

Trp Ala Ser Thr Arg His Thr
1 5

<210> 82
<211> 9
<212> PRT
<213> artificial sequence

<220>
<223> Amino Acid Sequence of Light Chain CDR3 of K090-02a010

<400> 82

Gln Gln Tyr Ser Ser Tyr Pro Leu Thr
1               5

<210>  83
<211>  5
<212>  PRT
<213>  artificial sequence

<220>
<223>  Amino Acid Sequence of Heavy Chain CDR1 of K090-02a014

<400>  83

Gly Tyr Tyr Ile His
1               5

<210>  84
<211>  17
<212>  PRT
<213>  artificial sequence

<220>
<223>  Amino Acid Sequence of Heavy Chain CDR2 of K090-02a014

<400>  84

Glu Ile Asn Pro Ser Thr Gly Asp Ile Thr Tyr Asn Gln Lys Phe Lys
1               5                   10                  15

Ala

<210>  85
<211>  9
<212>  PRT
<213>  artificial sequence

<220>
<223>  Amino Acid Sequence of Heavy Chain CDR3 of K090-02a014

<400>  85

Glu Arg Asn Tyr Asp Arg Phe Ala Tyr
1               5

<210>  86
<211>  12
<212>  PRT
<213>  artificial sequence

<220>
<223>  Amino Acid Sequence of Light Chain CDR1 of K090-02a014

<400>  86

Ser Val Ser Ser Ser Ile Ser Ser Ser Asn Leu His
1               5                   10

<210> 87
<211> 7
<212> PRT
<213> artificial sequence

<220>
<223> Amino Acid Sequence of Light Chain CDR2 of K090-02a014

<400> 87

Gly Thr Ser Asn Leu Ala Ser
1 5

<210> 88
<211> 9
<212> PRT
<213> artificial sequence

<220>
<223> Amino Acid Sequence of Light Chain CDR3 of K090-02a014

<400> 88

Gln Gln Trp Ser Ser Tyr Pro Leu Thr
1 5

<210> 89
<211> 5
<212> PRT
<213> artificial sequence

<220>
<223> Amino Acid Sequence of Heavy Chain CDR1 of K090-02a022

<400> 89

Asp Tyr Tyr Met Asn
1 5

<210> 90
<211> 17
<212> PRT
<213> artificial sequence

<220>
<223> Amino Acid Sequence of Heavy Chain CDR2 of K090-02a022

<400> 90

Arg Val Asn Pro Ser Asn Gly Gly Thr Ser Tyr Asn Gln Lys Phe Lys
1 5 10 15

Gly

<210> 91
<211> 10

<212> PRT
<213> artificial sequence

<220>
<223> Amino Acid Sequence of Heavy Chain CDR3 of K090-02a022

<400> 91

Ser Gly Glu Asn Tyr Tyr Ala Met Asp Tyr
1 5 10

<210> 92
<211> 11
<212> PRT
<213> artificial sequence

<220>
<223> Amino Acid Sequence of Light Chain CDR1 of K090-02a022

<400> 92

Arg Ala Ser Glu Asn Ile Tyr Ser Tyr Leu Ala
1 5 10

<210> 93
<211> 7
<212> PRT
<213> artificial sequence

<220>
<223> Amino Acid Sequence of Light Chain CDR2 of K090-02a022

<400> 93

Asn Gly Lys Thr Leu Ala Glu
1 5

<210> 94
<211> 9
<212> PRT
<213> artificial sequence

<220>
<223> Amino Acid Sequence of Light Chain CDR3 of K090-02a022

<400> 94

Gln Asn His Tyr Gly Val Pro Trp Thr
1 5

<210> 95
<211> 5
<212> PRT
<213> artificial sequence

<220>
<223> Amino Acid Sequence of Heavy Chain CDR1 of K090-02a034

<400> 95

Gly Tyr Tyr Met His
1 5

<210> 96
<211> 17
<212> PRT
<213> artificial sequence

<220>
<223> Amino Acid Sequence of Heavy Chain CDR2 of K090-02a034

<400> 96

Glu Ile Asn Pro Ser Thr Gly Asp Thr Thr Tyr Asn Gln Lys Phe Lys
1 5 10 15

Ala

<210> 97
<211> 9
<212> PRT
<213> artificial sequence

<220>
<223> Amino Acid Sequence of Heavy Chain CDR3 of K090-02a034

<400> 97

Glu Arg Asn Tyr Asp Arg Phe Ala Tyr
1 5

<210> 98
<211> 10
<212> PRT
<213> artificial sequence

<220>
<223> Amino Acid Sequence of Light Chain CDR1 of K090-02a034

<400> 98

Ser Ala Ser Ser Ser Val Ser Tyr Met Tyr
1 5 10

<210> 99
<211> 7
<212> PRT
<213> artificial sequence

<220>
<223> Amino Acid Sequence of Light Chain CDR2 of K090-02a034

<400> 99

Asp Thr Ser Asn Leu Ala Ser
1 5

<210>  100
<211>  9
<212>  PRT
<213>  artificial sequence

<220>
<223>  Amino Acid Sequence of Light Chain CDR3 of K090-02a034

<400>  100

Gln Gln Trp Ser Ser Tyr Pro Leu Thr
1               5

<210>  101
<211>  5
<212>  PRT
<213>  artificial sequence

<220>
<223>  Amino Acid Sequence of Heavy Chain CDR1 of K090-02a035

<400>  101

Ser Tyr Trp Met His
1               5

<210>  102
<211>  17
<212>  PRT
<213>  artificial sequence

<220>
<223>  Amino Acid Sequence of Heavy Chain CDR2 of K090-02a035

<400>  102

Asn Ile Asp Pro Ser Asp Ser Glu Thr His Tyr Asn Gln Lys Phe Lys
1               5                   10                  15

Asp

<210>  103
<211>  4
<212>  PRT
<213>  artificial sequence

<220>
<223>  Amino Acid Sequence of Heavy Chain CDR3 of K090-02a035

<400>  103

Asn Leu Gly Tyr
1

<210>  104

<211> 16
<212> PRT
<213> artificial sequence

<220>
<223> Amino Acid Sequence of Light Chain CDR1 of K090-02a035

<400> 104

Arg Ser Ser Gln Ser Leu Val His Ser Asn Gly Asn Thr Tyr Leu His
1 5 10 15

<210> 105
<211> 7
<212> PRT
<213> artificial sequence

<220>
<223> Amino Acid Sequence of Light Chain CDR2 of K090-02a035

<400> 105

Lys Val Ser Asn Arg Phe Ser
1 5

<210> 106
<211> 9
<212> PRT
<213> artificial sequence

<220>
<223> Amino Acid Sequence of Light Chain CDR3 of K090-02a035

<400> 106

Ser Gln Ser Thr His Val Pro Tyr Thr
1 5

<210> 107
<211> 5
<212> PRT
<213> artificial sequence

<220>
<223> Amino Acid Sequence of Heavy Chain CDR1 of K090-02b006

<400> 107

Asp Tyr Gly Met His
1 5

<210> 108
<211> 17
<212> PRT
<213> artificial sequence

<220>
<223> Amino Acid Sequence of Heavy Chain CDR2 of K090-02b006

<400> 108

Tyr Ile Ser Ser Gly Ser Ser Thr Ile Tyr Tyr Ala Asp Thr Val Lys
1 5 10 15

Gly

<210> 109
<211> 16
<212> PRT
<213> artificial sequence

<220>
<223> Amino Acid Sequence of Heavy Chain CDR3 of K090-02b006

<400> 109

His Ser Tyr Tyr Asn Tyr Asp Ile Gly Gly Tyr Tyr Ala Met Asp Tyr
1 5 10 15

<210> 110
<211> 11
<212> PRT
<213> artificial sequence

<220>
<223> Amino Acid Sequence of Light Chain CDR1 of K090-02b006

<400> 110

Arg Ala Ser Gln Glu Ile Ser Gly Tyr Leu Ser
1 5 10

<210> 111
<211> 7
<212> PRT
<213> artificial sequence

<220>
<223> Amino Acid Sequence of Light Chain CDR2 of K090-02b006

<400> 111

Ala Ala Ser Thr Leu Asp Ser
1 5

<210> 112
<211> 9
<212> PRT
<213> artificial sequence

<220>
<223> Amino Acid Sequence of Light Chain CDR3 of K090-02b006

<400> 112

Leu Gln Tyr Ala Ser Tyr Pro Leu Thr

1 5

<210> 113
<211> 5
<212> PRT
<213> artificial sequence

<220>
<223> Amino Acid Sequence of Heavy Chain CDR1 of Clone 4

<400> 113

Arg Tyr Ala Met Tyr
1 5

<210> 114
<211> 16
<212> PRT
<213> artificial sequence

<220>
<223> Amino Acid Sequence of Heavy Chain CDR2 of Clone 4

<400> 114

Ile Gly Asn Thr Gly Arg Tyr Thr Gly Tyr Gly Ser Ala Val Lys Gly
1 5 10 15

<210> 115
<211> 14
<212> PRT
<213> artificial sequence

<220>
<223> Amino Acid Sequence of Heavy Chain CDR3 of Clone 4

<400> 115

Ser Val Ser Pro Tyr Cys Cys Asp Ala Ala Asp Ile Asp Ala
1 5 10

<210> 116
<211> 10
<212> PRT
<213> artificial sequence

<220>
<223> Amino Acid Sequence of Light Chain CDR1 of Clone 4

<400> 116

Ser Gly Gly Ser Ser Gly Tyr Ala Tyr Gly
1 5 10

<210> 117
<211> 7
<212> PRT
<213> artificial sequence

<220>
<223>  Amino Acid Sequence of Light Chain CDR2 of Clone 4

<400>  117

Ser Asn Asn Asn Arg Pro Ser
1               5

<210>  118
<211>  11
<212>  PRT
<213>  artificial sequence

<220>
<223>  Amino Acid Sequence of Light Chain CDR3 of Clone 4

<400>  118

Gly Ser Val Asp Ser Ser Ser Tyr Ala Gly Ile
1               5                   10

<210>  119
<211>  5
<212>  PRT
<213>  artificial sequence

<220>
<223>  Amino Acid Sequence of Heavy Chain CDR1 of Clone 18

<400>  119

Ser Val Asn Met Phe
1               5

<210>  120
<211>  17
<212>  PRT
<213>  artificial sequence

<220>
<223>  Amino Acid Sequence of Heavy Chain CDR2 of Clone 18

<400>  120

Gly Ile Asp Asn Asp Ala Thr Phe Thr Leu Tyr Gly Ser Ala Val Lys
1               5                   10                  15

Gly

<210>  121
<211>  19
<212>  PRT
<213>  artificial sequence

<220>
<223>  Amino Acid Sequence of Heavy Chain CDR3 of Clone 18

<400> 121

Thr Leu Cys Ser Thr Thr Trp Gly Cys Gly Ala Tyr Ser Ala Gly Asp
1 5 10 15

Ile Asp Ala

<210> 122
<211> 12
<212> PRT
<213> artificial sequence

<220>
<223> Amino Acid Sequence of Light Chain CDR1 of Clone 18

<400> 122

Ser Gly Gly Gly Ser Ser Gly Tyr Gly Tyr Tyr Gly
1 5 10

<210> 123
<211> 7
<212> PRT
<213> artificial sequence

<220>
<223> Amino Acid Sequence of Light Chain CDR2 of Clone 18

<400> 123

Asn Asn Asn Lys Arg Pro Thr
1 5

<210> 124
<211> 10
<212> PRT
<213> artificial sequence

<220>
<223> Amino Acid Sequence of Light Chain CDR3 of Clone 18

<400> 124

Gly Ser Arg Asp Asn Thr Tyr Val Gly Ile
1 5 10

<210> 125
<211> 1431
<212> DNA
<213> artificial sequence

<220>
<223> Nucleic Acid Sequence of Heavy Chain of K090-01a002

<220>

```
<221>  CDS
<222>  (16)..(1422)

<400>  125
aagcttgccg ccacc atg aaa cac ctg tgg ttc ttc ctc ctc ctg gtg gcg        51
                 Met Lys His Leu Trp Phe Phe Leu Leu Leu Val Ala
                 1               5                   10

gcc ccc aga tgg gtc ctg tcc gag gtt cag ctg cag cag tct gga cct         99
Ala Pro Arg Trp Val Leu Ser Glu Val Gln Leu Gln Gln Ser Gly Pro
        15                  20                  25

gaa ctg gtg aag cct ggg gct tca gtg aag ata tca tgc aag tct tct        147
Glu Leu Val Lys Pro Gly Ala Ser Val Lys Ile Ser Cys Lys Ser Ser
    30                  35                  40

ggt tac tca ttc act cgc tac tac atg cac tgg gtg aag cac agt cct        195
Gly Tyr Ser Phe Thr Arg Tyr Tyr Met His Trp Val Lys His Ser Pro
45                  50                  55                  60

gaa aag agc ctt gag tgg att gga gag att aat cct agc act ggt gat        243
Glu Lys Ser Leu Glu Trp Ile Gly Glu Ile Asn Pro Ser Thr Gly Asp
                65                  70                  75

act acg tac aac cag aag ttc aag gcc aag gcc aca ttg act gta gac        291
Thr Thr Tyr Asn Gln Lys Phe Lys Ala Lys Ala Thr Leu Thr Val Asp
            80                  85                  90

aaa tcc tcc agc aca gcc tac atg cag ctc aag agc ctg aca tct gag        339
Lys Ser Ser Ser Thr Ala Tyr Met Gln Leu Lys Ser Leu Thr Ser Glu
        95                  100                 105

gac tct gca gtc tat tac tgt gca aga gag aag aga gat gat ggc gta        387
Asp Ser Ala Val Tyr Tyr Cys Ala Arg Glu Lys Arg Asp Asp Gly Val
    110                 115                 120

ctt gct tac tgg ggc caa ggg act ctg gtc act gtc tcg agc gcc aaa        435
Leu Ala Tyr Trp Gly Gln Gly Thr Leu Val Thr Val Ser Ser Ala Lys
125                 130                 135                 140

aca aca gcc cca tcg gtc tat cca ctg gcc cct gtg tgt gga gat aca        483
Thr Thr Ala Pro Ser Val Tyr Pro Leu Ala Pro Val Cys Gly Asp Thr
                145                 150                 155

act ggc tcc tcg gtg act cta gga tgc ctg gtc aag ggt tat ttc cct        531
Thr Gly Ser Ser Val Thr Leu Gly Cys Leu Val Lys Gly Tyr Phe Pro
            160                 165                 170

gag cca gtg acc ttg acc tgg aac tct gga tcc ctg tcc agt ggt gtg        579
Glu Pro Val Thr Leu Thr Trp Asn Ser Gly Ser Leu Ser Ser Gly Val
        175                 180                 185

cac acc ttc cca gct gtc ctg cag tct gac ctc tac acc ctc agc agc        627
His Thr Phe Pro Ala Val Leu Gln Ser Asp Leu Tyr Thr Leu Ser Ser
    190                 195                 200

tca gtg act gta acc tcg agc acc tgg ccc agc cag tcc atc acc tgc        675
Ser Val Thr Val Thr Ser Ser Thr Trp Pro Ser Gln Ser Ile Thr Cys
205                 210                 215                 220

aat gtg gcc cac ccg gca agc agc acc aag gtg gac aag aaa att gag        723
Asn Val Ala His Pro Ala Ser Ser Thr Lys Val Asp Lys Lys Ile Glu
                225                 230                 235
```

```
ccc cgg gga ccc aca atc aag ccc tgt cct cca tgc aaa tgc cca gca      771
Pro Arg Gly Pro Thr Ile Lys Pro Cys Pro Pro Cys Lys Cys Pro Ala
            240                 245                 250

cct aac ctc ttg ggt gga cca tcc gtc ttc atc ttc cct cca aag atc      819
Pro Asn Leu Leu Gly Gly Pro Ser Val Phe Ile Phe Pro Pro Lys Ile
        255                 260                 265

aag gat gta ctc atg atc tcc ctg agc ccc ata gtc aca tgt gtg gtg      867
Lys Asp Val Leu Met Ile Ser Leu Ser Pro Ile Val Thr Cys Val Val
    270                 275                 280

gtg gat gtg agc gag gat gac cca gat gtc cag atc agc tgg ttt gtg      915
Val Asp Val Ser Glu Asp Asp Pro Asp Val Gln Ile Ser Trp Phe Val
285                 290                 295                 300

aac aac gtg gaa gta cac aca gct cag aca caa acc cat aga gag gat      963
Asn Asn Val Glu Val His Thr Ala Gln Thr Gln Thr His Arg Glu Asp
                305                 310                 315

tac aac agt act ctc cgg gtg gtc agt gcc ctc ccc atc cag cac cag     1011
Tyr Asn Ser Thr Leu Arg Val Val Ser Ala Leu Pro Ile Gln His Gln
            320                 325                 330

gac tgg atg agt ggc aag gag ttc aaa tgc aag gtc aac aac aaa gac     1059
Asp Trp Met Ser Gly Lys Glu Phe Lys Cys Lys Val Asn Asn Lys Asp
        335                 340                 345

ctc cca gcg ccc atc gag aga acc atc tca aaa ccc aaa ggg tca gta     1107
Leu Pro Ala Pro Ile Glu Arg Thr Ile Ser Lys Pro Lys Gly Ser Val
    350                 355                 360

aga gct cca cag gta tat gtc ttg cct cca cca gaa gaa gag atg act     1155
Arg Ala Pro Gln Val Tyr Val Leu Pro Pro Pro Glu Glu Glu Met Thr
365                 370                 375                 380

aag aaa cag gtc act ctg acc tgc atg gtc aca gac ttc atg cct gaa     1203
Lys Lys Gln Val Thr Leu Thr Cys Met Val Thr Asp Phe Met Pro Glu
                385                 390                 395

gac att tac gtg gag tgg acc aac aac ggg aaa aca gag cta aac tac     1251
Asp Ile Tyr Val Glu Trp Thr Asn Asn Gly Lys Thr Glu Leu Asn Tyr
            400                 405                 410

aag aac act gaa cca gtc ctg gac tct gat ggt tct tac ttc atg tac     1299
Lys Asn Thr Glu Pro Val Leu Asp Ser Asp Gly Ser Tyr Phe Met Tyr
        415                 420                 425

agc aag ctg aga gtg gaa aag aag aac tgg gtg gaa aga aat agc tac     1347
Ser Lys Leu Arg Val Glu Lys Lys Asn Trp Val Glu Arg Asn Ser Tyr
    430                 435                 440

tcc tgt tca gtg gtc cac gag ggt ctg cac aat cac cac acg act aag     1395
Ser Cys Ser Val Val His Glu Gly Leu His Asn His His Thr Thr Lys
445                 450                 455                 460

agc ttc tcc cgg act ccg ggt aaa tga tagtctaga                       1431
Ser Phe Ser Arg Thr Pro Gly Lys
                465
```

<210> 126

<211> 468
<212> PRT
<213> artificial sequence

<220>
<223> Synthetic Construct

<400> 126

```
Met Lys His Leu Trp Phe Phe Leu Leu Leu Val Ala Ala Pro Arg Trp
1               5                   10                  15

Val Leu Ser Glu Val Gln Leu Gln Gln Ser Gly Pro Glu Leu Val Lys
            20                  25                  30

Pro Gly Ala Ser Val Lys Ile Ser Cys Lys Ser Ser Gly Tyr Ser Phe
        35                  40                  45

Thr Arg Tyr Tyr Met His Trp Val Lys His Ser Pro Glu Lys Ser Leu
    50                  55                  60

Glu Trp Ile Gly Glu Ile Asn Pro Ser Thr Gly Asp Thr Thr Tyr Asn
65                  70                  75                  80

Gln Lys Phe Lys Ala Lys Ala Thr Leu Thr Val Asp Lys Ser Ser Ser
                85                  90                  95

Thr Ala Tyr Met Gln Leu Lys Ser Leu Thr Ser Glu Asp Ser Ala Val
            100                 105                 110

Tyr Tyr Cys Ala Arg Glu Lys Arg Asp Asp Gly Val Leu Ala Tyr Trp
        115                 120                 125

Gly Gln Gly Thr Leu Val Thr Val Ser Ser Ala Lys Thr Thr Ala Pro
    130                 135                 140

Ser Val Tyr Pro Leu Ala Pro Val Cys Gly Asp Thr Thr Gly Ser Ser
145                 150                 155                 160

Val Thr Leu Gly Cys Leu Val Lys Gly Tyr Phe Pro Glu Pro Val Thr
                165                 170                 175

Leu Thr Trp Asn Ser Gly Ser Leu Ser Ser Gly Val His Thr Phe Pro
            180                 185                 190

Ala Val Leu Gln Ser Asp Leu Tyr Thr Leu Ser Ser Ser Val Thr Val
        195                 200                 205

Thr Ser Ser Thr Trp Pro Ser Gln Ser Ile Thr Cys Asn Val Ala His
    210                 215                 220
```

```
Pro Ala Ser Ser Thr Lys Val Asp Lys Lys Ile Glu Pro Arg Gly Pro
225                 230                 235                 240

Thr Ile Lys Pro Cys Pro Pro Cys Lys Cys Pro Ala Pro Asn Leu Leu
                245                 250                 255

Gly Gly Pro Ser Val Phe Ile Phe Pro Pro Lys Ile Lys Asp Val Leu
            260                 265                 270

Met Ile Ser Leu Ser Pro Ile Val Thr Cys Val Val Val Asp Val Ser
        275                 280                 285

Glu Asp Asp Pro Asp Val Gln Ile Ser Trp Phe Val Asn Asn Val Glu
    290                 295                 300

Val His Thr Ala Gln Thr Gln Thr His Arg Glu Asp Tyr Asn Ser Thr
305                 310                 315                 320

Leu Arg Val Val Ser Ala Leu Pro Ile Gln His Gln Asp Trp Met Ser
                325                 330                 335

Gly Lys Glu Phe Lys Cys Lys Val Asn Asn Lys Asp Leu Pro Ala Pro
            340                 345                 350

Ile Glu Arg Thr Ile Ser Lys Pro Lys Gly Ser Val Arg Ala Pro Gln
        355                 360                 365

Val Tyr Val Leu Pro Pro Pro Glu Glu Glu Met Thr Lys Lys Gln Val
    370                 375                 380

Thr Leu Thr Cys Met Val Thr Asp Phe Met Pro Glu Asp Ile Tyr Val
385                 390                 395                 400

Glu Trp Thr Asn Asn Gly Lys Thr Glu Leu Asn Tyr Lys Asn Thr Glu
                405                 410                 415

Pro Val Leu Asp Ser Asp Gly Ser Tyr Phe Met Tyr Ser Lys Leu Arg
            420                 425                 430

Val Glu Lys Lys Asn Trp Val Glu Arg Asn Ser Tyr Ser Cys Ser Val
        435                 440                 445

Val His Glu Gly Leu His Asn His His Thr Thr Lys Ser Phe Ser Arg
    450                 455                 460

Thr Pro Gly Lys
```

465

<210> 127
<211> 731
<212> DNA
<213> artificial sequence

<220>
<223> Nucleic Acid Sequence of Light Chain of K090-01a002

<220>
<221> CDS
<222> (16)..(720)

<400> 127
```
aagcttgccg ccacc atg agg ctc ctt gct cag ctt ctg ggg ctg cta atg       51
                 Met Arg Leu Leu Ala Gln Leu Leu Gly Leu Leu Met
                 1               5                   10

ctc tgg gtc cct gga tcc agt ggg gac atc cag atg acc cag tct ccc        99
Leu Trp Val Pro Gly Ser Ser Gly Asp Ile Gln Met Thr Gln Ser Pro
        15                  20                  25

aaa ttc ctg cct gta tca gca gga gac agg gtt acc atg acc tgc aag       147
Lys Phe Leu Pro Val Ser Ala Gly Asp Arg Val Thr Met Thr Cys Lys
    30                  35                  40

gcc agt cag agt gtg ggt aat aat gta gcc tgg tac caa cag aag cca       195
Ala Ser Gln Ser Val Gly Asn Asn Val Ala Trp Tyr Gln Gln Lys Pro
45                  50                  55                  60

gga cag tct cct aaa ctg ctg ata tac tat gca tcc aat cgc tac act       243
Gly Gln Ser Pro Lys Leu Leu Ile Tyr Tyr Ala Ser Asn Arg Tyr Thr
                65                  70                  75

gga gtc cct gat cgc ttc act ggc agt gga tct ggg aca gat ttc act       291
Gly Val Pro Asp Arg Phe Thr Gly Ser Gly Ser Gly Thr Asp Phe Thr
            80                  85                  90

ttc acc atc agc agt gtg cag gtt gaa gac ctg gca gtt tat ttc tgt       339
Phe Thr Ile Ser Ser Val Gln Val Glu Asp Leu Ala Val Tyr Phe Cys
        95                  100                 105

cag cag cat tat agc tct cct ctc acg ttc ggt gct ggg acc aag ctg       387
Gln Gln His Tyr Ser Ser Pro Leu Thr Phe Gly Ala Gly Thr Lys Leu
    110                 115                 120

gag ctg aaa cgg gct gat gct gca cca act gta tcc atc ttc cca cca       435
Glu Leu Lys Arg Ala Asp Ala Ala Pro Thr Val Ser Ile Phe Pro Pro
125                 130                 135                 140

tcc agt gag cag tta aca tct gga ggt gcc tca gtc gtg tgc ttc ttg       483
Ser Ser Glu Gln Leu Thr Ser Gly Gly Ala Ser Val Val Cys Phe Leu
                145                 150                 155

aac aac ttc tac ccc aaa gac atc aat gtc aag tgg aag att gat ggc       531
Asn Asn Phe Tyr Pro Lys Asp Ile Asn Val Lys Trp Lys Ile Asp Gly
            160                 165                 170

agt gaa cga caa aat ggc gtc ctg aac agt tgg act gat cag gac agc       579
Ser Glu Arg Gln Asn Gly Val Leu Asn Ser Trp Thr Asp Gln Asp Ser
```

```
        175                 180                 185

aaa gac agc acc tac agc atg agc agc acc ctc acg ttg acc aag gac           627
Lys Asp Ser Thr Tyr Ser Met Ser Ser Thr Leu Thr Leu Thr Lys Asp
    190                 195                 200

gag tat gaa cga cat aac agc tat acc tgt gag gcc act cac aag aca           675
Glu Tyr Glu Arg His Asn Ser Tyr Thr Cys Glu Ala Thr His Lys Thr
205                 210                 215                 220

tca act tca ccc att gtc aag agc ttc aac agg aat gag tgt tga               720
Ser Thr Ser Pro Ile Val Lys Ser Phe Asn Arg Asn Glu Cys
                225                 230

tagggcgcgc c                                                              731
```

```
<210>  128
<211>  234
<212>  PRT
<213>  artificial sequence

<220>
<223>  Synthetic Construct

<400>  128

Met Arg Leu Leu Ala Gln Leu Leu Gly Leu Leu Met Leu Trp Val Pro
1               5                   10                  15

Gly Ser Ser Gly Asp Ile Gln Met Thr Gln Ser Pro Lys Phe Leu Pro
            20                  25                  30

Val Ser Ala Gly Asp Arg Val Thr Met Thr Cys Lys Ala Ser Gln Ser
        35                  40                  45

Val Gly Asn Asn Val Ala Trp Tyr Gln Gln Lys Pro Gly Gln Ser Pro
    50                  55                  60

Lys Leu Leu Ile Tyr Tyr Ala Ser Asn Arg Tyr Thr Gly Val Pro Asp
65                  70                  75                  80

Arg Phe Thr Gly Ser Gly Ser Gly Thr Asp Phe Thr Phe Thr Ile Ser
                85                  90                  95

Ser Val Gln Val Glu Asp Leu Ala Val Tyr Phe Cys Gln Gln His Tyr
            100                 105                 110

Ser Ser Pro Leu Thr Phe Gly Ala Gly Thr Lys Leu Glu Leu Lys Arg
        115                 120                 125

Ala Asp Ala Ala Pro Thr Val Ser Ile Phe Pro Pro Ser Ser Glu Gln
    130                 135                 140
```

```
Leu Thr Ser Gly Gly Ala Ser Val Val Cys Phe Leu Asn Asn Phe Tyr
145                 150                 155                 160


Pro Lys Asp Ile Asn Val Lys Trp Lys Ile Asp Gly Ser Glu Arg Gln
                165                 170                 175


Asn Gly Val Leu Asn Ser Trp Thr Asp Gln Asp Ser Lys Asp Ser Thr
            180                 185                 190


Tyr Ser Met Ser Ser Thr Leu Thr Leu Thr Lys Asp Glu Tyr Glu Arg
        195                 200                 205


His Asn Ser Tyr Thr Cys Glu Ala Thr His Lys Thr Ser Thr Ser Pro
    210                 215                 220


Ile Val Lys Ser Phe Asn Arg Asn Glu Cys
225                 230


<210>  129
<211>  1428
<212>  DNA
<213>  artificial sequence

<220>
<223>  Nucleic Acid Sequence of Heavy Chain of K090-01a007


<220>
<221>  CDS
<222>  (16)..(1419)

<400>  129
aagcttgccg ccacc atg aaa cac ctg tgg ttc ttc ctc ctc ctg gtg gcg        51
                 Met Lys His Leu Trp Phe Phe Leu Leu Leu Val Ala
                 1               5                   10

gcc ccc aga tgg gtc ctg tcc cag gtc cac ctg cag cag tct ggg gct        99
Ala Pro Arg Trp Val Leu Ser Gln Val His Leu Gln Gln Ser Gly Ala
        15                  20                  25

gag ctg gtg aag cct ggg gct tca gtg aag ctg tcc tgc aag gct tct       147
Glu Leu Val Lys Pro Gly Ala Ser Val Lys Leu Ser Cys Lys Ala Ser
    30                  35                  40

ggc tac acc ttc acc agc tac tgg atg cac tgg gtg aag cag agg cct       195
Gly Tyr Thr Phe Thr Ser Tyr Trp Met His Trp Val Lys Gln Arg Pro
45                  50                  55                  60

gga caa ggc ctt gaa tgg att ggt aat att gac cct tat gat agt gaa       243
Gly Gln Gly Leu Glu Trp Ile Gly Asn Ile Asp Pro Tyr Asp Ser Glu
                65                  70                  75

act cac tac aat caa aag ttc aag gac aag gcc aca ttg act gta gac       291
Thr His Tyr Asn Gln Lys Phe Lys Asp Lys Ala Thr Leu Thr Val Asp
            80                  85                  90

aaa tcc tcc agc aca gcc tac atg cag ctc agc agc ctg aca tct gag       339
```

```
Lys Ser Ser Ser Thr Ala Tyr Met Gln Leu Ser Ser Leu Thr Ser Glu
        95                  100                 105

gac tct gcg gtc tat tac tgt gca agg gat agt aac tgg ggc tac ttt       387
Asp Ser Ala Val Tyr Tyr Cys Ala Arg Asp Ser Asn Trp Gly Tyr Phe
    110                 115                 120

gac tac tgg ggc caa ggc acc act ctc aca gtc tcg agc gcc aaa aca       435
Asp Tyr Trp Gly Gln Gly Thr Thr Leu Thr Val Ser Ser Ala Lys Thr
125                 130                 135                 140

aca gcc cca tcg gtc tat cca ctg gcc cct gtg tgt gga gat aca act       483
Thr Ala Pro Ser Val Tyr Pro Leu Ala Pro Val Cys Gly Asp Thr Thr
                145                 150                 155

ggc tcc tcg gtg act cta gga tgc ctg gtc aag ggt tat ttc cct gag       531
Gly Ser Ser Val Thr Leu Gly Cys Leu Val Lys Gly Tyr Phe Pro Glu
            160                 165                 170

cca gtg acc ttg acc tgg aac tct gga tcc ctg tcc agt ggt gtg cac       579
Pro Val Thr Leu Thr Trp Asn Ser Gly Ser Leu Ser Ser Gly Val His
        175                 180                 185

acc ttc cca gct gtc ctg cag tct gac ctc tac acc ctc agc agc tca       627
Thr Phe Pro Ala Val Leu Gln Ser Asp Leu Tyr Thr Leu Ser Ser Ser
    190                 195                 200

gtg act gta acc tcg agc acc tgg ccc agc cag tcc atc acc tgc aat       675
Val Thr Val Thr Ser Ser Thr Trp Pro Ser Gln Ser Ile Thr Cys Asn
205                 210                 215                 220

gtg gcc cac ccg gca agc agc acc aag gtg gac aag aaa att gag ccc       723
Val Ala His Pro Ala Ser Ser Thr Lys Val Asp Lys Lys Ile Glu Pro
                225                 230                 235

cgg gga ccc aca atc aag ccc tgt cct cca tgc aaa tgc cca gca cct       771
Arg Gly Pro Thr Ile Lys Pro Cys Pro Pro Cys Lys Cys Pro Ala Pro
            240                 245                 250

aac ctc ttg ggt gga cca tcc gtc ttc atc ttc cct cca aag atc aag       819
Asn Leu Leu Gly Gly Pro Ser Val Phe Ile Phe Pro Pro Lys Ile Lys
        255                 260                 265

gat gta ctc atg atc tcc ctg agc ccc ata gtc aca tgt gtg gtg gtg       867
Asp Val Leu Met Ile Ser Leu Ser Pro Ile Val Thr Cys Val Val Val
    270                 275                 280

gat gtg agc gag gat gac cca gat gtc cag atc agc tgg ttt gtg aac       915
Asp Val Ser Glu Asp Asp Pro Asp Val Gln Ile Ser Trp Phe Val Asn
285                 290                 295                 300

aac gtg gaa gta cac aca gct cag aca caa acc cat aga gag gat tac       963
Asn Val Glu Val His Thr Ala Gln Thr Gln Thr His Arg Glu Asp Tyr
                305                 310                 315

aac agt act ctc cgg gtg gtc agt gcc ctc ccc atc cag cac cag gac      1011
Asn Ser Thr Leu Arg Val Val Ser Ala Leu Pro Ile Gln His Gln Asp
            320                 325                 330

tgg atg agt ggc aag gag ttc aaa tgc aag gtc aac aac aaa gac ctc      1059
Trp Met Ser Gly Lys Glu Phe Lys Cys Lys Val Asn Asn Lys Asp Leu
        335                 340                 345
```

```
cca gcg ccc atc gag aga acc atc tca aaa ccc aaa ggg tca gta aga       1107
Pro Ala Pro Ile Glu Arg Thr Ile Ser Lys Pro Lys Gly Ser Val Arg
    350                 355                 360

gct cca cag gta tat gtc ttg cct cca cca gaa gaa gag atg act aag       1155
Ala Pro Gln Val Tyr Val Leu Pro Pro Pro Glu Glu Glu Met Thr Lys
365                 370                 375                 380

aaa cag gtc act ctg acc tgc atg gtc aca gac ttc atg cct gaa gac       1203
Lys Gln Val Thr Leu Thr Cys Met Val Thr Asp Phe Met Pro Glu Asp
                385                 390                 395

att tac gtg gag tgg acc aac aac ggg aaa aca gag cta aac tac aag       1251
Ile Tyr Val Glu Trp Thr Asn Asn Gly Lys Thr Glu Leu Asn Tyr Lys
            400                 405                 410

aac act gaa cca gtc ctg gac tct gat ggt tct tac ttc atg tac agc       1299
Asn Thr Glu Pro Val Leu Asp Ser Asp Gly Ser Tyr Phe Met Tyr Ser
        415                 420                 425

aag ctg aga gtg gaa aag aag aac tgg gtg gaa aga aat agc tac tcc       1347
Lys Leu Arg Val Glu Lys Lys Asn Trp Val Glu Arg Asn Ser Tyr Ser
    430                 435                 440

tgt tca gtg gtc cac gag ggt ctg cac aat cac cac acg act aag agc       1395
Cys Ser Val Val His Glu Gly Leu His Asn His His Thr Thr Lys Ser
445                 450                 455                 460

ttc tcc cgg act ccg ggt aaa tga tagtctaga                             1428
Phe Ser Arg Thr Pro Gly Lys
                465


<210>  130
<211>  467
<212>  PRT
<213>  artificial sequence

<220>
<223>  Synthetic Construct

<400>  130

Met Lys His Leu Trp Phe Phe Leu Leu Leu Val Ala Ala Pro Arg Trp
1               5                   10                  15


Val Leu Ser Gln Val His Leu Gln Gln Ser Gly Ala Glu Leu Val Lys
            20                  25                  30


Pro Gly Ala Ser Val Lys Leu Ser Cys Lys Ala Ser Gly Tyr Thr Phe
        35                  40                  45


Thr Ser Tyr Trp Met His Trp Val Lys Gln Arg Pro Gly Gln Gly Leu
    50                  55                  60


Glu Trp Ile Gly Asn Ile Asp Pro Tyr Asp Ser Glu Thr His Tyr Asn
65                  70                  75                  80
```

Gln Lys Phe Lys Asp Lys Ala Thr Leu Thr Val Asp Lys Ser Ser Ser
85 90 95

Thr Ala Tyr Met Gln Leu Ser Ser Leu Thr Ser Glu Asp Ser Ala Val
100 105 110

Tyr Tyr Cys Ala Arg Asp Ser Asn Trp Gly Tyr Phe Asp Tyr Trp Gly
115 120 125

Gln Gly Thr Thr Leu Thr Val Ser Ser Ala Lys Thr Thr Ala Pro Ser
130 135 140

Val Tyr Pro Leu Ala Pro Val Cys Gly Asp Thr Thr Gly Ser Ser Val
145 150 155 160

Thr Leu Gly Cys Leu Val Lys Gly Tyr Phe Pro Glu Pro Val Thr Leu
165 170 175

Thr Trp Asn Ser Gly Ser Leu Ser Ser Gly Val His Thr Phe Pro Ala
180 185 190

Val Leu Gln Ser Asp Leu Tyr Thr Leu Ser Ser Ser Val Thr Val Thr
195 200 205

Ser Ser Thr Trp Pro Ser Gln Ser Ile Thr Cys Asn Val Ala His Pro
210 215 220

Ala Ser Ser Thr Lys Val Asp Lys Lys Ile Glu Pro Arg Gly Pro Thr
225 230 235 240

Ile Lys Pro Cys Pro Pro Cys Lys Cys Pro Ala Pro Asn Leu Leu Gly
245 250 255

Gly Pro Ser Val Phe Ile Phe Pro Pro Lys Ile Lys Asp Val Leu Met
260 265 270

Ile Ser Leu Ser Pro Ile Val Thr Cys Val Val Val Asp Val Ser Glu
275 280 285

Asp Asp Pro Asp Val Gln Ile Ser Trp Phe Val Asn Asn Val Glu Val
290 295 300

His Thr Ala Gln Thr Gln Thr His Arg Glu Asp Tyr Asn Ser Thr Leu
305 310 315 320

Arg Val Val Ser Ala Leu Pro Ile Gln His Gln Asp Trp Met Ser Gly
325 330 335

```
Lys Glu Phe Lys Cys Lys Val Asn Asn Lys Asp Leu Pro Ala Pro Ile
            340                 345                 350

Glu Arg Thr Ile Ser Lys Pro Lys Gly Ser Val Arg Ala Pro Gln Val
        355                 360                 365

Tyr Val Leu Pro Pro Pro Glu Glu Glu Met Thr Lys Lys Gln Val Thr
    370                 375                 380

Leu Thr Cys Met Val Thr Asp Phe Met Pro Glu Asp Ile Tyr Val Glu
385                 390                 395                 400

Trp Thr Asn Asn Gly Lys Thr Glu Leu Asn Tyr Lys Asn Thr Glu Pro
                405                 410                 415

Val Leu Asp Ser Asp Gly Ser Tyr Phe Met Tyr Ser Lys Leu Arg Val
            420                 425                 430

Glu Lys Lys Asn Trp Val Glu Arg Asn Ser Tyr Ser Cys Ser Val Val
        435                 440                 445

His Glu Gly Leu His Asn His His Thr Thr Lys Ser Phe Ser Arg Thr
    450                 455                 460

Pro Gly Lys
465


<210>  131
<211>  746
<212>  DNA
<213>  artificial sequence

<220>
<223>  Nucleic Acid Sequence of Light Chain of K090-01a007


<220>
<221>  CDS
<222>  (16)..(735)

<400>  131
aagcttgccg ccacc atg agg ctc ctt gct cag ctt ctg ggg ctg cta atg        51
                 Met Arg Leu Leu Ala Gln Leu Leu Gly Leu Leu Met
                 1               5                   10

ctc tgg gtc cct gga tcc agt ggg gat gtt gtg atg acc caa act cca         99
Leu Trp Val Pro Gly Ser Ser Gly Asp Val Val Met Thr Gln Thr Pro
        15                  20                  25

ctc tcc ctg cct gtc agt ctt gga gat caa gcc tcc atc tct tgc aga        147
Leu Ser Leu Pro Val Ser Leu Gly Asp Gln Ala Ser Ile Ser Cys Arg
    30                  35                  40
```

```
tct agt cag agc ctt gta cac agt aat gga aac acc tat tta cat tgg        195
Ser Ser Gln Ser Leu Val His Ser Asn Gly Asn Thr Tyr Leu His Trp
45                  50                  55                  60

tac ctg cag aag cca ggc cag tct cca aag ctc ctg atc tac aaa gtt        243
Tyr Leu Gln Lys Pro Gly Gln Ser Pro Lys Leu Leu Ile Tyr Lys Val
                65                  70                  75

tcc aac cga ttt tct ggg gtc cca gac agg ttc agt ggc agt gga tca        291
Ser Asn Arg Phe Ser Gly Val Pro Asp Arg Phe Ser Gly Ser Gly Ser
            80                  85                  90

ggg aca gat ttc aca ctc aag atc agc aga gtg gag gct gag gat ctg        339
Gly Thr Asp Phe Thr Leu Lys Ile Ser Arg Val Glu Ala Glu Asp Leu
        95                  100                 105

gga gtt tat ttc tgc tct caa agt aca cat gtt ccg tgg acg ttc ggt        387
Gly Val Tyr Phe Cys Ser Gln Ser Thr His Val Pro Trp Thr Phe Gly
    110                 115                 120

gga ggc acc aag ctg gag ctg aaa cgg gct gat gct gca cca act gta        435
Gly Gly Thr Lys Leu Glu Leu Lys Arg Ala Asp Ala Ala Pro Thr Val
125                 130                 135                 140

tcc atc ttc cca cca tcc agt gag cag tta aca tct gga ggt gcc tca        483
Ser Ile Phe Pro Pro Ser Ser Glu Gln Leu Thr Ser Gly Gly Ala Ser
                145                 150                 155

gtc gtg tgc ttc ttg aac aac ttc tac ccc aaa gac atc aat gtc aag        531
Val Val Cys Phe Leu Asn Asn Phe Tyr Pro Lys Asp Ile Asn Val Lys
            160                 165                 170

tgg aag att gat ggc agt gaa cga caa aat ggc gtc ctg aac agt tgg        579
Trp Lys Ile Asp Gly Ser Glu Arg Gln Asn Gly Val Leu Asn Ser Trp
        175                 180                 185

act gat cag gac agc aaa gac agc acc tac agc atg agc agc acc ctc        627
Thr Asp Gln Asp Ser Lys Asp Ser Thr Tyr Ser Met Ser Ser Thr Leu
    190                 195                 200

acg ttg acc aag gac gag tat gaa cga cat aac agc tat acc tgt gag        675
Thr Leu Thr Lys Asp Glu Tyr Glu Arg His Asn Ser Tyr Thr Cys Glu
205                 210                 215                 220

gcc act cac aag aca tca act tca ccc att gtc aag agc ttc aac agg        723
Ala Thr His Lys Thr Ser Thr Ser Pro Ile Val Lys Ser Phe Asn Arg
                225                 230                 235

aat gag tgt tga tagggcgcgc c                                           746
Asn Glu Cys


<210>  132
<211>  239
<212>  PRT
<213>  artificial sequence

<220>
<223>  Synthetic Construct

<400>  132
```

```
Met Arg Leu Leu Ala Gln Leu Leu Gly Leu Leu Met Leu Trp Val Pro
1               5                   10                  15

Gly Ser Ser Gly Asp Val Val Met Thr Gln Thr Pro Leu Ser Leu Pro
            20                  25                  30

Val Ser Leu Gly Asp Gln Ala Ser Ile Ser Cys Arg Ser Ser Gln Ser
        35                  40                  45

Leu Val His Ser Asn Gly Asn Thr Tyr Leu His Trp Tyr Leu Gln Lys
    50                  55                  60

Pro Gly Gln Ser Pro Lys Leu Leu Ile Tyr Lys Val Ser Asn Arg Phe
65                  70                  75                  80

Ser Gly Val Pro Asp Arg Phe Ser Gly Ser Gly Ser Gly Thr Asp Phe
                85                  90                  95

Thr Leu Lys Ile Ser Arg Val Glu Ala Glu Asp Leu Gly Val Tyr Phe
            100                 105                 110

Cys Ser Gln Ser Thr His Val Pro Trp Thr Phe Gly Gly Gly Thr Lys
        115                 120                 125

Leu Glu Leu Lys Arg Ala Asp Ala Ala Pro Thr Val Ser Ile Phe Pro
    130                 135                 140

Pro Ser Ser Glu Gln Leu Thr Ser Gly Gly Ala Ser Val Val Cys Phe
145                 150                 155                 160

Leu Asn Asn Phe Tyr Pro Lys Asp Ile Asn Val Lys Trp Lys Ile Asp
                165                 170                 175

Gly Ser Glu Arg Gln Asn Gly Val Leu Asn Ser Trp Thr Asp Gln Asp
            180                 185                 190

Ser Lys Asp Ser Thr Tyr Ser Met Ser Ser Thr Leu Thr Leu Thr Lys
        195                 200                 205

Asp Glu Tyr Glu Arg His Asn Ser Tyr Thr Cys Glu Ala Thr His Lys
    210                 215                 220

Thr Ser Thr Ser Pro Ile Val Lys Ser Phe Asn Arg Asn Glu Cys
225                 230                 235
```

<210> 133
<211> 1446
<212> DNA

```
<213>  artificial sequence

<220>
<223>  Nucleic Acid Sequence of Heavy Chain of K090-01a016


<220>
<221>  CDS
<222>  (16)..(1437)

<400>  133
aagcttgccg ccacc atg aaa cac ctg tgg ttc ttc ctc ctc ctg gtg gcg        51
                 Met Lys His Leu Trp Phe Phe Leu Leu Leu Val Ala
                 1               5                   10

gcc ccc aga tgg gtc ctg tcc cag gtc cac ctg cag cag tct gga cct         99
Ala Pro Arg Trp Val Leu Ser Gln Val His Leu Gln Gln Ser Gly Pro
        15                  20                  25

gag ctg gtg aag cct ggg gct tca gtg aag ata tcc tgc aag gct tct        147
Glu Leu Val Lys Pro Gly Ala Ser Val Lys Ile Ser Cys Lys Ala Ser
    30                  35                  40

ggc tac acc ttc act gac tac tat ata aac tgg gtg aag cag aag cct        195
Gly Tyr Thr Phe Thr Asp Tyr Tyr Ile Asn Trp Val Lys Gln Lys Pro
45                  50                  55                  60

gga cag ggc ctt gag tgg att gga tat att gat cct tac aat gat ggt        243
Gly Gln Gly Leu Glu Trp Ile Gly Tyr Ile Asp Pro Tyr Asn Asp Gly
                65                  70                  75

tct aag tac aat gag aag ttc aaa ggc aag gcc aca ctg act tca gac        291
Ser Lys Tyr Asn Glu Lys Phe Lys Gly Lys Ala Thr Leu Thr Ser Asp
            80                  85                  90

aag tcc tcc agc aca gcc tac atg gag ctc agc agt ctg acc tct gag        339
Lys Ser Ser Ser Thr Ala Tyr Met Glu Leu Ser Ser Leu Thr Ser Glu
        95                  100                 105

gac tct gcg gtc tat tac tgt gca aga acg ccc tac tat agt ttc tat        387
Asp Ser Ala Val Tyr Tyr Cys Ala Arg Thr Pro Tyr Tyr Ser Phe Tyr
    110                 115                 120

agt tac ggg ttc tac ttt gac tac tgg ggc caa ggc acc act ctc aca        435
Ser Tyr Gly Phe Tyr Phe Asp Tyr Trp Gly Gln Gly Thr Thr Leu Thr
125                 130                 135                 140

gtc tcg agc gcc aaa aca aca gcc cca tcg gtc tat cca ctg gcc cct        483
Val Ser Ser Ala Lys Thr Thr Ala Pro Ser Val Tyr Pro Leu Ala Pro
                145                 150                 155

gtg tgt gga gat aca act ggc tcc tcg gtg act cta gga tgc ctg gtc        531
Val Cys Gly Asp Thr Thr Gly Ser Ser Val Thr Leu Gly Cys Leu Val
            160                 165                 170

aag ggt tat ttc cct gag cca gtg acc ttg acc tgg aac tct gga tcc        579
Lys Gly Tyr Phe Pro Glu Pro Val Thr Leu Thr Trp Asn Ser Gly Ser
        175                 180                 185

ctg tcc agt ggt gtg cac acc ttc cca gct gtc ctg cag tct gac ctc        627
Leu Ser Ser Gly Val His Thr Phe Pro Ala Val Leu Gln Ser Asp Leu
    190                 195                 200
```

```
tac acc ctc agc agc tca gtg act gta acc tcg agc acc tgg ccc agc      675
Tyr Thr Leu Ser Ser Ser Val Thr Val Thr Ser Ser Thr Trp Pro Ser
205                 210                 215                 220

cag tcc atc acc tgc aat gtg gcc cac ccg gca agc agc acc aag gtg      723
Gln Ser Ile Thr Cys Asn Val Ala His Pro Ala Ser Ser Thr Lys Val
                225                 230                 235

gac aag aaa att gag ccc cgg gga ccc aca atc aag ccc tgt cct cca      771
Asp Lys Lys Ile Glu Pro Arg Gly Pro Thr Ile Lys Pro Cys Pro Pro
            240                 245                 250

tgc aaa tgc cca gca cct aac ctc ttg ggt gga cca tcc gtc ttc atc      819
Cys Lys Cys Pro Ala Pro Asn Leu Leu Gly Gly Pro Ser Val Phe Ile
        255                 260                 265

ttc cct cca aag atc aag gat gta ctc atg atc tcc ctg agc ccc ata      867
Phe Pro Pro Lys Ile Lys Asp Val Leu Met Ile Ser Leu Ser Pro Ile
    270                 275                 280

gtc aca tgt gtg gtg gtg gat gtg agc gag gat gac cca gat gtc cag      915
Val Thr Cys Val Val Val Asp Val Ser Glu Asp Asp Pro Asp Val Gln
285                 290                 295                 300

atc agc tgg ttt gtg aac aac gtg gaa gta cac aca gct cag aca caa      963
Ile Ser Trp Phe Val Asn Asn Val Glu Val His Thr Ala Gln Thr Gln
                305                 310                 315

acc cat aga gag gat tac aac agt act ctc cgg gtg gtc agt gcc ctc     1011
Thr His Arg Glu Asp Tyr Asn Ser Thr Leu Arg Val Val Ser Ala Leu
            320                 325                 330

ccc atc cag cac cag gac tgg atg agt ggc aag gag ttc aaa tgc aag     1059
Pro Ile Gln His Gln Asp Trp Met Ser Gly Lys Glu Phe Lys Cys Lys
        335                 340                 345

gtc aac aac aaa gac ctc cca gcg ccc atc gag aga acc atc tca aaa     1107
Val Asn Asn Lys Asp Leu Pro Ala Pro Ile Glu Arg Thr Ile Ser Lys
    350                 355                 360

ccc aaa ggg tca gta aga gct cca cag gta tat gtc ttg cct cca cca     1155
Pro Lys Gly Ser Val Arg Ala Pro Gln Val Tyr Val Leu Pro Pro Pro
365                 370                 375                 380

gaa gaa gag atg act aag aaa cag gtc act ctg acc tgc atg gtc aca     1203
Glu Glu Glu Met Thr Lys Lys Gln Val Thr Leu Thr Cys Met Val Thr
                385                 390                 395

gac ttc atg cct gaa gac att tac gtg gag tgg acc aac aac ggg aaa     1251
Asp Phe Met Pro Glu Asp Ile Tyr Val Glu Trp Thr Asn Asn Gly Lys
            400                 405                 410

aca gag cta aac tac aag aac act gaa cca gtc ctg gac tct gat ggt     1299
Thr Glu Leu Asn Tyr Lys Asn Thr Glu Pro Val Leu Asp Ser Asp Gly
        415                 420                 425

tct tac ttc atg tac agc aag ctg aga gtg gaa aag aag aac tgg gtg     1347
Ser Tyr Phe Met Tyr Ser Lys Leu Arg Val Glu Lys Lys Asn Trp Val
    430                 435                 440

gaa aga aat agc tac tcc tgt tca gtg gtc cac gag ggt ctg cac aat     1395
Glu Arg Asn Ser Tyr Ser Cys Ser Val Val His Glu Gly Leu His Asn
445                 450                 455                 460
```

```
cac cac acg act aag agc ttc tcc cgg act ccg ggt aaa tga tagtctaga      1446
His His Thr Thr Lys Ser Phe Ser Arg Thr Pro Gly Lys
                465                 470


<210>  134
<211>  473
<212>  PRT
<213>  artificial sequence

<220>
<223>  Synthetic Construct

<400>  134

Met Lys His Leu Trp Phe Phe Leu Leu Leu Val Ala Ala Pro Arg Trp
1               5                   10                  15


Val Leu Ser Gln Val His Leu Gln Gln Ser Gly Pro Glu Leu Val Lys
            20                  25                  30


Pro Gly Ala Ser Val Lys Ile Ser Cys Lys Ala Ser Gly Tyr Thr Phe
        35                  40                  45


Thr Asp Tyr Tyr Ile Asn Trp Val Lys Gln Lys Pro Gly Gln Gly Leu
    50                  55                  60


Glu Trp Ile Gly Tyr Ile Asp Pro Tyr Asn Asp Gly Ser Lys Tyr Asn
65                  70                  75                  80


Glu Lys Phe Lys Gly Lys Ala Thr Leu Thr Ser Asp Lys Ser Ser Ser
                85                  90                  95


Thr Ala Tyr Met Glu Leu Ser Ser Leu Thr Ser Glu Asp Ser Ala Val
            100                 105                 110


Tyr Tyr Cys Ala Arg Thr Pro Tyr Tyr Ser Phe Tyr Ser Tyr Gly Phe
        115                 120                 125


Tyr Phe Asp Tyr Trp Gly Gln Gly Thr Thr Leu Thr Val Ser Ser Ala
    130                 135                 140


Lys Thr Thr Ala Pro Ser Val Tyr Pro Leu Ala Pro Val Cys Gly Asp
145                 150                 155                 160


Thr Thr Gly Ser Ser Val Thr Leu Gly Cys Leu Val Lys Gly Tyr Phe
                165                 170                 175


Pro Glu Pro Val Thr Leu Thr Trp Asn Ser Gly Ser Leu Ser Ser Gly
            180                 185                 190
```

Val His Thr Phe Pro Ala Val Leu Gln Ser Asp Leu Tyr Thr Leu Ser
195 200 205

Ser Ser Val Thr Val Thr Ser Ser Thr Trp Pro Ser Gln Ser Ile Thr
210 215 220

Cys Asn Val Ala His Pro Ala Ser Ser Thr Lys Val Asp Lys Lys Ile
225 230 235 240

Glu Pro Arg Gly Pro Thr Ile Lys Pro Cys Pro Pro Cys Lys Cys Pro
245 250 255

Ala Pro Asn Leu Leu Gly Gly Pro Ser Val Phe Ile Phe Pro Pro Lys
260 265 270

Ile Lys Asp Val Leu Met Ile Ser Leu Ser Pro Ile Val Thr Cys Val
275 280 285

Val Val Asp Val Ser Glu Asp Asp Pro Asp Val Gln Ile Ser Trp Phe
290 295 300

Val Asn Asn Val Glu Val His Thr Ala Gln Thr Gln Thr His Arg Glu
305 310 315 320

Asp Tyr Asn Ser Thr Leu Arg Val Val Ser Ala Leu Pro Ile Gln His
325 330 335

Gln Asp Trp Met Ser Gly Lys Glu Phe Lys Cys Lys Val Asn Asn Lys
340 345 350

Asp Leu Pro Ala Pro Ile Glu Arg Thr Ile Ser Lys Pro Lys Gly Ser
355 360 365

Val Arg Ala Pro Gln Val Tyr Val Leu Pro Pro Pro Glu Glu Glu Met
370 375 380

Thr Lys Lys Gln Val Thr Leu Thr Cys Met Val Thr Asp Phe Met Pro
385 390 395 400

Glu Asp Ile Tyr Val Glu Trp Thr Asn Asn Gly Lys Thr Glu Leu Asn
405 410 415

Tyr Lys Asn Thr Glu Pro Val Leu Asp Ser Asp Gly Ser Tyr Phe Met
420 425 430

Tyr Ser Lys Leu Arg Val Glu Lys Lys Asn Trp Val Glu Arg Asn Ser
435 440 445

```
Tyr Ser Cys Ser Val Val His Glu Gly Leu His Asn His His Thr Thr
    450                 455                 460


Lys Ser Phe Ser Arg Thr Pro Gly Lys
465                 470


<210>  135
<211>  743
<212>  DNA
<213>  artificial sequence

<220>
<223>  Nucleic Acid Sequence of Light Chain of K090-01a016


<220>
<221>  CDS
<222>  (16)..(732)

<400>  135
aagcttgccg ccacc atg agg ctc ctt gct cag ctt ctg ggg ctg cta atg        51
                 Met Arg Leu Leu Ala Gln Leu Leu Gly Leu Leu Met
                 1               5                   10

ctc tgg gtc cct gga tcc agt ggg gac att gtg ctg aca cag tct cct         99
Leu Trp Val Pro Gly Ser Ser Gly Asp Ile Val Leu Thr Gln Ser Pro
        15                  20                  25

gct tcc tta gct gta tct ctg ggg cag agg gcc acc atc tcc tgc agg        147
Ala Ser Leu Ala Val Ser Leu Gly Gln Arg Ala Thr Ile Ser Cys Arg
    30                  35                  40

gcc agc aaa agt gtc agt aca tct agc tat agt tac atg cac tgg tac        195
Ala Ser Lys Ser Val Ser Thr Ser Ser Tyr Ser Tyr Met His Trp Tyr
45                  50                  55                  60

caa cag aaa cca gga cag cca ccc aaa ctc ctc atc aag tat gca tcc        243
Gln Gln Lys Pro Gly Gln Pro Pro Lys Leu Leu Ile Lys Tyr Ala Ser
                65                  70                  75

tac cta gaa tct ggg gtt cct gcc agg ttc agt ggc agt ggg tct ggg        291
Tyr Leu Glu Ser Gly Val Pro Ala Arg Phe Ser Gly Ser Gly Ser Gly
            80                  85                  90

aca gac ttc acc ctc aac atc cat cct gtg gag gag gag gat gct gca        339
Thr Asp Phe Thr Leu Asn Ile His Pro Val Glu Glu Glu Asp Ala Ala
        95                  100                 105

aca tat tac tgt cag cac agt agg gag ttt ccg ctc acg ttc ggt gct        387
Thr Tyr Tyr Cys Gln His Ser Arg Glu Phe Pro Leu Thr Phe Gly Ala
    110                 115                 120

ggg acc aag ctg gag ctg aaa cgg gct gat gct gca cca act gta tcc        435
Gly Thr Lys Leu Glu Leu Lys Arg Ala Asp Ala Ala Pro Thr Val Ser
125                 130                 135                 140

atc ttc cca cca tcc agt gag cag tta aca tct gga ggt gcc tca gtc        483
Ile Phe Pro Pro Ser Ser Glu Gln Leu Thr Ser Gly Gly Ala Ser Val
                145                 150                 155
```

```
gtg tgc ttc ttg aac aac ttc tac ccc aaa gac atc aat gtc aag tgg         531
Val Cys Phe Leu Asn Asn Phe Tyr Pro Lys Asp Ile Asn Val Lys Trp
            160                 165                 170

aag att gat ggc agt gaa cga caa aat ggc gtc ctg aac agt tgg act         579
Lys Ile Asp Gly Ser Glu Arg Gln Asn Gly Val Leu Asn Ser Trp Thr
        175                 180                 185

gat cag gac agc aaa gac agc acc tac agc atg agc agc acc ctc acg         627
Asp Gln Asp Ser Lys Asp Ser Thr Tyr Ser Met Ser Ser Thr Leu Thr
    190                 195                 200

ttg acc aag gac gag tat gaa cga cat aac agc tat acc tgt gag gcc         675
Leu Thr Lys Asp Glu Tyr Glu Arg His Asn Ser Tyr Thr Cys Glu Ala
205                 210                 215                 220

act cac aag aca tca act tca ccc att gtc aag agc ttc aac agg aat         723
Thr His Lys Thr Ser Thr Ser Pro Ile Val Lys Ser Phe Asn Arg Asn
                225                 230                 235

gag tgt tga tagggcgcgc c                                                743
Glu Cys



<210>  136
<211>  238
<212>  PRT
<213>  artificial sequence

<220>
<223>  Synthetic Construct

<400>  136

Met Arg Leu Leu Ala Gln Leu Leu Gly Leu Leu Met Leu Trp Val Pro
1               5                   10                  15


Gly Ser Ser Gly Asp Ile Val Leu Thr Gln Ser Pro Ala Ser Leu Ala
            20                  25                  30


Val Ser Leu Gly Gln Arg Ala Thr Ile Ser Cys Arg Ala Ser Lys Ser
        35                  40                  45


Val Ser Thr Ser Ser Tyr Ser Tyr Met His Trp Tyr Gln Gln Lys Pro
    50                  55                  60


Gly Gln Pro Pro Lys Leu Leu Ile Lys Tyr Ala Ser Tyr Leu Glu Ser
65                  70                  75                  80


Gly Val Pro Ala Arg Phe Ser Gly Ser Gly Ser Gly Thr Asp Phe Thr
                85                  90                  95


Leu Asn Ile His Pro Val Glu Glu Glu Asp Ala Ala Thr Tyr Tyr Cys
            100                 105                 110
```

```
Gln His Ser Arg Glu Phe Pro Leu Thr Phe Gly Ala Gly Thr Lys Leu
        115                 120                 125

Glu Leu Lys Arg Ala Asp Ala Ala Pro Thr Val Ser Ile Phe Pro Pro
    130                 135                 140

Ser Ser Glu Gln Leu Thr Ser Gly Gly Ala Ser Val Val Cys Phe Leu
145                 150                 155                 160

Asn Asn Phe Tyr Pro Lys Asp Ile Asn Val Lys Trp Lys Ile Asp Gly
                165                 170                 175

Ser Glu Arg Gln Asn Gly Val Leu Asn Ser Trp Thr Asp Gln Asp Ser
            180                 185                 190

Lys Asp Ser Thr Tyr Ser Met Ser Ser Thr Leu Thr Leu Thr Lys Asp
        195                 200                 205

Glu Tyr Glu Arg His Asn Ser Tyr Thr Cys Glu Ala Thr His Lys Thr
    210                 215                 220

Ser Thr Ser Pro Ile Val Lys Ser Phe Asn Arg Asn Glu Cys
225                 230                 235


<210>  137
<211>  1440
<212>  DNA
<213>  artificial sequence

<220>
<223>  Nucleic Acid Sequence of Heavy Chain of K090-01a017


<220>
<221>  CDS
<222>  (16)..(1431)

<400>  137
aagcttgccg ccacc atg aaa cac ctg tgg ttc ttc ctc ctc ctg gtg gcg        51
                 Met Lys His Leu Trp Phe Phe Leu Leu Leu Val Ala
                 1               5                   10

gcc ccc aga tgg gtc ctg tcc gag gtt cag ctg cag cag tct gga gga         99
Ala Pro Arg Trp Val Leu Ser Glu Val Gln Leu Gln Gln Ser Gly Gly
        15                  20                  25

gag ctg gta agg cct ggg act tca gtg aag atg tcc tgc aag gca gct        147
Glu Leu Val Arg Pro Gly Thr Ser Val Lys Met Ser Cys Lys Ala Ala
    30                  35                  40

gga tac acc ttc act aac tac tgg ata ggt tgg gta aag cag agg cct        195
Gly Tyr Thr Phe Thr Asn Tyr Trp Ile Gly Trp Val Lys Gln Arg Pro
45                  50                  55                  60
```

```
gga cat ggc ctt gag tgg att gga gat ctt tac cct gga ggt ggt tat      243
Gly His Gly Leu Glu Trp Ile Gly Asp Leu Tyr Pro Gly Gly Gly Tyr
                65                  70                  75

act cac tac aat gag aag ttc aag ggc aag gcc aca ctg act gca gac      291
Thr His Tyr Asn Glu Lys Phe Lys Gly Lys Ala Thr Leu Thr Ala Asp
            80                  85                  90

aca tcc tcc agc aca gcc tac atg cag ctc agc agc ctg aca tct gag      339
Thr Ser Ser Ser Thr Ala Tyr Met Gln Leu Ser Ser Leu Thr Ser Glu
        95                  100                 105

gac tct gcc atc tat tac tgt gca aga gag agg gtt tac ttc gat ggt      387
Asp Ser Ala Ile Tyr Tyr Cys Ala Arg Glu Arg Val Tyr Phe Asp Gly
    110                 115                 120

agc cgg tac ttc gat gtc tgg ggc gca ggg acc acg gtc acc gtc tcg      435
Ser Arg Tyr Phe Asp Val Trp Gly Ala Gly Thr Thr Val Thr Val Ser
125                 130                 135                 140

agc gcc aaa aca aca gcc cca tcg gtc tat cca ctg gcc cct gtg tgt      483
Ser Ala Lys Thr Thr Ala Pro Ser Val Tyr Pro Leu Ala Pro Val Cys
                145                 150                 155

gga gat aca act ggc tcc tcg gtg act cta gga tgc ctg gtc aag ggt      531
Gly Asp Thr Thr Gly Ser Ser Val Thr Leu Gly Cys Leu Val Lys Gly
            160                 165                 170

tat ttc cct gag cca gtg acc ttg acc tgg aac tct gga tcc ctg tcc      579
Tyr Phe Pro Glu Pro Val Thr Leu Thr Trp Asn Ser Gly Ser Leu Ser
        175                 180                 185

agt ggt gtg cac acc ttc cca gct gtc ctg cag tct gac ctc tac acc      627
Ser Gly Val His Thr Phe Pro Ala Val Leu Gln Ser Asp Leu Tyr Thr
    190                 195                 200

ctc agc agc tca gtg act gta acc tcg agc acc tgg ccc agc cag tcc      675
Leu Ser Ser Ser Val Thr Val Thr Ser Ser Thr Trp Pro Ser Gln Ser
205                 210                 215                 220

atc acc tgc aat gtg gcc cac ccg gca agc agc acc aag gtg gac aag      723
Ile Thr Cys Asn Val Ala His Pro Ala Ser Ser Thr Lys Val Asp Lys
                225                 230                 235

aaa att gag ccc cgg gga ccc aca atc aag ccc tgt cct cca tgc aaa      771
Lys Ile Glu Pro Arg Gly Pro Thr Ile Lys Pro Cys Pro Pro Cys Lys
            240                 245                 250

tgc cca gca cct aac ctc ttg ggt gga cca tcc gtc ttc atc ttc cct      819
Cys Pro Ala Pro Asn Leu Leu Gly Gly Pro Ser Val Phe Ile Phe Pro
        255                 260                 265

cca aag atc aag gat gta ctc atg atc tcc ctg agc ccc ata gtc aca      867
Pro Lys Ile Lys Asp Val Leu Met Ile Ser Leu Ser Pro Ile Val Thr
    270                 275                 280

tgt gtg gtg gtg gat gtg agc gag gat gac cca gat gtc cag atc agc      915
Cys Val Val Val Asp Val Ser Glu Asp Asp Pro Asp Val Gln Ile Ser
285                 290                 295                 300

tgg ttt gtg aac aac gtg gaa gta cac aca gct cag aca caa acc cat      963
Trp Phe Val Asn Asn Val Glu Val His Thr Ala Gln Thr Gln Thr His
                305                 310                 315
```

aga gag gat tac aac agt act ctc cgg gtg gtc agt gcc ctc ccc atc 1011
Arg Glu Asp Tyr Asn Ser Thr Leu Arg Val Val Ser Ala Leu Pro Ile
320 325 330

cag cac cag gac tgg atg agt ggc aag gag ttc aaa tgc aag gtc aac 1059
Gln His Gln Asp Trp Met Ser Gly Lys Glu Phe Lys Cys Lys Val Asn
335 340 345

aac aaa gac ctc cca gcg ccc atc gag aga acc atc tca aaa ccc aaa 1107
Asn Lys Asp Leu Pro Ala Pro Ile Glu Arg Thr Ile Ser Lys Pro Lys
350 355 360

ggg tca gta aga gct cca cag gta tat gtc ttg cct cca cca gaa gaa 1155
Gly Ser Val Arg Ala Pro Gln Val Tyr Val Leu Pro Pro Pro Glu Glu
365 370 375 380

gag atg act aag aaa cag gtc act ctg acc tgc atg gtc aca gac ttc 1203
Glu Met Thr Lys Lys Gln Val Thr Leu Thr Cys Met Val Thr Asp Phe
385 390 395

atg cct gaa gac att tac gtg gag tgg acc aac aac ggg aaa aca gag 1251
Met Pro Glu Asp Ile Tyr Val Glu Trp Thr Asn Asn Gly Lys Thr Glu
400 405 410

cta aac tac aag aac act gaa cca gtc ctg gac tct gat ggt tct tac 1299
Leu Asn Tyr Lys Asn Thr Glu Pro Val Leu Asp Ser Asp Gly Ser Tyr
415 420 425

ttc atg tac agc aag ctg aga gtg gaa aag aag aac tgg gtg gaa aga 1347
Phe Met Tyr Ser Lys Leu Arg Val Glu Lys Lys Asn Trp Val Glu Arg
430 435 440

aat agc tac tcc tgt tca gtg gtc cac gag ggt ctg cac aat cac cac 1395
Asn Ser Tyr Ser Cys Ser Val Val His Glu Gly Leu His Asn His His
445 450 455 460

acg act aag agc ttc tcc cgg act ccg ggt aaa tga tagtctaga 1440
Thr Thr Lys Ser Phe Ser Arg Thr Pro Gly Lys
465 470

<210> 138
<211> 471
<212> PRT
<213> artificial sequence

<220>
<223> Synthetic Construct

<400> 138

Met Lys His Leu Trp Phe Phe Leu Leu Leu Val Ala Ala Pro Arg Trp
1 5 10 15

Val Leu Ser Glu Val Gln Leu Gln Gln Ser Gly Gly Glu Leu Val Arg
20 25 30

Pro Gly Thr Ser Val Lys Met Ser Cys Lys Ala Ala Gly Tyr Thr Phe
35 40 45

```
Thr Asn Tyr Trp Ile Gly Trp Val Lys Gln Arg Pro Gly His Gly Leu
    50                  55                  60

Glu Trp Ile Gly Asp Leu Tyr Pro Gly Gly Gly Tyr Thr His Tyr Asn
65                  70                  75                  80

Glu Lys Phe Lys Gly Lys Ala Thr Leu Thr Ala Asp Thr Ser Ser Ser
                85                  90                  95

Thr Ala Tyr Met Gln Leu Ser Ser Leu Thr Ser Glu Asp Ser Ala Ile
            100                 105                 110

Tyr Tyr Cys Ala Arg Glu Arg Val Tyr Phe Asp Gly Ser Arg Tyr Phe
        115                 120                 125

Asp Val Trp Gly Ala Gly Thr Thr Val Thr Val Ser Ser Ala Lys Thr
    130                 135                 140

Thr Ala Pro Ser Val Tyr Pro Leu Ala Pro Val Cys Gly Asp Thr Thr
145                 150                 155                 160

Gly Ser Ser Val Thr Leu Gly Cys Leu Val Lys Gly Tyr Phe Pro Glu
                165                 170                 175

Pro Val Thr Leu Thr Trp Asn Ser Gly Ser Leu Ser Ser Gly Val His
            180                 185                 190

Thr Phe Pro Ala Val Leu Gln Ser Asp Leu Tyr Thr Leu Ser Ser Ser
        195                 200                 205

Val Thr Val Thr Ser Ser Thr Trp Pro Ser Gln Ser Ile Thr Cys Asn
    210                 215                 220

Val Ala His Pro Ala Ser Ser Thr Lys Val Asp Lys Lys Ile Glu Pro
225                 230                 235                 240

Arg Gly Pro Thr Ile Lys Pro Cys Pro Pro Cys Lys Cys Pro Ala Pro
                245                 250                 255

Asn Leu Leu Gly Gly Pro Ser Val Phe Ile Phe Pro Pro Lys Ile Lys
            260                 265                 270

Asp Val Leu Met Ile Ser Leu Ser Pro Ile Val Thr Cys Val Val Val
        275                 280                 285

Asp Val Ser Glu Asp Asp Pro Asp Val Gln Ile Ser Trp Phe Val Asn
    290                 295                 300
```

```
Asn Val Glu Val His Thr Ala Gln Thr Gln Thr His Arg Glu Asp Tyr
305                 310                 315                 320

Asn Ser Thr Leu Arg Val Val Ser Ala Leu Pro Ile Gln His Gln Asp
                325                 330                 335

Trp Met Ser Gly Lys Glu Phe Lys Cys Lys Val Asn Asn Lys Asp Leu
            340                 345                 350

Pro Ala Pro Ile Glu Arg Thr Ile Ser Lys Pro Lys Gly Ser Val Arg
        355                 360                 365

Ala Pro Gln Val Tyr Val Leu Pro Pro Pro Glu Glu Glu Met Thr Lys
    370                 375                 380

Lys Gln Val Thr Leu Thr Cys Met Val Thr Asp Phe Met Pro Glu Asp
385                 390                 395                 400

Ile Tyr Val Glu Trp Thr Asn Asn Gly Lys Thr Glu Leu Asn Tyr Lys
                405                 410                 415

Asn Thr Glu Pro Val Leu Asp Ser Asp Gly Ser Tyr Phe Met Tyr Ser
            420                 425                 430

Lys Leu Arg Val Glu Lys Lys Asn Trp Val Glu Arg Asn Ser Tyr Ser
        435                 440                 445

Cys Ser Val Val His Glu Gly Leu His Asn His His Thr Thr Lys Ser
    450                 455                 460

Phe Ser Arg Thr Pro Gly Lys
465                 470
```

```
<210>  139
<211>  731
<212>  DNA
<213>  artificial sequence

<220>
<223>  Nucleic Acid Sequence of Light Chain of K090-01a017


<220>
<221>  CDS
<222>  (16)..(720)

<400>  139
aagcttgccg ccacc atg agg ctc ctt gct cag ctt ctg ggg ctg cta atg        51
                 Met Arg Leu Leu Ala Gln Leu Leu Gly Leu Leu Met
                 1               5                   10
```

```
ctc tgg gtc cct gga tcc agt ggg agt att gtg atg acc cag act ccc        99
Leu Trp Val Pro Gly Ser Ser Gly Ser Ile Val Met Thr Gln Thr Pro
        15                  20                  25

aaa ttc ctg cct gta tca gca gga gac agg gtt acc atg acc tgc aag       147
Lys Phe Leu Pro Val Ser Ala Gly Asp Arg Val Thr Met Thr Cys Lys
    30                  35                  40

gcc agt cag agt gtg ggt aat aat gta gcc tgg tac caa cag aag cca       195
Ala Ser Gln Ser Val Gly Asn Asn Val Ala Trp Tyr Gln Gln Lys Pro
45                  50                  55                  60

gga cag tct cct aaa ctg ctg ata tac tat gca tcc aat cgc tac act       243
Gly Gln Ser Pro Lys Leu Leu Ile Tyr Tyr Ala Ser Asn Arg Tyr Thr
                65                  70                  75

gga gtc cct gat cgc ttc act ggt agt gga tct ggg aca gat ttc act       291
Gly Val Pro Asp Arg Phe Thr Gly Ser Gly Ser Gly Thr Asp Phe Thr
            80                  85                  90

ttc acc atc agc agt gtg cag gtt gaa gac ctg gca gtt tat ttc tgt       339
Phe Thr Ile Ser Ser Val Gln Val Glu Asp Leu Ala Val Tyr Phe Cys
        95                  100                 105

cag cag cat tat agc tct ccg tgg acg ttc ggt gga ggc acc aag ctg       387
Gln Gln His Tyr Ser Ser Pro Trp Thr Phe Gly Gly Gly Thr Lys Leu
    110                 115                 120

gaa atc aaa cgg gct gat gct gca cca act gta tcc atc ttc cca cca       435
Glu Ile Lys Arg Ala Asp Ala Ala Pro Thr Val Ser Ile Phe Pro Pro
125                 130                 135                 140

tcc agt gag cag tta aca tct gga ggt gcc tca gtc gtg tgc ttc ttg       483
Ser Ser Glu Gln Leu Thr Ser Gly Gly Ala Ser Val Val Cys Phe Leu
                145                 150                 155

aac aac ttc tac ccc aaa gac atc aat gtc aag tgg aag att gat ggc       531
Asn Asn Phe Tyr Pro Lys Asp Ile Asn Val Lys Trp Lys Ile Asp Gly
            160                 165                 170

agt gaa cga caa aat ggc gtc ctg aac agt tgg act gat cag gac agc       579
Ser Glu Arg Gln Asn Gly Val Leu Asn Ser Trp Thr Asp Gln Asp Ser
        175                 180                 185

aaa gac agc acc tac agc atg agc agc acc ctc acg ttg acc aag gac       627
Lys Asp Ser Thr Tyr Ser Met Ser Ser Thr Leu Thr Leu Thr Lys Asp
    190                 195                 200

gag tat gaa cga cat aac agc tat acc tgt gag gcc act cac aag aca       675
Glu Tyr Glu Arg His Asn Ser Tyr Thr Cys Glu Ala Thr His Lys Thr
205                 210                 215                 220

tca act tca ccc att gtc aag agc ttc aac agg aat gag tgt tga           720
Ser Thr Ser Pro Ile Val Lys Ser Phe Asn Arg Asn Glu Cys
                225                 230

tagggcgcgc c                                                          731
```

<210>  140
<211>  234
<212>  PRT

<213> artificial sequence

<220>
<223> Synthetic Construct

<400> 140

Met Arg Leu Leu Ala Gln Leu Leu Gly Leu Leu Met Leu Trp Val Pro
1 5 10 15

Gly Ser Ser Gly Ser Ile Val Met Thr Gln Thr Pro Lys Phe Leu Pro
20 25 30

Val Ser Ala Gly Asp Arg Val Thr Met Thr Cys Lys Ala Ser Gln Ser
35 40 45

Val Gly Asn Asn Val Ala Trp Tyr Gln Gln Lys Pro Gly Gln Ser Pro
50 55 60

Lys Leu Leu Ile Tyr Tyr Ala Ser Asn Arg Tyr Thr Gly Val Pro Asp
65 70 75 80

Arg Phe Thr Gly Ser Gly Ser Gly Thr Asp Phe Thr Phe Thr Ile Ser
85 90 95

Ser Val Gln Val Glu Asp Leu Ala Val Tyr Phe Cys Gln Gln His Tyr
100 105 110

Ser Ser Pro Trp Thr Phe Gly Gly Gly Thr Lys Leu Glu Ile Lys Arg
115 120 125

Ala Asp Ala Ala Pro Thr Val Ser Ile Phe Pro Pro Ser Ser Glu Gln
130 135 140

Leu Thr Ser Gly Gly Ala Ser Val Val Cys Phe Leu Asn Asn Phe Tyr
145 150 155 160

Pro Lys Asp Ile Asn Val Lys Trp Lys Ile Asp Gly Ser Glu Arg Gln
165 170 175

Asn Gly Val Leu Asn Ser Trp Thr Asp Gln Asp Ser Lys Asp Ser Thr
180 185 190

Tyr Ser Met Ser Ser Thr Leu Thr Leu Thr Lys Asp Glu Tyr Glu Arg
195 200 205

His Asn Ser Tyr Thr Cys Glu Ala Thr His Lys Thr Ser Thr Ser Pro
210 215 220

```
Ile Val Lys Ser Phe Asn Arg Asn Glu Cys
225                 230


<210>  141
<211>  1431
<212>  DNA
<213>  artificial sequence

<220>
<223>  Nucleic Acid Sequence of Heavy Chain of K090-01a021


<220>
<221>  CDS
<222>  (16)..(1422)

<400>  141
aagcttgccg ccacc atg aaa cac ctg tgg ttc ttc ctc ctc ctg gtg gcg       51
                 Met Lys His Leu Trp Phe Phe Leu Leu Leu Val Ala
                 1               5                   10

gcc ccc aga tgg gtc ctg tcc cag gtg cag ctg aag cag tca gga cct        99
Ala Pro Arg Trp Val Leu Ser Gln Val Gln Leu Lys Gln Ser Gly Pro
        15                  20                  25

gag ctg gtg aag cct ggg gct tca gtg aag ata tcc tgc aag gct tct       147
Glu Leu Val Lys Pro Gly Ala Ser Val Lys Ile Ser Cys Lys Ala Ser
    30                  35                  40

ggt tac tca ttc act ggc tac tac atg cac tgg gtg aag caa agt cct       195
Gly Tyr Ser Phe Thr Gly Tyr Tyr Met His Trp Val Lys Gln Ser Pro
45                  50                  55                  60

gaa aag agc ctt gag tgg att gga gag att aat cct agc act ggt ggt       243
Glu Lys Ser Leu Glu Trp Ile Gly Glu Ile Asn Pro Ser Thr Gly Gly
                65                  70                  75

act acc tac aac cag aag ttc aag gcc aag gcc act ttg act gta gac       291
Thr Thr Tyr Asn Gln Lys Phe Lys Ala Lys Ala Thr Leu Thr Val Asp
            80                  85                  90

aaa tcc tcc agt aca gcc tac atg cag ctc aag agc ctg aca tct gag       339
Lys Ser Ser Ser Thr Ala Tyr Met Gln Leu Lys Ser Leu Thr Ser Glu
        95                  100                 105

gac tct gca gtc tat tac tgt gca aga gag aag aga gat gat ggc gta       387
Asp Ser Ala Val Tyr Tyr Cys Ala Arg Glu Lys Arg Asp Asp Gly Val
    110                 115                 120

ttt gct tac tgg ggc caa ggg act ctg gtc act gtc tcg agc gcc aaa       435
Phe Ala Tyr Trp Gly Gln Gly Thr Leu Val Thr Val Ser Ser Ala Lys
125                 130                 135                 140

aca aca gcc cca tcg gtc tat cca ctg gcc cct gtg tgt gga gat aca       483
Thr Thr Ala Pro Ser Val Tyr Pro Leu Ala Pro Val Cys Gly Asp Thr
                145                 150                 155

act ggc tcc tcg gtg act cta gga tgc ctg gtc aag ggt tat ttc cct       531
Thr Gly Ser Ser Val Thr Leu Gly Cys Leu Val Lys Gly Tyr Phe Pro
            160                 165                 170

gag cca gtg acc ttg acc tgg aac tct gga tcc ctg tcc agt ggt gtg       579
```

```
Glu Pro Val Thr Leu Thr Trp Asn Ser Gly Ser Leu Ser Ser Gly Val
        175                 180                 185

cac acc ttc cca gct gtc ctg cag tct gac ctc tac acc ctc agc agc        627
His Thr Phe Pro Ala Val Leu Gln Ser Asp Leu Tyr Thr Leu Ser Ser
    190                 195                 200

tca gtg act gta acc tcg agc acc tgg ccc agc cag tcc atc acc tgc        675
Ser Val Thr Val Thr Ser Ser Thr Trp Pro Ser Gln Ser Ile Thr Cys
205                 210                 215                 220

aat gtg gcc cac ccg gca agc agc acc aag gtg gac aag aaa att gag        723
Asn Val Ala His Pro Ala Ser Ser Thr Lys Val Asp Lys Lys Ile Glu
                225                 230                 235

ccc cgg gga ccc aca atc aag ccc tgt cct cca tgc aaa tgc cca gca        771
Pro Arg Gly Pro Thr Ile Lys Pro Cys Pro Pro Cys Lys Cys Pro Ala
            240                 245                 250

cct aac ctc ttg ggt gga cca tcc gtc ttc atc ttc cct cca aag atc        819
Pro Asn Leu Leu Gly Gly Pro Ser Val Phe Ile Phe Pro Pro Lys Ile
        255                 260                 265

aag gat gta ctc atg atc tcc ctg agc ccc ata gtc aca tgt gtg gtg        867
Lys Asp Val Leu Met Ile Ser Leu Ser Pro Ile Val Thr Cys Val Val
    270                 275                 280

gtg gat gtg agc gag gat gac cca gat gtc cag atc agc tgg ttt gtg        915
Val Asp Val Ser Glu Asp Asp Pro Asp Val Gln Ile Ser Trp Phe Val
285                 290                 295                 300

aac aac gtg gaa gta cac aca gct cag aca caa acc cat aga gag gat        963
Asn Asn Val Glu Val His Thr Ala Gln Thr Gln Thr His Arg Glu Asp
                305                 310                 315

tac aac agt act ctc cgg gtg gtc agt gcc ctc ccc atc cag cac cag       1011
Tyr Asn Ser Thr Leu Arg Val Val Ser Ala Leu Pro Ile Gln His Gln
            320                 325                 330

gac tgg atg agt ggc aag gag ttc aaa tgc aag gtc aac aac aaa gac       1059
Asp Trp Met Ser Gly Lys Glu Phe Lys Cys Lys Val Asn Asn Lys Asp
        335                 340                 345

ctc cca gcg ccc atc gag aga acc atc tca aaa ccc aaa ggg tca gta       1107
Leu Pro Ala Pro Ile Glu Arg Thr Ile Ser Lys Pro Lys Gly Ser Val
    350                 355                 360

aga gct cca cag gta tat gtc ttg cct cca cca gaa gaa gag atg act       1155
Arg Ala Pro Gln Val Tyr Val Leu Pro Pro Pro Glu Glu Glu Met Thr
365                 370                 375                 380

aag aaa cag gtc act ctg acc tgc atg gtc aca gac ttc atg cct gaa       1203
Lys Lys Gln Val Thr Leu Thr Cys Met Val Thr Asp Phe Met Pro Glu
                385                 390                 395

gac att tac gtg gag tgg acc aac aac ggg aaa aca gag cta aac tac       1251
Asp Ile Tyr Val Glu Trp Thr Asn Asn Gly Lys Thr Glu Leu Asn Tyr
            400                 405                 410

aag aac act gaa cca gtc ctg gac tct gat ggt tct tac ttc atg tac       1299
Lys Asn Thr Glu Pro Val Leu Asp Ser Asp Gly Ser Tyr Phe Met Tyr
        415                 420                 425
```

```
agc aag ctg aga gtg gaa aag aag aac tgg gtg gaa aga aat agc tac      1347
Ser Lys Leu Arg Val Glu Lys Lys Asn Trp Val Glu Arg Asn Ser Tyr
    430                 435                 440

tcc tgt tca gtg gtc cac gag ggt ctg cac aat cac cac acg act aag      1395
Ser Cys Ser Val Val His Glu Gly Leu His Asn His His Thr Thr Lys
445                 450                 455                 460

agc ttc tcc cgg act ccg ggt aaa tga tagtctaga                         1431
Ser Phe Ser Arg Thr Pro Gly Lys
                465


<210>  142
<211>  468
<212>  PRT
<213>  artificial sequence

<220>
<223>  Synthetic Construct

<400>  142

Met Lys His Leu Trp Phe Phe Leu Leu Leu Val Ala Ala Pro Arg Trp
1               5                   10                  15

Val Leu Ser Gln Val Gln Leu Lys Gln Ser Gly Pro Glu Leu Val Lys
            20                  25                  30

Pro Gly Ala Ser Val Lys Ile Ser Cys Lys Ala Ser Gly Tyr Ser Phe
        35                  40                  45

Thr Gly Tyr Tyr Met His Trp Val Lys Gln Ser Pro Glu Lys Ser Leu
    50                  55                  60

Glu Trp Ile Gly Glu Ile Asn Pro Ser Thr Gly Gly Thr Thr Tyr Asn
65                  70                  75                  80

Gln Lys Phe Lys Ala Lys Ala Thr Leu Thr Val Asp Lys Ser Ser Ser
                85                  90                  95

Thr Ala Tyr Met Gln Leu Lys Ser Leu Thr Ser Glu Asp Ser Ala Val
            100                 105                 110

Tyr Tyr Cys Ala Arg Glu Lys Arg Asp Asp Gly Val Phe Ala Tyr Trp
        115                 120                 125

Gly Gln Gly Thr Leu Val Thr Val Ser Ser Ala Lys Thr Thr Ala Pro
    130                 135                 140

Ser Val Tyr Pro Leu Ala Pro Val Cys Gly Asp Thr Thr Gly Ser Ser
145                 150                 155                 160
```

```
Val Thr Leu Gly Cys Leu Val Lys Gly Tyr Phe Pro Glu Pro Val Thr
                165                 170                 175

Leu Thr Trp Asn Ser Gly Ser Leu Ser Ser Gly Val His Thr Phe Pro
            180                 185                 190

Ala Val Leu Gln Ser Asp Leu Tyr Thr Leu Ser Ser Ser Val Thr Val
        195                 200                 205

Thr Ser Ser Thr Trp Pro Ser Gln Ser Ile Thr Cys Asn Val Ala His
    210                 215                 220

Pro Ala Ser Ser Thr Lys Val Asp Lys Lys Ile Glu Pro Arg Gly Pro
225                 230                 235                 240

Thr Ile Lys Pro Cys Pro Pro Cys Lys Cys Pro Ala Pro Asn Leu Leu
                245                 250                 255

Gly Gly Pro Ser Val Phe Ile Phe Pro Pro Lys Ile Lys Asp Val Leu
            260                 265                 270

Met Ile Ser Leu Ser Pro Ile Val Thr Cys Val Val Val Asp Val Ser
        275                 280                 285

Glu Asp Asp Pro Asp Val Gln Ile Ser Trp Phe Val Asn Asn Val Glu
    290                 295                 300

Val His Thr Ala Gln Thr Gln Thr His Arg Glu Asp Tyr Asn Ser Thr
305                 310                 315                 320

Leu Arg Val Val Ser Ala Leu Pro Ile Gln His Gln Asp Trp Met Ser
                325                 330                 335

Gly Lys Glu Phe Lys Cys Lys Val Asn Asn Lys Asp Leu Pro Ala Pro
            340                 345                 350

Ile Glu Arg Thr Ile Ser Lys Pro Lys Gly Ser Val Arg Ala Pro Gln
        355                 360                 365

Val Tyr Val Leu Pro Pro Pro Glu Glu Glu Met Thr Lys Lys Gln Val
    370                 375                 380

Thr Leu Thr Cys Met Val Thr Asp Phe Met Pro Glu Asp Ile Tyr Val
385                 390                 395                 400

Glu Trp Thr Asn Asn Gly Lys Thr Glu Leu Asn Tyr Lys Asn Thr Glu
                405                 410                 415
```

```
Pro Val Leu Asp Ser Asp Gly Ser Tyr Phe Met Tyr Ser Lys Leu Arg
            420                 425                 430

Val Glu Lys Lys Asn Trp Val Glu Arg Asn Ser Tyr Ser Cys Ser Val
        435                 440                 445

Val His Glu Gly Leu His Asn His His Thr Thr Lys Ser Phe Ser Arg
    450                 455                 460

Thr Pro Gly Lys
465


<210>  143
<211>  731
<212>  DNA
<213>  artificial sequence

<220>
<223>  Nucleic Acid Sequence of Light Chain of K090-01a021


<220>
<221>  CDS
<222>  (16)..(720)

<400>  143
aagcttgccg ccacc atg agg ctc ctt gct cag ctt ctg ggg ctg cta atg        51
                 Met Arg Leu Leu Ala Gln Leu Leu Gly Leu Leu Met
                 1               5                   10

ctc tgg gtc cct gga tcc agt ggg agt att gtg atg acc cag act ccc         99
Leu Trp Val Pro Gly Ser Ser Gly Ser Ile Val Met Thr Gln Thr Pro
        15                  20                  25

aaa ttc ctg cct gta tca gca gga gac agg gtt acc atg acc tgc aag        147
Lys Phe Leu Pro Val Ser Ala Gly Asp Arg Val Thr Met Thr Cys Lys
    30                  35                  40

gcc agt cag agt gtg ggt aat aat gta gcc tgg tac caa cag aag cca        195
Ala Ser Gln Ser Val Gly Asn Asn Val Ala Trp Tyr Gln Gln Lys Pro
45                  50                  55                  60

gga cag tct cct aaa ctg ctg ata tac tat gca tcc aat cgc tac act        243
Gly Gln Ser Pro Lys Leu Leu Ile Tyr Tyr Ala Ser Asn Arg Tyr Thr
                65                  70                  75

gga gtc cct gat cgc ttc act ggc agt gga tct ggg aca gat ttc cct        291
Gly Val Pro Asp Arg Phe Thr Gly Ser Gly Ser Gly Thr Asp Phe Pro
            80                  85                  90

ttc acc atc agc agt gtg cag gtt gaa gac ctg gca gtt tat ttc tgt        339
Phe Thr Ile Ser Ser Val Gln Val Glu Asp Leu Ala Val Tyr Phe Cys
        95                  100                 105

cag cag cat tat agc tct cct ctc acg ttc ggt gct ggg acc aag ctg        387
Gln Gln His Tyr Ser Ser Pro Leu Thr Phe Gly Ala Gly Thr Lys Leu
    110                 115                 120
```

```
gag ctg aaa cgg gct gat gct gca cca act gta tcc atc ttc cca cca          435
Glu Leu Lys Arg Ala Asp Ala Ala Pro Thr Val Ser Ile Phe Pro Pro
125                 130                 135                 140

tcc agt gag cag tta aca tct gga ggt gcc tca gtc gtg tgc ttc ttg          483
Ser Ser Glu Gln Leu Thr Ser Gly Gly Ala Ser Val Val Cys Phe Leu
                145                 150                 155

aac aac ttc tac ccc aaa gac atc aat gtc aag tgg aag att gat ggc          531
Asn Asn Phe Tyr Pro Lys Asp Ile Asn Val Lys Trp Lys Ile Asp Gly
            160                 165                 170

agt gaa cga caa aat ggc gtc ctg aac agt tgg act gat cag gac agc          579
Ser Glu Arg Gln Asn Gly Val Leu Asn Ser Trp Thr Asp Gln Asp Ser
        175                 180                 185

aaa gac agc acc tac agc atg agc agc acc ctc acg ttg acc aag gac          627
Lys Asp Ser Thr Tyr Ser Met Ser Ser Thr Leu Thr Leu Thr Lys Asp
    190                 195                 200

gag tat gaa cga cat aac agc tat acc tgt gag gcc act cac aag aca          675
Glu Tyr Glu Arg His Asn Ser Tyr Thr Cys Glu Ala Thr His Lys Thr
205                 210                 215                 220

tca act tca ccc att gtc aag agc ttc aac agg aat gag tgt tga              720
Ser Thr Ser Pro Ile Val Lys Ser Phe Asn Arg Asn Glu Cys
                225                 230

tagggcgcgc c                                                             731


<210>  144
<211>  234
<212>  PRT
<213>  artificial sequence

<220>
<223>  Synthetic Construct

<400>  144

Met Arg Leu Leu Ala Gln Leu Leu Gly Leu Leu Met Leu Trp Val Pro
1               5                   10                  15

Gly Ser Ser Gly Ser Ile Val Met Thr Gln Thr Pro Lys Phe Leu Pro
            20                  25                  30

Val Ser Ala Gly Asp Arg Val Thr Met Thr Cys Lys Ala Ser Gln Ser
        35                  40                  45

Val Gly Asn Asn Val Ala Trp Tyr Gln Gln Lys Pro Gly Gln Ser Pro
    50                  55                  60

Lys Leu Leu Ile Tyr Tyr Ala Ser Asn Arg Tyr Thr Gly Val Pro Asp
65                  70                  75                  80

Arg Phe Thr Gly Ser Gly Ser Gly Thr Asp Phe Pro Phe Thr Ile Ser
                85                  90                  95
```

Ser Val Gln Val Glu Asp Leu Ala Val Tyr Phe Cys Gln Gln His Tyr
100 105 110

Ser Ser Pro Leu Thr Phe Gly Ala Gly Thr Lys Leu Glu Leu Lys Arg
115 120 125

Ala Asp Ala Ala Pro Thr Val Ser Ile Phe Pro Pro Ser Ser Glu Gln
130 135 140

Leu Thr Ser Gly Gly Ala Ser Val Val Cys Phe Leu Asn Asn Phe Tyr
145 150 155 160

Pro Lys Asp Ile Asn Val Lys Trp Lys Ile Asp Gly Ser Glu Arg Gln
165 170 175

Asn Gly Val Leu Asn Ser Trp Thr Asp Gln Asp Ser Lys Asp Ser Thr
180 185 190

Tyr Ser Met Ser Ser Thr Leu Thr Leu Thr Lys Asp Glu Tyr Glu Arg
195 200 205

His Asn Ser Tyr Thr Cys Glu Ala Thr His Lys Thr Ser Thr Ser Pro
210 215 220

Ile Val Lys Ser Phe Asn Arg Asn Glu Cys
225 230

<210> 145
<211> 1440
<212> DNA
<213> artificial sequence

<220>
<223> Nucleic Acid Sequence of Heavy Chain of K090-01a026

<220>
<221> CDS
<222> (16)..(1431)

<400> 145
aagcttgccg ccacc atg aaa cac ctg tgg ttc ttc ctc ctc ctg gtg gcg 51
Met Lys His Leu Trp Phe Phe Leu Leu Leu Val Ala
1 5 10

gcc ccc aga tgg gtc ctg tcc cag gtc cac ctg cag cag tct gga gct 99
Ala Pro Arg Trp Val Leu Ser Gln Val His Leu Gln Gln Ser Gly Ala
15 20 25

gag ctg gta agg cct ggg act tca gtg aag atg tcc tgc aag gct gct 147
Glu Leu Val Arg Pro Gly Thr Ser Val Lys Met Ser Cys Lys Ala Ala
30 35 40

```
gga tac acc ttc acc aac tac tgg ata ggt tgg gta aaa cag agg cct           195
Gly Tyr Thr Phe Thr Asn Tyr Trp Ile Gly Trp Val Lys Gln Arg Pro
45                  50                  55                  60

gga cat ggc ctt gag tgg att gga gat att tac cct gga ggt ggt tat           243
Gly His Gly Leu Glu Trp Ile Gly Asp Ile Tyr Pro Gly Gly Gly Tyr
                65                  70                  75

act cac tac aat gag aag ttc aag ggc aag gcc aca ctg act gca gac           291
Thr His Tyr Asn Glu Lys Phe Lys Gly Lys Ala Thr Leu Thr Ala Asp
            80                  85                  90

aca tcc tcc agc aca gcc tac atg cag ctc agc agc ctg aca tct gag           339
Thr Ser Ser Ser Thr Ala Tyr Met Gln Leu Ser Ser Leu Thr Ser Glu
        95                  100                 105

gac tct gcc atc tat tac tgt gca aga gag agg gtt tac tac gat ggt           387
Asp Ser Ala Ile Tyr Tyr Cys Ala Arg Glu Arg Val Tyr Tyr Asp Gly
    110                 115                 120

agc cgg ttc ttc gat gtc tgg ggc gca ggg acc acg gtc acc gtc tcg           435
Ser Arg Phe Phe Asp Val Trp Gly Ala Gly Thr Thr Val Thr Val Ser
125                 130                 135                 140

agc gcc aaa aca aca gcc cca tcg gtc tat cca ctg gcc cct gtg tgt           483
Ser Ala Lys Thr Thr Ala Pro Ser Val Tyr Pro Leu Ala Pro Val Cys
                145                 150                 155

gga gat aca act ggc tcc tcg gtg act cta gga tgc ctg gtc aag ggt           531
Gly Asp Thr Thr Gly Ser Ser Val Thr Leu Gly Cys Leu Val Lys Gly
            160                 165                 170

tat ttc cct gag cca gtg acc ttg acc tgg aac tct gga tcc ctg tcc           579
Tyr Phe Pro Glu Pro Val Thr Leu Thr Trp Asn Ser Gly Ser Leu Ser
        175                 180                 185

agt ggt gtg cac acc ttc cca gct gtc ctg cag tct gac ctc tac acc           627
Ser Gly Val His Thr Phe Pro Ala Val Leu Gln Ser Asp Leu Tyr Thr
    190                 195                 200

ctc agc agc tca gtg act gta acc tcg agc acc tgg ccc agc cag tcc           675
Leu Ser Ser Ser Val Thr Val Thr Ser Ser Thr Trp Pro Ser Gln Ser
205                 210                 215                 220

atc acc tgc aat gtg gcc cac ccg gca agc agc acc aag gtg gac aag           723
Ile Thr Cys Asn Val Ala His Pro Ala Ser Ser Thr Lys Val Asp Lys
                225                 230                 235

aaa att gag ccc cgg gga ccc aca atc aag ccc tgt cct cca tgc aaa           771
Lys Ile Glu Pro Arg Gly Pro Thr Ile Lys Pro Cys Pro Pro Cys Lys
            240                 245                 250

tgc cca gca cct aac ctc ttg ggt gga cca tcc gtc ttc atc ttc cct           819
Cys Pro Ala Pro Asn Leu Leu Gly Gly Pro Ser Val Phe Ile Phe Pro
        255                 260                 265

cca aag atc aag gat gta ctc atg atc tcc ctg agc ccc ata gtc aca           867
Pro Lys Ile Lys Asp Val Leu Met Ile Ser Leu Ser Pro Ile Val Thr
    270                 275                 280

tgt gtg gtg gtg gat gtg agc gag gat gac cca gat gtc cag atc agc           915
Cys Val Val Val Asp Val Ser Glu Asp Asp Pro Asp Val Gln Ile Ser
```

```
285                 290                 295                 300

tgg ttt gtg aac aac gtg gaa gta cac aca gct cag aca caa acc cat      963
Trp Phe Val Asn Asn Val Glu Val His Thr Ala Gln Thr Gln Thr His
                305                 310                 315

aga gag gat tac aac agt act ctc cgg gtg gtc agt gcc ctc ccc atc     1011
Arg Glu Asp Tyr Asn Ser Thr Leu Arg Val Val Ser Ala Leu Pro Ile
            320                 325                 330

cag cac cag gac tgg atg agt ggc aag gag ttc aaa tgc aag gtc aac     1059
Gln His Gln Asp Trp Met Ser Gly Lys Glu Phe Lys Cys Lys Val Asn
        335                 340                 345

aac aaa gac ctc cca gcg ccc atc gag aga acc atc tca aaa ccc aaa     1107
Asn Lys Asp Leu Pro Ala Pro Ile Glu Arg Thr Ile Ser Lys Pro Lys
    350                 355                 360

ggg tca gta aga gct cca cag gta tat gtc ttg cct cca cca gaa gaa     1155
Gly Ser Val Arg Ala Pro Gln Val Tyr Val Leu Pro Pro Pro Glu Glu
365                 370                 375                 380

gag atg act aag aaa cag gtc act ctg acc tgc atg gtc aca gac ttc     1203
Glu Met Thr Lys Lys Gln Val Thr Leu Thr Cys Met Val Thr Asp Phe
                385                 390                 395

atg cct gaa gac att tac gtg gag tgg acc aac aac ggg aaa aca gag     1251
Met Pro Glu Asp Ile Tyr Val Glu Trp Thr Asn Asn Gly Lys Thr Glu
            400                 405                 410

cta aac tac aag aac act gaa cca gtc ctg gac tct gat ggt tct tac     1299
Leu Asn Tyr Lys Asn Thr Glu Pro Val Leu Asp Ser Asp Gly Ser Tyr
        415                 420                 425

ttc atg tac agc aag ctg aga gtg gaa aag aag aac tgg gtg gaa aga     1347
Phe Met Tyr Ser Lys Leu Arg Val Glu Lys Lys Asn Trp Val Glu Arg
    430                 435                 440

aat agc tac tcc tgt tca gtg gtc cac gag ggt ctg cac aat cac cac     1395
Asn Ser Tyr Ser Cys Ser Val Val His Glu Gly Leu His Asn His His
445                 450                 455                 460

acg act aag agc ttc tcc cgg act ccg ggt aaa tga tagtctaga           1440
Thr Thr Lys Ser Phe Ser Arg Thr Pro Gly Lys
                465                 470


<210>  146
<211>  471
<212>  PRT
<213>  artificial sequence

<220>
<223>  Synthetic Construct

<400>  146

Met Lys His Leu Trp Phe Phe Leu Leu Leu Val Ala Ala Pro Arg Trp
1               5                   10                  15


Val Leu Ser Gln Val His Leu Gln Gln Ser Gly Ala Glu Leu Val Arg
            20                  25                  30
```

```
Pro Gly Thr Ser Val Lys Met Ser Cys Lys Ala Ala Gly Tyr Thr Phe
        35                  40                  45

Thr Asn Tyr Trp Ile Gly Trp Val Lys Gln Arg Pro Gly His Gly Leu
    50                  55                  60

Glu Trp Ile Gly Asp Ile Tyr Pro Gly Gly Gly Tyr Thr His Tyr Asn
65                  70                  75                  80

Glu Lys Phe Lys Gly Lys Ala Thr Leu Thr Ala Asp Thr Ser Ser Ser
                85                  90                  95

Thr Ala Tyr Met Gln Leu Ser Ser Leu Thr Ser Glu Asp Ser Ala Ile
            100                 105                 110

Tyr Tyr Cys Ala Arg Glu Arg Val Tyr Tyr Asp Gly Ser Arg Phe Phe
        115                 120                 125

Asp Val Trp Gly Ala Gly Thr Thr Val Thr Val Ser Ser Ala Lys Thr
    130                 135                 140

Thr Ala Pro Ser Val Tyr Pro Leu Ala Pro Val Cys Gly Asp Thr Thr
145                 150                 155                 160

Gly Ser Ser Val Thr Leu Gly Cys Leu Val Lys Gly Tyr Phe Pro Glu
                165                 170                 175

Pro Val Thr Leu Thr Trp Asn Ser Gly Ser Leu Ser Ser Gly Val His
            180                 185                 190

Thr Phe Pro Ala Val Leu Gln Ser Asp Leu Tyr Thr Leu Ser Ser Ser
        195                 200                 205

Val Thr Val Thr Ser Ser Thr Trp Pro Ser Gln Ser Ile Thr Cys Asn
    210                 215                 220

Val Ala His Pro Ala Ser Ser Thr Lys Val Asp Lys Lys Ile Glu Pro
225                 230                 235                 240

Arg Gly Pro Thr Ile Lys Pro Cys Pro Pro Cys Lys Cys Pro Ala Pro
                245                 250                 255

Asn Leu Leu Gly Gly Pro Ser Val Phe Ile Phe Pro Pro Lys Ile Lys
            260                 265                 270

Asp Val Leu Met Ile Ser Leu Ser Pro Ile Val Thr Cys Val Val Val
```

```
        275                 280                 285

Asp Val Ser Glu Asp Asp Pro Asp Val Gln Ile Ser Trp Phe Val Asn
    290                 295                 300

Asn Val Glu Val His Thr Ala Gln Thr Gln Thr His Arg Glu Asp Tyr
305                 310                 315                 320

Asn Ser Thr Leu Arg Val Val Ser Ala Leu Pro Ile Gln His Gln Asp
                325                 330                 335

Trp Met Ser Gly Lys Glu Phe Lys Cys Lys Val Asn Asn Lys Asp Leu
            340                 345                 350

Pro Ala Pro Ile Glu Arg Thr Ile Ser Lys Pro Lys Gly Ser Val Arg
        355                 360                 365

Ala Pro Gln Val Tyr Val Leu Pro Pro Pro Glu Glu Glu Met Thr Lys
    370                 375                 380

Lys Gln Val Thr Leu Thr Cys Met Val Thr Asp Phe Met Pro Glu Asp
385                 390                 395                 400

Ile Tyr Val Glu Trp Thr Asn Asn Gly Lys Thr Glu Leu Asn Tyr Lys
                405                 410                 415

Asn Thr Glu Pro Val Leu Asp Ser Asp Gly Ser Tyr Phe Met Tyr Ser
            420                 425                 430

Lys Leu Arg Val Glu Lys Lys Asn Trp Val Glu Arg Asn Ser Tyr Ser
        435                 440                 445

Cys Ser Val Val His Glu Gly Leu His Asn His His Thr Thr Lys Ser
    450                 455                 460

Phe Ser Arg Thr Pro Gly Lys
465                 470
```

<210>  147
<211>  731
<212>  DNA
<213>  artificial sequence

<220>
<223>  Nucleic Acid Sequence of Light Chain of K090-01a026

<220>
<221>  CDS
<222>  (16)..(720)

<400> 147

```
aagcttgccg ccacc atg agg ctc ctt gct cag ctt ctg ggg ctg cta atg        51
                 Met Arg Leu Leu Ala Gln Leu Leu Gly Leu Leu Met
                 1               5                   10

ctc tgg gtc cct gga tcc agt ggg gac att gtg atg tca cag tct ccc         99
Leu Trp Val Pro Gly Ser Ser Gly Asp Ile Val Met Ser Gln Ser Pro
        15                  20                  25

aaa ttc ctg cct gta tca gca gga gac agg gtt acc atg acc tgc aag        147
Lys Phe Leu Pro Val Ser Ala Gly Asp Arg Val Thr Met Thr Cys Lys
    30                  35                  40

gcc agt cag agt gtg ggt aat aat gta gcc tgg tac caa cag aag cca        195
Ala Ser Gln Ser Val Gly Asn Asn Val Ala Trp Tyr Gln Gln Lys Pro
45                  50                  55                  60

gga cag tct cct aaa ctg ctg ata tac tat gca tcc aat cgc tac act        243
Gly Gln Ser Pro Lys Leu Leu Ile Tyr Tyr Ala Ser Asn Arg Tyr Thr
                65                  70                  75

gga gtc cct gac cgc ttc act ggc agt gga tct ggg aca gat ttc act        291
Gly Val Pro Asp Arg Phe Thr Gly Ser Gly Ser Gly Thr Asp Phe Thr
            80                  85                  90

ttc acc atc agc agt gtg cag gtt gaa gac ctg gca gtt tat ttc tgt        339
Phe Thr Ile Ser Ser Val Gln Val Glu Asp Leu Ala Val Tyr Phe Cys
        95                  100                 105

cag cag cat tat agc tct ccg tgg acg ttc ggt gga ggc acc aag ctg        387
Gln Gln His Tyr Ser Ser Pro Trp Thr Phe Gly Gly Gly Thr Lys Leu
    110                 115                 120

gaa ata aaa cgg gct gat gct gca cca act gta tcc atc ttc cca cca        435
Glu Ile Lys Arg Ala Asp Ala Ala Pro Thr Val Ser Ile Phe Pro Pro
125                 130                 135                 140

tcc agt gag cag tta aca tct gga ggt gcc tca gtc gtg tgc ttc ttg        483
Ser Ser Glu Gln Leu Thr Ser Gly Gly Ala Ser Val Val Cys Phe Leu
                145                 150                 155

aac aac ttc tac ccc aaa gac atc aat gtc aag tgg aag att gat ggc        531
Asn Asn Phe Tyr Pro Lys Asp Ile Asn Val Lys Trp Lys Ile Asp Gly
            160                 165                 170

agt gaa cga caa aat ggc gtc ctg aac agt tgg act gat cag gac agc        579
Ser Glu Arg Gln Asn Gly Val Leu Asn Ser Trp Thr Asp Gln Asp Ser
        175                 180                 185

aaa gac agc acc tac agc atg agc agc acc ctc acg ttg acc aag gac        627
Lys Asp Ser Thr Tyr Ser Met Ser Ser Thr Leu Thr Leu Thr Lys Asp
    190                 195                 200

gag tat gaa cga cat aac agc tat acc tgt gag gcc act cac aag aca        675
Glu Tyr Glu Arg His Asn Ser Tyr Thr Cys Glu Ala Thr His Lys Thr
205                 210                 215                 220

tca act tca ccc att gtc aag agc ttc aac agg aat gag tgt tga            720
Ser Thr Ser Pro Ile Val Lys Ser Phe Asn Arg Asn Glu Cys
                225                 230

tagggcgcgc c                                                           731
```

<210> 148
<211> 234
<212> PRT
<213> artificial sequence

<220>
<223> Synthetic Construct

<400> 148

```
Met Arg Leu Leu Ala Gln Leu Leu Gly Leu Leu Met Leu Trp Val Pro
1               5                   10                  15

Gly Ser Ser Gly Asp Ile Val Met Ser Gln Ser Pro Lys Phe Leu Pro
            20                  25                  30

Val Ser Ala Gly Asp Arg Val Thr Met Thr Cys Lys Ala Ser Gln Ser
        35                  40                  45

Val Gly Asn Asn Val Ala Trp Tyr Gln Gln Lys Pro Gly Gln Ser Pro
    50                  55                  60

Lys Leu Leu Ile Tyr Tyr Ala Ser Asn Arg Tyr Thr Gly Val Pro Asp
65                  70                  75                  80

Arg Phe Thr Gly Ser Gly Ser Gly Thr Asp Phe Thr Phe Thr Ile Ser
                85                  90                  95

Ser Val Gln Val Glu Asp Leu Ala Val Tyr Phe Cys Gln Gln His Tyr
            100                 105                 110

Ser Ser Pro Trp Thr Phe Gly Gly Gly Thr Lys Leu Glu Ile Lys Arg
        115                 120                 125

Ala Asp Ala Ala Pro Thr Val Ser Ile Phe Pro Pro Ser Ser Glu Gln
    130                 135                 140

Leu Thr Ser Gly Gly Ala Ser Val Val Cys Phe Leu Asn Asn Phe Tyr
145                 150                 155                 160

Pro Lys Asp Ile Asn Val Lys Trp Lys Ile Asp Gly Ser Glu Arg Gln
                165                 170                 175

Asn Gly Val Leu Asn Ser Trp Thr Asp Gln Asp Ser Lys Asp Ser Thr
            180                 185                 190

Tyr Ser Met Ser Ser Thr Leu Thr Leu Thr Lys Asp Glu Tyr Glu Arg
        195                 200                 205
```

```
His Asn Ser Tyr Thr Cys Glu Ala Thr His Lys Thr Ser Thr Ser Pro
    210                 215                 220

Ile Val Lys Ser Phe Asn Arg Asn Glu Cys
225                 230


<210>  149
<211>  1425
<212>  DNA
<213>  artificial sequence

<220>
<223>  Nucleic Acid Sequence of Heavy Chain of K090-01a009


<220>
<221>  CDS
<222>  (16)..(1416)

<400>  149
aagcttgccg ccacc atg aaa cac ctg tgg ttc ttc ctc ctc ctg gtg gcg        51
                 Met Lys His Leu Trp Phe Phe Leu Leu Leu Val Ala
                 1               5                   10

gcc ccc aga tgg gtc ctg tcc gag gtt cag ctg cag cag tct gga cct         99
Ala Pro Arg Trp Val Leu Ser Glu Val Gln Leu Gln Gln Ser Gly Pro
        15                  20                  25

gag ctg gtg aag cct ggg gct tca gtg gag ata tcc tgc aag gct tct        147
Glu Leu Val Lys Pro Gly Ala Ser Val Glu Ile Ser Cys Lys Ala Ser
    30                  35                  40

ggt tac tca ttt act ggc tac ttt atg aac tgg gtg aag cag agc caa        195
Gly Tyr Ser Phe Thr Gly Tyr Phe Met Asn Trp Val Lys Gln Ser Gln
45                  50                  55                  60

gga aag agc ctt gag tgg att gga cgt att aat cct gac aat ggt gat        243
Gly Lys Ser Leu Glu Trp Ile Gly Arg Ile Asn Pro Asp Asn Gly Asp
                65                  70                  75

att ttc tac aac cag aag ttc aag ggc aag gcc aca tta act gta gac        291
Ile Phe Tyr Asn Gln Lys Phe Lys Gly Lys Ala Thr Leu Thr Val Asp
            80                  85                  90

aaa tcc tct agc aca gcc cac atg gag ctc cgg agc ctg aca tct gag        339
Lys Ser Ser Ser Thr Ala His Met Glu Leu Arg Ser Leu Thr Ser Glu
        95                  100                 105

gac tct gca gtc tat tat tgt gca aga tcg tgg aac tgg tac ttc gat        387
Asp Ser Ala Val Tyr Tyr Cys Ala Arg Ser Trp Asn Trp Tyr Phe Asp
    110                 115                 120

gtc tgg ggc gca ggg acc acg gtc acc gtc tcg agc gcc aaa aca aca        435
Val Trp Gly Ala Gly Thr Thr Val Thr Val Ser Ser Ala Lys Thr Thr
125                 130                 135                 140

gcc cca tcg gtc tat cca ctg gcc cct gtg tgt gga gat aca act ggc        483
Ala Pro Ser Val Tyr Pro Leu Ala Pro Val Cys Gly Asp Thr Thr Gly
                145                 150                 155
```

```
tcc tcg gtg act cta gga tgc ctg gtc aag ggt tat ttc cct gag cca        531
Ser Ser Val Thr Leu Gly Cys Leu Val Lys Gly Tyr Phe Pro Glu Pro
            160                 165                 170

gtg acc ttg acc tgg aac tct gga tcc ctg tcc agt ggt gtg cac acc        579
Val Thr Leu Thr Trp Asn Ser Gly Ser Leu Ser Ser Gly Val His Thr
        175                 180                 185

ttc cca gct gtc ctg cag tct gac ctc tac acc ctc agc agc tca gtg        627
Phe Pro Ala Val Leu Gln Ser Asp Leu Tyr Thr Leu Ser Ser Ser Val
    190                 195                 200

act gta acc tcg agc acc tgg ccc agc cag tcc atc acc tgc aat gtg        675
Thr Val Thr Ser Ser Thr Trp Pro Ser Gln Ser Ile Thr Cys Asn Val
205                 210                 215                 220

gcc cac ccg gca agc agc acc aag gtg gac aag aaa att gag ccc cgg        723
Ala His Pro Ala Ser Ser Thr Lys Val Asp Lys Lys Ile Glu Pro Arg
                225                 230                 235

gga ccc aca atc aag ccc tgt cct cca tgc aaa tgc cca gca cct aac        771
Gly Pro Thr Ile Lys Pro Cys Pro Pro Cys Lys Cys Pro Ala Pro Asn
            240                 245                 250

ctc ttg ggt gga cca tcc gtc ttc atc ttc cct cca aag atc aag gat        819
Leu Leu Gly Gly Pro Ser Val Phe Ile Phe Pro Pro Lys Ile Lys Asp
        255                 260                 265

gta ctc atg atc tcc ctg agc ccc ata gtc aca tgt gtg gtg gtg gat        867
Val Leu Met Ile Ser Leu Ser Pro Ile Val Thr Cys Val Val Val Asp
    270                 275                 280

gtg agc gag gat gac cca gat gtc cag atc agc tgg ttt gtg aac aac        915
Val Ser Glu Asp Asp Pro Asp Val Gln Ile Ser Trp Phe Val Asn Asn
285                 290                 295                 300

gtg gaa gta cac aca gct cag aca caa acc cat aga gag gat tac aac        963
Val Glu Val His Thr Ala Gln Thr Gln Thr His Arg Glu Asp Tyr Asn
                305                 310                 315

agt act ctc cgg gtg gtc agt gcc ctc ccc atc cag cac cag gac tgg       1011
Ser Thr Leu Arg Val Val Ser Ala Leu Pro Ile Gln His Gln Asp Trp
            320                 325                 330

atg agt ggc aag gag ttc aaa tgc aag gtc aac aac aaa gac ctc cca       1059
Met Ser Gly Lys Glu Phe Lys Cys Lys Val Asn Asn Lys Asp Leu Pro
        335                 340                 345

gcg ccc atc gag aga acc atc tca aaa ccc aaa ggg tca gta aga gct       1107
Ala Pro Ile Glu Arg Thr Ile Ser Lys Pro Lys Gly Ser Val Arg Ala
    350                 355                 360

cca cag gta tat gtc ttg cct cca cca gaa gaa gag atg act aag aaa       1155
Pro Gln Val Tyr Val Leu Pro Pro Pro Glu Glu Glu Met Thr Lys Lys
365                 370                 375                 380

cag gtc act ctg acc tgc atg gtc aca gac ttc atg cct gaa gac att       1203
Gln Val Thr Leu Thr Cys Met Val Thr Asp Phe Met Pro Glu Asp Ile
                385                 390                 395

tac gtg gag tgg acc aac aac ggg aaa aca gag cta aac tac aag aac       1251
Tyr Val Glu Trp Thr Asn Asn Gly Lys Thr Glu Leu Asn Tyr Lys Asn
            400                 405                 410
```

```
act gaa cca gtc ctg gac tct gat ggt tct tac ttc atg tac agc aag       1299
Thr Glu Pro Val Leu Asp Ser Asp Gly Ser Tyr Phe Met Tyr Ser Lys
          415                 420                 425

ctg aga gtg gaa aag aag aac tgg gtg gaa aga aat agc tac tcc tgt       1347
Leu Arg Val Glu Lys Lys Asn Trp Val Glu Arg Asn Ser Tyr Ser Cys
      430                 435                 440

tca gtg gtc cac gag ggt ctg cac aat cac cac acg act aag agc ttc       1395
Ser Val Val His Glu Gly Leu His Asn His His Thr Thr Lys Ser Phe
445                 450                 455                 460

tcc cgg act ccg ggt aaa tga tagtctaga                                 1425
Ser Arg Thr Pro Gly Lys
                465


<210>  150
<211>  466
<212>  PRT
<213>  artificial sequence

<220>
<223>  Synthetic Construct

<400>  150

Met Lys His Leu Trp Phe Phe Leu Leu Leu Val Ala Ala Pro Arg Trp
1               5                   10                  15


Val Leu Ser Glu Val Gln Leu Gln Gln Ser Gly Pro Glu Leu Val Lys
            20                  25                  30


Pro Gly Ala Ser Val Glu Ile Ser Cys Lys Ala Ser Gly Tyr Ser Phe
        35                  40                  45


Thr Gly Tyr Phe Met Asn Trp Val Lys Gln Ser Gln Gly Lys Ser Leu
    50                  55                  60


Glu Trp Ile Gly Arg Ile Asn Pro Asp Asn Gly Asp Ile Phe Tyr Asn
65                  70                  75                  80


Gln Lys Phe Lys Gly Lys Ala Thr Leu Thr Val Asp Lys Ser Ser Ser
                85                  90                  95


Thr Ala His Met Glu Leu Arg Ser Leu Thr Ser Glu Asp Ser Ala Val
            100                 105                 110


Tyr Tyr Cys Ala Arg Ser Trp Asn Trp Tyr Phe Asp Val Trp Gly Ala
        115                 120                 125


Gly Thr Thr Val Thr Val Ser Ser Ala Lys Thr Thr Ala Pro Ser Val
    130                 135                 140
```

Tyr Pro Leu Ala Pro Val Cys Gly Asp Thr Thr Gly Ser Ser Val Thr
145 150 155 160

Leu Gly Cys Leu Val Lys Gly Tyr Phe Pro Glu Pro Val Thr Leu Thr
165 170 175

Trp Asn Ser Gly Ser Leu Ser Ser Gly Val His Thr Phe Pro Ala Val
180 185 190

Leu Gln Ser Asp Leu Tyr Thr Leu Ser Ser Ser Val Thr Val Thr Ser
195 200 205

Ser Thr Trp Pro Ser Gln Ser Ile Thr Cys Asn Val Ala His Pro Ala
210 215 220

Ser Ser Thr Lys Val Asp Lys Lys Ile Glu Pro Arg Gly Pro Thr Ile
225 230 235 240

Lys Pro Cys Pro Pro Cys Lys Cys Pro Ala Pro Asn Leu Leu Gly Gly
245 250 255

Pro Ser Val Phe Ile Phe Pro Pro Lys Ile Lys Asp Val Leu Met Ile
260 265 270

Ser Leu Ser Pro Ile Val Thr Cys Val Val Val Asp Val Ser Glu Asp
275 280 285

Asp Pro Asp Val Gln Ile Ser Trp Phe Val Asn Asn Val Glu Val His
290 295 300

Thr Ala Gln Thr Gln Thr His Arg Glu Asp Tyr Asn Ser Thr Leu Arg
305 310 315 320

Val Val Ser Ala Leu Pro Ile Gln His Gln Asp Trp Met Ser Gly Lys
325 330 335

Glu Phe Lys Cys Lys Val Asn Asn Lys Asp Leu Pro Ala Pro Ile Glu
340 345 350

Arg Thr Ile Ser Lys Pro Lys Gly Ser Val Arg Ala Pro Gln Val Tyr
355 360 365

Val Leu Pro Pro Pro Glu Glu Glu Met Thr Lys Lys Gln Val Thr Leu
370 375 380

Thr Cys Met Val Thr Asp Phe Met Pro Glu Asp Ile Tyr Val Glu Trp
385 390 395 400

```
Thr Asn Asn Gly Lys Thr Glu Leu Asn Tyr Lys Asn Thr Glu Pro Val
                405                 410                 415

Leu Asp Ser Asp Gly Ser Tyr Phe Met Tyr Ser Lys Leu Arg Val Glu
            420                 425                 430

Lys Lys Asn Trp Val Glu Arg Asn Ser Tyr Ser Cys Ser Val Val His
        435                 440                 445

Glu Gly Leu His Asn His His Thr Thr Lys Ser Phe Ser Arg Thr Pro
    450                 455                 460

Gly Lys
465


<210>  151
<211>  731
<212>  DNA
<213>  artificial sequence

<220>
<223>  Nucleic Acid Sequence of Light Chain of K090-01a009


<220>
<221>  CDS
<222>  (16)..(720)

<400>  151
aagcttgccg ccacc atg agg ctc ctt gct cag ctt ctg ggg ctg cta atg        51
                 Met Arg Leu Leu Ala Gln Leu Leu Gly Leu Leu Met
                 1               5                   10

ctc tgg gtc cct gga tcc agt ggg gac atc cag atg aca cag tct cca         99
Leu Trp Val Pro Gly Ser Ser Gly Asp Ile Gln Met Thr Gln Ser Pro
        15                  20                  25

gcc tcc cta tct gca tct gtg gga gaa act gtc acc atc aca tgt cga        147
Ala Ser Leu Ser Ala Ser Val Gly Glu Thr Val Thr Ile Thr Cys Arg
    30                  35                  40

gca agt gag aat att tac agt tat tta gca tgg tat cag cag aaa cag        195
Ala Ser Glu Asn Ile Tyr Ser Tyr Leu Ala Trp Tyr Gln Gln Lys Gln
45                  50                  55                  60

gga aaa tct cct cag ctc ctg gtc tat aat gca aaa acc tta gca gaa        243
Gly Lys Ser Pro Gln Leu Leu Val Tyr Asn Ala Lys Thr Leu Ala Glu
                65                  70                  75

ggt gtg cca tca agg ttc agt ggc agt gga tca ggc aca cag ttt tct        291
Gly Val Pro Ser Arg Phe Ser Gly Ser Gly Ser Gly Thr Gln Phe Ser
            80                  85                  90

ctg aag atc aac agc ctg cag cct gaa gat ttt ggg agt tat tac tgt        339
Leu Lys Ile Asn Ser Leu Gln Pro Glu Asp Phe Gly Ser Tyr Tyr Cys
        95                  100                 105
```

```
caa cat cat tat ggt act ccg tat acg ttc gga tcg ggg acc aag ctg        387
Gln His His Tyr Gly Thr Pro Tyr Thr Phe Gly Ser Gly Thr Lys Leu
    110                 115                 120

gaa ata aaa cgg gct gat gct gca cca act gta tcc atc ttc cca cca        435
Glu Ile Lys Arg Ala Asp Ala Ala Pro Thr Val Ser Ile Phe Pro Pro
125                 130                 135                 140

tcc agt gag cag tta aca tct gga ggt gcc tca gtc gtg tgc ttc ttg        483
Ser Ser Glu Gln Leu Thr Ser Gly Gly Ala Ser Val Val Cys Phe Leu
                145                 150                 155

aac aac ttc tac ccc aaa gac atc aat gtc aag tgg aag att gat ggc        531
Asn Asn Phe Tyr Pro Lys Asp Ile Asn Val Lys Trp Lys Ile Asp Gly
            160                 165                 170

agt gaa cga caa aat ggc gtc ctg aac agt tgg act gat cag gac agc        579
Ser Glu Arg Gln Asn Gly Val Leu Asn Ser Trp Thr Asp Gln Asp Ser
        175                 180                 185

aaa gac agc acc tac agc atg agc agc acc ctc acg ttg acc aag gac        627
Lys Asp Ser Thr Tyr Ser Met Ser Ser Thr Leu Thr Leu Thr Lys Asp
    190                 195                 200

gag tat gaa cga cat aac agc tat acc tgt gag gcc act cac aag aca        675
Glu Tyr Glu Arg His Asn Ser Tyr Thr Cys Glu Ala Thr His Lys Thr
205                 210                 215                 220

tca act tca ccc att gtc aag agc ttc aac agg aat gag tgt tga            720
Ser Thr Ser Pro Ile Val Lys Ser Phe Asn Arg Asn Glu Cys
                225                 230

tagggcgcgc c                                                           731


<210>  152
<211>  234
<212>  PRT
<213>  artificial sequence

<220>
<223>  Synthetic Construct

<400>  152

Met Arg Leu Leu Ala Gln Leu Leu Gly Leu Leu Met Leu Trp Val Pro
1               5                   10                  15


Gly Ser Ser Gly Asp Ile Gln Met Thr Gln Ser Pro Ala Ser Leu Ser
            20                  25                  30


Ala Ser Val Gly Glu Thr Val Thr Ile Thr Cys Arg Ala Ser Glu Asn
        35                  40                  45


Ile Tyr Ser Tyr Leu Ala Trp Tyr Gln Gln Lys Gln Gly Lys Ser Pro
    50                  55                  60


Gln Leu Leu Val Tyr Asn Ala Lys Thr Leu Ala Glu Gly Val Pro Ser
```

```
65                  70                  75                  80

Arg Phe Ser Gly Ser Gly Ser Gly Thr Gln Phe Ser Leu Lys Ile Asn
                85                  90                  95

Ser Leu Gln Pro Glu Asp Phe Gly Ser Tyr Tyr Cys Gln His His Tyr
            100                 105                 110

Gly Thr Pro Tyr Thr Phe Gly Ser Gly Thr Lys Leu Glu Ile Lys Arg
        115                 120                 125

Ala Asp Ala Ala Pro Thr Val Ser Ile Phe Pro Pro Ser Ser Glu Gln
    130                 135                 140

Leu Thr Ser Gly Gly Ala Ser Val Val Cys Phe Leu Asn Asn Phe Tyr
145                 150                 155                 160

Pro Lys Asp Ile Asn Val Lys Trp Lys Ile Asp Gly Ser Glu Arg Gln
                165                 170                 175

Asn Gly Val Leu Asn Ser Trp Thr Asp Gln Asp Ser Lys Asp Ser Thr
            180                 185                 190

Tyr Ser Met Ser Ser Thr Leu Thr Leu Thr Lys Asp Glu Tyr Glu Arg
        195                 200                 205

His Asn Ser Tyr Thr Cys Glu Ala Thr His Lys Thr Ser Thr Ser Pro
    210                 215                 220

Ile Val Lys Ser Phe Asn Arg Asn Glu Cys
225                 230


<210>  153
<211>  1440
<212>  DNA
<213>  artificial sequence

<220>
<223>  Nucleic Acid Sequence of Heavy Chain of K090-01a030


<220>
<221>  CDS
<222>  (16)..(1431)

<400>  153
aagcttgccg ccacc atg aaa cac ctg tgg ttc ttc ctc ctc ctg gtg gcg         51
                 Met Lys His Leu Trp Phe Phe Leu Leu Leu Val Ala
                 1               5                   10

gcc ccc aga tgg gtc ctg tcc cag gtc cag ctg cag cag tct ggg gct           99
Ala Pro Arg Trp Val Leu Ser Gln Val Gln Leu Gln Gln Ser Gly Ala
```

```
        15                  20                  25

gag ttg gta agg cct ggg act tca gtg aag atg tcc tgc aag gct gct        147
Glu Leu Val Arg Pro Gly Thr Ser Val Lys Met Ser Cys Lys Ala Ala
    30                  35                  40

gga tac acc ttc aca aac tac tgg ata ggt tgg gta aag cag agg cct        195
Gly Tyr Thr Phe Thr Asn Tyr Trp Ile Gly Trp Val Lys Gln Arg Pro
45                  50                  55                  60

gga cat ggc ctt gag tgg att gga gat att tac cct gga ggt ggt tat        243
Gly His Gly Leu Glu Trp Ile Gly Asp Ile Tyr Pro Gly Gly Gly Tyr
                65                  70                  75

act cac tac aat gag aag ttc aag ggc aag gcc aca ctg act gca gac        291
Thr His Tyr Asn Glu Lys Phe Lys Gly Lys Ala Thr Leu Thr Ala Asp
            80                  85                  90

aca tcc tcc agc aca gcc tac atg cag ctc agc agc ctg aca tct gag        339
Thr Ser Ser Ser Thr Ala Tyr Met Gln Leu Ser Ser Leu Thr Ser Glu
        95                  100                 105

gac tct gcc atc tat tac tgt gca aga gag agg gtt tac tac gat ggt        387
Asp Ser Ala Ile Tyr Tyr Cys Ala Arg Glu Arg Val Tyr Tyr Asp Gly
    110                 115                 120

agc cgg tac ttc gat gtc tgg ggc gca ggg acc acg gtc acc gtc tcg        435
Ser Arg Tyr Phe Asp Val Trp Gly Ala Gly Thr Thr Val Thr Val Ser
125                 130                 135                 140

agc gcc aaa aca aca gcc cca tcg gtc tat cca ctg gcc cct gtg tgt        483
Ser Ala Lys Thr Thr Ala Pro Ser Val Tyr Pro Leu Ala Pro Val Cys
                145                 150                 155

gga gat aca act ggc tcc tcg gtg act cta gga tgc ctg gtc aag ggt        531
Gly Asp Thr Thr Gly Ser Ser Val Thr Leu Gly Cys Leu Val Lys Gly
            160                 165                 170

tat ttc cct gag cca gtg acc ttg acc tgg aac tct gga tcc ctg tcc        579
Tyr Phe Pro Glu Pro Val Thr Leu Thr Trp Asn Ser Gly Ser Leu Ser
        175                 180                 185

agt ggt gtg cac acc ttc cca gct gtc ctg cag tct gac ctc tac acc        627
Ser Gly Val His Thr Phe Pro Ala Val Leu Gln Ser Asp Leu Tyr Thr
    190                 195                 200

ctc agc agc tca gtg act gta acc tcg agc acc tgg ccc agc cag tcc        675
Leu Ser Ser Ser Val Thr Val Thr Ser Ser Thr Trp Pro Ser Gln Ser
205                 210                 215                 220

atc acc tgc aat gtg gcc cac ccg gca agc agc acc aag gtg gac aag        723
Ile Thr Cys Asn Val Ala His Pro Ala Ser Ser Thr Lys Val Asp Lys
                225                 230                 235

aaa att gag ccc cgg gga ccc aca atc aag ccc tgt cct cca tgc aaa        771
Lys Ile Glu Pro Arg Gly Pro Thr Ile Lys Pro Cys Pro Pro Cys Lys
            240                 245                 250

tgc cca gca cct aac ctc ttg ggt gga cca tcc gtc ttc atc ttc cct        819
Cys Pro Ala Pro Asn Leu Leu Gly Gly Pro Ser Val Phe Ile Phe Pro
        255                 260                 265

cca aag atc aag gat gta ctc atg atc tcc ctg agc ccc ata gtc aca        867
```

```
Pro Lys Ile Lys Asp Val Leu Met Ile Ser Leu Ser Pro Ile Val Thr
    270                 275                 280

tgt gtg gtg gtg gat gtg agc gag gat gac cca gat gtc cag atc agc        915
Cys Val Val Val Asp Val Ser Glu Asp Asp Pro Asp Val Gln Ile Ser
285                 290                 295                 300

tgg ttt gtg aac aac gtg gaa gta cac aca gct cag aca caa acc cat        963
Trp Phe Val Asn Asn Val Glu Val His Thr Ala Gln Thr Gln Thr His
                305                 310                 315

aga gag gat tac aac agt act ctc cgg gtg gtc agt gcc ctc ccc atc       1011
Arg Glu Asp Tyr Asn Ser Thr Leu Arg Val Val Ser Ala Leu Pro Ile
            320                 325                 330

cag cac cag gac tgg atg agt ggc aag gag ttc aaa tgc aag gtc aac       1059
Gln His Gln Asp Trp Met Ser Gly Lys Glu Phe Lys Cys Lys Val Asn
        335                 340                 345

aac aaa gac ctc cca gcg ccc atc gag aga acc atc tca aaa ccc aaa       1107
Asn Lys Asp Leu Pro Ala Pro Ile Glu Arg Thr Ile Ser Lys Pro Lys
    350                 355                 360

ggg tca gta aga gct cca cag gta tat gtc ttg cct cca cca gaa gaa       1155
Gly Ser Val Arg Ala Pro Gln Val Tyr Val Leu Pro Pro Pro Glu Glu
365                 370                 375                 380

gag atg act aag aaa cag gtc act ctg acc tgc atg gtc aca gac ttc       1203
Glu Met Thr Lys Lys Gln Val Thr Leu Thr Cys Met Val Thr Asp Phe
                385                 390                 395

atg cct gaa gac att tac gtg gag tgg acc aac aac ggg aaa aca gag       1251
Met Pro Glu Asp Ile Tyr Val Glu Trp Thr Asn Asn Gly Lys Thr Glu
            400                 405                 410

cta aac tac aag aac act gaa cca gtc ctg gac tct gat ggt tct tac       1299
Leu Asn Tyr Lys Asn Thr Glu Pro Val Leu Asp Ser Asp Gly Ser Tyr
        415                 420                 425

ttc atg tac agc aag ctg aga gtg gaa aag aag aac tgg gtg gaa aga       1347
Phe Met Tyr Ser Lys Leu Arg Val Glu Lys Lys Asn Trp Val Glu Arg
    430                 435                 440

aat agc tac tcc tgt tca gtg gtc cac gag ggt ctg cac aat cac cac       1395
Asn Ser Tyr Ser Cys Ser Val Val His Glu Gly Leu His Asn His His
445                 450                 455                 460

acg act aag agc ttc tcc cgg act ccg ggt aaa tga tagtctaga             1440
Thr Thr Lys Ser Phe Ser Arg Thr Pro Gly Lys
                465                 470


<210>  154
<211>  471
<212>  PRT
<213>  artificial sequence

<220>
<223>  Synthetic Construct

<400>  154

Met Lys His Leu Trp Phe Phe Leu Leu Leu Val Ala Ala Pro Arg Trp
```

```
1               5                   10                  15

Val Leu Ser Gln Val Gln Leu Gln Gln Ser Gly Ala Glu Leu Val Arg
            20                  25                  30

Pro Gly Thr Ser Val Lys Met Ser Cys Lys Ala Ala Gly Tyr Thr Phe
        35                  40                  45

Thr Asn Tyr Trp Ile Gly Trp Val Lys Gln Arg Pro Gly His Gly Leu
    50                  55                  60

Glu Trp Ile Gly Asp Ile Tyr Pro Gly Gly Gly Tyr Thr His Tyr Asn
65                  70                  75                  80

Glu Lys Phe Lys Gly Lys Ala Thr Leu Thr Ala Asp Thr Ser Ser Ser
                85                  90                  95

Thr Ala Tyr Met Gln Leu Ser Ser Leu Thr Ser Glu Asp Ser Ala Ile
            100                 105                 110

Tyr Tyr Cys Ala Arg Glu Arg Val Tyr Tyr Asp Gly Ser Arg Tyr Phe
        115                 120                 125

Asp Val Trp Gly Ala Gly Thr Thr Val Thr Val Ser Ser Ala Lys Thr
    130                 135                 140

Thr Ala Pro Ser Val Tyr Pro Leu Ala Pro Val Cys Gly Asp Thr Thr
145                 150                 155                 160

Gly Ser Ser Val Thr Leu Gly Cys Leu Val Lys Gly Tyr Phe Pro Glu
                165                 170                 175

Pro Val Thr Leu Thr Trp Asn Ser Gly Ser Leu Ser Ser Gly Val His
            180                 185                 190

Thr Phe Pro Ala Val Leu Gln Ser Asp Leu Tyr Thr Leu Ser Ser Ser
        195                 200                 205

Val Thr Val Thr Ser Ser Thr Trp Pro Ser Gln Ser Ile Thr Cys Asn
    210                 215                 220

Val Ala His Pro Ala Ser Ser Thr Lys Val Asp Lys Lys Ile Glu Pro
225                 230                 235                 240

Arg Gly Pro Thr Ile Lys Pro Cys Pro Pro Cys Lys Cys Pro Ala Pro
                245                 250                 255
```

Asn Leu Leu Gly Gly Pro Ser Val Phe Ile Phe Pro Pro Lys Ile Lys
260 265 270

Asp Val Leu Met Ile Ser Leu Ser Pro Ile Val Thr Cys Val Val Val
275 280 285

Asp Val Ser Glu Asp Asp Pro Asp Val Gln Ile Ser Trp Phe Val Asn
290 295 300

Asn Val Glu Val His Thr Ala Gln Thr Gln Thr His Arg Glu Asp Tyr
305 310 315 320

Asn Ser Thr Leu Arg Val Val Ser Ala Leu Pro Ile Gln His Gln Asp
325 330 335

Trp Met Ser Gly Lys Glu Phe Lys Cys Lys Val Asn Asn Lys Asp Leu
340 345 350

Pro Ala Pro Ile Glu Arg Thr Ile Ser Lys Pro Lys Gly Ser Val Arg
355 360 365

Ala Pro Gln Val Tyr Val Leu Pro Pro Pro Glu Glu Glu Met Thr Lys
370 375 380

Lys Gln Val Thr Leu Thr Cys Met Val Thr Asp Phe Met Pro Glu Asp
385 390 395 400

Ile Tyr Val Glu Trp Thr Asn Asn Gly Lys Thr Glu Leu Asn Tyr Lys
405 410 415

Asn Thr Glu Pro Val Leu Asp Ser Asp Gly Ser Tyr Phe Met Tyr Ser
420 425 430

Lys Leu Arg Val Glu Lys Lys Asn Trp Val Glu Arg Asn Ser Tyr Ser
435 440 445

Cys Ser Val Val His Glu Gly Leu His Asn His His Thr Thr Lys Ser
450 455 460

Phe Ser Arg Thr Pro Gly Lys
465 470

<210> 155
<211> 731
<212> DNA
<213> artificial sequence

<220>
<223> Nucleic Acid Sequence of Light Chain of K090-01a030

<220>
<221> CDS
<222> (16)..(720)

<400> 155
```
aagcttgccg ccacc atg agg ctc ctt gct cag ctt ctg ggg ctg cta atg        51
                 Met Arg Leu Leu Ala Gln Leu Leu Gly Leu Leu Met
                 1               5                   10

ctc tgg gtc cct gga tcc agt ggg gac atc aag atg acc cag tct ccc         99
Leu Trp Val Pro Gly Ser Ser Gly Asp Ile Lys Met Thr Gln Ser Pro
        15                  20                  25

aaa ttc ctg cct gta tca gca gga gac agg gtt acc atg acc tgc aag        147
Lys Phe Leu Pro Val Ser Ala Gly Asp Arg Val Thr Met Thr Cys Lys
    30                  35                  40

gcc agt cag agt gtg ggt aat aat gta gcc tgg tac caa cag aag cca        195
Ala Ser Gln Ser Val Gly Asn Asn Val Ala Trp Tyr Gln Gln Lys Pro
45                  50                  55                  60

gga cag tct cct aaa ctg ctg ata tac tat gca tcc aat cgc tac act        243
Gly Gln Ser Pro Lys Leu Leu Ile Tyr Tyr Ala Ser Asn Arg Tyr Thr
                65                  70                  75

gga gtc cct gat cgc ttc act ggc agt gga tct ggg aca gat ttc act        291
Gly Val Pro Asp Arg Phe Thr Gly Ser Gly Ser Gly Thr Asp Phe Thr
            80                  85                  90

ttc acc atc agc agt gtg cag gtt gaa gac ctg gca gtt tat ttc tgt        339
Phe Thr Ile Ser Ser Val Gln Val Glu Asp Leu Ala Val Tyr Phe Cys
        95                  100                 105

cag cag cat tat agc tct ccg tgg acg ttc ggt gga ggc aca aag ttg        387
Gln Gln His Tyr Ser Ser Pro Trp Thr Phe Gly Gly Gly Thr Lys Leu
    110                 115                 120

gaa ata aaa cgg gct gat gct gca cca act gta tcc atc ttc cca cca        435
Glu Ile Lys Arg Ala Asp Ala Ala Pro Thr Val Ser Ile Phe Pro Pro
125                 130                 135                 140

tcc agt gag cag tta aca tct gga ggt gcc tca gtc gtg tgc ttc ttg        483
Ser Ser Glu Gln Leu Thr Ser Gly Gly Ala Ser Val Val Cys Phe Leu
                145                 150                 155

aac aac ttc tac ccc aaa gac atc aat gtc aag tgg aag att gat ggc        531
Asn Asn Phe Tyr Pro Lys Asp Ile Asn Val Lys Trp Lys Ile Asp Gly
            160                 165                 170

agt gaa cga caa aat ggc gtc ctg aac agt tgg act gat cag gac agc        579
Ser Glu Arg Gln Asn Gly Val Leu Asn Ser Trp Thr Asp Gln Asp Ser
        175                 180                 185

aaa gac agc acc tac agc atg agc agc acc ctc acg ttg acc aag gac        627
Lys Asp Ser Thr Tyr Ser Met Ser Ser Thr Leu Thr Leu Thr Lys Asp
    190                 195                 200

gag tat gaa cga cat aac agc tat acc tgt gag gcc act cac aag aca        675
Glu Tyr Glu Arg His Asn Ser Tyr Thr Cys Glu Ala Thr His Lys Thr
205                 210                 215                 220
```

```
tca act tca ccc att gtc aag agc ttc aac agg aat gag tgt tga          720
Ser Thr Ser Pro Ile Val Lys Ser Phe Asn Arg Asn Glu Cys
                225                 230

tagggcgcgc c                                                         731


<210>  156
<211>  234
<212>  PRT
<213>  artificial sequence

<220>
<223>  Synthetic Construct

<400>  156

Met Arg Leu Leu Ala Gln Leu Leu Gly Leu Leu Met Leu Trp Val Pro
1               5                   10                  15


Gly Ser Ser Gly Asp Ile Lys Met Thr Gln Ser Pro Lys Phe Leu Pro
            20                  25                  30


Val Ser Ala Gly Asp Arg Val Thr Met Thr Cys Lys Ala Ser Gln Ser
        35                  40                  45


Val Gly Asn Asn Val Ala Trp Tyr Gln Gln Lys Pro Gly Gln Ser Pro
    50                  55                  60


Lys Leu Leu Ile Tyr Tyr Ala Ser Asn Arg Tyr Thr Gly Val Pro Asp
65                  70                  75                  80


Arg Phe Thr Gly Ser Gly Ser Gly Thr Asp Phe Thr Phe Thr Ile Ser
                85                  90                  95


Ser Val Gln Val Glu Asp Leu Ala Val Tyr Phe Cys Gln Gln His Tyr
            100                 105                 110


Ser Ser Pro Trp Thr Phe Gly Gly Gly Thr Lys Leu Glu Ile Lys Arg
        115                 120                 125


Ala Asp Ala Ala Pro Thr Val Ser Ile Phe Pro Pro Ser Ser Glu Gln
    130                 135                 140


Leu Thr Ser Gly Gly Ala Ser Val Val Cys Phe Leu Asn Asn Phe Tyr
145                 150                 155                 160


Pro Lys Asp Ile Asn Val Lys Trp Lys Ile Asp Gly Ser Glu Arg Gln
                165                 170                 175


Asn Gly Val Leu Asn Ser Trp Thr Asp Gln Asp Ser Lys Asp Ser Thr
            180                 185                 190
```

```
Tyr Ser Met Ser Ser Thr Leu Thr Leu Thr Lys Asp Glu Tyr Glu Arg
        195                 200                 205

His Asn Ser Tyr Thr Cys Glu Ala Thr His Lys Thr Ser Thr Ser Pro
    210                 215                 220

Ile Val Lys Ser Phe Asn Arg Asn Glu Cys
225                 230


<210>  157
<211>  1431
<212>  DNA
<213>  artificial sequence

<220>
<223>  Nucleic Acid Sequence of Heavy Chain of K090-01a033


<220>
<221>  CDS
<222>  (16)..(1422)

<400>  157
aagcttgccg ccacc atg aaa cac ctg tgg ttc ttc ctc ctc ctg gtg gcg       51
                 Met Lys His Leu Trp Phe Phe Leu Leu Leu Val Ala
                 1               5                   10

gcc ccc aga tgg gtc ctg tcc cag gtg cag ctg aag cag tca gga cct        99
Ala Pro Arg Trp Val Leu Ser Gln Val Gln Leu Lys Gln Ser Gly Pro
        15                  20                  25

gaa ctg gtg aag cct ggg gct tca gtg aag ata tca tgc aag gct tct       147
Glu Leu Val Lys Pro Gly Ala Ser Val Lys Ile Ser Cys Lys Ala Ser
    30                  35                  40

ggt tac tca ttc act ggc tac tac atg cac tgg gtg aag caa agt cct       195
Gly Tyr Ser Phe Thr Gly Tyr Tyr Met His Trp Val Lys Gln Ser Pro
45                  50                  55                  60

gaa aag agc ctt gag tgg att gga gag att aat cct agc act ggt gat       243
Glu Lys Ser Leu Glu Trp Ile Gly Glu Ile Asn Pro Ser Thr Gly Asp
                65                  70                  75

act acc tac aac cag aag ttc aag gcc aag gcc aca ttg act gta gac       291
Thr Thr Tyr Asn Gln Lys Phe Lys Ala Lys Ala Thr Leu Thr Val Asp
            80                  85                  90

aaa tcc tcc agc aca gcc tac atg cag ctc aag agc ctg aca tct gag       339
Lys Ser Ser Ser Thr Ala Tyr Met Gln Leu Lys Ser Leu Thr Ser Glu
        95                  100                 105

gac tct gca gtc tat tac tgt gca aga gag aag aga gat gat ggc gta       387
Asp Ser Ala Val Tyr Tyr Cys Ala Arg Glu Lys Arg Asp Asp Gly Val
    110                 115                 120

ttt gct tac tgg ggc caa ggg act ctg gtc act gtc tcg agc gcc aaa       435
Phe Ala Tyr Trp Gly Gln Gly Thr Leu Val Thr Val Ser Ser Ala Lys
125                 130                 135                 140
```

```
aca aca gcc cca tcg gtc tat cca ctg gcc cct gtg tgt gga gat aca        483
Thr Thr Ala Pro Ser Val Tyr Pro Leu Ala Pro Val Cys Gly Asp Thr
                145                 150                 155

act ggc tcc tcg gtg act cta gga tgc ctg gtc aag ggt tat ttc cct        531
Thr Gly Ser Ser Val Thr Leu Gly Cys Leu Val Lys Gly Tyr Phe Pro
            160                 165                 170

gag cca gtg acc ttg acc tgg aac tct gga tcc ctg tcc agt ggt gtg        579
Glu Pro Val Thr Leu Thr Trp Asn Ser Gly Ser Leu Ser Ser Gly Val
        175                 180                 185

cac acc ttc cca gct gtc ctg cag tct gac ctc tac acc ctc agc agc        627
His Thr Phe Pro Ala Val Leu Gln Ser Asp Leu Tyr Thr Leu Ser Ser
    190                 195                 200

tca gtg act gta acc tcg agc acc tgg ccc agc cag tcc atc acc tgc        675
Ser Val Thr Val Thr Ser Ser Thr Trp Pro Ser Gln Ser Ile Thr Cys
205                 210                 215                 220

aat gtg gcc cac ccg gca agc agc acc aag gtg gac aag aaa att gag        723
Asn Val Ala His Pro Ala Ser Ser Thr Lys Val Asp Lys Lys Ile Glu
                225                 230                 235

ccc cgg gga ccc aca atc aag ccc tgt cct cca tgc aaa tgc cca gca        771
Pro Arg Gly Pro Thr Ile Lys Pro Cys Pro Pro Cys Lys Cys Pro Ala
            240                 245                 250

cct aac ctc ttg ggt gga cca tcc gtc ttc atc ttc cct cca aag atc        819
Pro Asn Leu Leu Gly Gly Pro Ser Val Phe Ile Phe Pro Pro Lys Ile
        255                 260                 265

aag gat gta ctc atg atc tcc ctg agc ccc ata gtc aca tgt gtg gtg        867
Lys Asp Val Leu Met Ile Ser Leu Ser Pro Ile Val Thr Cys Val Val
    270                 275                 280

gtg gat gtg agc gag gat gac cca gat gtc cag atc agc tgg ttt gtg        915
Val Asp Val Ser Glu Asp Asp Pro Asp Val Gln Ile Ser Trp Phe Val
285                 290                 295                 300

aac aac gtg gaa gta cac aca gct cag aca caa acc cat aga gag gat        963
Asn Asn Val Glu Val His Thr Ala Gln Thr Gln Thr His Arg Glu Asp
                305                 310                 315

tac aac agt act ctc cgg gtg gtc agt gcc ctc ccc atc cag cac cag       1011
Tyr Asn Ser Thr Leu Arg Val Val Ser Ala Leu Pro Ile Gln His Gln
            320                 325                 330

gac tgg atg agt ggc aag gag ttc aaa tgc aag gtc aac aac aaa gac       1059
Asp Trp Met Ser Gly Lys Glu Phe Lys Cys Lys Val Asn Asn Lys Asp
        335                 340                 345

ctc cca gcg ccc atc gag aga acc atc tca aaa ccc aaa ggg tca gta       1107
Leu Pro Ala Pro Ile Glu Arg Thr Ile Ser Lys Pro Lys Gly Ser Val
    350                 355                 360

aga gct cca cag gta tat gtc ttg cct cca cca gaa gaa gag atg act       1155
Arg Ala Pro Gln Val Tyr Val Leu Pro Pro Pro Glu Glu Glu Met Thr
365                 370                 375                 380

aag aaa cag gtc act ctg acc tgc atg gtc aca gac ttc atg cct gaa       1203
Lys Lys Gln Val Thr Leu Thr Cys Met Val Thr Asp Phe Met Pro Glu
```

```
                385                 390                 395

gac att tac gtg gag tgg acc aac aac ggg aaa aca gag cta aac tac       1251
Asp Ile Tyr Val Glu Trp Thr Asn Asn Gly Lys Thr Glu Leu Asn Tyr
            400                 405                 410

aag aac act gaa cca gtc ctg gac tct gat ggt tct tac ttc atg tac       1299
Lys Asn Thr Glu Pro Val Leu Asp Ser Asp Gly Ser Tyr Phe Met Tyr
        415                 420                 425

agc aag ctg aga gtg gaa aag aag aac tgg gtg gaa aga aat agc tac       1347
Ser Lys Leu Arg Val Glu Lys Lys Asn Trp Val Glu Arg Asn Ser Tyr
    430                 435                 440

tcc tgt tca gtg gtc cac gag ggt ctg cac aat cac cac acg act aag       1395
Ser Cys Ser Val Val His Glu Gly Leu His Asn His His Thr Thr Lys
445                 450                 455                 460

agc ttc tcc cgg act ccg ggt aaa tga tagtctaga                         1431
Ser Phe Ser Arg Thr Pro Gly Lys
                465


<210>  158
<211>  468
<212>  PRT
<213>  artificial sequence

<220>
<223>  Synthetic Construct

<400>  158

Met Lys His Leu Trp Phe Phe Leu Leu Leu Val Ala Ala Pro Arg Trp
1               5                   10                  15


Val Leu Ser Gln Val Gln Leu Lys Gln Ser Gly Pro Glu Leu Val Lys
            20                  25                  30


Pro Gly Ala Ser Val Lys Ile Ser Cys Lys Ala Ser Gly Tyr Ser Phe
        35                  40                  45


Thr Gly Tyr Tyr Met His Trp Val Lys Gln Ser Pro Glu Lys Ser Leu
    50                  55                  60


Glu Trp Ile Gly Glu Ile Asn Pro Ser Thr Gly Asp Thr Thr Tyr Asn
65                  70                  75                  80


Gln Lys Phe Lys Ala Lys Ala Thr Leu Thr Val Asp Lys Ser Ser Ser
                85                  90                  95


Thr Ala Tyr Met Gln Leu Lys Ser Leu Thr Ser Glu Asp Ser Ala Val
            100                 105                 110


Tyr Tyr Cys Ala Arg Glu Lys Arg Asp Asp Gly Val Phe Ala Tyr Trp
        115                 120                 125
```

Gly Gln Gly Thr Leu Val Thr Val Ser Ser Ala Lys Thr Thr Ala Pro
    130                 135                 140

Ser Val Tyr Pro Leu Ala Pro Val Cys Gly Asp Thr Thr Gly Ser Ser
145                 150                 155                 160

Val Thr Leu Gly Cys Leu Val Lys Gly Tyr Phe Pro Glu Pro Val Thr
                165                 170                 175

Leu Thr Trp Asn Ser Gly Ser Leu Ser Ser Gly Val His Thr Phe Pro
            180                 185                 190

Ala Val Leu Gln Ser Asp Leu Tyr Thr Leu Ser Ser Ser Val Thr Val
        195                 200                 205

Thr Ser Ser Thr Trp Pro Ser Gln Ser Ile Thr Cys Asn Val Ala His
    210                 215                 220

Pro Ala Ser Ser Thr Lys Val Asp Lys Lys Ile Glu Pro Arg Gly Pro
225                 230                 235                 240

Thr Ile Lys Pro Cys Pro Pro Cys Lys Cys Pro Ala Pro Asn Leu Leu
                245                 250                 255

Gly Gly Pro Ser Val Phe Ile Phe Pro Pro Lys Ile Lys Asp Val Leu
            260                 265                 270

Met Ile Ser Leu Ser Pro Ile Val Thr Cys Val Val Val Asp Val Ser
        275                 280                 285

Glu Asp Asp Pro Asp Val Gln Ile Ser Trp Phe Val Asn Asn Val Glu
    290                 295                 300

Val His Thr Ala Gln Thr Gln Thr His Arg Glu Asp Tyr Asn Ser Thr
305                 310                 315                 320

Leu Arg Val Val Ser Ala Leu Pro Ile Gln His Gln Asp Trp Met Ser
                325                 330                 335

Gly Lys Glu Phe Lys Cys Lys Val Asn Asn Lys Asp Leu Pro Ala Pro
            340                 345                 350

Ile Glu Arg Thr Ile Ser Lys Pro Lys Gly Ser Val Arg Ala Pro Gln
        355                 360                 365

Val Tyr Val Leu Pro Pro Pro Glu Glu Glu Met Thr Lys Lys Gln Val

```
    370                 375                 380

Thr Leu Thr Cys Met Val Thr Asp Phe Met Pro Glu Asp Ile Tyr Val
385                 390                 395                 400

Glu Trp Thr Asn Asn Gly Lys Thr Glu Leu Asn Tyr Lys Asn Thr Glu
                405                 410                 415

Pro Val Leu Asp Ser Asp Gly Ser Tyr Phe Met Tyr Ser Lys Leu Arg
            420                 425                 430

Val Glu Lys Lys Asn Trp Val Glu Arg Asn Ser Tyr Ser Cys Ser Val
        435                 440                 445

Val His Glu Gly Leu His Asn His His Thr Thr Lys Ser Phe Ser Arg
    450                 455                 460

Thr Pro Gly Lys
465


<210>  159
<211>  731
<212>  DNA
<213>  artificial sequence

<220>
<223>  Nucleic Acid Sequence of Light Chain of K090-01a033


<220>
<221>  CDS
<222>  (16)..(720)

<400>  159
aagcttgccg ccacc atg agg ctc ctt gct cag ctt ctg ggg ctg cta atg        51
                 Met Arg Leu Leu Ala Gln Leu Leu Gly Leu Leu Met
                 1               5                   10

ctc tgg gtc cct gga tcc agt ggg gac att gtg atg tca cag tct ccc        99
Leu Trp Val Pro Gly Ser Ser Gly Asp Ile Val Met Ser Gln Ser Pro
        15                  20                  25

aaa tcc atg tcc atg tca gta gga gag agg gtc gcc ttg agc tgc aag       147
Lys Ser Met Ser Met Ser Val Gly Glu Arg Val Ala Leu Ser Cys Lys
    30                  35                  40

gcc agt gag aat gtg ggt act tat gta tcc tgg tat caa cag aaa cca       195
Ala Ser Glu Asn Val Gly Thr Tyr Val Ser Trp Tyr Gln Gln Lys Pro
45                  50                  55                  60

gag cag tct cct aaa ctg ctg ata tac ggg gca tcc aac cgg tac act       243
Glu Gln Ser Pro Lys Leu Leu Ile Tyr Gly Ala Ser Asn Arg Tyr Thr
                65                  70                  75

ggg gtc ccc gat cgc ttc aca ggc agt gga tct gca aca gat ttc act       291
Gly Val Pro Asp Arg Phe Thr Gly Ser Gly Ser Ala Thr Asp Phe Thr
```

```
            80                  85                  90

ctg acc atc agc agt gtg cag gct gaa gac ctt gca gat tat tac tgt      339
Leu Thr Ile Ser Ser Val Gln Ala Glu Asp Leu Ala Asp Tyr Tyr Cys
        95                  100                 105

gga cag agt tac agc tat cct ctc acg ttc ggt gct ggg acc aag ctg      387
Gly Gln Ser Tyr Ser Tyr Pro Leu Thr Phe Gly Ala Gly Thr Lys Leu
    110                 115                 120

gag ctg aaa cgg gct gat gct gca cca act gta tcc atc ttc cca cca      435
Glu Leu Lys Arg Ala Asp Ala Ala Pro Thr Val Ser Ile Phe Pro Pro
125                 130                 135                 140

tcc agt gag cag tta aca tct gga ggt gcc tca gtc gtg tgc ttc ttg      483
Ser Ser Glu Gln Leu Thr Ser Gly Gly Ala Ser Val Val Cys Phe Leu
                145                 150                 155

aac aac ttc tac ccc aaa gac atc aat gtc aag tgg aag att gat ggc      531
Asn Asn Phe Tyr Pro Lys Asp Ile Asn Val Lys Trp Lys Ile Asp Gly
            160                 165                 170

agt gaa cga caa aat ggc gtc ctg aac agt tgg act gat cag gac agc      579
Ser Glu Arg Gln Asn Gly Val Leu Asn Ser Trp Thr Asp Gln Asp Ser
        175                 180                 185

aaa gac agc acc tac agc atg agc agc acc ctc acg ttg acc aag gac      627
Lys Asp Ser Thr Tyr Ser Met Ser Ser Thr Leu Thr Leu Thr Lys Asp
    190                 195                 200

gag tat gaa cga cat aac agc tat acc tgt gag gcc act cac aag aca      675
Glu Tyr Glu Arg His Asn Ser Tyr Thr Cys Glu Ala Thr His Lys Thr
205                 210                 215                 220

tca act tca ccc att gtc aag agc ttc aac agg aat gag tgt tga          720
Ser Thr Ser Pro Ile Val Lys Ser Phe Asn Arg Asn Glu Cys
                225                 230

tagggcgcgc c                                                         731


<210>  160
<211>  234
<212>  PRT
<213>  artificial sequence

<220>
<223>  Synthetic Construct

<400>  160

Met Arg Leu Leu Ala Gln Leu Leu Gly Leu Leu Met Leu Trp Val Pro
1               5                   10                  15


Gly Ser Ser Gly Asp Ile Val Met Ser Gln Ser Pro Lys Ser Met Ser
            20                  25                  30


Met Ser Val Gly Glu Arg Val Ala Leu Ser Cys Lys Ala Ser Glu Asn
        35                  40                  45
```

Val Gly Thr Tyr Val Ser Trp Tyr Gln Gln Lys Pro Glu Gln Ser Pro
50 55 60

Lys Leu Leu Ile Tyr Gly Ala Ser Asn Arg Tyr Thr Gly Val Pro Asp
65 70 75 80

Arg Phe Thr Gly Ser Gly Ser Ala Thr Asp Phe Thr Leu Thr Ile Ser
85 90 95

Ser Val Gln Ala Glu Asp Leu Ala Asp Tyr Tyr Cys Gly Gln Ser Tyr
100 105 110

Ser Tyr Pro Leu Thr Phe Gly Ala Gly Thr Lys Leu Glu Leu Lys Arg
115 120 125

Ala Asp Ala Ala Pro Thr Val Ser Ile Phe Pro Pro Ser Ser Glu Gln
130 135 140

Leu Thr Ser Gly Gly Ala Ser Val Val Cys Phe Leu Asn Asn Phe Tyr
145 150 155 160

Pro Lys Asp Ile Asn Val Lys Trp Lys Ile Asp Gly Ser Glu Arg Gln
165 170 175

Asn Gly Val Leu Asn Ser Trp Thr Asp Gln Asp Ser Lys Asp Ser Thr
180 185 190

Tyr Ser Met Ser Ser Thr Leu Thr Leu Thr Lys Asp Glu Tyr Glu Arg
195 200 205

His Asn Ser Tyr Thr Cys Glu Ala Thr His Lys Thr Ser Thr Ser Pro
210 215 220

Ile Val Lys Ser Phe Asn Arg Asn Glu Cys
225 230

<210> 161
<211> 1431
<212> DNA
<213> artificial sequence

<220>
<223> Nucleic Acid Sequence of Heavy Chain of K090-01a042

<220>
<221> CDS
<222> (16)..(1422)

<400> 161
aagcttgccg ccacc atg aaa cac ctg tgg ttc ttc ctc ctc ctg gtg gcg 51

```
                  Met Lys His Leu Trp Phe Phe Leu Leu Leu Val Ala
                  1               5                   10

gcc ccc aga tgg gtc ctg tcc cag gtc caa ctg cag cag cct ggg gct       99
Ala Pro Arg Trp Val Leu Ser Gln Val Gln Leu Gln Gln Pro Gly Ala
        15                  20                  25

gaa ctg gca aaa cct ggg gcc tca gtg aag atg tcc tgc aag gct tct      147
Glu Leu Ala Lys Pro Gly Ala Ser Val Lys Met Ser Cys Lys Ala Ser
    30                  35                  40

ggc tac acc ttt act agg tac tgg atg cat tgg gta aaa cag agg cct      195
Gly Tyr Thr Phe Thr Arg Tyr Trp Met His Trp Val Lys Gln Arg Pro
45                  50                  55                  60

gga cag ggt ctg gaa tgg att gga tac att aat cct agc agt ggt tat      243
Gly Gln Gly Leu Glu Trp Ile Gly Tyr Ile Asn Pro Ser Ser Gly Tyr
                65                  70                  75

act gag tac aat cag aag ttc aag gac aag gcc aca ttg act gca gac      291
Thr Glu Tyr Asn Gln Lys Phe Lys Asp Lys Ala Thr Leu Thr Ala Asp
            80                  85                  90

aaa tcc tcc agc aca gcc tac atg caa cta agc agc ctg aca tct gag      339
Lys Ser Ser Ser Thr Ala Tyr Met Gln Leu Ser Ser Leu Thr Ser Glu
        95                  100                 105

gac tct gca gtc tat tac tgt gta aga ctt ggt gac tac tcg tac tac      387
Asp Ser Ala Val Tyr Tyr Cys Val Arg Leu Gly Asp Tyr Ser Tyr Tyr
    110                 115                 120

ttt gac tac tgg ggc caa ggc acc act ctc aca gtc tcg agc gcc aaa      435
Phe Asp Tyr Trp Gly Gln Gly Thr Thr Leu Thr Val Ser Ser Ala Lys
125                 130                 135                 140

aca aca gcc cca tcg gtc tat cca ctg gcc cct gtg tgt gga gat aca      483
Thr Thr Ala Pro Ser Val Tyr Pro Leu Ala Pro Val Cys Gly Asp Thr
                145                 150                 155

act ggc tcc tcg gtg act cta gga tgc ctg gtc aag ggt tat ttc cct      531
Thr Gly Ser Ser Val Thr Leu Gly Cys Leu Val Lys Gly Tyr Phe Pro
            160                 165                 170

gag cca gtg acc ttg acc tgg aac tct gga tcc ctg tcc agt ggt gtg      579
Glu Pro Val Thr Leu Thr Trp Asn Ser Gly Ser Leu Ser Ser Gly Val
        175                 180                 185

cac acc ttc cca gct gtc ctg cag tct gac ctc tac acc ctc agc agc      627
His Thr Phe Pro Ala Val Leu Gln Ser Asp Leu Tyr Thr Leu Ser Ser
    190                 195                 200

tca gtg act gta acc tcg agc acc tgg ccc agc cag tcc atc acc tgc      675
Ser Val Thr Val Thr Ser Ser Thr Trp Pro Ser Gln Ser Ile Thr Cys
205                 210                 215                 220

aat gtg gcc cac ccg gca agc agc acc aag gtg gac aag aaa att gag      723
Asn Val Ala His Pro Ala Ser Ser Thr Lys Val Asp Lys Lys Ile Glu
                225                 230                 235

ccc cgg gga ccc aca atc aag ccc tgt cct cca tgc aaa tgc cca gca      771
Pro Arg Gly Pro Thr Ile Lys Pro Cys Pro Pro Cys Lys Cys Pro Ala
            240                 245                 250
```

```
cct aac ctc ttg ggt gga cca tcc gtc ttc atc ttc cct cca aag atc        819
Pro Asn Leu Leu Gly Gly Pro Ser Val Phe Ile Phe Pro Pro Lys Ile
        255                 260                 265

aag gat gta ctc atg atc tcc ctg agc ccc ata gtc aca tgt gtg gtg        867
Lys Asp Val Leu Met Ile Ser Leu Ser Pro Ile Val Thr Cys Val Val
    270                 275                 280

gtg gat gtg agc gag gat gac cca gat gtc cag atc agc tgg ttt gtg        915
Val Asp Val Ser Glu Asp Asp Pro Asp Val Gln Ile Ser Trp Phe Val
285                 290                 295                 300

aac aac gtg gaa gta cac aca gct cag aca caa acc cat aga gag gat        963
Asn Asn Val Glu Val His Thr Ala Gln Thr Gln Thr His Arg Glu Asp
                305                 310                 315

tac aac agt act ctc cgg gtg gtc agt gcc ctc ccc atc cag cac cag       1011
Tyr Asn Ser Thr Leu Arg Val Val Ser Ala Leu Pro Ile Gln His Gln
            320                 325                 330

gac tgg atg agt ggc aag gag ttc aaa tgc aag gtc aac aac aaa gac       1059
Asp Trp Met Ser Gly Lys Glu Phe Lys Cys Lys Val Asn Asn Lys Asp
        335                 340                 345

ctc cca gcg ccc atc gag aga acc atc tca aaa ccc aaa ggg tca gta       1107
Leu Pro Ala Pro Ile Glu Arg Thr Ile Ser Lys Pro Lys Gly Ser Val
    350                 355                 360

aga gct cca cag gta tat gtc ttg cct cca cca gaa gaa gag atg act       1155
Arg Ala Pro Gln Val Tyr Val Leu Pro Pro Pro Glu Glu Glu Met Thr
365                 370                 375                 380

aag aaa cag gtc act ctg acc tgc atg gtc aca gac ttc atg cct gaa       1203
Lys Lys Gln Val Thr Leu Thr Cys Met Val Thr Asp Phe Met Pro Glu
                385                 390                 395

gac att tac gtg gag tgg acc aac aac ggg aaa aca gag cta aac tac       1251
Asp Ile Tyr Val Glu Trp Thr Asn Asn Gly Lys Thr Glu Leu Asn Tyr
            400                 405                 410

aag aac act gaa cca gtc ctg gac tct gat ggt tct tac ttc atg tac       1299
Lys Asn Thr Glu Pro Val Leu Asp Ser Asp Gly Ser Tyr Phe Met Tyr
        415                 420                 425

agc aag ctg aga gtg gaa aag aag aac tgg gtg gaa aga aat agc tac       1347
Ser Lys Leu Arg Val Glu Lys Lys Asn Trp Val Glu Arg Asn Ser Tyr
    430                 435                 440

tcc tgt tca gtg gtc cac gag ggt ctg cac aat cac cac acg act aag       1395
Ser Cys Ser Val Val His Glu Gly Leu His Asn His His Thr Thr Lys
445                 450                 455                 460

agc ttc tcc cgg act ccg ggt aaa tga tagtctaga                         1431
Ser Phe Ser Arg Thr Pro Gly Lys
                465
```

<210> 162
<211> 468
<212> PRT
<213> artificial sequence

<220>

<223> Synthetic Construct

<400> 162

Met Lys His Leu Trp Phe Phe Leu Leu Leu Val Ala Ala Pro Arg Trp
1 5 10 15

Val Leu Ser Gln Val Gln Leu Gln Gln Pro Gly Ala Glu Leu Ala Lys
20 25 30

Pro Gly Ala Ser Val Lys Met Ser Cys Lys Ala Ser Gly Tyr Thr Phe
35 40 45

Thr Arg Tyr Trp Met His Trp Val Lys Gln Arg Pro Gly Gln Gly Leu
50 55 60

Glu Trp Ile Gly Tyr Ile Asn Pro Ser Ser Gly Tyr Thr Glu Tyr Asn
65 70 75 80

Gln Lys Phe Lys Asp Lys Ala Thr Leu Thr Ala Asp Lys Ser Ser Ser
85 90 95

Thr Ala Tyr Met Gln Leu Ser Ser Leu Thr Ser Glu Asp Ser Ala Val
100 105 110

Tyr Tyr Cys Val Arg Leu Gly Asp Tyr Ser Tyr Tyr Phe Asp Tyr Trp
115 120 125

Gly Gln Gly Thr Thr Leu Thr Val Ser Ser Ala Lys Thr Thr Ala Pro
130 135 140

Ser Val Tyr Pro Leu Ala Pro Val Cys Gly Asp Thr Thr Gly Ser Ser
145 150 155 160

Val Thr Leu Gly Cys Leu Val Lys Gly Tyr Phe Pro Glu Pro Val Thr
165 170 175

Leu Thr Trp Asn Ser Gly Ser Leu Ser Ser Gly Val His Thr Phe Pro
180 185 190

Ala Val Leu Gln Ser Asp Leu Tyr Thr Leu Ser Ser Ser Val Thr Val
195 200 205

Thr Ser Ser Thr Trp Pro Ser Gln Ser Ile Thr Cys Asn Val Ala His
210 215 220

Pro Ala Ser Ser Thr Lys Val Asp Lys Lys Ile Glu Pro Arg Gly Pro
225 230 235 240

Thr Ile Lys Pro Cys Pro Pro Cys Lys Cys Pro Ala Pro Asn Leu Leu
245 250 255

Gly Gly Pro Ser Val Phe Ile Phe Pro Pro Lys Ile Lys Asp Val Leu
260 265 270

Met Ile Ser Leu Ser Pro Ile Val Thr Cys Val Val Val Asp Val Ser
275 280 285

Glu Asp Asp Pro Asp Val Gln Ile Ser Trp Phe Val Asn Asn Val Glu
290 295 300

Val His Thr Ala Gln Thr Gln Thr His Arg Glu Asp Tyr Asn Ser Thr
305 310 315 320

Leu Arg Val Val Ser Ala Leu Pro Ile Gln His Gln Asp Trp Met Ser
325 330 335

Gly Lys Glu Phe Lys Cys Lys Val Asn Asn Lys Asp Leu Pro Ala Pro
340 345 350

Ile Glu Arg Thr Ile Ser Lys Pro Lys Gly Ser Val Arg Ala Pro Gln
355 360 365

Val Tyr Val Leu Pro Pro Pro Glu Glu Glu Met Thr Lys Lys Gln Val
370 375 380

Thr Leu Thr Cys Met Val Thr Asp Phe Met Pro Glu Asp Ile Tyr Val
385 390 395 400

Glu Trp Thr Asn Asn Gly Lys Thr Glu Leu Asn Tyr Lys Asn Thr Glu
405 410 415

Pro Val Leu Asp Ser Asp Gly Ser Tyr Phe Met Tyr Ser Lys Leu Arg
420 425 430

Val Glu Lys Lys Asn Trp Val Glu Arg Asn Ser Tyr Ser Cys Ser Val
435 440 445

Val His Glu Gly Leu His Asn His His Thr Thr Lys Ser Phe Ser Arg
450 455 460

Thr Pro Gly Lys
465

<210> 163
<211> 746

```
<212>  DNA
<213>  artificial sequence

<220>
<223>  Nucleic Acid Sequence of Light Chain of K090-01a042


<220>
<221>  CDS
<222>  (16)..(735)

<400>  163
aagcttgccg ccacc atg agg ctc ctt gct cag ctt ctg ggg ctg cta atg        51
                 Met Arg Leu Leu Ala Gln Leu Leu Gly Leu Leu Met
                 1               5                   10

ctc tgg gtc cct gga tcc agt ggg gat gtt gtg atg acc caa act cca         99
Leu Trp Val Pro Gly Ser Ser Gly Asp Val Val Met Thr Gln Thr Pro
        15                  20                  25

ctc tcc ctg cct gtc agt ctt gga gat caa gcc tcc atc tct tgc aga        147
Leu Ser Leu Pro Val Ser Leu Gly Asp Gln Ala Ser Ile Ser Cys Arg
    30                  35                  40

tct agt cag agc att gta cat agt aat gga aac acc tat tta gaa tgg        195
Ser Ser Gln Ser Ile Val His Ser Asn Gly Asn Thr Tyr Leu Glu Trp
45                  50                  55                  60

tac ctg cag aaa cca ggc cag tct cca aag ctc ctg atc tac aaa gtt        243
Tyr Leu Gln Lys Pro Gly Gln Ser Pro Lys Leu Leu Ile Tyr Lys Val
                65                  70                  75

tcc aac cga ttt tct ggg gtc cca gac agg ttc agt ggc agt gga tca        291
Ser Asn Arg Phe Ser Gly Val Pro Asp Arg Phe Ser Gly Ser Gly Ser
            80                  85                  90

ggg aca gat ttc aca ctc aag atc agc aga gtg gag gct gag gat ctg        339
Gly Thr Asp Phe Thr Leu Lys Ile Ser Arg Val Glu Ala Glu Asp Leu
        95                  100                 105

gga gtt tat tac tgc ttt caa ggt tca cat gtt ccg tgg acg ttc ggt        387
Gly Val Tyr Tyr Cys Phe Gln Gly Ser His Val Pro Trp Thr Phe Gly
    110                 115                 120

gga ggc acc aag ctg gag ctg aaa cgg gct gat gct gca cca act gta        435
Gly Gly Thr Lys Leu Glu Leu Lys Arg Ala Asp Ala Ala Pro Thr Val
125                 130                 135                 140

tcc atc ttc cca cca tcc agt gag cag tta aca tct gga ggt gcc tca        483
Ser Ile Phe Pro Pro Ser Ser Glu Gln Leu Thr Ser Gly Gly Ala Ser
                145                 150                 155

gtc gtg tgc ttc ttg aac aac ttc tac ccc aaa gac atc aat gtc aag        531
Val Val Cys Phe Leu Asn Asn Phe Tyr Pro Lys Asp Ile Asn Val Lys
            160                 165                 170

tgg aag att gat ggc agt gaa cga caa aat ggc gtc ctg aac agt tgg        579
Trp Lys Ile Asp Gly Ser Glu Arg Gln Asn Gly Val Leu Asn Ser Trp
        175                 180                 185

act gat cag gac agc aaa gac agc acc tac agc atg agc agc acc ctc        627
Thr Asp Gln Asp Ser Lys Asp Ser Thr Tyr Ser Met Ser Ser Thr Leu
    190                 195                 200
```

```
acg ttg acc aag gac gag tat gaa cga cat aac agc tat acc tgt gag        675
Thr Leu Thr Lys Asp Glu Tyr Glu Arg His Asn Ser Tyr Thr Cys Glu
205                 210                 215                 220

gcc act cac aag aca tca act tca ccc att gtc aag agc ttc aac agg        723
Ala Thr His Lys Thr Ser Thr Ser Pro Ile Val Lys Ser Phe Asn Arg
                225                 230                 235

aat gag tgt tga tagggcgcgc c                                           746
Asn Glu Cys



<210>  164
<211>  239
<212>  PRT
<213>  artificial sequence

<220>
<223>  Synthetic Construct

<400>  164

Met Arg Leu Leu Ala Gln Leu Leu Gly Leu Leu Met Leu Trp Val Pro
1               5                   10                  15


Gly Ser Ser Gly Asp Val Val Met Thr Gln Thr Pro Leu Ser Leu Pro
            20                  25                  30


Val Ser Leu Gly Asp Gln Ala Ser Ile Ser Cys Arg Ser Ser Gln Ser
        35                  40                  45


Ile Val His Ser Asn Gly Asn Thr Tyr Leu Glu Trp Tyr Leu Gln Lys
    50                  55                  60


Pro Gly Gln Ser Pro Lys Leu Leu Ile Tyr Lys Val Ser Asn Arg Phe
65                  70                  75                  80


Ser Gly Val Pro Asp Arg Phe Ser Gly Ser Gly Ser Gly Thr Asp Phe
                85                  90                  95


Thr Leu Lys Ile Ser Arg Val Glu Ala Glu Asp Leu Gly Val Tyr Tyr
            100                 105                 110


Cys Phe Gln Gly Ser His Val Pro Trp Thr Phe Gly Gly Gly Thr Lys
        115                 120                 125


Leu Glu Leu Lys Arg Ala Asp Ala Ala Pro Thr Val Ser Ile Phe Pro
    130                 135                 140


Pro Ser Ser Glu Gln Leu Thr Ser Gly Gly Ala Ser Val Val Cys Phe
145                 150                 155                 160
```

```
Leu Asn Asn Phe Tyr Pro Lys Asp Ile Asn Val Lys Trp Lys Ile Asp
                165                 170                 175

Gly Ser Glu Arg Gln Asn Gly Val Leu Asn Ser Trp Thr Asp Gln Asp
            180                 185                 190

Ser Lys Asp Ser Thr Tyr Ser Met Ser Ser Thr Leu Thr Leu Thr Lys
        195                 200                 205

Asp Glu Tyr Glu Arg His Asn Ser Tyr Thr Cys Glu Ala Thr His Lys
    210                 215                 220

Thr Ser Thr Ser Pro Ile Val Lys Ser Phe Asn Arg Asn Glu Cys
225                 230                 235


<210>  165
<211>  1449
<212>  DNA
<213>  artificial sequence

<220>
<223>  Nucleic Acid Sequence of Heavy Chain of K090-02a002


<220>
<221>  CDS
<222>  (16)..(1440)

<400>  165
aagcttgccg ccacc atg aaa cac ctg tgg ttc ttc ctc ctc ctg gtg gcg       51
                 Met Lys His Leu Trp Phe Phe Leu Leu Leu Val Ala
                 1               5                   10

gcc ccc aga tgg gtc ctg tcc gag gtg aag ctg gtg gag tct ggg gga        99
Ala Pro Arg Trp Val Leu Ser Glu Val Lys Leu Val Glu Ser Gly Gly
        15                  20                  25

ggc tta gtg aag cct gga ggg tcc cgg aaa ctc tcc tgt gca gcc tct       147
Gly Leu Val Lys Pro Gly Gly Ser Arg Lys Leu Ser Cys Ala Ala Ser
    30                  35                  40

gga ttc act ttc agt gac tat gga atg cac tgg gtc cgt cag gct cca       195
Gly Phe Thr Phe Ser Asp Tyr Gly Met His Trp Val Arg Gln Ala Pro
45                  50                  55                  60

gag aag ggg ctg gag tgg gtt gca tac att agt agt ggc agt agt acc       243
Glu Lys Gly Leu Glu Trp Val Ala Tyr Ile Ser Ser Gly Ser Ser Thr
                65                  70                  75

atc tac tat gca gac aca gtg aag ggc cga ttc acc atc tcc aga gac       291
Ile Tyr Tyr Ala Asp Thr Val Lys Gly Arg Phe Thr Ile Ser Arg Asp
            80                  85                  90

aat gcc aag aac acc ctg ttc ctg caa atg acc agt cta agg tct gag       339
Asn Ala Lys Asn Thr Leu Phe Leu Gln Met Thr Ser Leu Arg Ser Glu
        95                  100                 105
```

```
gac aca gcc atg tat tac tgt gca agg cat agt tac tat agt tat gac         387
Asp Thr Ala Met Tyr Tyr Cys Ala Arg His Ser Tyr Tyr Ser Tyr Asp
    110                 115                 120

gtt ggg ggt gac tat gtt atg gac tac tgg ggt caa gga acc tca gtc         435
Val Gly Gly Asp Tyr Val Met Asp Tyr Trp Gly Gln Gly Thr Ser Val
125                 130                 135                 140

acc gtc tcg agc gcc aaa aca aca gcc cca tcg gtc tat cca ctg gcc         483
Thr Val Ser Ser Ala Lys Thr Thr Ala Pro Ser Val Tyr Pro Leu Ala
                145                 150                 155

cct gtg tgt gga gat aca act ggc tcc tcg gtg act cta gga tgc ctg         531
Pro Val Cys Gly Asp Thr Thr Gly Ser Ser Val Thr Leu Gly Cys Leu
            160                 165                 170

gtc aag ggt tat ttc cct gag cca gtg acc ttg acc tgg aac tct gga         579
Val Lys Gly Tyr Phe Pro Glu Pro Val Thr Leu Thr Trp Asn Ser Gly
        175                 180                 185

tcc ctg tcc agt ggt gtg cac acc ttc cca gct gtc ctg cag tct gac         627
Ser Leu Ser Ser Gly Val His Thr Phe Pro Ala Val Leu Gln Ser Asp
    190                 195                 200

ctc tac acc ctc agc agc tca gtg act gta acc tcg agc acc tgg ccc         675
Leu Tyr Thr Leu Ser Ser Ser Val Thr Val Thr Ser Ser Thr Trp Pro
205                 210                 215                 220

agc cag tcc atc acc tgc aat gtg gcc cac ccg gca agc agc acc aag         723
Ser Gln Ser Ile Thr Cys Asn Val Ala His Pro Ala Ser Ser Thr Lys
                225                 230                 235

gtg gac aag aaa att gag ccc cgg gga ccc aca atc aag ccc tgt cct         771
Val Asp Lys Lys Ile Glu Pro Arg Gly Pro Thr Ile Lys Pro Cys Pro
            240                 245                 250

cca tgc aaa tgc cca gca cct aac ctc ttg ggt gga cca tcc gtc ttc         819
Pro Cys Lys Cys Pro Ala Pro Asn Leu Leu Gly Gly Pro Ser Val Phe
        255                 260                 265

atc ttc cct cca aag atc aag gat gta ctc atg atc tcc ctg agc ccc         867
Ile Phe Pro Pro Lys Ile Lys Asp Val Leu Met Ile Ser Leu Ser Pro
    270                 275                 280

ata gtc aca tgt gtg gtg gtg gat gtg agc gag gat gac cca gat gtc         915
Ile Val Thr Cys Val Val Val Asp Val Ser Glu Asp Asp Pro Asp Val
285                 290                 295                 300

cag atc agc tgg ttt gtg aac aac gtg gaa gta cac aca gct cag aca         963
Gln Ile Ser Trp Phe Val Asn Asn Val Glu Val His Thr Ala Gln Thr
                305                 310                 315

caa acc cat aga gag gat tac aac agt act ctc cgg gtg gtc agt gcc        1011
Gln Thr His Arg Glu Asp Tyr Asn Ser Thr Leu Arg Val Val Ser Ala
            320                 325                 330

ctc ccc atc cag cac cag gac tgg atg agt ggc aag gag ttc aaa tgc        1059
Leu Pro Ile Gln His Gln Asp Trp Met Ser Gly Lys Glu Phe Lys Cys
        335                 340                 345

aag gtc aac aac aaa gac ctc cca gcg ccc atc gag aga acc atc tca        1107
Lys Val Asn Asn Lys Asp Leu Pro Ala Pro Ile Glu Arg Thr Ile Ser
    350                 355                 360
```

aaa ccc aaa ggg tca gta aga gct cca cag gta tat gtc ttg cct cca 1155
Lys Pro Lys Gly Ser Val Arg Ala Pro Gln Val Tyr Val Leu Pro Pro
365 370 375 380

cca gaa gaa gag atg act aag aaa cag gtc act ctg acc tgc atg gtc 1203
Pro Glu Glu Glu Met Thr Lys Lys Gln Val Thr Leu Thr Cys Met Val
385 390 395

aca gac ttc atg cct gaa gac att tac gtg gag tgg acc aac aac ggg 1251
Thr Asp Phe Met Pro Glu Asp Ile Tyr Val Glu Trp Thr Asn Asn Gly
400 405 410

aaa aca gag cta aac tac aag aac act gaa cca gtc ctg gac tct gat 1299
Lys Thr Glu Leu Asn Tyr Lys Asn Thr Glu Pro Val Leu Asp Ser Asp
415 420 425

ggt tct tac ttc atg tac agc aag ctg aga gtg gaa aag aag aac tgg 1347
Gly Ser Tyr Phe Met Tyr Ser Lys Leu Arg Val Glu Lys Lys Asn Trp
430 435 440

gtg gaa aga aat agc tac tcc tgt tca gtg gtc cac gag ggt ctg cac 1395
Val Glu Arg Asn Ser Tyr Ser Cys Ser Val Val His Glu Gly Leu His
445 450 455 460

aat cac cac acg act aag agc ttc tcc cgg act ccg ggt aaa tga 1440
Asn His His Thr Thr Lys Ser Phe Ser Arg Thr Pro Gly Lys
465 470

tagtctaga 1449

<210> 166
<211> 474
<212> PRT
<213> artificial sequence

<220>
<223> Synthetic Construct

<400> 166

Met Lys His Leu Trp Phe Phe Leu Leu Leu Val Ala Ala Pro Arg Trp
1 5 10 15

Val Leu Ser Glu Val Lys Leu Val Glu Ser Gly Gly Gly Leu Val Lys
20 25 30

Pro Gly Gly Ser Arg Lys Leu Ser Cys Ala Ala Ser Gly Phe Thr Phe
35 40 45

Ser Asp Tyr Gly Met His Trp Val Arg Gln Ala Pro Glu Lys Gly Leu
50 55 60

Glu Trp Val Ala Tyr Ile Ser Ser Gly Ser Ser Thr Ile Tyr Tyr Ala
65 70 75 80

Asp Thr Val Lys Gly Arg Phe Thr Ile Ser Arg Asp Asn Ala Lys Asn

85 90 95

Thr Leu Phe Leu Gln Met Thr Ser Leu Arg Ser Glu Asp Thr Ala Met
100 105 110

Tyr Tyr Cys Ala Arg His Ser Tyr Tyr Ser Tyr Asp Val Gly Gly Asp
115 120 125

Tyr Val Met Asp Tyr Trp Gly Gln Gly Thr Ser Val Thr Val Ser Ser
130 135 140

Ala Lys Thr Thr Ala Pro Ser Val Tyr Pro Leu Ala Pro Val Cys Gly
145 150 155 160

Asp Thr Thr Gly Ser Ser Val Thr Leu Gly Cys Leu Val Lys Gly Tyr
165 170 175

Phe Pro Glu Pro Val Thr Leu Thr Trp Asn Ser Gly Ser Leu Ser Ser
180 185 190

Gly Val His Thr Phe Pro Ala Val Leu Gln Ser Asp Leu Tyr Thr Leu
195 200 205

Ser Ser Ser Val Thr Val Thr Ser Ser Thr Trp Pro Ser Gln Ser Ile
210 215 220

Thr Cys Asn Val Ala His Pro Ala Ser Ser Thr Lys Val Asp Lys Lys
225 230 235 240

Ile Glu Pro Arg Gly Pro Thr Ile Lys Pro Cys Pro Pro Cys Lys Cys
245 250 255

Pro Ala Pro Asn Leu Leu Gly Gly Pro Ser Val Phe Ile Phe Pro Pro
260 265 270

Lys Ile Lys Asp Val Leu Met Ile Ser Leu Ser Pro Ile Val Thr Cys
275 280 285

Val Val Val Asp Val Ser Glu Asp Asp Pro Asp Val Gln Ile Ser Trp
290 295 300

Phe Val Asn Asn Val Glu Val His Thr Ala Gln Thr Gln Thr His Arg
305 310 315 320

Glu Asp Tyr Asn Ser Thr Leu Arg Val Val Ser Ala Leu Pro Ile Gln
325 330 335

His Gln Asp Trp Met Ser Gly Lys Glu Phe Lys Cys Lys Val Asn Asn
340 345 350

Lys Asp Leu Pro Ala Pro Ile Glu Arg Thr Ile Ser Lys Pro Lys Gly
355 360 365

Ser Val Arg Ala Pro Gln Val Tyr Val Leu Pro Pro Pro Glu Glu Glu
370 375 380

Met Thr Lys Lys Gln Val Thr Leu Thr Cys Met Val Thr Asp Phe Met
385 390 395 400

Pro Glu Asp Ile Tyr Val Glu Trp Thr Asn Asn Gly Lys Thr Glu Leu
405 410 415

Asn Tyr Lys Asn Thr Glu Pro Val Leu Asp Ser Asp Gly Ser Tyr Phe
420 425 430

Met Tyr Ser Lys Leu Arg Val Glu Lys Lys Asn Trp Val Glu Arg Asn
435 440 445

Ser Tyr Ser Cys Ser Val Val His Glu Gly Leu His Asn His His Thr
450 455 460

Thr Lys Ser Phe Ser Arg Thr Pro Gly Lys
465 470

<210> 167
<211> 731
<212> DNA
<213> artificial sequence

<220>
<223> Nucleic Acid Sequence of Light Chain of K090-02a002

<220>
<221> CDS
<222> (16)..(720)

<400> 167
aagcttgccg ccacc atg agg ctc ctt gct cag ctt ctg ggg ctg cta atg 51
Met Arg Leu Leu Ala Gln Leu Leu Gly Leu Leu Met
1 5 10

ctc tgg gtc cct gga tcc agt ggg gac att gtg atg tca cag tct ccc 99
Leu Trp Val Pro Gly Ser Ser Gly Asp Ile Val Met Ser Gln Ser Pro
15 20 25

aaa ttc atg tcc aca tca gta gga gac agg gtc agc atc acc tgc aag 147
Lys Phe Met Ser Thr Ser Val Gly Asp Arg Val Ser Ile Thr Cys Lys
30 35 40

gcc agt cag gat gtg ggt act gct gta gcc tgg tat caa cag aaa cca 195

```
Ala Ser Gln Asp Val Gly Thr Ala Val Ala Trp Tyr Gln Gln Lys Pro
45                  50                  55                  60

ggg caa tct cct aaa cta ctg att tac tgg gca tcc acc cgg cac act         243
Gly Gln Ser Pro Lys Leu Leu Ile Tyr Trp Ala Ser Thr Arg His Thr
                65                  70                  75

gga gtc cct gat cgc ttc aca ggc agt gga tct ggg aca gat ttc act         291
Gly Val Pro Asp Arg Phe Thr Gly Ser Gly Ser Gly Thr Asp Phe Thr
            80                  85                  90

ctc acc att agc aat gtg cag tct gaa gac ttg gca gat tat ttc tgt         339
Leu Thr Ile Ser Asn Val Gln Ser Glu Asp Leu Ala Asp Tyr Phe Cys
        95                  100                 105

cag caa tat agc agc tat cct ctc acg ttc ggt gct ggg acc aag ctg         387
Gln Gln Tyr Ser Ser Tyr Pro Leu Thr Phe Gly Ala Gly Thr Lys Leu
    110                 115                 120

gaa ata aaa cgg gct gat gct gca cca act gta tcc atc ttc cca cca         435
Glu Ile Lys Arg Ala Asp Ala Ala Pro Thr Val Ser Ile Phe Pro Pro
125                 130                 135                 140

tcc agt gag cag tta aca tct gga ggt gcc tca gtc gtg tgc ttc ttg         483
Ser Ser Glu Gln Leu Thr Ser Gly Gly Ala Ser Val Val Cys Phe Leu
                145                 150                 155

aac aac ttc tac ccc aaa gac atc aat gtc aag tgg aag att gat ggc         531
Asn Asn Phe Tyr Pro Lys Asp Ile Asn Val Lys Trp Lys Ile Asp Gly
            160                 165                 170

agt gaa cga caa aat ggc gtc ctg aac agt tgg act gat cag gac agc         579
Ser Glu Arg Gln Asn Gly Val Leu Asn Ser Trp Thr Asp Gln Asp Ser
        175                 180                 185

aaa gac agc acc tac agc atg agc agc acc ctc acg ttg acc aag gac         627
Lys Asp Ser Thr Tyr Ser Met Ser Ser Thr Leu Thr Leu Thr Lys Asp
    190                 195                 200

gag tat gaa cga cat aac agc tat acc tgt gag gcc act cac aag aca         675
Glu Tyr Glu Arg His Asn Ser Tyr Thr Cys Glu Ala Thr His Lys Thr
205                 210                 215                 220

tca act tca ccc att gtc aag agc ttc aac agg aat gag tgt tga             720
Ser Thr Ser Pro Ile Val Lys Ser Phe Asn Arg Asn Glu Cys
                225                 230

tagggcgcgc c                                                            731


<210>  168
<211>  234
<212>  PRT
<213>  artificial sequence

<220>
<223>  Synthetic Construct

<400>  168

Met Arg Leu Leu Ala Gln Leu Leu Gly Leu Leu Met Leu Trp Val Pro
1               5                   10                  15
```

Gly Ser Ser Gly Asp Ile Val Met Ser Gln Ser Pro Lys Phe Met Ser
20 25 30

Thr Ser Val Gly Asp Arg Val Ser Ile Thr Cys Lys Ala Ser Gln Asp
35 40 45

Val Gly Thr Ala Val Ala Trp Tyr Gln Gln Lys Pro Gly Gln Ser Pro
50 55 60

Lys Leu Leu Ile Tyr Trp Ala Ser Thr Arg His Thr Gly Val Pro Asp
65 70 75 80

Arg Phe Thr Gly Ser Gly Ser Gly Thr Asp Phe Thr Leu Thr Ile Ser
85 90 95

Asn Val Gln Ser Glu Asp Leu Ala Asp Tyr Phe Cys Gln Gln Tyr Ser
100 105 110

Ser Tyr Pro Leu Thr Phe Gly Ala Gly Thr Lys Leu Glu Ile Lys Arg
115 120 125

Ala Asp Ala Ala Pro Thr Val Ser Ile Phe Pro Pro Ser Ser Glu Gln
130 135 140

Leu Thr Ser Gly Gly Ala Ser Val Val Cys Phe Leu Asn Asn Phe Tyr
145 150 155 160

Pro Lys Asp Ile Asn Val Lys Trp Lys Ile Asp Gly Ser Glu Arg Gln
165 170 175

Asn Gly Val Leu Asn Ser Trp Thr Asp Gln Asp Ser Lys Asp Ser Thr
180 185 190

Tyr Ser Met Ser Ser Thr Leu Thr Leu Thr Lys Asp Glu Tyr Glu Arg
195 200 205

His Asn Ser Tyr Thr Cys Glu Ala Thr His Lys Thr Ser Thr Ser Pro
210 215 220

Ile Val Lys Ser Phe Asn Arg Asn Glu Cys
225 230

<210> 169
<211> 1428
<212> DNA
<213> artificial sequence

<220>

<223>  Nucleic Acid Sequence of Heavy Chain of K090-02a006

<220>
<221>  CDS
<222>  (16)..(1419)

<400>  169
```
aagcttgccg ccacc atg aaa cac ctg tgg ttc ttc ctc ctc ctg gtg gcg       51
                 Met Lys His Leu Trp Phe Phe Leu Leu Leu Val Ala
                 1               5                   10

gcc ccc aga tgg gtc ctg tcc gag gtt cag ctg cag cag tct gga gct        99
Ala Pro Arg Trp Val Leu Ser Glu Val Gln Leu Gln Gln Ser Gly Ala
        15                  20                  25

gag ctg gtg aag cct ggg gct tca gtg aag ata tcc tgc agg gct tct       147
Glu Leu Val Lys Pro Gly Ala Ser Val Lys Ile Ser Cys Arg Ala Ser
    30                  35                  40

ggt tac tca ttc act ggc tac aac atg aac tgg gtg aag cag agc cat       195
Gly Tyr Ser Phe Thr Gly Tyr Asn Met Asn Trp Val Lys Gln Ser His
45                  50                  55                  60

gga aag agc ctt gag tgg att gga aat att aat cct tac tat ggt act       243
Gly Lys Ser Leu Glu Trp Ile Gly Asn Ile Asn Pro Tyr Tyr Gly Thr
                65                  70                  75

act aac tac aat cag aag ttc aag ggc aag gcc aca ttg act gta gac       291
Thr Asn Tyr Asn Gln Lys Phe Lys Gly Lys Ala Thr Leu Thr Val Asp
            80                  85                  90

aaa tct tcc agc aca gcc tac atg cag ctc aac agc ctg aca tct gag       339
Lys Ser Ser Ser Thr Ala Tyr Met Gln Leu Asn Ser Leu Thr Ser Glu
        95                  100                 105

gac tct gca gtc tat tac tgt gca agt gat ggt tac tac ggg gct atg       387
Asp Ser Ala Val Tyr Tyr Cys Ala Ser Asp Gly Tyr Tyr Gly Ala Met
    110                 115                 120

gac tac tgg ggt caa gga acc tca gtc acc gtc tcg agc gcc aaa aca       435
Asp Tyr Trp Gly Gln Gly Thr Ser Val Thr Val Ser Ser Ala Lys Thr
125                 130                 135                 140

aca gcc cca tcg gtc tat cca ctg gcc cct gtg tgt gga gat aca act       483
Thr Ala Pro Ser Val Tyr Pro Leu Ala Pro Val Cys Gly Asp Thr Thr
                145                 150                 155

ggc tcc tcg gtg act cta gga tgc ctg gtc aag ggt tat ttc cct gag       531
Gly Ser Ser Val Thr Leu Gly Cys Leu Val Lys Gly Tyr Phe Pro Glu
            160                 165                 170

cca gtg acc ttg acc tgg aac tct gga tcc ctg tcc agt ggt gtg cac       579
Pro Val Thr Leu Thr Trp Asn Ser Gly Ser Leu Ser Ser Gly Val His
        175                 180                 185

acc ttc cca gct gtc ctg cag tct gac ctc tac acc ctc agc agc tca       627
Thr Phe Pro Ala Val Leu Gln Ser Asp Leu Tyr Thr Leu Ser Ser Ser
    190                 195                 200

gtg act gta acc tcg agc acc tgg ccc agc cag tcc atc acc tgc aat       675
Val Thr Val Thr Ser Ser Thr Trp Pro Ser Gln Ser Ile Thr Cys Asn
205                 210                 215                 220
```

```
gtg gcc cac ccg gca agc agc acc aag gtg gac aag aaa att gag ccc       723
Val Ala His Pro Ala Ser Ser Thr Lys Val Asp Lys Lys Ile Glu Pro
                225                 230                 235

cgg gga ccc aca atc aag ccc tgt cct cca tgc aaa tgc cca gca cct       771
Arg Gly Pro Thr Ile Lys Pro Cys Pro Pro Cys Lys Cys Pro Ala Pro
            240                 245                 250

aac ctc ttg ggt gga cca tcc gtc ttc atc ttc cct cca aag atc aag       819
Asn Leu Leu Gly Gly Pro Ser Val Phe Ile Phe Pro Pro Lys Ile Lys
        255                 260                 265

gat gta ctc atg atc tcc ctg agc ccc ata gtc aca tgt gtg gtg gtg       867
Asp Val Leu Met Ile Ser Leu Ser Pro Ile Val Thr Cys Val Val Val
    270                 275                 280

gat gtg agc gag gat gac cca gat gtc cag atc agc tgg ttt gtg aac       915
Asp Val Ser Glu Asp Asp Pro Asp Val Gln Ile Ser Trp Phe Val Asn
285                 290                 295                 300

aac gtg gaa gta cac aca gct cag aca caa acc cat aga gag gat tac       963
Asn Val Glu Val His Thr Ala Gln Thr Gln Thr His Arg Glu Asp Tyr
                305                 310                 315

aac agt act ctc cgg gtg gtc agt gcc ctc ccc atc cag cac cag gac      1011
Asn Ser Thr Leu Arg Val Val Ser Ala Leu Pro Ile Gln His Gln Asp
            320                 325                 330

tgg atg agt ggc aag gag ttc aaa tgc aag gtc aac aac aaa gac ctc      1059
Trp Met Ser Gly Lys Glu Phe Lys Cys Lys Val Asn Asn Lys Asp Leu
        335                 340                 345

cca gcg ccc atc gag aga acc atc tca aaa ccc aaa ggg tca gta aga      1107
Pro Ala Pro Ile Glu Arg Thr Ile Ser Lys Pro Lys Gly Ser Val Arg
    350                 355                 360

gct cca cag gta tat gtc ttg cct cca cca gaa gaa gag atg act aag      1155
Ala Pro Gln Val Tyr Val Leu Pro Pro Pro Glu Glu Glu Met Thr Lys
365                 370                 375                 380

aaa cag gtc act ctg acc tgc atg gtc aca gac ttc atg cct gaa gac      1203
Lys Gln Val Thr Leu Thr Cys Met Val Thr Asp Phe Met Pro Glu Asp
                385                 390                 395

att tac gtg gag tgg acc aac aac ggg aaa aca gag cta aac tac aag      1251
Ile Tyr Val Glu Trp Thr Asn Asn Gly Lys Thr Glu Leu Asn Tyr Lys
            400                 405                 410

aac act gaa cca gtc ctg gac tct gat ggt tct tac ttc atg tac agc      1299
Asn Thr Glu Pro Val Leu Asp Ser Asp Gly Ser Tyr Phe Met Tyr Ser
        415                 420                 425

aag ctg aga gtg gaa aag aag aac tgg gtg gaa aga aat agc tac tcc      1347
Lys Leu Arg Val Glu Lys Lys Asn Trp Val Glu Arg Asn Ser Tyr Ser
    430                 435                 440

tgt tca gtg gtc cac gag ggt ctg cac aat cac cac acg act aag agc      1395
Cys Ser Val Val His Glu Gly Leu His Asn His His Thr Thr Lys Ser
445                 450                 455                 460

ttc tcc cgg act ccg ggt aaa tga tagtctaga                            1428
Phe Ser Arg Thr Pro Gly Lys
```

465

<210> 170
<211> 467
<212> PRT
<213> artificial sequence

<220>
<223> Synthetic Construct

<400> 170

Met Lys His Leu Trp Phe Phe Leu Leu Leu Val Ala Ala Pro Arg Trp
1               5                   10                  15

Val Leu Ser Glu Val Gln Leu Gln Gln Ser Gly Ala Glu Leu Val Lys
            20                  25                  30

Pro Gly Ala Ser Val Lys Ile Ser Cys Arg Ala Ser Gly Tyr Ser Phe
        35                  40                  45

Thr Gly Tyr Asn Met Asn Trp Val Lys Gln Ser His Gly Lys Ser Leu
    50                  55                  60

Glu Trp Ile Gly Asn Ile Asn Pro Tyr Tyr Gly Thr Thr Asn Tyr Asn
65                  70                  75                  80

Gln Lys Phe Lys Gly Lys Ala Thr Leu Thr Val Asp Lys Ser Ser Ser
                85                  90                  95

Thr Ala Tyr Met Gln Leu Asn Ser Leu Thr Ser Glu Asp Ser Ala Val
            100                 105                 110

Tyr Tyr Cys Ala Ser Asp Gly Tyr Tyr Gly Ala Met Asp Tyr Trp Gly
        115                 120                 125

Gln Gly Thr Ser Val Thr Val Ser Ser Ala Lys Thr Thr Ala Pro Ser
    130                 135                 140

Val Tyr Pro Leu Ala Pro Val Cys Gly Asp Thr Thr Gly Ser Ser Val
145                 150                 155                 160

Thr Leu Gly Cys Leu Val Lys Gly Tyr Phe Pro Glu Pro Val Thr Leu
                165                 170                 175

Thr Trp Asn Ser Gly Ser Leu Ser Ser Gly Val His Thr Phe Pro Ala
            180                 185                 190

Val Leu Gln Ser Asp Leu Tyr Thr Leu Ser Ser Ser Val Thr Val Thr
        195                 200                 205

Ser Ser Thr Trp Pro Ser Gln Ser Ile Thr Cys Asn Val Ala His Pro
    210                 215                 220

Ala Ser Ser Thr Lys Val Asp Lys Lys Ile Glu Pro Arg Gly Pro Thr
225                 230                 235                 240

Ile Lys Pro Cys Pro Pro Cys Lys Cys Pro Ala Pro Asn Leu Leu Gly
                245                 250                 255

Gly Pro Ser Val Phe Ile Phe Pro Pro Lys Ile Lys Asp Val Leu Met
            260                 265                 270

Ile Ser Leu Ser Pro Ile Val Thr Cys Val Val Val Asp Val Ser Glu
        275                 280                 285

Asp Asp Pro Asp Val Gln Ile Ser Trp Phe Val Asn Asn Val Glu Val
    290                 295                 300

His Thr Ala Gln Thr Gln Thr His Arg Glu Asp Tyr Asn Ser Thr Leu
305                 310                 315                 320

Arg Val Val Ser Ala Leu Pro Ile Gln His Gln Asp Trp Met Ser Gly
                325                 330                 335

Lys Glu Phe Lys Cys Lys Val Asn Asn Lys Asp Leu Pro Ala Pro Ile
            340                 345                 350

Glu Arg Thr Ile Ser Lys Pro Lys Gly Ser Val Arg Ala Pro Gln Val
        355                 360                 365

Tyr Val Leu Pro Pro Pro Glu Glu Glu Met Thr Lys Lys Gln Val Thr
    370                 375                 380

Leu Thr Cys Met Val Thr Asp Phe Met Pro Glu Asp Ile Tyr Val Glu
385                 390                 395                 400

Trp Thr Asn Asn Gly Lys Thr Glu Leu Asn Tyr Lys Asn Thr Glu Pro
                405                 410                 415

Val Leu Asp Ser Asp Gly Ser Tyr Phe Met Tyr Ser Lys Leu Arg Val
            420                 425                 430

Glu Lys Lys Asn Trp Val Glu Arg Asn Ser Tyr Ser Cys Ser Val Val
        435                 440                 445

His Glu Gly Leu His Asn His His Thr Thr Lys Ser Phe Ser Arg Thr

450 455 460

Pro Gly Lys
465

<210> 171
<211> 731
<212> DNA
<213> artificial sequence

<220>
<223> Nucleic Acid Sequence of Light Chain of K090-02a006

<220>
<221> CDS
<222> (16)..(720)

<400> 171
```
aagcttgccg ccacc atg agg ctc ctt gct cag ctt ctg ggg ctg cta atg        51
                 Met Arg Leu Leu Ala Gln Leu Leu Gly Leu Leu Met
                 1               5                   10

ctc tgg gtc cct gga tcc agt ggg gac atc aag atg acc cag tct cca         99
Leu Trp Val Pro Gly Ser Ser Gly Asp Ile Lys Met Thr Gln Ser Pro
        15                  20                  25

aaa ttc atg tcc aca tca gta gga gac agg gtc agc gtc acc tgc aag        147
Lys Phe Met Ser Thr Ser Val Gly Asp Arg Val Ser Val Thr Cys Lys
    30                  35                  40

gcc agt cag aat gtg ggt act tat gta gcc tgg tat caa cag aaa cca        195
Ala Ser Gln Asn Val Gly Thr Tyr Val Ala Trp Tyr Gln Gln Lys Pro
45                  50                  55                  60

ggg caa tct cct aaa gca ctg att tac tcg gca tcc tac cgg tac agt        243
Gly Gln Ser Pro Lys Ala Leu Ile Tyr Ser Ala Ser Tyr Arg Tyr Ser
                65                  70                  75

gga gtc cct gat cgc ttc aca ggc act gga tct ggg aca gat ttc act        291
Gly Val Pro Asp Arg Phe Thr Gly Thr Gly Ser Gly Thr Asp Phe Thr
            80                  85                  90

ctc acc atc agc aat gtg cag tct gaa gac ttg gca gag tat ttc tgt        339
Leu Thr Ile Ser Asn Val Gln Ser Glu Asp Leu Ala Glu Tyr Phe Cys
        95                  100                 105

cag caa tat tac aac tat ccg ctc acg ttc ggt gct ggg acc aag ctg        387
Gln Gln Tyr Tyr Asn Tyr Pro Leu Thr Phe Gly Ala Gly Thr Lys Leu
    110                 115                 120

gag ctg aaa cgg gct gat gct gca cca act gta tcc atc ttc cca cca        435
Glu Leu Lys Arg Ala Asp Ala Ala Pro Thr Val Ser Ile Phe Pro Pro
125                 130                 135                 140

tcc agt gag cag tta aca tct gga ggt gcc tca gtc gtg tgc ttc ttg        483
Ser Ser Glu Gln Leu Thr Ser Gly Gly Ala Ser Val Val Cys Phe Leu
                145                 150                 155

aac aac ttc tac ccc aaa gac atc aat gtc aag tgg aag att gat ggc        531
Asn Asn Phe Tyr Pro Lys Asp Ile Asn Val Lys Trp Lys Ile Asp Gly
```

```
            160                 165                 170

agt gaa cga caa aat ggc gtc ctg aac agt tgg act gat cag gac agc        579
Ser Glu Arg Gln Asn Gly Val Leu Asn Ser Trp Thr Asp Gln Asp Ser
        175                 180                 185

aaa gac agc acc tac agc atg agc agc acc ctc acg ttg acc aag gac        627
Lys Asp Ser Thr Tyr Ser Met Ser Ser Thr Leu Thr Leu Thr Lys Asp
    190                 195                 200

gag tat gaa cga cat aac agc tat acc tgt gag gcc act cac aag aca        675
Glu Tyr Glu Arg His Asn Ser Tyr Thr Cys Glu Ala Thr His Lys Thr
205                 210                 215                 220

tca act tca ccc att gtc aag agc ttc aac agg aat gag tgt tga            720
Ser Thr Ser Pro Ile Val Lys Ser Phe Asn Arg Asn Glu Cys
                225                 230

tagggcgcgc c                                                           731


<210>  172
<211>  234
<212>  PRT
<213>  artificial sequence

<220>
<223>  Synthetic Construct

<400>  172

Met Arg Leu Leu Ala Gln Leu Leu Gly Leu Leu Met Leu Trp Val Pro
1               5                   10                  15


Gly Ser Ser Gly Asp Ile Lys Met Thr Gln Ser Pro Lys Phe Met Ser
            20                  25                  30


Thr Ser Val Gly Asp Arg Val Ser Val Thr Cys Lys Ala Ser Gln Asn
        35                  40                  45


Val Gly Thr Tyr Val Ala Trp Tyr Gln Gln Lys Pro Gly Gln Ser Pro
    50                  55                  60


Lys Ala Leu Ile Tyr Ser Ala Ser Tyr Arg Tyr Ser Gly Val Pro Asp
65                  70                  75                  80


Arg Phe Thr Gly Thr Gly Ser Gly Thr Asp Phe Thr Leu Thr Ile Ser
                85                  90                  95


Asn Val Gln Ser Glu Asp Leu Ala Glu Tyr Phe Cys Gln Gln Tyr Tyr
            100                 105                 110


Asn Tyr Pro Leu Thr Phe Gly Ala Gly Thr Lys Leu Glu Leu Lys Arg
        115                 120                 125
```

```
Ala Asp Ala Ala Pro Thr Val Ser Ile Phe Pro Pro Ser Ser Glu Gln
    130                 135                 140

Leu Thr Ser Gly Gly Ala Ser Val Val Cys Phe Leu Asn Asn Phe Tyr
145                 150                 155                 160

Pro Lys Asp Ile Asn Val Lys Trp Lys Ile Asp Gly Ser Glu Arg Gln
                165                 170                 175

Asn Gly Val Leu Asn Ser Trp Thr Asp Gln Asp Ser Lys Asp Ser Thr
            180                 185                 190

Tyr Ser Met Ser Ser Thr Leu Thr Leu Thr Lys Asp Glu Tyr Glu Arg
        195                 200                 205

His Asn Ser Tyr Thr Cys Glu Ala Thr His Lys Thr Ser Thr Ser Pro
    210                 215                 220

Ile Val Lys Ser Phe Asn Arg Asn Glu Cys
225                 230


<210>  173
<211>  1449
<212>  DNA
<213>  artificial sequence

<220>
<223>  Nucleic Acid Sequence of Heavy Chain of K090-02a010


<220>
<221>  CDS
<222>  (16)..(1440)

<400>  173
aagcttgccg ccacc atg aaa cac ctg tgg ttc ttc ctc ctc ctg gtg gcg       51
                 Met Lys His Leu Trp Phe Phe Leu Leu Leu Val Ala
                 1               5                   10

gcc ccc aga tgg gtc ctg tcc gag gtg aag ctg gtg gaa tct ggg gga       99
Ala Pro Arg Trp Val Leu Ser Glu Val Lys Leu Val Glu Ser Gly Gly
        15                  20                  25

ggc tta gtg aag cct gga ggg tcc cgg aaa ctc tcc tgt gca gcc tct      147
Gly Leu Val Lys Pro Gly Gly Ser Arg Lys Leu Ser Cys Ala Ala Ser
    30                  35                  40

gga ttc act ttc agt gac tat gga atg cac tgg gtc cgt cag gct cca      195
Gly Phe Thr Phe Ser Asp Tyr Gly Met His Trp Val Arg Gln Ala Pro
45                  50                  55                  60

gag aag ggg ctg gag tgg gtt gca tac att aat agt ggc agt agt acc      243
Glu Lys Gly Leu Glu Trp Val Ala Tyr Ile Asn Ser Gly Ser Ser Thr
                65                  70                  75

atc tac tat gca gac aca gtg aag ggc cga ttc acc atc tcc aga gac      291
```

```
Ile Tyr Tyr Ala Asp Thr Val Lys Gly Arg Phe Thr Ile Ser Arg Asp
            80                  85                  90

aat gcc aag aac acc ctg ttc ctg caa atg acc agt cta agg tct ggg        339
Asn Ala Lys Asn Thr Leu Phe Leu Gln Met Thr Ser Leu Arg Ser Gly
        95                  100                 105

gac aca gcc atg tat tac tgt gca agg cat agt tac tat agt tat gac        387
Asp Thr Ala Met Tyr Tyr Cys Ala Arg His Ser Tyr Tyr Ser Tyr Asp
    110                 115                 120

gtt ggg ggt gac tat gtt atg gac tac tgg ggt caa gga acc tca gtc        435
Val Gly Gly Asp Tyr Val Met Asp Tyr Trp Gly Gln Gly Thr Ser Val
125                 130                 135                 140

acc gtc tcg agc gcc aaa aca aca gcc cca tcg gtc tat cca ctg gcc        483
Thr Val Ser Ser Ala Lys Thr Thr Ala Pro Ser Val Tyr Pro Leu Ala
                145                 150                 155

cct gtg tgt gga gat aca act ggc tcc tcg gtg act cta gga tgc ctg        531
Pro Val Cys Gly Asp Thr Thr Gly Ser Ser Val Thr Leu Gly Cys Leu
            160                 165                 170

gtc aag ggt tat ttc cct gag cca gtg acc ttg acc tgg aac tct gga        579
Val Lys Gly Tyr Phe Pro Glu Pro Val Thr Leu Thr Trp Asn Ser Gly
        175                 180                 185

tcc ctg tcc agt ggt gtg cac acc ttc cca gct gtc ctg cag tct gac        627
Ser Leu Ser Ser Gly Val His Thr Phe Pro Ala Val Leu Gln Ser Asp
    190                 195                 200

ctc tac acc ctc agc agc tca gtg act gta acc tcg agc acc tgg ccc        675
Leu Tyr Thr Leu Ser Ser Ser Val Thr Val Thr Ser Ser Thr Trp Pro
205                 210                 215                 220

agc cag tcc atc acc tgc aat gtg gcc cac ccg gca agc agc acc aag        723
Ser Gln Ser Ile Thr Cys Asn Val Ala His Pro Ala Ser Ser Thr Lys
                225                 230                 235

gtg gac aag aaa att gag ccc cgg gga ccc aca atc aag ccc tgt cct        771
Val Asp Lys Lys Ile Glu Pro Arg Gly Pro Thr Ile Lys Pro Cys Pro
            240                 245                 250

cca tgc aaa tgc cca gca cct aac ctc ttg ggt gga cca tcc gtc ttc        819
Pro Cys Lys Cys Pro Ala Pro Asn Leu Leu Gly Gly Pro Ser Val Phe
        255                 260                 265

atc ttc cct cca aag atc aag gat gta ctc atg atc tcc ctg agc ccc        867
Ile Phe Pro Pro Lys Ile Lys Asp Val Leu Met Ile Ser Leu Ser Pro
    270                 275                 280

ata gtc aca tgt gtg gtg gtg gat gtg agc gag gat gac cca gat gtc        915
Ile Val Thr Cys Val Val Val Asp Val Ser Glu Asp Asp Pro Asp Val
285                 290                 295                 300

cag atc agc tgg ttt gtg aac aac gtg gaa gta cac aca gct cag aca        963
Gln Ile Ser Trp Phe Val Asn Asn Val Glu Val His Thr Ala Gln Thr
                305                 310                 315

caa acc cat aga gag gat tac aac agt act ctc cgg gtg gtc agt gcc       1011
Gln Thr His Arg Glu Asp Tyr Asn Ser Thr Leu Arg Val Val Ser Ala
            320                 325                 330
```

```
ctc ccc atc cag cac cag gac tgg atg agt ggc aag gag ttc aaa tgc        1059
Leu Pro Ile Gln His Gln Asp Trp Met Ser Gly Lys Glu Phe Lys Cys
        335                 340                 345

aag gtc aac aac aaa gac ctc cca gcg ccc atc gag aga acc atc tca        1107
Lys Val Asn Asn Lys Asp Leu Pro Ala Pro Ile Glu Arg Thr Ile Ser
    350                 355                 360

aaa ccc aaa ggg tca gta aga gct cca cag gta tat gtc ttg cct cca        1155
Lys Pro Lys Gly Ser Val Arg Ala Pro Gln Val Tyr Val Leu Pro Pro
365                 370                 375                 380

cca gaa gaa gag atg act aag aaa cag gtc act ctg acc tgc atg gtc        1203
Pro Glu Glu Glu Met Thr Lys Lys Gln Val Thr Leu Thr Cys Met Val
                385                 390                 395

aca gac ttc atg cct gaa gac att tac gtg gag tgg acc aac aac ggg        1251
Thr Asp Phe Met Pro Glu Asp Ile Tyr Val Glu Trp Thr Asn Asn Gly
            400                 405                 410

aaa aca gag cta aac tac aag aac act gaa cca gtc ctg gac tct gat        1299
Lys Thr Glu Leu Asn Tyr Lys Asn Thr Glu Pro Val Leu Asp Ser Asp
        415                 420                 425

ggt tct tac ttc atg tac agc aag ctg aga gtg gaa aag aag aac tgg        1347
Gly Ser Tyr Phe Met Tyr Ser Lys Leu Arg Val Glu Lys Lys Asn Trp
    430                 435                 440

gtg gaa aga aat agc tac tcc tgt tca gtg gtc cac gag ggt ctg cac        1395
Val Glu Arg Asn Ser Tyr Ser Cys Ser Val Val His Glu Gly Leu His
445                 450                 455                 460

aat cac cac acg act aag agc ttc tcc cgg act ccg ggt aaa tga            1440
Asn His His Thr Thr Lys Ser Phe Ser Arg Thr Pro Gly Lys
                465                 470

tagtctaga                                                              1449


<210>  174
<211>  474
<212>  PRT
<213>  artificial sequence

<220>
<223>  Synthetic Construct

<400>  174

Met Lys His Leu Trp Phe Phe Leu Leu Leu Val Ala Ala Pro Arg Trp
1               5                   10                  15


Val Leu Ser Glu Val Lys Leu Val Glu Ser Gly Gly Gly Leu Val Lys
            20                  25                  30


Pro Gly Gly Ser Arg Lys Leu Ser Cys Ala Ala Ser Gly Phe Thr Phe
        35                  40                  45


Ser Asp Tyr Gly Met His Trp Val Arg Gln Ala Pro Glu Lys Gly Leu
    50                  55                  60
```

Glu Trp Val Ala Tyr Ile Asn Ser Gly Ser Ser Thr Ile Tyr Tyr Ala
65 70 75 80

Asp Thr Val Lys Gly Arg Phe Thr Ile Ser Arg Asp Asn Ala Lys Asn
85 90 95

Thr Leu Phe Leu Gln Met Thr Ser Leu Arg Ser Gly Asp Thr Ala Met
100 105 110

Tyr Tyr Cys Ala Arg His Ser Tyr Tyr Ser Tyr Asp Val Gly Gly Asp
115 120 125

Tyr Val Met Asp Tyr Trp Gly Gln Gly Thr Ser Val Thr Val Ser Ser
130 135 140

Ala Lys Thr Thr Ala Pro Ser Val Tyr Pro Leu Ala Pro Val Cys Gly
145 150 155 160

Asp Thr Thr Gly Ser Ser Val Thr Leu Gly Cys Leu Val Lys Gly Tyr
165 170 175

Phe Pro Glu Pro Val Thr Leu Thr Trp Asn Ser Gly Ser Leu Ser Ser
180 185 190

Gly Val His Thr Phe Pro Ala Val Leu Gln Ser Asp Leu Tyr Thr Leu
195 200 205

Ser Ser Ser Val Thr Val Thr Ser Ser Thr Trp Pro Ser Gln Ser Ile
210 215 220

Thr Cys Asn Val Ala His Pro Ala Ser Ser Thr Lys Val Asp Lys Lys
225 230 235 240

Ile Glu Pro Arg Gly Pro Thr Ile Lys Pro Cys Pro Pro Cys Lys Cys
245 250 255

Pro Ala Pro Asn Leu Leu Gly Gly Pro Ser Val Phe Ile Phe Pro Pro
260 265 270

Lys Ile Lys Asp Val Leu Met Ile Ser Leu Ser Pro Ile Val Thr Cys
275 280 285

Val Val Val Asp Val Ser Glu Asp Asp Pro Asp Val Gln Ile Ser Trp
290 295 300

Phe Val Asn Asn Val Glu Val His Thr Ala Gln Thr Gln Thr His Arg

305 310 315 320

Glu Asp Tyr Asn Ser Thr Leu Arg Val Val Ser Ala Leu Pro Ile Gln
325 330 335

His Gln Asp Trp Met Ser Gly Lys Glu Phe Lys Cys Lys Val Asn Asn
340 345 350

Lys Asp Leu Pro Ala Pro Ile Glu Arg Thr Ile Ser Lys Pro Lys Gly
355 360 365

Ser Val Arg Ala Pro Gln Val Tyr Val Leu Pro Pro Pro Glu Glu Glu
370 375 380

Met Thr Lys Lys Gln Val Thr Leu Thr Cys Met Val Thr Asp Phe Met
385 390 395 400

Pro Glu Asp Ile Tyr Val Glu Trp Thr Asn Asn Gly Lys Thr Glu Leu
405 410 415

Asn Tyr Lys Asn Thr Glu Pro Val Leu Asp Ser Asp Gly Ser Tyr Phe
420 425 430

Met Tyr Ser Lys Leu Arg Val Glu Lys Lys Asn Trp Val Glu Arg Asn
435 440 445

Ser Tyr Ser Cys Ser Val Val His Glu Gly Leu His Asn His His Thr
450 455 460

Thr Lys Ser Phe Ser Arg Thr Pro Gly Lys
465 470

<210> 175
<211> 798
<212> DNA
<213> artificial sequence

<220>
<223> Nucleic Acid Sequence of Light Chain of K090-02a010

<220>
<221> CDS
<222> (16)..(720)

<400> 175
aagcttgccg ccacc atg agg ctc ctt gct cag ctt ctg ggg ctg cta atg 51
Met Arg Leu Leu Ala Gln Leu Leu Gly Leu Leu Met
1 5 10

ctc tgg gtc cct gga tcc agt ggg gac att gtg ctg aca cag tct ccc 99
Leu Trp Val Pro Gly Ser Ser Gly Asp Ile Val Leu Thr Gln Ser Pro

```
        15                  20                  25

aaa ttc atg tcc aca tca gta gga gac agg gtc agc atc acc tgc aag        147
Lys Phe Met Ser Thr Ser Val Gly Asp Arg Val Ser Ile Thr Cys Lys
    30                  35                  40

gcc agt cag gat gtg ggt act gct gta gcc tgg tat caa cag aaa cca        195
Ala Ser Gln Asp Val Gly Thr Ala Val Ala Trp Tyr Gln Gln Lys Pro
45                  50                  55                  60

ggg caa tct cct aaa cta ctg att tac tgg gca tcc acc cgg cac act        243
Gly Gln Ser Pro Lys Leu Leu Ile Tyr Trp Ala Ser Thr Arg His Thr
                65                  70                  75

gga gtc cct gat cgc ttc aca ggc agt gga tct ggg aca gat ttc act        291
Gly Val Pro Asp Arg Phe Thr Gly Ser Gly Ser Gly Thr Asp Phe Thr
            80                  85                  90

ctc acc att agc aat gtg cag tct gaa gac ttg gca gat tat ttc tgt        339
Leu Thr Ile Ser Asn Val Gln Ser Glu Asp Leu Ala Asp Tyr Phe Cys
        95                  100                 105

cag caa tat agc agc tat ccg ctc acg ttc ggt gct ggg acc aag ctg        387
Gln Gln Tyr Ser Ser Tyr Pro Leu Thr Phe Gly Ala Gly Thr Lys Leu
    110                 115                 120

gaa ata aaa cgg gct gat gct gca cca act gta tcc atc ttc cca cca        435
Glu Ile Lys Arg Ala Asp Ala Ala Pro Thr Val Ser Ile Phe Pro Pro
125                 130                 135                 140

tcc agt gag cag tta aca tct gga ggt gcc tca gtc gtg tgc ttc ttg        483
Ser Ser Glu Gln Leu Thr Ser Gly Gly Ala Ser Val Val Cys Phe Leu
                145                 150                 155

aac aac ttc tac ccc aaa gac atc aat gtc aag tgg aag att gat ggc        531
Asn Asn Phe Tyr Pro Lys Asp Ile Asn Val Lys Trp Lys Ile Asp Gly
            160                 165                 170

agt gaa cga caa aat ggc gtc ctg aac agt tgg act gat cag gac agc        579
Ser Glu Arg Gln Asn Gly Val Leu Asn Ser Trp Thr Asp Gln Asp Ser
        175                 180                 185

aaa gac agc acc tac agc atg agc agc acc ctc acg ttg acc aag gac        627
Lys Asp Ser Thr Tyr Ser Met Ser Ser Thr Leu Thr Leu Thr Lys Asp
    190                 195                 200

gag tat gaa cga cat aac agc tat acc tgt gag gcc act cac aag aca        675
Glu Tyr Glu Arg His Asn Ser Tyr Thr Cys Glu Ala Thr His Lys Thr
205                 210                 215                 220

tca act tca ccc att gtc aag agc ttc aac agg aat gag tgt tga            720
Ser Thr Ser Pro Ile Val Lys Ser Phe Asn Arg Asn Glu Cys
                225                 230

tagggcgcgc ctctagatga tcattccctt tagtgagggt taatgcttcg agcagacatg      780

ataagataca ttgatgag                                                    798
```

<210>  176
<211>  234
<212>  PRT
<213>  artificial sequence

<220>
<223> Synthetic Construct

<400> 176

Met Arg Leu Leu Ala Gln Leu Leu Gly Leu Leu Met Leu Trp Val Pro
1 5 10 15

Gly Ser Ser Gly Asp Ile Val Leu Thr Gln Ser Pro Lys Phe Met Ser
20 25 30

Thr Ser Val Gly Asp Arg Val Ser Ile Thr Cys Lys Ala Ser Gln Asp
35 40 45

Val Gly Thr Ala Val Ala Trp Tyr Gln Gln Lys Pro Gly Gln Ser Pro
50 55 60

Lys Leu Leu Ile Tyr Trp Ala Ser Thr Arg His Thr Gly Val Pro Asp
65 70 75 80

Arg Phe Thr Gly Ser Gly Ser Gly Thr Asp Phe Thr Leu Thr Ile Ser
85 90 95

Asn Val Gln Ser Glu Asp Leu Ala Asp Tyr Phe Cys Gln Gln Tyr Ser
100 105 110

Ser Tyr Pro Leu Thr Phe Gly Ala Gly Thr Lys Leu Glu Ile Lys Arg
115 120 125

Ala Asp Ala Ala Pro Thr Val Ser Ile Phe Pro Pro Ser Ser Glu Gln
130 135 140

Leu Thr Ser Gly Gly Ala Ser Val Val Cys Phe Leu Asn Asn Phe Tyr
145 150 155 160

Pro Lys Asp Ile Asn Val Lys Trp Lys Ile Asp Gly Ser Glu Arg Gln
165 170 175

Asn Gly Val Leu Asn Ser Trp Thr Asp Gln Asp Ser Lys Asp Ser Thr
180 185 190

Tyr Ser Met Ser Ser Thr Leu Thr Leu Thr Lys Asp Glu Tyr Glu Arg
195 200 205

His Asn Ser Tyr Thr Cys Glu Ala Thr His Lys Thr Ser Thr Ser Pro
210 215 220

Ile Val Lys Ser Phe Asn Arg Asn Glu Cys

```
225                 230


<210>  177
<211>  1428
<212>  DNA
<213>  artificial sequence

<220>
<223>  Nucleic Acid Sequence of Heavy Chain of K090-02a014


<220>
<221>  CDS
<222>  (16)..(1419)

<400>  177
aagcttgccg ccacc atg aaa cac ctg tgg ttc ttc ctc ctc ctg gtg gcg        51
                 Met Lys His Leu Trp Phe Phe Leu Leu Leu Val Ala
                 1               5                   10

gcc ccc aga tgg gtc ctg tcc gag gtt cag ctt cag cag tct gga cct         99
Ala Pro Arg Trp Val Leu Ser Glu Val Gln Leu Gln Gln Ser Gly Pro
        15                  20                  25

gaa ctg gtg aag cct ggg gct tca gtg aag ata tcc tgc aag gct tct        147
Glu Leu Val Lys Pro Gly Ala Ser Val Lys Ile Ser Cys Lys Ala Ser
    30                  35                  40

ggt tac tca ttc act ggc tac tac ata cac tgg gtg aag caa agt cct        195
Gly Tyr Ser Phe Thr Gly Tyr Tyr Ile His Trp Val Lys Gln Ser Pro
45                  50                  55                  60

gaa aag agc ctt gag tgg att gga gag att aat cct agc act ggt gat        243
Glu Lys Ser Leu Glu Trp Ile Gly Glu Ile Asn Pro Ser Thr Gly Asp
                65                  70                  75

att acc tac aac cag aaa ttc aag gcc aag gcc aca ttg act gta gac        291
Ile Thr Tyr Asn Gln Lys Phe Lys Ala Lys Ala Thr Leu Thr Val Asp
            80                  85                  90

aaa tcc tcc agc aca gcc tac atg cag ctc aag agc ctg aca tct gat        339
Lys Ser Ser Ser Thr Ala Tyr Met Gln Leu Lys Ser Leu Thr Ser Asp
        95                  100                 105

gac tct gca gtc tat tac tgt gca aga gag cgg aat tac gac cgg ttt        387
Asp Ser Ala Val Tyr Tyr Cys Ala Arg Glu Arg Asn Tyr Asp Arg Phe
    110                 115                 120

gct tac tgg ggc caa ggg act ctg gtc act gtc tcg agc gcc aaa aca        435
Ala Tyr Trp Gly Gln Gly Thr Leu Val Thr Val Ser Ser Ala Lys Thr
125                 130                 135                 140

aca gcc cca tcg gtc tat cca ctg gcc cct gtg tgt gga gat aca act        483
Thr Ala Pro Ser Val Tyr Pro Leu Ala Pro Val Cys Gly Asp Thr Thr
                145                 150                 155

ggc tcc tcg gtg act cta gga tgc ctg gtc aag ggt tat ttc cct gag        531
Gly Ser Ser Val Thr Leu Gly Cys Leu Val Lys Gly Tyr Phe Pro Glu
            160                 165                 170

cca gtg acc ttg acc tgg aac tct gga tcc ctg tcc agt ggt gtg cac        579
Pro Val Thr Leu Thr Trp Asn Ser Gly Ser Leu Ser Ser Gly Val His
```

```
        175                 180                 185

acc ttc cca gct gtc ctg cag tct gac ctc tac acc ctc agc agc tca         627
Thr Phe Pro Ala Val Leu Gln Ser Asp Leu Tyr Thr Leu Ser Ser Ser
    190                 195                 200

gtg act gta acc tcg agc acc tgg ccc agc cag tcc atc acc tgc aat         675
Val Thr Val Thr Ser Ser Thr Trp Pro Ser Gln Ser Ile Thr Cys Asn
205                 210                 215                 220

gtg gcc cac ccg gca agc agc acc aag gtg gac aag aaa att gag ccc         723
Val Ala His Pro Ala Ser Ser Thr Lys Val Asp Lys Lys Ile Glu Pro
                225                 230                 235

cgg gga ccc aca atc aag ccc tgt cct cca tgc aaa tgc cca gca cct         771
Arg Gly Pro Thr Ile Lys Pro Cys Pro Pro Cys Lys Cys Pro Ala Pro
            240                 245                 250

aac ctc ttg ggt gga cca tcc gtc ttc atc ttc cct cca aag atc aag         819
Asn Leu Leu Gly Gly Pro Ser Val Phe Ile Phe Pro Pro Lys Ile Lys
        255                 260                 265

gat gta ctc atg atc tcc ctg agc ccc ata gtc aca tgt gtg gtg gtg         867
Asp Val Leu Met Ile Ser Leu Ser Pro Ile Val Thr Cys Val Val Val
    270                 275                 280

gat gtg agc gag gat gac cca gat gtc cag atc agc tgg ttt gtg aac         915
Asp Val Ser Glu Asp Asp Pro Asp Val Gln Ile Ser Trp Phe Val Asn
285                 290                 295                 300

aac gtg gaa gta cac aca gct cag aca caa acc cat aga gag gat tac         963
Asn Val Glu Val His Thr Ala Gln Thr Gln Thr His Arg Glu Asp Tyr
                305                 310                 315

aac agt act ctc cgg gtg gtc agt gcc ctc ccc atc cag cac cag gac        1011
Asn Ser Thr Leu Arg Val Val Ser Ala Leu Pro Ile Gln His Gln Asp
            320                 325                 330

tgg atg agt ggc aag gag ttc aaa tgc aag gtc aac aac aaa gac ctc        1059
Trp Met Ser Gly Lys Glu Phe Lys Cys Lys Val Asn Asn Lys Asp Leu
        335                 340                 345

cca gcg ccc atc gag aga acc atc tca aaa ccc aaa ggg tca gta aga        1107
Pro Ala Pro Ile Glu Arg Thr Ile Ser Lys Pro Lys Gly Ser Val Arg
    350                 355                 360

gct cca cag gta tat gtc ttg cct cca cca gaa gaa gag atg act aag        1155
Ala Pro Gln Val Tyr Val Leu Pro Pro Pro Glu Glu Glu Met Thr Lys
365                 370                 375                 380

aaa cag gtc act ctg acc tgc atg gtc aca gac ttc atg cct gaa gac        1203
Lys Gln Val Thr Leu Thr Cys Met Val Thr Asp Phe Met Pro Glu Asp
                385                 390                 395

att tac gtg gag tgg acc aac aac ggg aaa aca gag cta aac tac aag        1251
Ile Tyr Val Glu Trp Thr Asn Asn Gly Lys Thr Glu Leu Asn Tyr Lys
            400                 405                 410

aac act gaa cca gtc ctg gac tct gat ggt tct tac ttc atg tac agc        1299
Asn Thr Glu Pro Val Leu Asp Ser Asp Gly Ser Tyr Phe Met Tyr Ser
        415                 420                 425

aag ctg aga gtg gaa aag aag aac tgg gtg gaa aga aat agc tac tcc        1347
```

```
Lys Leu Arg Val Glu Lys Lys Asn Trp Val Glu Arg Asn Ser Tyr Ser
    430                 435                 440

tgt tca gtg gtc cac gag ggt ctg cac aat cac cac acg act aag agc      1395
Cys Ser Val Val His Glu Gly Leu His Asn His His Thr Thr Lys Ser
445                 450                 455                 460

ttc tcc cgg act ccg ggt aaa tga tagtctaga                            1428
Phe Ser Arg Thr Pro Gly Lys
                465
```

<210> 178
<211> 467
<212> PRT
<213> artificial sequence

<220>
<223> Synthetic Construct

<400> 178

```
Met Lys His Leu Trp Phe Phe Leu Leu Leu Val Ala Ala Pro Arg Trp
1               5                   10                  15

Val Leu Ser Glu Val Gln Leu Gln Gln Ser Gly Pro Glu Leu Val Lys
            20                  25                  30

Pro Gly Ala Ser Val Lys Ile Ser Cys Lys Ala Ser Gly Tyr Ser Phe
        35                  40                  45

Thr Gly Tyr Tyr Ile His Trp Val Lys Gln Ser Pro Glu Lys Ser Leu
    50                  55                  60

Glu Trp Ile Gly Glu Ile Asn Pro Ser Thr Gly Asp Ile Thr Tyr Asn
65                  70                  75                  80

Gln Lys Phe Lys Ala Lys Ala Thr Leu Thr Val Asp Lys Ser Ser Ser
                85                  90                  95

Thr Ala Tyr Met Gln Leu Lys Ser Leu Thr Ser Asp Asp Ser Ala Val
            100                 105                 110

Tyr Tyr Cys Ala Arg Glu Arg Asn Tyr Asp Arg Phe Ala Tyr Trp Gly
        115                 120                 125

Gln Gly Thr Leu Val Thr Val Ser Ser Ala Lys Thr Thr Ala Pro Ser
    130                 135                 140

Val Tyr Pro Leu Ala Pro Val Cys Gly Asp Thr Thr Gly Ser Ser Val
145                 150                 155                 160

Thr Leu Gly Cys Leu Val Lys Gly Tyr Phe Pro Glu Pro Val Thr Leu
```

165 170 175

Thr Trp Asn Ser Gly Ser Leu Ser Ser Gly Val His Thr Phe Pro Ala
180 185 190

Val Leu Gln Ser Asp Leu Tyr Thr Leu Ser Ser Ser Val Thr Val Thr
195 200 205

Ser Ser Thr Trp Pro Ser Gln Ser Ile Thr Cys Asn Val Ala His Pro
210 215 220

Ala Ser Ser Thr Lys Val Asp Lys Lys Ile Glu Pro Arg Gly Pro Thr
225 230 235 240

Ile Lys Pro Cys Pro Pro Cys Lys Cys Pro Ala Pro Asn Leu Leu Gly
245 250 255

Gly Pro Ser Val Phe Ile Phe Pro Pro Lys Ile Lys Asp Val Leu Met
260 265 270

Ile Ser Leu Ser Pro Ile Val Thr Cys Val Val Val Asp Val Ser Glu
275 280 285

Asp Asp Pro Asp Val Gln Ile Ser Trp Phe Val Asn Asn Val Glu Val
290 295 300

His Thr Ala Gln Thr Gln Thr His Arg Glu Asp Tyr Asn Ser Thr Leu
305 310 315 320

Arg Val Val Ser Ala Leu Pro Ile Gln His Gln Asp Trp Met Ser Gly
325 330 335

Lys Glu Phe Lys Cys Lys Val Asn Asn Lys Asp Leu Pro Ala Pro Ile
340 345 350

Glu Arg Thr Ile Ser Lys Pro Lys Gly Ser Val Arg Ala Pro Gln Val
355 360 365

Tyr Val Leu Pro Pro Pro Glu Glu Glu Met Thr Lys Lys Gln Val Thr
370 375 380

Leu Thr Cys Met Val Thr Asp Phe Met Pro Glu Asp Ile Tyr Val Glu
385 390 395 400

Trp Thr Asn Asn Gly Lys Thr Glu Leu Asn Tyr Lys Asn Thr Glu Pro
405 410 415

```
Val Leu Asp Ser Asp Gly Ser Tyr Phe Met Tyr Ser Lys Leu Arg Val
            420                 425                 430


Glu Lys Lys Asn Trp Val Glu Arg Asn Ser Tyr Ser Cys Ser Val Val
        435                 440                 445


His Glu Gly Leu His Asn His His Thr Thr Lys Ser Phe Ser Arg Thr
    450                 455                 460


Pro Gly Lys
465


<210>  179
<211>  798
<212>  DNA
<213>  artificial sequence

<220>
<223>  Nucleic Acid Sequence of Light Chain of K090-02a014


<220>
<221>  CDS
<222>  (16)..(723)

<400>  179
aagcttgccg ccacc atg agg ctc ctt gct cag ctt ctg ggg ctg cta atg        51
                 Met Arg Leu Leu Ala Gln Leu Leu Gly Leu Leu Met
                 1               5                   10

ctc tgg gtc cct gga tcc agt ggg agt att gtg atg acc cag act cca         99
Leu Trp Val Pro Gly Ser Ser Gly Ser Ile Val Met Thr Gln Thr Pro
        15                  20                  25

gca ctc atg gct gca tct cca ggg gag aag gtc acc atc acc tgc agt        147
Ala Leu Met Ala Ala Ser Pro Gly Glu Lys Val Thr Ile Thr Cys Ser
    30                  35                  40

gtc agc tca agt ata agt tcc agc aac ttg cac tgg tac cag cag aag        195
Val Ser Ser Ser Ile Ser Ser Ser Asn Leu His Trp Tyr Gln Gln Lys
45                  50                  55                  60

tca gaa acc tcc ccc aaa ccc tgg att tat ggc aca tcc aac ctg gct        243
Ser Glu Thr Ser Pro Lys Pro Trp Ile Tyr Gly Thr Ser Asn Leu Ala
                65                  70                  75

tct gga gtc cct gtt cgc ttc agt ggc agt gga tct ggg acc tct tat        291
Ser Gly Val Pro Val Arg Phe Ser Gly Ser Gly Ser Gly Thr Ser Tyr
            80                  85                  90

tct ctc aca atc agc agc atg gag gct gaa gat gct gcc act tat tac        339
Ser Leu Thr Ile Ser Ser Met Glu Ala Glu Asp Ala Ala Thr Tyr Tyr
        95                  100                 105

tgt caa cag tgg agt agt tac ccg ctc acg ttc ggt gct ggg acc aag        387
Cys Gln Gln Trp Ser Ser Tyr Pro Leu Thr Phe Gly Ala Gly Thr Lys
    110                 115                 120

ctg gaa ata aaa cgg gct gat gct gca cca act gta tcc atc ttc cca        435
```

```
Leu Glu Ile Lys Arg Ala Asp Ala Ala Pro Thr Val Ser Ile Phe Pro
125                 130                 135                 140

cca tcc agt gag cag tta aca tct gga ggt gcc tca gtc gtg tgc ttc      483
Pro Ser Ser Glu Gln Leu Thr Ser Gly Gly Ala Ser Val Val Cys Phe
                145                 150                 155

ttg aac aac ttc tac ccc aaa gac atc aat gtc aag tgg aag att gat      531
Leu Asn Asn Phe Tyr Pro Lys Asp Ile Asn Val Lys Trp Lys Ile Asp
            160                 165                 170

ggc agt gaa cga caa aat ggc gtc ctg aac agt tgg act gat cag gac      579
Gly Ser Glu Arg Gln Asn Gly Val Leu Asn Ser Trp Thr Asp Gln Asp
        175                 180                 185

agc aaa gac agc acc tac agc atg agc agc acc ctc acg ttg acc aag      627
Ser Lys Asp Ser Thr Tyr Ser Met Ser Ser Thr Leu Thr Leu Thr Lys
    190                 195                 200

gac gag tat gaa cga cat aac agc tat acc tgt gag gcc act cac aag      675
Asp Glu Tyr Glu Arg His Asn Ser Tyr Thr Cys Glu Ala Thr His Lys
205                 210                 215                 220

aca tca act tca ccc att gtc aag agc ttc aac agg aat gag tgt tga      723
Thr Ser Thr Ser Pro Ile Val Lys Ser Phe Asn Arg Asn Glu Cys
                225                 230                 235

tagggcgcgc ctctagatga tcattccctt tagtgagggt taatgcttcg agcagacatg     783

ataagataca ttgat                                                      798


<210>  180
<211>  235
<212>  PRT
<213>  artificial sequence

<220>
<223>  Synthetic Construct

<400>  180

Met Arg Leu Leu Ala Gln Leu Leu Gly Leu Leu Met Leu Trp Val Pro
1               5                   10                  15


Gly Ser Ser Gly Ser Ile Val Met Thr Gln Thr Pro Ala Leu Met Ala
            20                  25                  30


Ala Ser Pro Gly Glu Lys Val Thr Ile Thr Cys Ser Val Ser Ser Ser
        35                  40                  45


Ile Ser Ser Ser Asn Leu His Trp Tyr Gln Gln Lys Ser Glu Thr Ser
    50                  55                  60


Pro Lys Pro Trp Ile Tyr Gly Thr Ser Asn Leu Ala Ser Gly Val Pro
65                  70                  75                  80


Val Arg Phe Ser Gly Ser Gly Ser Gly Thr Ser Tyr Ser Leu Thr Ile
```

```
                85                  90                  95

Ser Ser Met Glu Ala Glu Asp Ala Ala Thr Tyr Tyr Cys Gln Gln Trp
            100                 105                 110

Ser Ser Tyr Pro Leu Thr Phe Gly Ala Gly Thr Lys Leu Glu Ile Lys
        115                 120                 125

Arg Ala Asp Ala Ala Pro Thr Val Ser Ile Phe Pro Pro Ser Ser Glu
    130                 135                 140

Gln Leu Thr Ser Gly Gly Ala Ser Val Val Cys Phe Leu Asn Asn Phe
145                 150                 155                 160

Tyr Pro Lys Asp Ile Asn Val Lys Trp Lys Ile Asp Gly Ser Glu Arg
                165                 170                 175

Gln Asn Gly Val Leu Asn Ser Trp Thr Asp Gln Asp Ser Lys Asp Ser
            180                 185                 190

Thr Tyr Ser Met Ser Ser Thr Leu Thr Leu Thr Lys Asp Glu Tyr Glu
        195                 200                 205

Arg His Asn Ser Tyr Thr Cys Glu Ala Thr His Lys Thr Ser Thr Ser
    210                 215                 220

Pro Ile Val Lys Ser Phe Asn Arg Asn Glu Cys
225                 230                 235


<210>  181
<211>  1431
<212>  DNA
<213>  artificial sequence

<220>
<223>  Nucleic Acid Sequence of Heavy Chain of K090-02a022


<220>
<221>  CDS
<222>  (16)..(1422)

<400>  181
aagcttgccg ccacc atg aaa cac ctg tgg ttc ttc ctc ctc ctg gtg gcg        51
                 Met Lys His Leu Trp Phe Phe Leu Leu Leu Val Ala
                 1               5                   10

gcc ccc aga tgg gtc ctg tcc cag gtc caa ctg cag cag cct gga cct         99
Ala Pro Arg Trp Val Leu Ser Gln Val Gln Leu Gln Gln Pro Gly Pro
        15                  20                  25

gag ctg gtg aag cct ggg gct tca gtg aag atg tcc tgt aag gct tct        147
Glu Leu Val Lys Pro Gly Ala Ser Val Lys Met Ser Cys Lys Ala Ser
```

```
    30                  35                  40

gga tac aca ttc act gac tac tac atg aac tgg gtg aag cag agt cat      195
Gly Tyr Thr Phe Thr Asp Tyr Tyr Met Asn Trp Val Lys Gln Ser His
45                  50                  55                  60

gga aag agc ctt gag tgg att gga cgt gtt aat cct agc aat ggt ggt      243
Gly Lys Ser Leu Glu Trp Ile Gly Arg Val Asn Pro Ser Asn Gly Gly
                65                  70                  75

act agc tac aac cag aag ttc aag ggc aag gcc aca ttg aca gta gac      291
Thr Ser Tyr Asn Gln Lys Phe Lys Gly Lys Ala Thr Leu Thr Val Asp
            80                  85                  90

aaa tcc ctc agc aca gcc tac atg cag ctc aac agc ctg aca tct gag      339
Lys Ser Leu Ser Thr Ala Tyr Met Gln Leu Asn Ser Leu Thr Ser Glu
        95                  100                 105

gac tct gcg gtc tat tac tgt gca aga agt gga gag aat tac tat gct      387
Asp Ser Ala Val Tyr Tyr Cys Ala Arg Ser Gly Glu Asn Tyr Tyr Ala
    110                 115                 120

atg gac tac tgg ggt caa gga acc tca gtc acc gtc tcg agc gcc aaa      435
Met Asp Tyr Trp Gly Gln Gly Thr Ser Val Thr Val Ser Ser Ala Lys
125                 130                 135                 140

aca aca gcc cca tcg gtc tat cca ctg gcc cct gtg tgt gga gat aca      483
Thr Thr Ala Pro Ser Val Tyr Pro Leu Ala Pro Val Cys Gly Asp Thr
                145                 150                 155

act ggc tcc tcg gtg act cta gga tgc ctg gtc aag ggt tat ttc cct      531
Thr Gly Ser Ser Val Thr Leu Gly Cys Leu Val Lys Gly Tyr Phe Pro
            160                 165                 170

gag cca gtg acc ttg acc tgg aac tct gga tcc ctg tcc agt ggt gtg      579
Glu Pro Val Thr Leu Thr Trp Asn Ser Gly Ser Leu Ser Ser Gly Val
        175                 180                 185

cac acc ttc cca gct gtc ctg cag tct gac ctc tac acc ctc agc agc      627
His Thr Phe Pro Ala Val Leu Gln Ser Asp Leu Tyr Thr Leu Ser Ser
    190                 195                 200

tca gtg act gta acc tcg agc acc tgg ccc agc cag tcc atc acc tgc      675
Ser Val Thr Val Thr Ser Ser Thr Trp Pro Ser Gln Ser Ile Thr Cys
205                 210                 215                 220

aat gtg gcc cac ccg gca agc agc acc aag gtg gac aag aaa att gag      723
Asn Val Ala His Pro Ala Ser Ser Thr Lys Val Asp Lys Lys Ile Glu
                225                 230                 235

ccc cgg gga ccc aca atc aag ccc tgt cct cca tgc aaa tgc cca gca      771
Pro Arg Gly Pro Thr Ile Lys Pro Cys Pro Pro Cys Lys Cys Pro Ala
            240                 245                 250

cct aac ctc ttg ggt gga cca tcc gtc ttc atc ttc cct cca aag atc      819
Pro Asn Leu Leu Gly Gly Pro Ser Val Phe Ile Phe Pro Pro Lys Ile
        255                 260                 265

aag gat gta ctc atg atc tcc ctg agc ccc ata gtc aca tgt gtg gtg      867
Lys Asp Val Leu Met Ile Ser Leu Ser Pro Ile Val Thr Cys Val Val
    270                 275                 280

gtg gat gtg agc gag gat gac cca gat gtc cag atc agc tgg ttt gtg      915
```

```
Val Asp Val Ser Glu Asp Asp Pro Asp Val Gln Ile Ser Trp Phe Val
285                 290                 295                 300

aac aac gtg gaa gta cac aca gct cag aca caa acc cat aga gag gat        963
Asn Asn Val Glu Val His Thr Ala Gln Thr Gln Thr His Arg Glu Asp
                305                 310                 315

tac aac agt act ctc cgg gtg gtc agt gcc ctc ccc atc cag cac cag       1011
Tyr Asn Ser Thr Leu Arg Val Val Ser Ala Leu Pro Ile Gln His Gln
            320                 325                 330

gac tgg atg agt ggc aag gag ttc aaa tgc aag gtc aac aac aaa gac       1059
Asp Trp Met Ser Gly Lys Glu Phe Lys Cys Lys Val Asn Asn Lys Asp
        335                 340                 345

ctc cca gcg ccc atc gag aga acc atc tca aaa ccc aaa ggg tca gta       1107
Leu Pro Ala Pro Ile Glu Arg Thr Ile Ser Lys Pro Lys Gly Ser Val
    350                 355                 360

aga gct cca cag gta tat gtc ttg cct cca cca gaa gaa gag atg act       1155
Arg Ala Pro Gln Val Tyr Val Leu Pro Pro Pro Glu Glu Glu Met Thr
365                 370                 375                 380

aag aaa cag gtc act ctg acc tgc atg gtc aca gac ttc atg cct gaa       1203
Lys Lys Gln Val Thr Leu Thr Cys Met Val Thr Asp Phe Met Pro Glu
                385                 390                 395

gac att tac gtg gag tgg acc aac aac ggg aaa aca gag cta aac tac       1251
Asp Ile Tyr Val Glu Trp Thr Asn Asn Gly Lys Thr Glu Leu Asn Tyr
            400                 405                 410

aag aac act gaa cca gtc ctg gac tct gat ggt tct tac ttc atg tac       1299
Lys Asn Thr Glu Pro Val Leu Asp Ser Asp Gly Ser Tyr Phe Met Tyr
        415                 420                 425

agc aag ctg aga gtg gaa aag aag aac tgg gtg gaa aga aat agc tac       1347
Ser Lys Leu Arg Val Glu Lys Lys Asn Trp Val Glu Arg Asn Ser Tyr
    430                 435                 440

tcc tgt tca gtg gtc cac gag ggt ctg cac aat cac cac acg act aag       1395
Ser Cys Ser Val Val His Glu Gly Leu His Asn His His Thr Thr Lys
445                 450                 455                 460

agc ttc tcc cgg act ccg ggt aaa tga tagtctaga                         1431
Ser Phe Ser Arg Thr Pro Gly Lys
                465


<210>  182
<211>  468
<212>  PRT
<213>  artificial sequence

<220>
<223>  Synthetic Construct

<400>  182

Met Lys His Leu Trp Phe Phe Leu Leu Leu Val Ala Ala Pro Arg Trp
1               5                   10                  15


Val Leu Ser Gln Val Gln Leu Gln Gln Pro Gly Pro Glu Leu Val Lys
```

```
            20                  25                  30

Pro Gly Ala Ser Val Lys Met Ser Cys Lys Ala Ser Gly Tyr Thr Phe
        35                  40                  45

Thr Asp Tyr Tyr Met Asn Trp Val Lys Gln Ser His Gly Lys Ser Leu
    50                  55                  60

Glu Trp Ile Gly Arg Val Asn Pro Ser Asn Gly Gly Thr Ser Tyr Asn
65                  70                  75                  80

Gln Lys Phe Lys Gly Lys Ala Thr Leu Thr Val Asp Lys Ser Leu Ser
                85                  90                  95

Thr Ala Tyr Met Gln Leu Asn Ser Leu Thr Ser Glu Asp Ser Ala Val
            100                 105                 110

Tyr Tyr Cys Ala Arg Ser Gly Glu Asn Tyr Tyr Ala Met Asp Tyr Trp
        115                 120                 125

Gly Gln Gly Thr Ser Val Thr Val Ser Ser Ala Lys Thr Thr Ala Pro
    130                 135                 140

Ser Val Tyr Pro Leu Ala Pro Val Cys Gly Asp Thr Thr Gly Ser Ser
145                 150                 155                 160

Val Thr Leu Gly Cys Leu Val Lys Gly Tyr Phe Pro Glu Pro Val Thr
                165                 170                 175

Leu Thr Trp Asn Ser Gly Ser Leu Ser Ser Gly Val His Thr Phe Pro
            180                 185                 190

Ala Val Leu Gln Ser Asp Leu Tyr Thr Leu Ser Ser Ser Val Thr Val
        195                 200                 205

Thr Ser Ser Thr Trp Pro Ser Gln Ser Ile Thr Cys Asn Val Ala His
    210                 215                 220

Pro Ala Ser Ser Thr Lys Val Asp Lys Lys Ile Glu Pro Arg Gly Pro
225                 230                 235                 240

Thr Ile Lys Pro Cys Pro Pro Cys Lys Cys Pro Ala Pro Asn Leu Leu
                245                 250                 255

Gly Gly Pro Ser Val Phe Ile Phe Pro Pro Lys Ile Lys Asp Val Leu
            260                 265                 270
```

Met Ile Ser Leu Ser Pro Ile Val Thr Cys Val Val Val Asp Val Ser
275 280 285

Glu Asp Asp Pro Asp Val Gln Ile Ser Trp Phe Val Asn Asn Val Glu
290 295 300

Val His Thr Ala Gln Thr Gln Thr His Arg Glu Asp Tyr Asn Ser Thr
305 310 315 320

Leu Arg Val Val Ser Ala Leu Pro Ile Gln His Gln Asp Trp Met Ser
325 330 335

Gly Lys Glu Phe Lys Cys Lys Val Asn Asn Lys Asp Leu Pro Ala Pro
340 345 350

Ile Glu Arg Thr Ile Ser Lys Pro Lys Gly Ser Val Arg Ala Pro Gln
355 360 365

Val Tyr Val Leu Pro Pro Pro Glu Glu Glu Met Thr Lys Lys Gln Val
370 375 380

Thr Leu Thr Cys Met Val Thr Asp Phe Met Pro Glu Asp Ile Tyr Val
385 390 395 400

Glu Trp Thr Asn Asn Gly Lys Thr Glu Leu Asn Tyr Lys Asn Thr Glu
405 410 415

Pro Val Leu Asp Ser Asp Gly Ser Tyr Phe Met Tyr Ser Lys Leu Arg
420 425 430

Val Glu Lys Lys Asn Trp Val Glu Arg Asn Ser Tyr Ser Cys Ser Val
435 440 445

Val His Glu Gly Leu His Asn His His Thr Thr Lys Ser Phe Ser Arg
450 455 460

Thr Pro Gly Lys
465

<210> 183
<211> 795
<212> DNA
<213> artificial sequence

<220>
<223> Nucleic Acid Sequence of Light Chain of K090-02a022

<220>
<221> CDS

```
<222>  (16)..(720)

<400>  183
aagcttgccg ccacc atg agg ctc ctt gct cag ctt ctg ggg ctg cta atg        51
                 Met Arg Leu Leu Ala Gln Leu Leu Gly Leu Leu Met
                 1               5                   10

ctc tgg gtc cct gga tcc agt ggg gac atc cag atg acc cag tct cca        99
Leu Trp Val Pro Gly Ser Ser Gly Asp Ile Gln Met Thr Gln Ser Pro
        15                  20                  25

gcc tcc cta tct gca tct gtg gga gaa act gtc acc atc aca tgt cga       147
Ala Ser Leu Ser Ala Ser Val Gly Glu Thr Val Thr Ile Thr Cys Arg
    30                  35                  40

gca agt gag aat att tac agt tat tta gca tgg tat cag cag aaa cag       195
Ala Ser Glu Asn Ile Tyr Ser Tyr Leu Ala Trp Tyr Gln Gln Lys Gln
45                  50                  55                  60

gga aaa tct cct cag ctc ctg gtc tat aat gga aaa acc tta gca gaa       243
Gly Lys Ser Pro Gln Leu Leu Val Tyr Asn Gly Lys Thr Leu Ala Glu
                65                  70                  75

ggt gtg tca tca agg ttc agt ggc agt gga tca ggc aca cag ttt tct       291
Gly Val Ser Ser Arg Phe Ser Gly Ser Gly Ser Gly Thr Gln Phe Ser
            80                  85                  90

ctg aag att aac agc ctg cag cct gaa gat ttt ggg agt tat tac tgt       339
Leu Lys Ile Asn Ser Leu Gln Pro Glu Asp Phe Gly Ser Tyr Tyr Cys
        95                  100                 105

caa aat cat tat ggt gtt ccg tgg acg ttc gga gga ggc acc aag ctg       387
Gln Asn His Tyr Gly Val Pro Trp Thr Phe Gly Gly Gly Thr Lys Leu
    110                 115                 120

gaa ata aaa cgg gct gat gct gca cca act gta tcc atc ttc cca cca       435
Glu Ile Lys Arg Ala Asp Ala Ala Pro Thr Val Ser Ile Phe Pro Pro
125                 130                 135                 140

tcc agt gag cag tta aca tct gga ggt gcc tca gtc gtg tgc ttc ttg       483
Ser Ser Glu Gln Leu Thr Ser Gly Gly Ala Ser Val Val Cys Phe Leu
                145                 150                 155

aac aac ttc tac ccc aaa gac atc aat gtc aag tgg aag att gat ggc       531
Asn Asn Phe Tyr Pro Lys Asp Ile Asn Val Lys Trp Lys Ile Asp Gly
            160                 165                 170

agt gaa cga caa aat ggc gtc ctg aac agt tgg act gat cag gac agc       579
Ser Glu Arg Gln Asn Gly Val Leu Asn Ser Trp Thr Asp Gln Asp Ser
        175                 180                 185

aaa gac agc acc tac agc atg agc agc acc ctc acg ttg acc aag gac       627
Lys Asp Ser Thr Tyr Ser Met Ser Ser Thr Leu Thr Leu Thr Lys Asp
    190                 195                 200

gag tat gaa cga cat aac agc tat acc tgt gag gcc act cac aag aca       675
Glu Tyr Glu Arg His Asn Ser Tyr Thr Cys Glu Ala Thr His Lys Thr
205                 210                 215                 220

tca act tca ccc att gtc aag agc ttc aac agg aat gag tgt tga           720
Ser Thr Ser Pro Ile Val Lys Ser Phe Asn Arg Asn Glu Cys
                225                 230
```

tagggcgcgc ctctagatga tcattccctt tagtgagggt taatgcttcg agcagacatg 780

ataagataca ttgat 795

<210> 184
<211> 234
<212> PRT
<213> artificial sequence

<220>
<223> Synthetic Construct

<400> 184

Met Arg Leu Leu Ala Gln Leu Leu Gly Leu Leu Met Leu Trp Val Pro
1               5                   10                  15

Gly Ser Ser Gly Asp Ile Gln Met Thr Gln Ser Pro Ala Ser Leu Ser
            20                  25                  30

Ala Ser Val Gly Glu Thr Val Thr Ile Thr Cys Arg Ala Ser Glu Asn
        35                  40                  45

Ile Tyr Ser Tyr Leu Ala Trp Tyr Gln Gln Lys Gln Gly Lys Ser Pro
    50                  55                  60

Gln Leu Leu Val Tyr Asn Gly Lys Thr Leu Ala Glu Gly Val Ser Ser
65                  70                  75                  80

Arg Phe Ser Gly Ser Gly Ser Gly Thr Gln Phe Ser Leu Lys Ile Asn
                85                  90                  95

Ser Leu Gln Pro Glu Asp Phe Gly Ser Tyr Tyr Cys Gln Asn His Tyr
            100                 105                 110

Gly Val Pro Trp Thr Phe Gly Gly Gly Thr Lys Leu Glu Ile Lys Arg
        115                 120                 125

Ala Asp Ala Ala Pro Thr Val Ser Ile Phe Pro Pro Ser Ser Glu Gln
    130                 135                 140

Leu Thr Ser Gly Gly Ala Ser Val Val Cys Phe Leu Asn Asn Phe Tyr
145                 150                 155                 160

Pro Lys Asp Ile Asn Val Lys Trp Lys Ile Asp Gly Ser Glu Arg Gln
                165                 170                 175

Asn Gly Val Leu Asn Ser Trp Thr Asp Gln Asp Ser Lys Asp Ser Thr
            180                 185                 190

```
Tyr Ser Met Ser Ser Thr Leu Thr Leu Thr Lys Asp Glu Tyr Glu Arg
        195                 200                 205

His Asn Ser Tyr Thr Cys Glu Ala Thr His Lys Thr Ser Thr Ser Pro
    210                 215                 220

Ile Val Lys Ser Phe Asn Arg Asn Glu Cys
225                 230

<210>  185
<211>  1428
<212>  DNA
<213>  artificial sequence

<220>
<223>  Nucleic Acid Sequence of Heavy Chain of K090-02a034


<220>
<221>  CDS
<222>  (16)..(1419)

<400>  185
aagcttgccg ccacc atg aaa cac ctg tgg ttc ttc ctc ctc ctg gtg gcg       51
                 Met Lys His Leu Trp Phe Phe Leu Leu Leu Val Ala
                 1               5                   10

gcc ccc aga tgg gtc ctg tcc gag gtt cag ctt cag cag tct gga cct        99
Ala Pro Arg Trp Val Leu Ser Glu Val Gln Leu Gln Gln Ser Gly Pro
        15                  20                  25

gag ctg gtg aag cct ggg gct tca gtg aag ata tcc tgc aag gct tct       147
Glu Leu Val Lys Pro Gly Ala Ser Val Lys Ile Ser Cys Lys Ala Ser
    30                  35                  40

ggt tac tca ttc act ggc tac tac atg cac tgg gtg aag caa agt cct       195
Gly Tyr Ser Phe Thr Gly Tyr Tyr Met His Trp Val Lys Gln Ser Pro
45                  50                  55                  60

gaa aag agc ctt gag tgg att gga gag att aat cct agc act ggt gat       243
Glu Lys Ser Leu Glu Trp Ile Gly Glu Ile Asn Pro Ser Thr Gly Asp
                65                  70                  75

act acc tac aac cag aag ttc aag gcc aag gcc aca ttg act gta gac       291
Thr Thr Tyr Asn Gln Lys Phe Lys Ala Lys Ala Thr Leu Thr Val Asp
            80                  85                  90

aaa tcc tcc agc aca gcc tac ata cag ctc aag agc ctg aca tct gag       339
Lys Ser Ser Ser Thr Ala Tyr Ile Gln Leu Lys Ser Leu Thr Ser Glu
        95                  100                 105

gac tct gca gtc tat tac tgt gca aga gag cgg aat tac gac cgg ttt       387
Asp Ser Ala Val Tyr Tyr Cys Ala Arg Glu Arg Asn Tyr Asp Arg Phe
    110                 115                 120

gct tac tgg ggc caa ggg act ctg gtc act gtc tcg agc gcc aaa aca       435
Ala Tyr Trp Gly Gln Gly Thr Leu Val Thr Val Ser Ser Ala Lys Thr
125                 130                 135                 140

aca gcc cca tcg gtc tat cca ctg gcc cct gtg tgt gga gat aca act       483
```

```
Thr Ala Pro Ser Val Tyr Pro Leu Ala Pro Val Cys Gly Asp Thr Thr
                145                 150                 155

ggc tcc tcg gtg act cta gga tgc ctg gtc aag ggt tat ttc cct gag         531
Gly Ser Ser Val Thr Leu Gly Cys Leu Val Lys Gly Tyr Phe Pro Glu
            160                 165                 170

cca gtg acc ttg acc tgg aac tct gga tcc ctg tcc agt ggt gtg cac         579
Pro Val Thr Leu Thr Trp Asn Ser Gly Ser Leu Ser Ser Gly Val His
        175                 180                 185

acc ttc cca gct gtc ctg cag tct gac ctc tac acc ctc agc agc tca         627
Thr Phe Pro Ala Val Leu Gln Ser Asp Leu Tyr Thr Leu Ser Ser Ser
    190                 195                 200

gtg act gta acc tcg agc acc tgg ccc agc cag tcc atc acc tgc aat         675
Val Thr Val Thr Ser Ser Thr Trp Pro Ser Gln Ser Ile Thr Cys Asn
205                 210                 215                 220

gtg gcc cac ccg gca agc agc acc aag gtg gac aag aaa att gag ccc         723
Val Ala His Pro Ala Ser Ser Thr Lys Val Asp Lys Lys Ile Glu Pro
                225                 230                 235

cgg gga ccc aca atc aag ccc tgt cct cca tgc aaa tgc cca gca cct         771
Arg Gly Pro Thr Ile Lys Pro Cys Pro Pro Cys Lys Cys Pro Ala Pro
            240                 245                 250

aac ctc ttg ggt gga cca tcc gtc ttc atc ttc cct cca aag atc aag         819
Asn Leu Leu Gly Gly Pro Ser Val Phe Ile Phe Pro Pro Lys Ile Lys
        255                 260                 265

gat gta ctc atg atc tcc ctg agc ccc ata gtc aca tgt gtg gtg gtg         867
Asp Val Leu Met Ile Ser Leu Ser Pro Ile Val Thr Cys Val Val Val
    270                 275                 280

gat gtg agc gag gat gac cca gat gtc cag atc agc tgg ttt gtg aac         915
Asp Val Ser Glu Asp Asp Pro Asp Val Gln Ile Ser Trp Phe Val Asn
285                 290                 295                 300

aac gtg gaa gta cac aca gct cag aca caa acc cat aga gag gat tac         963
Asn Val Glu Val His Thr Ala Gln Thr Gln Thr His Arg Glu Asp Tyr
                305                 310                 315

aac agt act ctc cgg gtg gtc agt gcc ctc ccc atc cag cac cag gac        1011
Asn Ser Thr Leu Arg Val Val Ser Ala Leu Pro Ile Gln His Gln Asp
            320                 325                 330

tgg atg agt ggc aag gag ttc aaa tgc aag gtc aac aac aaa gac ctc        1059
Trp Met Ser Gly Lys Glu Phe Lys Cys Lys Val Asn Asn Lys Asp Leu
        335                 340                 345

cca gcg ccc atc gag aga acc atc tca aaa ccc aaa ggg tca gta aga        1107
Pro Ala Pro Ile Glu Arg Thr Ile Ser Lys Pro Lys Gly Ser Val Arg
    350                 355                 360

gct cca cag gta tat gtc ttg cct cca cca gaa gaa gag atg act aag        1155
Ala Pro Gln Val Tyr Val Leu Pro Pro Pro Glu Glu Glu Met Thr Lys
365                 370                 375                 380

aaa cag gtc act ctg acc tgc atg gtc aca gac ttc atg cct gaa gac        1203
Lys Gln Val Thr Leu Thr Cys Met Val Thr Asp Phe Met Pro Glu Asp
                385                 390                 395
```

```
att tac gtg gag tgg acc aac aac ggg aaa aca gag cta aac tac aag      1251
Ile Tyr Val Glu Trp Thr Asn Asn Gly Lys Thr Glu Leu Asn Tyr Lys
            400                 405                 410

aac act gaa cca gtc ctg gac tct gat ggt tct tac ttc atg tac agc      1299
Asn Thr Glu Pro Val Leu Asp Ser Asp Gly Ser Tyr Phe Met Tyr Ser
        415                 420                 425

aag ctg aga gtg gaa aag aag aac tgg gtg gaa aga aat agc tac tcc      1347
Lys Leu Arg Val Glu Lys Lys Asn Trp Val Glu Arg Asn Ser Tyr Ser
    430                 435                 440

tgt tca gtg gtc cac gag ggt ctg cac aat cac cac acg act aag agc      1395
Cys Ser Val Val His Glu Gly Leu His Asn His His Thr Thr Lys Ser
445                 450                 455                 460

ttc tcc cgg act ccg ggt aaa tga tagtctaga                            1428
Phe Ser Arg Thr Pro Gly Lys
                465


<210>  186
<211>  467
<212>  PRT
<213>  artificial sequence

<220>
<223>  Synthetic Construct

<400>  186

Met Lys His Leu Trp Phe Phe Leu Leu Leu Val Ala Ala Pro Arg Trp
1               5                   10                  15


Val Leu Ser Glu Val Gln Leu Gln Gln Ser Gly Pro Glu Leu Val Lys
            20                  25                  30


Pro Gly Ala Ser Val Lys Ile Ser Cys Lys Ala Ser Gly Tyr Ser Phe
        35                  40                  45


Thr Gly Tyr Tyr Met His Trp Val Lys Gln Ser Pro Glu Lys Ser Leu
    50                  55                  60


Glu Trp Ile Gly Glu Ile Asn Pro Ser Thr Gly Asp Thr Thr Tyr Asn
65                  70                  75                  80


Gln Lys Phe Lys Ala Lys Ala Thr Leu Thr Val Asp Lys Ser Ser Ser
                85                  90                  95


Thr Ala Tyr Ile Gln Leu Lys Ser Leu Thr Ser Glu Asp Ser Ala Val
            100                 105                 110


Tyr Tyr Cys Ala Arg Glu Arg Asn Tyr Asp Arg Phe Ala Tyr Trp Gly
        115                 120                 125
```

Gln Gly Thr Leu Val Thr Val Ser Ser Ala Lys Thr Thr Ala Pro Ser
    130                 135                 140

Val Tyr Pro Leu Ala Pro Val Cys Gly Asp Thr Thr Gly Ser Ser Val
145                 150                 155                 160

Thr Leu Gly Cys Leu Val Lys Gly Tyr Phe Pro Glu Pro Val Thr Leu
                165                 170                 175

Thr Trp Asn Ser Gly Ser Leu Ser Ser Gly Val His Thr Phe Pro Ala
            180                 185                 190

Val Leu Gln Ser Asp Leu Tyr Thr Leu Ser Ser Ser Val Thr Val Thr
        195                 200                 205

Ser Ser Thr Trp Pro Ser Gln Ser Ile Thr Cys Asn Val Ala His Pro
    210                 215                 220

Ala Ser Ser Thr Lys Val Asp Lys Lys Ile Glu Pro Arg Gly Pro Thr
225                 230                 235                 240

Ile Lys Pro Cys Pro Pro Cys Lys Cys Pro Ala Pro Asn Leu Leu Gly
                245                 250                 255

Gly Pro Ser Val Phe Ile Phe Pro Pro Lys Ile Lys Asp Val Leu Met
            260                 265                 270

Ile Ser Leu Ser Pro Ile Val Thr Cys Val Val Val Asp Val Ser Glu
        275                 280                 285

Asp Asp Pro Asp Val Gln Ile Ser Trp Phe Val Asn Asn Val Glu Val
    290                 295                 300

His Thr Ala Gln Thr Gln Thr His Arg Glu Asp Tyr Asn Ser Thr Leu
305                 310                 315                 320

Arg Val Val Ser Ala Leu Pro Ile Gln His Gln Asp Trp Met Ser Gly
                325                 330                 335

Lys Glu Phe Lys Cys Lys Val Asn Asn Lys Asp Leu Pro Ala Pro Ile
            340                 345                 350

Glu Arg Thr Ile Ser Lys Pro Lys Gly Ser Val Arg Ala Pro Gln Val
        355                 360                 365

Tyr Val Leu Pro Pro Pro Glu Glu Glu Met Thr Lys Lys Gln Val Thr
    370                 375                 380

```
Leu Thr Cys Met Val Thr Asp Phe Met Pro Glu Asp Ile Tyr Val Glu
385                 390                 395                 400


Trp Thr Asn Asn Gly Lys Thr Glu Leu Asn Tyr Lys Asn Thr Glu Pro
                405                 410                 415


Val Leu Asp Ser Asp Gly Ser Tyr Phe Met Tyr Ser Lys Leu Arg Val
            420                 425                 430


Glu Lys Lys Asn Trp Val Glu Arg Asn Ser Tyr Ser Cys Ser Val Val
        435                 440                 445


His Glu Gly Leu His Asn His His Thr Thr Lys Ser Phe Ser Arg Thr
    450                 455                 460


Pro Gly Lys
465


<210>  187
<211>  796
<212>  DNA
<213>  artificial sequence

<220>
<223>  Nucleic Acid Sequence of Light Chain of K090-02a034


<220>
<221>  CDS
<222>  (16)..(717)

<220>
<221>  misc_feature
<222>  (775)..(775)
<223>  n is a, c, g, t or u

<400>  187
aagcttgccg ccacc atg agg ctc ctt gct cag ctt ctg ggg ctg cta atg       51
                 Met Arg Leu Leu Ala Gln Leu Leu Gly Leu Leu Met
                 1               5                   10

ctc tgg gtc cct gga tcc agt ggg caa att gtt ctc tcc cag tct cca        99
Leu Trp Val Pro Gly Ser Ser Gly Gln Ile Val Leu Ser Gln Ser Pro
        15                  20                  25

gca atc atg tct gca tct cca ggg gag aag gtc acc atg acc tgc agt       147
Ala Ile Met Ser Ala Ser Pro Gly Glu Lys Val Thr Met Thr Cys Ser
    30                  35                  40

gcc agc tca agt gta agt tac atg tac tgg tac cag cag aag cca ggc       195
Ala Ser Ser Ser Val Ser Tyr Met Tyr Trp Tyr Gln Gln Lys Pro Gly
45                  50                  55                  60

tcc tcc ccc aga ctc ctg att tat gac aca tcc aac ctt gct tct gga       243
Ser Ser Pro Arg Leu Leu Ile Tyr Asp Thr Ser Asn Leu Ala Ser Gly
                65                  70                  75
```

```
gtc cct gtt cgc ttc agt ggc agt ggg tct ggg acc tct tac tct ctc        291
Val Pro Val Arg Phe Ser Gly Ser Gly Ser Gly Thr Ser Tyr Ser Leu
            80                  85                  90

aca atc agc cga atg gag gct gaa gat gct gcc act tat tac tgc cag        339
Thr Ile Ser Arg Met Glu Ala Glu Asp Ala Ala Thr Tyr Tyr Cys Gln
        95                  100                 105

cag tgg agt agt tac ccg ctc acg ttc ggt gct ggg acc aag ctg gag        387
Gln Trp Ser Ser Tyr Pro Leu Thr Phe Gly Ala Gly Thr Lys Leu Glu
    110                 115                 120

ctg aaa cgg gct gat gct gca cca act gta tcc atc ttc cca cca tcc        435
Leu Lys Arg Ala Asp Ala Ala Pro Thr Val Ser Ile Phe Pro Pro Ser
125                 130                 135                 140

agt gag cag tta aca tct gga ggt gcc tca gtc gtg tgc ttc ttg aac        483
Ser Glu Gln Leu Thr Ser Gly Gly Ala Ser Val Val Cys Phe Leu Asn
                145                 150                 155

aac ttc tac ccc aaa gac atc aat gtc aag tgg aag att gat ggc agt        531
Asn Phe Tyr Pro Lys Asp Ile Asn Val Lys Trp Lys Ile Asp Gly Ser
            160                 165                 170

gaa cga caa aat ggc gtc ctg aac agt tgg act gat cag gac agc aaa        579
Glu Arg Gln Asn Gly Val Leu Asn Ser Trp Thr Asp Gln Asp Ser Lys
        175                 180                 185

gac agc acc tac agc atg agc agc acc ctc acg ttg acc aag gac gag        627
Asp Ser Thr Tyr Ser Met Ser Ser Thr Leu Thr Leu Thr Lys Asp Glu
    190                 195                 200

tat gaa cga cat aac agc tat acc tgt gag gcc act cac aag aca tca        675
Tyr Glu Arg His Asn Ser Tyr Thr Cys Glu Ala Thr His Lys Thr Ser
205                 210                 215                 220

act tca ccc att gtc aag agc ttc aac agg aat gag tgt tga                717
Thr Ser Pro Ile Val Lys Ser Phe Asn Arg Asn Glu Cys
                225                 230

tagggcgcgc ctctagatga tcattccctt tagtgagggt taatgcttcg agcagacnat      777

gataagatac attgatgag                                                   796


<210>  188
<211>  233
<212>  PRT
<213>  artificial sequence

<220>
<223>  Synthetic Construct

<400>  188

Met Arg Leu Leu Ala Gln Leu Leu Gly Leu Leu Met Leu Trp Val Pro
1               5                   10                  15


Gly Ser Ser Gly Gln Ile Val Leu Ser Gln Ser Pro Ala Ile Met Ser
            20                  25                  30
```

Ala Ser Pro Gly Glu Lys Val Thr Met Thr Cys Ser Ala Ser Ser Ser
35 40 45

Val Ser Tyr Met Tyr Trp Tyr Gln Gln Lys Pro Gly Ser Ser Pro Arg
50 55 60

Leu Leu Ile Tyr Asp Thr Ser Asn Leu Ala Ser Gly Val Pro Val Arg
65 70 75 80

Phe Ser Gly Ser Gly Ser Gly Thr Ser Tyr Ser Leu Thr Ile Ser Arg
85 90 95

Met Glu Ala Glu Asp Ala Ala Thr Tyr Tyr Cys Gln Gln Trp Ser Ser
100 105 110

Tyr Pro Leu Thr Phe Gly Ala Gly Thr Lys Leu Glu Leu Lys Arg Ala
115 120 125

Asp Ala Ala Pro Thr Val Ser Ile Phe Pro Pro Ser Ser Glu Gln Leu
130 135 140

Thr Ser Gly Gly Ala Ser Val Val Cys Phe Leu Asn Asn Phe Tyr Pro
145 150 155 160

Lys Asp Ile Asn Val Lys Trp Lys Ile Asp Gly Ser Glu Arg Gln Asn
165 170 175

Gly Val Leu Asn Ser Trp Thr Asp Gln Asp Ser Lys Asp Ser Thr Tyr
180 185 190

Ser Met Ser Ser Thr Leu Thr Leu Thr Lys Asp Glu Tyr Glu Arg His
195 200 205

Asn Ser Tyr Thr Cys Glu Ala Thr His Lys Thr Ser Thr Ser Pro Ile
210 215 220

Val Lys Ser Phe Asn Arg Asn Glu Cys
225 230

<210> 189
<211> 1413
<212> DNA
<213> artificial sequence

<220>
<223> Nucleic Acid Sequence of Heavy Chain of K090-02a035

<220>

```
<221>  CDS
<222>  (16)..(1404)

<400>  189
aagcttgccg ccacc atg aaa cac ctg tgg ttc ttc ctc ctc ctg gtg gcg        51
                 Met Lys His Leu Trp Phe Phe Leu Leu Leu Val Ala
                 1               5                   10

gcc ccc aga tgg gtc ctg tcc cag gtc cag ctg cag cag tct ggg gcc        99
Ala Pro Arg Trp Val Leu Ser Gln Val Gln Leu Gln Gln Ser Gly Ala
        15                  20                  25

gag ctg gtg aag cct ggg gct tca gtg aag ctg tcc tgc aag act tct        147
Glu Leu Val Lys Pro Gly Ala Ser Val Lys Leu Ser Cys Lys Thr Ser
    30                  35                  40

ggt tac acc ttc acc agc tac tgg atg cac tgg gtg aag cag agg cct        195
Gly Tyr Thr Phe Thr Ser Tyr Trp Met His Trp Val Lys Gln Arg Pro
45                  50                  55                  60

gga caa ggc ctt gaa tgg att ggt aat att gac cct tct gat agt gaa        243
Gly Gln Gly Leu Glu Trp Ile Gly Asn Ile Asp Pro Ser Asp Ser Glu
                65                  70                  75

act cac tac aat caa aag ttc aag gac aag gcc aca ttg act gtt gac        291
Thr His Tyr Asn Gln Lys Phe Lys Asp Lys Ala Thr Leu Thr Val Asp
            80                  85                  90

aaa tcc tcc agc aca gcc tac atg cag ctc agc ggc ctg aca tct gag        339
Lys Ser Ser Ser Thr Ala Tyr Met Gln Leu Ser Gly Leu Thr Ser Glu
        95                  100                 105

gac tct gcg gtc tat tac tgt gca aga aac ttg ggc tac tgg ggt caa        387
Asp Ser Ala Val Tyr Tyr Cys Ala Arg Asn Leu Gly Tyr Trp Gly Gln
    110                 115                 120

gga acc tca gtc acc gtc tcg agc gcc aaa aca aca gcc cca tcg gtc        435
Gly Thr Ser Val Thr Val Ser Ser Ala Lys Thr Thr Ala Pro Ser Val
125                 130                 135                 140

tat cca ctg gcc cct gtg tgt gga gat aca act ggc tcc tcg gtg act        483
Tyr Pro Leu Ala Pro Val Cys Gly Asp Thr Thr Gly Ser Ser Val Thr
                145                 150                 155

cta gga tgc ctg gtc aag ggt tat ttc cct gag cca gtg acc ttg acc        531
Leu Gly Cys Leu Val Lys Gly Tyr Phe Pro Glu Pro Val Thr Leu Thr
            160                 165                 170

tgg aac tct gga tcc ctg tcc agt ggt gtg cac acc ttc cca gct gtc        579
Trp Asn Ser Gly Ser Leu Ser Ser Gly Val His Thr Phe Pro Ala Val
        175                 180                 185

ctg cag tct gac ctc tac acc ctc agc agc tca gtg act gta acc tcg        627
Leu Gln Ser Asp Leu Tyr Thr Leu Ser Ser Ser Val Thr Val Thr Ser
    190                 195                 200

agc acc tgg ccc agc cag tcc atc acc tgc aat gtg gcc cac ccg gca        675
Ser Thr Trp Pro Ser Gln Ser Ile Thr Cys Asn Val Ala His Pro Ala
205                 210                 215                 220

agc agc acc aag gtg gac aag aaa att gag ccc cgg gga ccc aca atc        723
Ser Ser Thr Lys Val Asp Lys Lys Ile Glu Pro Arg Gly Pro Thr Ile
                225                 230                 235
```

```
aag ccc tgt cct cca tgc aaa tgc cca gca cct aac ctc ttg ggt gga         771
Lys Pro Cys Pro Pro Cys Lys Cys Pro Ala Pro Asn Leu Leu Gly Gly
            240                 245                 250

cca tcc gtc ttc atc ttc cct cca aag atc aag gat gta ctc atg atc         819
Pro Ser Val Phe Ile Phe Pro Pro Lys Ile Lys Asp Val Leu Met Ile
        255                 260                 265

tcc ctg agc ccc ata gtc aca tgt gtg gtg gtg gat gtg agc gag gat         867
Ser Leu Ser Pro Ile Val Thr Cys Val Val Val Asp Val Ser Glu Asp
    270                 275                 280

gac cca gat gtc cag atc agc tgg ttt gtg aac aac gtg gaa gta cac         915
Asp Pro Asp Val Gln Ile Ser Trp Phe Val Asn Asn Val Glu Val His
285                 290                 295                 300

aca gct cag aca caa acc cat aga gag gat tac aac agt act ctc cgg         963
Thr Ala Gln Thr Gln Thr His Arg Glu Asp Tyr Asn Ser Thr Leu Arg
                305                 310                 315

gtg gtc agt gcc ctc ccc atc cag cac cag gac tgg atg agt ggc aag        1011
Val Val Ser Ala Leu Pro Ile Gln His Gln Asp Trp Met Ser Gly Lys
            320                 325                 330

gag ttc aaa tgc aag gtc aac aac aaa gac ctc cca gcg ccc atc gag        1059
Glu Phe Lys Cys Lys Val Asn Asn Lys Asp Leu Pro Ala Pro Ile Glu
        335                 340                 345

aga acc atc tca aaa ccc aaa ggg tca gta aga gct cca cag gta tat        1107
Arg Thr Ile Ser Lys Pro Lys Gly Ser Val Arg Ala Pro Gln Val Tyr
    350                 355                 360

gtc ttg cct cca cca gaa gaa gag atg act aag aaa cag gtc act ctg        1155
Val Leu Pro Pro Pro Glu Glu Glu Met Thr Lys Lys Gln Val Thr Leu
365                 370                 375                 380

acc tgc atg gtc aca gac ttc atg cct gaa gac att tac gtg gag tgg        1203
Thr Cys Met Val Thr Asp Phe Met Pro Glu Asp Ile Tyr Val Glu Trp
                385                 390                 395

acc aac aac ggg aaa aca gag cta aac tac aag aac act gaa cca gtc        1251
Thr Asn Asn Gly Lys Thr Glu Leu Asn Tyr Lys Asn Thr Glu Pro Val
            400                 405                 410

ctg gac tct gat ggt tct tac ttc atg tac agc aag ctg aga gtg gaa        1299
Leu Asp Ser Asp Gly Ser Tyr Phe Met Tyr Ser Lys Leu Arg Val Glu
        415                 420                 425

aag aag aac tgg gtg gaa aga aat agc tac tcc tgt tca gtg gtc cac        1347
Lys Lys Asn Trp Val Glu Arg Asn Ser Tyr Ser Cys Ser Val Val His
    430                 435                 440

gag ggt ctg cac aat cac cac acg act aag agc ttc tcc cgg act ccg        1395
Glu Gly Leu His Asn His His Thr Thr Lys Ser Phe Ser Arg Thr Pro
445                 450                 455                 460

ggt aaa tga tagtctaga                                                  1413
Gly Lys
```

<210> 190

<211> 462
<212> PRT
<213> artificial sequence

<220>
<223> Synthetic Construct

<400> 190

```
Met Lys His Leu Trp Phe Phe Leu Leu Leu Val Ala Ala Pro Arg Trp
1               5                   10                  15

Val Leu Ser Gln Val Gln Leu Gln Gln Ser Gly Ala Glu Leu Val Lys
            20                  25                  30

Pro Gly Ala Ser Val Lys Leu Ser Cys Lys Thr Ser Gly Tyr Thr Phe
        35                  40                  45

Thr Ser Tyr Trp Met His Trp Val Lys Gln Arg Pro Gly Gln Gly Leu
    50                  55                  60

Glu Trp Ile Gly Asn Ile Asp Pro Ser Asp Ser Glu Thr His Tyr Asn
65                  70                  75                  80

Gln Lys Phe Lys Asp Lys Ala Thr Leu Thr Val Asp Lys Ser Ser Ser
                85                  90                  95

Thr Ala Tyr Met Gln Leu Ser Gly Leu Thr Ser Glu Asp Ser Ala Val
            100                 105                 110

Tyr Tyr Cys Ala Arg Asn Leu Gly Tyr Trp Gly Gln Gly Thr Ser Val
        115                 120                 125

Thr Val Ser Ser Ala Lys Thr Thr Ala Pro Ser Val Tyr Pro Leu Ala
    130                 135                 140

Pro Val Cys Gly Asp Thr Thr Gly Ser Ser Val Thr Leu Gly Cys Leu
145                 150                 155                 160

Val Lys Gly Tyr Phe Pro Glu Pro Val Thr Leu Thr Trp Asn Ser Gly
                165                 170                 175

Ser Leu Ser Ser Gly Val His Thr Phe Pro Ala Val Leu Gln Ser Asp
            180                 185                 190

Leu Tyr Thr Leu Ser Ser Ser Val Thr Val Thr Ser Ser Thr Trp Pro
        195                 200                 205

Ser Gln Ser Ile Thr Cys Asn Val Ala His Pro Ala Ser Ser Thr Lys
    210                 215                 220
```

Val Asp Lys Lys Ile Glu Pro Arg Gly Pro Thr Ile Lys Pro Cys Pro
225 230 235 240

Pro Cys Lys Cys Pro Ala Pro Asn Leu Leu Gly Gly Pro Ser Val Phe
245 250 255

Ile Phe Pro Pro Lys Ile Lys Asp Val Leu Met Ile Ser Leu Ser Pro
260 265 270

Ile Val Thr Cys Val Val Val Asp Val Ser Glu Asp Asp Pro Asp Val
275 280 285

Gln Ile Ser Trp Phe Val Asn Asn Val Glu Val His Thr Ala Gln Thr
290 295 300

Gln Thr His Arg Glu Asp Tyr Asn Ser Thr Leu Arg Val Val Ser Ala
305 310 315 320

Leu Pro Ile Gln His Gln Asp Trp Met Ser Gly Lys Glu Phe Lys Cys
325 330 335

Lys Val Asn Asn Lys Asp Leu Pro Ala Pro Ile Glu Arg Thr Ile Ser
340 345 350

Lys Pro Lys Gly Ser Val Arg Ala Pro Gln Val Tyr Val Leu Pro Pro
355 360 365

Pro Glu Glu Glu Met Thr Lys Lys Gln Val Thr Leu Thr Cys Met Val
370 375 380

Thr Asp Phe Met Pro Glu Asp Ile Tyr Val Glu Trp Thr Asn Asn Gly
385 390 395 400

Lys Thr Glu Leu Asn Tyr Lys Asn Thr Glu Pro Val Leu Asp Ser Asp
405 410 415

Gly Ser Tyr Phe Met Tyr Ser Lys Leu Arg Val Glu Lys Lys Asn Trp
420 425 430

Val Glu Arg Asn Ser Tyr Ser Cys Ser Val Val His Glu Gly Leu His
435 440 445

Asn His His Thr Thr Lys Ser Phe Ser Arg Thr Pro Gly Lys
450 455 460

<210> 191

```
<211>  812
<212>  DNA
<213>  artificial sequence

<220>
<223>  Nucleic Acid Sequence of Light Chain of K090-02a035


<220>
<221>  CDS
<222>  (16)..(735)

<400>  191
aagcttgccg ccacc atg agg ctc ctt gct cag ctt ctg ggg ctg cta atg        51
                 Met Arg Leu Leu Ala Gln Leu Leu Gly Leu Leu Met
                 1               5                   10

ctc tgg gtc cct gga tcc agt ggg gac atc aag atg acc cag tct cca         99
Leu Trp Val Pro Gly Ser Ser Gly Asp Ile Lys Met Thr Gln Ser Pro
        15                  20                  25

ctc tcc ctg cct gtc agt ctt gga gat caa gcc tcc atc tct tgc aga        147
Leu Ser Leu Pro Val Ser Leu Gly Asp Gln Ala Ser Ile Ser Cys Arg
    30                  35                  40

tct agt cag agc ctt gta cac agt aat gga aac acc tat tta cat tgg        195
Ser Ser Gln Ser Leu Val His Ser Asn Gly Asn Thr Tyr Leu His Trp
45                  50                  55                  60

tac ctg cag aag cca ggc cag tct cca aag ctc ctg atc tac aaa gtt        243
Tyr Leu Gln Lys Pro Gly Gln Ser Pro Lys Leu Leu Ile Tyr Lys Val
                65                  70                  75

tcc aac cga ttt tct ggg gtc cca gac agg ttc agt ggc agt gga tca        291
Ser Asn Arg Phe Ser Gly Val Pro Asp Arg Phe Ser Gly Ser Gly Ser
            80                  85                  90

ggg aca gat ttc aca ctc aag atc agc aga gtg gag gct gag gat ctg        339
Gly Thr Asp Phe Thr Leu Lys Ile Ser Arg Val Glu Ala Glu Asp Leu
        95                  100                 105

gga gtt tat ttc tgc tct caa agt aca cat gtt ccg tac acg ttc gga        387
Gly Val Tyr Phe Cys Ser Gln Ser Thr His Val Pro Tyr Thr Phe Gly
    110                 115                 120

ggg ggg acc aag ctg gaa atc aaa cgg gct gat gct gca cca act gta        435
Gly Gly Thr Lys Leu Glu Ile Lys Arg Ala Asp Ala Ala Pro Thr Val
125                 130                 135                 140

tcc atc ttc cca cca tcc agt gag cag tta aca tct gga ggt gcc tca        483
Ser Ile Phe Pro Pro Ser Ser Glu Gln Leu Thr Ser Gly Gly Ala Ser
                145                 150                 155

gtc gtg tgc ttc ttg aac aac ttc tac ccc aaa gac atc aat gtc aag        531
Val Val Cys Phe Leu Asn Asn Phe Tyr Pro Lys Asp Ile Asn Val Lys
            160                 165                 170

tgg aag att gat ggc agt gaa cga caa aat ggc gtc ctg aac agt tgg        579
Trp Lys Ile Asp Gly Ser Glu Arg Gln Asn Gly Val Leu Asn Ser Trp
        175                 180                 185

act gat cag gac agc aaa gac agc acc tac agc atg agc agc acc ctc        627
Thr Asp Gln Asp Ser Lys Asp Ser Thr Tyr Ser Met Ser Ser Thr Leu
```

190 195 200

acg ttg acc aag gac gag tat gaa cga cat aac agc tat acc tgt gag 675
Thr Leu Thr Lys Asp Glu Tyr Glu Arg His Asn Ser Tyr Thr Cys Glu
205 210 215 220

gcc act cac aag aca tca act tca ccc att gtc aag agc ttc aac agg 723
Ala Thr His Lys Thr Ser Thr Ser Pro Ile Val Lys Ser Phe Asn Arg
225 230 235

aat gag tgt tga tagggcgcgc ctctagatga tcattccctt tagtgagggt 775
Asn Glu Cys

taatgcttcg agcagacatg ataagataca ttgatga 812

<210> 192
<211> 239
<212> PRT
<213> artificial sequence

<220>
<223> Synthetic Construct

<400> 192

Met Arg Leu Leu Ala Gln Leu Leu Gly Leu Leu Met Leu Trp Val Pro
1 5 10 15

Gly Ser Ser Gly Asp Ile Lys Met Thr Gln Ser Pro Leu Ser Leu Pro
20 25 30

Val Ser Leu Gly Asp Gln Ala Ser Ile Ser Cys Arg Ser Ser Gln Ser
35 40 45

Leu Val His Ser Asn Gly Asn Thr Tyr Leu His Trp Tyr Leu Gln Lys
50 55 60

Pro Gly Gln Ser Pro Lys Leu Leu Ile Tyr Lys Val Ser Asn Arg Phe
65 70 75 80

Ser Gly Val Pro Asp Arg Phe Ser Gly Ser Gly Ser Gly Thr Asp Phe
85 90 95

Thr Leu Lys Ile Ser Arg Val Glu Ala Glu Asp Leu Gly Val Tyr Phe
100 105 110

Cys Ser Gln Ser Thr His Val Pro Tyr Thr Phe Gly Gly Gly Thr Lys
115 120 125

Leu Glu Ile Lys Arg Ala Asp Ala Ala Pro Thr Val Ser Ile Phe Pro
130 135 140

```
Pro Ser Ser Glu Gln Leu Thr Ser Gly Gly Ala Ser Val Val Cys Phe
145                 150                 155                 160

Leu Asn Asn Phe Tyr Pro Lys Asp Ile Asn Val Lys Trp Lys Ile Asp
                165                 170                 175

Gly Ser Glu Arg Gln Asn Gly Val Leu Asn Ser Trp Thr Asp Gln Asp
            180                 185                 190

Ser Lys Asp Ser Thr Tyr Ser Met Ser Ser Thr Leu Thr Leu Thr Lys
        195                 200                 205

Asp Glu Tyr Glu Arg His Asn Ser Tyr Thr Cys Glu Ala Thr His Lys
    210                 215                 220

Thr Ser Thr Ser Pro Ile Val Lys Ser Phe Asn Arg Asn Glu Cys
225                 230                 235


<210>  193
<211>  1449
<212>  DNA
<213>  artificial sequence

<220>
<223>  Nucleic Acid Sequence of Heavy Chain of K090-02b006


<220>
<221>  CDS
<222>  (16)..(1440)

<400>  193
aagcttgccg ccacc atg aaa cac ctg tgg ttc ttc ctc ctc ctg gtg gcg        51
                 Met Lys His Leu Trp Phe Phe Leu Leu Leu Val Ala
                 1               5                   10

gcc ccc aga tgg gtc ctg tcc gaa gtg cag ctg gtg gag tct ggg gga         99
Ala Pro Arg Trp Val Leu Ser Glu Val Gln Leu Val Glu Ser Gly Gly
        15                  20                  25

ggc tta gtg aag cct gga ggg tcc cgg aaa ctc tcc tgt gca gcc tct        147
Gly Leu Val Lys Pro Gly Gly Ser Arg Lys Leu Ser Cys Ala Ala Ser
    30                  35                  40

gga ttc act ttc agt gac tat gga atg cac tgg gtc cgt cag gct cca        195
Gly Phe Thr Phe Ser Asp Tyr Gly Met His Trp Val Arg Gln Ala Pro
45                  50                  55                  60

gag aag ggg ctg gag tgg gtt gca tac att agt agt ggc agt agt acc        243
Glu Lys Gly Leu Glu Trp Val Ala Tyr Ile Ser Ser Gly Ser Ser Thr
                65                  70                  75

atc tac tat gca gac aca gtg aag ggc cga ttc acc atc tcc aga gac        291
Ile Tyr Tyr Ala Asp Thr Val Lys Gly Arg Phe Thr Ile Ser Arg Asp
            80                  85                  90

aat gcc aag aac acc ctg ttc ctg caa atg acc agt cta agg tct gag        339
```

```
Asn Ala Lys Asn Thr Leu Phe Leu Gln Met Thr Ser Leu Arg Ser Glu
        95                  100                 105

gac aca gcc ata tat tac tgt gta agg cat agt tac tat aat tac gac      387
Asp Thr Ala Ile Tyr Tyr Cys Val Arg His Ser Tyr Tyr Asn Tyr Asp
    110                 115                 120

att ggg ggt tac tat gct atg gac tac tgg ggt caa gga acc tca gtc      435
Ile Gly Gly Tyr Tyr Ala Met Asp Tyr Trp Gly Gln Gly Thr Ser Val
125                 130                 135                 140

acc gtc tcg agc gcc aaa aca aca gcc cca tcg gtc tat cca ctg gcc      483
Thr Val Ser Ser Ala Lys Thr Thr Ala Pro Ser Val Tyr Pro Leu Ala
                145                 150                 155

cct gtg tgt gga gat aca act ggc tcc tcg gtg act cta gga tgc ctg      531
Pro Val Cys Gly Asp Thr Thr Gly Ser Ser Val Thr Leu Gly Cys Leu
            160                 165                 170

gtc aag ggt tat ttc cct gag cca gtg acc ttg acc tgg aac tct gga      579
Val Lys Gly Tyr Phe Pro Glu Pro Val Thr Leu Thr Trp Asn Ser Gly
        175                 180                 185

tcc ctg tcc agt ggt gtg cac acc ttc cca gct gtc ctg cag tct gac      627
Ser Leu Ser Ser Gly Val His Thr Phe Pro Ala Val Leu Gln Ser Asp
    190                 195                 200

ctc tac acc ctc agc agc tca gtg act gta acc tcg agc acc tgg ccc      675
Leu Tyr Thr Leu Ser Ser Ser Val Thr Val Thr Ser Ser Thr Trp Pro
205                 210                 215                 220

agc cag tcc atc acc tgc aat gtg gcc cac ccg gca agc agc acc aag      723
Ser Gln Ser Ile Thr Cys Asn Val Ala His Pro Ala Ser Ser Thr Lys
                225                 230                 235

gtg gac aag aaa att gag ccc cgg gga ccc aca atc aag ccc tgt cct      771
Val Asp Lys Lys Ile Glu Pro Arg Gly Pro Thr Ile Lys Pro Cys Pro
            240                 245                 250

cca tgc aaa tgc cca gca cct aac ctc ttg ggt gga cca tcc gtc ttc      819
Pro Cys Lys Cys Pro Ala Pro Asn Leu Leu Gly Gly Pro Ser Val Phe
        255                 260                 265

atc ttc cct cca aag atc aag gat gta ctc atg atc tcc ctg agc ccc      867
Ile Phe Pro Pro Lys Ile Lys Asp Val Leu Met Ile Ser Leu Ser Pro
    270                 275                 280

ata gtc aca tgt gtg gtg gtg gat gtg agc gag gat gac cca gat gtc      915
Ile Val Thr Cys Val Val Val Asp Val Ser Glu Asp Asp Pro Asp Val
285                 290                 295                 300

cag atc agc tgg ttt gtg aac aac gtg gaa gta cac aca gct cag aca      963
Gln Ile Ser Trp Phe Val Asn Asn Val Glu Val His Thr Ala Gln Thr
                305                 310                 315

caa acc cat aga gag gat tac aac agt act ctc cgg gtg gtc agt gcc     1011
Gln Thr His Arg Glu Asp Tyr Asn Ser Thr Leu Arg Val Val Ser Ala
            320                 325                 330

ctc ccc atc cag cac cag gac tgg atg agt ggc aag gag ttc aaa tgc     1059
Leu Pro Ile Gln His Gln Asp Trp Met Ser Gly Lys Glu Phe Lys Cys
        335                 340                 345
```

```
aag gtc aac aac aaa gac ctc cca gcg ccc atc gag aga acc atc tca        1107
Lys Val Asn Asn Lys Asp Leu Pro Ala Pro Ile Glu Arg Thr Ile Ser
    350                 355                 360

aaa ccc aaa ggg tca gta aga gct cca cag gta tat gtc ttg cct cca        1155
Lys Pro Lys Gly Ser Val Arg Ala Pro Gln Val Tyr Val Leu Pro Pro
365                 370                 375                 380

cca gaa gaa gag atg act aag aaa cag gtc act ctg acc tgc atg gtc        1203
Pro Glu Glu Glu Met Thr Lys Lys Gln Val Thr Leu Thr Cys Met Val
                385                 390                 395

aca gac ttc atg cct gaa gac att tac gtg gag tgg acc aac aac ggg        1251
Thr Asp Phe Met Pro Glu Asp Ile Tyr Val Glu Trp Thr Asn Asn Gly
            400                 405                 410

aaa aca gag cta aac tac aag aac act gaa cca gtc ctg gac tct gat        1299
Lys Thr Glu Leu Asn Tyr Lys Asn Thr Glu Pro Val Leu Asp Ser Asp
        415                 420                 425

ggt tct tac ttc atg tac agc aag ctg aga gtg gaa aag aag aac tgg        1347
Gly Ser Tyr Phe Met Tyr Ser Lys Leu Arg Val Glu Lys Lys Asn Trp
    430                 435                 440

gtg gaa aga aat agc tac tcc tgt tca gtg gtc cac gag ggt ctg cac        1395
Val Glu Arg Asn Ser Tyr Ser Cys Ser Val Val His Glu Gly Leu His
445                 450                 455                 460

aat cac cac acg act aag agc ttc tcc cgg act ccg ggt aaa tga            1440
Asn His His Thr Thr Lys Ser Phe Ser Arg Thr Pro Gly Lys
                465                 470

tagtctaga                                                              1449


<210>  194
<211>  474
<212>  PRT
<213>  artificial sequence

<220>
<223>  Synthetic Construct

<400>  194

Met Lys His Leu Trp Phe Phe Leu Leu Leu Val Ala Ala Pro Arg Trp
1               5                   10                  15


Val Leu Ser Glu Val Gln Leu Val Glu Ser Gly Gly Gly Leu Val Lys
            20                  25                  30


Pro Gly Gly Ser Arg Lys Leu Ser Cys Ala Ala Ser Gly Phe Thr Phe
        35                  40                  45


Ser Asp Tyr Gly Met His Trp Val Arg Gln Ala Pro Glu Lys Gly Leu
    50                  55                  60


Glu Trp Val Ala Tyr Ile Ser Ser Gly Ser Ser Thr Ile Tyr Tyr Ala
65                  70                  75                  80
```

Asp Thr Val Lys Gly Arg Phe Thr Ile Ser Arg Asp Asn Ala Lys Asn
85 90 95

Thr Leu Phe Leu Gln Met Thr Ser Leu Arg Ser Glu Asp Thr Ala Ile
100 105 110

Tyr Tyr Cys Val Arg His Ser Tyr Tyr Asn Tyr Asp Ile Gly Gly Tyr
115 120 125

Tyr Ala Met Asp Tyr Trp Gly Gln Gly Thr Ser Val Thr Val Ser Ser
130 135 140

Ala Lys Thr Thr Ala Pro Ser Val Tyr Pro Leu Ala Pro Val Cys Gly
145 150 155 160

Asp Thr Thr Gly Ser Ser Val Thr Leu Gly Cys Leu Val Lys Gly Tyr
165 170 175

Phe Pro Glu Pro Val Thr Leu Thr Trp Asn Ser Gly Ser Leu Ser Ser
180 185 190

Gly Val His Thr Phe Pro Ala Val Leu Gln Ser Asp Leu Tyr Thr Leu
195 200 205

Ser Ser Ser Val Thr Val Thr Ser Ser Thr Trp Pro Ser Gln Ser Ile
210 215 220

Thr Cys Asn Val Ala His Pro Ala Ser Ser Thr Lys Val Asp Lys Lys
225 230 235 240

Ile Glu Pro Arg Gly Pro Thr Ile Lys Pro Cys Pro Pro Cys Lys Cys
245 250 255

Pro Ala Pro Asn Leu Leu Gly Gly Pro Ser Val Phe Ile Phe Pro Pro
260 265 270

Lys Ile Lys Asp Val Leu Met Ile Ser Leu Ser Pro Ile Val Thr Cys
275 280 285

Val Val Val Asp Val Ser Glu Asp Asp Pro Asp Val Gln Ile Ser Trp
290 295 300

Phe Val Asn Asn Val Glu Val His Thr Ala Gln Thr Gln Thr His Arg
305 310 315 320

Glu Asp Tyr Asn Ser Thr Leu Arg Val Val Ser Ala Leu Pro Ile Gln

```
            325                 330                 335

His Gln Asp Trp Met Ser Gly Lys Glu Phe Lys Cys Lys Val Asn Asn
        340                 345                 350

Lys Asp Leu Pro Ala Pro Ile Glu Arg Thr Ile Ser Lys Pro Lys Gly
    355                 360                 365

Ser Val Arg Ala Pro Gln Val Tyr Val Leu Pro Pro Pro Glu Glu Glu
370                 375                 380

Met Thr Lys Lys Gln Val Thr Leu Thr Cys Met Val Thr Asp Phe Met
385                 390                 395                 400

Pro Glu Asp Ile Tyr Val Glu Trp Thr Asn Asn Gly Lys Thr Glu Leu
                405                 410                 415

Asn Tyr Lys Asn Thr Glu Pro Val Leu Asp Ser Asp Gly Ser Tyr Phe
            420                 425                 430

Met Tyr Ser Lys Leu Arg Val Glu Lys Lys Asn Trp Val Glu Arg Asn
        435                 440                 445

Ser Tyr Ser Cys Ser Val Val His Glu Gly Leu His Asn His His Thr
    450                 455                 460

Thr Lys Ser Phe Ser Arg Thr Pro Gly Lys
465                 470
```

```
<210>  195
<211>  797
<212>  DNA
<213>  artificial sequence

<220>
<223>  Nucleic Acid Sequence of Light Chain of K090-02b006


<220>
<221>  CDS
<222>  (16)..(720)

<400>  195
aagcttgccg ccacc atg agg ctc ctt gct cag ctt ctg ggg ctg cta atg      51
                 Met Arg Leu Leu Ala Gln Leu Leu Gly Leu Leu Met
                 1               5                   10

ctc tgg gtc cct gga tcc agt ggg gac att gtg ctg aca cag tct cca       99
Leu Trp Val Pro Gly Ser Ser Gly Asp Ile Val Leu Thr Gln Ser Pro
        15                  20                  25

tcc tcc tta tct gcc tct ctg gga gaa aga gtc agt ctc act tgt cgg      147
Ser Ser Leu Ser Ala Ser Leu Gly Glu Arg Val Ser Leu Thr Cys Arg
```

```
    30                  35                  40

gca agt cag gaa att agt ggt tac tta agc tgg ctt cag cag aaa cca        195
Ala Ser Gln Glu Ile Ser Gly Tyr Leu Ser Trp Leu Gln Gln Lys Pro
45                  50                  55                  60

gat gga act att aaa cgc ctg atc tac gcc gca tcc act tta gat tct        243
Asp Gly Thr Ile Lys Arg Leu Ile Tyr Ala Ala Ser Thr Leu Asp Ser
                65                  70                  75

ggt gtc cca aaa agg ttc agt ggc agt agg tct ggg tca gat tat tct        291
Gly Val Pro Lys Arg Phe Ser Gly Ser Arg Ser Gly Ser Asp Tyr Ser
            80                  85                  90

ctc acc atc agc agc ctt gag tct gaa gat ttt gca gac tat tac tgt        339
Leu Thr Ile Ser Ser Leu Glu Ser Glu Asp Phe Ala Asp Tyr Tyr Cys
        95                  100                 105

cta caa tat gct agt tat cct ctc acg ttc ggt gct ggg acc aag ctg        387
Leu Gln Tyr Ala Ser Tyr Pro Leu Thr Phe Gly Ala Gly Thr Lys Leu
    110                 115                 120

gaa atc aaa cgg gct gat gct gca cca act gta tcc atc ttc cca cca        435
Glu Ile Lys Arg Ala Asp Ala Ala Pro Thr Val Ser Ile Phe Pro Pro
125                 130                 135                 140

tcc agt gag cag tta aca tct gga ggt gcc tca gtc gtg tgc ttc ttg        483
Ser Ser Glu Gln Leu Thr Ser Gly Gly Ala Ser Val Val Cys Phe Leu
                145                 150                 155

aac aac ttc tac ccc aaa gac atc aat gtc aag tgg aag att gat ggc        531
Asn Asn Phe Tyr Pro Lys Asp Ile Asn Val Lys Trp Lys Ile Asp Gly
            160                 165                 170

agt gaa cga caa aat ggc gtc ctg aac agt tgg act gat cag gac agc        579
Ser Glu Arg Gln Asn Gly Val Leu Asn Ser Trp Thr Asp Gln Asp Ser
        175                 180                 185

aaa gac agc acc tac agc atg agc agc acc ctc acg ttg acc aag gac        627
Lys Asp Ser Thr Tyr Ser Met Ser Ser Thr Leu Thr Leu Thr Lys Asp
    190                 195                 200

gag tat gaa cga cat aac agc tat acc tgt gag gcc act cac aag aca        675
Glu Tyr Glu Arg His Asn Ser Tyr Thr Cys Glu Ala Thr His Lys Thr
205                 210                 215                 220

tca act tca ccc att gtc aag agc ttc aac agg aat gag tgt tga            720
Ser Thr Ser Pro Ile Val Lys Ser Phe Asn Arg Asn Glu Cys
                225                 230

tagggcgcgc ctctagatga tcattccctt tagtgagggt taatgcttcg agcagacatg      780

ataagataca ttgatga                                                     797


<210>  196
<211>  234
<212>  PRT
<213>  artificial sequence

<220>
<223>  Synthetic Construct
```

<400> 196

Met Arg Leu Leu Ala Gln Leu Leu Gly Leu Leu Met Leu Trp Val Pro
1               5                   10                  15

Gly Ser Ser Gly Asp Ile Val Leu Thr Gln Ser Pro Ser Ser Leu Ser
            20                  25                  30

Ala Ser Leu Gly Glu Arg Val Ser Leu Thr Cys Arg Ala Ser Gln Glu
        35                  40                  45

Ile Ser Gly Tyr Leu Ser Trp Leu Gln Gln Lys Pro Asp Gly Thr Ile
    50                  55                  60

Lys Arg Leu Ile Tyr Ala Ala Ser Thr Leu Asp Ser Gly Val Pro Lys
65                  70                  75                  80

Arg Phe Ser Gly Ser Arg Ser Gly Ser Asp Tyr Ser Leu Thr Ile Ser
                85                  90                  95

Ser Leu Glu Ser Glu Asp Phe Ala Asp Tyr Tyr Cys Leu Gln Tyr Ala
            100                 105                 110

Ser Tyr Pro Leu Thr Phe Gly Ala Gly Thr Lys Leu Glu Ile Lys Arg
        115                 120                 125

Ala Asp Ala Ala Pro Thr Val Ser Ile Phe Pro Pro Ser Ser Glu Gln
    130                 135                 140

Leu Thr Ser Gly Gly Ala Ser Val Val Cys Phe Leu Asn Asn Phe Tyr
145                 150                 155                 160

Pro Lys Asp Ile Asn Val Lys Trp Lys Ile Asp Gly Ser Glu Arg Gln
                165                 170                 175

Asn Gly Val Leu Asn Ser Trp Thr Asp Gln Asp Ser Lys Asp Ser Thr
            180                 185                 190

Tyr Ser Met Ser Ser Thr Leu Thr Leu Thr Lys Asp Glu Tyr Glu Arg
        195                 200                 205

His Asn Ser Tyr Thr Cys Glu Ala Thr His Lys Thr Ser Thr Ser Pro
    210                 215                 220

Ile Val Lys Ser Phe Asn Arg Asn Glu Cys
225                 230

<210> 197

<211>  369
<212>  DNA
<213>  artificial sequence

<220>
<223>  Nucleic Acid Sequence of Heavy Chain of Clone 4

<220>
<221>  CDS
<222>  (1)..(369)

<400>  197

```
gcg gtg acg ttg gac gag tcc ggg ggc ggc ctc cag acg ccc gga aga        48
Ala Val Thr Leu Asp Glu Ser Gly Gly Gly Leu Gln Thr Pro Gly Arg
1               5                   10                  15

gcg ctc agc ctc gtc tgt aag gcc tcc ggg ttc acc ttc agc cgt tac        96
Ala Leu Ser Leu Val Cys Lys Ala Ser Gly Phe Thr Phe Ser Arg Tyr
            20                  25                  30

gcc atg tac tgg gtg cga cag gcg ccc ggc aag ggg ctg gag ttc gtc       144
Ala Met Tyr Trp Val Arg Gln Ala Pro Gly Lys Gly Leu Glu Phe Val
        35                  40                  45

gct ggt att ggc aac act ggt aga tac aca ggc tac ggg tcg gcg gtg       192
Ala Gly Ile Gly Asn Thr Gly Arg Tyr Thr Gly Tyr Gly Ser Ala Val
    50                  55                  60

aag ggc cgt gcc acc atc tcg agg gac agc ggg cag agc aca gtg agg       240
Lys Gly Arg Ala Thr Ile Ser Arg Asp Ser Gly Gln Ser Thr Val Arg
65                  70                  75                  80

ctg caa ctg aac aac ctc agg gct gag gac acc ggc aac tac tac tgc       288
Leu Gln Leu Asn Asn Leu Arg Ala Glu Asp Thr Gly Asn Tyr Tyr Cys
                85                  90                  95

gcc aaa agt gtt agt cct tac tgt tgt gat gct gct gac atc gac gca       336
Ala Lys Ser Val Ser Pro Tyr Cys Cys Asp Ala Ala Asp Ile Asp Ala
            100                 105                 110

tgg ggc cac ggg acc gaa gtc atc gtc tcc tcc                           369
Trp Gly His Gly Thr Glu Val Ile Val Ser Ser
        115                 120
```

<210>  198
<211>  123
<212>  PRT
<213>  artificial sequence

<220>
<223>  Synthetic Construct

<400>  198

```
Ala Val Thr Leu Asp Glu Ser Gly Gly Gly Leu Gln Thr Pro Gly Arg
1               5                   10                  15

Ala Leu Ser Leu Val Cys Lys Ala Ser Gly Phe Thr Phe Ser Arg Tyr
            20                  25                  30
```

```
Ala Met Tyr Trp Val Arg Gln Ala Pro Gly Lys Gly Leu Glu Phe Val
        35                  40                  45

Ala Gly Ile Gly Asn Thr Gly Arg Tyr Thr Gly Tyr Gly Ser Ala Val
    50                  55                  60

Lys Gly Arg Ala Thr Ile Ser Arg Asp Ser Gly Gln Ser Thr Val Arg
65                  70                  75                  80

Leu Gln Leu Asn Asn Leu Arg Ala Glu Asp Thr Gly Asn Tyr Tyr Cys
                85                  90                  95

Ala Lys Ser Val Ser Pro Tyr Cys Cys Asp Ala Ala Asp Ile Asp Ala
            100                 105                 110

Trp Gly His Gly Thr Glu Val Ile Val Ser Ser
        115                 120


<210>  199
<211>  318
<212>  DNA
<213>  artificial sequence

<220>
<223>  Nucleic Acid Sequence of Light Chain of Clone4


<220>
<221>  CDS
<222>  (1)..(318)

<400>  199
gct agc act cag ccg tcc tcg gtg tca gca aac cca gga gaa acc gtc        48
Ala Ser Thr Gln Pro Ser Ser Val Ser Ala Asn Pro Gly Glu Thr Val
1               5                   10                  15

aag atc acc tgc tcc ggg ggt agc agt ggc tat gct tat ggc tgg tac        96
Lys Ile Thr Cys Ser Gly Gly Ser Ser Gly Tyr Ala Tyr Gly Trp Tyr
            20                  25                  30

cag cag aag tct cct ggc agt gcc cct gtc act ctg ctc tat agc aac       144
Gln Gln Lys Ser Pro Gly Ser Ala Pro Val Thr Leu Leu Tyr Ser Asn
        35                  40                  45

aac aac aga ccc tcg gac atc cct tca cga ttc tcc ggt tcc aaa tcc       192
Asn Asn Arg Pro Ser Asp Ile Pro Ser Arg Phe Ser Gly Ser Lys Ser
    50                  55                  60

ggc tcc aca gcc aca tta acc atc act ggg gtc caa gcc gag gac gag       240
Gly Ser Thr Ala Thr Leu Thr Ile Thr Gly Val Gln Ala Glu Asp Glu
65                  70                  75                  80

gct gtc tat ttc tgt ggg agt gta gac agc agc agt tat gct ggt ata       288
Ala Val Tyr Phe Cys Gly Ser Val Asp Ser Ser Ser Tyr Ala Gly Ile
                85                  90                  95
```

```
ttt ggg gcc ggg aca acc ctg acc gtc cta                          318
Phe Gly Ala Gly Thr Thr Leu Thr Val Leu
            100                 105


<210>  200
<211>  106
<212>  PRT
<213>  artificial sequence

<220>
<223>  Synthetic Construct

<400>  200

Ala Ser Thr Gln Pro Ser Ser Val Ser Ala Asn Pro Gly Glu Thr Val
1               5                   10                  15


Lys Ile Thr Cys Ser Gly Gly Ser Ser Gly Tyr Ala Tyr Gly Trp Tyr
            20                  25                  30


Gln Gln Lys Ser Pro Gly Ser Ala Pro Val Thr Leu Leu Tyr Ser Asn
        35                  40                  45


Asn Asn Arg Pro Ser Asp Ile Pro Ser Arg Phe Ser Gly Ser Lys Ser
    50                  55                  60


Gly Ser Thr Ala Thr Leu Thr Ile Thr Gly Val Gln Ala Glu Asp Glu
65                  70                  75                  80


Ala Val Tyr Phe Cys Gly Ser Val Asp Ser Ser Ser Tyr Ala Gly Ile
                85                  90                  95


Phe Gly Ala Gly Thr Thr Leu Thr Val Leu
            100                 105


<210>  201
<211>  384
<212>  DNA
<213>  artificial sequence

<220>
<223>  Nucleic Acid Sequence of Heavy Chain of Clone18


<220>
<221>  CDS
<222>  (1)..(384)

<400>  201
gcg gtg acg ttg gac gag tcc ggg ggc ggc ctc cag acg ccc gga gga      48
Ala Val Thr Leu Asp Glu Ser Gly Gly Gly Leu Gln Thr Pro Gly Gly
1               5                   10                  15

acg ctc agc ctc gtc tgc aag gcc tcc ggg ttc gac ttc agc agc gtc      96
Thr Leu Ser Leu Val Cys Lys Ala Ser Gly Phe Asp Phe Ser Ser Val
```

```
            20                  25                  30

aac atg ttc tgg gtg cga cag gcg ccc ggc aag ggg ctg gag ttc gtc        144
Asn Met Phe Trp Val Arg Gln Ala Pro Gly Lys Gly Leu Glu Phe Val
        35                  40                  45

gct ggt att gat aac gat gct act ttc aca ctc tac ggg tcg gcg gtg        192
Ala Gly Ile Asp Asn Asp Ala Thr Phe Thr Leu Tyr Gly Ser Ala Val
    50                  55                  60

aag ggc cgt gcc tcc atc tcg agg gac aac ggg cag agc aca gtg agg        240
Lys Gly Arg Ala Ser Ile Ser Arg Asp Asn Gly Gln Ser Thr Val Arg
65                  70                  75                  80

ctg cag ctg aac aac ctc agg gct gag gac acc ggc acc tac tac tgc        288
Leu Gln Leu Asn Asn Leu Arg Ala Glu Asp Thr Gly Thr Tyr Tyr Cys
                85                  90                  95

gcc aaa act ctt tgt agt act act tgg ggt tgt ggt gct tat agt gct        336
Ala Lys Thr Leu Cys Ser Thr Thr Trp Gly Cys Gly Ala Tyr Ser Ala
            100                 105                 110

gga gat atc gac gca tgg ggc cac ggg acc gaa gtc atc gtc tcc tcc        384
Gly Asp Ile Asp Ala Trp Gly His Gly Thr Glu Val Ile Val Ser Ser
        115                 120                 125


<210>  202
<211>  128
<212>  PRT
<213>  artificial sequence

<220>
<223>  Synthetic Construct

<400>  202

Ala Val Thr Leu Asp Glu Ser Gly Gly Gly Leu Gln Thr Pro Gly Gly
1               5                   10                  15


Thr Leu Ser Leu Val Cys Lys Ala Ser Gly Phe Asp Phe Ser Ser Val
            20                  25                  30


Asn Met Phe Trp Val Arg Gln Ala Pro Gly Lys Gly Leu Glu Phe Val
        35                  40                  45


Ala Gly Ile Asp Asn Asp Ala Thr Phe Thr Leu Tyr Gly Ser Ala Val
    50                  55                  60


Lys Gly Arg Ala Ser Ile Ser Arg Asp Asn Gly Gln Ser Thr Val Arg
65                  70                  75                  80


Leu Gln Leu Asn Asn Leu Arg Ala Glu Asp Thr Gly Thr Tyr Tyr Cys
                85                  90                  95


Ala Lys Thr Leu Cys Ser Thr Thr Trp Gly Cys Gly Ala Tyr Ser Ala
            100                 105                 110
```

```
Gly Asp Ile Asp Ala Trp Gly His Gly Thr Glu Val Ile Val Ser Ser
        115                 120                 125


<210>  203
<211>  321
<212>  DNA
<213>  artificial sequence

<220>
<223>  Nucleic Acid Sequence of Light Chain of Clone 18


<220>
<221>  CDS
<222>  (1)..(321)

<400>  203
gct agc act cag ccg tcc tcg gtg tca gca aac cca ggg gaa act gtc       48
Ala Ser Thr Gln Pro Ser Ser Val Ser Ala Asn Pro Gly Glu Thr Val
1               5                   10                  15

aag atc acc tgt tcc ggg ggt ggc agc agt ggc tat ggt tac tat ggc       96
Lys Ile Thr Cys Ser Gly Gly Gly Ser Ser Gly Tyr Gly Tyr Tyr Gly
            20                  25                  30

tgg tat cag cag aag tca cct ggc agt gct cct gtc act gtg atc tat      144
Trp Tyr Gln Gln Lys Ser Pro Gly Ser Ala Pro Val Thr Val Ile Tyr
        35                  40                  45

aac aac aac aag aga ccc acg gac atc cct tca cga ttc tcc ggt gcc      192
Asn Asn Asn Lys Arg Pro Thr Asp Ile Pro Ser Arg Phe Ser Gly Ala
    50                  55                  60

ctc tct ggc tcc aca gcc aca tta acc atc act ggg gtc caa gtc gag      240
Leu Ser Gly Ser Thr Ala Thr Leu Thr Ile Thr Gly Val Gln Val Glu
65                  70                  75                  80

gac gag gct gtc tat tac tgt ggg agt agg gac aac act tat gtt ggt      288
Asp Glu Ala Val Tyr Tyr Cys Gly Ser Arg Asp Asn Thr Tyr Val Gly
                85                  90                  95

ata ttt ggg gcc ggg aca acc ctg acc gtc cta                          321
Ile Phe Gly Ala Gly Thr Thr Leu Thr Val Leu
            100                 105


<210>  204
<211>  107
<212>  PRT
<213>  artificial sequence

<220>
<223>  Synthetic Construct

<400>  204

Ala Ser Thr Gln Pro Ser Ser Val Ser Ala Asn Pro Gly Glu Thr Val
1               5                   10                  15
```

```
Lys Ile Thr Cys Ser Gly Gly Gly Ser Ser Gly Tyr Gly Tyr Tyr Gly
            20                  25                  30

Trp Tyr Gln Gln Lys Ser Pro Gly Ser Ala Pro Val Thr Val Ile Tyr
        35                  40                  45

Asn Asn Asn Lys Arg Pro Thr Asp Ile Pro Ser Arg Phe Ser Gly Ala
    50                  55                  60

Leu Ser Gly Ser Thr Ala Thr Leu Thr Ile Thr Gly Val Gln Val Glu
65                  70                  75                  80

Asp Glu Ala Val Tyr Tyr Cys Gly Ser Arg Asp Asn Thr Tyr Val Gly
                85                  90                  95

Ile Phe Gly Ala Gly Thr Thr Leu Thr Val Leu
            100                 105
```

## Description

[Technical Field]

The present invention relates to an antibody that specifically binds to Glypican-1 (GPC-1), and related technology, method, agent, and the like.

[Background Art]

It was found that Glypican-1 molecules are significantly more strongly expressed in esophageal cancer cells than in normal cells and can be used as tumor markers (Patent Literature 1). Antibodies that specifically bind to Glypican-1 have also been isolated (Patent Literature 1). However, an effective therapeutic drug that targets Glypican-1 has not yet been found.

[Citation List]

[Patent Literature]

[PTL 1] International Publication No. WO 2015/098112

[Summary of Invention]

[Solution to Problem]

In one aspect, the present invention provides an anti-gypican-1 antibody having intracellular invasion activity. Such activity was not found in conventional anti-Glypican-1 antibodies. The present invention can be used in various therapeutic applications that were inconceivable with conventional art by utilizing intracellular invasion activity of the antibody of the invention.

Thus, in another aspect, the present invention also provides a composition for preventing or treating Glypican-1 positive cancer, comprising a complex of a substance that binds to Glypican-1 (e.g., anti-Glypican-1 antibody) and an agent having cytotoxic activity.

In another aspect, the present invention provides an anti-Glypican-1 antibody having a binding activity that is stronger than conventional antibodies, or a fragment thereof. Such an antibody or fragment thereof is useful as a diagnostic agent for cancer or the like.

Such an antibody or a fragment thereof is also applicable as a companion diagnostic drug, companion therapeutic drug, and the like.

In view of the above, for example, the present invention provides the following items.

<Antibody>

(Item 1A)

An anti-human Glypican-1 antibody or antigen binding fragment thereof, wherein the antibody is selected from the group consisting of:

(a) an antibody comprising amino acid sequences of heavy chain CDR1, heavy chain CDR2, and heavy chain CDR3 set forth in SEQ ID NOs: 53, 54, and 55, respectively, and light chain CDR1, light chain CDR2, and light chain CDR3 set forth in SEQ ID NOs: 56, 57, and 58, respectively;
(b) an antibody comprising amino acid sequences of heavy chain CDR1, heavy chain CDR2, and heavy chain CDR3 set forth in SEQ ID NOs: 5, 6, and 7, respectively, and light chain CDR1, light chain CDR2, and light chain CDR3 set forth in SEQ ID NOs: 8, 9, and 10, respectively;
(c) an antibody comprising amino acid sequences of heavy chain CDR1, heavy chain CDR2, and heavy chain CDR3 set forth in SEQ ID NOs: 11, 12, and 13, respectively, and light chain CDR1, light chain CDR2, and light chain CDR3 set forth in SEQ ID NOs: 14, 15, and 16, respectively;
(d) an antibody comprising amino acid sequences of heavy chain CDR1, heavy chain CDR2, and heavy chain CDR3 set forth in SEQ ID NOs: 17, 18, and 19, respectively, and light chain CDR1, light chain CDR2, and light chain CDR3 set forth in SEQ ID NOs: 20, 21, and 22, respectively;
(e) an antibody comprising amino acid sequences of heavy chain CDR1, heavy chain CDR2, and heavy chain CDR3

set forth in SEQ ID NOs: 23, 24, and 25, respectively, and light chain CDR1, light chain CDR2, and light chain CDR3 set forth in SEQ ID NOs: 26, 27, and 28, respectively;
(f) an antibody comprising amino acid sequences of heavy chain CDR1, heavy chain CDR2, and heavy chain CDR3 set forth in SEQ ID NOs: 29, 30, and 31, respectively, and light chain CDR1, light chain CDR2, and light chain CDR3 set forth in SEQ ID NOs: 32, 33, and 34, respectively;
(g) an antibody comprising amino acid sequences of heavy chain CDR1, heavy chain CDR2, and heavy chain CDR3 set forth in SEQ ID NOs: 35, 36, and 37, respectively, and light chain CDR1, light chain CDR2, and light chain CDR3 set forth in SEQ ID NOs: 38, 39, and 40, respectively;
(h) an antibody comprising amino acid sequences of heavy chain CDR1, heavy chain CDR2, and heavy chain CDR3 set forth in SEQ ID NOs: 41, 42, and 43, respectively, and light chain CDR1, light chain CDR2, and light chain CDR3 set forth in SEQ ID NOs: 44, 45, and 46, respectively;
(i) an antibody comprising amino acid sequences of heavy chain CDR1, heavy chain CDR2, and heavy chain CDR3 set forth in SEQ ID NOs: 47, 48, and 49, respectively, and light chain CDR1, light chain CDR2, and light chain CDR3 set forth in SEQ ID NOs: 50, 51, and 52, respectively;
(j) an antibody comprising amino acid sequences of heavy chain CDR1, heavy chain CDR2, and heavy chain CDR3 set forth in SEQ ID NOs: 59, 60, and 61, respectively, and light chain CDR1, light chain CDR2, and light chain CDR3 set forth in SEQ ID NOs: 62, 63, and 64, respectively;
(k) an antibody comprising amino acid sequences of heavy chain CDR1, heavy chain CDR2, and heavy chain CDR3 set forth in SEQ ID NOs: 65, 66, and 67, respectively, and light chain CDR1, light chain CDR2, and light chain CDR3 set forth in SEQ ID NOs: 68, 69, and 70, respectively;
(l) an antibody comprising amino acid sequences of heavy chain CDR1, heavy chain CDR2, and heavy chain CDR3 set forth in SEQ ID NOs: 71, 72, and 73, respectively, and light chain CDR1, light chain CDR2, and light chain CDR3 set forth in SEQ ID NOs: 74, 75, and 76, respectively;
(m) an antibody comprising amino acid sequences of heavy chain CDR1, heavy chain CDR2, and heavy chain CDR3 set forth in SEQ ID NOs: 77, 78, and 79, respectively, and light chain CDR1, light chain CDR2, and light chain CDR3 set forth in SEQ ID NOs: 80, 81, and 82, respectively;
(n) an antibody comprising amino acid sequences of heavy chain CDR1, heavy chain CDR2, and heavy chain CDR3 set forth in SEQ ID NOs: 83, 84, and 85, respectively, and light chain CDR1, light chain CDR2, and light chain CDR3 set forth in SEQ ID NOs: 86, 87, and 88, respectively;
(o) an antibody comprising amino acid sequences of heavy chain CDR1, heavy chain CDR2, and heavy chain CDR3 set forth in SEQ ID NOs: 89, 90, and 91, respectively, and light chain CDR1, light chain CDR2, and light chain CDR3 set forth in SEQ ID NOs: 92, 93, and 94, respectively;
(p) an antibody comprising amino acid sequences of heavy chain CDR1, heavy chain CDR2, and heavy chain CDR3 set forth in SEQ ID NOs: 95, 96, and 97, respectively, and light chain CDR1, light chain CDR2, and light chain CDR3 set forth in SEQ ID NOs: 98, 99, and 100, respectively;
(q) an antibody comprising amino acid sequences of heavy chain CDR1, heavy chain CDR2, and heavy chain CDR3 set forth in SEQ ID NOs: 101, 102, and 103, respectively, and light chain CDR1, light chain CDR2, and light chain CDR3 set forth in SEQ ID NOs: 104, 105, and 106, respectively;
(r) an antibody comprising amino acid sequences of heavy chain CDR1, heavy chain CDR2, and heavy chain CDR3 set forth in SEQ ID NOs: 107, 108, and 109, respectively, and light chain CDR1, light chain CDR2, and light chain CDR3 set forth in SEQ ID NOs: 110, 111, and 112, respectively; and
(s) a mutant of an antibody selected from (a) to (r) comprising at least one substitution, addition, or deletion in a CDR moiety.

(Item 1A-1)

The antibody or antigen binding fragment thereof of item 1A, wherein the antibody is selected from the group consisting of (a), (b), (d), (e), (g), (h), (i), (j), (k), (1), (m), and (n).

(Item 2A)

The antibody or antigen binding fragment thereof of item 1A, wherein the antibody is selected from the group consisting of:

(a) an antibody comprising amino acid sequences of a heavy chain set forth in SEQ ID NO: 158 and a light chain set forth in SEQ ID NO: 160;
(b) an antibody comprising amino acid sequences of a heavy chain set forth in SEQ ID NO: 126 and a light chain set forth in SEQ ID NO: 128;

(c) an antibody comprising amino acid sequences of a heavy chain set forth in SEQ ID NO: 130 and a light chain set forth in SEQ ID NO: 132;
(d) an antibody comprising amino acid sequences of a heavy chain set forth in SEQ ID NO: 134 and a light chain set forth in SEQ ID NO: 136;
(e) an antibody comprising amino acid sequences of a heavy chain set forth in SEQ ID NO: 138 and a light chain set forth in SEQ ID NO: 140;
(f) an antibody comprising amino acid sequences of a heavy chain set forth in SEQ ID NO: 142 and a light chain set forth in SEQ ID NO: 144;
(g) an antibody comprising amino acid sequences of a heavy chain set forth in SEQ ID NO: 146 and a light chain set forth in SEQ ID NO: 148;
(h) an antibody comprising amino acid sequences of a heavy chain set forth in SEQ ID NO: 150 and a light chain set forth in SEQ ID NO: 152;
(i) an antibody comprising amino acid sequences of a heavy chain set forth in SEQ ID NO: 154 and a light chain set forth in SEQ ID NO: 156;
(j) an antibody comprising amino acid sequences of a heavy chain set forth in SEQ ID NO: 162 and a light chain set forth in SEQ ID NO: 164;
(k) an antibody comprising amino acid sequences of a heavy chain set forth in SEQ ID NO: 166 and a light chain set forth in SEQ ID NO: 168;
(l) an antibody comprising amino acid sequences of a heavy chain set forth in SEQ ID NO: 170 and a light chain set forth in SEQ ID NO: 172;
(m) an antibody comprising amino acid sequences of a heavy chain set forth in SEQ ID NO: 174 and a light chain set forth in SEQ ID NO: 176;
(n) an antibody comprising amino acid sequences of a heavy chain set forth in SEQ ID NO: 178 and a light chain set forth in SEQ ID NO: 180;
(o) an antibody comprising amino acid sequences of a heavy chain set forth in SEQ ID NO: 182 and a light chain set forth in SEQ ID NO: 184;
(p) an antibody comprising amino acid sequences of a heavy chain set forth in SEQ ID NO: 186 and a light chain set forth in SEQ ID NO: 188;
(q) an antibody comprising amino acid sequences of a heavy chain set forth in SEQ ID NO: 190 and a light chain set forth in SEQ ID NO: 192;
(r) an antibody comprising amino acid sequences of a heavy chain set forth in SEQ ID NO: 194 and a light chain set forth in SEQ ID NO: 196; and
(s) a mutant of an antibody selected from (a) to (r) comprising at least one substitution, addition, or deletion.

(Item 2A-1)

The antibody or antigen binding fragment thereof of item 2A, wherein the antibody is selected from the group consisting of (a), (b), (d), (e), (g), (h), (i), (j), (k), (l), (m), and (n).

(Item 3A)

The antibody or antigen binding fragment thereof of item 1A or 2A, wherein the mutant comprises at least one substitution, addition, or deletion in a framework of the antibody.

(Item 4A)

The antibody or antigen binding fragment thereof of any one of items 1A to 3A, which binds to human Glypican-1 at a binding constant of about 10 nM or less.

(Item 5A)

The antibody or antigen binding fragment thereof of any one of items 1A to 4A, having an internalization activity of about 30% or greater with respect to Glypican-1 positive cells after 6 hours from starting incubation with the antibody or antigen fragment thereof in an internalization assay.

(Item 5A-1)

The antibody or antigen binding fragment thereof of any one of items 1A to 4A, having an internalization activity

of about 30% or greater with respect to Glypican-1 positive cells after 2 hours from starting incubation with the antibody or antigen fragment thereof in an internalization assay.

(Item 6A)

The antibody or antigen binding fragment thereof of any one of items 1A to 5A, having an internalization activity of about 50% or greater with respect to Glypican-1 positive cells after 6 hours from starting incubation with the antibody or antigen fragment thereof in an internalization assay.

(Item 6A-1)

The antibody or antigen binding fragment thereof of any one of items 1A to 4A and 5A-1, having an internalization activity of about 50% or greater with respect to Glypican-1 positive cells after 2 hours from starting incubation with the antibody or antigen fragment thereof in an internalization assay.

(Item 7A)

The antibody or antigen binding fragment thereof of any one of items 1A to 6A, having an internalization activity of about 60% or greater with respect to Glypican-1 positive cells after 6 hours from starting incubation with the antibody or antigen fragment thereof in an internalization assay.

(Item 7A-1)

The antibody or antigen binding fragment thereof of any one of items 1A to 4A, 5A-1, and 6A-1 having an internalization activity of about 60% or greater with respect to Glypican-1 positive cells after 2 hours from starting incubation with the antibody or antigen fragment thereof in an internalization assay.

(Item 8A)

The antibody or antigen binding fragment thereof of any one of items 1A to 7A, wherein the antibody is an antibody selected from a monoclonal antibody, a polyclonal antibody, a chimeric antibody, a humanized antibody, a human antibody, a multifunctional antibody, a bispecific or oligospecific antibody, a single chain antibody, an scFV, a diabody, an sc(Fv)2 (single chain (Fv)2), and an scFv-Fc.

(Item 9A)

A pharmaceutical composition comprising the antibody or antigen binding fragment thereof of any one of items 1A to 8A.

(Item 10A)

The pharmaceutical composition of item 9A, wherein the pharmaceutical composition is for intracellular invasion of an active ingredient.

<Complex>

(Item 1B)

A complex of an anti-Glypican-1 antibody having activity for intracellular invasion into Glypican-1 positive cells or antigen binding fragment thereof and an agent having cytotoxic activity.

(Item 2B)

The complex of item 1B, wherein a substance that binds to the Glypican-1 is operably linked to the agent having cytotoxic activity via a linker.

(Item 3B)

The complex of item 1B or 2B, wherein an epitope of the antibody comprises:

(a) positions 33 to 61 of SEQ ID NO: 2;
(b) positions 339 to 358 and/or 388 to 421 of SEQ ID NO: 2;
(c) positions 430 to 530 of SEQ ID NO: 2;
(d) positions 33 to 61, 339 to 358, and/or 388 to 421 of SEQ ID NO: 2;
(e) positions 339 to 358, 388 to 421, and/or 430 to 530 of SEQ ID NO: 2; or
(f) positions 33 to 61, 339 to 358, 388 to 421, and/or 430 to 530 of SEQ ID NO: 2.

(Item 4B)

The complex of item 3B, wherein the epitope of the antibody comprises:

(a) positions 33 to 61 of SEQ ID NO: 2;
(b) positions 339 to 358 and 388 to 421 of SEQ ID NO: 2;
(c) positions 33 to 61, 339 to 358, and 388 to 421 of SEQ ID NO: 2; or
(d) positions 33 to 61, 339 to 358, 388 to 421, and 430 to 530 of SEQ ID NO: 2.

(Item 5B)

The complex of any one of items 1B to 4B, wherein the antibody is selected from the group consisting of:

(a) an antibody comprising amino acid sequences of heavy chain CDR1, heavy chain CDR2, and heavy chain CDR3 set forth in SEQ ID NOs: 53, 54, and 55, respectively, and light chain CDR1, light chain CDR2, and light chain CDR3 set forth in SEQ ID NOs: 56, 57, and 58, respectively;
(b) an antibody comprising amino acid sequences of heavy chain CDR1, heavy chain CDR2, and heavy chain CDR3 set forth in SEQ ID NOs: 5, 6, and 7, respectively, and light chain CDR1, light chain CDR2, and light chain CDR3 set forth in SEQ ID NOs: 8, 9, and 10, respectively;
(c) an antibody comprising amino acid sequences of heavy chain CDR1, heavy chain CDR2, and heavy chain CDR3 set forth in SEQ ID NOs: 11, 12, and 13, respectively, and light chain CDR1, light chain CDR2, and light chain CDR3 set forth in SEQ ID NOs: 14, 15, and 16, respectively;
(d) an antibody comprising amino acid sequences of heavy chain CDR1, heavy chain CDR2, and heavy chain CDR3 set forth in SEQ ID NOs: 17, 18, and 19, respectively, and light chain CDR1, light chain CDR2, and light chain CDR3 set forth in SEQ ID NOs: 20, 21, and 22, respectively;
(e) an antibody comprising amino acid sequences of heavy chain CDR1, heavy chain CDR2, and heavy chain CDR3 set forth in SEQ ID NOs: 23, 24, and 25, respectively, and light chain CDR1, light chain CDR2, and light chain CDR3 set forth in SEQ ID NOs: 26, 27, and 28, respectively;
(f) an antibody comprising amino acid sequences of heavy chain CDR1, heavy chain CDR2, and heavy chain CDR3 set forth in SEQ ID NOs: 29, 30, and 31, respectively, and light chain CDR1, light chain CDR2, and light chain CDR3 set forth in SEQ ID NOs: 32, 33, and 34, respectively;
(g) an antibody comprising amino acid sequences of heavy chain CDR1, heavy chain CDR2, and heavy chain CDR3 set forth in SEQ ID NOs: 35, 36, and 37, respectively, and light chain CDR1, light chain CDR2, and light chain CDR3 set forth in SEQ ID NOs: 38, 39, and 40, respectively;
(h) an antibody comprising amino acid sequences of heavy chain CDR1, heavy chain CDR2, and heavy chain CDR3 set forth in SEQ ID NOs: 41, 42, and 43, respectively, and light chain CDR1, light chain CDR2, and light chain CDR3 set forth in SEQ ID NOs: 44, 45, and 46, respectively;
(i) an antibody comprising amino acid sequences of heavy chain CDR1, heavy chain CDR2, and heavy chain CDR3 set forth in SEQ ID NOs: 47, 48, and 49, respectively, and light chain CDR1, light chain CDR2, and light chain CDR3 set forth in SEQ ID NOs: 50, 51, and 52, respectively;
(j) an antibody comprising amino acid sequences of heavy chain CDR1, heavy chain CDR2, and heavy chain CDR3 set forth in SEQ ID NOs: 59, 60, and 61, respectively, and light chain CDR1, light chain CDR2, and light chain CDR3 set forth in SEQ ID NOs: 62, 63, and 64, respectively;
(k) an antibody comprising amino acid sequences of heavy chain CDR1, heavy chain CDR2, and heavy chain CDR3 set forth in SEQ ID NOs: 65, 66, and 67, respectively, and light chain CDR1, light chain CDR2, and light chain CDR3 set forth in SEQ ID NOs: 68, 69, and 70, respectively;
(l) an antibody comprising amino acid sequences of heavy chain CDR1, heavy chain CDR2, and heavy chain CDR3

set forth in SEQ ID NOs: 71, 72, and 73, respectively, and light chain CDR1, light chain CDR2, and light chain CDR3 set forth in SEQ ID NOs: 74, 75, and 76, respectively;
(m) an antibody comprising amino acid sequences of heavy chain CDR1, heavy chain CDR2, and heavy chain CDR3 set forth in SEQ ID NOs: 77, 78, and 79, respectively, and light chain CDR1, light chain CDR2, and light chain CDR3 set forth in SEQ ID NOs: 80, 81, and 82, respectively;
(n) an antibody comprising amino acid sequences of heavy chain CDR1, heavy chain CDR2, and heavy chain CDR3 set forth in SEQ ID NOs: 83, 84, and 85, respectively, and light chain CDR1, light chain CDR2, and light chain CDR3 set forth in SEQ ID NOs: 86, 87, and 88, respectively;
(o) an antibody comprising amino acid sequences of heavy chain CDR1, heavy chain CDR2, and heavy chain CDR3 set forth in SEQ ID NOs: 89, 90, and 91, respectively, and light chain CDR1, light chain CDR2, and light chain CDR3 set forth in SEQ ID NOs: 92, 93, and 94, respectively;
(p) an antibody comprising amino acid sequences of heavy chain CDR1, heavy chain CDR2, and heavy chain CDR3 set forth in SEQ ID NOs: 95, 96, and 97, respectively, and light chain CDR1, light chain CDR2, and light chain CDR3 set forth in SEQ ID NOs: 98, 99, and 100, respectively;
(q) an antibody comprising amino acid sequences of heavy chain CDR1, heavy chain CDR2, and heavy chain CDR3 set forth in SEQ ID NOs: 101, 102, and 103, respectively, and light chain CDR1, light chain CDR2, and light chain CDR3 set forth in SEQ ID NOs: 104, 105, and 106, respectively;
(r) an antibody comprising amino acid sequences of heavy chain CDR1, heavy chain CDR2, and heavy chain CDR3 set forth in SEQ ID NOs: 107, 108, and 109, respectively, and light chain CDR1, light chain CDR2, and light chain CDR3 set forth in SEQ ID NOs: 110, 111, and 112, respectively;
(s) an antibody comprising amino acid sequences of heavy chain CDR1, heavy chain CDR2, and heavy chain CDR3 set forth in SEQ ID NOs: 113, 114, and 115, respectively, and light chain CDR1, light chain CDR2, and light chain CDR3 set forth in SEQ ID NOs: 116, 117, and 118, respectively;
(t) an antibody comprising amino acid sequences of heavy chain CDR1, heavy chain CDR2, and heavy chain CDR3 set forth in SEQ ID NOs: 125, 126, and 127, respectively, and light chain CDR1, light chain CDR2, and light chain CDR3 set forth in SEQ ID NOs: 128, 129, and 130, respectively; and
(u) a mutant of an antibody selected from (a) to (t) comprising at least one substitution, addition, or deletion.

(Item 5B-1)

The complex of any one of item 5B, wherein the antibody is selected from the group consisting of (a), (b), (d), (e), (g), (h), (i), (j), (k), (1), (m), (n), (s), and (t).

(Item 6B)

The complex of any one of item 5B, wherein the antibody is selected from the group consisting of:

(a) an antibody comprising amino acid sequences of a heavy chain set forth in SEQ ID NO: 158 and a light chain set forth in SEQ ID NO: 160;
(b) an antibody comprising amino acid sequences of a heavy chain set forth in SEQ ID NO: 126 and a light chain set forth in SEQ ID NO: 128;
(c) an antibody comprising amino acid sequences of a heavy chain set forth in SEQ ID NO: 130 and a light chain set forth in SEQ ID NO: 132;
(d) an antibody comprising amino acid sequences of a heavy chain set forth in SEQ ID NO: 134 and a light chain set forth in SEQ ID NO: 136;
(e) an antibody comprising amino acid sequences of a heavy chain set forth in SEQ ID NO: 138 and a light chain set forth in SEQ ID NO: 140;
(f) an antibody comprising amino acid sequences of a heavy chain set forth in SEQ ID NO: 142 and a light chain set forth in SEQ ID NO: 144;
(g) an antibody comprising amino acid sequences of a heavy chain set forth in SEQ ID NO: 146 and a light chain set forth in SEQ ID NO: 148;
(h) an antibody comprising amino acid sequences of a heavy chain set forth in SEQ ID NO: 150 and a light chain set forth in SEQ ID NO: 152;
(i) an antibody comprising amino acid sequences of a heavy chain set forth in SEQ ID NO: 154 and a light chain set forth in SEQ ID NO: 156;
(j) an antibody comprising amino acid sequences of a heavy chain set forth in SEQ ID NO: 162 and a light chain set forth in SEQ ID NO: 164;
(k) an antibody comprising amino acid sequences of a heavy chain set forth in SEQ ID NO: 166 and a light chain

set forth in SEQ ID NO: 168;
(l) an antibody comprising amino acid sequences of a heavy chain set forth in SEQ ID NO: 170 and a light chain set forth in SEQ ID NO: 172;
(m) an antibody comprising amino acid sequences of a heavy chain set forth in SEQ ID NO: 174 and a light chain set forth in SEQ ID NO: 176;
(n) an antibody comprising amino acid sequences of a heavy chain set forth in SEQ ID NO: 178 and a light chain set forth in SEQ ID NO: 180;
(o) an antibody comprising amino acid sequences of a heavy chain set forth in SEQ ID NO: 182 and a light chain set forth in SEQ ID NO: 184;
(p) an antibody comprising amino acid sequences of a heavy chain set forth in SEQ ID NO: 186 and a light chain set forth in SEQ ID NO: 188;
(q) an antibody comprising amino acid sequences of a heavy chain set forth in SEQ ID NO: 190 and a light chain set forth in SEQ ID NO: 192;
(r) an antibody comprising amino acid sequences of a heavy chain set forth in SEQ ID NO: 194 and a light chain set forth in SEQ ID NO: 196;
(s) an antibody comprising amino acid sequences of a heavy chain set forth in SEQ ID NO: 198 and a light chain set forth in SEQ ID NO: 200;
(t) an antibody comprising amino acid sequences of a heavy chain set forth in SEQ ID NO: 202 and a light chain set forth in SEQ ID NO: 204; and
(u) a mutant of an antibody selected from (a) to (t) comprising at least one substitution, addition, or deletion.

(Item 6B-1)

The complex of any one of item 6B, wherein the antibody is selected from the group consisting of (a), (b), (d), (e), (g), (h), (i), (j), (k), (1), (m), (n), (s), and (t).

(Item 7B)

The complex of any one of items 1B to 6B, wherein the complex exhibits an $IC_{50}$ of about 0.5 nM or less in Glypican-1 positive cells.

<Composition>

(Item 1C)

A composition for preventing or treating Glypican-1 positive cancer, comprising the complex of any one of items 1B to 7B.

(Item 2C)

The composition of item 1C, wherein cancer cells with the Glypican-1 positive cancer express a high level of glypican-1 on a cell surface.

(Item 3C)

The composition of item 2C, wherein the cancer cells with the Glypican-1 positive cancer have an anti-Glypican-1 antibody binding capacity of about 15000 or greater in an assay using QIFIKIT®.

(Item 4C)

The composition of item 3C, wherein the Glypican-1 positive cancer is selected from esophageal cancer, pancreatic cancer, cervical cancer, lung cancer, head and neck cancer, breast cancer, uterine leiomyosarcoma, prostate cancer, and any combination thereof.

(Item 5C)

The composition of item 3C or 4C, wherein the Glypican-1 positive cancer is esophageal cancer, which comprises esophageal cancer at a lymph node metastasis site, squamous cell carcinoma, and/or adenocarcinoma.

(Item 6C)

The composition of any one of item 5C, wherein the esophageal cancer comprises squamous cell carcinoma.

<Detection agent and diagnostic method>

(Item 1D)

A detection agent for identifying esophageal cancer, comprising the antibody or fragment thereof of any one of items 1A to 8A.

(Item 2D)

The detection agent of item 1D, wherein the antibody or fragment thereof is labeled.

(Item 3D)

The detection agent of item 1D or 2D, wherein the esophageal cancer is Glypican-1 positive.

(Item 4D)

The detection agent of any one of items 1D to 3D, wherein the esophageal cancer comprises esophageal cancer at a lymph node metastasis site, squamous cell carcinoma, and/or adenocarcinoma.

(Item 5D)

The detection agent of any one of items 1D to 4D, wherein the esophageal cancer comprises squamous cell carcinoma.

(Item 6D)

The detection agent of any one of items 1D to 5D for administration to a patient determined to have developed Glypican-1 positive esophageal cancer.

(Item 7D)

A method of using expression of Glypican-1 in a target sample as an indicator of esophageal cancer, the method comprising:

contacting the detection agent of any one of items 1D to 6D with the target sample;
measuring an amount of expression of Glypican-1 in the target sample; and
comparing amounts of expression of Glypican-1 in the target sample and a normal sample.

(Item 8D)

A diagnostic drug for determining whether a subject is in need of therapy of esophageal cancer, comprising the antibody or antigen binding fragment thereof of any one of items 1A to 8A.

<Companion reagent>

(Item 1E)

A companion reagent for determining whether a subject is in need of cancer therapy with a Glypican-1 inhibitor, comprising the antibody or antigen binding fragment thereof of any one of items 1A to 8A or the detection agent of any one of items 1D to 6D, wherein the reagent is contacted with a target sample, and an amount of expression of Glypican-1 in the target sample is measured, wherein the amount of expression of Glypican-1 in the target sample exceeding an amount of expression of Glypican-1 in a normal sample indicates that the target is in need of therapy with a Glypican-1 inhibitor.

(Item 2E)

A composition for preventing or treating malignant tumor comprising the complex of any one of items 1B to 7B, wherein a subject having the malignant tumor has a higher expression of Glypican-1 than a normal individual.

<Syngenic nonhuman animals>

(Item 1F)

A nonhuman animal to which cells that express a cancer antigen are grafted, wherein the cancer antigen is syngenic with the nonhuman animal.

(Item 2F)

The nonhuman animal of item 1F, wherein the cancer antigen is Glypican-1.

(Item 3F)

The nonhuman animal of item 1F or 2F, wherein the cells are selected from the group consisting of an LLC cell strain, a 4T1 cell strain, an MH-1 cell strain, a CT26 cell strain, an MC38 cell strain, and a B1610 cell strain, which have been modified to express Glypican-1.

(Item 4F)

The nonhuman animal of any one of items 1F to 3F, wherein the nonhuman animal is a mouse, and the cells are of an LLC cell strain modified to express mouse Glypican-1.

The present invention is intended so that one or more of the features described above can be provided not only as the explicitly disclosed combinations, but also as other combinations thereof. Additional embodiments and advantages of the present invention are recognized by those skilled in the art by reading and understanding the following detailed description as needed.

[Advantageous Effects of Invention]

The present invention provides an antibody that binds to Glypican-1 with better specificity than conventional antibodies. It was found that such an antibody can be utilized as a detection agent used for diagnosis/companion therapy of esophageal cancer, and a complex of the antibody and a drug is very effective in treating esophageal cancer. Surprisingly, it was found that a complex of the antibody and a drug is effective on Glypican-1 positive cancer such as pancreatic cancer and cervical cancer in addition to esophageal cancer.

[Brief Description of Drawings]

[Figure 1] Figure **1** depicts a schematic diagram of an ADC used in an ADC assay using an anticancer agent binding secondary antibody.

[Figure 2] Figure **2** shows results of anticancer agent sensitivity of TE14 cells. The vertical axis indicates the ratio of the viable cell count with respect to the control, and the horizontal axis indicates the concentration of anticancer agent.

[Figure 3] Figure **3** shows a table summarizing ADC IC50, $K_D$ measured by Biacore, $K_D$ measured by FACS, mGPC1 intersection, and epitope of each clone.

[Figure 4] Figure **4** shows results of ADC assays in LK2, TE8, and TE14 cells.

[Figure 5] Figure **5** shows a table summarizing the results obtained in Figure **4.**

[Figure 6] Figure **6** depicts a schematic diagram of a structure of an exemplary antibody-drug conjugate (ADC).

[Figure 7] Figure **7** shows results of analyzing binding affinity between 01a033 and 01a033 ADC's Glypican-1. The vertical axis indicates OD (450 nm to 630 nm) and the horizontal axis indicates the antibody concentration.

[Figure 8] Figure **8** shows the GPC1 antibody binding capacity of esophageal cancer cell strain TE4, TE5, TE6, TE8, TE9, TE10, TE11, TE11, TE14, and TE15 cells.

[Figure 9] Figure **9** shows data from analyzing the expression of GPC1 in various cancer cell strains by FACS. The

figure shows the reactivity of an isotype control antibody and the reactivity of a clone 01a033 antibody. The arrows indicate the reactivity of a clone 01a033 antibody.

[Figure 10] Figure **10** shows results of an internalization assay. The results were measured at 0, 1, 2, 3, 4, 6, 12, and 24 hours. The cell strain used was TE8.

[Figure 11] Figure **11** shows % internalized at 4°C or 37°C at 0, 1, 2, 3, 4, 6, 12, and 24 hours of 01a033. The vertical axis indicates % internalized and the horizontal axis indicates time.

[Figure 12] Figure **12** shows results of cell growth suppression effects of GPC1 ADC on TE4, TE5, TE6, TE8, TE9, TE10, TE11, TE11, TE14, TE15, and LK2 cells. The vertical axis of each graph indicates the growth rate compared to no treatment (%), and the horizontal axis indicates the antibody concentration.

[Figure 13] Figure **13** shows results of cell growth suppression effects of GPC1 ADC on BxPC3, HeLa, T3M4, and ME180 cells. The vertical axis of each graph indicates the growth rate compared to no treatment (%), and the horizontal axis indicates the antibody concentration.

[Figure 14] Figure **14** is a table summarizing IC50 deduced from the results obtained in Figures **12** and **13.**

[Figure 15] Figure **15** shows the rate of change in the body weight obtained by a single dose toxicity test. The vertical axis indicates the rate of change in the body weight, and the horizontal axis indicates the number of days after treatment.

[Figure 16] Figure **16** shows results of hematological inspection in a safety test for a single dose. White blood cells (WBC), hemoglobin (Hb), and platelets (Plt) were evaluated.

[Figure 17] Figure **17** shows results of hematological inspection in a safety test for a single dose. Alanine aminotransferase (ALT), amylase (Amy), and chromium (Cr) were evaluated.

[Figure 18] Figure **18** schematically depicts an in vivo efficacy test of GPC1 ADC using a pancreatic cancer cell strain. The error bar indicates mean ± SEM.

[Figure 19] Figure **19** shows results of antitumor effect in vivo of GPC1 ADC using a pancreatic cancer cell strain. The vertical axis indicates the tumor volume ($mm^3$), and the horizontal axis indicates the number of days after treatment.

[Figure 20] Figure **20** shows the tumor weight (mg) on day 36 after treatment in an in vivo efficacy test of GPC1 ADC using a pancreatic cancer cell strain. The p value is determined by Scheffe's method following one-way ANOVA.

[Figure 21] Figure **21** shows the rate of change in the body weight of a mouse in an in vivo efficacy test of GPC1 ADC using a pancreatic cancer cell strain. The vertical axis indicates the rate of change in the body weight, and the horizontal axis indicates the number of days after treatment. The error bar indicates mean ± SEM.

[Figure 22] Figure **22** indicates results of an immunohistochemical staining of tumor tissue with administration of a GPC1 ADC. After BxPC3 subcutaneous tumorigenesis, (1) PBS, (2) 10 mg/kg of control ADC, and (3) 1 mg/kg, 3 mg/kg, and 10 mg/kg of GPC1-ADC were administered to the tail vein once, and the tumor was extracted after 24 hours. The black dots indicate cells arrested at the G2/M phase.

[Figure 23] Figure **23** shows results of analysis of the GPC1 expression by immunohistochemical staining in BxPC3 and PDX graft models. After deparaffinization of a section of a paraffin embedded tissue, the section was dehydrated with alcohol. Immunohistochemical staining on GPC1 was performed by using an anti-GPC1 antibody (Atlas Antibodies: HPA030571) and ChemMate Envision kit HRP 500T (Dako: K5007).

[Figure 24] Figure **24** schematically depicts an in vivo efficacy test on GPC1 ADC using pancreatic cancer PDX.

[Figure 25] Figure **25** shows results of an anti-tumor effect in vivo of GPC1 ADC using pancreatic cancer PDX. The vertical axis indicates the tumor volume ($mm^3$), and the horizontal axis indicates the number of days after treatment. The error bar indicates mean ± SEM.

[Figure 26] Figure **26** shows the tumor weight (mg) on day 28 after treatment in an in vivo efficacy test of GPC1 ADC using pancreatic cancer PDX. The p value is determined by Scheffe's method following one-way ANOVA.

[Figure 27] Figure **27** shows the rate of change in the body weight of a mouse in an in vivo efficacy test of GPC1 ADC using pancreatic cancer PDX. The vertical axis indicates the rate of change in the body weight, and the horizontal axis indicates the number of days after treatment. The error bar indicates mean ± SEM.

[Figure 28] Figure **28** schematically depicts an in vivo efficacy test on GPC1 ADC using a cervical cancer cell strain.

[Figure 29] Figure **29** shows results of an antitumor effect in vivo of GPC1 ADC using a cervical cancer cell strain. The vertical axis indicates the tumor volume ($mm^3$), and the horizontal axis indicates the number of days after treatment. The error bar indicates mean ± SEM.

[Figure 30] Figure **30** shows the tumor weight (mg) on day 32 after treatment in an in vivo efficacy test on GPC1 ADC using a cervical cancer cell strain. The p value is determined by Scheffe's method following one-way ANOVA.

[Figure 31] Figure **31** shows the rate of change in the body weight of a mouse in an in vivo efficacy test on a GPC1 ADC using a cervical cancer cell strain. The vertical axis indicates the rate of change in the body weight, and the horizontal axis indicates the number of days after treatment. The error bar indicates mean ± SEM.

[Figure 32] Figure **32** indicates the results of immunohistochemical staining of tumor tissue administered with GPC1 ADC. After ME180 subcutaneous tumorigenesis, (1) PBS, (2) 10 mg/kg of control ADC, and (3) 10 mg/kg of GPC1-ADC were administered to the tail vein once, and the tumor was extracted after 24 hours. The black dots indicate cells arrested at the G2/M phase.

[Figure 33] Figure **33** shows results for antitumor effect of ADC and unlabeled antibody on TE4. The vertical axis indicates the rate of growth relative to no treatment (%), and the horizontal axis indicates ADC or antibody concentration.

[Figure 34] Figure **34** shows results for antitumor effect of ADC and unlabeled antibody on TE5. The vertical axis indicates the rate of growth relative to no treatment (%), and the horizontal axis indicates ADC or antibody concentration.

[Figure 35] Figure **35** shows results for antitumor effect of ADC and unlabeled antibody on TE6. The vertical axis indicates the rate of growth relative to no treatment (%), and the horizontal axis indicates ADC or antibody concentration.

[Figure 36] Figure **36** shows results for antitumor effect of ADC and unlabeled antibody on TE8. The vertical axis indicates the rate of growth relative to no treatment (%), and the horizontal axis indicates ADC or antibody concentration.

[Figure 37] Figure **37** shows results for antitumor effect of ADC and unlabeled antibody on TE9. The vertical axis indicates the rate of growth relative to no treatment (%), and the horizontal axis indicates ADC or antibody concentration.

[Figure 38] Figure **38** shows results for antitumor effect of ADC and unlabeled antibody on TE10. The vertical axis indicates the rate of growth relative to no treatment (%), and the horizontal axis indicates ADC or antibody concentration.

[Figure 39] Figure **39** shows results for antitumor effect of ADC and unlabeled antibody on TE11. The vertical axis indicates the rate of growth relative to no treatment (%), and the horizontal axis indicates ADC or antibody concentration.

[Figure 40] Figure **40** shows results for antitumor effect of ADC and unlabeled antibody on TE14. The vertical axis indicates the rate of growth relative to no treatment (%), and the horizontal axis indicates ADC or antibody concentration.

[Figure 41] Figure **41** shows results for antitumor effect of ADC and unlabeled antibody on TE15. The vertical axis indicates the rate of growth relative to no treatment (%), and the horizontal axis indicates ADC or antibody concentration.

[Figure 42] Figure **42** shows results for antitumor effect of ADC and unlabeled antibody on HeLa. The vertical axis indicates the rate of growth relative to no treatment (%), and the horizontal axis indicates ADC or antibody concentration.

[Figure 43] Figure **43** shows results for antitumor effect of ADC and unlabeled antibody on ME180. The vertical axis indicates the rate of growth relative to no treatment (%), and the horizontal axis indicates ADC or antibody concentration.

[Figure 44] Figure **44** schematically depicts the test of Example 12 (in vivo antitumor effect of an unlabeled anti-GPC1 antibody on a GPC1 positive cell strain).

[Figure 45] Figure **45** shows results for in vivo antitumor effect of an unlabeled anti-GPC1 antibody on GPC1 positive cell strain. The vertical axis of the left graph indicates the tumor volume ($mm^3$), and the horizontal axis indicates the number of days after treatment. The right graph shows the tumor weight (mg) on day 31 after treatment.

[Figure 46] Figure **46** shows comparison of results of hematological analysis in a safety test with a control IgG vs anti-GPC1 antibody in male mice (n = 4). The left column in the table indicates the endpoints, white blood cells (WBC), lymphocytes (Ly), monocytes (Mo), granulocytes (Gr), red blood cells (RBC), hemoglobin (Hb), hematocrit value (Hct), and platelets (Plt). The p value was calculated by Student's t-test. The p value is determined by Student's t-test.

[Figure 47] Figure **47** shows comparison of results of hematological analysis in a safety test with a control IgG vs anti-GPC1 antibody in female mice (n = 4). The left column in the table indicates the endpoints. White blood cells (WBC), lymphocytes (Ly), monocytes (Mo), granulocytes (Gr), red blood cells (RBC), hemoglobin (Hb), hematocrit value (Hct), and platelets (Plt) were evaluated. The p-value is determined by Student's t-test.

[Figure 48] Figure **48** shows comparison of results of hematological analysis in a safety test with a control IgG vs anti-GPC1 antibody in male mice (n = 4). The left column in the table indicates the endpoints. Albumin (ALb), alkaline phosphatase (ALP), alanine aminotransferase (ALT), amylase (Amy), total bilirubin (T-Bil), blood urea nitrogen (BUN), calcium (Ca), phosphorous (P), chromium (Cr), glutamine (Glu), sodium (Na), potassium (K), total protein (TP), and globulin (Glob) were evaluated.

[Figure 49] Figure **49** shows comparison of results of hematological analysis in a safety test with a control IgG vs anti-GPC1 antibody in female mice (n = 4). The left column in the table indicates the endpoints. Albumin (ALb), alkaline phosphatase (ALP), alanine aminotransferase (ALT), amylase (Amy), total bilirubin (T-Bil), blood urea nitrogen (BUN), calcium (Ca), phosphorous (P), chromium (Cr), glutamine (Glu), sodium (Na), potassium (K), total

protein (TP), and globulin (Glob) were evaluated.

[Figure 50] Figure **50** shows results for growth suppression effect of clones 01a033, 01a002, and 02a010 on DU145 cells and MDA-MB231 cells. The vertical axis of each graph indicates the rate of growth compared to no treatment (%), and the horizontal axis indicates the antibody concentration.

[Figure 51] Figure **51** shows results for growth suppression effect of ADC of clones other than clones 01a033, 01a002, and 02a010 on DU145 cells. The vertical axis of each graph indicates the rate of growth compared to no treatment (%), and the horizontal axis indicates the antibody concentration.

[Figure 52] Figure **52** shows results for growth suppression effect of ADC of clones other than clones 01a033, 01a002, and 02a010 on DU145 cells. The vertical axis of each graph indicates the rate of growth compared to no treatment (%), and the horizontal axis indicates the antibody concentration.

[Figure 53] Figure **53** shows a table of $IC_{50}$ values of ADC of each clone against TE14 cells and $EC_{50}$ thereof against DU145 cells.

[Figure 54] Figure **54** shows the growth suppression effect against LLC-control-7 and LLC-mGPC1-16 cells. The vertical axis of each graph indicates the rate of growth compared to no treatment (%), and the horizontal axis indicates the antibody concentration.

[Figure 55] Figure **55** schematically depicts the test in Example 17.

[Figure 56] Figure **56** shows results for an in vivo antitumor effect of GPC1 ADC in a mouse grafted with LLC-mGPC1-16 cells. The vertical axis indicates the tumor volume ($mm^3$), and the horizontal axis indicates the number of days after treatment. The error bar indicates mean $\pm$ SEM (n = 5) .

[Figure 57] Figure **57** shows the tumor weight (mg) on day 24 after treatment in an in vivo efficacy test of GPC1 ADC in a mouse grafted with LLC-mGPC1-16 cells.

[Figure 58] Figure **58** shows the change in body weight of a mouse in an in vivo efficacy test of GPC1 ADC in a mouse grafted with LLC-mGPC1-16 cells. The error bar indicates mean $\pm$ SEM (n = 5).

[Description of Embodiments]

The present invention is described hereinafter. Throughout the entire specification, a singular expression should be understood as encompassing the concept thereof in plural form, unless specifically noted otherwise. Thus, singular articles (e.g., "a", "an", "the", and the like in the case of English) should also be understood as encompassing the concept thereof in plural form, unless specifically noted otherwise. Further, the terms used herein should be understood to be used in the meaning that is commonly used in the art, unless specifically noted otherwise. Thus, unless defined otherwise, all terminologies and scientific technical terms that are used herein have the same meaning as the general understanding of those skilled in the art to which the present invention pertains. In case of a contradiction, the present specification (including the definitions) takes precedence.

(Definitions)

First, the terms and common technologies used herein are described.

As used herein, "Glypican-1", "GPC-1", or "GPC1" are terms that are used interchangeably, which are glycosylphosphatidylinositol (GPI) anchored cell surface proteoglycans having heparan sulfate. Glypican-1 is understood to be associated with cell adhesion, migration, lipoprotein metabolism, growth factor activity regulation, and suppression of blood coagulation. Glypican-1 is considered to bind to several fibroblast growth factors (FGF) such as FGF-1, FGF-2, and FGF-7. Glypican-1 is understood to function as an extracellular chaperone of VEGF165 and assist in the recovery of receptor binding capacity after oxidation. Currently, 6 types of Glypicans, i.e., Glypican-1 to Glypican-6, are known. Meanwhile, in relation to cancer, Glypican family members are not necessarily recognized as a cancer marker. The members appear to be unrelated to one another. Glypican-1 is registered as accession number P35052 in UniProt (see http://www.uniprot.org/uniprot/P35052), and registered as NP_002072.2 (progenitor amino acid sequence) and NM_002081.2 (mRNA) in NCBI, and as X54232.1 (mRNA), BC051279.1 (mRNA), and AC110619.3 (genomic) in EMBL, GenBank, and DDBJ. Such information can all be utilized herein, which is incorporated herein by reference. For Glypican-1, see David G et al., J Cell Biol. 1990 Dec; 111 (6 Pt 2): 3165-76; Haecker U et al., Nat Rev Mol Cell Biol. 2005 Jul; 6(7): 530-41; Aikawa T et al., J Clin Invest. 2008 Jan; 118(1): 89-99.; Matsuda K, et al., Cancer Res. 2001 Jul 15; 61(14): 5562-9., and the like. SEQ ID NO: 1 is a representative example of the nucleic acid sequence (full length) of human Glypican-1, and SEQ ID NO: 2 is a representative example of the amino acid sequence. SEQ ID NO: 3 is a representative example of the nucleic acid sequence (full length) of mouse Glypican-1, and SEQ ID NO: 4 is a representative example of the amino acid sequence. When used for the objectives herein, it is understood that "Glypican-1", "GPC-1", or "GPC1" can be used herein as not only as a protein (or a nucleic acid encoding the same) having an amino acid sequence set forth in a specific sequence number of accession number, but also as a functionally active analog or derivative thereof, a functionally active fragment thereof, homolog thereof, or a mutant encoded by a nucleic acid hybridizing to a nucleic

acid encoding the protein under highly stringent conditions or low stringent conditions, as long as it is in alignment with the specific objective of the invention.

As used herein, "derivative", "analog", or "mutant" preferably includes, without intending to be limiting, molecules comprising a substantially homologous region in a target protein (e.g., Glypican-1 or antibody). In various embodiments, such a molecule is at least 30%, 40%, 50%, 60%, 70%, 80%, 90%, 95%, or 99% identical compared to a sequence aligned by a computer homology program that is known in the art throughout an amino acid sequence of the same size, or a nucleic acid encoding such a molecule can hybridize with a sequence encoding a constituent protein under (highly) stringent conditions, moderately stringent conditions, or non-stringent conditions. This is a product from modifying a protein by an amino acid substitution, deletion, and addition, respectively, and refers to a protein whose derivative still exhibits, although not necessarily to the same degree, the biological function of the original protein. For example, the biological function of such a protein can be found by a suitable and available in vitro assay that is described herein or known in the art. As used herein, "functionally active" refers to a polypeptide, a fragment, or a derivative having a structural function, regulating function, or biochemical function of a protein such as biological activity in accordance with the embodiment associated with the polypeptide, fragment or derivative of the invention.

In the present invention, humans are mainly discussed with regard to Glypican-1. Meanwhile, many animals other than humans such as chimpanzees (Pantroglodytes) (K7B6W5), rhesus monkeys (Macaca mulatta) (F6VPW9), mice (Mus musculus) (Q9QZF2), rats (Rattus norvegicus) (P35053), and chickens (Gallus gallus) (F1P150) are known to express Glypican-1 proteins. Thus, it is understood that these animals, especially mammals, are also within the scope of the invention. Preferably, the functional domain of Glypican-1, such as the extracellular domain (about 500 amino acids, including 12 cysteine residues) and the hydrophobic region of the C-terminus (GPI-anchor domain) are conserved.

As used herein, a fragment of Glypican-1 is a polypeptide comprising any region of Glypican-1, which does not necessarily have a biological function of a naturally-occurring Glypican-1, as long as the fragment functions as the objective (e.g., marker or therapeutic target) of the invention.

Thus, a typical nucleic acid sequence of Glypican-1 can be:

(a) a polynucleotide having the base sequence set forth in SEQ ID NO: 1 or a fragment sequence thereof;
(b) a polynucleotide encoding a polypeptide consisting of the amino acid sequence set forth in SEQ ID NO: 2 or a fragment thereof;
(c) a polynucleotide encoding a modified polypeptide having one or more amino acids with a mutation selected from the group consisting of substitution, addition, and deletion in the amino acid sequence set forth in SEQ ID NO: 2 or a fragment thereof, the modified polypeptide having biological activity;
(d) a polynucleotide, which is a splice mutant or allelic mutant of the base sequence set forth in SEQ ID NO: 1 or a fragment thereof;
(e) a polynucleotide encoding a species homolog of a polypeptide consisting of the amino acid sequence set forth in SEQ ID NO: 2 or a fragment thereof;
(f) a polynucleotide encoding a polypeptide having biological activity and hybridizing to a polynucleotide of any one of (a) to (e) under stringent conditions; or
(g) a polynucleotide encoding a polypeptide which consists of a base sequence that has at least 70%, at least 80%, at least 90%, at least 95%, at least 96%, at least 97%, at least 98%, or at least 99% identity to a polynucleotide of any one of (a) to (e) or a complementary sequence thereof and has biological activity. In this regard, biological activity typically refers to activity of Glypican-1 or being identifiable from other proteins in the same organism as a marker.

The amino acid sequence of Glypican-1 can be:

(a) a polypeptide consisting of the amino acid sequence set forth in SEQ ID NO: 2 or a fragment thereof;
(b) a polypeptide having one or more amino acids with a mutation selected from the group consisting of substitution, addition, and deletion in the amino acid sequence set forth in SEQ ID NO: 2, and having biological activity;
(c) a polypeptide encoded by a splice mutant or allelic mutant of the base sequence set forth in SEQ ID NO: 1;
(d) a polypeptide, which is a species homolog of the amino acid sequence set forth in SEQ ID NO: 2; or
(e) a polypeptide having an amino acid sequence with at least 70%, at least 80%, at least 90%, at least 95%, at least 96%, at least 97%, at least 98%, or at least 99% identity to a polypeptide of any one of (a) to (d) and having biological activity. In this regard, biological activity typically refers to activity of Glypican-1 or being identifiable from other proteins in the same organism as a marker (e.g., including a region that can function as a specific epitope when used as an antigen).

In relation to the present invention, a "substance that binds to Glypican-1", "Glypican-1 binding agent" or "Glypican-1 interaction molecule" is a molecule or a substance that at least temporarily binds to Glypican-1. It is preferably

and advantageously capable of displaying (e.g., labeled or in a labelable state) that the substance or molecule is bound for detection purposes, and is advantageously further bound to a therapeutic agent. Examples of a substance that binds to Glypican-1 include antibodies, bindable peptides, peptidomimetics, and the like. Preferably, a substance that binds to Glypican-1 has intracellular invasion (internalization) activity. As used herein, a "binding protein" or "binding peptide" with respect to Glypican-1 refers to any protein or peptide that binds to Glypican-1 including, but not limited to, antibodies directed to Glypican-1 (e.g., polyclonal antibody or monoclonal antibody), antibody fragments, and function equivalents.

As used herein, "protein", "polypeptide", "oligopeptide", and "peptide" are used in the same meaning, referring to a polymer of amino acids of any length. Such a polymer may be straight, branched, or cyclic. Amino acids may be naturally-occurring, non-naturally occurring, or altered amino acids. These terms can also encompass those assembled into a complex of a plurality of polypeptide chains. These terms also encompass naturally-occurring or artificially-altered amino acid polymers. Examples of such an alteration include disulfide bond formation, glycosylation, lipidation, acetylation, phosphorylation, and any other manipulation or alteration (e.g., conjugation with a label component). This definition also encompasses, for example, polypeptides comprising one or more analogs of amino acids (e.g., including non-naturally-occurring amino acids or the like), peptide-like compounds (e.g., peptoid), and other alterations known in the art. As used herein, "amino acid" is a general term for organic compounds with an amino group and a carboxyl group. When the antibody according to an embodiment of the invention comprises a "specific amino acid sequence", any of the amino acids in the amino acid sequence may be chemically modified. Further, any of the amino acids in the amino acid sequence may be forming a salt or a solvate. Further, any of the amino acids in the amino acid sequence may have an L form or a D form. Even for such cases, the protein according to an embodiment of the invention is considered as comprising the "specific amino acid sequence" described above. Examples of known chemical modifications applied to an amino acid comprised in a protein in vivo include modifications of the N-terminus (e.g., acetylation, myristylation, and the like), modifications of the C-terminus (e.g., amidation, addition of glycosylphosphatidylinositol and the like), modifications of a side chain (e.g., phosphorylation, glycosylation, and the like) and the like. The modifications may be naturally-occurring or non-naturally-occurring, as long as the objective of the present invention is met.

As used herein, "polynucleotide", "oligonucleotide", and "nucleic acid" are used in the same meaning, referring to a polymer of nucleotides of any length. These terms also encompass "oligonucleotide derivative" and "polynucleotide derivative". The "oligonucleotide derivative" and "polynucleotide derivative" are interchangeably used and refer to an oligonucleotide or polynucleotide comprising a derivative of a nucleotide or an oligonucleotide or having a bond between nucleotides that is different from normal bonds. Specific examples of such oligonucleotides include: 2'-O-methyl-ribonucleotide; oligonucleotide derivatives with a phosphodiester bond in an oligonucleotide converted into phosphorothioate bond; oligonucleotide derivatives with a phosphodiester bond in an oligonucleotide converted into an N3'-P5' phosphoramidate bond; oligonucleotide derivatives with a ribose and a phosphodiester bond in an oligonucleotide converted into a peptide nucleic acid bond; oligonucleotide derivatives with a uracil in an oligonucleotide substituted with a C-5 propynyl uracil; oligonucleotide derivatives with uracil in an oligonucleotide substituted with a C-5 thiazole uracil; oligonucleotide derivatives with a cytosine in an oligonucleotide substituted with a C-5 propynyl cytosine; oligonucleotide derivatives with a cytosine in an oligonucleotide substituted with a phenoxazine-modified cytosine; oligonucleotide derivatives with a ribose in DNA substituted with a 2'-O-propylribose; oligonucleotide derivatives with a ribose in an oligonucleotide substituted with a 2'-methoxyethoxy ribose; and the like. Unless noted otherwise, specific nucleic acid sequences are intended to encompass sequences that are explicitly set forth, as well as their conservatively altered variants (e.g., degenerate codon substitutes) and complementary sequences. Specifically, a degenerate codon substitute can be achieved by making a sequence in which the third position of one or more selected (or all) codons is substituted with a mixed base and/or deoxyinosine residue (Batzer et al., Nucleic Acid Res. 19: 5081 (1991); Ohtsuka et al., J. Biol. Chem. 260: 2605-2608 (1985); Rossolini et al., Mol. Cell. Probes 8: 91-98 (1994)). As used herein, "nucleic acid" is also interchangeably used with gene, cDNA, mRNA, oligonucleotide, and polynucleotide. As used herein, "nucleotide" may be naturally occurring or non-naturally-occurring.

As used herein, "gene" refers to an agent that defines a genetic trait. A "gene" may refer to a "polynucleotide", "oligonucleotide", or "nucleic acid".

As used herein, "homology" of genes refers to the degree of identity of two or more genetic sequences with respect to one another, and having "homology" generally refers to having a high degree of identity or similarity. Therefore, the identity or similarity of sequences is higher when homology of two genes is high. Whether two types of genes have homology can be found by direct comparison of sequences or by a hybridization method under stringent conditions for nucleic acids. When two genetic sequences are directly compared, the genes are homologous typically if DNA sequences are at least 50% identical, preferably at least 70% identical, and more preferably at least 80%, 90%, 95%, 96%, 97%, 98%, or 99% identical between the genetic sequences. Thus, as used herein, "homolog" or "homologous gene product" refers to a protein in another species, preferably mammal, exerting the same biological function as a protein constituent of a complex, which will be further described herein. Such a homolog is also known as "ortholog gene product". It is understood that such a homolog, homologous gene product, ortholog gene product, or the like can also be used, as long as they are in alignment with the objective of the invention.

Amino acids may be mentioned herein by either their commonly known three letter symbols or their one character symbols recommended by the IUPAC-IUB Biochemical Nomenclature Commission. Similarly, nucleotides may be mentioned by their commonly recognized one character codes. Comparison of similarity, identity, and homology of an amino acid sequence and a base sequence is calculated herein by using a sequence analysis tool BLAST with default parameters. For example, identity can be searched using BLAST 2.2.28 (published on April 2, 2013) of the NCBI. Herein, values for identity generally refer to a value obtained when aligned under the default conditions using BLAST. However, when a higher value is obtained by changing a parameter, the highest value is considered the value of identity. When identity is evaluated in a plurality of regions, the highest value thereamong is considered the value of identity. Similarity is a value calculated by taking into consideration a similar amino acid in addition to identity.

In one embodiment of the invention, "several" may be, for example, 10, 8, 6, 5, 4, 3 or 2, or a value less than any one of the values. It is known that a polypeptide with one or several amino acid residue deletions, additions, insertions, or substitutions by other amino acids maintains its biological activity (Mark et al., Proc Natl Acad Sci USA. 1984 Sep; 81(18): 5662-5666., Zoller et al., Nucleic Acids Res. 1982 Oct 25; 10(20): 6487-6500., Wang et al., Science. 1984 Jun 29; 224 (4656): 1431-1433.) An antibody with a deletion or the like can be made, for example, by site-directed mutagenesis, random mutagenesis, biopanning using an antibody phage library, or the like. For example, KOD-Plus-Mutagenesis Kit (TOYOBO CO., LTD.) can be used for site-directed mutagenesis. An antibody with the same activity as the wild-type can be selected from mutant antibodies introduced with a deletion or the like by performing various characterizations in FACS analysis, ELISA, or the like.

In one embodiment of the invention, "90% or greater" may be, for example, 90, 95, 96, 97, 98, 99 or 100% or greater, or within the range of any two such values. For the "homology", the percentage of the number of homologous amino acids in two or a plurality of amino acid sequences may be calculated in accordance with a method that is known in the art. Before calculating the percentage, amino acid sequences in a group of amino acid sequences to be compared are aligned. A space is introduced in a portion of amino acid sequences when it is necessary to maximize the percentage of the same amino acids. An alignment method, method of calculating the percentage, comparison method, and computer programs associated therewith have been well known in the art (e.g., BLAST, GENETYX, and the like). As used herein, "homology" can be represented by a value measured with BLAST of the NCBI, unless specifically noted otherwise. Blastp can be used in the default setting for an algorithm for comparing amino acid sequences with BLAST. Results of measurement are expressed in a numerical form as Positives or Identities.

As used herein, "polynucleotide which hybridizes under a stringent condition" refers to commonly used, well-known conditions in the art. Such a polynucleotide can be obtained by using a method such as colony hybridization, plaque hybridization, or southern blot hybridization while using a polynucleotide selected from the polynucleotides of the inventions as a probe. Specifically, the polynucleotide refers to a polynucleotide that can be identified by using a filter with immobilized DNA from a colony or plaque and performing hybridization at 65°C in the presence of 0.7 to 1.0 M NaCl, and then using an SSC (saline-sodium citrate) solution with 0.1 to 2× concentration (composition of an SSC solution with 1× concentration is 150 mM sodium chloride and 15 mM sodium citrate) to wash the filter under the condition of 65°C. For "stringent condition", the following are examples of conditions that can be used. (1) low ionic strength and a high temperature are used for washing (e.g., 0.015 M sodium chloride/0.0015 M sodium citrate/0.1% sodium dodecyl sulfate at 50°C), (2) a denaturing agent such as formamide is used in hybridization (e.g., 50% (v/v) formamide, 0.1% bovine serum albumin/0.1% ficoll/0.1% polyvinyl pyrrolidone/50 mM sodium phosphate buffer with a pH of 6.5, 750 mM sodium chloride, and 75 mM sodium citrate at 42°C), and (3) a solution comprising 20% formamide, 5 × SSC, 50 mM sodium phosphate (pH 7.6), 5 × Denhardt's solution, 10% dextran sulfate, and 20 mg/ml denatured sheared salmon sperm DNA, is incubated overnight at 37°C and then a filter is washed with 1 × SSC at about 37 to 50°C. The formamide concentration may be 50% or greater. Washing time can be 5, 15, 30, 60, or 120 minutes or longer. A plurality of elements such as temperature and salt concentration are conceivable as elements affecting the stringency of hybridization reactions. Ausubel et al., Current Protocols in Molecular Biology, Wiley Interscience Publishers, (1995) can be referred for details. "Highly stringent condition", for example, is 0.0015 M sodium chloride, 0.0015 M sodium citrate, and 65-68°C or 0.015 M sodium chloride, 0.0015 M sodium citrate, 50% formamide, and 42°C. Hybridization can be performed in accordance with the method described in experimental publications such as Molecular Cloning 2nd ed., Current Protocols in Molecular Biology, Supplement 1-38, DNA Cloning 1: Core Techniques, A Practical Approach, Second Edition, Oxford University Press (1995). In this regard, a sequence comprising only an A sequence or only a T sequence is preferably excluded from a sequence that hybridizes under stringent conditions. A moderately stringent condition can be readily determined by those skilled in the art based on, for example, the length of a DNA, and is shown in Sambrook et al., Molecular Cloning: A Laboratory Manual, Third Ed., Vol.1, 7.42-7.45 Cold Spring Harbor Laboratory Press, 2001, including, for a nitrocellulose filters, use of hybridization conditions of a pre-wash solution of 1.0 mM EDTA (pH 8.0), 0.5% SDS, and 5 × SSC, and about 50% formamide and 2 × SSC - 6 × SSC at about 40-50°C (or other similar hybridization solutions such as a Stark's solution in about 50% formamide at about 42°C) and washing conditions of 0.5 × SSC, 0.1% SDS at about 60°C. Thus, the polypeptides used herein encompass polypeptides encoded by a nucleic acid molecule that hybridizes under highly or moderately stringent conditions to a nucleic acid molecule encoding a polypeptide described herein in particular.

As used herein, a "purified" substance or biological agent (e.g., nucleic acid, protein, or the like) refers to a substance or a biological agent having at least a part of an agent naturally accompanying the substance or biological agent removed. Thus, the purity of a biological agent in a purified biological agent is generally higher than the purity in the normal state of the biological agent (i.e., concentrated). The term "purified" as used herein refers to the presence of preferably at least 75% by weight, more preferably at least 85% by weight, still more preferably at least 95% by weight, and most preferably at least 98% by weight of a biological agent of the same type. The substance or biological agent used herein is preferably a "purified" substance. An "isolated" substance or biological agent (e.g., nucleic acid, protein, or the like) as used herein refers to a substance or biological agent having agents that naturally accompany the substance or biological agent substantially removed. The term "isolated" as used herein varies depending on the objective. Thus, the term does not necessarily have to be represented by purity. However, when necessary, the term refers to the presence of preferably at least 75% by weight, more preferably at least 85% by weight, still more preferably at least 95% by weight, and most preferably at least 98% by weight of a biological agent of the same type. The substance used herein is preferably an "isolated" substance or biological agent.

As used herein, a "corresponding" amino acid, nucleic acid, or moiety refers to an amino acid or a nucleotide which has or is expected to have, in a certain polypeptide molecule or polynucleotide molecule (e.g., Glypican-1), similar action as a given amino acid, nucleotide, or moiety in a benchmark polypeptide or a polynucleotide for comparison, and, particularly for enzyme molecules, refers to an amino acid which is present at a similar position in an active site and makes a similar contribution to catalytic activity and refers to a corresponding moiety in a complex molecule (e.g., heparan sulfate or the like). For example, for an antisense molecule, it can be a similar moiety in an ortholog corresponding to a specific moiety of the antisense molecule. A corresponding amino acid can be a specific amino acid subjected to, for example, cysteination, glutathionylation, S-S bond formation, oxidation (e.g., oxidation of methionine side chain), formylation, acetylation, phosphorylation, glycosylation, myristylation, or the like. Alternatively, a corresponding amino acid can be an amino acid responsible for dimerization. Such a "corresponding" amino acid or nucleic acid may be a region or a domain over a certain range. Thus, it is referred herein as a "corresponding" region or domain in such a case. Such a corresponding region or domain is useful for designing a complex molecule in the present invention.

As used herein, a "corresponding" gene (e.g., polynucleotide sequence or molecule) refers to a gene (e.g., polynucleotide sequence or molecule) of a certain species which has or is expected to have similar action as a given gene in a benchmark species for comparison. If there is a plurality of genes having such action, the corresponding gene refers to a gene having the same evolutionary origin. Hence, a gene corresponding to a certain gene may be an ortholog of such a gene. Thus, for each human Glypican-1, a corresponding Glypican-1 can be found in other animals (especially mammals). Such a corresponding gene can be identified by using a technology that is well known in the art. For example, a corresponding gene in a certain animal (e.g., mouse) can be found by searching a database comprising sequences of the animal from using the sequence of SEQ ID NO: 1, 2, or the like as a query sequence, as a benchmark gene of the corresponding gene (e.g., Glypican-1).

As used herein, "fragment" refers to a polypeptide or polynucleotide with a sequence length of 1 to n-1 with respect to the full length polypeptide or polynucleotide (with length n). The length of a fragment can be appropriately changed in accordance with the objective. Examples of the lower limit of such a length include 3, 4, 5, 6, 7, 8, 9, 10, 15, 20, 25, 30, 40, 50 and more amino acids for a polypeptide. Lengths represented by an integer that is not specifically mentioned herein (e.g., 11 and the like) can also be suitable as a lower limit. Further, examples of the length include 5, 6, 7, 8, 9, 10, 15, 20, 25, 30, 40, 50, 75, 100, and more nucleotides for a polynucleotide. Lengths represented by an integer that is not specifically mentioned herein (e.g., 11 and the like) can also be suitable as a lower limit. As used herein, such a fragment is understood to be within the scope of the present invention, for example when a full length version functions as a marker or a target molecule, as long as the fragment itself also functions as a marker or a target molecule.

According to the present invention, the term "activity" as used herein refers to a function of a molecule in the broadest sense. Activity generally encompasses, but is not intended to be limited to, biological function, biochemical function, physical function, and chemical function of a molecule. Examples of activity include enzymatic activity, ability to interact with another molecule, ability to activate, promote, stabilize, inhibit, suppress, or destabilize a function of another molecule, stability, and ability to localize at a specific position in a cell. When applicable, the term is also directed to a function of a protein complex in the broadest sense.

As used herein, "biological function", with regard to a gene or a nucleic acid molecule or polypeptide related thereto, refers to a specific function that the gene, nucleic acid molecule, or polypeptide can have in vivo. Examples thereof include, but are not limited to, production of a specific antibody, enzymatic activity, impartation of resistance, and the like. Examples thereof include, but are not limited to, functions of Glypican-1 involved in apoptosis of GPC-1 positive tumor cells (e.g., esophageal cancer cells), cleavage of caspase-3, phosphorylation of AKT, and the like. As used herein, biological function can be exerted by "biological activity". As used herein, "biological activity" refers to activity that a certain agent (e.g., polynucleotide, protein, or the like) can have in vivo, including activity exerting a variety

of functions (e.g., transcription promoting activity) such as the activity of activating or deactivating a molecule from interaction with another molecule. When two agents interact, the biological activity thereof can be understood as the bond between the two molecules and the biological change resulting therefrom, e.g., the two molecules are bound when precipitation of one of the molecules with an antibody results in co-precipitation of the other molecule. Thus, one method of determination includes observing such co-precipitation. If an agent is for example an enzyme, the biological activity thereof encompasses the enzymatic activity thereof. Another example includes binding of a ligand to a corresponding receptor when an agent is a ligand. Such biological activity can be measured by a technology that is well known in the art. Thus, "activity" refers to various measurable indicators that indicate or reveal the bond (either directly or indirectly) or affect a response (i.e., having a measurable effect in response to some exposure or stimulation). Examples thereof include the affinity of a compound that directly binds to the polypeptide or polynucleotide of the invention, the amount of proteins upstream or downstream after some stimulation or event, and a scale of another similar function.

As used herein, "expression" of a gene, a polynucleotide, a polypeptide, or the like refers to the gene or the like being subjected to a certain action in vivo to be converted into another form. Preferably, expression refers to a gene, a polynucleotide, or the like being transcribed and translated into a form of a polypeptide. However, transcription to make an mRNA is also one embodiment of expression. Thus, "expression product" as used herein encompasses such a polypeptide and protein, and mRNA. More preferably, such a polypeptide form can be a form which has undergone post-translation processing. For example, the expression level of Glypican-1 can be determined by any method. Specifically, the expression level of Glypican-1 can be found by evaluating the amount of mRNA of Glypican-1, the amount of Glypican-1 protein, and the biological activity of Glypican-1 protein. Such a measurement value can be used in companion diagnosis. The amount of protein or mRNA of Glypican-1 can be determined by the method described in detail in other parts of the specification or other methods known in the art.

As used herein, "functional equivalent" refers to any entity having the same function of interest but a different structure relative to the original target entity. Thus, it is understood that a functional equivalent of " Glypican-1" or an antibody thereof encompasses mutants and variants (e.g., amino acid sequence variants and the like) of Glypican-1 or antibody thereof that are not Glypican-1 or antibody thereof itself, which have the biological action of Glypican-1 or antibody thereof or can change, upon action, into Glypican-1 or the antibody thereof itself, or a mutant or variant of Glypican-1 or the antibody thereof (e.g., including nucleic acids encoding Glypican-1 or an antibody thereof itself and mutants and variants of Glypican-1 or antibody thereof, and vectors, cells, and the like comprising such a nucleic acid). It is understood, even without specifically mentioning, that a functional equivalent of Glypican-1 or an antibody thereof can be used in the same manner as Glypican-1 or antibody thereof. A functional equivalent can be found by searching a database or the like. As used herein, "search" refers to utilizing a certain nucleic acid base sequence electronically, biologically, or by another method to find another nucleic acid base sequence having a specific function and/or property. Examples of electronic search include, but are not limited to, BLAST (Altschul et al., J. Mol. Biol. 215: 403-410 (1990)), FASTA (Pearson & Lipman, Proc. Natl. Acad. Sci., USA 85: 2444-2448 (1988)), Smith and Waterman method (Smith and Waterman, J. Mol. Biol. 147: 195-197 (1981)), Needleman and Wunsch method (Needleman and Wunsch, J. Mol. Biol. 48: 443-453 (1970)) and the like. Examples of biological search include, but are not limited to, stringent hybridization, a macroarray with a genomic DNA applied to a nylon membrane or the like or a microarray with a genomic DNA applied to a glass plate (microarray assay), PCR, in situ hybridization, and the like. Herein, a gene used in the present invention is intended to include corresponding genes identified by such electronic search or biological search.

As a functional equivalent of the invention, it is possible to use an amino acid sequence with one or more amino acid insertions, substitutions, or deletions, or addition to one or both ends. As used herein, "one or more amino acid insertions, substitutions, or deletions, or addition to one or both ends in an amino acid sequence" refers to an alteration with a substitution of a plurality of amino acids or the like to the extent that can occur naturally by a well-known technical method such as site-directed mutagenesis or natural mutation. An altered amino acid sequence can have, for example, 1 to 30, preferably 1 to 20, more preferably 1 to 9, still more preferably 1 to 5, and especially preferably 1 to 2 amino acid insertions, substitutions, or deletions, or additions to one or both ends. Preferably, an altered amino acid sequence may be an amino acid sequence having one or more (preferably 1 or several, or 1, 2, 3 or 4) conservative substitutions in the amino acid sequence of Glypican-1. "Conservative substitution" refers herein to a substitution of one or more amino acid residues with other chemically similar amino acid residue so as not to substantially alter a function of a protein. Examples thereof include substitutions of a hydrophobic residue with another hydrophobic residue, substitutions of a polar residue with another polar residue having the same charge, and the like. Functionally similar amino acids that can be substituted in this manner are known in the art for each amino acid. Specific examples include alanine, valine, isoleucine, leucine, proline, tryptophan, phenylalanine, methionine, and the like for nonpolar (hydrophobic) amino acids, and glycine, serine, threonine, tyrosine, glutamine, asparagine, cysteine, and the like for polar (neutral) amino acids. Examples of positively charged (basic) amino acids include arginine, histidine, lysine, and the like. Further, examples of a negatively-charged (acidic) amino acid include aspartic acid, glutamic acid, and the like.

As used herein, "inhibitor" refers to a substance or agent that inhibits biological action of a receptor or a cell against a target entity (e.g., receptor or cell). A Glypican-1 inhibitor of the invention is an agent that can temporarily or

permanently reduce or eliminate a function of a target Glypican-1, a cell expressing Glypican-1, or the like. Examples of such an agent include, but are not limited to, antibodies, antigen binding fragments thereof, derivatives, functional equivalents, antisense nucleic acids, RNAi agents such as siRNAs, other nucleic acid forms, and the like. In one embodiment of the invention, an "anti-Glypican-1 antibody" includes antibodies capable of binding to Glypican-1. While the production method of such an anti-Glypican-1 antibody is not particularly limited, the antibody can be produced by, for example, immunizing a mammal or avian with Glypican-1. An "antigen binding fragment" of an anti-Glypican-1 antibody refers to a fragment of an antibody retaining the ability to bind to Glypican-1. Examples of such an antigen binding fragment include, but are not limited to, single chain antibodies, scFvs, Fab fragments, $F(ab')_2$ fragments, and the like.

It is understood that a "functional equivalent" of an "antibody to Glypican-1 (anti-Glypican-1 antibody) or an antigen binding fragment thereof" includes, for antibodies, antibodies having binding activity of Glypican-1 and fragments thereof themselves, as well as chimeric antibodies, humanized antibodies, human antibodies, multifunctional antibodies, bispecific or oligospecific antibodies, single chain antibodies, scFvs, diabodies, sc(FV)2 (single chain (Fv)2), scFv-Fcs, and the like.

An anti-Glypican-1 antibody according to one embodiment of the invention is preferably an anti-Glypican-1 antibody that specifically binds to a specific epitope of Glypican-1 from the viewpoint of especially strong suppression of growth of malignant tumors.

An anti-Glypican-1 antibody according to one embodiment of the invention can be a monoclonal antibody. A monoclonal antibody can act more efficiently on Glypican-1 compared to polyclonal antibodies. It is preferable to immunize chickens with Glypican-1 from the viewpoint of efficiently producing anti-Glypican-1 monoclonal antibodies.

The antibody class of the anti-Glypican-1 antibody according to one embodiment of the invention is not particularly limited. For example, the class may be IgM, IgD, IgG, IgA, IgE, or IgY.

The anti-Glypican-1 antibody according to one embodiment of the present invention may be an antibody fragment having antigen binding activity (hereinafter, also referred to as "antigen binding fragment"). In such a case, there is an effect of improved stability, antibody production efficiency, or the like.

The anti-Glypican-1 antibody according to one embodiment of the invention may be a fusion protein. The fusion protein may comprise a polypeptide or oligopeptide bound to the N or C-terminus of an anti-Glypican-1 antibody. The oligopeptide in this regard may be an His-tag. The fusion protein may also be fused to a mouse, human, or chicken antibody partial sequence. Such fusion proteins are also encompassed as one form of the anti-Glypican-1 antibody according to the present embodiment.

The anti-Glypican-1 antibody according to one embodiment of the invention may be, for example, an antibody obtained via the step of immunizing an organism with a purified Glypican-1, Glypican-1-expressing cell, or Glypican-1 containing lipid membrane. It is preferable that a Glypican-1-expressing cell is used for immunization from the viewpoint of enhancing a therapeutic effect against Glypican-1 positive cancer.

The anti-Glypican-1 antibody according to one embodiment of the invention may be an antibody having a CDR set of an antibody obtained via the step of immunizing an organism with a purified Glypican-1, Glypican-1-expressing cell, or Glypican-1 containing lipid membrane. It is preferable that a Glypican-1-expressing cell is used for immunization from the viewpoint of enhancing a therapeutic effect against Glypican-1 positive cancer. A CDR set is a set of heavy chain CDRs 1, 2, and 3 and light chain CDRs 1, 2, and 3.

"Glypican-1 expressing cell" in one embodiment of the invention may be obtained, for example, by introducing a polynucleotide encoding Glypican-1 into a cell and having the Glypican-1 expressed. Glypican-1 in this regard encompasses Glypican-1 fragments. Further, "Glypican-1-containing lipid membrane" in one embodiment of the invention may be obtained, for example, by mixing Glypican-1 and a lipid bilayer. Glypican-1 in this regard encompasses Glypican-1 fragments. Further, the anti-Glypican-1 antibody according to one embodiment of the invention is preferably an antibody obtained via the step of immunizing a chicken with an antigen or an antibody having a CDR set of such an antibody from the viewpoint of enhancing a therapeutic effect against Glypican-1 positive cancer.

The anti-Glypican-1 antibody of the invention may have any binding capacity as long as the objective can be accomplished. For example, it is sufficient for the anti-Glypican-1 antibody of the invention to have internalization activity and/or ADC activity even if the antibody has low affinity to Glypican-1. However, the antibody preferably has a strong binding capacity for the purpose of diagnosis and companion reagent. For example, the $K_D$ value (kd/ka) can be $1.0 \times 10^{-7}$ (M) or less, $1.0 \times 10^{-8}$ (M) or less, $1.0 \times 10^{-9}$(M) or less, or $1.0 \times 10^{-10}$ or less. Preferably, the binding capacity of an anti-Glypican-1 antibody for the purpose of diagnosis and companion reagent is $K_D$ value (kd/ka) of $1.0 \times 10^{-8}$(M) or less.

The anti-Glypican-1 antibody according to one embodiment of the invention may be an antibody that binds to a wild-type or mutant Glypican-1. Mutant Glypican-1 includes mutants due to individual differences in the DNA sequences. The amino acid sequence of wild-type or mutant Glypican-1 is preferably 80% or more, more preferably 90% or more, more preferably 95% or more, and especially preferably 98% or more homologous to the amino acid sequence set forth in SEQ ID NO: 2.

As used herein, an "antibody" includes a molecule capable of specifically binding to a specific epitope on an

antigen or a population thereof. An antibody may be a polyclonal antibody or a monoclonal antibody. Antibodies can have various forms such as one or more forms selected from the group consisting of full length antibodies (antibodies with a Fab region and an Fc region), Fv antibodies, Fab antibodies, F(ab')2 antibodies, Fab' antibodies, diabodies, single stranded (single chain) antibodies (e.g., scFv), sc(Fv)2 (single chain (Fv)2), scFv-Fc, dsFv, multispecific antibodies (e.g., oligospecific antibodies and bispecific antibodies), diabodies, peptides or polypeptides with an antigen binding property, chimeric antibodies (e.g., mouse-human chimeric antibodies, chicken-human chimeric antibodies, and the like), mouse antibodies, chicken antibodies, humanized antibodies, human antibodies, and an equivalent thereof. Antibodies also encompass modified and unmodified antibodies. Modified antibodies may be formed by an antibody binding to various molecules such as polyethylene glycol. A modified antibody can be obtained by applying chemical modification to an antibody using a known approach. Such an antibody can also be covalently bound, or fused by recombination, to an enzyme such as alkaline phosphatase, horseradish peroxidase, or $\alpha$ galactosidase. The anti-Glypican-1 antibodies or the like used herein can be of any origin, type, shape, or the like, as long as the antibodies bind to a Glypican-1 protein. Specifically, known antibodies such as a nonhuman animal antibody (e.g., a mouse antibody, a rat antibody, or a camel antibody), a human antibody, a chimeric antibody, or a humanized antibody can be used. In the present invention, a monoclonal or polyclonal antibody can be utilized, but a monoclonal antibody is preferable. It is preferable that an antibody binds specifically to a Glypican-1 protein. Further, antibodies encompass modified and unmodified antibodies. Modified antibodies may be formed by an antibody binding to various molecules such as polyethylene glycol. A modified antibody can be obtained by applying a chemical modification to an antibody by using a known approach.

"Polyclonal antibody" in one embodiment of the invention can be produced, for example, by administering an immunogen comprising an antigen of interest to mammals (e.g., rat, mouse, rabbit, cow, monkey, or the like), birds or the like in order to induce production of a polyclonal antibody specific to the antigen. An immunogen may be administered by injection of one or more immunizing agents and, when desired, an adjuvant. An adjuvant may be used to increase immune responses and may comprise a Freund's adjuvant (complete or incomplete), mineral gel (aluminum hydroxide or the like), surfactant (lysolecithin or the like), or the like. Immunization protocols are known in the art and, in some cases, may be implemented by any method that induces an immune response in accordance with the selected host organism (Tanpakushitsu Jikken Handobukku [Protein experiment handbook], Yodosha (2003): 86-91).

"Monoclonal antibody" in one embodiment of the invention encompasses individual antibodies constituting a population that are antibodies corresponding to substantially a single epitope except for antibodies having a mutation that can occur naturally in small amounts. Further, individual antibodies constituting a population may be antibodies that are substantially the same except for antibodies having a mutation that can occur naturally in small amounts. Monoclonal antibodies are highly specific, which are different from common polyclonal antibodies that typically include different antibodies corresponding to different epitopes. In addition to their specificity, monoclonal antibodies are useful in that they can be synthesized from a hybridoma culture which is not contaminated with other immunoglobulins. The description "monoclonal" may indicate a characteristic of being obtained from a substantially homogeneous antibody population. However, such a description does not mean that antibodies must be produced by a specific method. For example, monoclonal antibodies may be made by a method similar to the hybridoma method described in "Kohler G, Milstein C., Nature. 1975 Aug 7; 256 (5517): 495-497". Alternatively, monoclonal antibodies may be made by a method similar to the recombinant method described in US Patent No. 4816567. Monoclonal antibodies may also be isolated from a phage antibody library using a method similar to the technique that is described in, "Clackson et al., Nature. 1991 Aug 15; 352 (6336): 624-628." or "Marks et al., J Mol Biol. 1991 Dec 5; 222(3): 581-597". Monoclonal antibodies may also be made by the method described in "Tanpakushitsu Jikken Handobukku [Protein experiment handbook], Yodosha (2003): 92-96".

Antibodies can be mass-produced by using any approach that is known in the art. Examples of representative antibody mass production system construction and antibody manufacture include the following. Specifically, an H chain antibody expression vector and L chain antibody expression vector are transfected into CHO cells. The cells are cultured using a selection reagent G418 and Zeocin and cloned by limiting dilution. After cloning, clones stably expressing antibodies are selected by ELISA. The culture is expanded with the selected CHO cells, and the culture supernatant comprising antibodies are collected. Antibodies can be purified from the collected culture supernatant by Protein A or Protein G purification.

"Fv antibody" in one embodiment of the invention is an antibody comprising an antigen recognition site. This region comprises a dimer of one heavy chain variable domain non-covalently bound to one light chain variable domain. In this configuration, three CDRs of each variable domain can interact with one another to form an antigen binding site on the surface of a VH-VL dimer.

"Fab antibody" in one embodiment of the invention is, for example, a fragment obtained by treating an antibody comprising an Fab region and an Fc region with proteinase papain, which is an antibody in which about half of the N-terminus side of the H chain is bound to the entire L chain via some disulfide bonds. Fabs can be obtained, for example, by treating the anti-Glypican-1 antibody according to the embodiments of the invention comprising an Fab region and an Fc region with proteinase papain.

"F(ab')2 antibody" in one embodiment of the invention is a fragment obtained by treating an antibody comprising

an Fab region and an Fc region with proteinase pepsin, which is an antibody comprising two sites corresponding to Fabs. F(ab')2 can be obtained, for example, by treating the anti-Glypican-1 antibody according to an embodiments of the invention comprising an Fab region and an Fc region with proteinase pepsin. For example, F(ab')2 can be made by binding the following Fab' with a thioether bond or a disulfide bond.

"Fab' antibody" in one embodiment of the invention is an antibody obtained, for example, by cleaving a disulfide bond at a hinge region of F(ab')2 . For example, this can be obtained through treating F(ab')2 with a reducing agent dithiothreitol.

"scFv antibody" in one embodiment of the invention is an antibody comprising VH and VL that are linked with a suitable peptide linker. scFv antibodies can be produced, for example, by obtaining a cDNA encoding VH and VL of the anti-Glypican-1 antibody according to an embodiment of the invention, constructing a polynucleotide encoding VH-peptide linker-VL, incorporating the polynucleotide into a vector, and using a cell for expression.

"Diabody" in one embodiment of the invention is an antibody having divalent antigen binding activity. Divalent antigen binding activity can be configured to be identical or configured such that one of them has a different antigen binding activity. A diabody can be produced, for example, by constructing a polynucleotide encoding scFv such that the length of the amino acid sequence of a peptide linker is 8 residues or less, incorporating the resulting polynucleotide into a vector, and using a cell for expression.

"dsFv" in one embodiment of the invention is an antibody in which a polypeptide introduced with cysteine residues in VH and VL is bound via a disulfide bond between the cysteine residues. The position to which cysteine residues are introduced can be selected based on steric structure prediction of an antibody in accordance with the method demonstrated by Reiter et al (Reiter et al., Protein Eng. 1994 May; 7(5): 697-704).

"Peptide or polypeptide with antigen binding affinity" in one embodiment of the invention is an antibody comprised of antibody VH, VL or CDR1, 2 or 3 thereof. A peptide comprising a plurality of CDRs can be bound directly or via a suitable peptide linker.

The production method of the aforementioned Fv antibody, Fab antibody, F(ab')$^2$ antibody, Fab' antibody, scFv antibody, diabody, dsFv antibody, and peptide or polypeptide with antigen binding affinity (hereinafter, also referred to as "Fv antibodies and the like") is not particularly limited. Fv antibodies can be produced, for example, by incorporating a DNA encoding a region of the Fv antibodies and the like in the anti-Glypican-1 antibody according to an embodiment of the invention into an expression vector and using an expression cell. Further, Fv antibodies may be produced by a chemical synthesis method such as Fmoc (fluorenylmethyloxycarbonyl) or tBOC (t-butyloxycarbonyl) method. It should be noted that the antigen binding fragment according to one embodiment of the invention may be one or more types of the Fv antibodies and the like described above.

"Chimeric antibody" in one embodiment of the invention is, for example, a variable region of an antibody linked to a constant region of an antibody between xenogenic organisms and can be constructed by a genetic engineering technology. A mouse-human chimeric antibody can be made by, for example, the method described in "Roguska et al., Proc Natl Acad Sci USA. 1994 Feb 1; 91(3): 969-973." For example, the basic method of making a mouse-human chimeric antibody links a mouse leader sequence and a variable region sequence in a cloned cDNA with a sequence encoding a human antibody constant region already present in an expression vector of a mammalian cell. After linking the mouse leader sequence and variable region sequence in a cloned cDNA with the sequence encoding a human antibody constant region, the resultant sequence may be linked to a mammalian cell expression vector. A fragment of a human antibody constant region can be from any human antibody H chain constant region and human antibody L chain constant region. Examples of human H chain fragment include C$\gamma$1, C$\gamma$2, C$\gamma$3, and C$\gamma$4, and examples of L chain fragment include C$\lambda$ and C$\kappa$.

"Humanized antibody" in one embodiment of the invention is, for example, an antibody, which has one or more CDRs from nonhuman species, a framework region (FR) from a human immunoglobulin, and a constant region from human immunoglobulin and binds to a desired antigen. Antibodies can be humanized using various approaches known in the art (Almagro et al., Front Biosci. 2008 Jan 1; 13: 1619-1633). Examples thereof include CDR grafting (Ozaki et al., Blood. 1999 Jun 1; 93(11): 3922-3930.), Re-surfacing (Roguska et al., Proc Natl Acad Sci U S A. 1994 Feb 1; 91(3): 969-973.), FR shuffle (Damschroder et al., Mol Immunol. 2007 Apr; 44(11): 3049-3060. Epub 2007 Jan 22.) and the like. An amino acid residue of a human FR region may be substituted with a corresponding residue from a CDR donor antibody in order to alter (preferably in order to improve) the antigen bond. The FR substitution can be performed by a method that is well known in the art (Riechmann et al., Nature. 1988 Mar 24; 332 (6162): 323-327.) For example, an FR residue that is important for antigen binding may be identified by modeling an interaction between a CDR and an FR residue. Further, an abnormal FR residue at a specific position may be identified by sequence comparison.

"Human antibody" in one embodiment of the invention is, for example, an antibody in which a region comprising a variable region and constant region of a heavy chain and variable region and constant region of a light chain constituting the antibody is derived from a gene encoding a human immunoglobulin. Main production methods include a method using a transgenic mouse for making human antibodies, phage display method, and the like. A method using a transgenic mouse for making human antibodies produces human antibodies with diverse antigen binding capacities instead of

mouse antibodies if a functional human Ig gene is introduced into an endogenous Ig knockout mouse. Furthermore, this mouse can be immunized to obtain human monoclonal antibodies by a conventional hybridoma method. This can be made, for example, by the method described in "Lonberg et al., Int Rev Immunol. 1995; 13(1): 65-93." The phage display method is a system that typically expresses an exogenous gene as a fusion protein such that phage infectivity is not lost on the N-terminus side of a coat protein (g3p, g10p, or the like) of fibrous phage such as an E. coli virus M13 or T7. Antibodies can be made, for example, by the method described in "Vaughan et al., Nat Biotechnol. 1996 Mar; 14(3): 309-314".

Antibodies may also be prepared by grafting a heavy chain CDR or light chain CDR of the anti-Glypican-1 antibody according to an embodiment of the invention onto any antibody by CDR-grafting (Ozaki et al., Blood. 1999 Jun 1; 93(11): 3922-3930). Alternatively, antibodies can be obtained by linking a DNA encoding a heavy chain CDR or light chain CDR of the anti-Glypican-1 antibody according to an embodiment of the invention and a DNA encoding a region excluding a heavy chain CDR or light chain CDR of a known antibody derived from a human or a nonhuman organism to a vector in accordance with a known method in the art and then using a known cell for expression. In doing so, a known method in the art (e.g., method of allowing amino acid residues of an antibody to randomly mutate and screening for antibodies with high reactivity, phage display method, or the like) may be used to optimize the region excluding a heavy chain CDR or light chain CDR in order to enhance the efficiency of anti-Glypican-1 antibody acting upon a target antigen. Further, an FR region may be optimized by using, for example, FR shuffle (Damschroder et al., Mol Immunol. 2007 Apr; 44(11): 3049-3060. Epub 2007 Jan 22.) or a method of replacing a vernier zone amino acid residue or packaging residue (Japanese Laid-Open Publication No. 2006-241026 or Foote et al., J Mol Biol.1992 Mar 20; 224(2): 487-499).

"Heavy chain" in one embodiment of the invention is typically the main constituent element of a full-length antibody. A heavy chain is generally bound to a light chain by a disulfide bond and non-covalent bond. A region called a variable region (VH), which has an amino acid sequence that is not constant, even among antibodies in the same class of the same species, is present in a domain on the N-terminus side of a heavy chain. VH is generally known to greatly contribute to the specificity and affinity to an antigen. For example, "Reiter et al., J Mol Biol. 1999 Jul 16; 290(3): 685-98." describes that a molecule with only a VH, when made, bound to an antigen with specificity and high level of affinity. Furthermore, "Wolfson W, Chem Biol. 2006 Dec; 13(12): 1243-1244." describes that there are antibodies having only a heavy chain without a light chain among camel antibodies.

"CDR (complementarity determining region)" in one embodiment of the invention is a region that is actually in contact with an antigen to form a binding site in an antibody. CDRs are generally located on an Fv (variable region: including heavy chain variable region (VH) and light chain variable region (VL)) of an antibody. Further, CDRs generally have CDR1, CDR2, and CDR3 consisting of about 5 to 30 amino acid residues. In addition, CDRs of a heavy chain are particularly known for their contribution to binding of an antibody to an antigen. Among the CDRs, CDR3 is known to contribute the most in binding of an antibody to an antigen. For example, "Willy et al., Biochemical and Biophysical Research Communications Volume 356, Issue 1, 27 April 2007, Pages 124-128" describes that a heavy chain CDR3 was altered to elevate the binding capacity of an antibody. An Fv region other than the CDRs is called a framework region (FR), consisting of FR1, FR2, FR3, and FR4, which are conserved relatively well among antibodies (Kabat et al., "Sequence of Proteins of Immunological Interest" US Dept. Health and Human Services, 1983.) Specifically, it is understood that a factor characterizing the reactivity of an antibody is in CDRs, especially heavy chain CDRs.

A plurality of methods for defining CDRs and determining the positions thereof have been reported. For example, the Kabat definition (Sequences of Proteins of Immunological Interest, 5th ed., Public Health Service, National Institutes of Health, Bethesda, MD. (1991)) or the Chothia definition (Chothia et al., J. Mol. Biol., 1987; 196: 901-917) may be used. One embodiment of the invention uses the Kabat definition as an optimal example, but the definition is not necessarily limited thereto. Further, the definitions may be determined in some cases after considering both the Kabat definition and the Chothia definition. For example, an overlapping portion of CDR according to each of the definitions, or a portion comprising CDRs according to both of the definitions can be deemed the CDR. A specific example of such a method is the method of Martin et al using Oxford Molecular's AbM antibody modeling software, which is a proposal combining the Kabat definition and the Chothia definition (Proc.Natl.Acad.Sci.USA, 1989; 86: 9268-9272). Such CDR information can be used to produce a mutant that can be used in the present invention. Such an antibody mutant comprises one or several (e.g., 2, 3, 4, 5, 6, 7, 8, 9, or 10) substitutions, additions, or deletions in the framework of the original antibody. However, a mutant can be produced such that the CDR does not comprise a mutation.

As used herein, "antigen" refers to any substrate that can be specifically bound by an antibody molecule. As used herein, "immunogen" refers to an antigen, which can initiate lymphocyte activation that leads to an antigen specific immune response. As used herein, "epitope" or "antigen determinant" refers to a site in an antigen molecule to which an antibody or a lymphocyte receptor binds. A method of determining an epitope is well known in the art. Such an epitope can be determined by those skilled in the art by using a well-known and conventional technology when a primary sequence of an amino acid or a nucleic acid is provided. It is understood that the antibody of the invention having other sequences can be similarly used, as long as the epitope is the same.

It is understood that antibodies with any specificity may be used as the antibody used herein, as long as false positive reactions are reduced. Thus, the antibodies used herein may be a polyclonal antibody or a monoclonal antibody.

As used herein, "means" refers to anything that can be a tool for accomplishing an objective (e.g., detection, diagnosis, or therapy). As used herein, "selective recognition means" in particular refers to means capable of recognizing a certain subject differently from others.

As used herein, "marker (substance or gene)" refers to a substance that can be an indicator for tracking whether a target is in or at risk of being in a certain state (e.g., diseased state, disorder state, level of or presence of malignant state, or the like). Examples of such a marker include genes, gene products, metabolites, enzymes, and the like. In the present invention, detection, diagnosis, preliminary detection, prediction, or prediagnosis of a certain state (e.g., state of a disease such as cancer) can be materialized by using an agent or means specific to a marker associated with such a state, or a composition, kit, or system comprising the same or the like. As used herein, "expression product" (also referred to as a gene product) refers to a protein or mRNA encoded by a gene. It was found herein that a gene product (Glypican-1), which has not been proven for therapy of GPC-1 positive cancer such as esophageal cancer, can be used as a therapeutic target of GPC-1 positive cancer such as esophageal cancer.

"Cancer" targeted by the present invention includes one or more types selected from the group consisting of lung cancer, esophageal cancer, gastric cancer, liver cancer, pancreatic cancer, renal cancer, adrenal cancer, bile duct cancer, breast cancer, colon cancer, small intestine cancer, ovarian cancer, uterine cancer, bladder cancer, prostate cancer, ureteral cancer, renal pelvis cancer, ureteral cancer, penile cancer, testicular cancer, cerebral tumor, cancer of the central nervous system, cancer of the peripheral nervous system, head and neck cancer, glioma, glioblastoma multiform, skin cancer, melanoma, thyroid cancer, salivary gland cancer, malignant lymphoma, carcinoma, sarcoma, leukemia, and hematologic malignancy. Ovarian cancer in this regard includes, for example, ovarian serous adenocarcinoma and ovarian clear cell adenocarcinoma. Uterine cancer includes, for example, endometrial cancer and cervical cancer. Head and neck cancer includes, for example, oral cavity cancer, pharyngeal cancer, nasal cavity cancer, paranasal cancer, salivary gland cancer, and thyroid cancer. Lung cancer includes, for example, non-small-cell lung cancer and small cell lung cancer. Malignant tumor may be PD-L1 positive.

As used herein, "esophageal cancer" is used in the normal sense and used broadly in the sense to include cancer in the esophagus. Esophageal cancer includes, but is not limited to, squamous cell carcinoma as well as adenocarcinoma, esophageal cancer at a lymph node metastasis site, and the like. It is understood that about half the incidences of esophageal cancer in Japanese patients develop near the center of the esophagus in the chest, and then 1/4 of the incidences develops in the lower portion of the esophagus. It is understood that the present invention targets both types of esophageal cancers. Although not wishing to be bound by any theory, the present invention is expected to be usable as an indicator for all esophageal cancers including squamous cell carcinoma as well as adenocarcinoma, and esophageal cancer at a lymph node metastasis site.

As used herein, "pancreatic cancer" refers to a malignant tumor that has developed from the pancreas, but generally refers to pancreatic ductal adenocarcinoma, and encompasses tumors of other parts of the pancreas.

As used herein, "cervical cancer" is a type of uterine cancer. Uterine cancer includes cervical cancer and endometrial cancer. Cervical cancer develops from the portion called the cervix at the uterine entrance.

As used herein, "lung cancer" is a malignant tumor from epithelial cells developing in the lung, including small cell lung cancer, non-small cell lung cancer, and the like. Non-small cell lung cancer includes adenocarcinoma, squamous cell carcinoma, and large cell cancer.

As used herein, "head and neck cancer" is a tumor of the head and neck, referring to tumor developed in a portion from the face to the neck, such as the nose, mouth, throat, upper jaw, lower jaw, or ear.

As used herein, "breast cancer" is a tumor developed in the mamma, including ductal carcinoma, lobular carcinoma, and the like.

As used herein, "subject (person)" refers to a target subjected to diagnosis, detection, therapy, or the like of the invention (e.g., an organism such as a human or a cell, blood, serum, or the like extracted from an organism).

As used herein, "sample" refers to any substance obtained from a subject or the like. For example, serum and the like are encompassed thereby. Those skilled in the art can appropriately select a preferred sample based on the descriptions herein.

As used herein, "agent" is used broadly and may be any substance or other elements (e.g., energy, radiation, heat, electricity, and other forms of energy) as long as the intended objective can be achieved. Examples of such a substance include, but are not limited to, protein, polypeptide, oligopeptide, peptide, polynucleotide, oligonucleotide, nucleotide, nucleic acid (including, for example, DNAs such as cDNA and genomic DNA and RNAs such as mRNA), polysaccharide, oligosaccharide, lipid, organic small molecule (e.g., hormone, ligand, information transmitting substance, organic small molecule, molecule synthesized by combinatorial chemistry, small molecule that can be used as medicine (e.g., small molecule ligand and the like)) and composite molecule thereof. A substance that binds to Glypican-1 can also be such an agent. Typical examples of an agent specific to a polynucleotide include, but are not limited to, a polynucleotide having complementarity with a certain sequence homology (e.g., 70% or greater sequence identity) to

the sequence of the polynucleotide, polypeptide such as a transcription factor that binds to a promoter region, and the like. Typical examples of an agent specific to a polypeptide include, but are not limited to, an antibody directed specifically to the polypeptide or a derivative or analog thereof (e.g., single chain antibody), a specific ligand or receptor when the polypeptide is a receptor or ligand, a substrate when the polypeptide is an enzyme, and the like.

As used herein, "diagnosis" refers to identifying various parameters associated with a disease, disorder, condition (e.g., malignant tumor), or the like in a subject to determine the current or future state of such a disease, disorder, or condition. The condition in the body can be investigated by using the method, apparatus, or system of the invention. Such information can be used to select and determine various parameters of a formulation or method for the treatment or prevention to be administered, disease, disorder, or condition in a subject, or the like. As used herein, "diagnosis" when narrowly defined refers to diagnosis of the current state, but when broadly defined includes "early diagnosis", "predictive diagnosis", "prediagnosis", and the like. Since the diagnostic method of the invention in principle can utilize what comes out from a body and can be conducted away from a medical practitioner such as a physician, the present invention is industrially useful. In order to clarify that diagnosis can be conducted away from a medical practitioner such as a physician, the term as used herein may be particularly called "assisting" "predictive diagnosis, prediagnosis, or diagnosis".

As used herein, "detecting drug (agent)" or "inspection drug (agent)" broadly refers to all agents capable of detecting or inspecting a target of interest.

As used herein, "diagnostic drug (agent)" broadly refers to all agents capable of diagnosing a condition of interest (e.g., disease such as esophageal cancer or the like) .

As used herein, "therapy" refers to the prevention of exacerbation, preferably maintaining of the current state, more preferably alleviation, and still more preferably elimination of a disease or disorder (e.g., esophageal cancer) when such a condition has developed, including being capable of exerting a prophylactic effect or an effect of improving a disease of a patient or one or more symptoms accompanying the disease. Preliminary diagnosis with suitable therapy may be referred to as "companion therapy" and a diagnostic drug therefor may be referred to as "companion diagnostic agent".

As used herein, "therapeutic drug (agent)" broadly refers to all agents capable of treating a condition of interest (e.g., diseases such as esophageal cancer or the like). In one embodiment of the invention, "therapeutic drug" may be a pharmaceutical composition comprising an active ingredient and one or more pharmacologically acceptable carriers. A pharmaceutical composition can be manufactured, for example, by mixing an active ingredient and the carriers by any method known in the technical field of pharmaceuticals. Further, usage mode of a therapeutic drug is not limited, as long as it is used for therapy. A therapeutic drug may be the active ingredient alone or a mixture of an active ingredient and any ingredient. Further, the shape of the carriers is not particularly limited. For example, the carrier may be a solid or liquid (e.g., buffer solution). It should be noted that a therapeutic drug of esophageal cancer includes a drug (prophylactic drug) for preventing esophageal cancer or a growth inhibitor for esophageal cancer cells.

As used herein, "prevention (prophylaxis)" refers to the action of taking a measure against a disease or disorder (e.g., esophageal cancer) from being in such a condition prior to being in such a condition. For example, it is possible to use the agent of the invention to perform diagnosis, and optionally use the agent of the invention to prevent or take measures to prevent esophageal cancer or the like.

As used herein, "prophylactic drug (agent)" broadly refers to all agents capable of preventing a condition of interest (e.g., diseases such as esophageal cancer or the like).

As used herein, "interaction", for two substances, refers to applying a force (e.g., intermolecular force (Van der Waals force), hydrogen bond, hydrophobic interaction, or the like) between one substance and the other substance. Generally, two substances that have interacted are in a conjugated or bound state. The detection, inspection, and diagnosis in the invention can be materialized by utilizing such an interaction.

As used herein, the term "bond (binding)" refers to a physical or chemical interaction between two substances or between combinations thereof. A bond includes an ionic bond, non-ionic bond, hydrogen bond, Van der Waals bond, hydrophobic interaction, and the like. A physical interaction (bond) can be direct or indirect. Indirect physical interaction (bond) is mediated by or is due to an effect of another protein or compound. A direct bond refers to an interaction, which does not occur through or due to an effect of another protein or compound and does not substantially involve another intermediate.

Thus, an "agent" (or detection agent or the like) that "specifically" interacts (or binds) to a biological agent such as a polynucleotide or a polypeptide as used herein encompasses agents with affinity to the biological agent such as a polynucleotide or polypeptide that is typically similar or higher, preferably significantly (e.g., statistically significantly) higher, than affinity to other unrelated polynucleotides or polypeptides (especially those with less than 30% identity). Such affinity can be measured, for example, by hybridization assay, binding assay, or the like.

As used herein, "specific" interaction (or binding) of a first substance or agent with a second substance or agent refers to the first substance or agent interacting with (or binding to) the second substance or agent at a higher affinity than with substances or agents other than the second substance or agent (especially other substances or agents in a

sample comprising the second substance or agent). Examples of an interaction (or bond) specific to a substance or agent include, but are not limited to, hybridization in a nucleic acid, antigen-antibody reaction in a protein, enzyme-substrate reaction, other nucleic acid-protein reactions, protein-lipid interaction, nucleic acid-lipid interaction, and the like. Thus, if substances or agents are both nucleic acids, a first substance or agent "specifically interacting" with a second substance or agent encompasses the first substance or agent having at least partial complementarity to the second substance or agent. Further, examples of a first substance or agent "specifically" interacting with (or binding to) a second substance or agent if substances or agents are both proteins include, but are not limited to, interaction by an antigen-antibody reaction, interaction by a receptor-ligand reaction, enzyme-substrate interaction, and the like. If two types of substances or agents include a protein and a nucleic acid, a first substance or agent "specifically" interacting with (or binding to) a second substance or factor encompasses an interaction (or a bond) between an antibody and an antigen thereof. Such a specific interaction or binding reaction can be utilized to detect or quantify a target in a sample.

As used herein, "detection" or "quantification" of polynucleotide or polypeptide expression can be accomplished using a suitable method including, for example, an immunological measuring method and measurement of mRNAs, including a bond to or interaction with a detection agent, inspection agent, or diagnostic agent. Examples of a molecular biological measuring method include Northern blot, dot blot, PCR, and the like. Examples of an immunological measurement method include ELISA using a microtiter plate, RIA, fluorescent antibody method, luminescence immunoassay (LIA), immunoprecipitation (IP), single radial immunodiffusion (SRID), turbidimetric immunoassay (TIA), Western blot, immunohistochemical staining, and the like. Further, examples of a quantification method include ELISA, RIA, and the like. This may also be performed using a gene analysis method with an array (e.g., DNA array, protein array). DNA arrays are outlined extensively in (Ed. by Shujunsha, Saibo Kogaku Bessatsu "DNA Maikuroarei to Saishin PCR ho" [Cellular engineering, Extra issue, "DNA Microarrays and Latest PCR Methods"]. Protein arrays are discussed in detail in Nat Genet. 2002 Dec; 32 Suppl: 526-532. Examples of a method of analyzing gene expression include, but are not limited to, RT-PCR, RACE, SSCP, immunoprecipitation, two-hybrid system, in vitro translation, and the like, in addition to the methods described above. Such additional analysis methods are described in, for example, Genomu Kaiseki Jikkenho Nakamura Yusuke Labo Manyuaru [Genome analysis experimental method Yusuke Nakamura Lab Manual], Ed. by Yusuke Nakamura, Yodosha (2002) and the like. The entirety of the descriptions therein is incorporated herein by reference.

As used herein, "amount of expression" refers to the amount of polypeptide, mRNA, or the like that is expressed in a cell, tissue, or the like of interest. Examples of such an amount of expression include the amount of expression of the polypeptide of the invention at a protein level assessed by any suitable method including an immunological measurement method such as ELISA, RIA, fluorescent antibody method, Western blot, and immunohistochemical staining by using the antibody of the invention, and the amount of expression of the polypeptide used in the present invention at an mRNA level assessed by any suitable method including a molecular biological measuring method such as Northern blot, dot blot, and PCR. "Change in amount of expression" refers to an increase or decrease in the amount of expression of the polypeptide used in the present invention at a protein level or mRNA level assessed by any suitable method including the aforementioned immunological measuring method or molecular biological measuring method. A variety of detection or diagnosis based on a marker can be performed by measuring the amount of expression of a certain marker.

As used herein, "decrease" or "suppression" of activity or expression product (e.g., protein, transcript (RNA or the like)) or synonyms thereof refers to a decrease in the quantity, quality, or effect of a specific activity, transcript, or protein, or activity that decreases the same. Among decrease, "elimination" refers to activity, expression product, or the like being less than the detection limit and is especially referred to as "elimination". As used herein, "elimination" is encompassed by "decrease" or "suppression".

As used herein, "increase" or "activation" of activity or expression product (e.g., protein, transcript (RNA or the like)) or synonyms thereof refers to an increase in the quantity, quality, or effect of a specific activity, transcript, or protein, or activity that increases the same.

As used herein, "label" refers to an entity (e.g., substance, energy, electromagnetic wave, or the like) for distinguishing a molecule or substance of interest from others. Such a method of labeling includes RI (radioisotope) method, fluorescence method, biotin method, chemiluminescent method, and the like. When a plurality of markers of the invention or agents or means for capturing the same are labeled by a fluorescence method, labeling is performed with fluorescent substances having different fluorescent emission maximum wavelengths. It is preferable that the difference in fluorescent emission maximum wavelengths is 10 nm or greater. When labeling a ligand, any label that does not affect the function can be used. However, Alexa™ Fluor is desirable as a fluorescent substance. Alexa™ Fluor is a water-soluble fluorescent dye obtained by modifying coumarin, rhodamine, fluorescein, cyanine, or the like. This is a series compatible with a wide range of fluorescence wavelengths. Relative to other fluorescent dyes for the corresponding wavelength, Alexa™ Fluor is very stable, bright, and has a low pH sensitivity. Combinations of fluorescent dyes with fluorescence maximum wavelength of 10 nm or greater include a combination of Alexa™ 555 and Alexa™ 633, combination of Alexa™ 488 and Alexa™ 555, and the like. When a nucleic acid is labeled, any label can be used that can bind to a base portion thereof. However, it is preferable to use a cyanine dye (e.g., Cy3, Cy5 or the like of the CyDye™

series), rhodamine 6G reagent, 2-acetylaminofluorene (AAF), AAIF (iodine derivative of AAF), or the like. Examples of a fluorescent substance with a difference in fluorescent emission maximum wavelengths of 10 nm or greater include a combination of Cy5 and a rhodamine 6G reagent, a combination of Cy3 and fluorescein, a combination of a rhodamine 6G reagent and fluorescein, and the like. The present invention can utilize such a label to alter a target of interest to be detectable by the detecting means to be used. Such alteration is known in the art. Those skilled in the art can appropriately carry out such a method in accordance with the label and target of interest.

As used herein, "tag" refers to a substance for distinguishing a molecule by a specific recognition mechanism such as receptor-ligand, or more specifically, a substance serving the role of a binding partner for binding a specific substance (e.g., having a relationship such as biotin-avidin or biotin-streptavidin). A tag can be within the scope of "label". Accordingly, a specific substance to which a tag is bound can distinguish the specific substance by contacting a substrate, to which a binding partner of a tag sequence is bound. Such a tag or label is well known in the art. Typical examples of tag sequences include, but are not limited to, myc tag, His tag, HA, Avi tag, and the like. Such a tag may be bound to the detection agent, inspection agent, or diagnostic agent of the invention.

As used herein, "in vivo" refers to inside of a living organism. In a specific context, "in a living organism" refers to the position where a substance of interest should be disposed.

As used herein, "in vitro" refers to a state where a portion of a living organism is extracted or freed "outside of a living organism" (e.g., into a test tube) for various research purposes. This is a term that is an antonym of in vivo.

As used herein, "ex vivo" refers to a series of operations of a certain treatment performed outside the body, but the sample is intended to be returned into the body later. In the present invention, this can include an embodiment envisioned to treat a certain cell in an organism with an agent of the invention and return the cell to the patient.

As used herein, "kit" refers to a unit generally providing portions to be provided (e.g., inspection drug, diagnostic drug, therapeutic drug, antibody, label, manual, and the like) in two or more separate sections. This form of a kit is preferred when a composition that should not be provided in a mixed state and is preferably mixed immediately before use for safety reasons or the like is intended to be provided. Such a kit advantageously comprises an instruction or manual describing how the provided portions (e.g., inspection drug, diagnostic drug, or therapeutic drug) are used or how a reagent should be handled. When the kit is used herein as a reagent kit, the kit generally comprises an instruction describing how to use an inspection drug, diagnostic drug, therapeutic drug, antibody, and the like.

As used herein, "instruction" is a document explaining the method of use of the present invention for a physician or other users. The instruction has a description of the detection method of the invention, method of use of a diagnostic agent, or instruction to administer a medicament or the like. Further, an instruction may have a description instructing oral administration or administration to the esophagus (e.g., by injection or the like) as a site of administration. The instruction is prepared in accordance with a format specified by the regulatory agency of the country in which the present invention is practiced (e.g., the Ministry of Health, Labor and Welfare in Japan, Food and Drug Administration (FDA) in the U.S., or the like), with an explicit description showing approval by the regulatory agency. The instruction is a so-called package insert and is typically provided in, but not limited to, paper media. The instructions can also be provided in a form such as electronic media (e.g., web sites provided on the Internet or emails).

As used herein, "intracellular invasion (internalize/internalization) refers to intake of a substance bound to an antigen by a cell by endocytosis or phagocytosis mediated by the substance bound to an antigen on a cell surface. A substance bound to Glypican-1 having such activity (e.g., anti-Glypican-1 antibody) can make an active ingredient of interest undergo intracellular invasion into a cell expressing Glypican-1 on the cell surface to exert a desired effect due to the active ingredient of interest in Glypian-1 expressing cells. Examples of the active ingredient of interest include, but are not limited to, agents with cytotoxic activity, anticancer agents, contrast media, siRNAs, antisense nucleic acids, ribozymes, and the like.

As used herein, "antibody-drug conjugate (ADC)" refers to an antibody chemically linked to one or more active ingredients of interest, or an antigen binding fragment thereof. In a preferred embodiment, ADC is operably linked via a linker. As used herein, "operably linked" refers to a relationship where a linked substance can operate in a predicted manner. Examples of active ingredients of interest that can be contained in an ADC include, but are not limited to, agents with cytotoxic activity, anticancer agents, contrast media, siRNAs, antisense nucleic acids, ribozymes, and the like. A linker that can be used include cleavable linkers and non-cleavable linkers. Examples of cleavable linkers include, but are not limited to, linkers with a sequence cleaved by a protease, acid labile linkers, disulfide linkers, and the like. Examples of non-cleavable linkers include, but are not limited to, MCC linkers and the like.

As used herein, "cytotoxic activity" refers to, for example, inducing a pathological change to a cell, or inducing cell depth by not only direct trauma, but also any structural or functional damage to cells such as cleavage of DNA or formation of a dimer of a base, cleavage of a chromosome, damage to the cell division system, or reduction in various enzymatic activity to directly or indirectly block the function of cells. Therefore, examples agent having cytotoxic activity" include, but are not limited to, alkylating agents, tumor necrosis factor inhibitors, intercalators, microtubule inhibitors, kinase inhibitors, proteasome inhibitors, topoisomerase inhibitors, and the like.

As used herein, "half maximal inhibitory concentration ($IC_{50}$)" refers to a concentration of a compound required

for killing 50% of cells. $IC_{50}$ is measured herein using the method described in Example 7.

(Preferred Embodiments)

The preferred embodiments of the invention are explained hereinafter. It is understood that the embodiments provided hereinafter are provided to facilitate better understanding of the present invention, so that the scope of the invention should not be limited by the following descriptions. Thus, it is apparent that those skilled in the art can refer to the descriptions herein to make appropriate modifications within the scope of the invention. It is also understood that the following embodiments of the invention can be used individually or as a combination.

(Antibodies)

The inventors have found new clones of an antibody that specifically binds to human Glypican-1 as shown in Example 1. Thus, in one aspect, the present invention provides an anti-human Glypican-1 antibody or antigen binding fragment thereof, wherein the antibody is selected from the group consisting-of:

(a) an antibody comprising amino acid sequences of heavy chain CDR1, heavy chain CDR2, and heavy chain CDR3 set forth in SEQ ID NO: 53, 54, and 55, respectively, and light chain CDR1, light chain CDR2, and light chain CDR3 set forth in SEQ ID NO: 56, 57, and 58, respectively (clone K090-01a033);
(b) an antibody comprising amino acid sequences of heavy chain CDR1, heavy chain CDR2, and heavy chain CDR3 set forth in SEQ ID NOs: 5, 6, and 7, respectively, and light chain CDR1, light chain CDR2, and light chain CDR3 set forth in SEQ ID NOs: 8, 9, and 10, respectively (clone K090-01a002);
(c) an antibody comprising amino acid sequences of heavy chain CDR1, heavy chain CDR2, and heavy chain CDR3 set forth in SEQ ID NOs: 11, 12, and 13, respectively, and light chain CDR1, light chain CDR2, and light chain CDR3 set forth in SEQ ID NOs: 14, 15, and 16, respectively (clone K090-01a007);
(d) an antibody comprising amino acid sequences of heavy chain CDR1, heavy chain CDR2, and heavy chain CDR3 set forth in SEQ ID NOs: 17, 18, and 19, respectively, and light chain CDR1, light chain CDR2, and light chain CDR3 set forth in SEQ ID NOs: 20, 21, and 22, respectively (clone K090-01a016);
(e) an antibody comprising amino acid sequences of heavy chain CDR1, heavy chain CDR2, and heavy chain CDR3 set forth in SEQ ID NOs: 23, 24, and 25, respectively, and light chain CDR1, light chain CDR2, and light chain CDR3 set forth in SEQ ID NOs: 26, 27, and 28, respectively (clone K090-01a017);
(f) an antibody comprising amino acid sequences of heavy chain CDR1, heavy chain CDR2, and heavy chain CDR3 set forth in SEQ ID NOs: 29, 30, and 31, respectively, and light chain CDR1, light chain CDR2, and light chain CDR3 set forth in SEQ ID NOs: 32, 33, and 34, respectively (clone K090-01a021);
(g) an antibody comprising amino acid sequences of heavy chain CDR1, heavy chain CDR2, and heavy chain CDR3 set forth in SEQ ID NOs: 35, 36, and 37, respectively, and light chain CDR1, light chain CDR2, and light chain CDR3 set forth in SEQ ID NOs: 38, 39, and 40, respectively (clone K090-01a026);
(h) an antibody comprising amino acid sequences of heavy chain CDR1, heavy chain CDR2, and heavy chain CDR3 set forth in SEQ ID NOs: 41, 42, and 43, respectively, and light chain CDR1, light chain CDR2, and light chain CDR3 set forth in SEQ ID NOs: 44, 45, and 46, respectively (clone K090-01a009);
(i) an antibody comprising amino acid sequences of heavy chain CDR1, heavy chain CDR2, and heavy chain CDR3 set forth in SEQ ID NOs: 47, 48, and 49, respectively, and light chain CDR1, light chain CDR2, and light chain CDR3 set forth in SEQ ID NOs: 50, 51, and 52, respectively (clone K090-01a030);
(j) an antibody comprising amino acid sequences of heavy chain CDR1, heavy chain CDR2, and heavy chain CDR3 set forth in SEQ ID NOs: 59, 60, and 61, respectively, and light chain CDR1, light chain CDR2, and light chain CDR3 set forth in SEQ ID NOs: 62, 63, and 64, respectively (clone K090-01a042);
(k) an antibody comprising amino acid sequences of heavy chain CDR1, heavy chain CDR2, and heavy chain CDR3 set forth in SEQ ID NOs: 65, 66, and 67, respectively, and light chain CDR1, light chain CDR2, and light chain CDR3 set forth in SEQ ID NOs: 68, 69, and 70, respectively (clone K090-02a002);
(l) an antibody comprising amino acid sequences of heavy chain CDR1, heavy chain CDR2, and heavy chain CDR3 set forth in SEQ ID NOs: 71, 72, and 73, respectively, and light chain CDR1, light chain CDR2, and light chain CDR3 set forth in SEQ ID NOs: 74, 75, and 76, respectively (clone K090-02a006);
(m) an antibody comprising amino acid sequences of heavy chain CDR1, heavy chain CDR2, and heavy chain CDR3 set forth in SEQ ID NOs: 77, 78, and 79, respectively, and light chain CDR1, light chain CDR2, and light chain CDR3 set forth in SEQ ID NOs: 80, 81, and 82, respectively (clone K090-02a010);
(n) an antibody comprising amino acid sequences of heavy chain CDR1, heavy chain CDR2, and heavy chain CDR3 set forth in SEQ ID NOs: 83, 84, and 85, respectively, and light chain CDR1, light chain CDR2, and light chain CDR3 set forth in SEQ ID NOs: 86, 87, and 88, respectively (clone K090-02a014);

(o) an antibody comprising amino acid sequences of heavy chain CDR1, heavy chain CDR2, and heavy chain CDR3 set forth in SEQ ID NOs: 89, 90, and 91, respectively, and light chain CDR1, light chain CDR2, and light chain CDR3 set forth in SEQ ID NOs: 92, 93, and 94, respectively (clone K090-02a022);
(p) an antibody comprising amino acid sequences of heavy chain CDR1, heavy chain CDR2, and heavy chain CDR3 set forth in SEQ ID NOs: 95, 96, and 97, respectively, and light chain CDR1, light chain CDR2, and light chain CDR3 set forth in SEQ ID NOs: 98, 99, and 100, respectively (clone K090-02a034);
(q) an antibody comprising amino acid sequences of heavy chain CDR1, heavy chain CDR2, and heavy chain CDR3 set forth in SEQ ID NOs: 101, 102, and 103, respectively, and light chain CDR1, light chain CDR2, and light chain CDR3 set forth in SEQ ID NOs: 104, 105, and 106, respectively (clone K090-02a035);
(r) an antibody comprising amino acid sequences of heavy chain CDR1, heavy chain CDR2, and heavy chain CDR3 set forth in SEQ ID NOs: 107, 108, and 109, respectively, and light chain CDR1, light chain CDR2, and light chain CDR3 set forth in SEQ ID NOs: 110, 111, and 112, respectively (clone K090-02b006); and
(s) a mutant of an antibody selected from (a) to (r) comprising at least one substitution, addition, or deletion in a CDR moiety

In a preferred embodiment, the antibody or antigen binding fragment thereof that can be used in the present invention has sequences of heavy chain CDRs and light chain CDRs of the following clone: 01a033, 01a002, 02a010, 02a002, 02a014, 02b006, 01a042, 01a017, 01a026, 01a016, 01a030, or 01a009. These clones exhibited excellent EC50 values against TE14 cells as well as DU145 cells.

In a certain embodiment, the antibody of the invention is selected from the group consisting of:

(a) an antibody comprising amino acid sequences of a heavy chain set forth in SEQ ID NO: 158 and a light chain set forth in SEQ ID NO: 160 (clone K090-01a033);
(b) an antibody comprising amino acid sequences of a heavy chain set forth in SEQ ID NO: 126 and a light chain set forth in SEQ ID NO: 128 (clone K090-01a002);
(c) an antibody comprising amino acid sequences of a heavy chain set forth in SEQ ID NO: 130 and a light chain set forth in SEQ ID NO: 132 (clone K090-01a007);
(d) an antibody comprising amino acid sequences of a heavy chain set forth in SEQ ID NO: 134 and a light chain set forth in SEQ ID NO: 136 (clone K090-01a016);
(e) an antibody comprising amino acid sequences of a heavy chain set forth in SEQ ID NO: 138 and a light chain set forth in SEQ ID NO: 140 (clone K090-01a017);
(f) an antibody comprising amino acid sequences of a heavy chain set forth in SEQ ID NO: 142 and a light chain set forth in SEQ ID NO: 144 (clone K090-01a021);
(g) an antibody comprising amino acid sequences of a heavy chain set forth in SEQ ID NO: 146 and a light chain set forth in SEQ ID NO: 148 (clone K090-01a026);
(h) an antibody comprising amino acid sequences of a heavy chain set forth in SEQ ID NO: 150 and a light chain set forth in SEQ ID NO: 152 (clone K090-01a009);
(i) an antibody comprising amino acid sequences of a heavy chain set forth in SEQ ID NO: 154 and a light chain set forth in SEQ ID NO: 156 (clone K090-01a030);
(j) an antibody comprising amino acid sequences of a heavy chain set forth in SEQ ID NO: 162 and a light chain set forth in SEQ ID NO: 164 (clone K090-01a042);
(k) an antibody comprising amino acid sequences of a heavy chain set forth in SEQ ID NO: 166 and a light chain set forth in SEQ ID NO: 168 (clone K090-02a002);
(l) an antibody comprising amino acid sequences of a heavy chain set forth in SEQ ID NO: 170 and a light chain set forth in SEQ ID NO: 172 (clone K090-02a006);
(m) an antibody comprising amino acid sequences of a heavy chain set forth in SEQ ID NO: 174 and a light chain set forth in SEQ ID NO: 176 (clone K090-02a010);
(n) an antibody comprising amino acid sequences of a heavy chain set forth in SEQ ID NO: 178 and a light chain set forth in SEQ ID NO: 180 (clone K090-02a014);
(o) an antibody comprising amino acid sequences of a heavy chain set forth in SEQ ID NO: 182 and a light chain set forth in SEQ ID NO: 184 (clone K090-02a022);
(p) an antibody comprising amino acid sequences of a heavy chain set forth in SEQ ID NO: 186 and a light chain set forth in SEQ ID NO: 188 (clone K090-02a034);
(q) an antibody comprising amino acid sequences of a heavy chain set forth in SEQ ID NO: 190 and a light chain set forth in SEQ ID NO: 192 (clone K090-02a035);
(r) an antibody comprising amino acid sequences of a heavy chain set forth in SEQ ID NO: 194 and a light chain set forth in SEQ ID NO: 196 (clone K090-02b006); and (s) a mutant of an antibody selected from (a) to (r) comprising at least one substitution, addition, or deletion.

In a preferred embodiment, the antibody or antigen binding fragment thereof that can be used in the present invention has sequences of heavy chains and light chains of the following clone: 01a033, 01a002, 02a010, 02a002, 02a014, 02b006, 01a042, 01a017, 01a026, 01a016, 01a030, or 01a009. These clones exhibited excellent EC50 values against TE14 cells as well as DU145 cells.

In another embodiment, the mutant of the antibody described above can have an amino acid sequence that is at least about 50%, about 60%, about 70%, about 80%, about 90%, about 95%, about 98%, or about 99% identical to the amino acid sequence of the original antibody. The mutant of the antibody described above can comprise at least one substitution, addition, or deletion in a framework.

The antibody that can be used in the present invention preferably binds to human Glypican-1 with a binding affinity at a binding constant of about 10 nM or less, but it is understood that the binding affinity can be weaker than the above binding affinity if the antibody has internalization activity and desired ADC activity.

Those skilled in the art understand that an antibody is useful as an antibody used in an ADC or an antibody for diagnosis/detection if the antibody has internalization activity of at least about 30% with respect to GPC-1 positive cells (e.g., TE8 cells). This is because drug efficacy can be observed when any antibody having internalization activity of at least 30% is used in an ADC. Therefore, in a specific embodiment, the antibody of the invention has an internalization activity of about 30% or greater, about 40% or greater, about 50% or greater, about 60% or greater, about 70% or greater, about 80% or greater, or about 90% or greater with respect to GPC-1 positive cells (e.g., TE8 cells) after 6 hours from starting incubation with the antibody or antigen fragment thereof in an internalization assay. In another embodiment, the antibody of the invention has an internalization activity of about 30% or greater, about 40% or greater, about 50% or greater, about 60% or greater, about 70% or greater, about 80% or greater, or about 90% or greater with respect to GPC-1 positive cells (e.g., TE8 cells) after 2 hours from starting incubation with the antibody or antigen fragment thereof in an internalization assay. In another embodiment, the antibody of the invention has internalization activity of preferably about 50% or greater and more preferably about 60% or greater with respect to GPC-1 positive cells (e.g., TE8 cells) after 6 hours from starting incubation with the antibody or antigen fragment thereof in an internalization assay. In another embodiment, the antibody of the invention has internalization activity of preferably about 50% or greater and more preferably about 60% or greater with respect to GPC-1 positive cells (e.g., TE8 cells) after 2 hours from starting incubation with the antibody or antigen fragment thereof in an internalization assay. For example, internalization activity can be measured by an assay using TE8 cells as described in the Examples. A value obtained by subtracting the percentage of residual Glypican-1 on the cell surface from 100% was used as the % internalized herein.

In still another embodiment, the antibody of the invention has an internalization activity of at least about 60% or greater with respect to Glypican-1 positive cells expressing Glypican-1 at a high level on the cell surface. Expression of Glypican-1 at a high level means having anti-Glypican-1 antibody binding capacity of about 15000 or greater by using clone 01a033 in an assay using QIFIKIT®. The measurement method of antibody binding capacity is described in detail in Example 5.

In still another embodiment, the antibody of the invention can be an antibody selected from a monoclonal antibody, a polyclonal antibody, a chimeric antibody, a humanized antibody, a human antibody, a multifunctional antibody, a bispecific or oligospecific antibody, a single chain antibody, an scFV, a diabody, an sc(Fv)2 (single chain (Fv)2), and an scFv-Fc.

The antibody of the invention has high intracellular invasion (internalization) activity with respect to Glypican-1 positive cells. Such activity was not found in conventional anti-Glypican-1 antibodies. Therefore, the present invention can be used in various therapeutic applications that were not envisioned in conventional art by utilizing high internalization activity of the antibody of the invention. Therefore, in a specific embodiment, the present invention provides a pharmaceutical composition comprising the anti-Glypican-1 antibody described above. In still another embodiment, the pharmaceutical composition of the invention can be for intercellular invasion of an active ingredient. Examples of the active ingredient include, but are not limited to, agents with cytotoxic activity, anticancer agents, contrast media, siRNAs, antisense nucleic acids, ribozymes, and the like.

A siRNA is an RNA molecule having a double stranded RNA moiety consisting of about 15 to about 40 bases. An siRNA has a function of cleaving an mRNA of a target gene with a sequence that is complementary to an antisense strand of the siRNA, and suppressing the expression of the target gene. For example, the siRNA used herein is an RNA comprising a double stranded RNA moiety consisting of a sense RNA strand consisting of a sequence that is homologous to a contiguous RNA sequence in an mRNA encoding Glypican-1 in a Glypican-1 positive cell or an mRNA encoding a gene affecting cell viability, and an antisense RNA strand consisting of a sequence that is complementary to the sense RNA sequence.

Design and manufacture of such siRNA and mutant siRNA described below are within the technical competence of those skilled in the art. Those skilled in the art can appropriately select any contiguous RNA regions of mRNA that is a transcription product in a target cell to make a double stranded RNA corresponding to the region within the ordinary practice. Those skilled in the art can also appropriately select an siRNA sequence having a stronger RNAi effect from an mRNA sequence that is a transcription product of the sequence using a known method.

For the preparation of siRNA, conditions such as (1) absence of 4 or more consecutive G or C, (2) absence of 4 or more consecutive A or T, or (3) less than 9 bases of G or C can be added. The length of a double stranded RNA moiety, as bases, is 15 to 40 bases, preferably 15 to 30 bases, more preferably 15 to 25 bases, still more preferably 18 to 23 bases, and most preferably 19 to 21 bases. It is understood that the upper and lower limits thereof are not limited to such specific numbers. The limits can be any combination of the listed numbers. The terminal structure of a sense or antisense strand of siRNA is not particularly limited, but can be appropriately selected depending on the objective. For example, the structure can have a blunt end or a cohesive end (overhang), and a structural type with a protruding 3' terminus is preferred. An siRNA with an overhang consisting of several bases, preferably 1 to 3 bases, and more preferably 2 bases at the 3' terminus of a sense RNA strand and an antisense RNA strand is preferred for often having a significant effect of suppressing the expression of a target gene. The type of bases of an overhang is not particularly limited, which can be either a base constituting an RNA or a base constituting a DNA. Examples of a preferred overhang sequence include dTdT (2 bp of deoxy T) at the 3' end and the like. Examples of preferred siRNA include, but are not limited to, siRNA with dTdT (2 bp of deoxy T) at the 3' terminus of all siRNA sense/antisense strands.

Furthermore, it is also possible to use an siRNA in which one to several nucleotides are deleted, substituted, inserted, and/or added at one or both of the sense strand and antisense strand of the siRNA described above. One to several bases as used herein is not particularly limited, but is preferably 1 to 4 bases, more preferably 1 to 3 bases, and most preferably 1 to 2 bases. Specific examples of such mutations include, but are not limited to, mutations resulting in 0 to 3 bases at the 3' overhang portion, mutations that change the base sequence of the 3'-overhang portion to another base sequence, mutations resulting in the lengths of the sense RNA strand and antisense RNA strand being different by 1 to 3 bases due to insertion, addition, or deletion of bases, mutations substituting a base in the sense strand and/or antisense strand with another base, and the like. However, it is necessary that the sense strand and antisense strand can hybridize in such mutant siRNAs, and these mutant siRNAs have the ability to suppress gene expression that is equivalent to that of siRNAs without any mutation.

An siRNA can also be a molecule with a structure in which one end is closed, such as an siRNA with a hairpin structure (Short Hairpin RNA; shRNA). An shRNA is an RNA comprising a sense strand RNA of a specific sequence of a target gene, an antisense strand RNA consisting of a sequence complementary to the sense strand sequence, and a linker sequence for connecting the two strands, wherein the sense strand portion hybridizes with the antisense strand portion to form a double-stranded RNA portion.

In order to make the siRNA of the invention, a known method, such as a method using chemical synthesis or a method using a gene recombination technique, can be appropriately used. With a method using synthesis, a double-stranded RNA can be synthesized based on sequence information by using a common method. With a method using a gene recombination technique, an siRNA can be made by constructing an expression vector encoding a sense strand sequence and an antisense strand sequence and introducing the vector into a host cell, and then obtaining each of sense strand RNA and antisense strand RNA produced by transcription. It is also possible to make a desired double-stranded RNA by expressing an shRNA forming a hairpin structure, comprising a sense strand of a specific sequence of a target gene, an antisense strand consisting of a sequence complementary to the sense strand sequence, and a linker sequence for linking the two strands.

For an siRNA, all or some of nucleic acids constituting the siRNA can be a naturally-occurring or modified nucleic acid, as long as it has activity to suppress the expression of a target gene. A modified nucleic acid refers to a nucleic acid, which has a modification at a nucleoside (base moiety, sugar moiety) and/or an inter-nucleoside binding site and has a structure that is different from that of a naturally occurring nucleic acid.

In one embodiment, an active ingredient that is intracellularly migrated by the pharmaceutical composition of the invention can be an antisense nucleic acid. A technology that is well known to those skilled in the art can be used as a method of utilizing an antisense nucleic acid. An antisense nucleic acid can inhibit the expression of a target gene by inhibiting various processes such as transcription, splicing, or translation (Hirashima and Inoue, Shin-seikagaku Jikken Kouza 2 [New Biochemical Experiment Course 2] Kakusan IV Idenshi no Fukusei to Hatsugen [Replication and Expression of Gene of Nucleic Acid IV], Ed. by the Japanese Biochemical Society, Tokyo Kagaku Dojin, 1993, 319-347).

In one embodiment, designing an antisense sequence that is complementary to an untranslated region in the vicinity of the 5' terminus of mRNA encoding a gene in a target cell is considered effective for inhibiting the translation of the gene. A sequence that is complementary to a 3' untranslated region or a coding region can also be used. In this manner, the antisense nucleic acid used in the present invention also encompasses nucleic acids comprising an antisense sequence of the sequence of not only the translated region, but also the untranslated region of a gene. An antisense nucleic acid to be used is linked downstream of a suitable promoter, and preferably a sequence comprising a transcription termination signal is linked to the 3' side. A nucleic acid prepared in this manner can be transformed into a cell by using a known method. The sequence of an antisense nucleic acid is preferably a sequence that is complementary to a gene to be transformed or a portion thereof. However, such a sequence does not need to be fully complementary, as long as the gene expression can be effectively suppressed. A transcribed RNA preferably has complementarity that is 90% or greater, and most preferably 95% or greater, with respect to a transcript of a target gene. In order to effectively inhibit

the expression of a target gene using an antisense nucleic acid, it is preferable that the length of the antisense nucleic acid is at least 12 bases and less than 25 bases, but the antisense nucleic acid of the invention is not necessarily limited to this length. For example, the length may be 11 bases or less, 100 bases or more, or 500 bases or more. An antisense nucleic acid may be comprised of only DNA, but may comprise a nucleic acid other than DNAs, such as a locked nucleic acid (LNA). As one embodiment, an antisense nucleic acid used in the invention can be an LNA containing antisense nucleic acid comprising LNA at the 5' terminus or LNA at the 3' terminus. In an embodiment using the antisense nucleic acid in the present invention, the antisense sequence can be designed using the method described in, for example, Hirashima and Inoue, Shin-seikagaku Jikkenn Kouza 2 [New Biochemical Experiment Course 2] Kakusan IV Idenshi no Fukusei to Hatsugen [Replication and Expression of Gene of Nucleic Acid IV], Ed. by the Japanese Biochemical Society, Tokyo Kagaku Dojin, 1993, 319-347.

In one embodiment, an active ingredient that is intracellularly migrated by the pharmaceutical composition of the invention can be a ribozyme or DNA encoding a ribozyme. A ribozyme refers to an RNA molecule having catalytic activity. While there are ribozymes with various activities, a study focusing on ribozymes particularly as an enzyme for cleaving an RNA has made it possible to design a ribozyme that site-specifically cleaves an RNA. There are ribozymes with a size of 400 nucleotides or more as in M1 RNA included in RNase P and group I intron ribozymes, but there are also those with an active domain of about 40 nucleotides called hammerhead or hair-pin ribozymes (Makoto Koizumi and Eiko Otsuka, Protein, Nucleic Acid and Enzyme, 1990, 35, 2191).

Hairpin ribozymes are also useful for the objective of the invention. Such a ribozyme is found, for example, in the minus strand of a tobacco ringspot virus satellite RNA (Buzayan J M, Nature, 1986, 323, 349). It is demonstrated that target specific RNA-cleaving ribozymes can also be created from hairpin ribozymes (Kikuchi, Y. & Sasaki, N., Nucl. Acids Res, 1991, 19, 6751., Yo Kikuchi, Kagaku to Seibutsu [Chemistry and Biology], 1992, 30, 112).

(Complex)

In another aspect, the present invention provides a complex of a substance that binds to Glypican-1 and an agent having efficacy such as cytotoxic activity. As shown in the Examples, Glypican-1 is highly expressed only in specific cells. Thus, the complex of the invention can specifically act on Glypican-1 positive cells. More specifically, the complex of the invention targets Glypican-1 positive cells expressing Glypican-1 at a high level on a cell surface. Thus, the complex of the invention does not exhibit an effect of suppressing cell growth against lung cancer derived LK2 cell strains expressing Glypican-1 at a low level (Figure **12**). Expression of Glypican-1 at a high level means having anti-Glypican-1 antibody binding capacity of about 15000 or greater using clone 01a033 in an assay using QIFIKIT®. A measurement method of antibody binding capacity is described in detail in Example 5.

In some embodiments, a substance that binds to Glypican-1 can bind to an agent with toxic activity in any binding mode. For example, a substance that binds to Glypican-1 can covalently or non-covalently link to an agent having toxic activity. In a preferred embodiment, a substance that binds to Glypican-1 can be linked to an agent having cytotoxic activity via a linker. In such a case, the substance that bind to Glypican-1 is operably linked to the agent having cytotoxic activity. Therefore, a linker used in the complex of the invention can be any linker, as long as the substance that binds to Glypican-1 is operably linked to the agent having cytotoxic activity.

In a specific embodiment, it is preferable that a linker is stable in blood, but is cleaved after intracellular invasion. For example, a linker can be designed so that the linker has a cleavable site, which is not degraded by an enzyme that is present in blood, but is cleaved by an enzyme that is present only in cells. Examples of such a linker include linkers having intralysosomal enzyme cleaved sequence (valine-citrulline (Val-Citr)). Those skilled in the art can design an appropriate linker having a suitable enzyme cleaved site using a well-known method.

In still another specific embodiment, the linker used in the complex of the invention can comprise a cathepsin cleaved sequence. Such a linker has, for example,
the MC-Val-Citr-PAB- portion of the structure (MC-Val-Citr-PAB-agent)

[Chemical Formula 1]

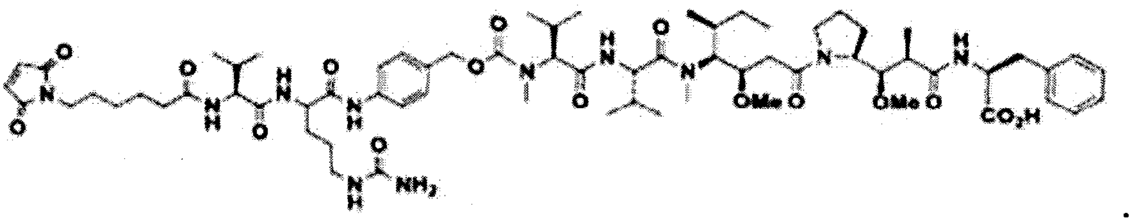

In another embodiment, a linker used in the complex of the invention can be an acid labile linker. An acid labile

linker responsive to a pH can be used by utilizing an acidic pH in the endosome after intracellular invasion. Examples of such a linker include hydrazone and the like.

In still another embodiment, a linker used in the complex of the invention can be a disulfide linker. Examples of such a linker include N-succinimidyl4-(2-pyridylthio)butanoate (SPDB), N-succinimidyl4-(2-pyridylthio)pentanoate (SPP), and the like.

A linker used in the complex of the invention can be a noncleavable linker. Examples of noncleavable linkers include, but are not limited to, maleimidomethylcyclohexane-1-carboxylate (MCC linker) and the like.

The agent can be any agent known to have cytotoxicity. Examples thereof include auristatin (monomethyl auristatin-E (MMAE), monomethyl auristatin-F (MMAF), and the like), maytansinoids (DM1 and DM4), calicheamicin, duocarmycin, pyrrolobenzothiazepine (PBD), and topoisomerase inhibitors. Those skilled in the art can appropriately select an agent that is used in a complex, depending on the sensitivity of the targeted cancer to an agent. The agent sensitivity with respect to cancer is well known in the art. Even if sensitivity of a specific cancer to a specific agent were not known, those skilled in the art can readily find the sensitivity of an agent with respect to a cancer cell strain by using a cell strain of a targeted cancer as in Example 2 or the like.

The complex of the invention can exhibit cytotoxic activity by invasion into cells. Therefore, in a certain embodiment, a substance that binds to Glypican-1 can have activity for intracellular invasion into a target cell. In another embodiment, cytotoxic activity can be exhibited by an agent having cytotoxic activity itself having cell permeability.

In some embodiments, the complex of the invention exhibits an $IC_{50}$ of about 0.5 nM ($5.0 \times 10^{-10}$ M) or less in Glypican-1 positive cells. In another embodiment, the complex of the invention exhibits an $IC_{50}$ of about 0.1 nM ($1.0 \times 10^{-10}$ M) or less in Glypican-1 positive cells. In still another embodiment, the complex of the invention exhibits an $IC_{50}$ of about 0.05 nM ($5.0 \times 10^{-11}$ M) or less in Glypican-1 positive cells. In a specific embodiment, the complex of the invention exhibits an $IC_{50}$ of about 0.03 nM ($3.0 \times 10^{-11}$ M) or less in Glypican-1 positive cells. In a more specific embodiment, the complex of the invention exhibits an $IC_{50}$ of about 0.02 nM ($2.0 \times 10^{-11}$ M) or less in Glypican-1 positive cells.

In a specific embodiment, a substance that binds to Glypican-1 can be an antibody or an antigen binding fragment thereof.

In a specific embodiment, a substance that binds to Glypican-1 can be an antibody or an antigen binding fragment thereof, wherein an epitope of the antibody can be:

(a) positions 33 to 61 of SEQ ID NO: 2;
(b) positions 339 to 358 and/or 388 to 421 of SEQ ID NO: 2;
(c) positions 430 to 530 of SEQ ID NO: 2;
(d) positions 33 to 61, 339 to 358, and/or 388 to 421 of SEQ ID NO: 2;
(e) positions 339 to 358, 388 to 421, and/or 430 to 530 of SEQ ID NO: 2; or
(f) positions 33 to 61, 339 to 358, 388 to 421, and/or 430 to 530 of SEQ ID NO: 2. More preferably, an epitope of the antibody can be or comprise:

 (a) positions 33 to 61 of SEQ ID NO: 2;
 (b) positions 339 to 358 and 388 to 421 of SEQ ID NO: 2;
 (c) positions 33 to 61, 339 to 358, and 388 to 421 of SEQ ID NO: 2; or
 (d) positions 33 to 61, 339 to 358, 388 to 421, and 430 to 530 of SEQ ID NO: 2.

In another specific embodiment, an antibody used in the complex of the invention is selected from the group consisting of:

(a) an antibody comprising amino acid sequences of heavy chain CDR1, heavy chain CDR2, and heavy chain CDR3 set forth in SEQ ID NOs: 53, 54, and 55, respectively, and light chain CDR1, light chain CDR2, and light chain CDR3 set forth in SEQ ID NOs: 56, 57, and 58, respectively (clone K090-01a033);
(b) an antibody comprising amino acid sequences of heavy chain CDR1, heavy chain CDR2, and heavy chain CDR3 set forth in SEQ ID NOs: 5, 6, and 7, respectively, and light chain CDR1, light chain CDR2, and light chain CDR3 set forth in SEQ ID NOs: 8, 9, and 10, respectively (clone K090-01a002);
(c) an antibody comprising amino acid sequences of heavy chain CDR1, heavy chain CDR2, and heavy chain CDR3 set forth in SEQ ID NOs: 11, 12, and 13, respectively, and light chain CDR1, light chain CDR2, and light chain CDR3 set forth in SEQ ID NOs: 14, 15, and 16, respectively (clone K090-01a007);
(d) an antibody comprising amino acid sequences of heavy chain CDR1, heavy chain CDR2, and heavy chain CDR3 set forth in SEQ ID NOs: 17, 18, and 19, respectively, and light chain CDR1, light chain CDR2, and light chain CDR3 set forth in SEQ ID NOs: 20, 21, and 22, respectively (clone K090-01a016);
(e) an antibody comprising amino acid sequences of heavy chain CDR1, heavy chain CDR2, and heavy chain CDR3

set forth in SEQ ID NOs: 23, 24, and 25, respectively, and light chain CDR1, light chain CDR2, and light chain CDR3 set forth in SEQ ID NOs: 26, 27, and 28, respectively (clone K090-01a017);

(f) an antibody comprising amino acid sequences of heavy chain CDR1, heavy chain CDR2, and heavy chain CDR3 set forth in SEQ ID NOs: 29, 30, and 31, respectively, and light chain CDR1, light chain CDR2, and light chain CDR3 set forth in SEQ ID NOs: 32, 33, and 34, respectively (clone K090-01a021);

(g) an antibody comprising amino acid sequences of heavy chain CDR1, heavy chain CDR2, and heavy chain CDR3 set forth in SEQ ID NOs: 35, 36, and 37, respectively, and light chain CDR1, light chain CDR2, and light chain CDR3 set forth in SEQ ID NOs: 38, 39, and 40, respectively (clone K090-01a026);

(h) an antibody comprising amino acid sequences of heavy chain CDR1, heavy chain CDR2, and heavy chain CDR3 set forth in SEQ ID NOs: 41, 42, and 43, respectively, and light chain CDR1, light chain CDR2, and light chain CDR3 set forth in SEQ ID NOs: 44, 45, and 46, respectively (clone K090-01a009);

(i) an antibody comprising amino acid sequences of heavy chain CDR1, heavy chain CDR2, and heavy chain CDR3 set forth in SEQ ID NOs: 47, 48, and 49, respectively, and light chain CDR1, light chain CDR2, and light chain CDR3 set forth in SEQ ID NOs: 50, 51, and 52, respectively (clone K090-01a030);

(j) an antibody comprising amino acid sequences of heavy chain CDR1, heavy chain CDR2, and heavy chain CDR3 set forth in SEQ ID NOs: 59, 60, and 61, respectively, and light chain CDR1, light chain CDR2, and light chain CDR3 set forth in SEQ ID NOs: 62, 63, and 64, respectively (clone K090-01a042);

(k) an antibody comprising amino acid sequences of heavy chain CDR1, heavy chain CDR2, and heavy chain CDR3 set forth in SEQ ID NOs: 65, 66, and 67, respectively, and light chain CDR1, light chain CDR2, and light chain CDR3 set forth in SEQ ID NOs: 68, 69, and 70, respectively (clone K090-02a002);

(l) an antibody comprising amino acid sequences of heavy chain CDR1, heavy chain CDR2, and heavy chain CDR3 set forth in SEQ ID NOs: 71, 72, and 73, respectively, and light chain CDR1, light chain CDR2, and light chain CDR3 set forth in SEQ ID NOs: 74, 75, and 76, respectively (clone K090-02a006);

(m) an antibody comprising amino acid sequences of heavy chain CDR1, heavy chain CDR2, and heavy chain CDR3 set forth in SEQ ID NOs: 77, 78, and 79, respectively, and light chain CDR1, light chain CDR2, and light chain CDR3 set forth in SEQ ID NOs: 80, 81, and 82, respectively (clone K090-02a010);

(n) an antibody comprising amino acid sequences of heavy chain CDR1, heavy chain CDR2, and heavy chain CDR3 set forth in SEQ ID NOs: 83, 84, and 85, respectively, and light chain CDR1, light chain CDR2, and light chain CDR3 set forth in SEQ ID NOs: 86, 87, and 88, respectively (clone K090-02a014);

(o) an antibody comprising amino acid sequences of heavy chain CDR1, heavy chain CDR2, and heavy chain CDR3 set forth in SEQ ID NOs: 89, 90, and 91, respectively, and light chain CDR1, light chain CDR2, and light chain CDR3 set forth in SEQ ID NOs: 92, 93, and 94, respectively (clone K090-02a022);

(p) an antibody comprising amino acid sequences of heavy chain CDR1, heavy chain CDR2, and heavy chain CDR3 set forth in SEQ ID NOs: 95, 96, and 97, respectively, and light chain CDR1, light chain CDR2, and light chain CDR3 set forth in SEQ ID NOs: 98, 99, and 100, respectively (clone K090-02a034);

(q) an antibody comprising amino acid sequences of heavy chain CDR1, heavy chain CDR2, and heavy chain CDR3 set forth in SEQ ID NOs: 101, 102, and 103, respectively, and light chain CDR1, light chain CDR2, and light chain CDR3 set forth in SEQ ID NOs: 104, 105, and 106, respectively (clone K090-02a035);

(r) an antibody comprising amino acid sequences of heavy chain CDR1, heavy chain CDR2, and heavy chain CDR3 set forth in SEQ ID NOs: 107, 108, and 109, respectively, and light chain CDR1, light chain CDR2, and light chain CDR3 set forth in SEQ ID NOs: 110, 111, and 112, respectively (clone K090-02b006);

(s) an antibody comprising amino acid sequences of heavy chain CDR1, heavy chain CDR2, and heavy chain CDR3 set forth in SEQ ID NOs: 113, 114, and 115, respectively, and light chain CDR1, light chain CDR2, and light chain CDR3 set forth in SEQ ID NOs: 116, 117, and 118, respectively (clone 4);

(t) an antibody comprising amino acid sequences of heavy chain CDR1, heavy chain CDR2, and heavy chain CDR3 set forth in SEQ ID NOs: 125, 126, and 127, respectively, and light chain CDR1, light chain CDR2, and light chain CDR3 set forth in SEQ ID NOs: 128, 129, and 130, respectively (clone 18); and

(u) a mutant of an antibody selected from (a) to (t) comprising at least one substitution, addition, or deletion.

In a preferred embodiment, the antibody or antigen binding fragment thereof that can be used in the present invention has sequences of heavy chain CDRs and light chain CDRs of the following clone: 01a033, 01a002, 02a010, 02a002, clone 18, 02a014, 02b006, 01a042, 01a017, 01a026, 01a016, 01a030, clone 4, or 01a009. These clones exhibited excellent EC50 values against TE14 cells as well as DU145 cells.

In still another embodiment, the antibody that is used in the complex of the invention is selected from the group consisting of:

(a) an antibody comprising amino acid sequences of a heavy chain set forth in SEQ ID NO: 158 and a light chain set forth in SEQ ID NO: 160 (clone K090-01a033);

(b) an antibody comprising amino acid sequences of a heavy chain set forth in SEQ ID NO: 126 and a light chain set forth in SEQ ID NO: 128 (clone K090-01a002);
(c) an antibody comprising amino acid sequences of a heavy chain set forth in SEQ ID NO: 130 and a light chain set forth in SEQ ID NO: 132 (clone K090-01a007);
(d) an antibody comprising amino acid sequences of a heavy chain set forth in SEQ ID NO: 134 and a light chain set forth in SEQ ID NO: 136 (clone K090-01a016);
(e) an antibody comprising amino acid sequences of a heavy chain set forth in SEQ ID NO: 138 and a light chain set forth in SEQ ID NO: 140 (clone K090-01a017);
(f) an antibody comprising amino acid sequences of a heavy chain set forth in SEQ ID NO: 142 and a light chain set forth in SEQ ID NO: 144 (clone K090-01a021);
(g) an antibody comprising amino acid sequences of a heavy chain set forth in SEQ ID NO: 146 and a light chain set forth in SEQ ID NO: 148 (clone K090-01a026);
(h) an antibody comprising amino acid sequences of a heavy chain set forth in SEQ ID NO: 150 and a light chain set forth in SEQ ID NO: 152 (clone K090-01a009);
(i) an antibody comprising amino acid sequences of a heavy chain set forth in SEQ ID NO: 154 and a light chain set forth in SEQ ID NO: 156 (clone K090-01a030);
(j) an antibody comprising amino acid sequences of a heavy chain set forth in SEQ ID NO: 162 and a light chain set forth in SEQ ID NO: 164 (clone K090-01a042);
(k) an antibody comprising amino acid sequences of a heavy chain set forth in SEQ ID NO: 166 and a light chain set forth in SEQ ID NO: 168 (clone K090-02a002);
(l) an antibody comprising amino acid sequences of a heavy chain set forth in SEQ ID NO: 170 and a light chain set forth in SEQ ID NO: 172 (clone K090-02a006);
(m) an antibody comprising amino acid sequences of a heavy chain set forth in SEQ ID NO: 174 and a light chain set forth in SEQ ID NO: 176 (clone K090-02a010);
(n) an antibody comprising amino acid sequences of a heavy chain set forth in SEQ ID NO: 178 and a light chain set forth in SEQ ID NO: 180 (clone K090-02a014);
(o) an antibody comprising amino acid sequences of a heavy chain set forth in SEQ ID NO: 182 and a light chain set forth in SEQ ID NO: 184 (clone K090-02a022);
(p) an antibody comprising amino acid sequences of a heavy chain set forth in SEQ ID NO: 186 and a light chain set forth in SEQ ID NO: 188 (clone K090-02a034);
(q) an antibody comprising amino acid sequences of a heavy chain set forth in SEQ ID NO: 190 and a light chain set forth in SEQ ID NO: 192 (clone K090-02a035);
(r) an antibody comprising amino acid sequences of a heavy chain set forth in SEQ ID NO: 194 and a light chain set forth in SEQ ID NO: 196 (clone K090-02b006);
(s) an antibody comprising amino acid sequences of a heavy chain set forth in SEQ ID NO: 198 and a light chain set forth in SEQ ID NO: 200 (clone 4);
(t) an antibody comprising amino acid sequences of a heavy chain set forth in SEQ ID NO: 202 and a light chain set forth in SEQ ID NO: 204 (clone 18); and
(u) a mutant of an antibody selected from (a) to (t) comprising at least one substitution, addition, or deletion.

In a preferred embodiment, the antibody or antigen binding fragment that can be used in the present invention has sequences of heavy chains and light chains of the following clone: 01a033, 01a002, 02a010, 02a002, clone 18, 02a014, 02b006, 01a042, 01a017, 01a026, 01a016, 01a030, clone 4, or 01a009. These clones exhibited excellent EC50 values against TE14 cells as well as DU145 cells.

In another embodiment, a mutant of the antibody described above can have an amino acid sequence that is at least about 50%, about 60%, about 70%, about 80%, about 90%, about 95%, about 98%, or about 99% identical to the amino acid sequence of the original antibody. The mutant of the antibody described above can comprise at least one substitution, addition, or deletion in a framework.

(Therapeutic drug)

In another aspect, the present invention provides a composition for preventing or treating Glypican-1 positive cancer. The composition of the invention comprises the complex described above of a substance that binds to Glypican-1 and an agent having cytotoxic activity. More specifically, the target of prevention or treatment of the composition of the invention is a Glypican-1 positive cell expressing Glypican-1 at a high level on a cell surface. Expression of Glypican-1 at a high level means having an anti-Glypican-1 antibody binding capacity of about 15000 or greater using clone 01a033 in an assay using QIFIKIT®. A measurement method of antibody binding capacity is described in detail in Example 5.

In some embodiments, a substance that binds to Glypican-1 can be an anti-Glypican-1 antibody or antigen binding fragment thereof having activity for intracellular invasion into Glypican-1 positive cells. An epitope of an anti-Glypican-1 antibody or antigen binding fragment thereof having activity for intracellular invasion into Glypican-1 positive cells can be:

(a) positions 33 to 61 of SEQ ID NO: 2;
(b) positions 339 to 358 and/or 388 to 421 of SEQ ID NO: 2;
(c) positions 430 to 530 of SEQ ID NO: 2;
(d) positions 33 to 61, 339 to 358, and/or 388 to 421 of SEQ ID NO: 2;
(e) positions 339 to 358, 388 to 421, and/or 430 to 530 of SEQ ID NO: 2; or
(f) positions 33 to 61, 339 to 358, 388 to 421, and/or 430 to 530 of SEQ ID NO: 2. More preferably, an epitope of an anti-glypican-1 antibody or antigen binding fragment thereof having activity for intracellular invasion into Glypican-1 positive cells can be or comprise:

(a) positions 33 to 61 of SEQ ID NO: 2;
(b) positions 339 to 358 and 388 to 421 of SEQ ID NO: 2;
(c) positions 33 to 61, 339 to 358, and 388 to 421 of SEQ ID NO: 2; or
(d) positions 33 to 61, 339 to 358, 388 to 421, and 430 to 530 of SEQ ID NO: 2.

In some embodiments, examples of anti-Glypican-1 antibody having activity for intracellular invasion into Glypican-1 positive cells include clone K090-01a002, K090-01a007, K090-01a016, K090-01a017, K090-01a021, K090-01a026, K090-01a009, K090-01a030, K090-01a033, K090-01a042, K090-02a002, K090-02a006, K090-02a010, K090-02a014, K090-02a022, K090-02a034, K090-02a035, K090-02b006, clone 4, and clone 18, and mutants of these antibodies comprising at least one substitution, addition, or deletion. These antibodies are described above in detail.

In some embodiments, Glypican-1 positive cancer is selected from esophageal cancer, pancreatic cancer, cervical cancer, lung cancer, head and neck cancer, breast cancer, uterine leiomyosarcoma, prostate cancer, and any combination thereof. Esophageal cancer can comprise esophageal cancer at a lymph node metastasis site, squamous cell carcinoma, and/or adenocarcinoma.

(Detection agent)

In still another aspect, the present invention provides a detection agent for identifying esophageal cancer, comprising an anti-Glypican-1 antibody or fragment thereof. The inventors have created 18 new clones of an atni-Glypican-1 antibody, and found that these clones have intracellular invasion (internalization) activity. Glypican-1 positive cancer (e.g., esophageal cancer) can be specifically detected by utilizing such a property to specifically detect cells into which a labeled anti-Glypican-1 antibody has intracellularly migrated.

The new clones disclosed herein have been found to have intracellular invasion (internalization) activity that was not observed in conventional antibodies. Therefore, in one embodiment, an anti-Glypican-1 antibody can be labeled. In a preferred embodiment, the label is in an inactive state in blood but is activated to be identifiable upon invasion into a Glypican-1 positive cell (e.g., esophageal cancer cell). As a result, Glypican-1 positive cells (e.g., esophageal cancer cells) can be detected with high specificity.

In a specific embodiment, examples of labels include RI (radioisotope), fluorescent labels, biotins, chemiluminescent labels, and the like. When a plurality of labels are used for labeling by the fluorescence method, fluorescent substances having different fluorescent emission maximum wavelengths are used for labeling. It is preferable that the difference in fluorescent emission maximum wavelengths is 10 nm or greater. Since an antibody is labeled, any substance can be used as long as the function of the antibody (especially the binding capacity or internalization activity) is not affected, but Alexa™ Fluor is desirable as the fluorescent substance. Alexa™ Fluor is a water-soluble fluorescent dye obtained by modifying coumarin, rhodamine, fluorescein, cyanine, or the like. This is a series compatible with a wide range of fluorescence wavelengths. Relative to other fluorescent dyes for the corresponding wavelength, Alexa™ Fluor is very stable, bright, and has low pH sensitivity. Examples of combinations of fluorescent dyes with fluorescence maximum wavelength of 10 nm or greater include, but are not limited to, a combination of Alexa™ 555 and Alexa™ 633, combination of Alexa™ 488 and Alexa™ 555, and the like.

In some embodiments, esophageal cancer can comprise esophageal cancer at a lymph node metastasis site, squamous cell carcinoma, and/or adenocarcinoma. In still another embodiment, an agent can be administered to a patient determined to have developed Glypican-1 positive esophageal cancer.

In another aspect, the present invention provides a method of using expression of Glypican-1 in a target sample as an indicator of esophageal cancer, the method comprising: contacting the detection agent described above with the target sample; measuring an amount of expression of Glypican-1 in the target sample; and comparing amounts of

expression of Glypican-1 in the target sample and a normal sample.

Since the new clones of anti-Glypican-1 antibodies disclosed herein can specifically detect esophageal cancer cells, the clones can also be provided as a diagnostic agent for determining whether a subject is in need of therapy of esophageal cancer.

The detection agent, inspection agent, or diagnostic agent of the invention can be used as a detection kit, inspection kit, or diagnostic kit.

(Kit)

In one aspect, the present invention provides a kit for detection, inspection, and/or diagnosis for carrying out the method for detection, inspection, and/or diagnosis according to the present invention. The kit comprises the detection agent, inspection agent, and/or diagnostic agent of the invention. As an embodiment thereof, any embodiment described herein can be used alone or in combination.

(Companion reagent)

In another aspect, the present invention provides a companion reagent for determining whether a subject is in need of cancer therapy with a Glypican-1 inhibitor, comprising a new clone of an anti-Glypican-1 antibody disclosed herein or the detection agent described above, wherein the reagent is contacted with a target sample, and an amount of expression of Glypican-1 in the target sample is measured, wherein the amount of expression of Glypican-1 in the target sample exceeding an amount of expression of Gypican-1 in a normal sample indicates that the target is in need of therapy with the Glypican-1 inhibitor. If a test is conducted on whether esophageal cancer is Glypican-1 positive in advance using a companion diagnostic drug, the therapeutic efficacy of esophageal cancer therapy targeting Glypican-1 can be examined. If the examination yields a result of Glypican-1 positive, the esophageal cancer therapy targeting Glypican-1 can be determined to be effective. In one embodiment of the invention, "companion diagnosis" comprises diagnosis that is conducted in order to assist optimal dosing by testing and predicting the individual difference in the efficacy and side effects of an agent for the patients.

In another aspect, the present invention provides a composition for preventing or treating malignant tumor comprising a complex of a substance that binds to Glypican-1 and an agent having cytotoxic activity, wherein a subject having the malignant tumor has a higher expression of Glypican-1 than a normal individual. In some embodiments, whether Glypican-1 is more highly expressed than in normal individuals can be determined by using the detection agent or diagnostic agent described above.

(Syngenic nonhuman animals)

In still another embodiment, the present invention provides a nonhuman animal (syngenic nonhuman animal) to which cells that express a cancer antigen are grafted, wherein the cancer antigen is syngenic with the nonhuman animal. Since the antigen expressed from grafted cells and proteins expressed in normal cells are proteins originating from the same animal for such an animal, the efficacy of an anticancer agent and toxicity to normal cells can be evaluated equally.

In some embodiments, cells expressing a cancer antigen are of cancer cell derived cell strain. In a specific embodiment, cells expressing a cancer antigen are of cancer cell-derived cell strain. In a specific embodiment, the cell strain can be a cell strain modified to express a cancer antigen given that the cancer antigen is syngenic with the nonhuman animal, or a cell strain naturally expressing the cancer antigen. In a preferred embodiment, a cancer antigen can evaluate the efficacy and/or toxicity of an anticancer agent with respect to Glypican-1 positive cancer (e.g., esophageal cancer, pancreatic cancer, cervical cancer, lung cancer, head and neck cancer, breast cancer, uterine leiomyosarcoma, or prostate cancer) by using a cell expressing Glypican-1. Examples of cell strains that can be used include, but are not limited to, LLC (mouse lung cancer cell strain), 4T1 (mouse breast cancer cell strain), MH-1 (mouse ovarian cancer cell strain), CT26 (mouse colon cancer cell strain), MC38 (mouse colon cancer cells strain), B1610 (mouse melanoma cell strain), and the like. In a preferred embodiment, a cell strain that can be used is an LLC cell strain.

In some embodiment, a nonhuman animal is a nonhuman mammal, preferably a rodent (e.g., mouse, rat, hamster, or the like). In a preferred embodiment, a nonhuman animal is a mouse (syngenic mouse).

In a specific embodiment in the present invention, a cell strain modified to express mouse Glypican-1 (e.g., LLC cell strain) can be used. In a preferred embodiment, the nonhuman animal is a mouse, and the cells are of an LLC cell strain modified to express mouse Glypican-1.

As used herein, "or" is used when "at least one or more" of the listed matters in the sentence can be employed. When explicitly described herein as "within the range of two values", the range also includes the two values themselves.

Reference literatures such as scientific literatures, patents, and patent applications cited herein are incorporated

herein by reference to the same extent that the entirety of each document is specifically described.

As described above, the present invention has been described while showing preferred embodiments to facilitate understanding. The present invention is described hereinafter based on Examples. The above descriptions and the following Examples are not provided to limit the present invention, but for the sole purpose of exemplification. Thus, the scope of the present invention is not limited to the embodiments and Examples specifically described herein and is limited only by the scope of claims.

[Examples]

The Examples are described hereinafter. The animals used in the following Examples were handled based on the Declaration of Helsinki while complying with the guidelines stipulated by the National Institutes of Biomedical Innovation, Health and Nutrition when needed. For reagents, the specific products described in the Examples were used. However, the reagents can be substituted with an equivalent product from another manufacturer (Sigma-Aldrich, Wako Pure Chemical, Nacalai Tesque, R & D Systems, USCN Life Science INC, or the like).

(Example 1: Antibody production)

(Materials and Methods)

In order to produce an mAb to human Glypican-1 (GPC1), 4 to 6 week old mice with an MRL or C3H background were immunized with a recombinant human GPC1 protein (R&D systems). After 4 to 5 intraperitoneal immunizations, lymphocytes were collected from the spleen and inguinal lymph node of the immunized mice. Total RNA was isolated from lymphocytes by using a reagent for RNA extraction (ISOGEN, NIPPN GENE CO., LTD.) First strand cDNA was synthesized with SuperScript III Reverse Transcriptase (Thermo Fisher Scientific Inc.) Immunoglobulin $V_H$ and $V_L$ genes were amplified by PCR from the resulting cDNA and inserted into a pSCCA5-E8d vector (MBL). PCR primers were designed in accordance with IMGT (http://www.imgt.org/). E. coli (DH12S, Thermo Fisher Scientific Inc.) was transformed with a plasmid DNA comprising a cDNA library. The number of independent transformants was estimated to be about $1.5 \times 10^8$. Next, the transformants were infected with helper phage M13K07. A phage expressing the scFv form of an antibody on the surface was obtained from the culture supernatant of the liquid medium. The obtained phase was selected by a known panning method (Marks et al., 1991; Suzuki et al., 2007; Kurosawa et al., 2008). In summary, a human Glypican-1 protein conjugated to Dynabeads (VERITAS) was used as an antigen in the first to third panning. Next, cell strain TE14 that expresses GPC1 on the surface was used for the 4th to 5th panning. The selected phages were screened by ELISA using a human GPC1 immobilized 96-well plate, and then were further screened by cell-ELISA using a cell strain stably transformed with human GPC1. Lastly, reactivity of the selected phages to human GPC1 was studied by flow cytometry (FC500, Beckman Coulter Inc.) by using human GPC1 transformants and TE14. 18 types of clones were obtained as a result.

To form a complete immunogloblulin (IgG) protein, the $V_H$ and $V_L$ genes of the obtained clones were incorporated into a genetically singular mouse IgG2a expression vector (Mammalian PowerExpress System, TOYOBO). The expression vector was straightened by an enzymatic reaction and transformed into a CHO-K1 cell strain by electroporation. After 10 days of culture, an antibody in an IgG form of the resulting clone was collected from the CHO culture supernatant and purified in an affinity column packed with nProteinA Sepharose 4 Fast Flow (GE healthcare). The reactivity of the purified antibody to human GPC1 was retested by flow cytometry using human GPC1 highly expressing cell strain TE14.

For clones #4, #17, and #18, antibodies were produced as described above based on sequence information obtained by the method described in International Publication No. WO 2015/098112, which is incorporated herein by reference.

(Example 2: ADC assay combining anti-GPC-1 antibody and MMAF binding secondary antibody)

(Materials and Methods)

(Examining anticancer agent sensitivity of TE14 cells)

2000 TE14 cells were added to a 96-well plate at 90 μl. The cells were cultured overnight at 37°C in a 5% CO2 incubator. The next day, 10 μl of an anticancer agent concentrated 10-fold relative to the final concentration was added to each well, so that the total amount was 100 μl. The cells were cultured for 6 days at 37°C in a 5% CO2 incubator. The cell viability was measured by using a CellTiter-Glo Luminescent Cell Viability Assay reagent to detect the amount of ATP. RPMI1640 + 10% FBS + 1% PS was used as the medium. MMAE (model number: 474645-27-7, ALB Technology) and MMAF (model number: 745017-94-1, ALB Technology) were used as the anticancer agent.

(ADC used in the assay)

Figure **1** depicts a schematic diagram of an ADC used in an assay. This Example used a secondary antibody (MORADEC) to the anti-GPCI antibody made in Example 1, to which an anticancer agent (MMAF) was bound via a linker.
This Example used the following clone sets.

[Table 1]

| | |
|---|---|
| **1set** | **mIgG2a biolegend** |
| | **clone4** |
| | **clone17** |
| | **clone18** |
| | **02a010** |
| | **02a014** |
| | **02a022** |
| | **02a034** |
| | |
| **2 set** | **mIgG2a biolegend** |
| | **02a035** |
| | **02b006** |
| | **01a002** |
| | **01a007** |
| | **01a01b** |
| | **01a017** |
| | **01a021** |
| | |
| **3 set** | **mIgG2a biolegend** |
| | **01a026** |
| | **01a009** |
| | **01a030** |
| | **01a033 01a042** |
| | **02a002** |
| | **02a006** |

2000 TE14 cells were added to a 96-well plate at 80 μl. The cells were cultured overnight at 37°C in a 5% CO2 incubator. The next day, 10 μl of an anticancer agent bound secondary antibody and 10 μl of a primary antibody concentrated 10-fold relative to the final concentration was added to each well, so that the total amount was 100 μl. The cells were cultured for 6 days at 37°C in a 5% CO2 incubator. The cell viability was measured by using a CellTiter-Glo Luminescent Cell Viability Assay reagent to detect the amount of ATP. RPMI1640 + 10% FBS + 1% PS was used as the medium. The final concentrations of the primary antibody were 0, 0.004, 0.0156, 0.0625, 0.25, and 1.0 nM. 2 μg/ml of Fab-aMFc-CL-MMAF (model number: AM202AF-50, Moradec) was used as the secondary antibody.

(Results)

The results are shown in Figure **2.** The anticancer agents MMAE and MMAF exhibited an $IC_{50}$ of 0.926 nM and 26.8 nM, respectively. TE14 cells were confirmed to be anticancer sensitive.
Figure **3** shows results of an ADC assay of clones exhibiting an $IC_{50}$ of 0.5 nM or less. Although not wishing

to be bound by any theory, this value is used herein as a baseline for evaluating an ADC as exhibiting a certain degree of activity. ADC using clone 01a033 exhibited the highest ADC activity. It is understood that a high ADC activity of 0.5 nM or less is exhibited due to having a high internalization activity. Thus, it is suggested that epitopes that are important for internalization are, as shown in Figure **3,** (a) positions 33 to 61 of SEQ ID NO: 2; (b) positions 339 to 358 and 388 to 421 of SEQ ID NO: 2; (c) positions 33 to 61, 339 to 358, and 388 to 42 of SEQ ID NO: 2; and (d) positions 33 to 61, 339 to 358, 388 to 421, and 430 to 530 of SEQ ID NO: 2.

Figures **4** and **5** show results of conducting ADC assays using LK2, TE8, and TE14 cell strain. An antitumor effect due to an ADC of an anti-GPC-1 antibody was hardly exhibited with LK2, which is a lung cancer derived cell strain with low expression of Glypican-1, while a potent antitumor effect due to an ADC of an anti-GPC-1 antibody was exhibited with TE8 and TE14, which are esophageal cancer derived cell strains. An ADC of clone 01a033 exhibited a particularly potent antitumor effect on TE8 and TE14 while exhibiting high specificity.

(Example 3: Production of ADC)

The ADC of the invention was produced as follows.

*140 ml of 1 mg/ml BioLegend mouse IgG2aK isotype control MOPC173 (Part# 92394, lot# B222287) and 70 ml of 2 mg/ml mouse anti-GPC-1 clone 01a033 (lot# 160316) were obtained.
*First, conjugation was performed in a small scale. 2 ml of αGPC1 was concentrated to about 0.8 ml (4.4 mg/ml), and 2 ml of MOPC173 was concentrated to about 0.4 ml (4.4 mg/ml). Different reducing conditions were set using 50 μl of each mAb for each condition to achieve a final drug-antibody ratio (DAR) of about 4. mAb was conjugated to maleimidocaproyl-valine-citrulline-p-aminobenzyloxy carbonyl-monomethyl auristatin-F (MC-vc-PAB-MMAF). The conjugation was performed using a maleimide-cysteine based method of first reducing an inter-strand disulfide bond of mAb by TCEP at 37°C, and then binding a maleimide moiety of a drug to the reduced cysteine. The profile of a complex was analyzed by hydrophobic interaction chromatography (HIC).
*Next, optimal conditions for conjugation in a large scale determined in small scale were used. First, 138 ml of MOPC173 was concentrated to a 28 ml (4.88 ml/ml) stock, and 68 ml of αGPC1 was concentrated to a 26.2 ml (5.14 mg/ml) stock. mAb was reduced with TCEP at 37°C, and then reacted with drug-linker-MC-vc-PAB-MMAF.
*The ADC was desalinated with a Sephadex G50 column, and unreacted toxins were removed, and then the buffer was exchanged with PBS. The ADC was then filtered and sterilized, and diluted to 1 mg/ml with a sterilized PBS buffer. The ADC was fractionated into a 1 ml tube and then stored at 4°C.
*The drug-antibody ratio (DAR) determined by the ratio of $A_{248nm}$:$A_{280nm}$ was 3.8 for MOPC173-CL-MMAF, and 4.1 for αGPC1-CL-MMAF.
*Size-exclusion chromatography (SEC) and hydrophobic interaction chromatography (HIC) were used to analyze unconjugated mAb and two ADCs. According to the analysis of unconjugated mAb before and after concentration by SEC and HIC, the properties of mAb did not change even when the mAb was concentrated to about 5 mg/ml. MOPC173 was present as a monomer, and αGPC1 was also present mostly as a monomer, but a small aggregation peak was observed. Both mAbs conjugated well with MC-vc-PAB-MMAF. The HIC profile of ADC exhibited excellent peaks of DAR = 2, 4, 6, and 8, and enables peak integration method for determining DAR. Figure **6** depicts a schematic diagram of a structure of an exemplary ADC.

(Example 4: Antigen affinity analysis of an unlabeled antibody and GPC1 ADC)

(Materials and Methods)

(Buffer preparation)

The buffer used in this Example was prepared as follows.

*Coating buffer: PBS (-).
*Dilution buffer: 10% Block Ace was used as the diluent. 10% Block Ace was prepared by dissolving 4g of Block Ace powder into 100 mL of MiliQ, and then diluting 10-fold with PBS (-). *Blocking buffer: A buffer was prepared by dissolving 4 g of Block Ace powder into 100 mL of MiliQ, and then diluting 4-fold with PBS (-). A solution prepared by dissolving 4 g of Block Ace powder into 100 mL of MiliQ was used as the stock solution (100% solution).
*Washing buffer: 1 pack of PBS-T (Sigma, P3563) was dissolved in 1 L of ultrapure water (0.05% Tween 20/PBS).

(Primary antibody preparation)

The primary antibodies used in this Example were prepared. GPC1-CL-MMAF and anti-GPCl monoclonal antibodies (01a033) were diluted stepwise to 100, 20, 4, 0.8, 0.16, 0.032, 0.0064, 0.00128, 0.000256, 0.0000512, and 0.00001024 nM with a dilution buffer. Recombinant human Glypican-1 proteins (CF, model number: 4519-GP-050, volume: 50 μg) were used as the antigen.

(Methodology)

Recombinant human GPC1 was diluted to 2.5 μg/mL with PBS (-) and added to 64 wells of Immuno 96well MicroWell Solid Plate (Maxisorp, Nunc) at 50 μL/well, and the plate was sealed. The plate was shaken, and liquid was confirmed to spread throughout the plate. Next, the following procedure was followed.

*Incubate (4°C overnight)
*Wash (200 μL/well × 3)
*Add blocking buffer to the 96-well microplate at 200 μL/well.
*React for 1 hour at 37°C.
*Wash (200 μL/well, three times)
*Dilute (1) GPC1-CL-MMAF and (2) anti-GPCl monoclonal antibody (01a033) with a dilution buffer in a 10-fold dilution series from 100 nM, and add the mixture to the 96-well microplate at 100 μL/well.
*React for 1 hour at 37°C.
*Wash (200 μL/well × 3)
*Dilute Anti-Mouse IgG (H+L) Antibody, Human Serum Adsorbed and Peroxidase labeled (KPL, cat.No.: 474-1806) with a dilution buffer (10% Block Ace) to 0.2 μg/ml (prepare a 5000-fold dilution upon use), and add the mixture to the 96-well microplate at 100 μL/well.
*React for 1 hour at 37°C.
*Wash (200 pL/well, three times)
*Return TMB (SurModics, TMBW-1000-01) to normal temperature before use, and add the TMB to the 96-well microplate at 100 μL/well.
*Agitate (protected from light, room temperature)
*Discontinue at 50 μL/well of 1N-sulfuric acid.
*Measure absorbance at 450 nm.
*Analyze data with GraphPad Prism 6.04.

(Results)

Results are shown in Figure **7.** Controls mIgG2a and mIgG2a ADCs did not exhibit any affinity to an antigen, whereas 01a033 and 01a033 ADCs exhibited high affinity. Further, the affinity levels of 01a033 and 01a033 ADCs are about the same, indicating that the binding capacity of the antibody itself is not compromised when a drug is conjugated.

(Example 5: Analysis of antibody binding capacity)

The amount of antigen per one cell of Glypican-1 expressed on the cell surface of various cell strains was quantified as Antibody Binding Capacity (ABC) by using QIFIKIT®. Measurement was taken in accordance with the instruction manual of QIFIKIT® by using anti-Glypican-1 antibody clone 01a033 as the antibody. $1 \times 10^5$ cells and 10 μg/ml of clone 01a033 were used.

(Results)

As is apparent from Figure **8,** GPC1 was highly expressed on the surface of esophageal cancer cell strains TE4, TE5, TE6, TE8, TE9, TE10, TE11, TE14, and TE15 cells. Human lung squamous cell carcinoma derived cell strain LK2 cells exhibited low expression. Human pancreatic cancer cell strains BxPC3 cells and T3M4 cells, and cervical cancer cell strains HeLa cells and ME180 cells also exhibited the same degree of expression as esophageal cancer cell strains. Figure **9** shows data from analyzing the reactivity of GPC1 and an isotype control antibody or a clone 01a033 antibody in various cancer cell strains by FACS. It can be seen that the reactivity with a clone 01a033 antibody is low and the amount of expression of GPC1 is low in LK2 cells.

(Example 6: Internalization assay on GPC1 ADC (01a033))

This Example studied the internalization (intracellular invasion) activity using various GPC1 positive cell strains for GPC1 ADC (01a033) MMAF.

(Materials and Methods)

Detection was performed on cells treated with GPC1 ADC (01a033) MMAF with anti-GPCl mAb (biotin-labeled 02b006) and PE-labeled streptavidin.

TE8, HeLa, ME180, BxPC3, and T3M4 cell strains were detached and collected from a 10 cm plate using 0.02% EDTA. Cells were suspended in RPMI1640 + 10% FBS + 100 U/ml penicillin +100 μg/ml streptomycin, and two 1.5 ml tubes were created at 1.0 × $10^6$ cells/0.9 ml. To lower the internalization activity, the tubes were left standing on ice for 1 hour. 1 mg/ml of GPC1 ADC (01a033) MMAF was diluted to 24 μg/mL (160 nM) with ice-cooled RPMI1640 + 10% FBS + 100 U/ml penicillin +100 μg/ml streptomycin. 100 μl of the ADC was added and suspended in the cell suspension and incubated on ice for 30 minutes to stain the antigens on the cell surface. After 30 minutes, the cell suspension was dispensed into 7 tubes each at 100 μl each. The cell suspension was separated into 4°C incubation group and 37°C incubation group in 14 tubes. The incubation times were 0 h, 1 h, 2 h, 3 h, 4 h, 6 h, 12 h, and 24 h. After reaching the incubation time, the cells were centrifuged at 1500 rpm at 4°C for 5 minutes to remove the supernatant. The cells were washed with 100 μl of ice cooled PBS + 0.2% BSA and subjected to centrifugation to wash the cells. Washing operation was performed three times in total. 50 μL of biotin-labeled anti-GPCl antibody 02b006 at a concentration of 1 μg/ml was added and suspended. The antigens on the cell surface were stained for 30 minutes on ice (GPC1 that was not internalized is stained). After reaching the incubation time, the cells were centrifuged at 1500 rpm at 4°C for 5 minutes to remove the supernatant. The cells were washed with 100 μl of ice cooled PBS + 0.2% BSA and subjected to centrifugation to wash the cells. Washing operation was performed three times in total. Streptavidin-PE (PharmMingen, #554061)was diluted 200-fold with ice-cooled PBS + 0.2% BSA. 50 μL was added to suspend the cells. The suspension was allowed to react under protection from light for 30 minutes on ice. After reaching the incubation time, the cells were centrifuged at 1500 rpm at 4°C for 5 minutes to remove the supernatant. The cells were washed with 100 μl of ice cooled PBS + 0.2% BSA and subjected to centrifugation to wash the cells. Washing operation was performed three times in total. 150 μL of ice-cooled PBS + 0.2% BSA was added for measurement with FACS Canto II and analysis with FlowJo ver 8.87.

(Results)

The results are shown in Figures **10** and **11.** Figure **10** shows histograms of the amount of Glypican-1 remaining on the TE8 cell surface for each elapsed time after addition of clone 01a033 ADC. The blue line is the result of staining with clone 02b006 in the absence of 01a033 ADC. This state would exhibit the maximum reactivity (100% of Glypican-1 remains on the cell surface). Since treatment at 4°C stops the physiological function of the cells, this is a condition under which endocytosis is suppressed and internalization does not occur, whereas 37°C is a temperature that is the most suitable for internalization. It can be seen from the orange color that Glypican-1 on the cell surface decreases with the passage of incubation time at 37°C with addition of antibodies. Meanwhile, the residual amount of Glypican-1 on the cell surface hardly changed at 4°C as shown in green. Figure **11** shows a value found by subtracting the percentage of residual Glypican-1 on the cell surface from 100% as % internalized. Figure **11** clearly shows that the anti-Glypican-1 antibody of the invention has high internalization activity against various cell strains including esophageal cancer, pancreatic cancer, and cervical cancer derived cell strains.

(Example 7: in vitro ADC assay using anti-GPCl antibody)

(Materials and Methods)

Preparation of a conjugate of mouse anti-Glypican-1 mAb (01a033), mouse IgG2a (biolegend #400224) as a control, and mc-vc-PAB-MMAF was commissioned to MORADEC. An anticancer agent was conjugated to a cysteine residue via a cleavable linker. The DAR (drug-antibody ratio) was as follows. GPC1-CL-MMAF DAR = 3.8, mouse IgG2a-CL-MMAF DAR = 4.1.

Specifically, the following assay was conducted.

(1) The cells were seeded on Thermo Fisher Scientific's 96-well white plate (model no.: 136101) (90 μl cell suspension). RPMI1640 + 10% FBS + 100 U/ml penicillin + 100 μg/ml streptomycin was used as the medium. The cells were seeded in 60 wells, and 100 μL of medium was added to 36 wells, and cultured in a 5% CO2 incubator at 37°C.
(2) The next day, 10 μL of ADC was added to the cells (total amount: 100 μL).

(3) After 144 hours of culture, a CellTiter-Glo™ Luminescence Cell Viability Assay reagent (Promega) was added and mixed at 100 μL/well.

(4) Measurements were taken with a plate reader.

(5) Analysis was conducted with GraphPad Prism 6.

The $IC_{50}$ values were calculated from the following equation (Hossain MM, Hosono-Fukao T, Tang R, Sugaya N, van Kuppevelt TH, Jenniskens GJ, Kimata K, Rosen SD, Uchimura K (2010) Direct detection of HSulf-1 and HSulf-2 activities on extracellular heparan sulphate and their inhibition by PI-88. Glycobiology 20(2): 175-186.)

$$IC50 = 10^{\wedge}(Log[A][B] \times (50-C)/(D-C) + Log[B])$$

A: High concentration straddling 50%

B: Low concentration straddling 50%

C: Inhibition rate at B

D: Inhibition rate at A

(Results)

Figures **12** and **13** show results for antitumor effect on each cell strain. As shown, GPC1 ADC exhibits an effect of suppressing cell growth against GPC1 positive esophageal cancer cell strains. Interestingly, an antitumor effect was hardly exhibited against LK2 cells (human lung squamous cell carcinoma derived cell strain) that hardly express GPC1 (Figure **12**). This indicates that ADC exerts an antitumor effect specifically against GPC1 positive cells. Surprisingly, it was revealed that GPC1 ADC also exhibits an effect of suppressing cell growth on pancreatic cancer derived cell strains BxPC3 cells and T3M4 cells and cervical cancer derived cell strains HeLa cells and ME180 cells (Figure **13**). This indicates that the ADC of the invention also exerts the same effect of suppressing cell growth on GPC1 positive cancer (e.g., pancreatic cancer, cervical cancer, and the like) highly expressing GPC1 in the same manner as esophageal cancer. Meanwhile, the ADC of the invention did not exhibit an effect of suppressing cell growth against LK2 cell strains that express GPC1 at a low level. Figure **14** is a table summarizing $IC_{50}$ deduced from the results obtained from Figures **12** and **13.**

(Example 8: Safety test on GPC1 ADC)

(Materials and Methods)

A single dose (1 ml) of control PBS, control ADC (50 mg/kg), anti-GPCI ADC (3 mg/kg), anti-GPCI ADC (15 mg/kg), and anti-GPCI ADC (50 mg/kg) was intraperitoneally administered on day 1 to a group of 4 male and 4 female C57BL/6J mice (8W). The mice were dissected after 7 days from administration.

Endpoints

*Blood collection (Blood cell count, biochemical test)

(Results)

The results are shown in Figure **15.** Significant change in the body weight was not observed in mice other than the group of mice administered with extremely large dose such as 50 mg/kg of ADC. As described in detail in the following Examples, sufficient antitumor effect is exhibited even with administration of the GPC1 ADC of the invention at 1 mg/kg. Thus, it is understood that an antitumor effect is achieved without exhibiting toxicity within the normal range of dosages. Figure **16** shows results of a hematological test. While an increase in the while blood cells was observed in a high dose administration group, this was not GPC1-ADC specific. Mild anemia was observed only in the 50 mg/kg ADC administration group. Abnormal platelet count specific to each group was not observed. Figure **17** shows results of a hematological biochemical test. An abnormality in the liver function was found in the high dose administration group, but this was not GPC1-ADC specific. Specific abnormality was not observed for amylase. Significant toxicity due to ADC administration was not observed from the results of the hematological test.

(Example 9: In vivo efficacy test on GPC1 ADC using pancreatic cancer cell strain)

Figure **18** schematically shows the test in this Example. Specifically, 5.0 × $10^6$ cells of the BxPC3 cell strain were subcutaneously grafted into female SCID mice. When the tumor size reached about 130 $mm^3$ after grafting, administration of PBS as a control, control ADC (10 mg/kg), anti-GPCl ADC (1 mg/kg), anti-GPCl ADC (3 mg/kg), and anti-GPC1 ADC (10 mg/kg) was started, which were intravenously administered on day 0, day 4, day 8, and day 12, with the day starting the administration considered 0 day. The tumor volume and body weight were measured on day 0, day 4, day 8, day 12, day 16, day 20, day 24, day 28, day 32, and day 36.

After subcutaneous tumorigenesis of BxPC3, (1) PBS, (2) control ADC at 10 mg/kg, and (3) GPC1-ADC at 1 mg/kg, 3 mg/kg, and 10 mg/kg were administered once into the tail vein. The tumor was extracted after 24 hours. After deparaffinization of a section of a paraffin embedded tissue, the slice was dehydrated with alcohol. Immunohistochemical staining on Phospho-Histon H3 (SerlO) was performed using an anti-Phospho-Histon H3 (SerlO) antibody (Cell Signaling Technology: #9701) and ChemMate Envision kit HRP 500T (Dako: K5007).

(Results)

The results are shown in Figures **19** to **21.** An antitumor effect was not exhibited in the PBS administration group and control ADC administration group, whereas a significant antitumor effect was exhibited in all groups administered with anti-GPCl ADC in mice grafted with pancreatic cancer cell strain, BxPC3 cell strain (Figures **19** and **20**). Surprisingly, not only was the tumor growth suppressed, but also the tumor volume was significantly reduced in the groups administered with anti-GPCl ADC at 3 mg/kg and 10 mg/kg (Figure **19**). This revealed that the ADC of the invention exhibits a very high antitumor effect. Further, a significant change in the body weight was not observed in the anti-GPCl ADC administration groups from any dosage tested. Thus, it is understood that toxicity due to anti-GPC1 ADC administration is low (Figure **21**).

As shown in Figure **22,** administration of GPC1 ADC resulted in G2/M phase arrest in tumor tissue of a pancreatic cancer cell strain grafted model. This indicates that cell division of pancreatic cancer is suppressed by uptake of the GPC1 ADC of the invention into pancreatic cancer cells, then cleavage of a linker moiety to free MMAF in the cytoplasm, and arrest of cell cycle at the G2/M phase via tubulin polymerization inhibition. The results of Figure **22** show that the GPC1 ADC of the invention can suppress or stop cell growth in tumor tissue.

(Example 10: In vivo efficacy test on GPC1 ADC using pancreatic cancer PDX)

Pancreatic cancer PDX (Patient-derived tumor xenograft) refers to residual tissue from pathological analysis of cancer tissue upon pancreatic cancer surgery from a pancreatic cancer patient. This Example used a pancreatic cancer model created by subcutaneously grafting in superimmunodeficient mice such as NOG mice. While human tumor hardly survives in SCID mice after grafting, NOG mice have more severe immunodeficiency than SCID mouse, so that human tumor is more likely to survive. For this reason, a PDX model has an environment that is closer to human tumor than a mouse model to which a cell strain is grafted. Thus, information obtained with a PDX model is useful in evaluating drug efficacy.

(Examination of GPC1 expression in pancreatic cancer PDX)

After deparaffinization of a section of a paraffin embedded tissue, the slice was dehydrated with alcohol. Immunohistochemical staining on GPC1 was performed by using an anti-GPCl antibody (Atlas Antibodies: HPA030571) and ChemMate Envision kit HRP 500T (Dako: K5007).

Figure **24** schematically depicts the test in this Example. Specifically, pancreatic cancer PDX was subcutaneously grafted into 6 week old female NOG mice. When the tumor size reached about 130 $mm^3$ after grafting, administration of PBS as a control, control ADC (10 mg/kg), anti-GPCl ADC (1 mg/kg), anti-GPCl ADC (3 mg/kg), and anti-GPC1 ADC (10 mg/kg) was started, which were intravenously administered on day 0, day 4, day 8, and day 12, with the day starting the administration considered 0 day. The tumor volume and body weight were measured on day 0, day 4, day 8, day 12, day 16, day 20, day 24, and day 28.

(Results)

GPC1 was expressed in tumor tissue of the pancreatic cancer PDX used in this Example at an amount of expression that is close to tumor tissue in BxPC3 graft model (Figure **23).** Figures **25** and **26** show the results for antitumor effect on pancreatic cancer PDX due to anti-GPCl ADC. While an anti-GPCl ADC exhibited an antitumor effect in vivo in a model using pancreatic PDX, the efficacy was slightly lower for the pancreatic cancer PDX than BxPC3 graft model.

It is conceivable that the sensitivity of the ADC agent MMAF itself was weaker than BxPC3 for the pancreatic cancer PDX. It is suggested that the efficacy is further enhanced if an agent other than MMAF is used. Further, the body weight slightly decreased on day 16 in the anti-GPCl ADC (10 mg/kg) administration group in the anti-GPCl ADC administration group, but the body weight recovered thereafter to the level at the start of measurement, indicating that the effect of an anti-GPCl ADC on the change in body weight is reversible. Therefore, it is likely that toxicity due to administration of an anti-GPCl ADC is low (Figure **27**).

(Example 11: In vivo efficacy test on GPC1 ADC using cervical cancer cell strain)

Figure **28** schematically shows the test in this Example. Specifically, 1.0 $\times$ $10^6$ cells of ME180 cell strain were subcutaneously grafted into female SCID mice. When the tumor size reached about 120 $mm^3$ after grafting, administration of PBS as a control, control ADC (10 mg/kg), anti-GPCl ADC (1 mg/kg), anti-GPCl ADC (3 mg/kg), and anti-GPC1 ADC (10 mg/kg) was started, which were intravenously administered on day 0, day 4, day 8, and day 12, with the day starting the administration considered 0 day. The tumor volume and body weight were measured on day 0, day 4, day 8, day 12, day 16, day 20, day 24, day 28, and day 32.
After subcutaneous tumorigenesis of ME180, (1) PBS, (2) control ADC at 10 mg/kg, and (3) GPC1-ADC at 10 mg/kg were administered once into the tail vein. The tumor was extracted after 24 hours. After deparaffinization of a section of a paraffin embedded tissue, the slice was dehydrated with alcohol. Immunohistochemical staining on Phospho-Histon H3 (SerlO) was performed by using an anti-Phospho-Histon H3 (SerlO) antibody (Cell Signaling Technology: #9701) and ChemMate Envision kit HRP 500T (Dako: K5007).

(Results)

The results are shown in Figures **29** to **31.** In mice grafted with cervical cancer cell strain ME 180, an antitumor effect was not exhibited in the PBS administration group and control ADC administration group, whereas a slight antitumor effect was exhibited in groups administered with an anti-GPCl ADC at 1 mg/kg and 3 mg/kg, and a significant antitumor effect was exhibited in the group administered with anti-GPCl ADC at 10 mg/kg (Figures **29** and **30**). Surprisingly, not only was the tumor growth suppressed, but also the tumor volume was significantly reduced in the groups administered with anti-GPCl ADC at 10 mg/kg (Figure **29**). This revealed that the ADC of the invention exhibits a very high antitumor effect. Further, a significant change in the body weight was not observed in the anti-GPCl ADC administration groups from any dosage tested. Thus, it is understood that toxicity due to anti-GPC1 ADC administration is low (Figure **31**).
As shown in Figure **32,** administration of GPC1 ADC resulted in G2/M phase arrest in tumor tissue of a cervical cancer cell strain graft model. This indicates that cell division of cervical cancer is suppressed by uptake of the GPC1 ADC of the invention into pancreatic cancer cells, then cleavage of a linker moiety to free MMAF in the cytoplasm, and arrest of cell cycle at the G2/M phase via tubulin polymerization inhibition. The results of Figure **32** show that the GPC1 ADC of the invention can suppress or stop cell growth in tumor tissue.

(Example 12: Examination of antitumor effect of unlabeled antibody)

This Example examined the antitumor effect of unlabeled antibodies (antibodies that are not conjugated to an agent having cytotoxic activity).

(Materials and Methods)

2000 cells were added to a 96-well plate at 90 μl. The cells were cultured overnight at 37°C in a 5% CO2 incubator. The next day, 10 μl of an unlabeled primary antibody concentrated 10-fold relative to the final concentration was added to each well, so that the total amount was 100 μl. The cells were cultured for 6 days at 37°C in a 5% CO2 incubator. The cell viability was measured by using a CellTiter-Glo Luminescent Cell Viability Assay reagent to detect the amount of ATP.

(Results)

The results are shown in Figures **33** to **43.** An antitumor effect in vitro due to 01a033 unlabeled antibody was not observed against any cell strain. Likewise, an antitumor effect was not observed with a higher concentration of antibodies. Therefore, it was revealed that a 01a033 antibody does not have an antitumor effect in itself, but exhibits an antitumor effect only as an ADC.

(Example 13: In vivo antitumor effect of unlabeled anti-GPCl antibody on GPC1 positive cell strain)

Figure **44** schematically shows the test in this Example. Specifically, 2.0 $\times$ $10^6$ cells of TE14 cell strain were subcutaneously grafted into female SCID mice (6 week old, n = 9). When the tumor size reached about 100 $mm^3$ on about day 10 to 14 after grafting, administration of control mouse IgG2a (sigma, M7769) and anti-GPCl antibody 01a033 each at 10 mg/kg was started, which were intraperitoneally administered on day 0, day 3, day 7, day 10, day 14, and day 17, with the day starting the administration considered 0 day. The tumor volume was measured on day 0, day 3, day 7, day 10, day 14, day 17, day 21, day 24, day 28, and day 31.

(Results)

The results are shown in Figure **45.** As shown, it was revealed that the unlabeled anti-GPCl antibody 01a033 that is not conjugated to an agent having cytotoxic activity exhibits nearly the same degree of tumor growth as control IgG, thus exhibiting no antitumor effect. This result shows that anti-GPCl antibody 01a033 does not have ADCC activity. While Example 5 shows that anti-GPCl antibody 01a033 has high internalization activity, the anti-GPCl antibody 01a033 does not exhibit ADCC activity, potentially due to high internalization activity.

(Example 14: Safety test on anti-GPCl antibody 01a033 using mice)

1 mg/body of each of control mouse IgG2a (Sigma, M7769) and anti-GPCl antibody 01a033 was intraperitoneally administered to a group of 4 male and 4 female C57BL/6J mice (8w). The following items were evaluated on day 7.
Blood collection endpoints: WBC, RBC, Hb, Plt, T-Bil, ALT, ALP, Amy, BUN, Cr, Ca, P, TP, Alb, Na, K, Glob, and Glu, automatic blood cell counter: VetScan HMII, animal biochemical blood analyzer: VetScan VS2.

(Results)

The results are shown in Figures **46** to **49.** Toxicity was not found after administering unlabeled antibody of clone 01a033 used in ADC itself to mice.

(Example 15: ADC assay combining anti-GPC-1 antibody and MMAF binding secondary antibody in DU145 cells)

(Materials and Methods)

2000 DU145 cells were added to a 96-well plate at 80 μl. The cells were cultured overnight at 37°C in a 5% CO2 incubator. The next day, 10 μl each of anticancer agent binding secondary antibody (2 μg/ml) and primary antibody concentrated 10-fold relative to the final concentration was added to each well, so that the total amount was 100 μl. The cells were cultured for 3 days at 37°C in a 5% CO2 incubator. The cell viability was measured by using a CellTiter-Glo Luminescent Cell Viability Assay reagent to detect the amount of ATP. RPMI1640 + 10% FBS + 1% PS was used as the medium. The final concentrations of the primary antibodies were 0, 0.004, 0.0156, 0.0625, 0.25, 1.0, and 40 nM. 2 μg/ml of Fab-aMFc-CL-MMAF (model number AM202AF-50, Moradec) was used as the secondary antibody. The EC50 value was analyzed with GraphPad Prism 6. The same experiment was conducted using 4000 MDA-MB231 cells as the control.

(Results)

The results are shown in Figure **50.** Clones 01a033, 01a002, and 02a010 exhibited a high efficacy, i.e., high growth suppressing effect, against prostate cancer cell strain DU145 cells. None of the clones exhibited a growth suppressing effect against MDA-MD231 used as a control. Surprisingly, a higher $EC_{50}$ value than the antibody-drug complex (MIL-38, $EC_{50}$: 0.8572 nM) described in International Publication No. WO 2016/168885 was exhibited by using any of the clones (Figure **50**). In particular, the activity was 37-fold higher for clone 01a033 compared to MIL-38. A slight action attenuation was observed at high concentrations for all of the clones. Although not wishing to be bound by any theory, it is presumed that action attenuation at a high concentration (4.0 nM) is induced by excessive primary antibodies leading to a competitive reaction with an anticancer agent conjugated secondary antibodies. In view of the results in this Example, the antibody-drug complex of the invention exhibits a better effect of suppressing growth on DU145 cells than existing antibody-drug complexes (e.g., MIL-38).
The same test was conducted for clones other than clones 01a033, 01a002, and 02a010. The $EC_{50}$ value for DU145 cells was calculated (Figures **51** to **53**). For comparison, the $EC_{50}$ value of the antibody-drug complex (MIL-38) described in International Publication No. WO 2016/168885 for DU145 cells was used. A better $EC_{50}$ value than MIL-

38 was exhibited for all clones except clones 02a034, 02a022, 01a021, 02a006, 02a035, and 01a007. These clones also exhibit good $IC_{50}$ value of 0.5 nM or less with respect to TE14, demonstrating that the clones are also effective against different cell strains.

(Example 16: Establishment of mouse GPC1 (mGPC1) expressing LLC (mouse lung cancer cell strain) and in vitro ADC assay using anti-GPC1 antibody)

(Materials and Methods)

An mGPC1 expression vector (pcDNA3.1-mGPC1) and control vector (pcDNA3.1V5/His) were transfected using Lipofectamine® 2000 into LLC cell strain (mouse lung cancer cell strain). Cells subjected to gene transfer were selected by adding G418 to a medium, and the formed colony was picked up. The expression of mGPC1 was analyzed by FACS using anti-GPCl antibody clone 01a033. LLC-mGPC1-16 cells were established as an mGPC1 stable expression strain, and LLC-control-7 was established as the control.

Preparation of a conjugate of mouse anti-glypican-1 mAb (01a033), mouse IgG2a (biolegend #400224) as a control, and mc-vc-PAB-MMAF was commissioned to MORADEC. An anticancer agent was conjugated to a cysteine residue via a cleavable linker. The DAR (drug-antibody ratio) was as follows. GPC1-CL-MMAF DAR = 3.8, mouse IgG2a-CL-MMAF DAR = 4.1.

Specifically, the following assay was conducted.

(1) 1000 each of LLC-control-7 cells that does not express mGPC1 and LLC-mGPC1-16 cells were seeded on a Thermo Fisher Scientific's 96-well white plate (model no.: 136101) (90 μl cell suspension). RPMI1640 + 10% FBS + 100 U/ml penicillin +100 μg/ml streptomycin was used as the medium. The cells were seeded in 60 wells and 100 μL of medium was added to 36 wells, and cultured in a 5% CO2 incubator at 37°C.
(2) The next day, 10 μL of ADC was added to the cells (total amount: 100 μL).
(3) After 72 hours of culturing, CellTiter-Glo™ Luminescence Cell Viability Assay reagent (Promega) was added and mixed at 100 μL/well.
(4) Measurements were taken with a plate reader.
(5) Analysis was conducted with GraphPad Prism 6.

The IC50 values were calculated from the following equation (Hossain MM, Hosono-Fukao T, Tang R, Sugaya N, van Kuppevelt TH, Jenniskens GJ, Kimata K, Rosen SD, Uchimura K (2010) Direct detection of HSulf-1 and HSulf-2 activities on extracellular heparan sulphate and their inhibition by PI-88. Glycobiology 20(2): 175-186.)

$$IC50 = 10^{\wedge}(Log[A][B] \times (50-C)/(D-C) + Log[B])$$

A: High concentration straddling 50%
B: Low concentration straddling 50%
C: Inhibition rate at B
D: Inhibition rate at A

The results are shown in Figure **54.** Clone 01a033 exhibits a low effect of suppressing growth against LLC-control-7 that does not express mouse GPC-1, while exhibiting a 17-fold higher effect of suppressing growth relative to LLC-control-7 against LLC-mGPC1-16 that expressed mouse GPC-1. It was confirmed that a cell strain stably expressing mouse GPC1 was able to be established.

(Example 17: In vivo efficacy test on GPC1 ADC with a mouse syngenic model using mGPC1 expressing LLC cell strain)

A mouse model (syngenic mouse) with GPC1 expressed in normal cells that is consistent with GPC1 expressed from cancer cells (i.e., both mouse GPC1) can be prepared by grafting the cell strain expressing mouse GPC1 established in Example 16 into mice. The efficacy and safety can be more accurately evaluated in an experiment using such a mouse model.

(Materials and Methods)

Figure **55** schematically depicts the test in this Example. Specifically, $5.0 \times 10^6$ cells of LLC-mGPC1-16 were

subcutaneously grafted into female C57BL/6 mice. When the tumor size reached about 70 $mm^3$ after grafting, administration of PBS as a control, control ADC (10 mg/kg), anti-GPCl ADC (1 mg/kg), anti-GPCl ADC (3 mg/kg), and anti-GPC1 ADC (10 mg/kg) was started, which were intravenously administered on day 0, day 4, day 8, and day 12, with the day starting the administration considered 0 day. The tumor volume and body weight were measured on day 0, day 4, day 8, day 12, day 16, day 20, and day 24.

(Results)

The results are shown in Figures **56** and **57.** Tumor growth was able be significantly delayed compared to the control group in the group administered with anti-GPCl ADC (1 mg/kg). Tumor hardly grew in the groups administered with anti-GPCl ADC (3 mg/kg) and anti-GPCl ADC (10 mg/kg). In this manner, clone 01a033 also resulted in a high antitumor effect against tumor expressing mouse GPC-1.

Figure **58** shows the results for the change in body weight of syngenic mice. A transient decrease in body weight was observed in the group administered with a high concentration anti-GPCl ADC (10 mg/kg), but the body weight tended to recover after 15 days from the start of administration. Significant toxicity was not observed.

(Examples 18: Application example of detection, diagnosis, and companion)

(1) Cancer detection/diagnosis

Cancer can be detected by labeling anti-glypican-1 antibodies with radioisotope (RI) or the like and administering the labeled anti-glypican-1 antibodies to a patient to study the accumulation at a cancer site, thus enabling early diagnosis of cancer, determination of the site of metastasis, and determination of the therapeutic effect of an anticancer agent. Since the anti-glypican-1 antibody of the invention exhibits high internalization activity against cells expressing glypican-1, glypican-1 positive cancer cells can be specifically detected/diagnosed.

(2) Companion reagent

A companion diagnostic drug of an antigen medicament targeting glypican-1 is prepared by using a new clone to establish sandwich ELISA and quantify glypican-1 in the cancer patient serum.

As described above, the present invention is exemplified by the use of its preferred embodiments. However, it is understood that the scope of the present invention should be interpreted solely based on the Claims. It is also understood that any patent, any patent application, and any references cited herein should be incorporated herein by reference in the same manner as the contents are specifically described herein.

The present application claims priority to Japanese Patent Application No. 2017-90054 (filed on April 28, 2017). It is understood that the entire content of the specification of the application is incorporated herein by reference.

[Industrial Applicability]

A medicament for treating or preventing cancer has been provided. Technologies that can be used in industries (pharmaceutical, etc.) based on such a technology are provided.

[Sequence Listing Free Text]

SEQ ID NO: 1: human glypican-1 nucleic acid sequence (NM_002081.2)
SEQ ID NO: 2: human glypican-1 amino acid sequence (P35052)
SEQ ID NO: 3: mouse glypican-1 nucleic acid sequence (NM_016696.4)
SEQ ID NO: 4: mouse glypican-1 amino acid sequence (Q9QZF2)
SEQ ID NO: 5: amino acid sequence of heavy chain CDR1 of K090-01a002
SEQ ID NO: 6: amino acid sequence of heavy chain CDR2 of K090-01a002
SEQ ID NO: 7: amino acid sequence of heavy chain CDR3 of K090-01a002
SEQ ID NO: 8: amino acid sequence of light chain CDR1 of K090-01a002
SEQ ID NO: 9: amino acid sequence of light chain CDR2 of K090-01a002
SEQ ID NO: 10: amino acid sequence of light chain CDR3 of K090-01a002
SEQ ID NO: 11: amino acid sequence of heavy chain CDR1 of K090-01a007
SEQ ID NO: 12: amino acid sequence of heavy chain CDR2 of K090-01a007

SEQ ID NO: 13: amino acid sequence of heavy chain CDR3 of K090-01a007
SEQ ID NO: 14: amino acid sequence of light chain CDR1 of K090-01a007
SEQ ID NO: 15: amino acid sequence of light chain CDR2 of K090-01a007
SEQ ID NO: 16: amino acid sequence of light chain CDR3 of K090-01a007
SEQ ID NO: 17: amino acid sequence of heavy chain CDR1 of K090-01a016
SEQ ID NO: 18: amino acid sequence of heavy chain CDR2 of K090-01a016
SEQ ID NO: 19: amino acid sequence of heavy chain CDR3 of K090-01a016
SEQ ID NO: 20: amino acid sequence of light chain CDR1 of K090-01a016
SEQ ID NO: 21: amino acid sequence of light chain CDR2 of K090-01a016
SEQ ID NO: 22: amino acid sequence of light chain CDR3 of K090-01a016
SEQ ID NO: 23: amino acid sequence of heavy chain CDR1 of K090-01a017
SEQ ID NO: 24: amino acid sequence of heavy chain CDR2 of K090-01a017
SEQ ID NO: 25: amino acid sequence of heavy chain CDR3 of K090-01a017
SEQ ID NO: 26: amino acid sequence of light chain CDR1 of K090-01a017
SEQ ID NO: 27: amino acid sequence of light chain CDR2 of K090-01a017
SEQ ID NO: 28: amino acid sequence of light chain CDR3 of K090-01a017
SEQ ID NO: 29: amino acid sequence of heavy chain CDR1 of K090-01a021
SEQ ID NO: 30: amino acid sequence of heavy chain CDR2 of K090-01a021
SEQ ID NO: 31: amino acid sequence of heavy chain CDR3 of K090-01a021
SEQ ID NO: 32: amino acid sequence of light chain CDR1 of K090-01a021
SEQ ID NO: 33: amino acid sequence of light chain CDR2 of K090-01a021
SEQ ID NO: 34: amino acid sequence of light chain CDR3 of K090-01a021
SEQ ID NO: 35: amino acid sequence of heavy chain CDR1 of K090-01a026
SEQ ID NO: 36: amino acid sequence of heavy chain CDR2 of K090-01a026
SEQ ID NO: 37: amino acid sequence of heavy chain CDR3 of K090-01a026
SEQ ID NO: 38: amino acid sequence of light chain CDR1 of K090-01a026
SEQ ID NO: 39: amino acid sequence of light chain CDR2 of K090-01a026
SEQ ID NO: 40: amino acid sequence of light chain CDR3 of K090-01a026
SEQ ID NO: 41: amino acid sequence of heavy chain CDR1 of K090-01a009
SEQ ID NO: 42: amino acid sequence of heavy chain CDR2 of K090-01a009
SEQ ID NO: 43: amino acid sequence of heavy chain CDR3 of K090-01a009
SEQ ID NO: 44: amino acid sequence of light chain CDR1 of K090-01a009
SEQ ID NO: 45: amino acid sequence of light chain CDR2 of K090-01a009
SEQ ID NO: 46: amino acid sequence of light chain CDR3 of K090-01a009
SEQ ID NO: 47: amino acid sequence of heavy chain CDR1 of K090-01a030
SEQ ID NO: 48: amino acid sequence of heavy chain CDR2 of K090-01a030
SEQ ID NO: 49: amino acid sequence of heavy chain CDR3 of K090-01a030
SEQ ID NO: 50: amino acid sequence of light chain CDR1 of K090-01a030
SEQ ID NO: 51: amino acid sequence of light chain CDR2 of K090-01a030
SEQ ID NO: 52: amino acid sequence of light chain CDR3 of K090-01a030
SEQ ID NO: 53: amino acid sequence of heavy chain CDR1 of K090-01a033
SEQ ID NO: 54: amino acid sequence of heavy chain CDR2 of K090-01a033
SEQ ID NO: 55: amino acid sequence of heavy chain CDR3 of K090-01a033
SEQ ID NO: 56: amino acid sequence of light chain CDR1 of K090-01a033
SEQ ID NO: 57: amino acid sequence of light chain CDR2 of K090-01a033
SEQ ID NO: 58: amino acid sequence of light chain CDR3 of K090-01a033
SEQ ID NO: 59: amino acid sequence of heavy chain CDR1 of K090-01a042
SEQ ID NO: 60: amino acid sequence of heavy chain CDR2 of K090-01a042
SEQ ID NO: 61: amino acid sequence of heavy chain CDR3 of K090-01a042
SEQ ID NO: 62: amino acid sequence of light chain CDR1 of K090-01a042
SEQ ID NO: 63: amino acid sequence of light chain CDR2 of K090-01a042
SEQ ID NO: 64: amino acid sequence of light chain CDR3 of K090-01a042
SEQ ID NO: 65: amino acid sequence of heavy chain CDR1 of K090-02a002
SEQ ID NO: 66: amino acid sequence of heavy chain CDR2 of K090-02a002
SEQ ID NO: 67: amino acid sequence of heavy chain CDR3 of K090-02a002
SEQ ID NO: 68: amino acid sequence of light chain CDR1 of K090-02a002
SEQ ID NO: 69: amino acid sequence of light chain CDR2 of K090-02a002
SEQ ID NO: 70: amino acid sequence of light chain CDR3 of K090-02a002

SEQ ID NO: 71: amino acid sequence of heavy chain CDR1 of K090-02a006
SEQ ID NO: 72: amino acid sequence of heavy chain CDR2 of K090-02a006
SEQ ID NO: 73: amino acid sequence of heavy chain CDR3 of K090-02a006
SEQ ID NO: 74: amino acid sequence of light chain CDR1 of K090-02a006
SEQ ID NO: 75: amino acid sequence of light chain CDR2 of K090-02a006
SEQ ID NO: 76: amino acid sequence of light chain CDR3 of K090-02a006
SEQ ID NO: 77: amino acid sequence of heavy chain CDR1 of K090-02a010
SEQ ID NO: 78: amino acid sequence of heavy chain CDR2 of K090-02a010
SEQ ID NO: 79: amino acid sequence of heavy chain CDR3 of K090-02a010
SEQ ID NO: 80: amino acid sequence of light chain CDR1 of K090-02a010
SEQ ID NO: 81: amino acid sequence of light chain CDR2 of K090-02a010
SEQ ID NO: 82: amino acid sequence of light chain CDR3 of K090-02a010
SEQ ID NO: 83: amino acid sequence of heavy chain CDR1 of K090-02a014
SEQ ID NO: 84: amino acid sequence of heavy chain CDR2 of K090-02a014
SEQ ID NO: 85: amino acid sequence of heavy chain CDR3 of K090-02a014
SEQ ID NO: 86: amino acid sequence of light chain CDR1 of K090-02a014
SEQ ID NO: 87: amino acid sequence of light chain CDR2 of K090-02a014
SEQ ID NO: 88: amino acid sequence of light chain CDR3 of K090-02a014
SEQ ID NO: 89: amino acid sequence of heavy chain CDR1 of K090-02a022
SEQ ID NO: 90: amino acid sequence of heavy chain CDR2 of K090-02a022
SEQ ID NO: 91: amino acid sequence of heavy chain CDR3 of K090-02a022
SEQ ID NO: 92: amino acid sequence of light chain CDR1 of K090-02a022
SEQ ID NO: 93: amino acid sequence of light chain CDR2 of K090-02a022
SEQ ID NO: 94: amino acid sequence of light chain CDR3 of K090-02a022
SEQ ID NO: 95: amino acid sequence of heavy chain CDR1 of K090-02a034
SEQ ID NO: 96: amino acid sequence of heavy chain CDR2 of K090-02a034
SEQ ID NO: 97: amino acid sequence of heavy chain CDR3 of K090-02a034
SEQ ID NO: 98: amino acid sequence of light chain CDR1 of K090-02a034
SEQ ID NO: 99: amino acid sequence of light chain CDR2 of K090-02a034
SEQ ID NO: 100: amino acid sequence of light chain CDR3 of K090-02a034
SEQ ID NO: 101: amino acid sequence of heavy chain CDR1 of K090-02a035
SEQ ID NO: 102: amino acid sequence of heavy chain CDR2 of K090-02a035
SEQ ID NO: 103: amino acid sequence of heavy chain CDR3 of K090-02a035
SEQ ID NO: 104: amino acid sequence of light chain CDR1 of K090-02a035
SEQ ID NO: 105: amino acid sequence of light chain CDR2 of K090-02a035
SEQ ID NO: 106: amino acid sequence of light chain CDR3 of K090-02a035
SEQ ID NO: 107: amino acid sequence of heavy chain CDR1 of K090-02b006
SEQ ID NO: 108: amino acid sequence of heavy chain CDR2 of K090-02b006
SEQ ID NO: 109: amino acid sequence of heavy chain CDR3 of K090-02b006
SEQ ID NO: 110: amino acid sequence of light chain CDR1 of K090-02b006
SEQ ID NO: 111: amino acid sequence of light chain CDR2 of K090-02b006
SEQ ID NO: 112: amino acid sequence of light chain CDR3 of K090-02b006
SEQ ID NO: 113: amino acid sequence of heavy chain CDR1 of clone 4
SEQ ID NO: 114: amino acid sequence of heavy chain CDR2 of clone 4
SEQ ID NO: 115: amino acid sequence of heavy chain CDR3 of clone 4
SEQ ID NO: 116: amino acid sequence of light chain CDR1 of clone 4
SEQ ID NO: 117: amino acid sequence of light chain CDR2 of clone 4
SEQ ID NO: 118: amino acid sequence of light chain CDR3 of clone 4
SEQ ID NO: 119: amino acid sequence of heavy chain CDR1 of clone 18
SEQ ID NO: 120: amino acid sequence of heavy chain CDR2 of clone 18
SEQ ID NO: 121: amino acid sequence of heavy chain CDR3 of clone 18
SEQ ID NO: 122: amino acid sequence of light chain CDR1 of clone 18
SEQ ID NO: 123: amino acid sequence of light chain CDR2 of clone 18
SEQ ID NO: 124: amino acid sequence of light chain CDR3 of clone 18
SEQ ID NO: 125: nucleic acid sequence of heavy chain of K090-01a002
SEQ ID NO: 126: amino acid sequence of heavy chain of K090-01a002
SEQ ID NO: 127: nucleic acid sequence of light chain of K090-01a002
SEQ ID NO: 128: amino acid sequence of light chain of K090-01a002

SEQ ID NO: 129: nucleic acid sequence of heavy chain of K090-01a007
SEQ ID NO: 130: amino acid sequence of heavy chain of K090-01a007
SEQ ID NO: 131: nucleic acid sequence of light chain of K090-01a007
SEQ ID NO: 132: amino acid sequence of light chain of K090-01a007
SEQ ID NO: 133: nucleic acid sequence of heavy chain of K090-01a016
SEQ ID NO: 134: amino acid sequence of heavy chain of K090-01a016
SEQ ID NO: 135: nucleic acid sequence of light chain of K090-01a016
SEQ ID NO: 136: amino acid sequence of light chain of K090-01a016
SEQ ID NO: 137: nucleic acid sequence of heavy chain of K090-01a017
SEQ ID NO: 138: amino acid sequence of heavy chain of K090-01a017
SEQ ID NO: 139: nucleic acid sequence of light chain of K090-01a017
SEQ ID NO: 140: amino acid sequence of light chain of K090-01a017
SEQ ID NO: 141: nucleic acid sequence of heavy chain of K090-01a021
SEQ ID NO: 142: amino acid sequence of heavy chain of K090-01a021
SEQ ID NO: 143: nucleic acid sequence of light chain of K090-01a021
SEQ ID NO: 144: amino acid sequence of light chain of K090-01a021
SEQ ID NO: 145: nucleic acid sequence of heavy chain of K090-01a026
SEQ ID NO: 146: amino acid sequence of heavy chain of K090-01a026
SEQ ID NO: 147: nucleic acid sequence of light chain of K090-01a026
SEQ ID NO: 148: amino acid sequence of light chain of K090-01a026
SEQ ID NO: 149: nucleic acid sequence of heavy chain of K090-01a009
SEQ ID NO: 150: amino acid sequence of heavy chain of K090-01a009
SEQ ID NO: 151: nucleic acid sequence of light chain of K090-01a009
SEQ ID NO: 152: amino acid sequence of light chain of K090-01a009
SEQ ID NO: 153: nucleic acid sequence of heavy chain of K090-01a030
SEQ ID NO: 154: amino acid sequence of heavy chain of K090-01a030
SEQ ID NO: 155: nucleic acid sequence of light chain of K090-01a030
SEQ ID NO: 156: amino acid sequence of light chain of K090-01a030
SEQ ID NO: 157: nucleic acid sequence of heavy chain of K090-01a033
SEQ ID NO: 158: amino acid sequence of heavy chain of K090-01a033
SEQ ID NO: 159: nucleic acid sequence of light chain of K090-01a033
SEQ ID NO: 160: amino acid sequence of light chain of K090-01a033
SEQ ID NO: 161: nucleic acid sequence of heavy chain of K090-01a042
SEQ ID NO: 162: amino acid sequence of heavy chain of K090-01a042
SEQ ID NO: 163: nucleic acid sequence of light chain of K090-01a042
SEQ ID NO: 164: amino acid sequence of light chain of K090-01a042
SEQ ID NO: 165: nucleic acid sequence of heavy chain of K090-02a002
SEQ ID NO: 166: amino acid sequence of heavy chain of K090-02a002
SEQ ID NO: 167: nucleic acid sequence of light chain of K090-02a002
SEQ ID NO: 168: amino acid sequence of light chain of K090-02a002
SEQ ID NO: 169: nucleic acid sequence of heavy chain of K090-02a006
SEQ ID NO: 170: amino acid sequence of heavy chain of K090-02a006
SEQ ID NO: 171: nucleic acid sequence of light chain of K090-02a006
SEQ ID NO: 172: amino acid sequence of light chain of K090-02a006
SEQ ID NO: 173: nucleic acid sequence of heavy chain of K090-02a010
SEQ ID NO: 174: amino acid sequence of heavy chain of K090-02a010
SEQ ID NO: 175: nucleic acid sequence of light chain of K090-02a010
SEQ ID NO: 176: amino acid sequence of light chain of K090-02a010
SEQ ID NO: 177: nucleic acid sequence of heavy chain of K090-02a014
SEQ ID NO: 178: amino acid sequence of heavy chain of K090-02a014
SEQ ID NO: 179: nucleic acid sequence of light chain of K090-02a014
SEQ ID NO: 180: amino acid sequence of light chain of K090-02a014
SEQ ID NO: 181: nucleic acid sequence of heavy chain of K090-02a022
SEQ ID NO: 182: amino acid sequence of heavy chain of K090-02a022
SEQ ID NO: 183: nucleic acid sequence of light chain of K090-02a022
SEQ ID NO: 184: amino acid sequence of light chain of K090-02a022
SEQ ID NO: 185: nucleic acid sequence of heavy chain of K090-02a034
SEQ ID NO: 186: amino acid sequence of heavy chain of K090-02a034

SEQ ID NO: 187: nucleic acid sequence of light chain of K090-02a034
SEQ ID NO: 188: amino acid sequence of light chain of K090-02a034
SEQ ID NO: 189: nucleic acid sequence of heavy chain of K090-02a035
SEQ ID NO: 190: amino acid sequence of heavy chain of K090-02a035
SEQ ID NO: 191: nucleic acid sequence of light chain of K090-02a035
SEQ ID NO: 192: amino acid sequence of light chain of K090-02a035
SEQ ID NO: 193: nucleic acid sequence of heavy chain of K090-02b006
SEQ ID NO: 194: amino acid sequence of heavy chain of K090-02b006
SEQ ID NO: 195: nucleic acid sequence of light chain of K090-02b006
SEQ ID NO: 196: amino acid sequence of light chain of K090-02b006
SEQ ID NO: 197: nucleic acid sequence of heavy chain of clone 4
SEQ ID NO: 198: amino acid sequence of heavy chain of clone 4
SEQ ID NO: 199: nucleic acid sequence of light chain of clone 4
SEQ ID NO: 200: amino acid sequence of light chain of clone 4
SEQ ID NO: 201: nucleic acid sequence of heavy chain of clone 18
SEQ ID NO: 202: amino acid sequence of heavy chain of clone 18
SEQ ID NO: 203: nucleic acid sequence of light chain of clone 18
SEQ ID NO: 204: amino acid sequence of light chain of clone 18

## Claims

1. An anti-human glypican-1 antibody or antigen binding fragment thereof, wherein the antibody is selected from the group consisting of:

    (a) an antibody comprising amino acid sequences of heavy chain CDR1, heavy chain CDR2, and heavy chain CDR3 set forth in SEQ ID NOs: 53, 54, and 55, respectively, and light chain CDR1, light chain CDR2, and light chain CDR3 set forth in SEQ ID NOs: 56, 57, and 58, respectively;
    (b) an antibody comprising amino acid sequences of heavy chain CDR1, heavy chain CDR2, and heavy chain CDR3 set forth in SEQ ID NOs: 5, 6, and 7, respectively, and light chain CDR1, light chain CDR2, and light chain CDR3 set forth in SEQ ID NOs: 8, 9, and 10, respectively;
    (c) an antibody comprising amino acid sequences of heavy chain CDR1, heavy chain CDR2, and heavy chain CDR3 set forth in SEQ ID NOs: 11, 12, and 13, respectively, and light chain CDR1, light chain CDR2, and light chain CDR3 set forth in SEQ ID NOs: 14, 15, and 16, respectively;
    (d) an antibody comprising amino acid sequences of heavy chain CDR1, heavy chain CDR2, and heavy chain CDR3 set forth in SEQ ID NOs: 17, 18, and 19, respectively, and light chain CDR1, light chain CDR2, and light chain CDR3 set forth in SEQ ID NOs: 20, 21, and 22, respectively;
    (e) an antibody comprising amino acid sequences of heavy chain CDR1, heavy chain CDR2, and heavy chain CDR3 set forth in SEQ ID NOs: 23, 24, and 25, respectively, and light chain CDR1, light chain CDR2, and light chain CDR3 set forth in SEQ ID NOs: 26, 27, and 28, respectively;
    (f) an antibody comprising amino acid sequences of heavy chain CDR1, heavy chain CDR2, and heavy chain CDR3 set forth in SEQ ID NOs: 29, 30, and 31, respectively, and light chain CDR1, light chain CDR2, and light chain CDR3 set forth in SEQ ID NOs: 32, 33, and 34, respectively;
    (g) an antibody comprising amino acid sequences of heavy chain CDR1, heavy chain CDR2, and heavy chain CDR3 set forth in SEQ ID NOs: 35, 36, and 37, respectively, and light chain CDR1, light chain CDR2, and light chain CDR3 set forth in SEQ ID NOs: 38, 39, and 40, respectively;
    (h) an antibody comprising amino acid sequences of heavy chain CDR1, heavy chain CDR2, and heavy chain CDR3 set forth in SEQ ID NOs: 41, 42, and 43, respectively, and light chain CDR1, light chain CDR2, and light chain CDR3 set forth in SEQ ID NOs: 44, 45, and 46, respectively;
    (i) an antibody comprising amino acid sequences of heavy chain CDR1, heavy chain CDR2, and heavy chain CDR3 set forth in SEQ ID NOs: 47, 48, and 49, respectively, and light chain CDR1, light chain CDR2, and light chain CDR3 set forth in SEQ ID NOs: 50, 51, and 52, respectively;
    (j) an antibody comprising amino acid sequences of heavy chain CDR1, heavy chain CDR2, and heavy chain CDR3 set forth in SEQ ID NOs: 59, 60, and 61, respectively, and light chain CDR1, light chain CDR2, and light chain CDR3 set forth in SEQ ID NOs: 62, 63, and 64, respectively;

(k) an antibody comprising amino acid sequences of heavy chain CDR1, heavy chain CDR2, and heavy chain CDR3 set forth in SEQ ID NOs: 65, 66, and 67, respectively, and light chain CDR1, light chain CDR2, and light chain CDR3 set forth in SEQ ID NOs: 68, 69, and 70, respectively;
(l) an antibody comprising amino acid sequences of heavy chain CDR1, heavy chain CDR2, and heavy chain CDR3 set forth in SEQ ID NOs: 71, 72, and 73, respectively, and light chain CDR1, light chain CDR2, and light chain CDR3 set forth in SEQ ID NOs: 74, 75, and 76, respectively;
(m) an antibody comprising amino acid sequences of heavy chain CDR1, heavy chain CDR2, and heavy chain CDR3 set forth in SEQ ID NOs: 77, 78, and 79, respectively, and light chain CDR1, light chain CDR2, and light chain CDR3 set forth in SEQ ID NOs: 80, 81, and 82, respectively;
(n) an antibody comprising amino acid sequences of heavy chain CDR1, heavy chain CDR2, and heavy chain CDR3 set forth in SEQ ID NOs: 83, 84, and 85, respectively, and light chain CDR1, light chain CDR2, and light chain CDR3 set forth in SEQ ID NOs: 86, 87, and 88, respectively;
(o) an antibody comprising amino acid sequences of heavy chain CDR1, heavy chain CDR2, and heavy chain CDR3 set forth in SEQ ID NOs: 89, 90, and 91, respectively, and light chain CDR1, light chain CDR2, and light chain CDR3 set forth in SEQ ID NOs: 92, 93, and 94, respectively;
(p) an antibody comprising amino acid sequences of heavy chain CDR1, heavy chain CDR2, and heavy chain CDR3 set forth in SEQ ID NOs: 95, 96, and 97, respectively, and light chain CDR1, light chain CDR2, and light chain CDR3 set forth in SEQ ID NOs: 98, 99, and 100, respectively;
(q) an antibody comprising amino acid sequences of heavy chain CDR1, heavy chain CDR2, and heavy chain CDR3 set forth in SEQ ID NOs: 101, 102, and 103, respectively, and light chain CDR1, light chain CDR2, and light chain CDR3 set forth in SEQ ID NOs: 104, 105, and 106, respectively;
(r) an antibody comprising amino acid sequences of heavy chain CDR1, heavy chain CDR2, and heavy chain CDR3 set forth in SEQ ID NOs: 107, 108, and 109, respectively, and light chain CDR1, light chain CDR2, and light chain CDR3 set forth in SEQ ID NOs: 110, 111, and 112, respectively; and
(s) a mutant of an antibody selected from (a) to (r) comprising at least one substitution, addition, or deletion in a CDR moiety.

**2.** The antibody or antigen binding fragment thereof of claim 1, wherein the antibody is selected from the group consisting of:

(a) an antibody comprising amino acid sequences of a heavy chain set forth in SEQ ID NO: 158 and a light chain set forth in SEQ ID NO: 160;
(b) an antibody comprising amino acid sequences of a heavy chain set forth in SEQ ID NO: 126 and a light chain set forth in SEQ ID NO: 128;
(c) an antibody comprising amino acid sequences of a heavy chain set forth in SEQ ID NO: 130 and a light chain set forth in SEQ ID NO: 132;
(d) an antibody comprising amino acid sequences of a heavy chain set forth in SEQ ID NO: 134 and a light chain set forth in SEQ ID NO: 136;
(e) an antibody comprising amino acid sequences of a heavy chain set forth in SEQ ID NO: 138 and a light chain set forth in SEQ ID NO: 140;
(f) an antibody comprising amino acid sequences of a heavy chain set forth in SEQ ID NO: 142 and a light chain set forth in SEQ ID NO: 144;
(g) an antibody comprising amino acid sequences of a heavy chain set forth in SEQ ID NO: 146 and a light chain set forth in SEQ ID NO: 148;
(h) an antibody comprising amino acid sequences of a heavy chain set forth in SEQ ID NO: 150 and a light chain set forth in SEQ ID NO: 152;
(i) an antibody comprising amino acid sequences of a heavy chain set forth in SEQ ID NO: 154 and a light chain set forth in SEQ ID NO: 156;
(j) an antibody comprising amino acid sequences of a heavy chain set forth in SEQ ID NO: 162 and a light chain set forth in SEQ ID NO: 164;
(k) an antibody comprising amino acid sequences of a heavy chain set forth in SEQ ID NO: 166 and a light chain set forth in SEQ ID NO: 168;
(l) an antibody comprising amino acid sequences of a heavy chain set forth in SEQ ID NO: 170 and a light chain set forth in SEQ ID NO: 172;
(m) an antibody comprising amino acid sequences of a heavy chain set forth in SEQ ID NO: 174 and a light chain set forth in SEQ ID NO: 176;
(n) an antibody comprising amino acid sequences of a heavy chain set forth in SEQ ID NO: 178 and a light chain set forth in SEQ ID NO: 180;

(o) an antibody comprising amino acid sequences of a heavy chain set forth in SEQ ID NO: 182 and a light chain set forth in SEQ ID NO: 184;
(p) an antibody comprising amino acid sequences of a heavy chain set forth in SEQ ID NO: 186 and a light chain set forth in SEQ ID NO: 188;
(q) an antibody comprising amino acid sequences of a heavy chain set forth in SEQ ID NO: 190 and a light chain set forth in SEQ ID NO: 192;
(r) an antibody comprising amino acid sequences of a heavy chain set forth in SEQ ID NO: 194 and a light chain set forth in SEQ ID NO: 196; and
(s) a mutant of an antibody selected from (a) to (r) comprising at least one substitution, addition, or deletion.

**3.** The antibody or antigen binding fragment thereof of claim 1 or 2, wherein the mutant comprises at least one substitution, addition, or deletion in a framework of the antibody.

**4.** The antibody or antigen binding fragment thereof of any one of claims 1 to 3, which binds to human glypican-1 at a binding constant of about 10 nM or less.

**5.** The antibody or antigen binding fragment thereof of any one of claims 1 to 4, having an internalization activity of about 30% or greater with respect to glypican-1 positive cells after 2 hours from starting incubation with the antibody or antigen fragment thereof in an internalization assay.

**6.** The antibody or antigen binding fragment thereof of any one of claims 1 to 5, having an internalization activity of about 50% or greater with respect to glypican-1 positive cells after 2 hours from starting incubation with the antibody or antigen fragment thereof in an internalization assay.

**7.** The antibody or antigen binding fragment thereof of any one of claims 1 to 6, having an internalization activity of about 60% or greater with respect to glypican-1 positive cells after 2 hours from starting incubation with the antibody or antigen fragment thereof in an internalization assay.

**8.** The antibody or antigen binding fragment thereof of any one of claims 1 to 7, wherein the antibody is an antibody selected from a monoclonal antibody, a polyclonal antibody, a chimeric antibody, a humanized antibody, a human antibody, a multifunctional antibody, a bispecific or oligospecific antibody, a single chain antibody, an scFV, a diabody, an sc(Fv)2 (single chain (Fv)2), and an scFv-Fc.

**9.** A pharmaceutical composition comprising the antibody or antigen binding fragment thereof of any one of claims 1 to 8.

**10.** The pharmaceutical composition of claim 9, wherein the pharmaceutical composition is for intracellular invasion of an active ingredient.

**11.** A complex of an anti-glypican-1 antibody having activity for intracellular invasion into glypican-1 positive cells or antigen binding fragment thereof and an agent having cytotoxic activity.

**12.** The complex of claim 11, wherein the anti-glypican-1 antibody or antigen binding fragment thereof is operably linked to the agent having cytotoxic activity via a linker.

**13.** The complex of claim 12 or 13, wherein an epitope of the antibody comprises:

(a) positions 33 to 61 of SEQ ID NO: 2;
(b) positions 339 to 358 and/or 388 to 421 of SEQ ID NO: 2;
(c) positions 430 to 530 of SEQ ID NO: 2;
(d) positions 33 to 61, 339 to 358, and/or 388 to 421 of SEQ ID NO: 2;
(e) positions 339 to 358, 388 to 421, and/or 430 to 530 of SEQ ID NO: 2; or
(f) positions 33 to 61, 339 to 358, 388 to 421, and/or 430 to 530 of SEQ ID NO: 2.

**14.** The complex of claim 13, wherein the epitope of the antibody comprises:

(a) positions 33 to 61 of SEQ ID NO: 2;
(b) positions 339 to 358 and 388 to 421 of SEQ ID NO: 2;
(c) positions 33 to 61, 339 to 358, and 388 to 42 of SEQ ID NO: 2; or

(d) positions 33 to 61, 339 to 358, 388 to 421, and 430 to 530 of SEQ ID NO: 2.

**15.** The complex of any one of claims 11 to 14, wherein the antibody is selected from the group consisting of:

(a) an antibody comprising amino acid sequences of heavy chain CDR1, heavy chain CDR2, and heavy chain CDR3 set forth in SEQ ID NOs: 53, 54, and 55, respectively, and light chain CDR1, light chain CDR2, and light chain CDR3 set forth in SEQ ID NOs: 56, 57, and 58, respectively;
(b) an antibody comprising amino acid sequences of heavy chain CDR1, heavy chain CDR2, and heavy chain CDR3 set forth in SEQ ID NOs: 5, 6, and 7, respectively, and light chain CDR1, light chain CDR2, and light chain CDR3 set forth in SEQ ID NOs: 8, 9, and 10, respectively;
(c) an antibody comprising amino acid sequences of heavy chain CDR1, heavy chain CDR2, and heavy chain CDR3 set forth in SEQ ID NOs: 11, 12, and 13, respectively, and light chain CDR1, light chain CDR2, and light chain CDR3 set forth in SEQ ID NOs: 14, 15, and 16, respectively;
(d) an antibody comprising amino acid sequences of heavy chain CDR1, heavy chain CDR2, and heavy chain CDR3 set forth in SEQ ID NOs: 17, 18, and 19, respectively, and light chain CDR1, light chain CDR2, and light chain CDR3 set forth in SEQ ID NOs: 20, 21, and 22, respectively;
(e) an antibody comprising amino acid sequences of heavy chain CDR1, heavy chain CDR2, and heavy chain CDR3 set forth in SEQ ID NOs: 23, 24, and 25, respectively, and light chain CDR1, light chain CDR2, and light chain CDR3 set forth in SEQ ID NOs: 26, 27, and 28, respectively;
(f) an antibody comprising amino acid sequences of heavy chain CDR1, heavy chain CDR2, and heavy chain CDR3 set forth in SEQ ID NOs: 29, 30, and 31, respectively, and light chain CDR1, light chain CDR2, and light chain CDR3 set forth in SEQ ID NOs: 32, 33, and 34, respectively;
(g) an antibody comprising amino acid sequences of heavy chain CDR1, heavy chain CDR2, and heavy chain CDR3 set forth in SEQ ID NOs: 35, 36, and 37, respectively, and light chain CDR1, light chain CDR2, and light chain CDR3 set forth in SEQ ID NOs: 38, 39, and 40, respectively;
(h) an antibody comprising amino acid sequences of heavy chain CDR1, heavy chain CDR2, and heavy chain CDR3 set forth in SEQ ID NOs: 41, 42, and 43, respectively, and light chain CDR1, light chain CDR2, and light chain CDR3 set forth in SEQ ID NOs: 44, 45, and 46, respectively;
(i) an antibody comprising amino acid sequences of heavy chain CDR1, heavy chain CDR2, and heavy chain CDR3 set forth in SEQ ID NOs: 47, 48, and 49, respectively, and light chain CDR1, light chain CDR2, and light chain CDR3 set forth in SEQ ID NOs: 50, 51, and 52, respectively;
(j) an antibody comprising amino acid sequences of heavy chain CDR1, heavy chain CDR2, and heavy chain CDR3 set forth in SEQ ID NOs: 59, 60, and 61, respectively, and light chain CDR1, light chain CDR2, and light chain CDR3 set forth in SEQ ID NOs: 62, 63, and 64, respectively;
(k) an antibody comprising amino acid sequences of heavy chain CDR1, heavy chain CDR2, and heavy chain CDR3 set forth in SEQ ID NOs: 65, 66, and 67, respectively, and light chain CDR1, light chain CDR2, and light chain CDR3 set forth in SEQ ID NOs: 68, 69, and 70, respectively;
(l) an antibody comprising amino acid sequences of heavy chain CDR1, heavy chain CDR2, and heavy chain CDR3 set forth in SEQ ID NOs: 71, 72, and 73, respectively, and light chain CDR1, light chain CDR2, and light chain CDR3 set forth in SEQ ID NOs: 74, 75, and 76, respectively;
(m) an antibody comprising amino acid sequences of heavy chain CDR1, heavy chain CDR2, and heavy chain CDR3 set forth in SEQ ID NOs: 77, 78, and 79, respectively, and light chain CDR1, light chain CDR2, and light chain CDR3 set forth in SEQ ID NOs: 80, 81, and 82, respectively;
(n) an antibody comprising amino acid sequences of heavy chain CDR1, heavy chain CDR2, and heavy chain CDR3 set forth in SEQ ID NOs: 83, 84, and 85, respectively, and light chain CDR1, light chain CDR2, and light chain CDR3 set forth in SEQ ID NOs: 86, 87, and 88, respectively;
(o) an antibody comprising amino acid sequences of heavy chain CDR1, heavy chain CDR2, and heavy chain CDR3 set forth in SEQ ID NOs: 89, 90, and 91, respectively, and light chain CDR1, light chain CDR2, and light chain CDR3 set forth in SEQ ID NOs: 92, 93, and 94, respectively;
(p) an antibody comprising amino acid sequences of heavy chain CDR1, heavy chain CDR2, and heavy chain CDR3 set forth in SEQ ID NOs: 95, 96, and 97, respectively, and light chain CDR1, light chain CDR2, and light chain CDR3 set forth in SEQ ID NOs: 98, 99, and 100, respectively;
(q) an antibody comprising amino acid sequences of heavy chain CDR1, heavy chain CDR2, and heavy chain CDR3 set forth in SEQ ID NOs: 101, 102, and 103, respectively, and light chain CDR1, light chain CDR2, and light chain CDR3 set forth in SEQ ID NOs: 104, 105, and 106, respectively;
(r) an antibody comprising amino acid sequences of heavy chain CDR1, heavy chain CDR2, and heavy chain CDR3 set forth in SEQ ID NOs: 107, 108, and 109, respectively, and light chain CDR1, light chain CDR2, and light chain CDR3 set forth in SEQ ID NOs: 110, 111, and 112, respectively;

(s) an antibody comprising amino acid sequences of heavy chain CDR1, heavy chain CDR2, and heavy chain CDR3 set forth in SEQ ID NOs: 113, 114, and 115, respectively, and light chain CDR1, light chain CDR2, and light chain CDR3 set forth in SEQ ID NOs: 116, 117, and 118, respectively;
(t) an antibody comprising amino acid sequences of heavy chain CDR1, heavy chain CDR2, and heavy chain CDR3 set forth in SEQ ID NOs: 125, 126, and 127, respectively, and light chain CDR1, light chain CDR2, and light chain CDR3 set forth in SEQ ID NOs: 128, 129, and 130, respectively; and
(u) a mutant of an antibody selected from (a) to (t) comprising at least one substitution, addition, or deletion.

**16.** The complex of claim 15, wherein the antibody is selected from the group consisting of:

(a) an antibody comprising amino acid sequences of a heavy chain set forth in SEQ ID NO: 158 and a light chain set forth in SEQ ID NO: 160;
(b) an antibody comprising amino acid sequences of a heavy chain set forth in SEQ ID NO: 126 and a light chain set forth in SEQ ID NO: 128;
(c) an antibody comprising amino acid sequences of a heavy chain set forth in SEQ ID NO: 130 and a light chain set forth in SEQ ID NO: 132;
(d) an antibody comprising amino acid sequences of a heavy chain set forth in SEQ ID NO: 134 and a light chain set forth in SEQ ID NO: 136;
(e) an antibody comprising amino acid sequences of a heavy chain set forth in SEQ ID NO: 138 and a light chain set forth in SEQ ID NO: 140;
(f) an antibody comprising amino acid sequences of a heavy chain set forth in SEQ ID NO: 142 and a light chain set forth in SEQ ID NO: 144;
(g) an antibody comprising amino acid sequences of a heavy chain set forth in SEQ ID NO: 146 and a light chain set forth in SEQ ID NO: 148;
(h) an antibody comprising amino acid sequences of a heavy chain set forth in SEQ ID NO: 150 and a light chain set forth in SEQ ID NO: 152;
(i) an antibody comprising amino acid sequences of a heavy chain set forth in SEQ ID NO: 154 and a light chain set forth in SEQ ID NO: 156;
(j) an antibody comprising amino acid sequences of a heavy chain set forth in SEQ ID NO: 162 and a light chain set forth in SEQ ID NO: 164;
(k) an antibody comprising amino acid sequences of a heavy chain set forth in SEQ ID NO: 166 and a light chain set forth in SEQ ID NO: 168;
(l) an antibody comprising amino acid sequences of a heavy chain set forth in SEQ ID NO: 170 and a light chain set forth in SEQ ID NO: 172;
(m) an antibody comprising amino acid sequences of a heavy chain set forth in SEQ ID NO: 174 and a light chain set forth in SEQ ID NO: 176;
(n) an antibody comprising amino acid sequences of a heavy chain set forth in SEQ ID NO: 178 and a light chain set forth in SEQ ID NO: 180;
(o) an antibody comprising amino acid sequences of a heavy chain set forth in SEQ ID NO: 182 and a light chain set forth in SEQ ID NO: 184;
(p) an antibody comprising amino acid sequences of a heavy chain set forth in SEQ ID NO: 186 and a light chain set forth in SEQ ID NO: 188;
(q) an antibody comprising amino acid sequences of a heavy chain set forth in SEQ ID NO: 190 and a light chain set forth in SEQ ID NO: 192;
(r) an antibody comprising amino acid sequences of a heavy chain set forth in SEQ ID NO: 194 and a light chain set forth in SEQ ID NO: 196;
(s) an antibody comprising amino acid sequences of a heavy chain set forth in SEQ ID NO: 198 and a light chain set forth in SEQ ID NO: 200;
(t) an antibody comprising amino acid sequences of a heavy chain set forth in SEQ ID NO: 202 and a light chain set forth in SEQ ID NO: 204; and
(u) a mutant of an antibody selected from (a) to (t) comprising at least one substitution, addition, or deletion.

**17.** The complex of any one of claims 11 to 16, wherein the complex exhibits an $IC_{50}$ of about 0.5 nM or less in glypican-1 positive cells.

**18.** A composition for preventing or treating glypican-1 positive cancer, comprising the complex of any one of claims 11 to 17.

**19.** The composition of claim 18, wherein cancer cells with the glypican-1 positive cancer express a high level of glypican-1 on a cell surface.

**20.** The composition of claim 19, wherein the cancer cells with the glypican-1 positive cancer have an anti-glypican-1 antibody binding capacity of about 15000 or greater in an assay using QIFIKIT®.

**21.** The composition of claim 20, wherein the glypican-1 positive cancer is selected from esophageal cancer, pancreatic cancer, cervical cancer, lung cancer, head and neck cancer, breast cancer, uterine leiomyosarcoma, prostate cancer, and any combination thereof.

**22.** The composition of claim 20 or 21, wherein the glypican-1 positive cancer is esophageal cancer, which comprises esophageal cancer at a lymph node metastasis site, squamous cell carcinoma, and/or adenocarcinoma.

**23.** The composition of claim 22, wherein the esophageal cancer comprises squamous cell carcinoma.

**24.** A detection agent for identifying esophageal cancer, comprising the antibody or fragment thereof of any one of claims 1 to 8.

**25.** The detection agent of claim 24, wherein the antibody or fragment thereof is labeled.

**26.** The detection agent of claim 24 or 25, wherein the esophageal cancer is glypican-1 positive.

**27.** The detection agent of any one of claims 24 to 26, wherein the esophageal cancer comprises esophageal cancer at a lymph node metastasis site, squamous cell carcinoma, and/or adenocarcinoma.

**28.** The detection agent of any one of claims 24 to 27, wherein the esophageal cancer comprises squamous cell carcinoma.

**29.** The detection agent of any one of claims 24 to 28 for administration to a patient determined to have developed glypican-1 positive esophageal cancer.

**30.** A method of using expression of glypican-1 in a target sample as an indicator of esophageal cancer, the method comprising:

contacting the detection agent of any one of claims 24 to 29 with the target sample;
measuring an amount of expression of glypican-1 in the target sample; and
comparing amounts of expression of glypican-1 in the target sample and a normal sample.

**31.** A diagnostic drug for determining whether a subject is in need of therapy of esophageal cancer, comprising the antibody or antigen binding fragment thereof of any one of claims 1 to 8.

**32.** A companion reagent for determining whether a subject is in need of cancer therapy with a glypican-1 inhibitor, comprising the antibody or antigen binding fragment thereof of any one of claims 1 to 8 or the detection agent of any one of claims 24 to 29, wherein the reagent is contacted with a target sample, and an amount of expression of glypican-1 in the target sample is measured, wherein the amount of expression of glypican-1 in the target sample exceeding an amount of expression of glypican-1 in a normal sample indicates that the target is in need of therapy with a glypican-1 inhibitor.

**33.** A composition for preventing or treating malignant tumor comprising the complex of any one of claims 11 to 17, wherein a subject having the malignant tumor has a higher expression of glypican-1 than a normal individual.

**34.** A nonhuman animal to which cells that express a cancer antigen are grafted, wherein the cancer antigen is syngenic with the nonhuman animal.

**35.** The nonhuman animal of claim 34, wherein the cancer antigen is glypican-1.

**36.** The nonhuman animal of claim 34 or 35, wherein the cells are selected from the group consisting of an LLC cell strain, a 4T1 cell strain, an MH-1 cell strain, a CT26 cell strain, an MC38 cell strain, and a B1610 cell strain, which

have been modified to express glypican-1.

**37.** The nonhuman animal of any one of claims 34 to 36, wherein the nonhuman animal is a mouse, and the cells are of an LLC cell strain modified to express mouse glypican-1.

FIG.1

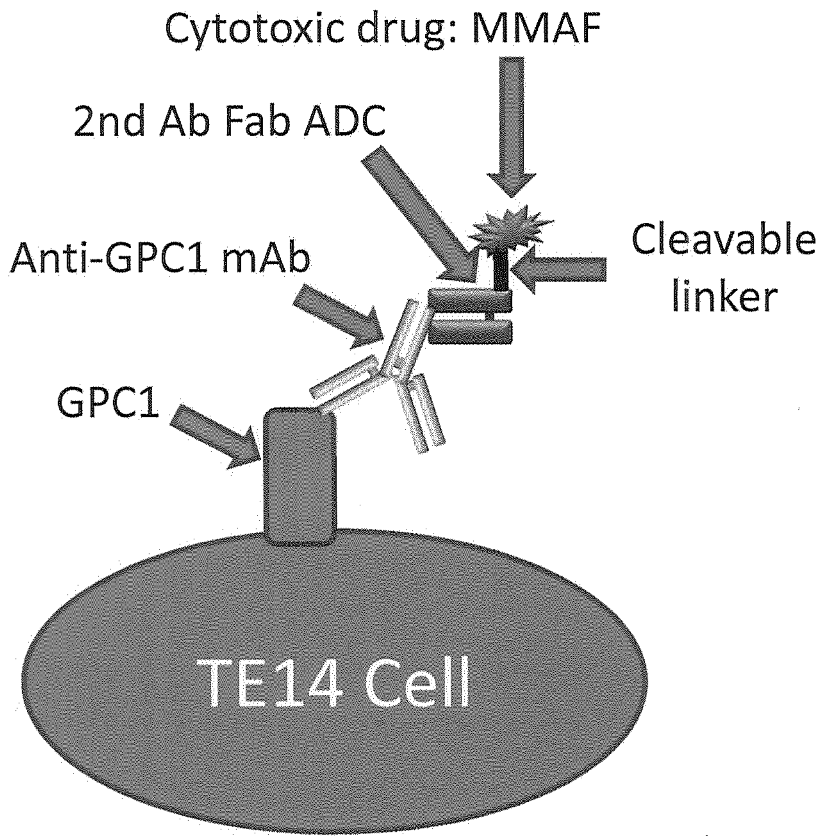


| | |
|---|---|
| 1set | mIgG2a biolegend |
| | clone4 |
| | clone17 |
| | clone18 |
| | 02a010 |
| | 02a014 |
| | 02a022 |
| | 02a034 |

| | |
|---|---|
| 2 set | mIgG2a biolegend |
| | 02a035 |
| | 02b006 |
| | 01a002 |
| | 01a007 |
| | 01a016 |
| | 01a017 |
| | 01a021 |

| | |
|---|---|
| 3 set | mIgG2a biolegend |
| | 01a026 |
| | 01a009 |
| | 01a030 |
| | 01a033 |
| | 01a042 |
| | 02a002 |
| | 02a006 |

FIG.2

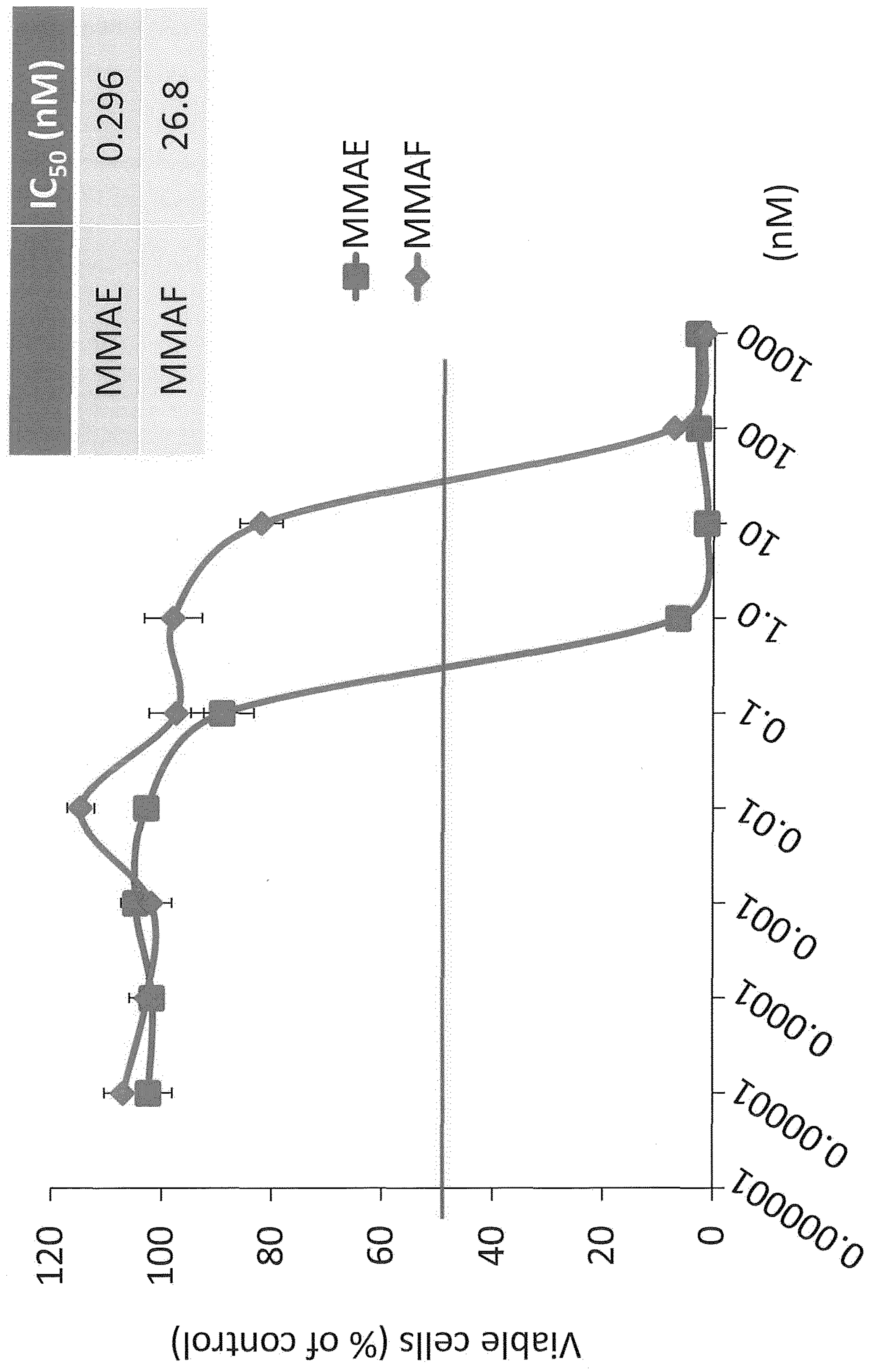
IC$_{50}$ (nM)
MMAE 0.296
MMAF 26.8
MMAE
MMAF
Viable cells (% of control)
120
100
80
60
40
20
0
0.0000001
0.000001
0.0001
0.001
0.01
0.1
1.0
10
100
1000
(nM)

FIG.3

| Clone | ADC IC50 (nM) TE14 2nd Ab-MMAF | assay set | $K_D$ (nM) Biacore | $K_D$(nM) FACS | mGPC1 intersection | epitope |
|---|---|---|---|---|---|---|
| 01a033 | **0.014** | 3 set | **8.9** | **0.5834** | **high** | **339-358, 388-421** |
| 02a002 | **0.024** | 3 set | **1.12** | **2.249** | **weak** | **33-61, 339-358, 388-421** |
| 01a002 | 0.031 | 2 set | 4.99 | 0.6381 | high | 339-358, 388-421 |
| 02a010 | 0.042 | 1 set | 1.28 | 2.251 | weak | 33-61 |
| 01a030 | 0.045 | 3 set | 42.8 | 5.238 | weak | 339-358, 388-421 |
| clone18 | 0.052 | 1 set | 3.21 | 1.522 | negative | not determined |
| 01a042 | 0.059 | 3 set | 16.2 | 6.363 | high | 33-61, 339-358, 388-421 |
| 01a026 | 0.062 | 3 set | 12400 | 11.19 | weak | 339-358, 388-421 |
| 02a014 | 0.070 | 1 set | 2.52 | 1.72 | high | 339-358, 388-421 |
| 02b006 | 0.112 | 2 set | 16.3 | 3.199 | weak | 33-61 |
| 01a009 | 0.187 | 3 set | 26.9 | 4.47 | negative | 33-61, 339-358, 388-421, 430-530 |
| 01a017 | 0.222 | 2 set | 24.9 | 5.92 | weak | 339-358, 388-421 |
| clone4 | 0.423 | 1 set | 3.21 | 0.804 | high | 339-358, 388-421 |

# FIG.4

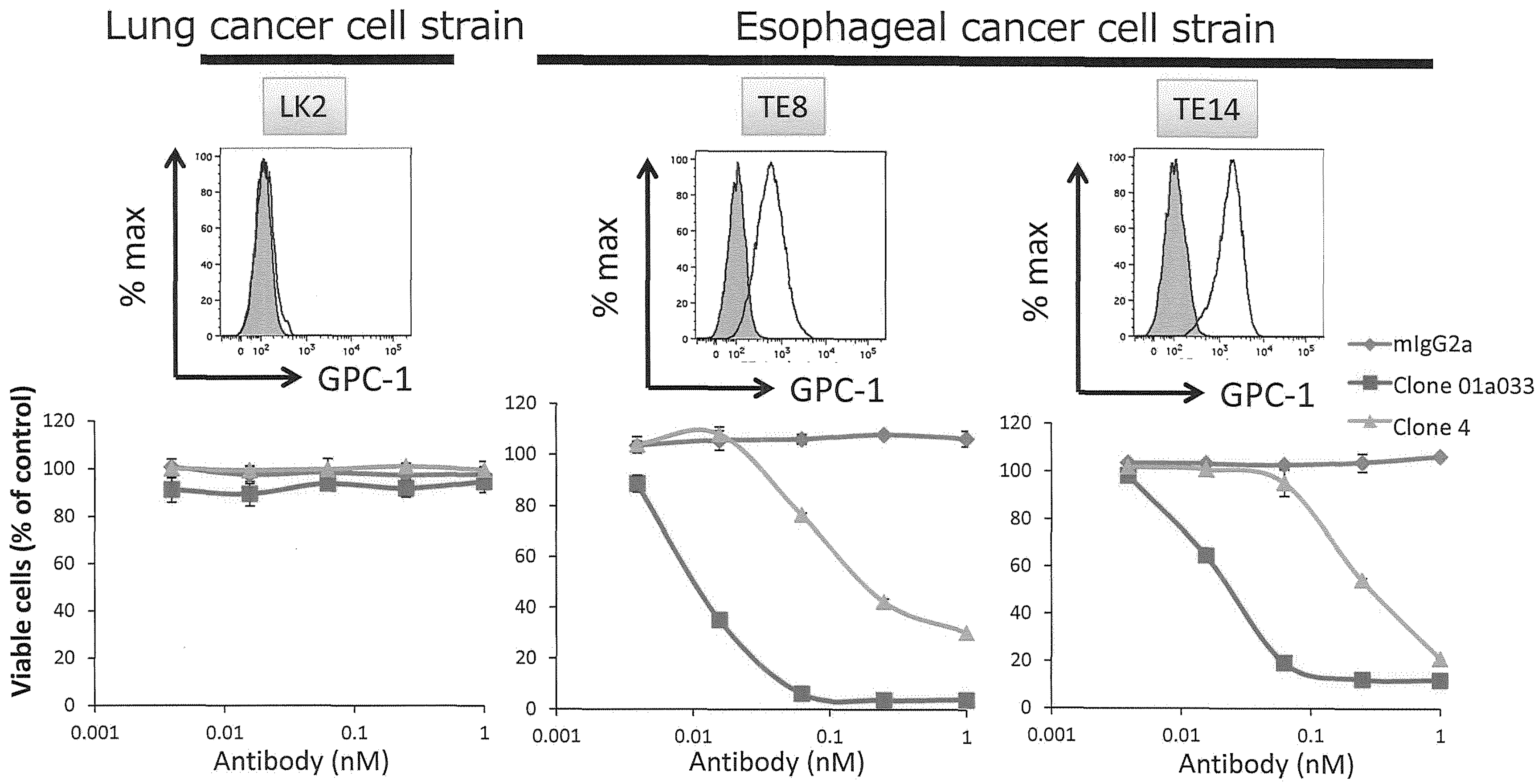


LK2:

| clone | $IC_{50}$ (nM) |
|---|---|
| mIgG2a | n.d. |
| 01a033 | n.d. |
| 4 | n.d. |

TE8:

| clone | $IC_{50}$ (nM) |
|---|---|
| mIgG2a | n.d. |
| 01a033 | 0.0104 |
| 4 | 0.186 |

TE14:

| clone | $IC_{50}$ (nM) |
|---|---|
| mIgG2a | n.d. |
| 01a033 | 0.0244 |
| 4 | 0.309 |

FIG.5

| | | anti-GPC1 mAb IC50 (nM) | | IC50 (nM) | IC50 (nM) | | |
|---|---|---|---|---|---|---|---|
| | GPC1 | 01a033 | clone4 | MMAE | MMAF | cells/well | Secondary antibody concentration |
| TE8 | high | 0.0104 | 0.186 | 0.304 | 42.8 | 1,500 | 2.0 μg/ml |
| TE14 | high | 0.0244 | 0.309 | 0.296 | 26.8 | 2,000 | 2.0 μg/ml |
| LK2 | low | n.d. | n.d. | 0.235 | 31.9 | 2,000 | 2.0 μg/ml |

FIG.6

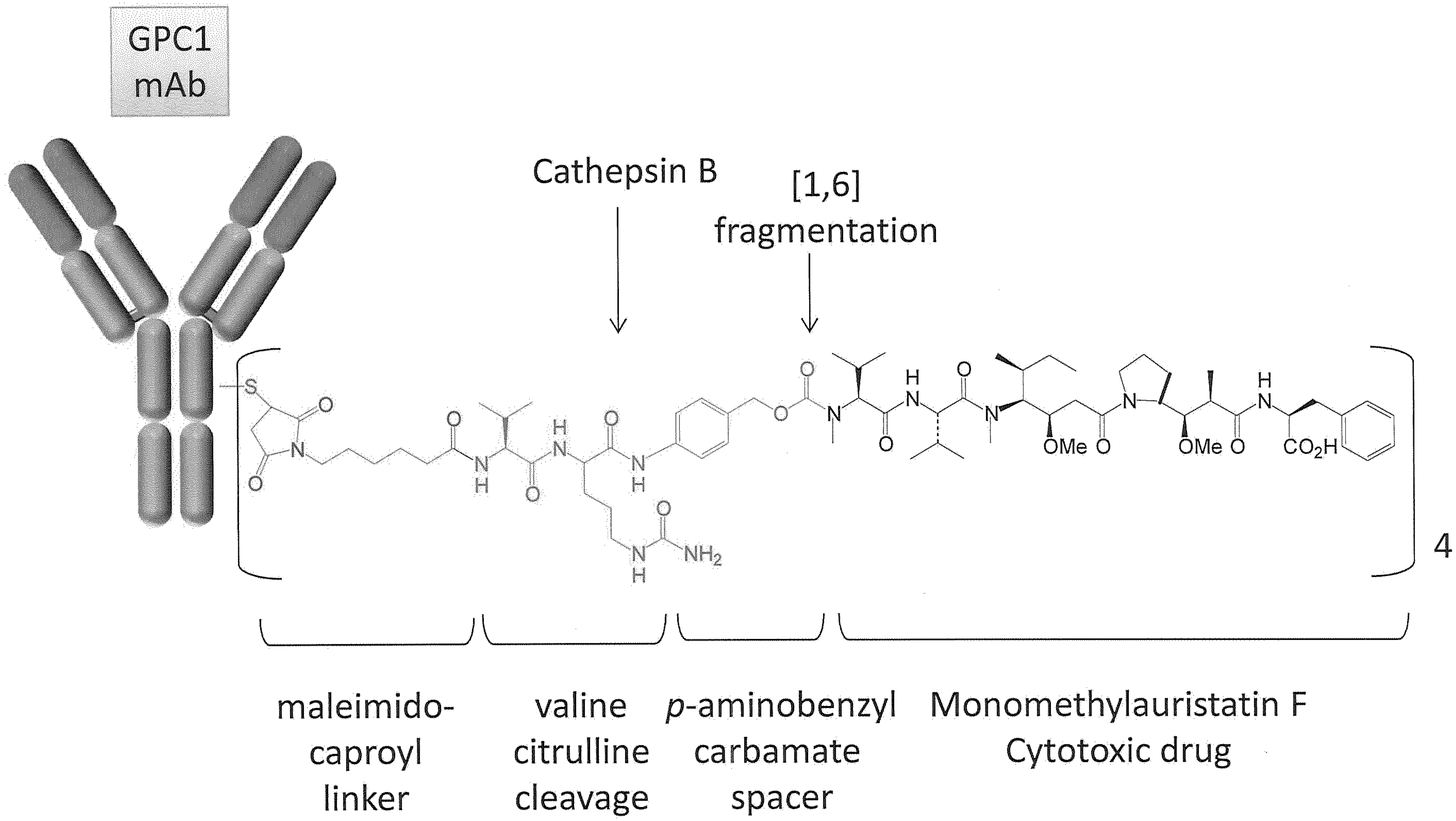
GPC1
mAb
Cathepsin B
[1,6]
fragmentation
4
maleimido-
caproyl
linker
valine
citrulline
cleavage
p-aminobenzyl
carbamate
spacer
Monomethylauristatin F
Cytotoxic drug

FIG.7

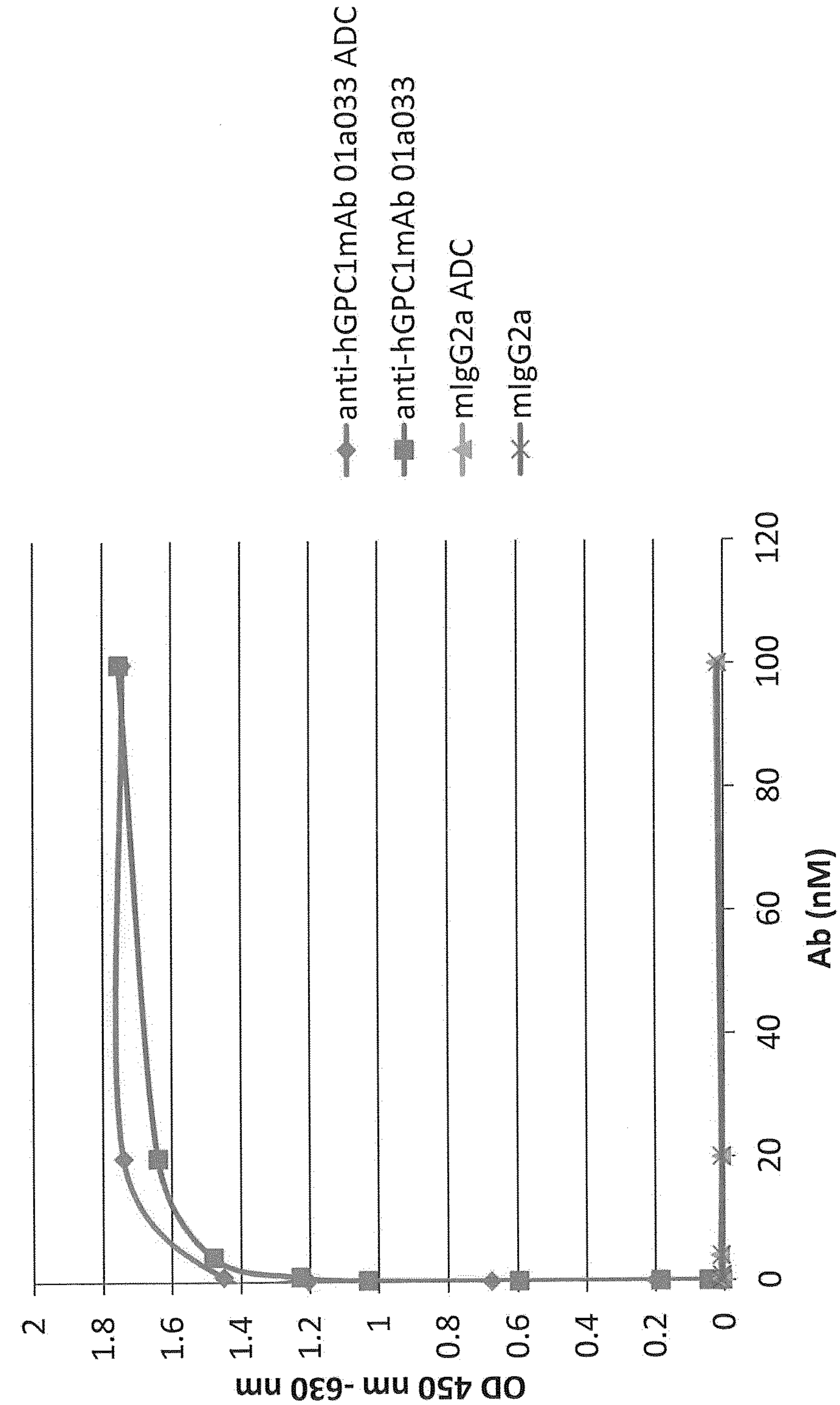
OD 450 nm -630 nm
2
1.8
1.6
1.4
1.2
1
0.8
0.6
0.4
0.2
0
0
20
40
60
80
100
120
Ab (nM)
anti-hGPC1mAb 01a033 ADC
anti-hGPC1mAb 01a033
mIgG2a ADC
mIgG2a

# FIG.8

| | GPC1 antibody binding capacity |
|---|---|
| TE4 | 32,567 |
| TE5 | 89,233 |
| TE6 | 94,959 |
| TE8 | 249,304 |
| TE9 | 132,169 |
| TE10 | 71,425 |
| TE11 | 206,909 |
| TE14 | 219,581 |
| TE15 | 91,755 |
| BxPC3 | 93,290 |
| T3M4 | 76,850 |
| HeLa | 62,584 |
| ME180 | 78,437 |
| LK2 | 13,394 |

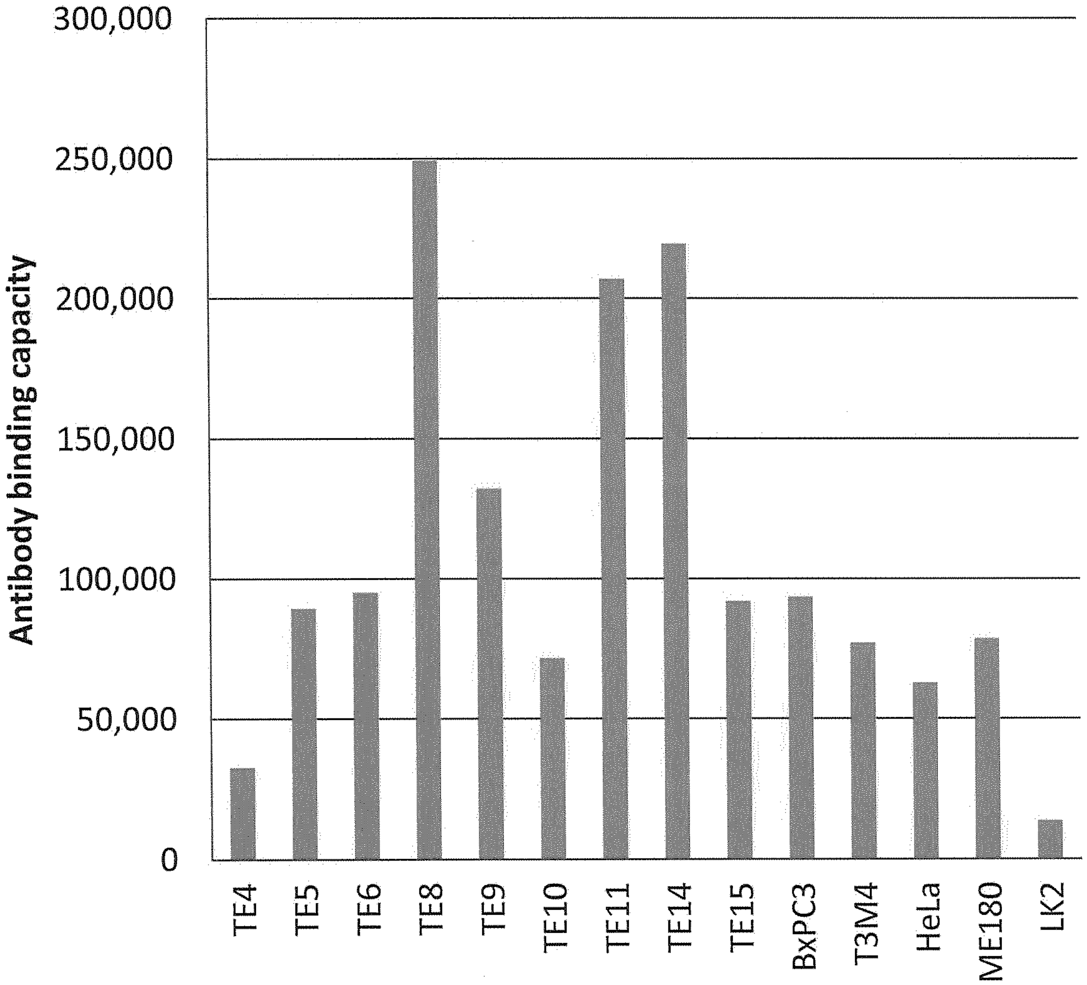

FIG.9

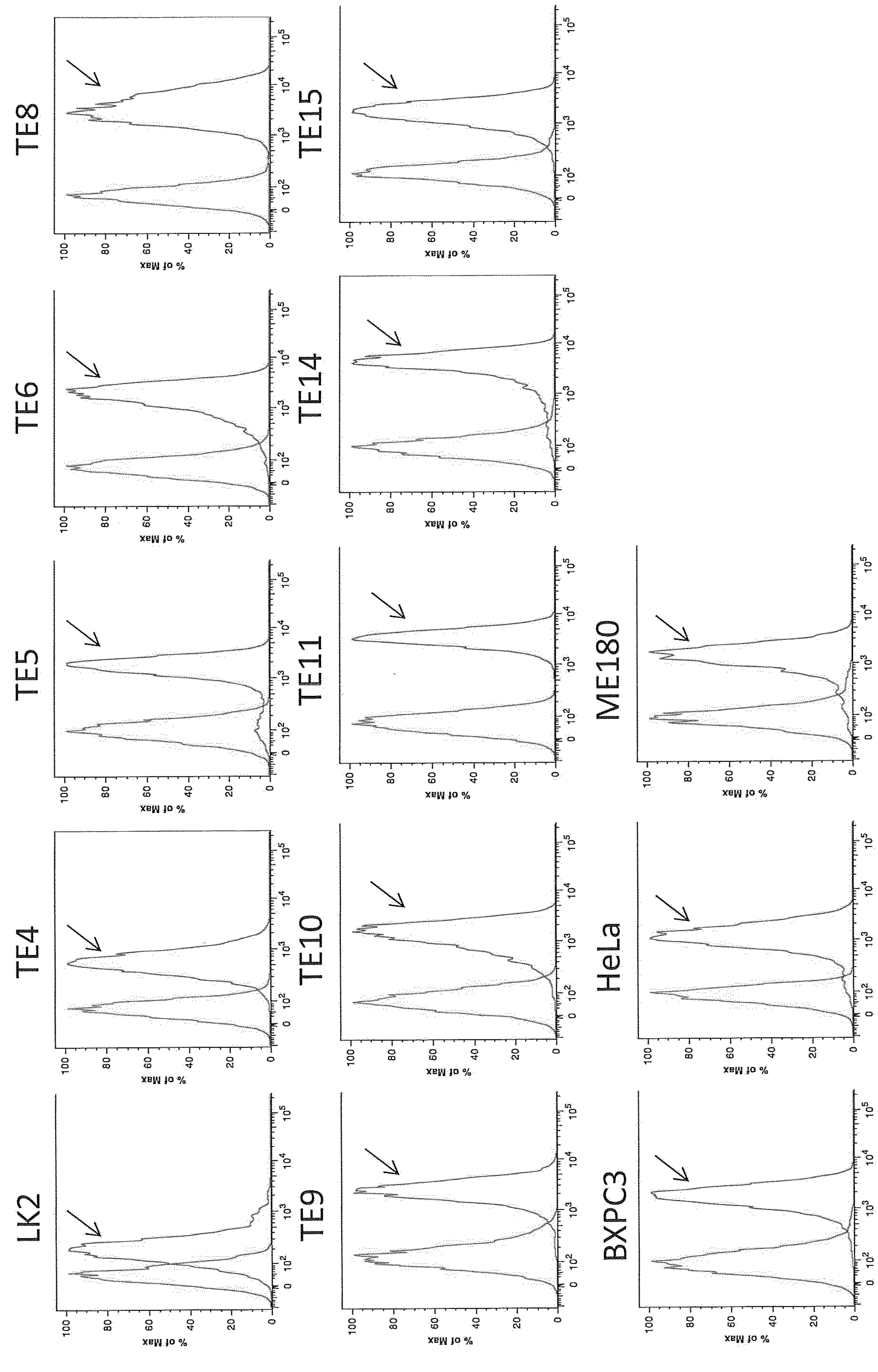
LK2
TE4
TE5
TE6
TE8
TE9
TE10
TE11
TE14
TE15
BXPC3
HeLa
ME180
% of Max

# FIG.10

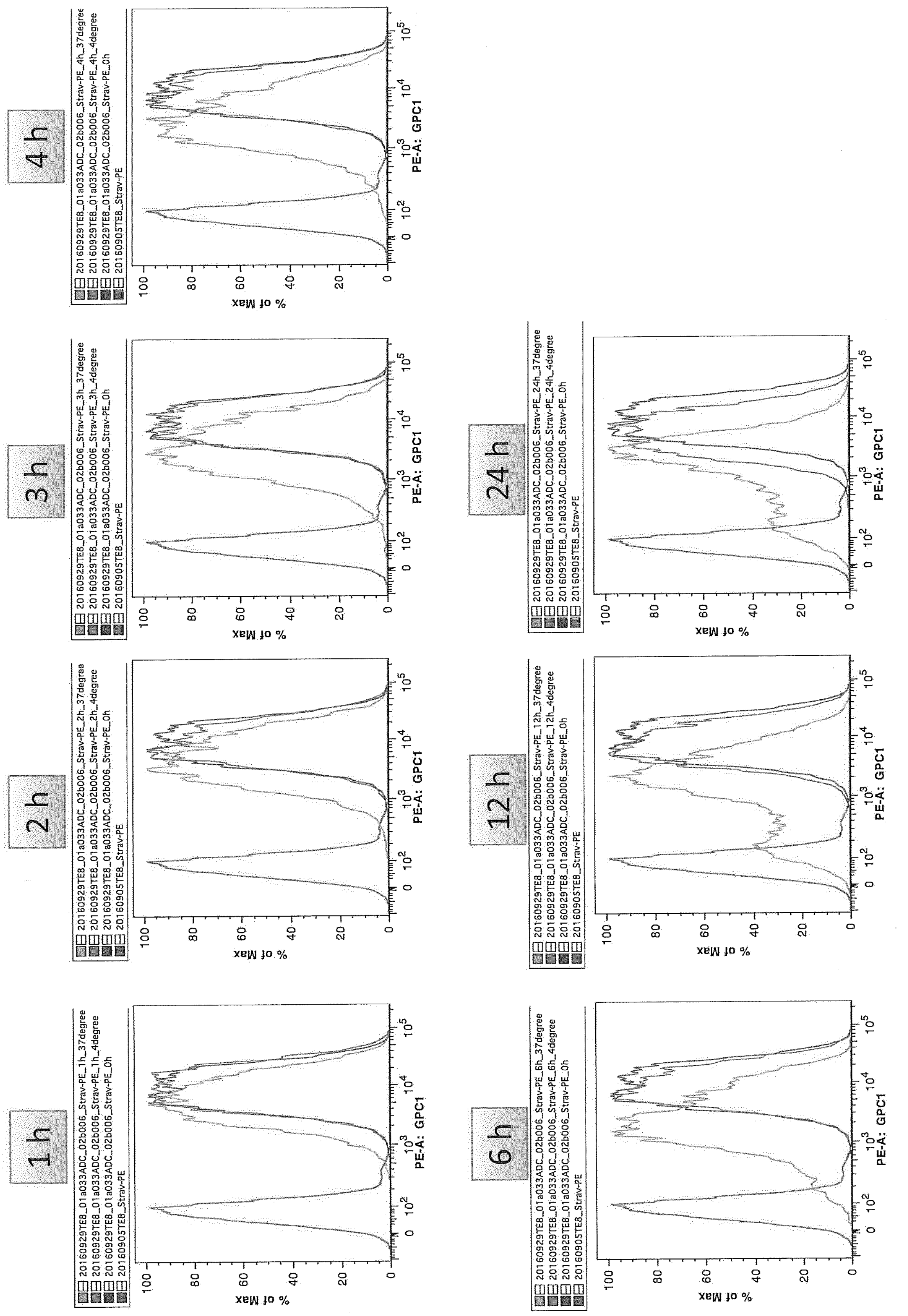
1 h
2 h
3 h
4 h
6 h
12 h
24 h
20160929TE8_01a033ADC_02b006_Strav-PE_1h_37degree
20160929TE8_01a033ADC_02b006_Strav-PE_1h_4degree
20160929TE8_01a033ADC_02b006_Strav-PE_0h
20160905TE8_Strav-PE
20160929TE8_01a033ADC_02b006_Strav-PE_2h_37degree
20160929TE8_01a033ADC_02b006_Strav-PE_2h_4degree
20160929TE8_01a033ADC_02b006_Strav-PE_3h_37degree
20160929TE8_01a033ADC_02b006_Strav-PE_3h_4degree
20160929TE8_01a033ADC_02b006_Strav-PE_4h_37degree
20160929TE8_01a033ADC_02b006_Strav-PE_4h_4degree
20160929TE8_01a033ADC_02b006_Strav-PE_6h_37degree
20160929TE8_01a033ADC_02b006_Strav-PE_6h_4degree
20160929TE8_01a033ADC_02b006_Strav-PE_12h_37degree
20160929TE8_01a033ADC_02b006_Strav-PE_12h_4degree
20160929TE8_01a033ADC_02b006_Strav-PE_24h_37degree
20160929TE8_01a033ADC_02b006_Strav-PE_24h_4degree
% of Max
PE-A: GPC1

FIG.11

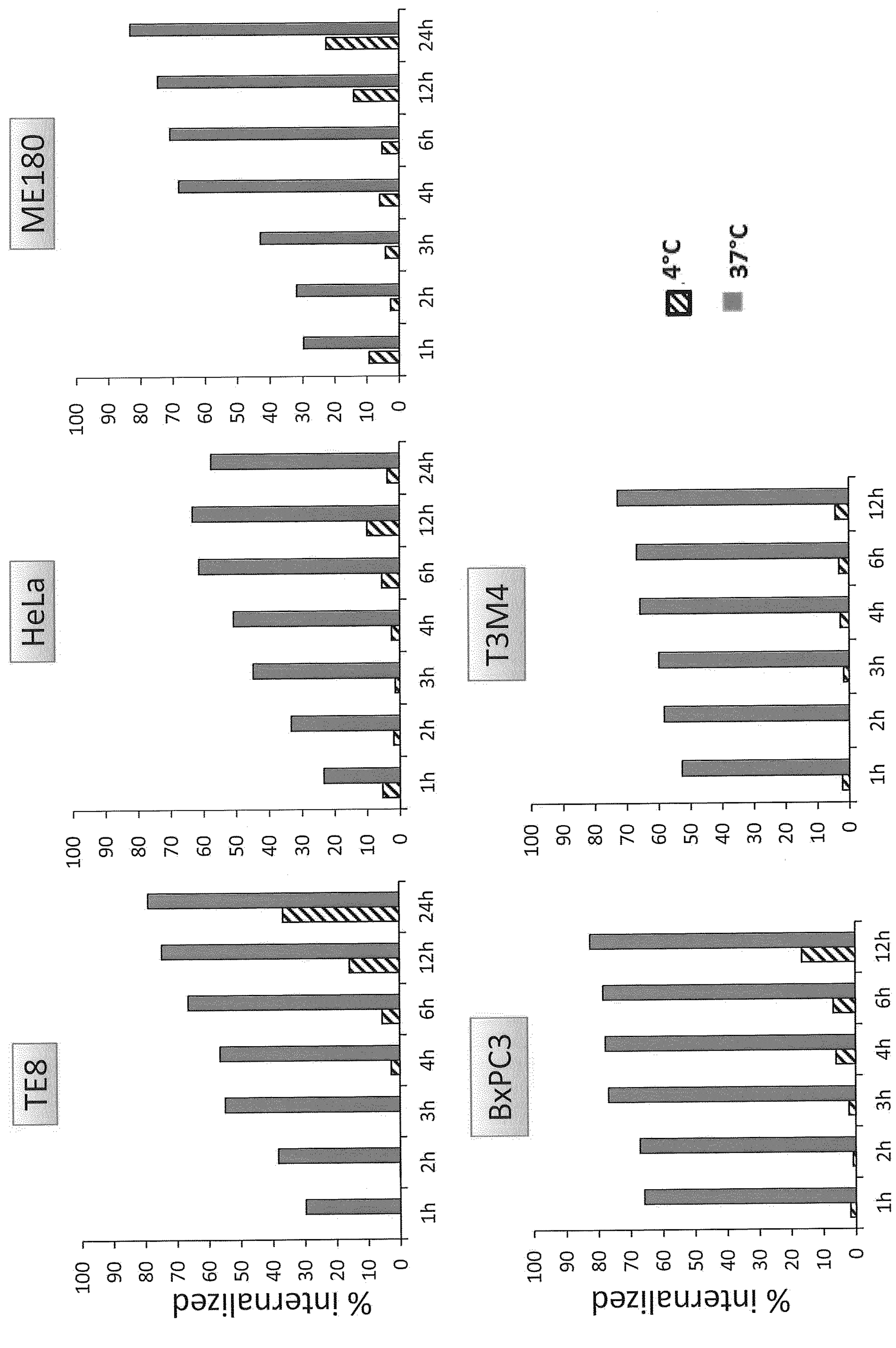
TE8
HeLa
ME180
BxPC3
T3M4
% internalized
100
90
80
70
60
50
40
30
20
10
0
1h
2h
3h
4h
6h
12h
24h
4°C
37°C

FIG.12

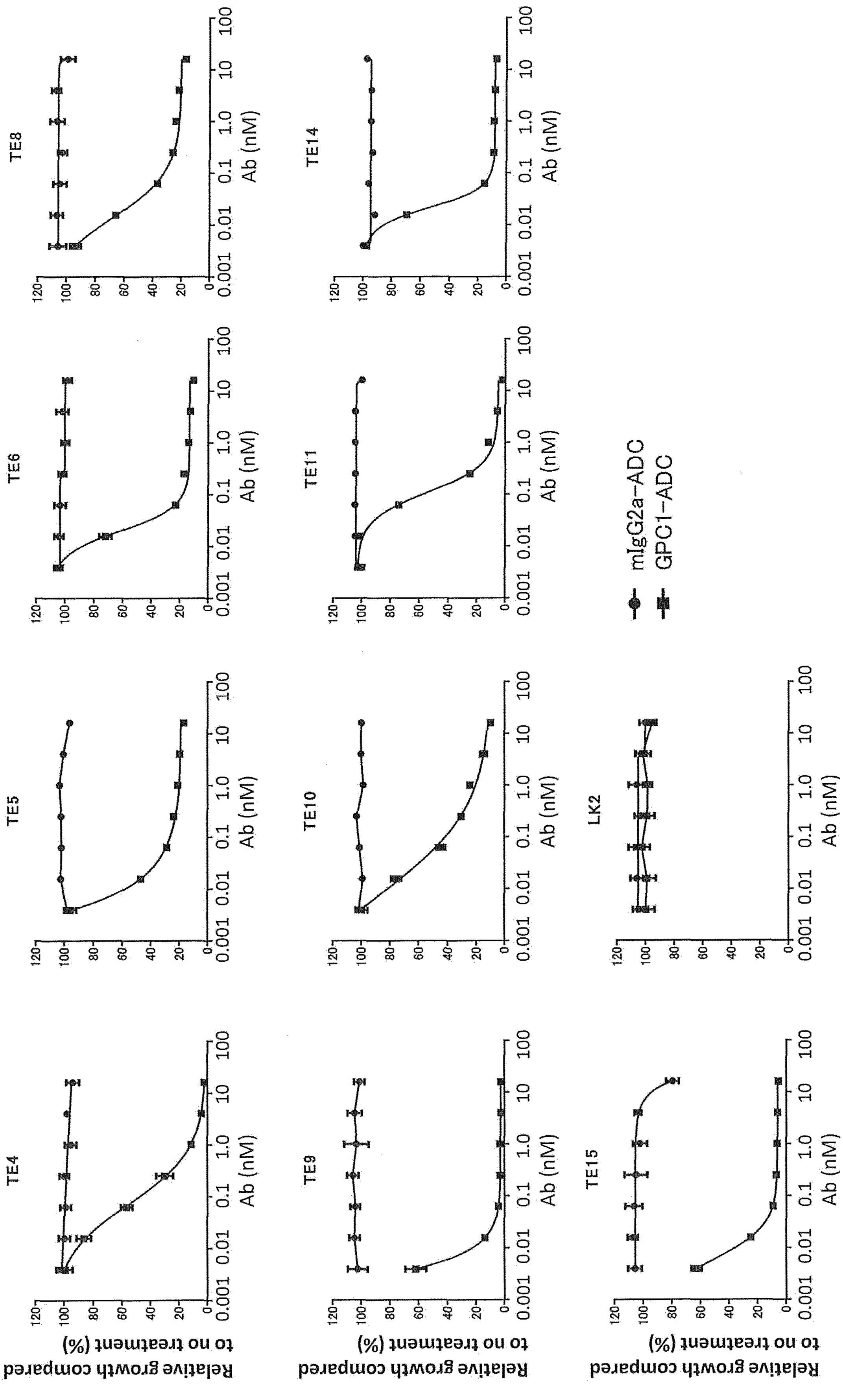
TE4
TE5
TE6
TE8
TE9
TE10
TE11
TE14
TE15
LK2
Relative growth compared to no treatment (%)
Ab (nM)
0.001 0.01 0.1 1.0 10 100
0 20 40 60 80 100 120
mIgG2a-ADC
GPC1-ADC

FIG.13

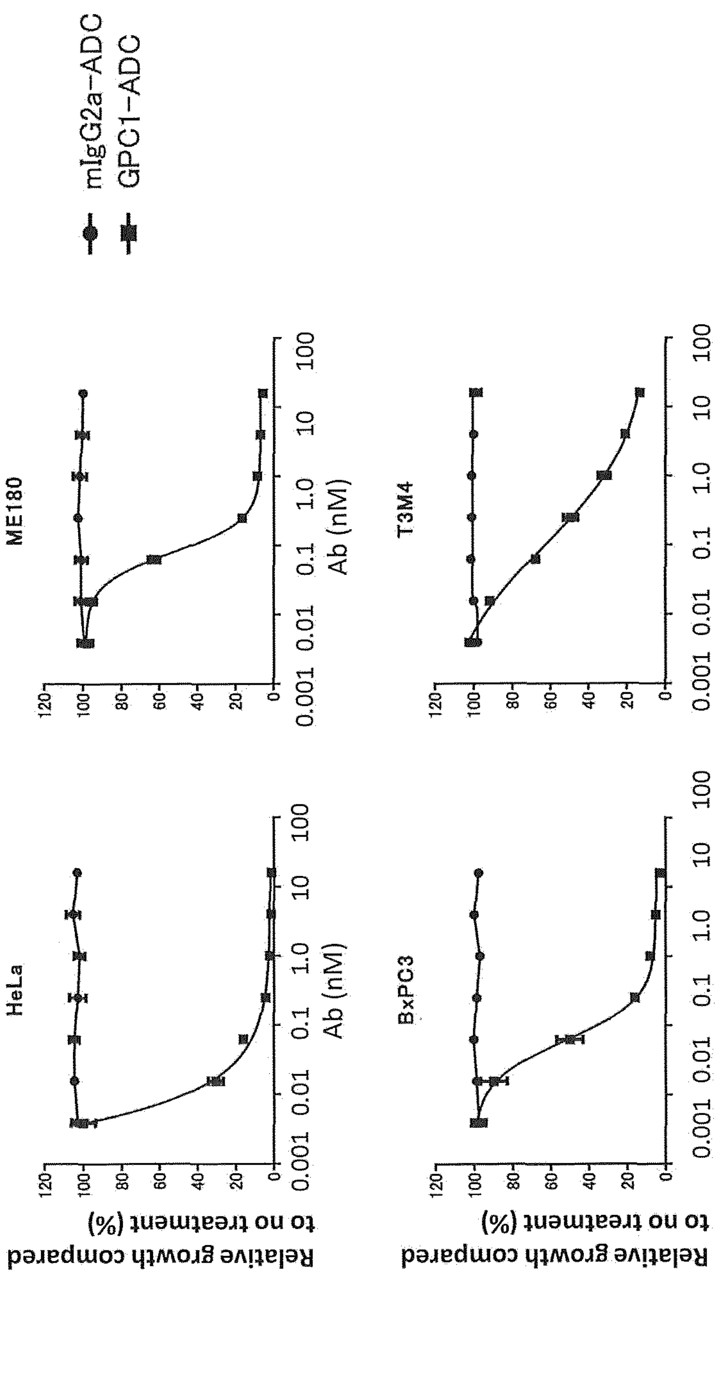
HeLa
ME180
BxPC3
T3M4
Relative growth compared to no treatment (%)
Ab (nM)
0.001 0.01 0.1 1.0 10 100
0 20 40 60 80 100 120
mIgG2a-ADC
GPC1-ADC

# FIG.14

| | GPC1 antibody binding capacity | anti-GPC1 mAb (01a033) ADC (nM) | MMAE (nM) | MMAF (nM) | cells/well |
|---|---|---|---|---|---|
| TE4 | 32,567 | 0.0898 | 0.043 | 21.9 | 1,500 |
| TE5 | 89,233 | 0.0578 | 0.137 | 27.5 | 2,000 |
| TE6 | 94,959 | 0.0291 | 0.353 | 36.0 | 2,000 |
| TE8 | 249,304 | 0.0334 | 0.304 | 42.8 | 1,500 |
| TE9 | 132,169 | 0.0055 | 0.099 | 25 | 1,000 |
| TE10 | 71,425 | 0.0492 | 0.185 | 28.0 | 2,000 |
| TE11 | 206,909 | 0.1235 | 0.338 | 31.4 | 1,500 |
| TE14 | 219,581 | 0.0257 | 0.296 | 26.8 | 2,000 |
| TE15 | 91,755 | 0.0063 | 0.165 | 20.4 | 2,000 |
| BxPC3 | 93,290 | 0.0629 | 0.310 | 31.3 | 1,500 |
| T3M4 | 76,850 | 0.243 | 0.327 | 24.4 | 1,500 |
| HeLa | 62,584 | 0.0110 | 0.035 | 29.7 | 1,500 |
| ME180 | 78,437 | 0.0930 | 0.002 | 35.4 | 2,000 |
| LK2 | 13,394 | n.d. | 0.235 | 31.9 | 2,000 |

FIG.15

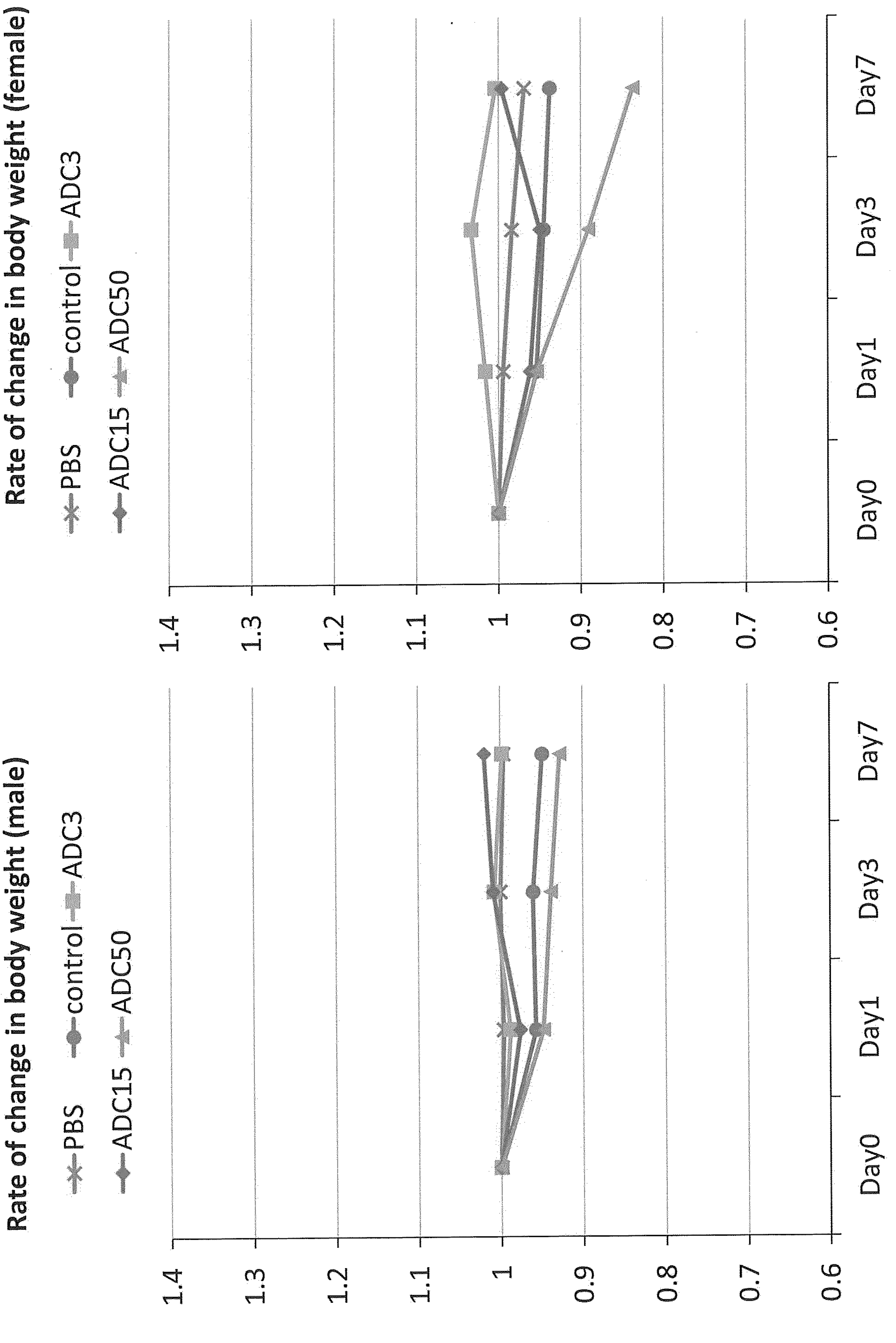

Rate of change in body weight (male)
PBS
control
ADC3
ADC15
ADC50
1.4
1.3
1.2
1.1
1
0.9
0.8
0.7
0.6
Day0
Day1
Day3
Day7
Rate of change in body weight (female)
PBS
control
ADC3
ADC15
ADC50
1.4
1.3
1.2
1.1
1
0.9
0.8
0.7
0.6
Day0
Day1
Day3
Day7

FIG.16

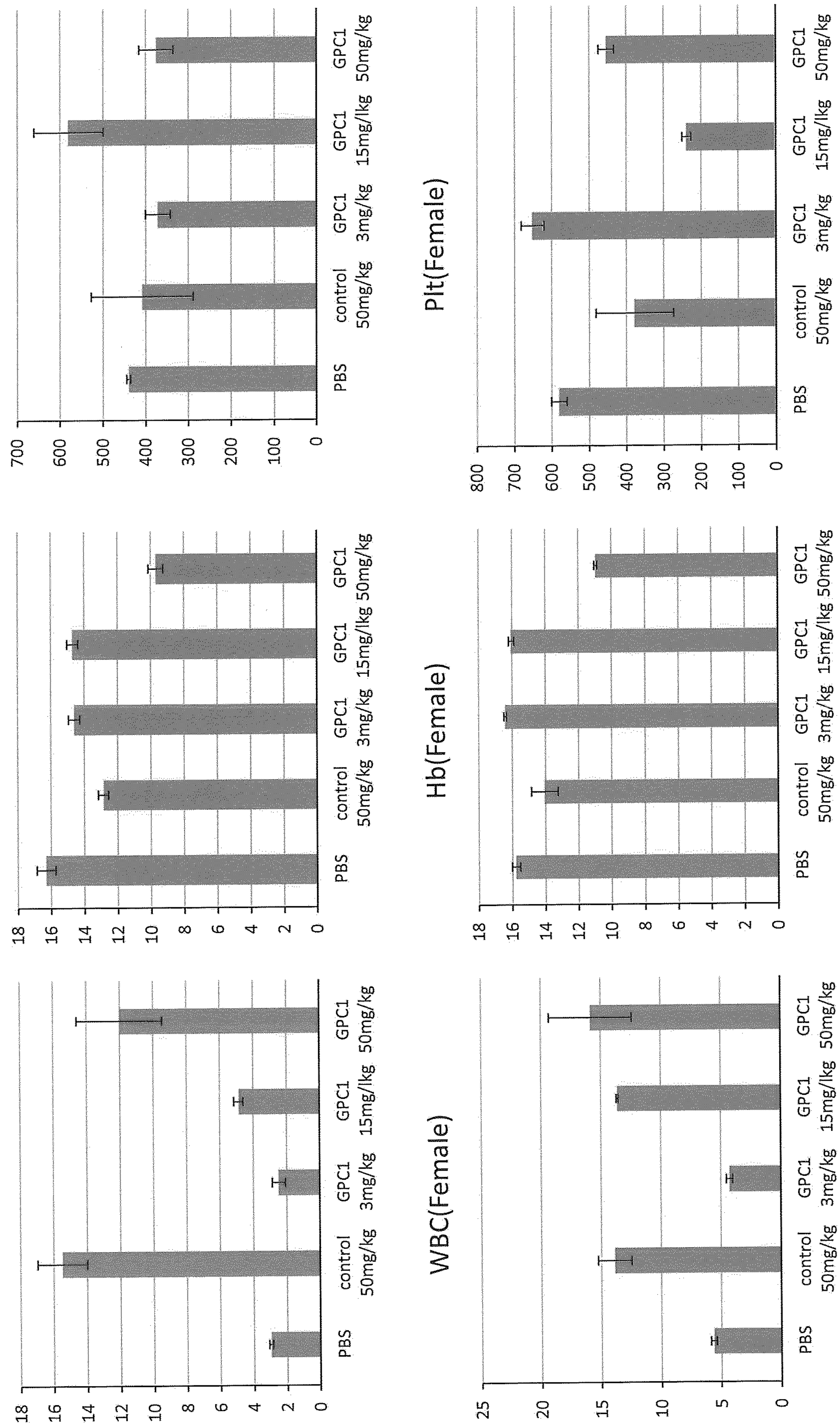
WBC(Male)
0
2
4
6
8
10
12
14
16
18
Hb(Male)
0
2
4
6
8
10
12
14
16
18
Plt(Male)
0
100
200
300
400
500
600
700
WBC(Female)
0
5
10
15
20
25
Hb(Female)
0
2
4
6
8
10
12
14
16
18
Plt(Female)
0
100
200
300
400
500
600
700
800
PBS
control 50mg/kg
GPC1 3mg/kg
GPC1 15mg/lkg
GPC1 50mg/kg

FIG.17

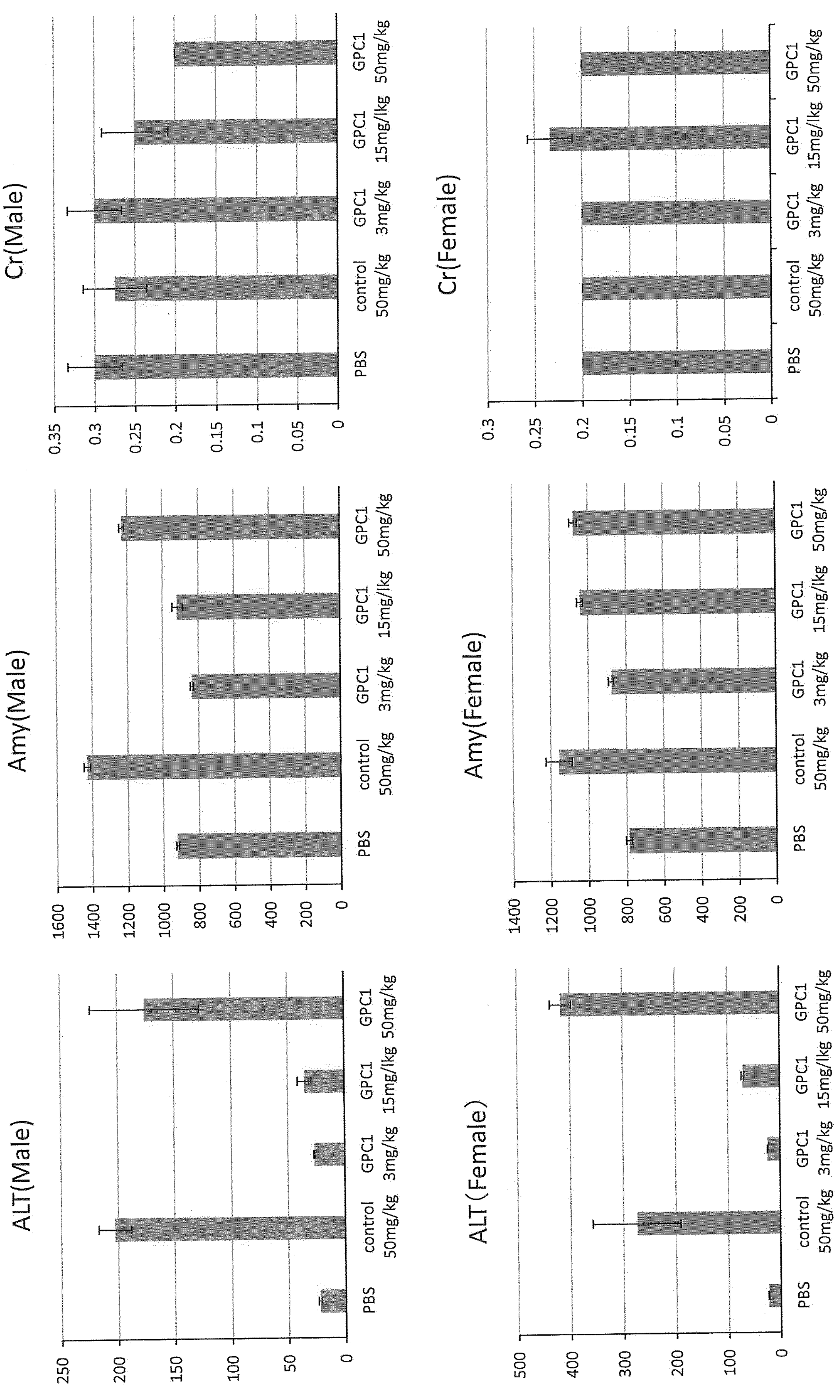
ALT(Male)
250
200
150
100
50
0
PBS
control 50mg/kg
GPC1 3mg/kg
GPC1 15mg/lkg
GPC1 50mg/kg
Amy(Male)
1600
1400
1200
1000
800
600
400
200
0
Cr(Male)
0.35
0.3
0.25
0.2
0.15
0.1
0.05
0
ALT(Female)
500
400
300
200
100
0
Amy(Female)
1400
1200
1000
800
600
400
200
0
Cr(Female)
0.3
0.25
0.2
0.15
0.1
0.05
0

FIG.18

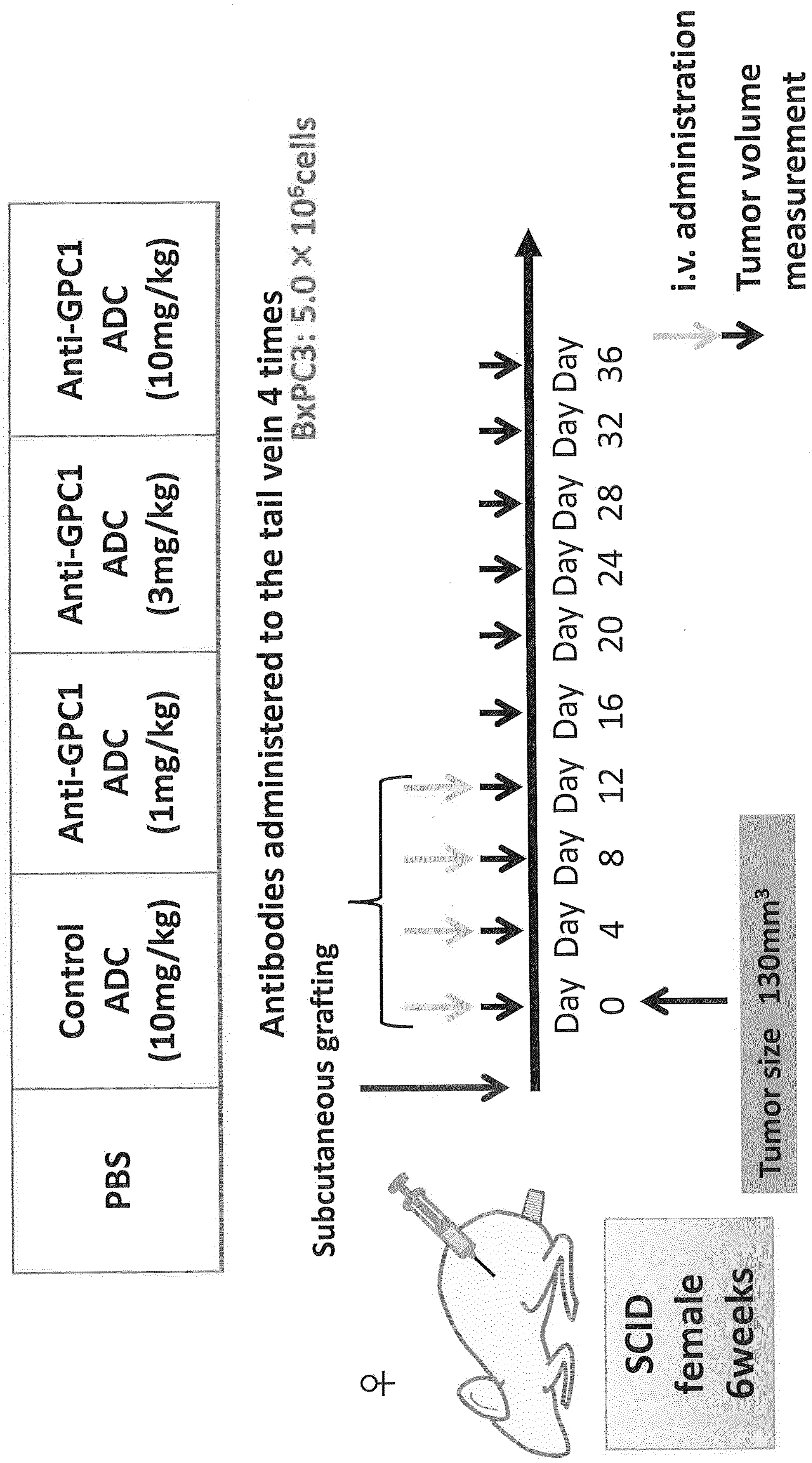
PBS
Control ADC (10mg/kg)
Anti-GPC1 ADC (1mg/kg)
Anti-GPC1 ADC (3mg/kg)
Anti-GPC1 ADC (10mg/kg)
Antibodies administered to the tail vein 4 times
BxPC3: 5.0 × 10⁶cells
Subcutaneous grafting
♀
SCID female 6weeks
Day 0
Day 4
Day 8
Day 12
Day 16
Day 20
Day 24
Day 28
Day 32
Day 36
Tumor size 130mm³
i.v. administration
Tumor volume measurement

FIG.19

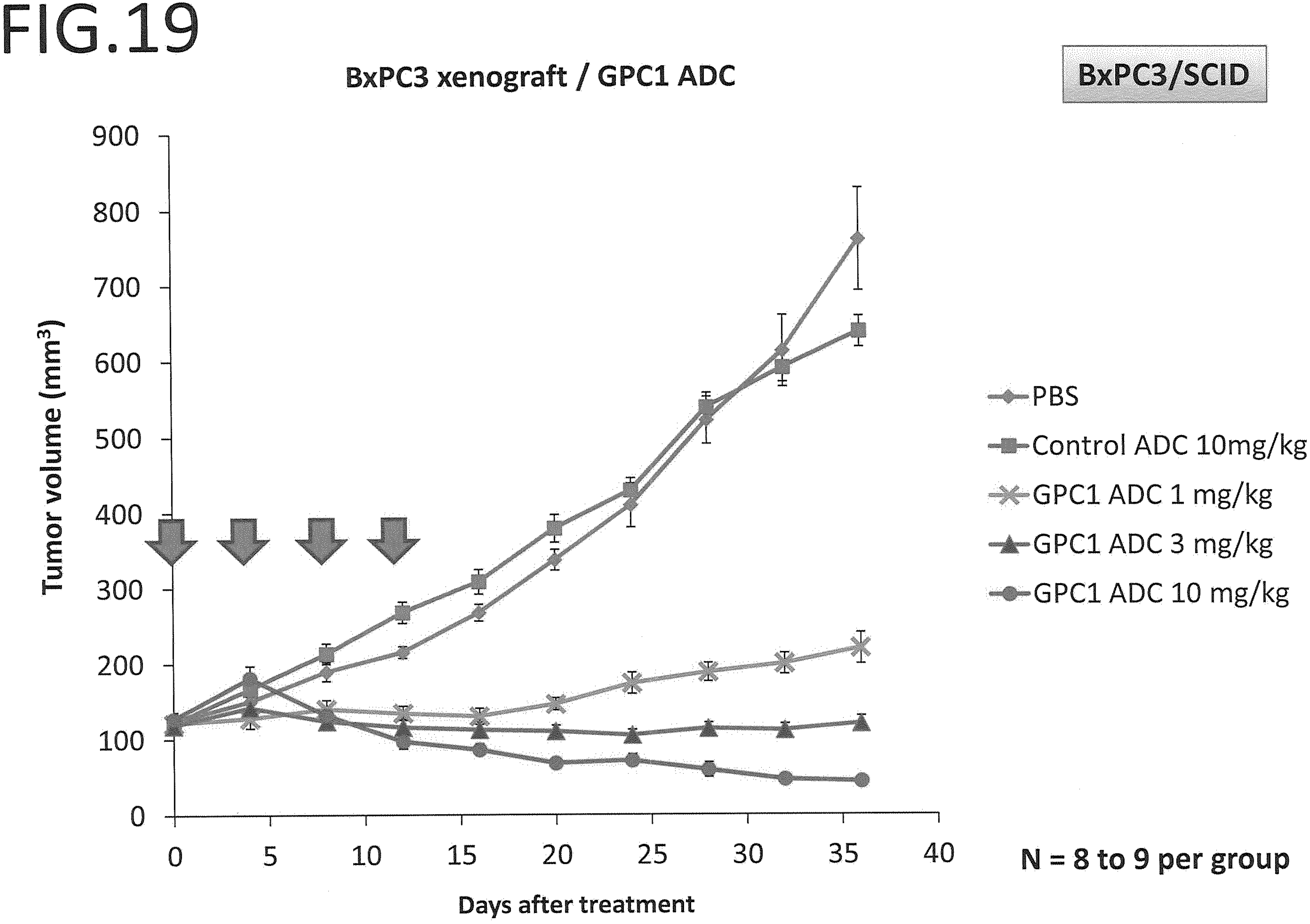
BxPC3 xenograft / GPC1 ADC
BxPC3/SCID
Tumor volume (mm³)
900
800
700
600
500
400
300
200
100
0
0
5
10
15
20
25
30
35
40
Days after treatment
PBS
Control ADC 10mg/kg
GPC1 ADC 1 mg/kg
GPC1 ADC 3 mg/kg
GPC1 ADC 10 mg/kg
N = 8 to 9 per group

FIG.20

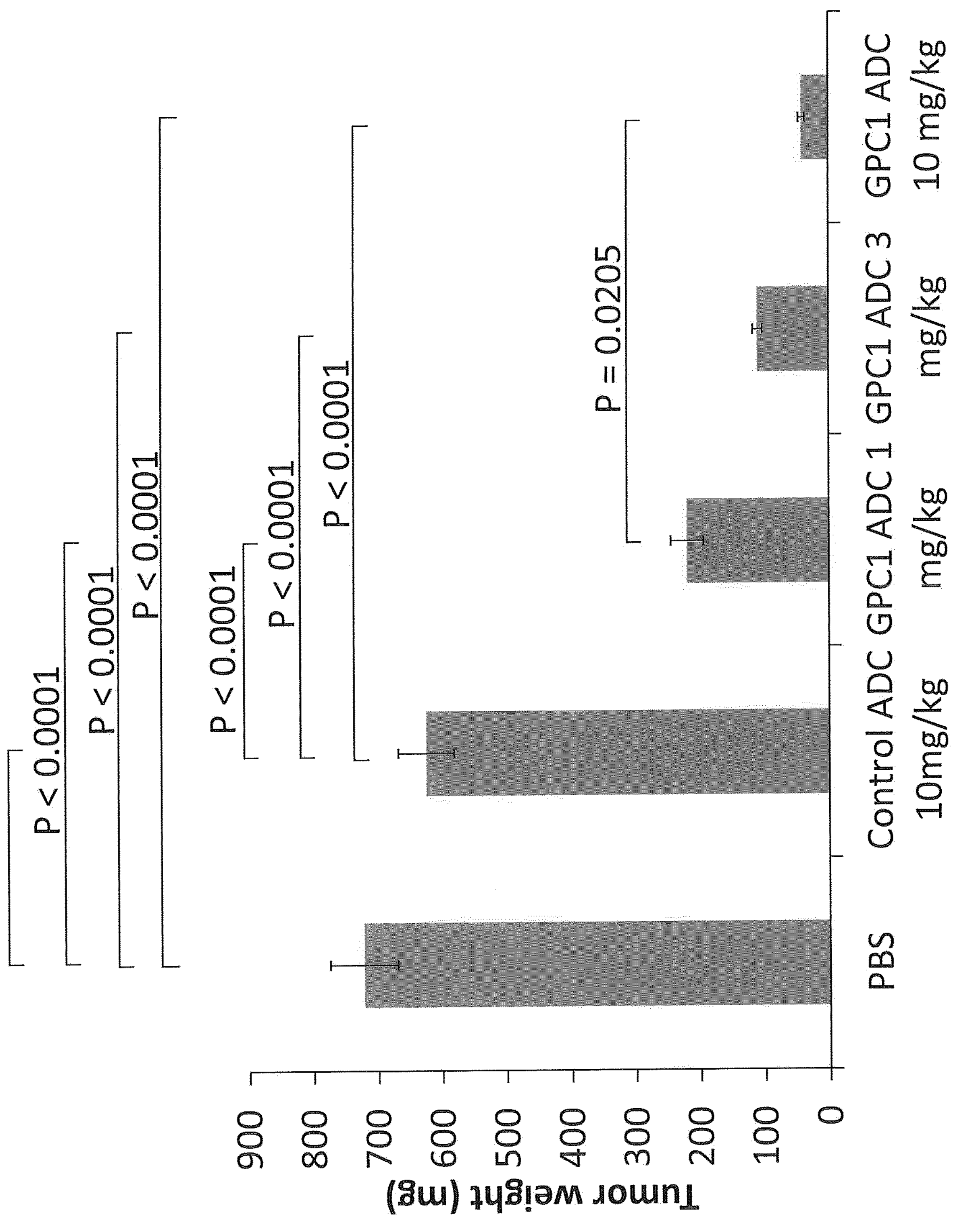
BxPC3/SCID
Tumor weight (mg)
900
800
700
600
500
400
300
200
100
0
PBS
Control ADC 10mg/kg
GPC1 ADC 1 mg/kg
GPC1 ADC 3 mg/kg
GPC1 ADC 10 mg/kg
N.S.
P < 0.0001
P < 0.0001
P < 0.0001
P < 0.0001
P < 0.0001
P < 0.0001
P = 0.0205

FIG.21

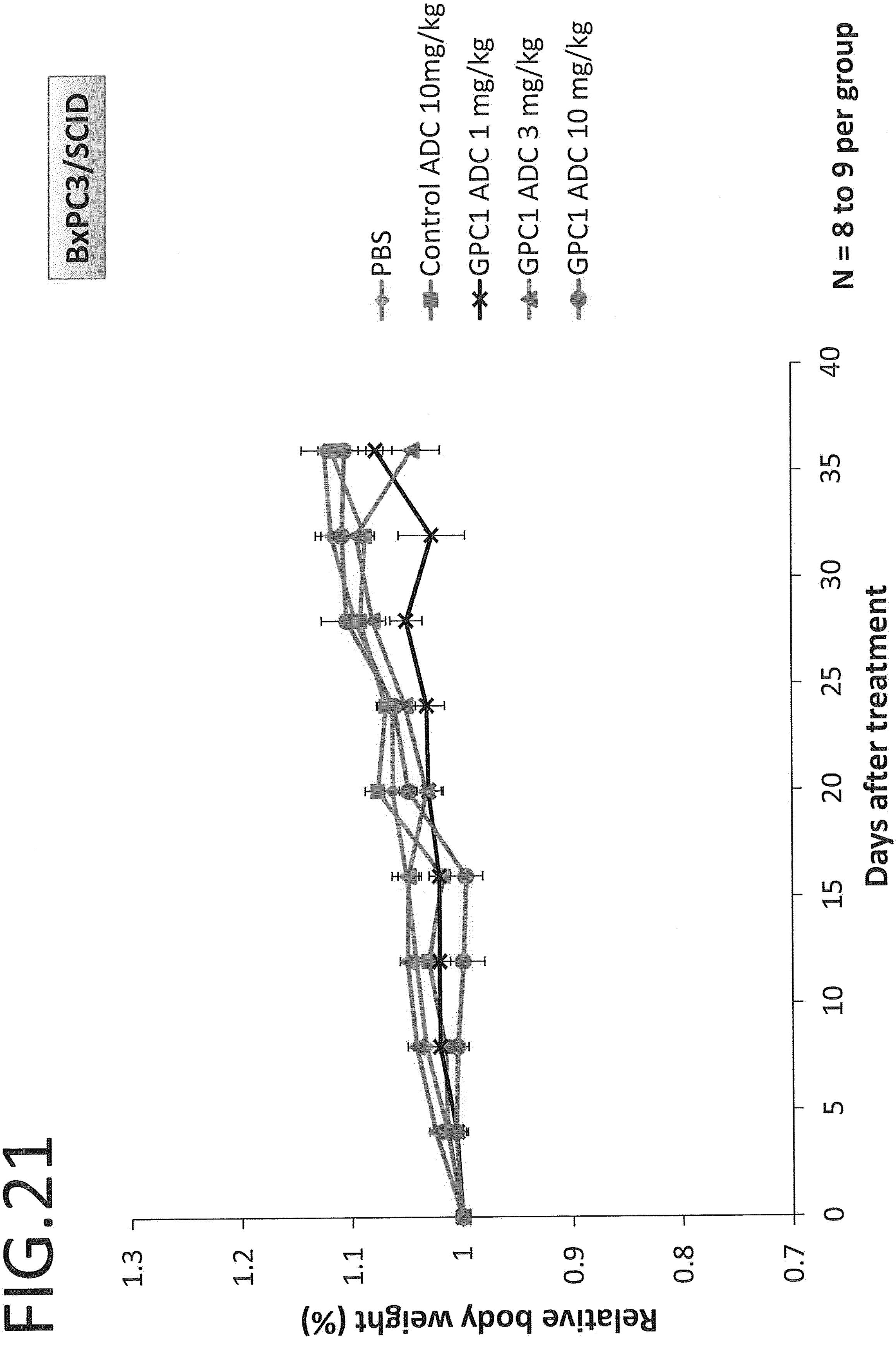
BxPC3/SCID
Relative body weight (%)
1.3
1.2
1.1
1
0.9
0.8
0.7
0
5
10
15
20
25
30
35
40
Days after treatment
PBS
Control ADC 10mg/kg
GPC1 ADC 1 mg/kg
GPC1 ADC 3 mg/kg
GPC1 ADC 10 mg/kg
N = 8 to 9 per group

FIG.22

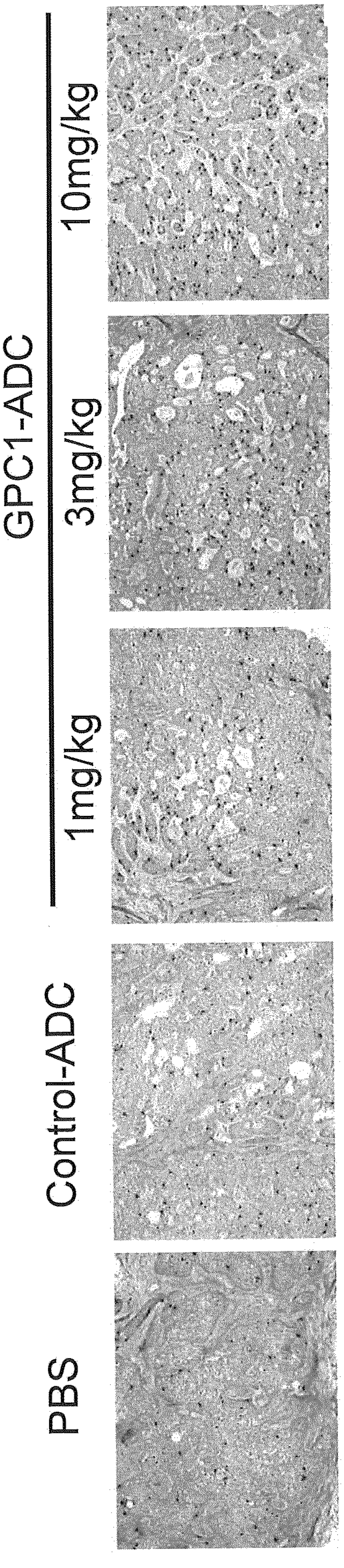

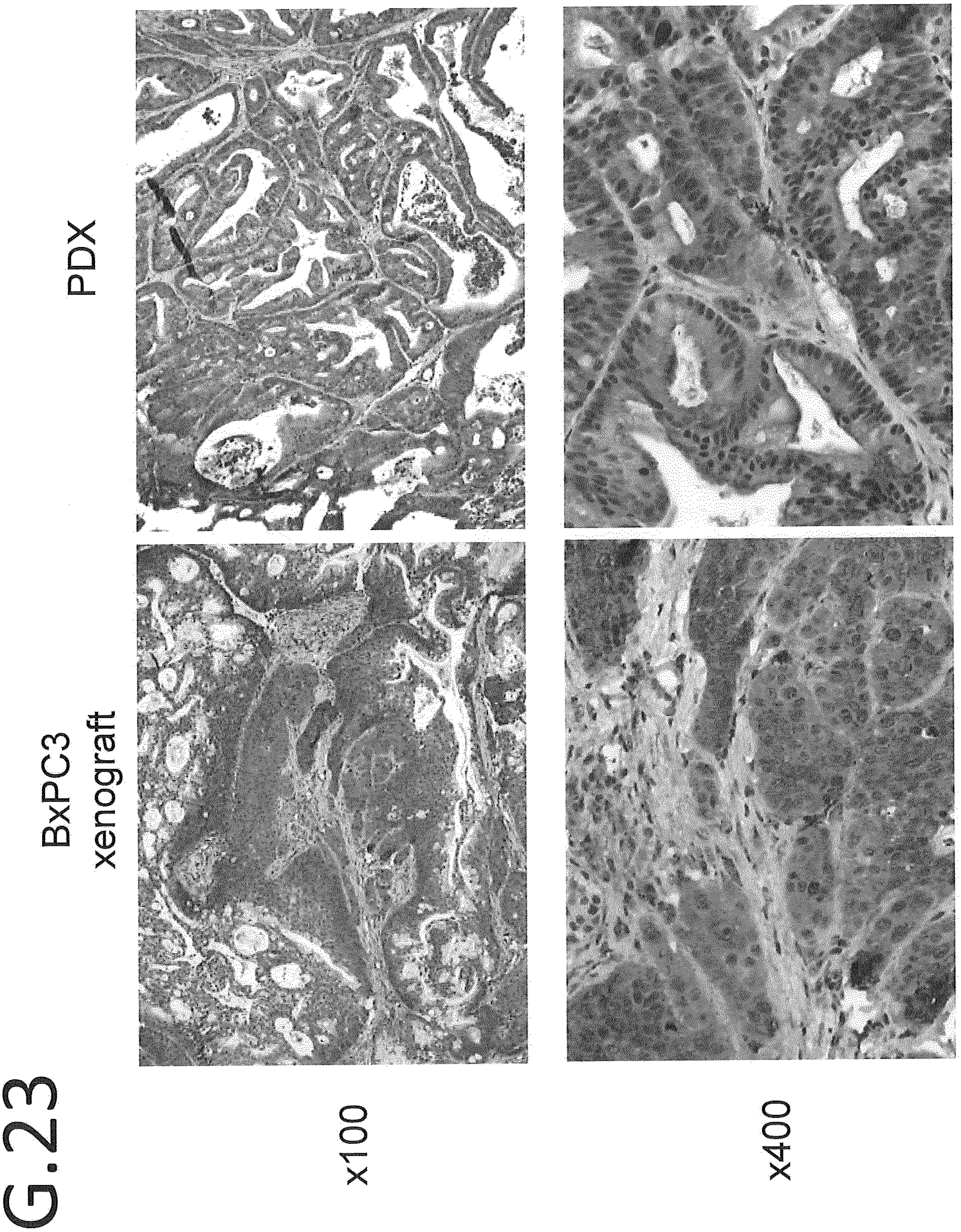


FIG.23

FIG.24

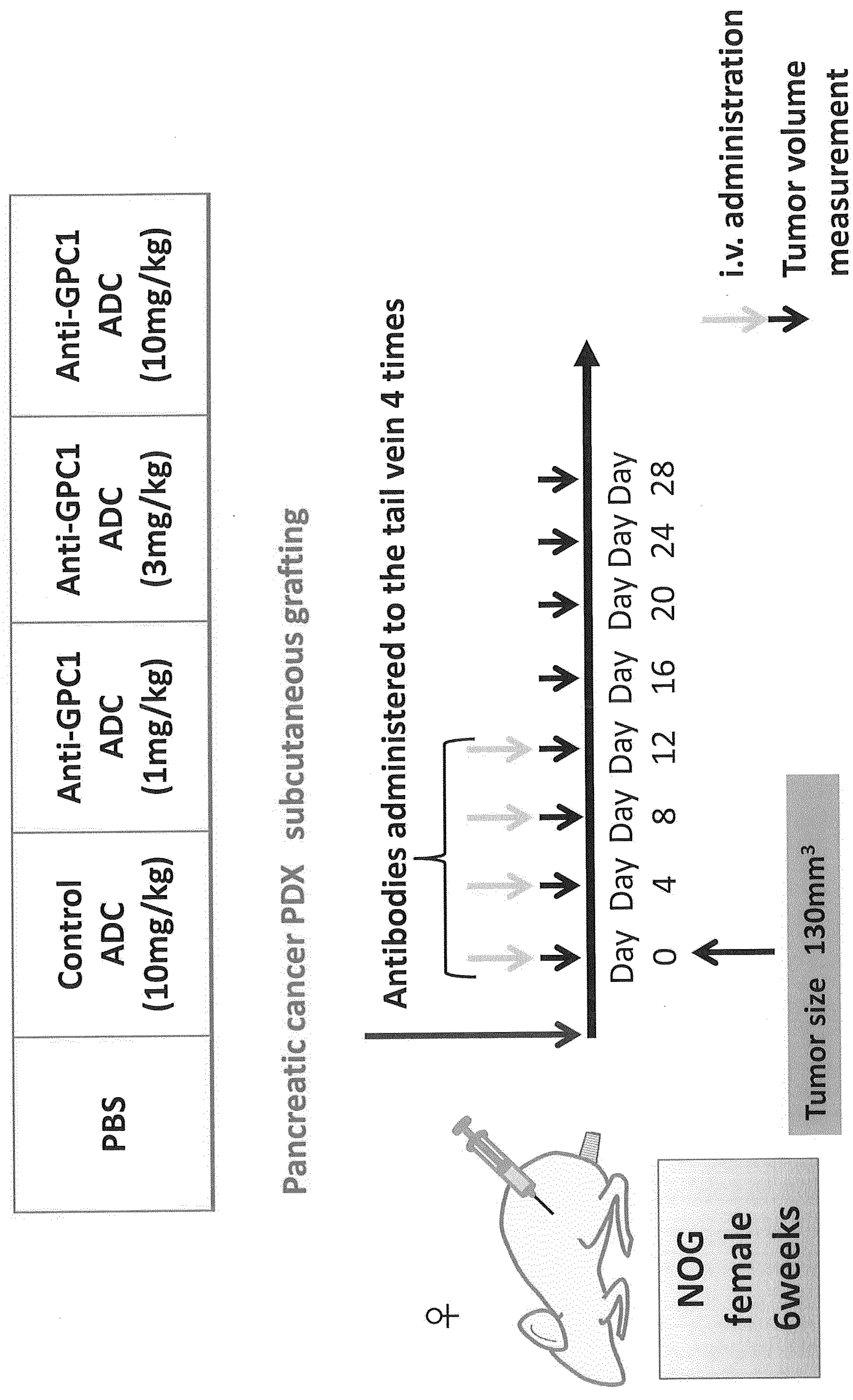
PBS
Control ADC (10mg/kg)
Anti-GPC1 ADC (1mg/kg)
Anti-GPC1 ADC (3mg/kg)
Anti-GPC1 ADC (10mg/kg)
Pancreatic cancer PDX subcutaneous grafting
Antibodies administered to the tail vein 4 times
Day 0
Day 4
Day 8
Day 12
Day 16
Day 20
Day 24
Day 28
Tumor size 130mm3
NOG female 6weeks
i.v. administration
Tumor volume measurement

FIG.25

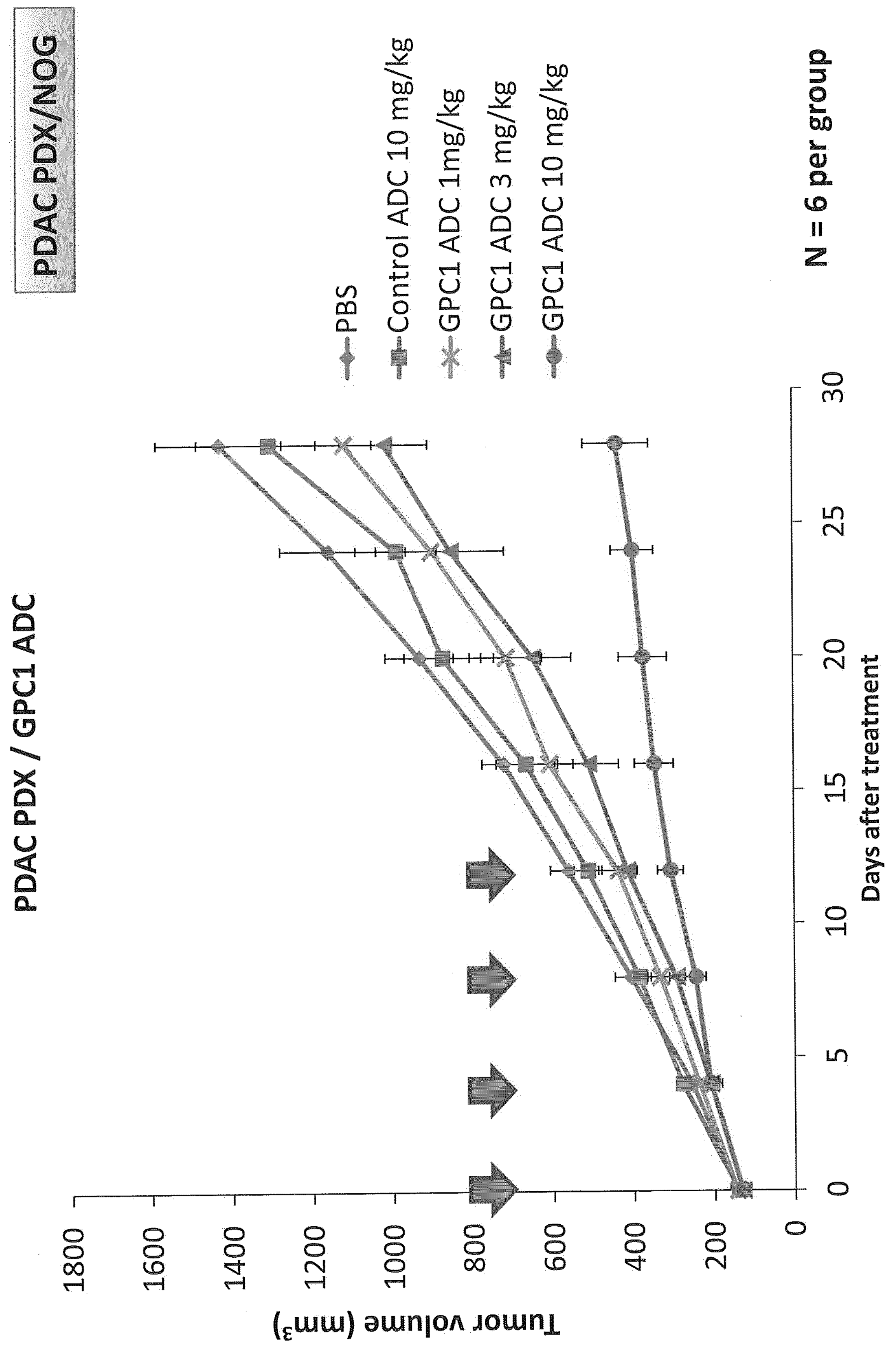
PDAC PDX/NOG
PDAC PDX / GPC1 ADC
PBS
Control ADC 10 mg/kg
GPC1 ADC 1mg/kg
GPC1 ADC 3 mg/kg
GPC1 ADC 10 mg/kg
N = 6 per group
Tumor volume (mm³)
0
200
400
600
800
1000
1200
1400
1600
1800
Days after treatment
0
5
10
15
20
25
30

FIG.26

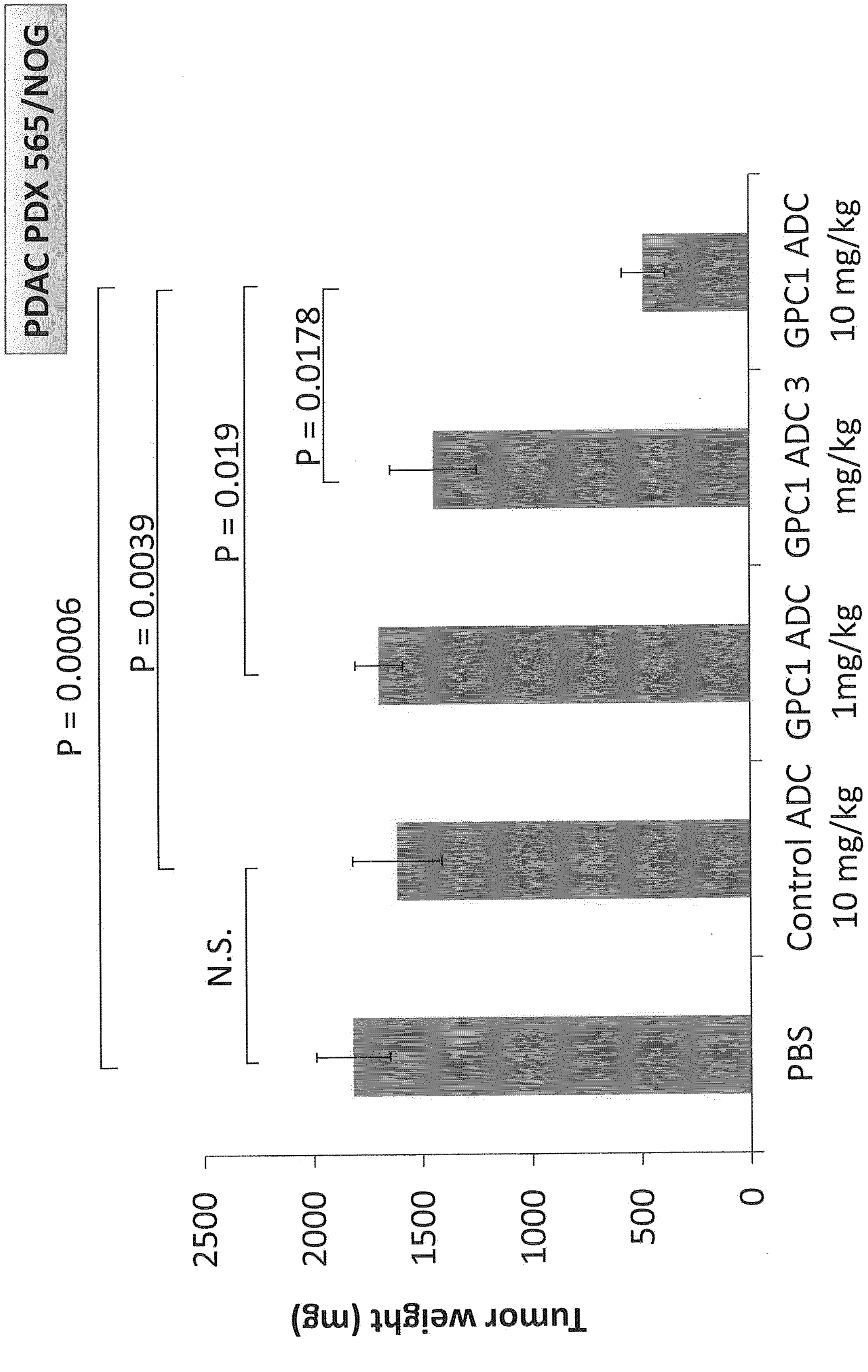
PDAC PDX 565/NOG
P = 0.0006
P = 0.0039
P = 0.019
P = 0.0178
N.S.
Tumor weight (mg)
2500
2000
1500
1000
500
0
PBS
Control ADC
10 mg/kg
GPC1 ADC
1mg/kg
GPC1 ADC 3
mg/kg
GPC1 ADC
10 mg/kg

FIG.27

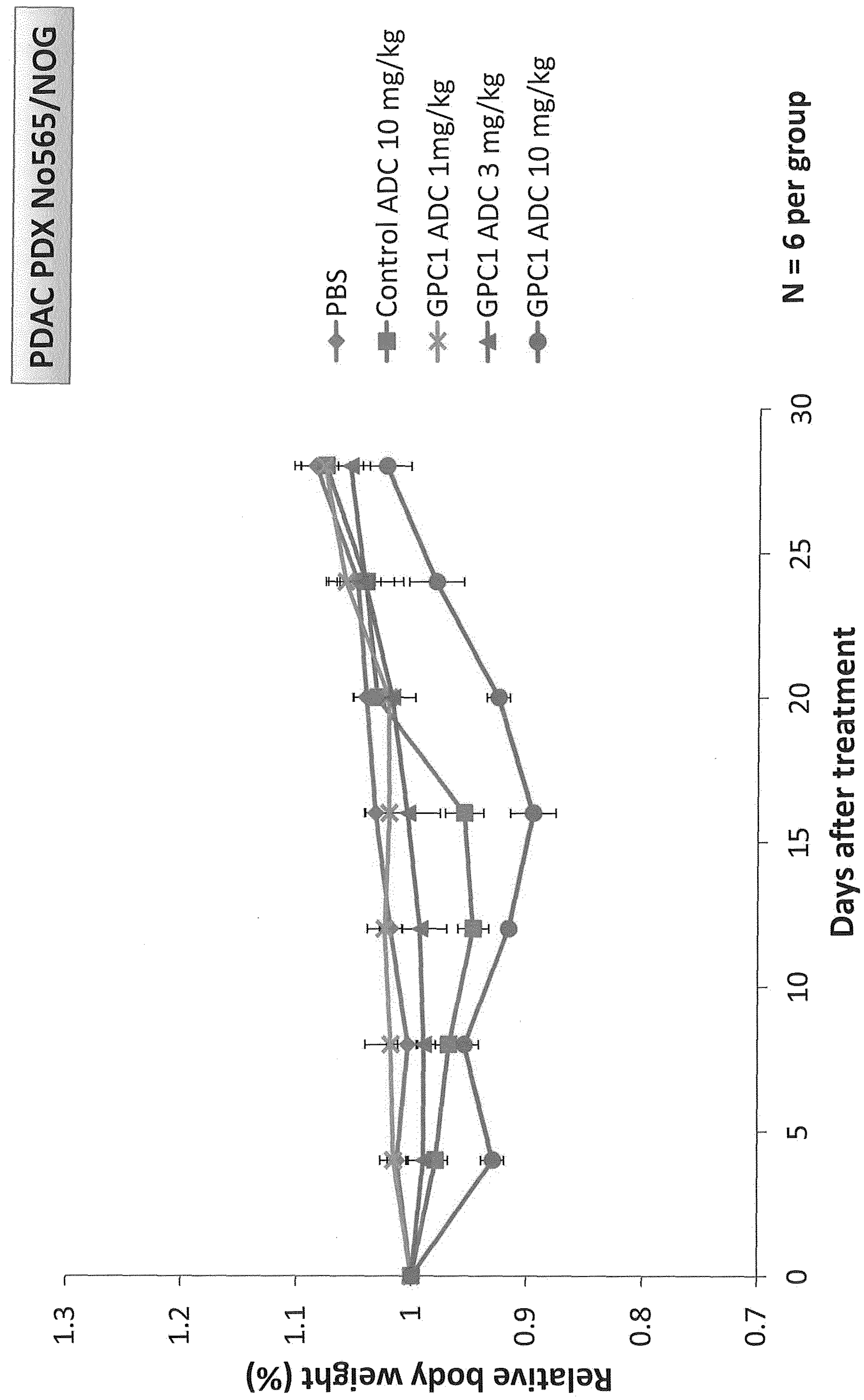
PDAC PDX No565/NOG
PBS
Control ADC 10 mg/kg
GPC1 ADC 1mg/kg
GPC1 ADC 3 mg/kg
GPC1 ADC 10 mg/kg
N = 6 per group
Relative body weight (%)
1.3
1.2
1.1
1
0.9
0.8
0.7
0
5
10
15
20
25
30
Days after treatment

FIG.28

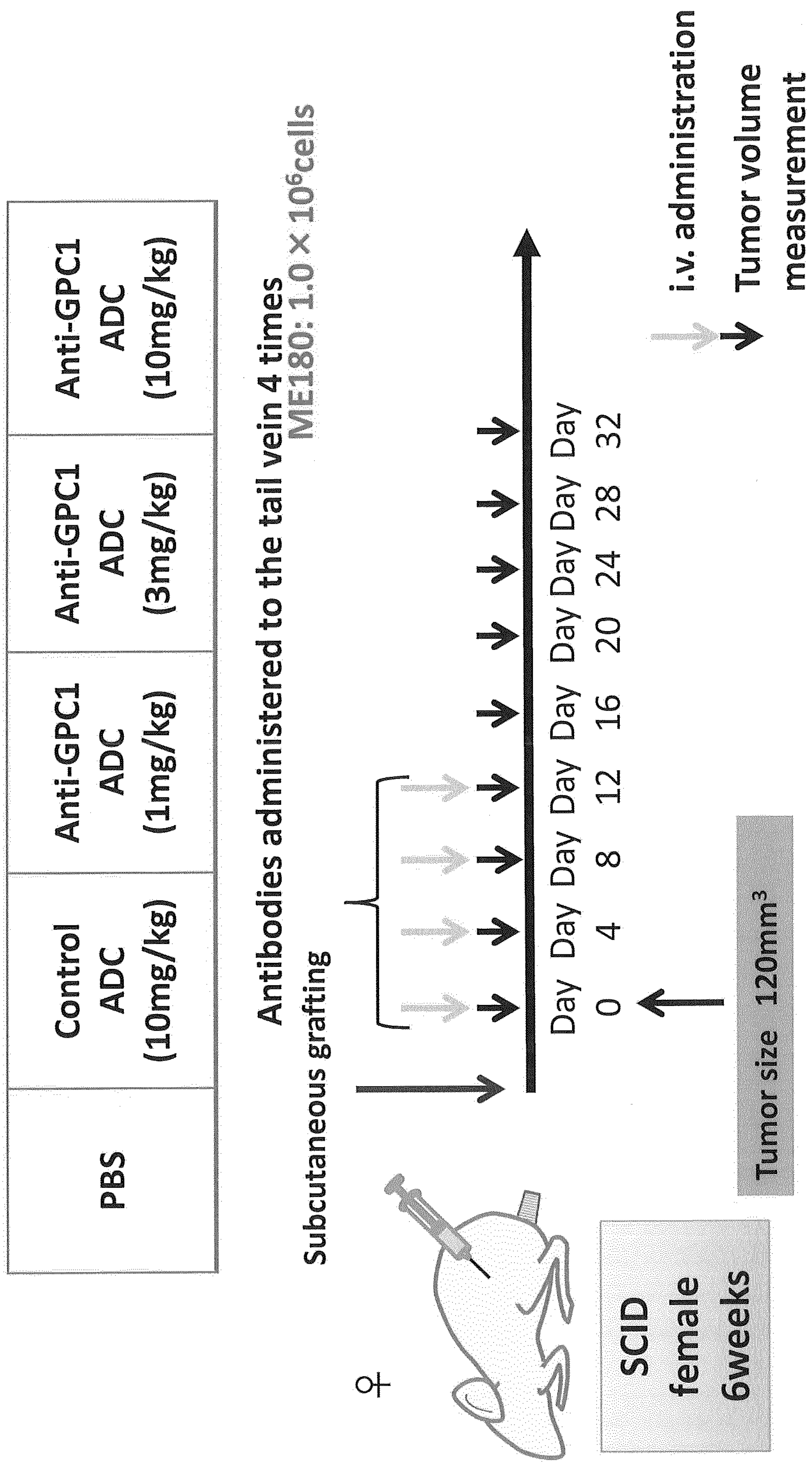
PBS
Control ADC (10mg/kg)
Anti-GPC1 ADC (1mg/kg)
Anti-GPC1 ADC (3mg/kg)
Anti-GPC1 ADC (10mg/kg)
♀
SCID female 6weeks
Subcutaneous grafting
Antibodies administered to the tail vein 4 times
ME180: 1.0×10⁶cells
Day 0
Day 4
Day 8
Day 12
Day 16
Day 20
Day 24
Day 28
Day 32
Tumor size 120mm³
i.v. administration
Tumor volume measurement

FIG.29

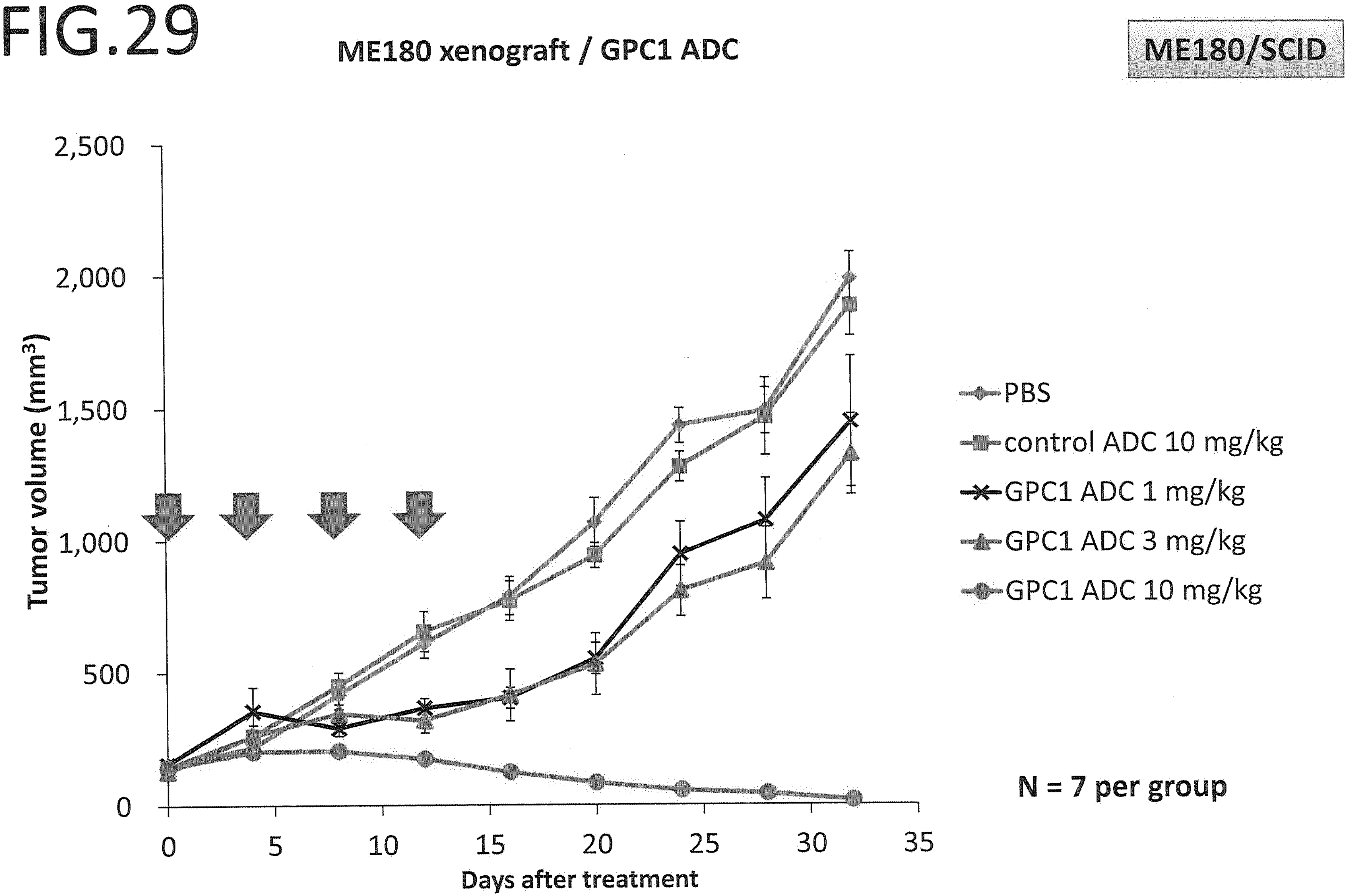
ME180 xenograft / GPC1 ADC
ME180/SCID
2,500
2,000
1,500
1,000
500
0
Tumor volume (mm3)
0
5
10
15
20
25
30
35
Days after treatment
PBS
control ADC 10 mg/kg
GPC1 ADC 1 mg/kg
GPC1 ADC 3 mg/kg
GPC1 ADC 10 mg/kg
N = 7 per group

FIG.30

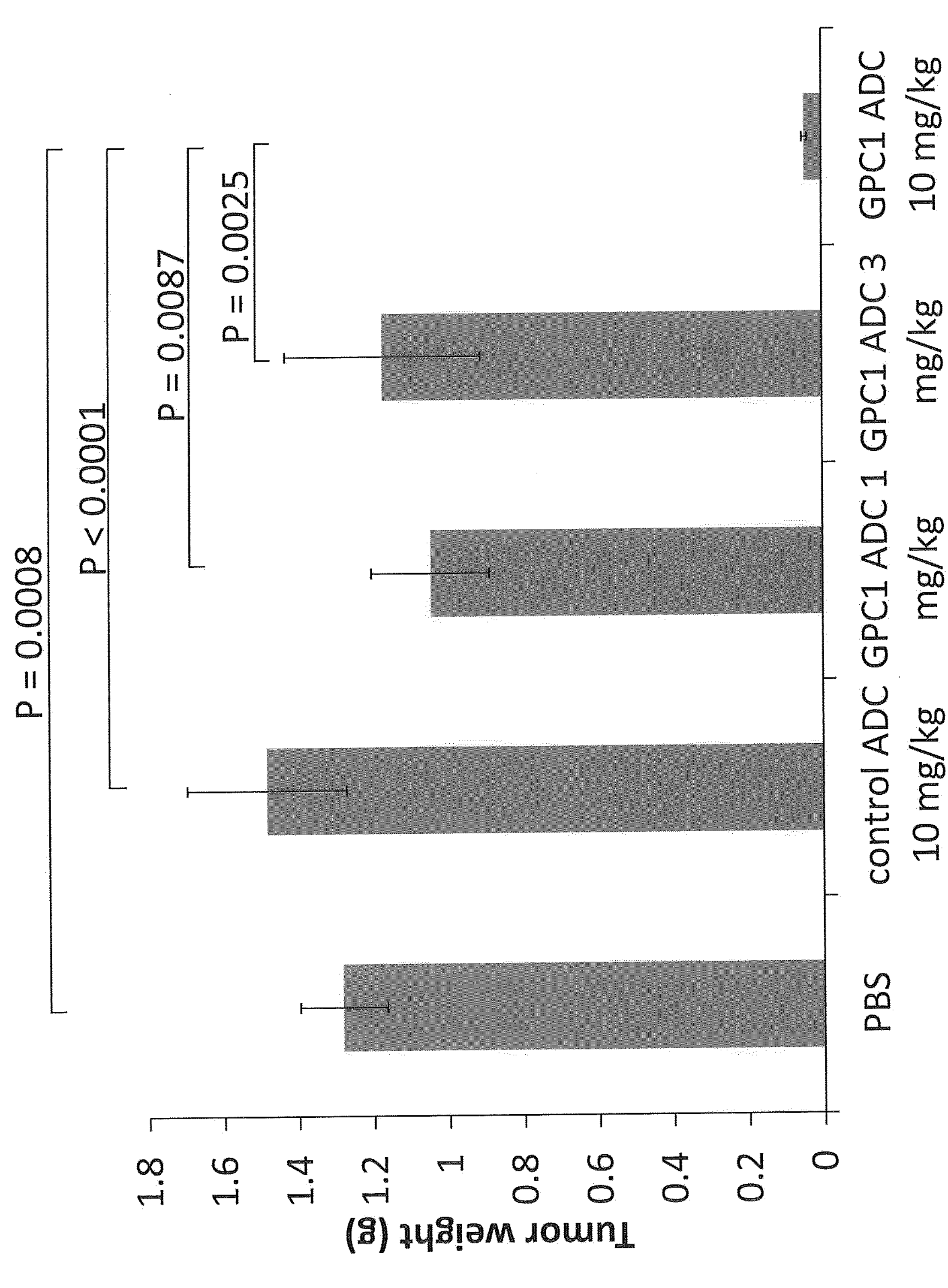
ME180/SCID
Tumor weight (g)
1.8
1.6
1.4
1.2
1
0.8
0.6
0.4
0.2
0
PBS
control ADC 10 mg/kg
GPC1 ADC 1 mg/kg
GPC1 ADC 3 mg/kg
GPC1 ADC 10 mg/kg
P = 0.0008
P < 0.0001
P = 0.0087
P = 0.0025

FIG.31

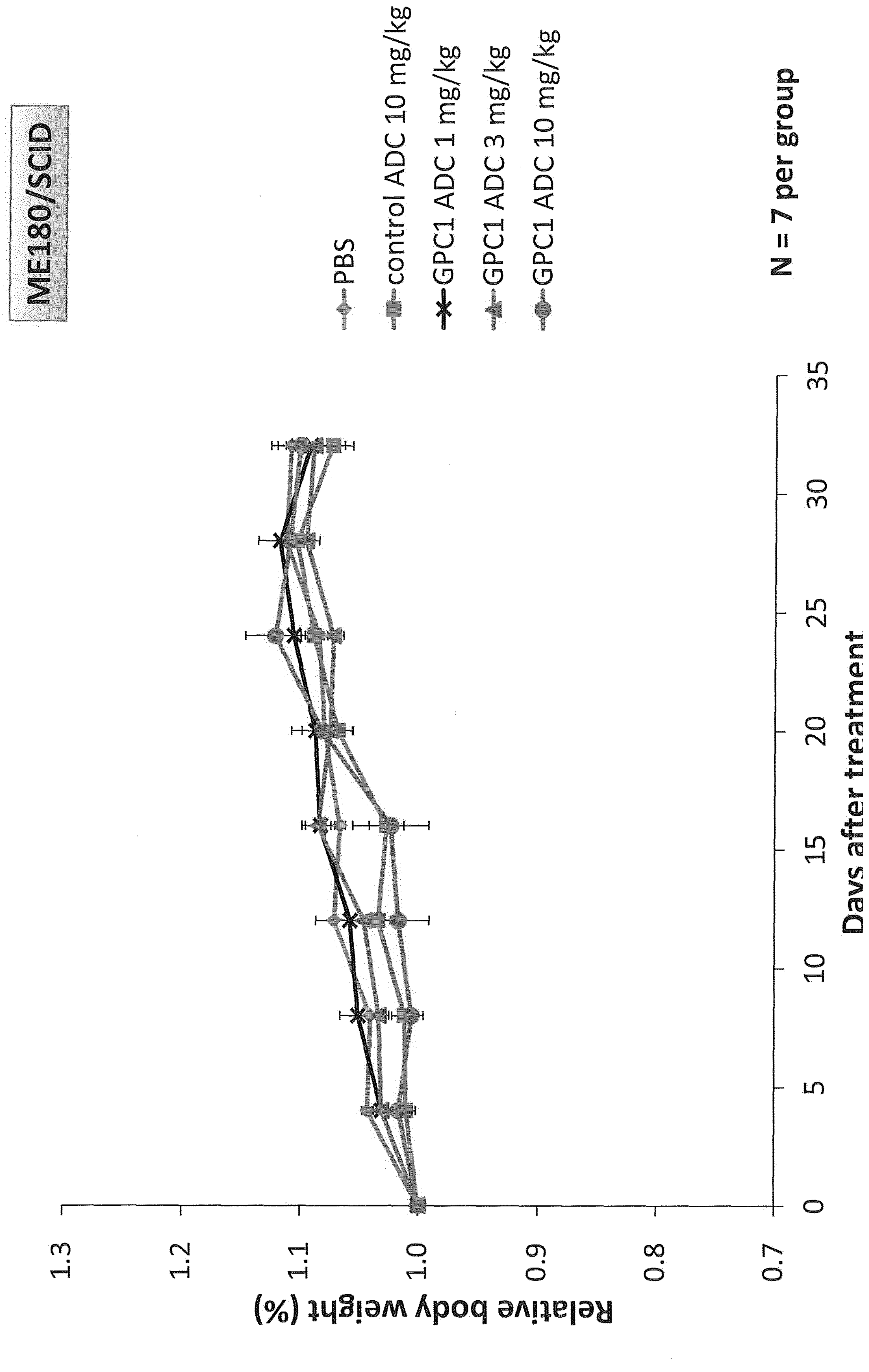
ME180/SCID
PBS
control ADC 10 mg/kg
GPC1 ADC 1 mg/kg
GPC1 ADC 3 mg/kg
GPC1 ADC 10 mg/kg
N = 7 per group
Relative body weight (%)
1.3
1.2
1.1
1.0
0.9
0.8
0.7
Days after treatment
0
5
10
15
20
25
30
35

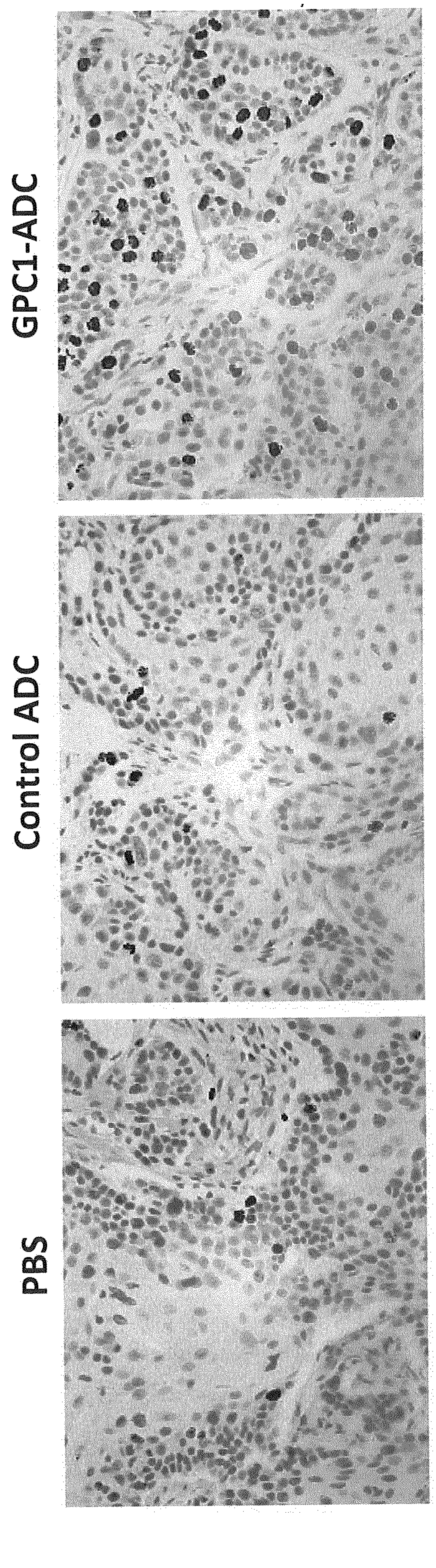


FIG.32

# FIG.33

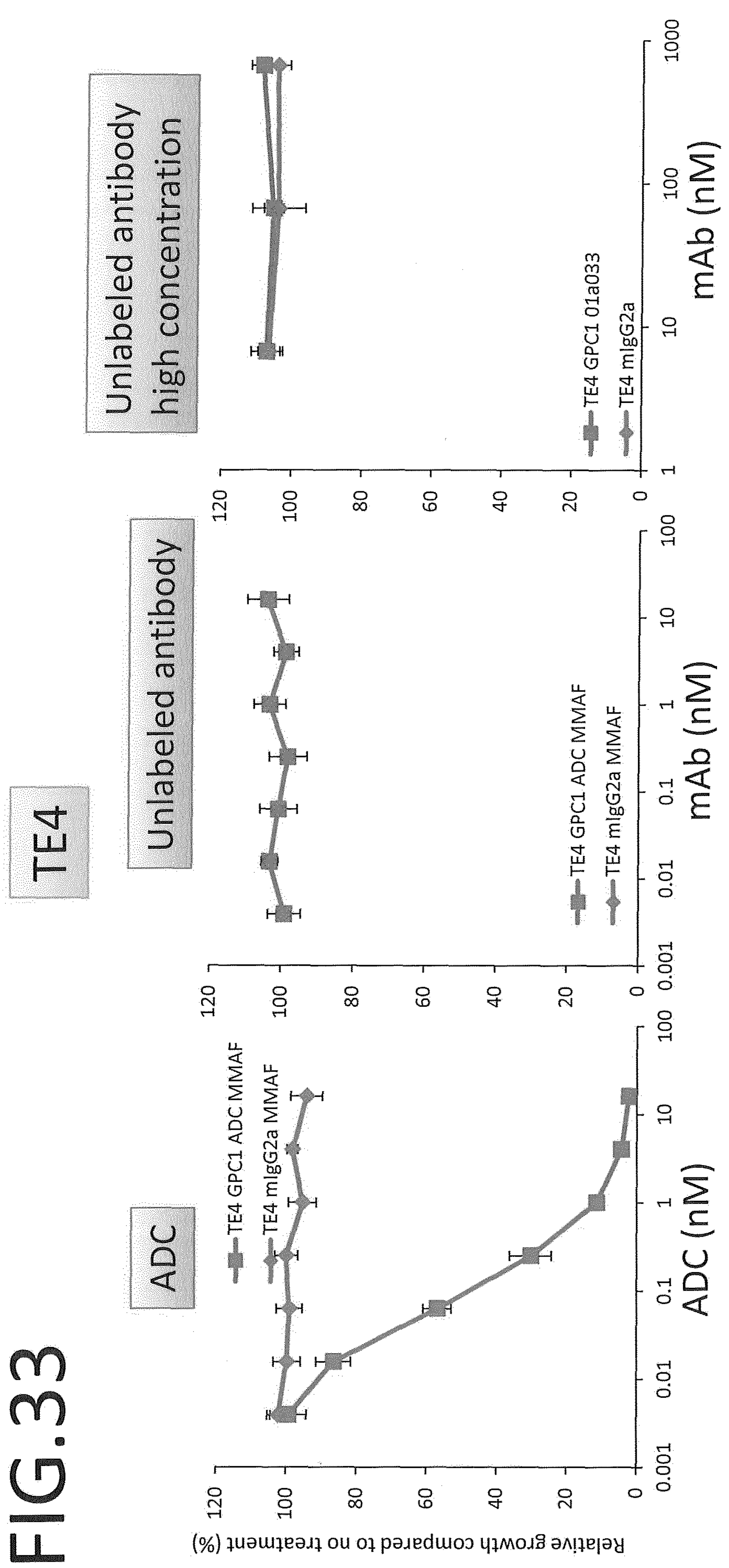
TE4
ADC
TE4 GPC1 ADC MMAF
TE4 mIgG2a MMAF
Relative growth compared to no treatment (%)
120
100
80
60
40
20
0
0.001
0.01
0.1
1
10
100
ADC (nM)
Unlabeled antibody
TE4 GPC1 ADC MMAF
TE4 mIgG2a MMAF
mAb (nM)
Unlabeled antibody
high concentration
TE4 GPC1 01a033
TE4 mIgG2a
1000
mAb (nM)

FIG.34

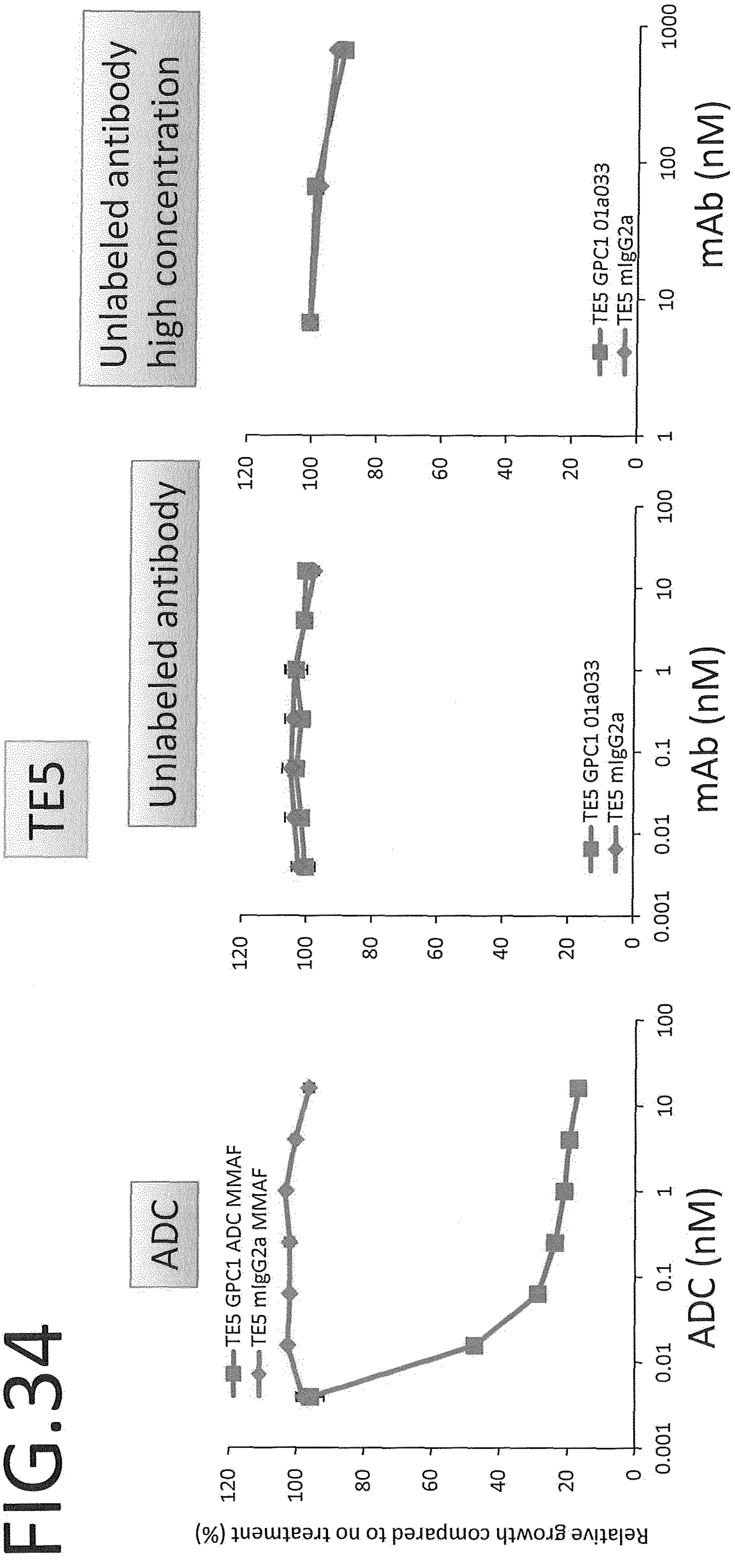
TE5
ADC
TE5 GPC1 ADC MMAF
TE5 mIgG2a MMAF
Relative growth compared to no treatment (%)
ADC (nM)
Unlabeled antibody
TE5 GPC1 01a033
TE5 mIgG2a
mAb (nM)
Unlabeled antibody high concentration
TE5 GPC1 01a033
TE5 mIgG2a
mAb (nM)
120
100
80
60
40
20
0
0.001
0.01
0.1
1
10
100
1000

FIG.35

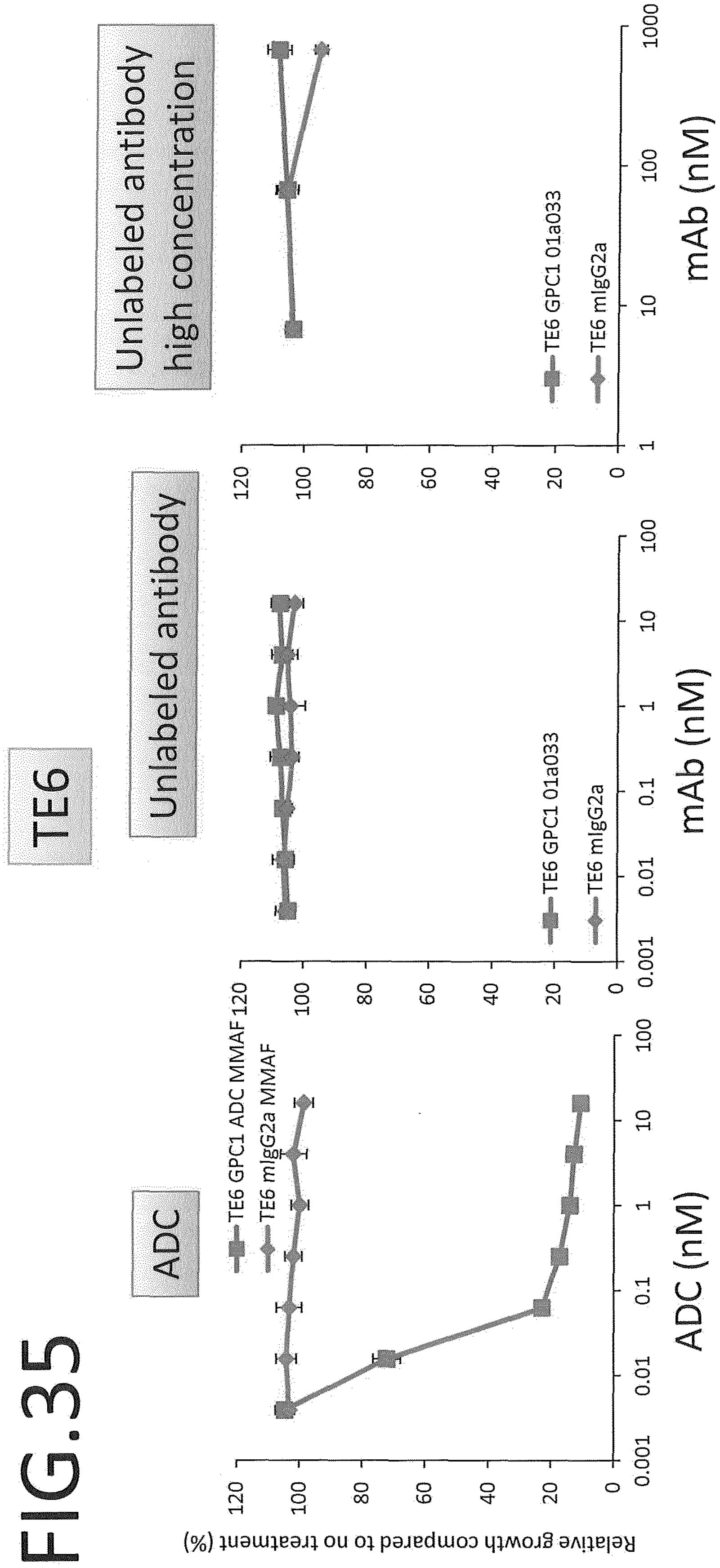
TE6
ADC
Unlabeled antibody
Unlabeled antibody high concentration
TE6 GPC1 ADC MMAF
TE6 mIgG2a MMAF
TE6 GPC1 01a033
TE6 mIgG2a
Relative growth compared to no treatment (%)
ADC (nM)
mAb (nM)
mAb (nM)
0
20
40
60
80
100
120
0.001
0.01
0.1
1
10
100
1000

FIG.36

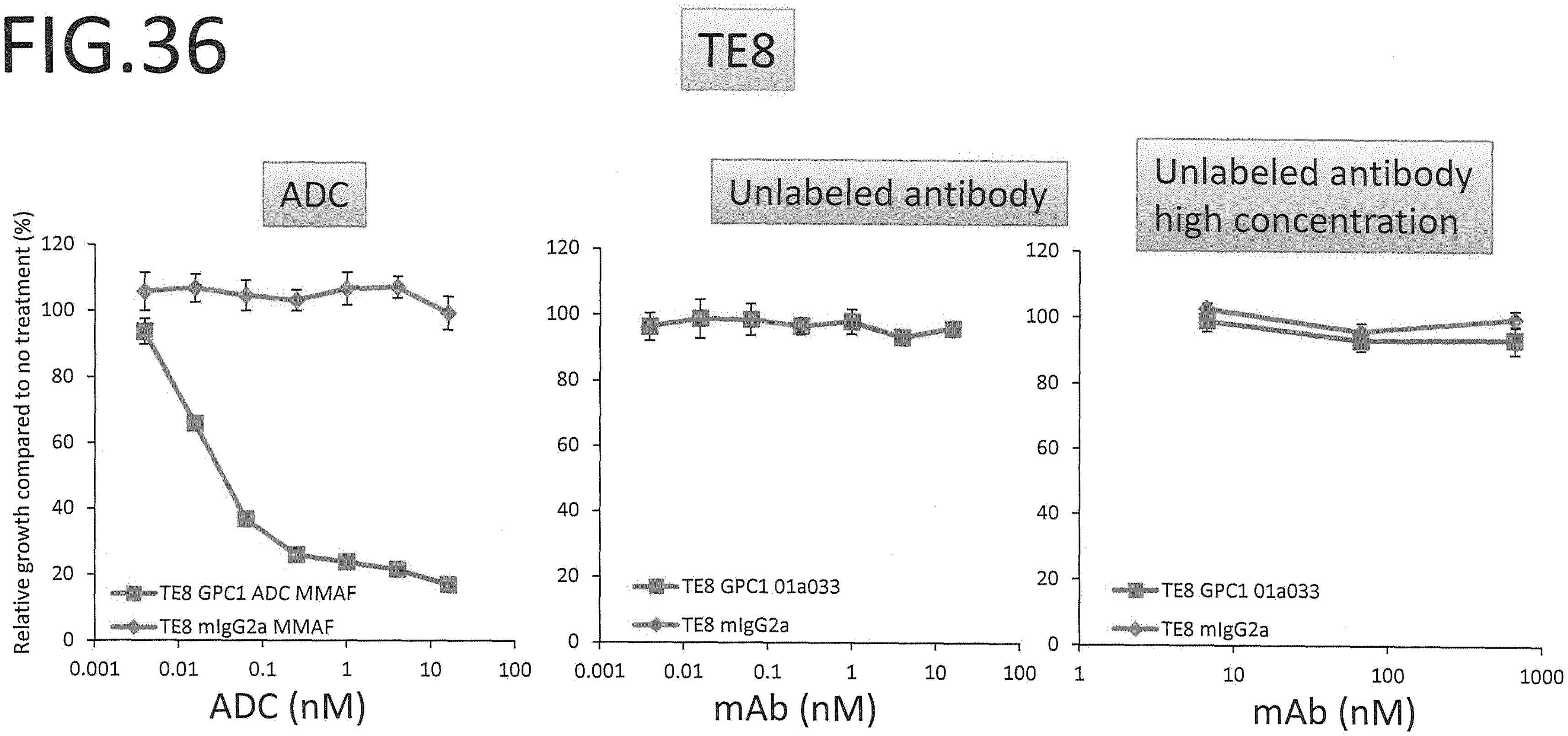
TE8
ADC
Unlabeled antibody
Unlabeled antibody high concentration
Relative growth compared to no treatment (%)
120
100
80
60
40
20
0
0.001
0.01
0.1
1
10
100
1000
TE8 GPC1 ADC MMAF
TE8 mIgG2a MMAF
TE8 GPC1 01a033
TE8 mIgG2a
ADC (nM)
mAb (nM)

FIG.37

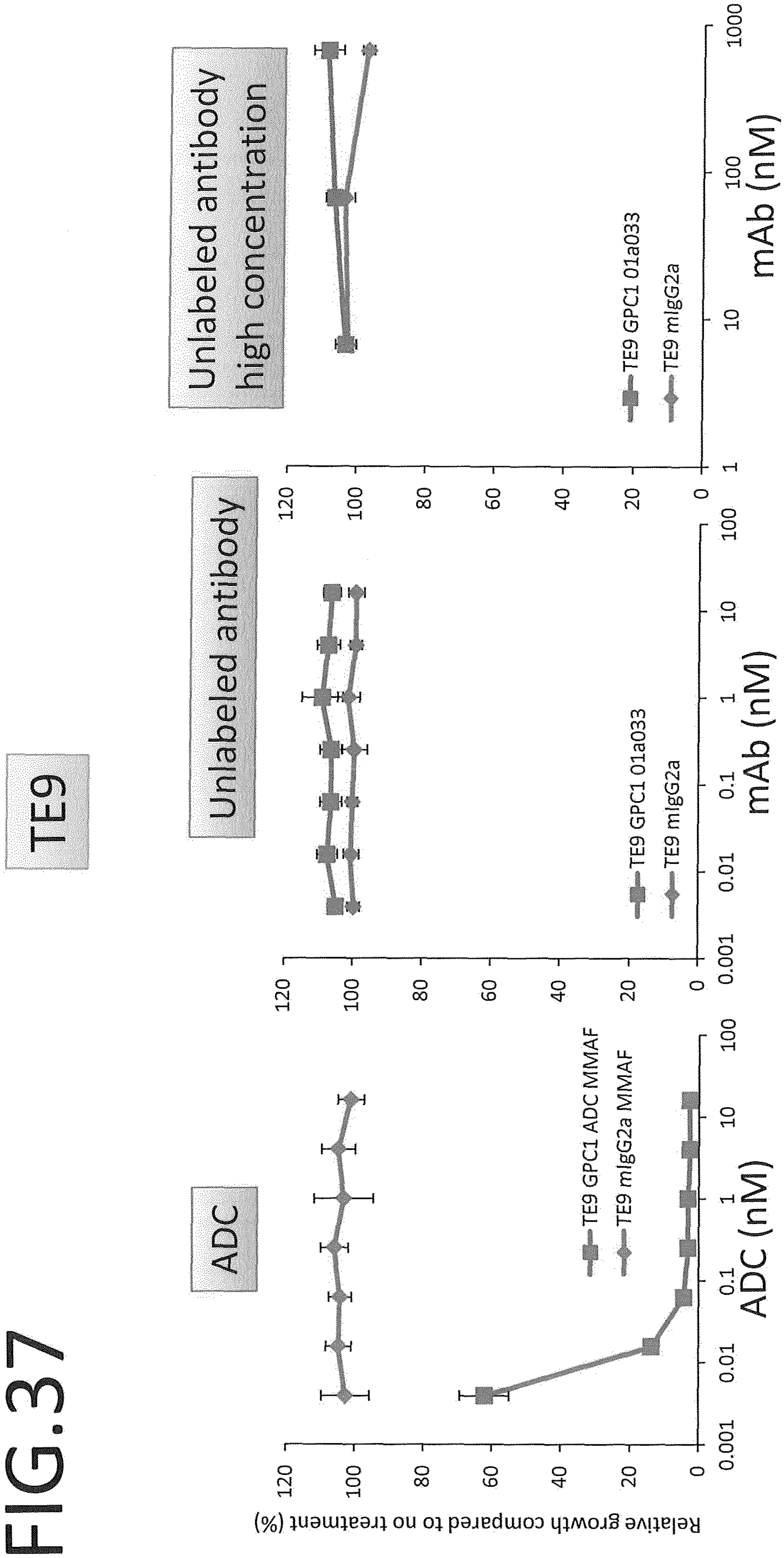
TE9
ADC
Unlabeled antibody
Unlabeled antibody high concentration
Relative growth compared to no treatment (%)
TE9 GPC1 ADC MMAF
TE9 mIgG2a MMAF
TE9 GPC1 01a033
TE9 mIgG2a
ADC (nM)
mAb (nM)
120
100
80
60
40
20
0
0.001
0.01
0.1
1
10
100
1000

FIG.38

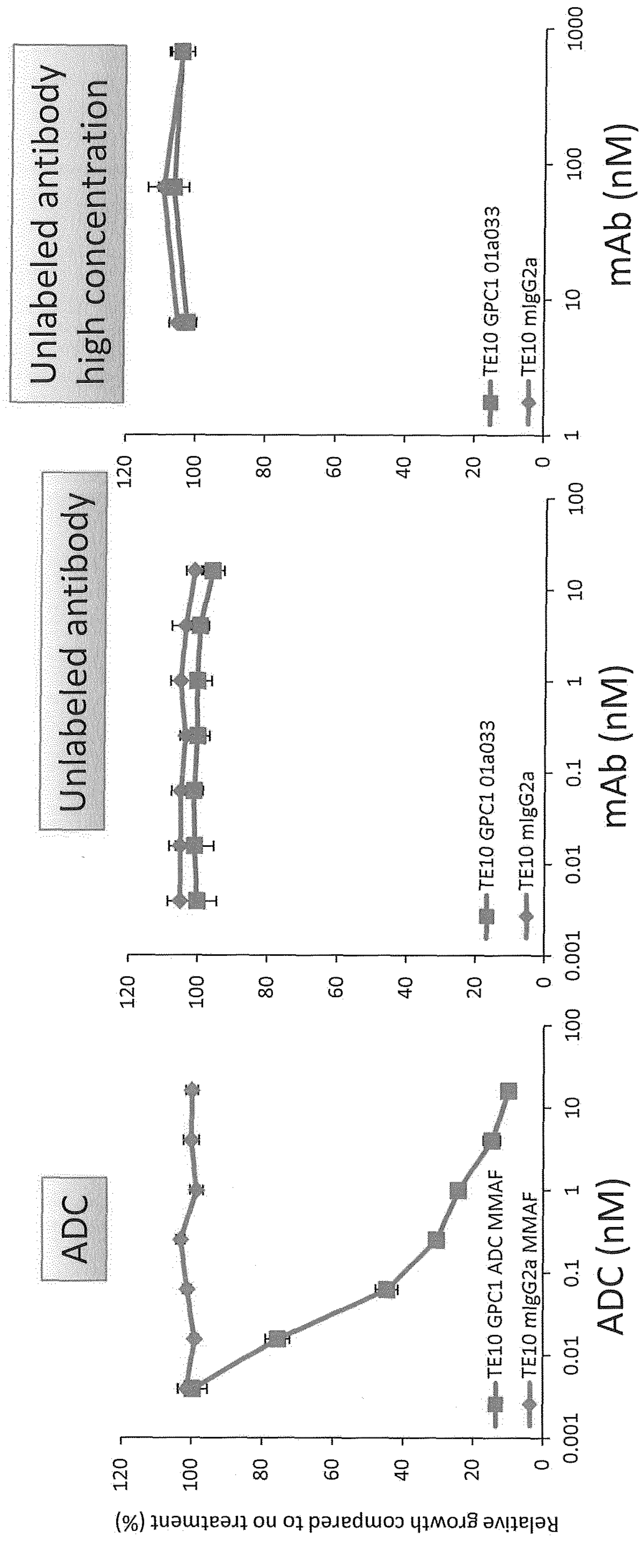
TE10
ADC
Unlabeled antibody
Unlabeled antibody high concentration
Relative growth compared to no treatment (%)
120
100
80
60
40
20
0
0.001
0.01
0.1
1
10
100
1000
ADC (nM)
mAb (nM)
TE10 GPC1 ADC MMAF
TE10 mIgG2a MMAF
TE10 GPC1 01a033
TE10 mIgG2a

FIG.39

TE11

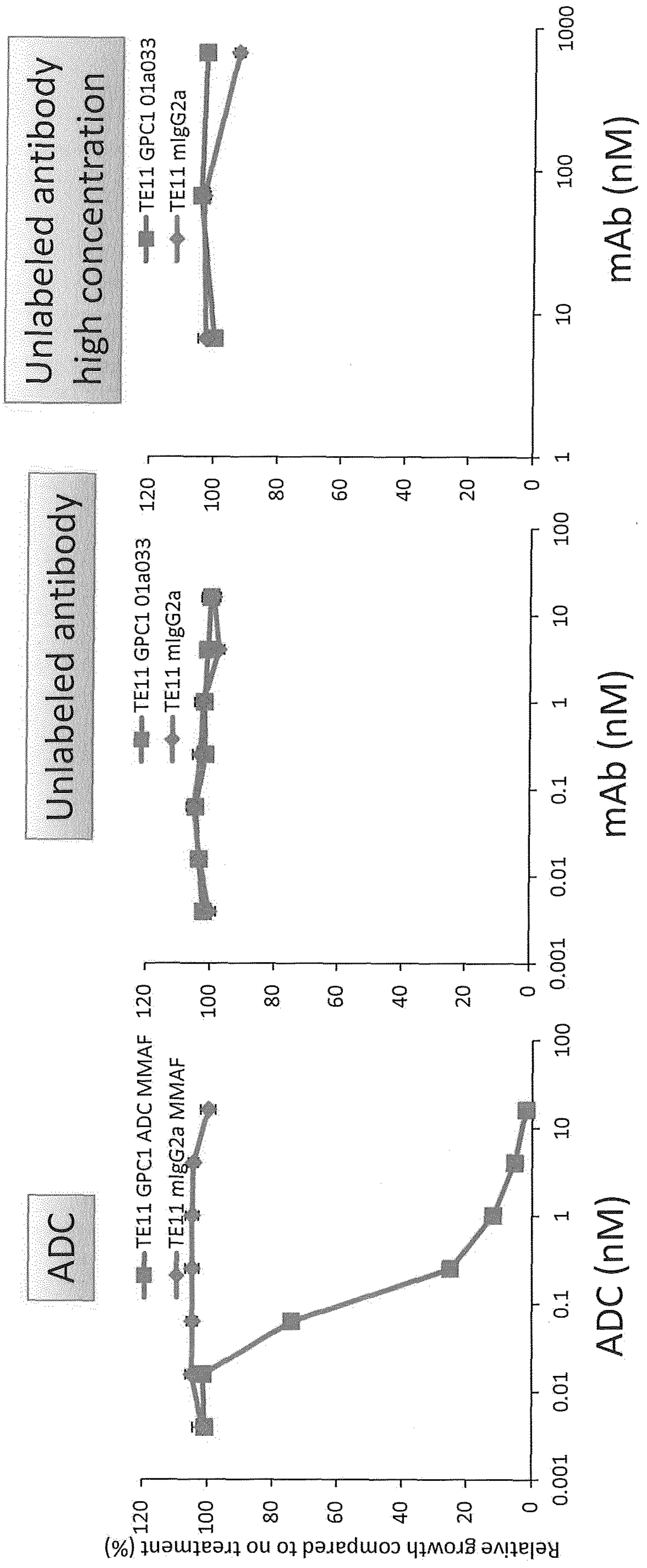
ADC
TE11 GPC1 ADC MMAF
TE11 mIgG2a MMAF
Relative growth compared to no treatment (%)
120
100
80
60
40
20
0
0.001
0.01
0.1
1
10
100
ADC (nM)
Unlabeled antibody
TE11 GPC1 01a033
TE11 mIgG2a
mAb (nM)
Unlabeled antibody high concentration
TE11 GPC1 01a033
TE11 mIgG2a
1000
mAb (nM)

FIG.40

TE14

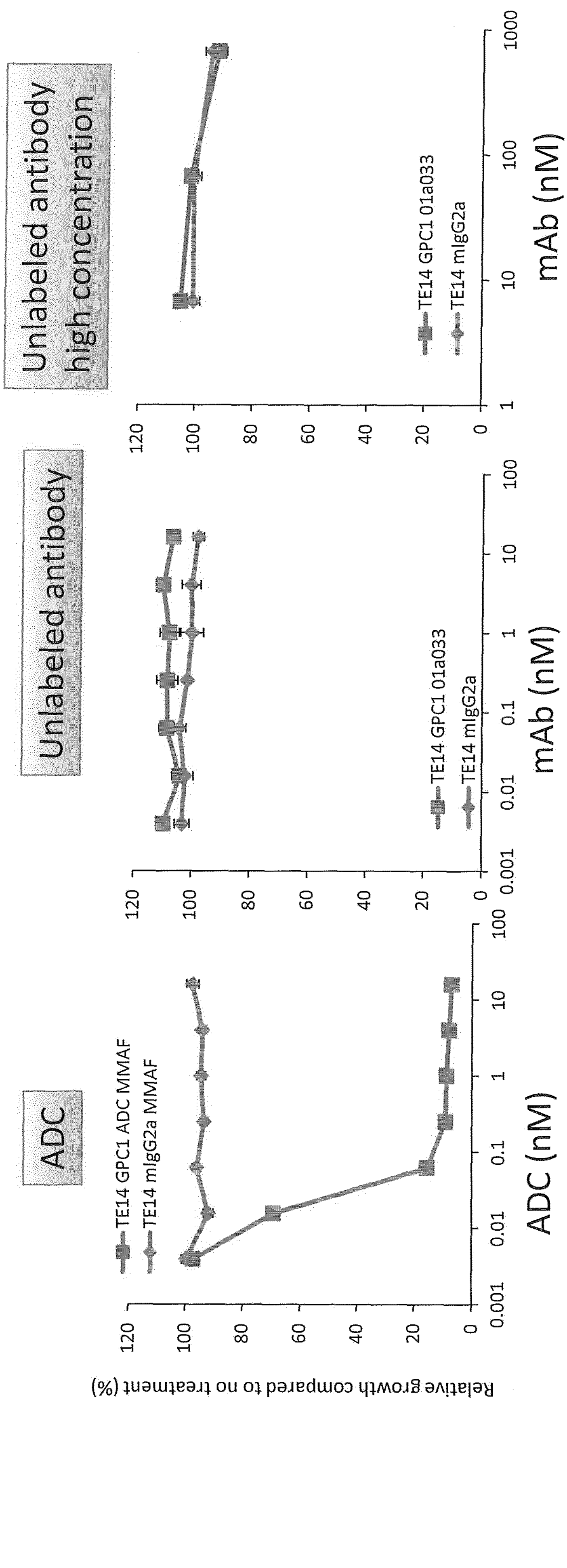

ADC
Unlabeled antibody
Unlabeled antibody high concentration
Relative growth compared to no treatment (%)
120
100
80
60
40
20
0
0.001
0.01
0.1
1
10
100
1000
TE14 GPC1 ADC MMAF
TE14 mIgG2a MMAF
TE14 GPC1 01a033
TE14 mIgG2a
ADC (nM)
mAb (nM)

FIG.41

TE15

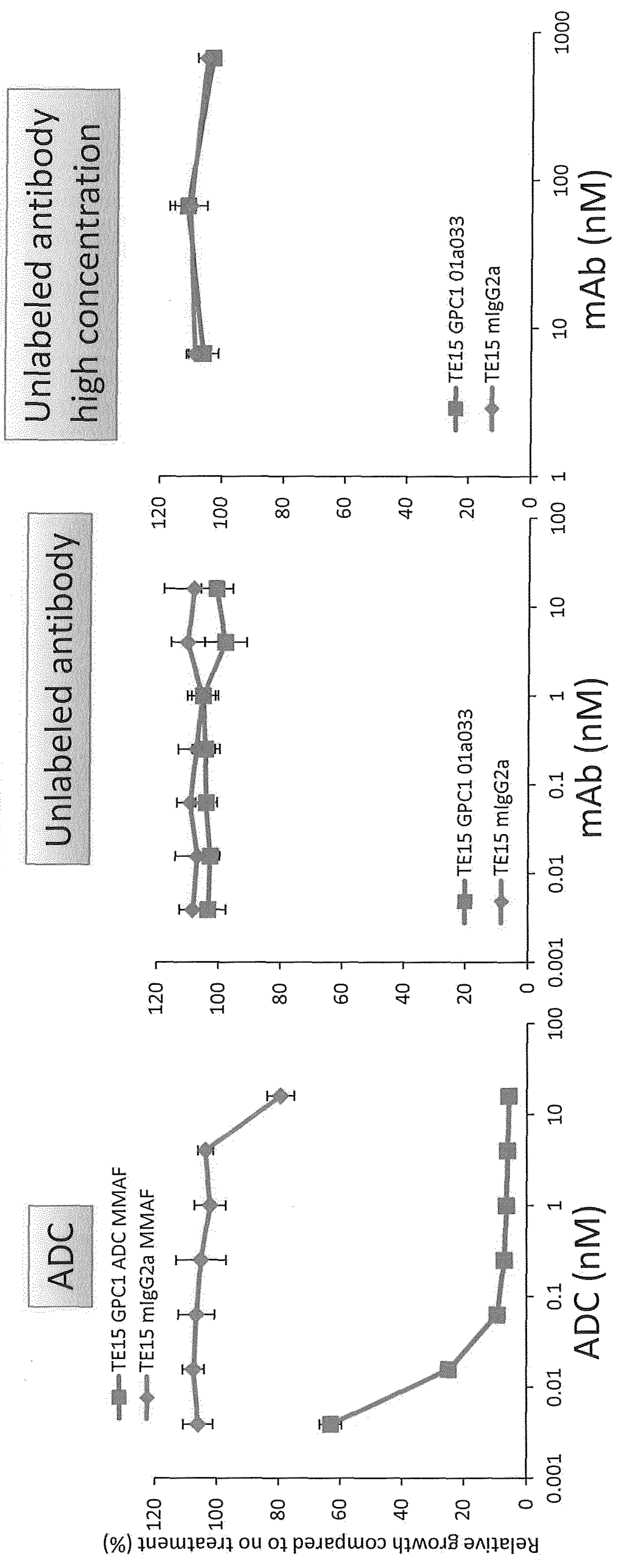
ADC
TE15 GPC1 ADC MMAF
TE15 mIgG2a MMAF
Relative growth compared to no treatment (%)
120
100
80
60
40
20
0
0.001
0.01
0.1
1
10
100
ADC (nM)
Unlabeled antibody
TE15 GPC1 01a033
TE15 mIgG2a
mAb (nM)
Unlabeled antibody high concentration
TE15 GPC1 01a033
TE15 mIgG2a
1000
mAb (nM)

FIG.42

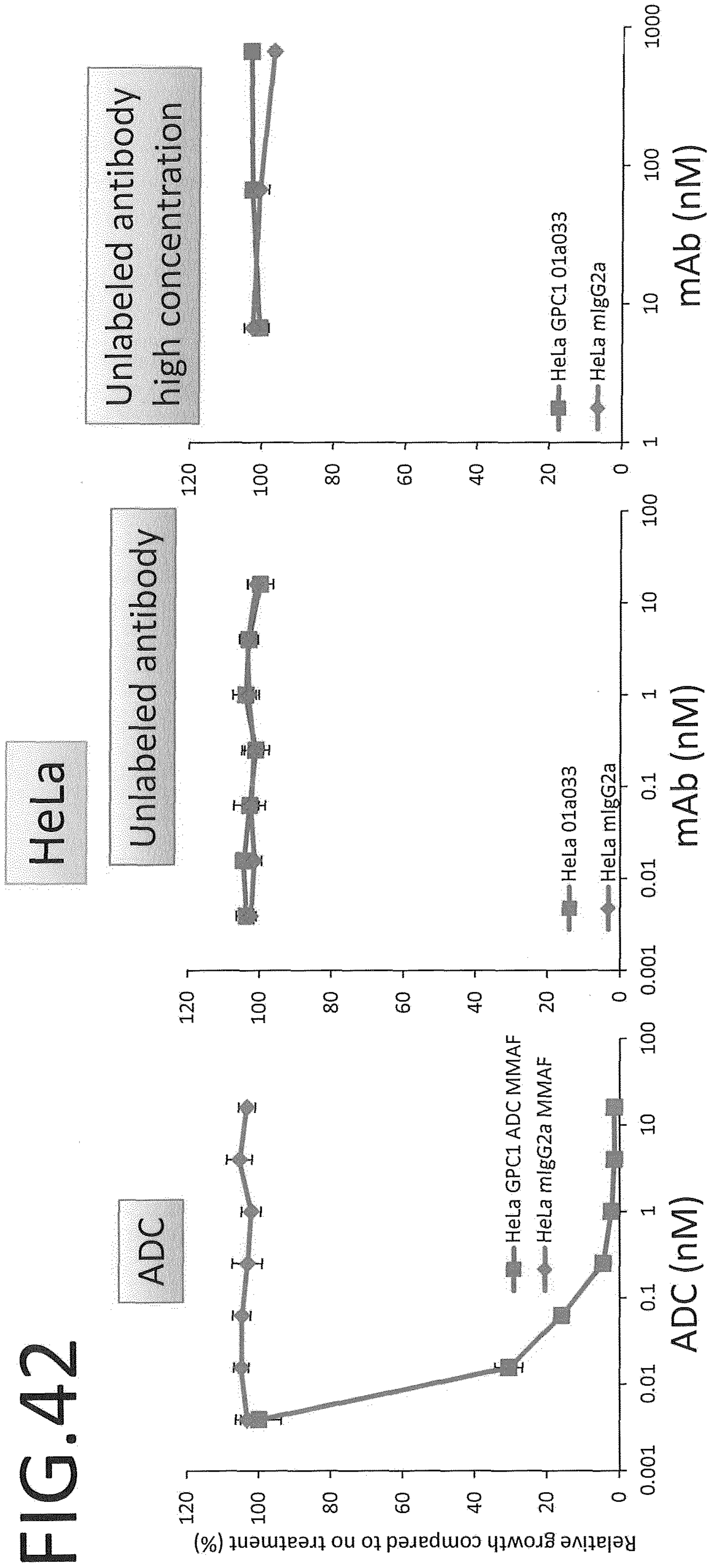
HeLa
ADC
HeLa GPC1 ADC MMAF
HeLa mIgG2a MMAF
Relative growth compared to no treatment (%)
ADC (nM)
Unlabeled antibody
HeLa 01a033
HeLa mIgG2a
mAb (nM)
Unlabeled antibody
high concentration
HeLa GPC1 01a033
HeLa mIgG2a
mAb (nM)

FIG.43

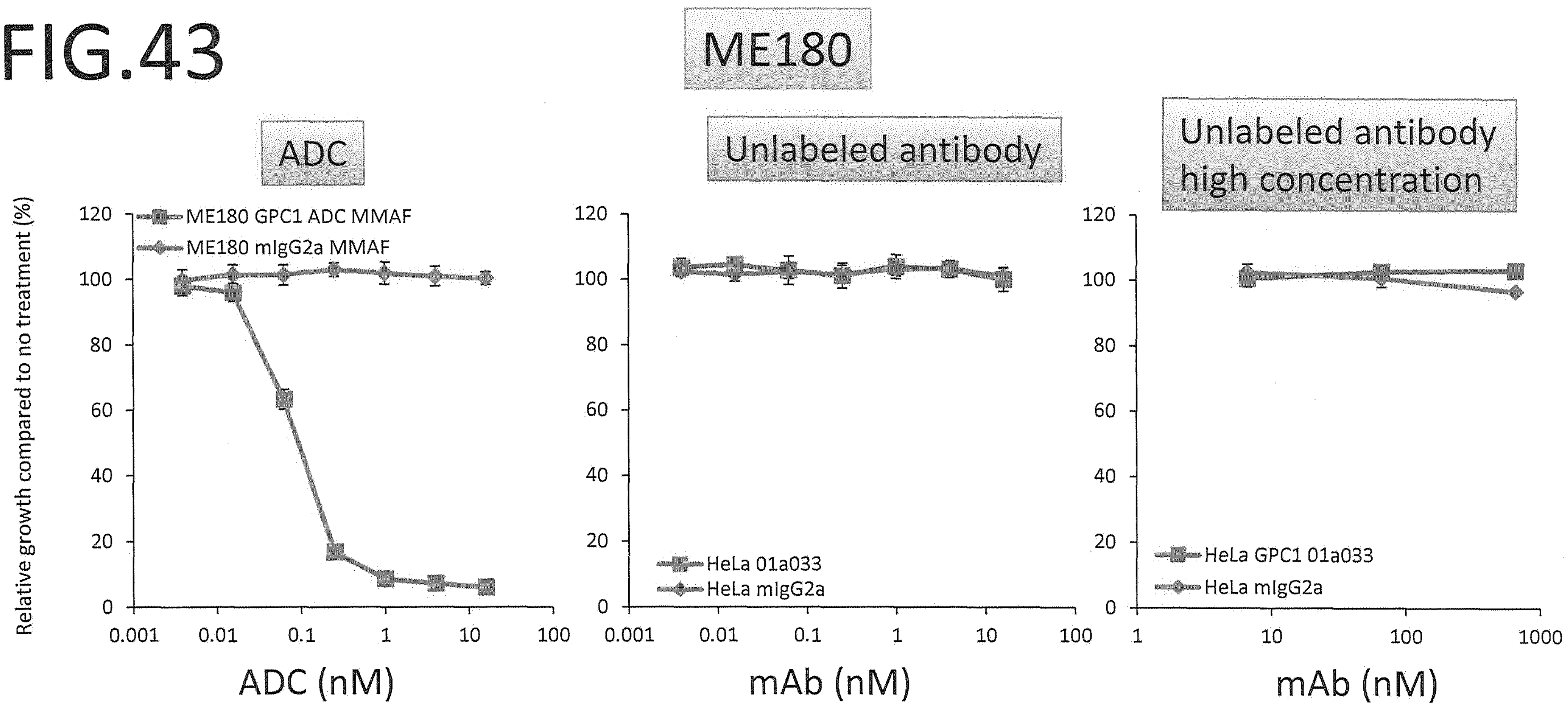
ME180
ADC
Unlabeled antibody
Unlabeled antibody high concentration
Relative growth compared to no treatment (%)
120
100
80
60
40
20
0
ME180 GPC1 ADC MMAF
ME180 mIgG2a MMAF
0.001
0.01
0.1
1
10
100
ADC (nM)
HeLa 01a033
HeLa mIgG2a
mAb (nM)
HeLa GPC1 01a033
HeLa mIgG2a
1
10
100
1000
mAb (nM)

FIG.44

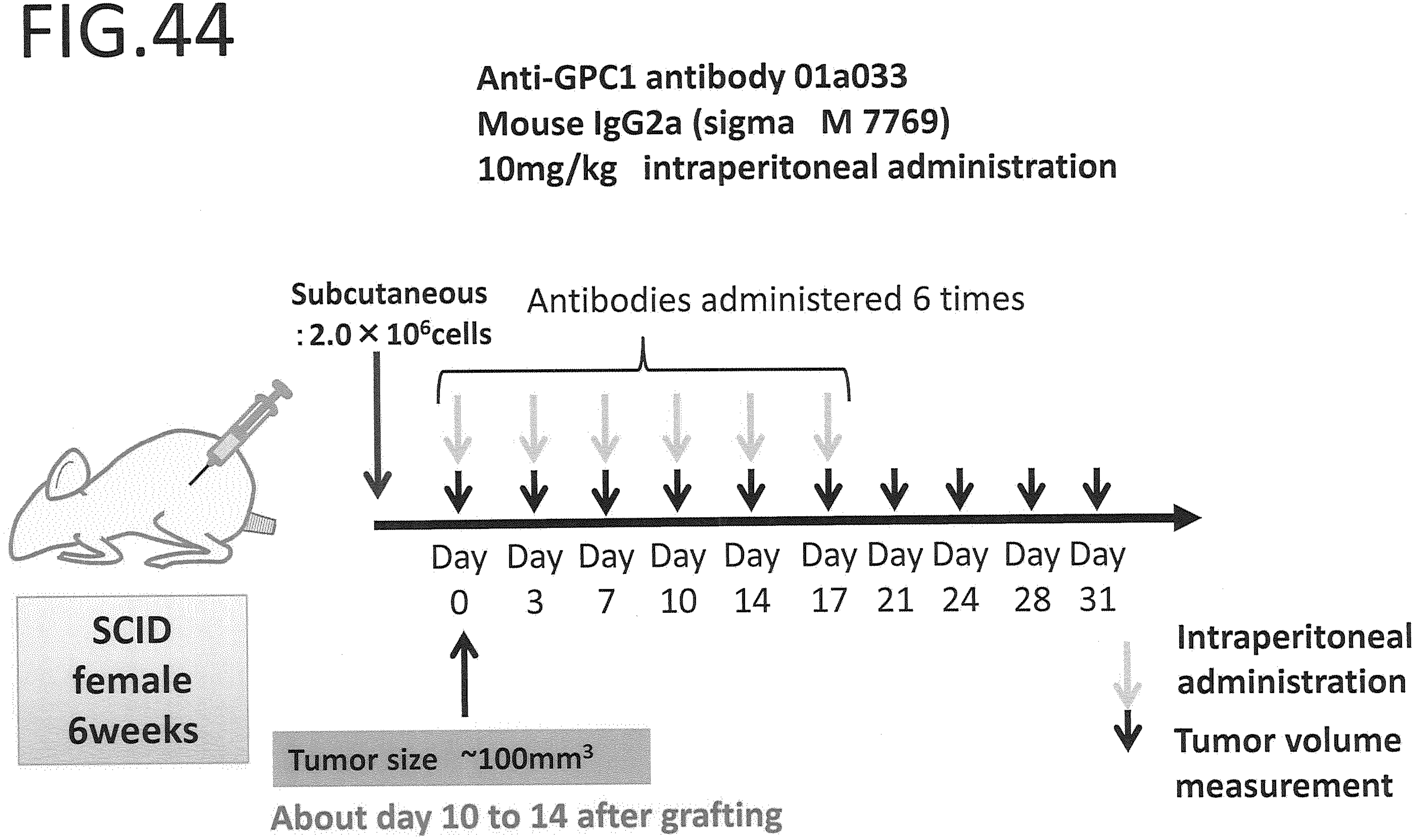
Anti-GPC1 antibody 01a033
Mouse IgG2a (sigma M 7769)
10mg/kg intraperitoneal administration
Subcutaneous
: 2.0 × 10⁶cells
Antibodies administered 6 times
Day 0
Day 3
Day 7
Day 10
Day 14
Day 17
Day 21
Day 24
Day 28
Day 31
SCID
female
6weeks
Tumor size ~100mm³
About day 10 to 14 after grafting
Intraperitoneal administration
Tumor volume measurement

FIG.45

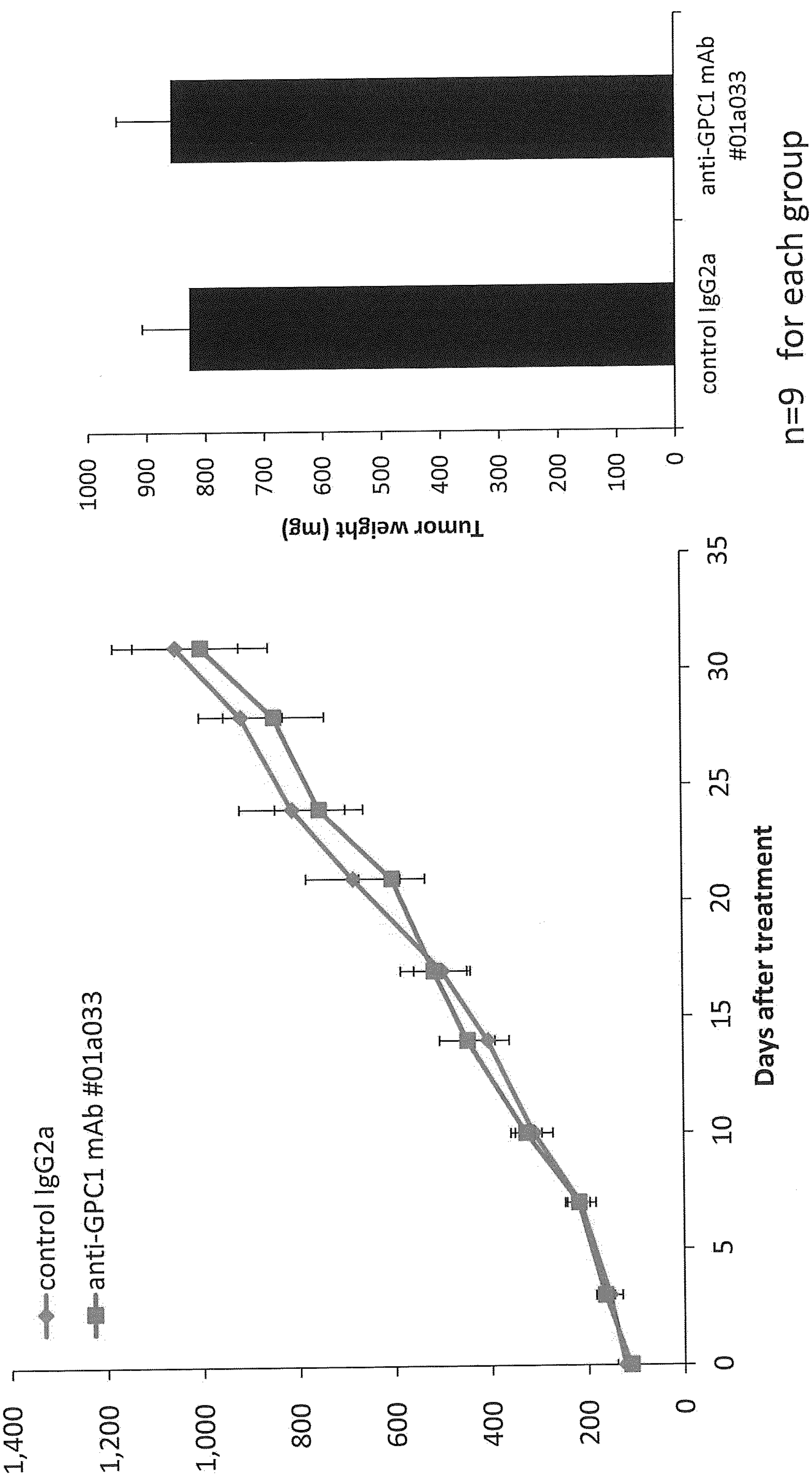

control IgG2a
anti-GPC1 mAb #01a033
Tumor volume (mm3)
1,400
1,200
1,000
800
600
400
200
0
0
5
10
15
20
25
30
35
Days after treatment
Tumor weight (mg)
1000
900
800
700
600
500
400
300
200
100
0
control IgG2a
anti-GPC1 mAb #01a033
n=9 for each group

# FIG.46

## Control IgG vs Anti-GPC-1 mAb (♂)

| | Control IgG (n=4) | Anti-GPC1 Ab (n=4) | p value |
|---|---|---|---|
| WBC($10^9$/l) | 2.39±1.83 | 4.34±1.27 | 0.131 |
| Ly(%) | 96.6±0.49 | 96.2±1.65 | 0.639 |
| Mo(%) | 1.85±1.23 | 1.55±0.58 | 0.675 |
| Gr(%) | 1.58±0.74 | 2.28±1.51 | 0.438 |
| RBC($10^{12}$/l) | 11.56±0.313 | 11.70±0.603 | 0.689 |
| Hb | 17.63±0.19 | 18.15±0.44 | 0.0692 |
| Hct | 51.85±1.28 | 52.9±3.29 | 0.571 |
| Plt($10^9$/l) | 18.95±0.66 | 19.08±0.67 | 0.798 |

FIG.47

Control IgG vs Anti-GPC-1 mAb(♀)

| | Control IgG (n=3) | Anti-GPC1 Ab (n=3) | p value |
|---|---|---|---|
| WBC($10^9$/l) | 4.29±2.03 | 3.79±1.06 | 0.681 |
| Ly(%) | 95.7±1.47 | 96.1±1.05 | 0.731 |
| Mo(%) | 1.28±0.72 | 1.93±0.29 | 0.146 |
| Gr(%) | 3.05±1.48 | 2.08±1.30 | 0.360 |
| RBC($10^{12}$/l) | 11.28±0.576 | 11.64±0.293 | 0.290 |
| Hb | 17.1±0.82 | 17.9±0.22 | 0.100 |
| Hct | 50.4±2.33 | 52.1±1.13 | 0.248 |
| Plt($10^9$/l) | 18.03±0.15 | 18.2±0.42 | 0.466 |

FIG.48

Control IgG vs Anti-GPC-1 mAb (♂)

| | Control IgG (n=4) | Anti-GPC1 Ab(n=4) | p value |
|---|---|---|---|
| Alb (g/dl) | 4.4±0.42 | 4.8±1.34 | 0.590 |
| ALP (U/l) | 98±12.7 | 104.8±28.3 | 0.679 |
| ALT (U/l) | 25±9.42 | 19.8±5.80 | 0.379 |
| Amy (U/l) | 921±30.4 | 918±54.4 | 0.924 |
| T-Bil (mg/dl) | 0.2±0 | 0.225±0.05 | 0.356 |
| BUN (mg/dl) | 21±2.65 | 19.8±2.22 | 0.525 |
| Ca (mg/dl) | 10.28±0.300 | 10±0.141 | 0.147 |
| P (mg/dl) | 8.68±0.86 | 9.38±0.33 | 0.180 |
| Cr (mg/dl) | 0.225±0.05 | 0.275±0.096 | 0.390 |
| Glu (mg/dl) | 153.75±12.82 | 152.25±32.81 | 0.9357 |
| Na (mmol/l) | 149.75±2.75 | 151.75±2.5 | 0.323 |
| K (mmol/l) | 8.45±0.1 | 8±1.31 | 0.510 |
| TP (g/dl) | 6.38±0.41 | 6.28±0.435 | 0.750 |
| Glob (g/dl) | 2.1±0.082 | 1.98±0.15 | 0.194 |

FIG.49

Control IgG vs Anti-GPC-1 mAb (♀)

| | Control IgG (n=3) | Anti-GPC1 Ab(n=3) | p value |
|---|---|---|---|
| Alb (g/dl) | 4.2±0.84 | 4.6±0.1 | 0.424 |
| ALP (U/l) | 81.3±27.8 | 85±27.9 | 0.870 |
| ALT (U/l) | 23.3±4.51 | 27.8±2.87 | 0.171 |
| Amy (U/l) | 787.7±78.5 | 806.5±44.4 | 0.700 |
| T-Bil (mg/dl) | 0.2±0 | 0.2±0.08 | 1 |
| BUN (mg/dl) | 20.3±2.08 | 17.5±1.29 | 0.0749 |
| Ca (mg/dl) | 9.8±0.361 | 9.9±0.33 | 0.653 |
| P (mg/dl) | 0.886±0.757 | 8.4±0 | 0.886 |
| Cr (mg/dl) | 0.267±0.115 | 0.3±0.082 | 0.670 |
| Glu (mg/dl) | 115±4.36 | 149.75±41.83 | 0.221 |
| Na (mmol/l) | 147.3±2.51 | 145.5±4.51 | 0.559 |
| K (mmol/l) | 8.2±0.283 | 8.43±2.39 | 0.904 |
| TP (g/dl) | 5.97±0.493 | 6.3±0.244 | 0.286 |
| Glob (g/dl) | 1.77±0.289 | 1.6±0.1 | 0.398 |

# Fig.50

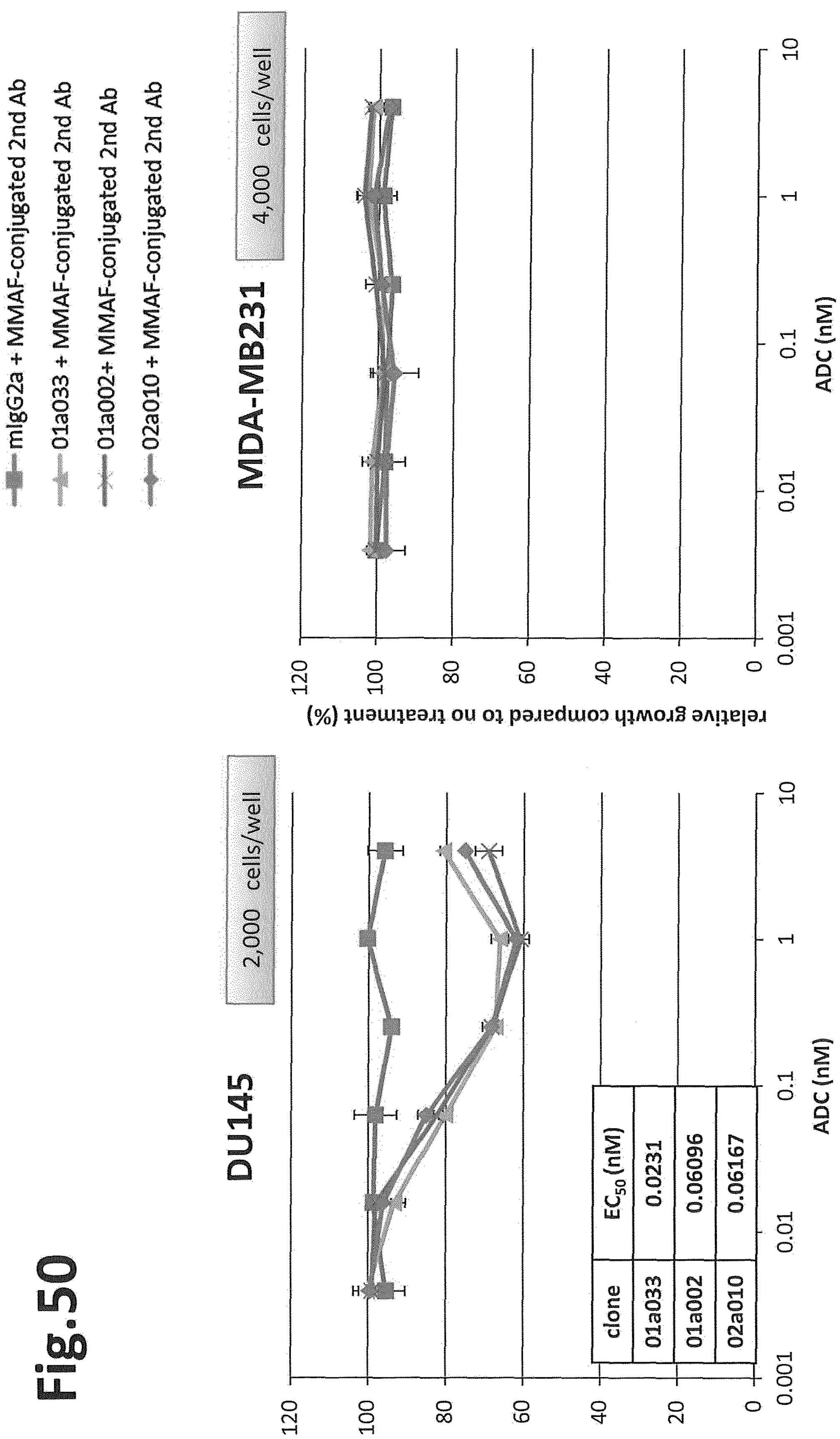


| clone | $EC_{50}$ (nM) |
|---|---|
| 01a033 | 0.0231 |
| 01a002 | 0.06096 |
| 02a010 | 0.06167 |

Fig.51

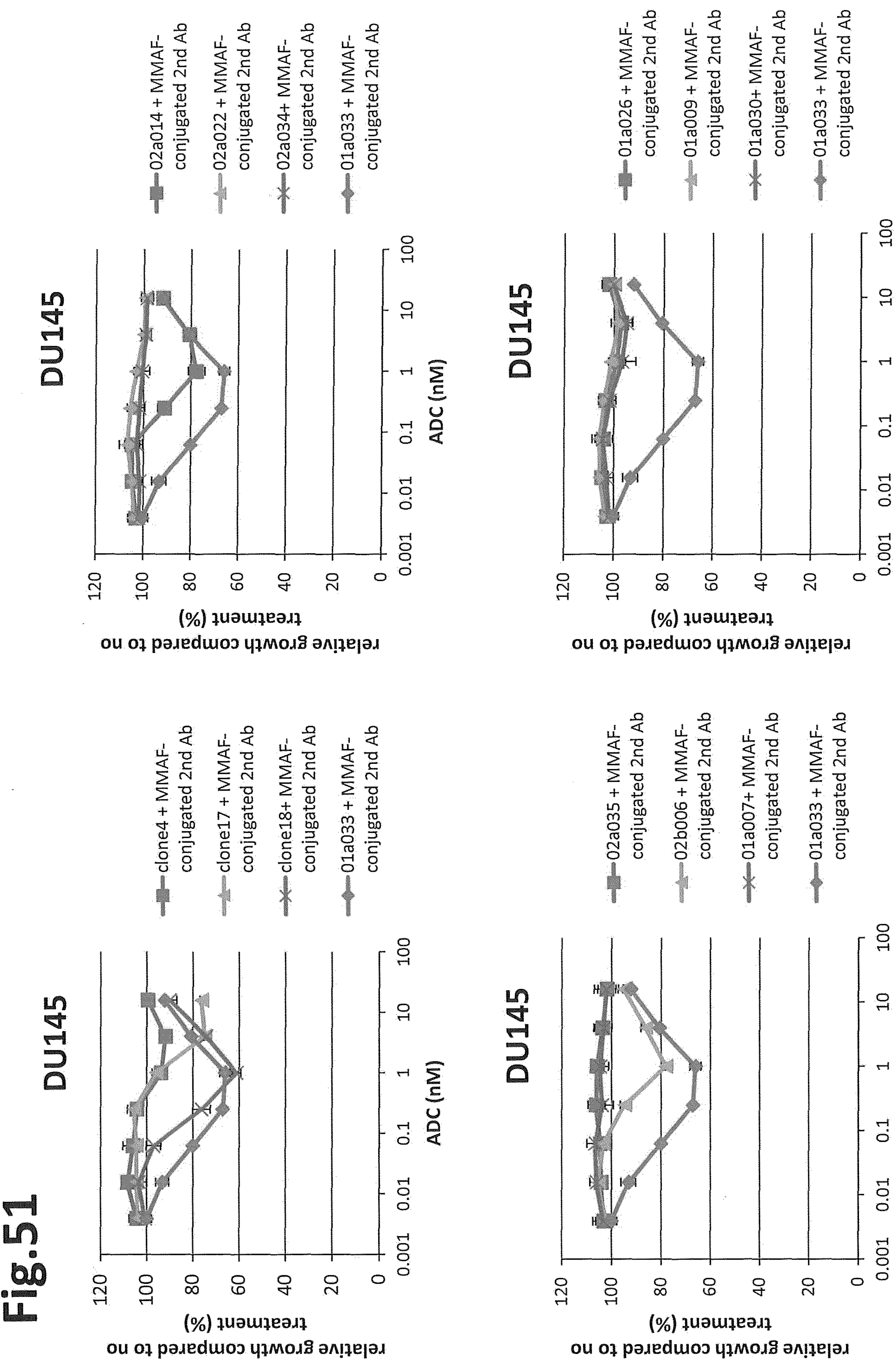
DU145
relative growth compared to no treatment (%)
ADC (nM)
clone4 + MMAF-conjugated 2nd Ab
clone17 + MMAF-conjugated 2nd Ab
clone18+ MMAF-conjugated 2nd Ab
01a033 + MMAF-conjugated 2nd Ab
DU145
relative growth compared to no treatment (%)
ADC (nM)
02a014 + MMAF-conjugated 2nd Ab
02a022 + MMAF-conjugated 2nd Ab
02a034+ MMAF-conjugated 2nd Ab
01a033 + MMAF-conjugated 2nd Ab
DU145
relative growth compared to no treatment (%)
ADC (nM)
02a035 + MMAF-conjugated 2nd Ab
02b006 + MMAF-conjugated 2nd Ab
01a007+ MMAF-conjugated 2nd Ab
01a033 + MMAF-conjugated 2nd Ab
DU145
relative growth compared to no treatment (%)
ADC (nM)
01a026 + MMAF-conjugated 2nd Ab
01a009 + MMAF-conjugated 2nd Ab
01a030+ MMAF-conjugated 2nd Ab
01a033 + MMAF-conjugated 2nd Ab

Fig.52

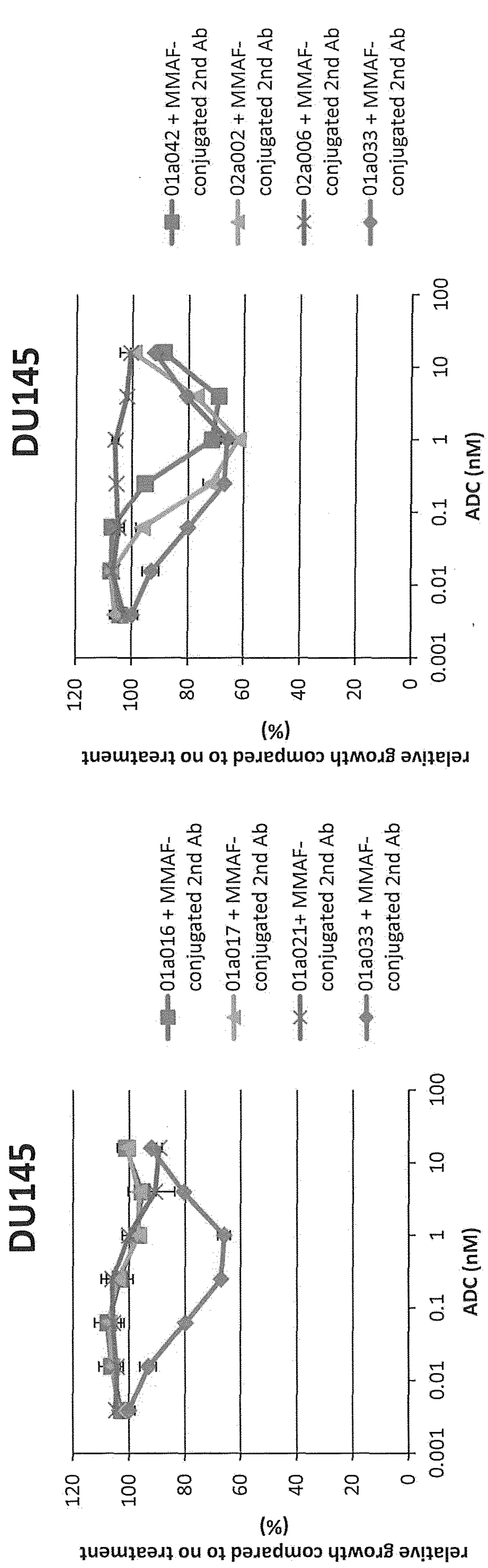
DU145
relative growth compared to no treatment (%)
ADC (nM)
01a016 + MMAF-conjugated 2nd Ab
01a017 + MMAF-conjugated 2nd Ab
01a021+ MMAF-conjugated 2nd Ab
01a033 + MMAF-conjugated 2nd Ab
DU145
relative growth compared to no treatment (%)
ADC (nM)
01a042 + MMAF-conjugated 2nd Ab
02a002 + MMAF-conjugated 2nd Ab
02a006 + MMAF-conjugated 2nd Ab
01a033 + MMAF-conjugated 2nd Ab

Fig.53

| Clone | IC50 value (nM) in TE14 | EC50 value (nM) in DU145 |
|---|---|---|
| 01a033 | 0.014 | 0.0231 |
| 01a002 | 0.031 | 0.0610 |
| 02a010 | 0.042 | 0.0617 |
| 02a002 | 0.024 | 0.0662 |
| clone18 | 0.052 | 0.0940 |
| 02a014 | 0.07 | 0.240 |
| 02b006 | 0.112 | 0.249 |
| 01a042 | 0.059 | 0.264 |
| 01a017 | 0.222 | 0.272 |
| 01a026 | 0.062 | 0.273 |
| 01a016 | 0.295 | 0.274 |
| 01a030 | 0.045 | 0.283 |
| clone4 | 0.423 | 0.292 |
| 01a009 | 0.187 | 0.696 |
| MIL-38 | - | 0.857 |
| 02a034 | not determined | 1.21 |
| 02a022 | not determined | 1.24 |
| 01a021 | not determined | 1.00 |
| 02a006 | not determined | 3.66 |
| 02a035 | not determined | 4.10 |
| 01a007 | not determined | 4.71 |

Fig.54

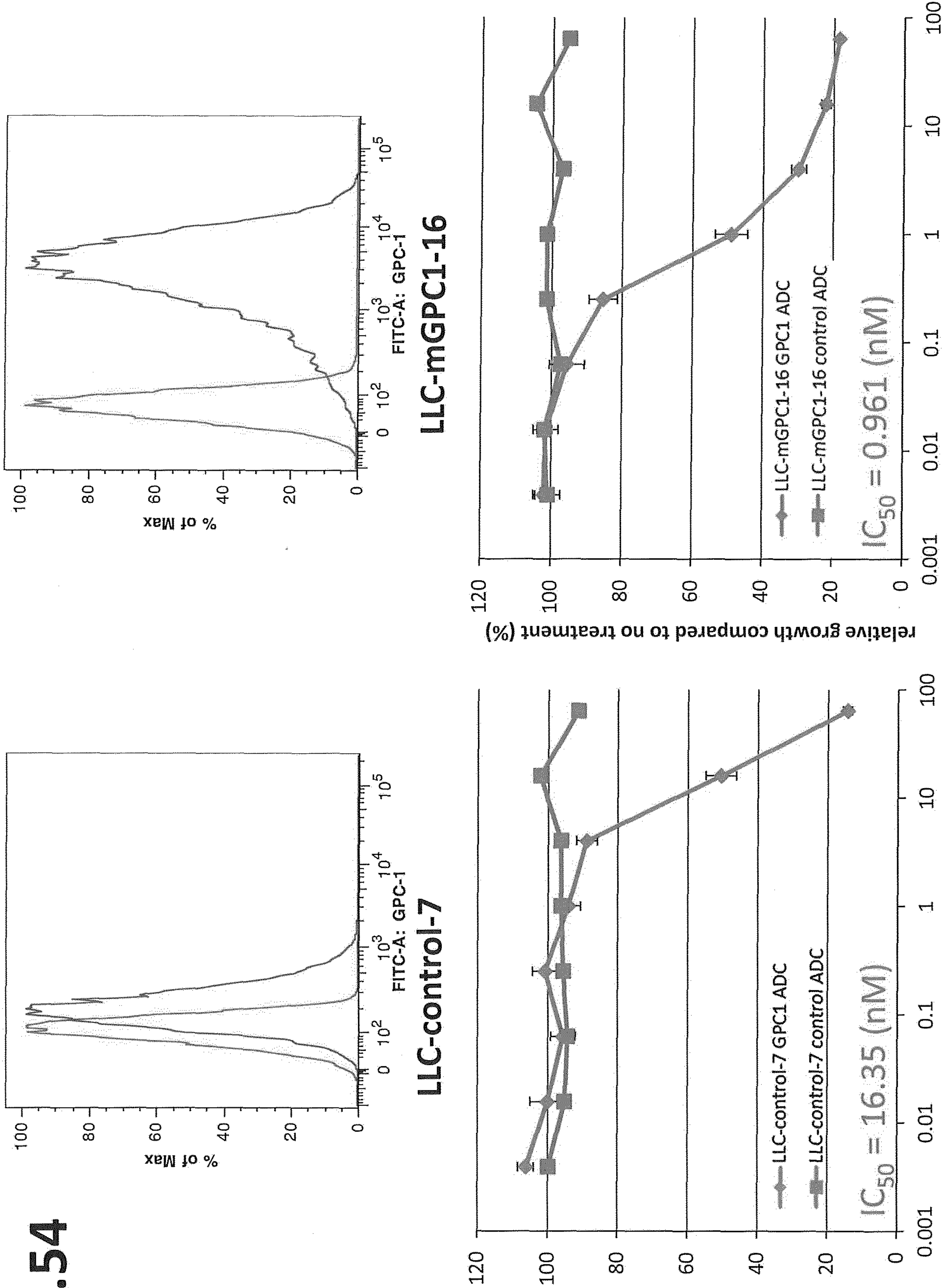

LLC-control-7
% of Max
FITC-A: GPC-1
relative growth compared to no treatment (%)
ADC (nM)
LLC-control-7 GPC1 ADC
LLC-control-7 control ADC
$IC_{50}$ = 16.35 (nM)
LLC-mGPC1-16
% of Max
FITC-A: GPC-1
relative growth compared to no treatment (%)
ADC (nM)
LLC-mGPC1-16 GPC1 ADC
LLC-mGPC1-16 control ADC
$IC_{50}$ = 0.961 (nM)

# Fig.55

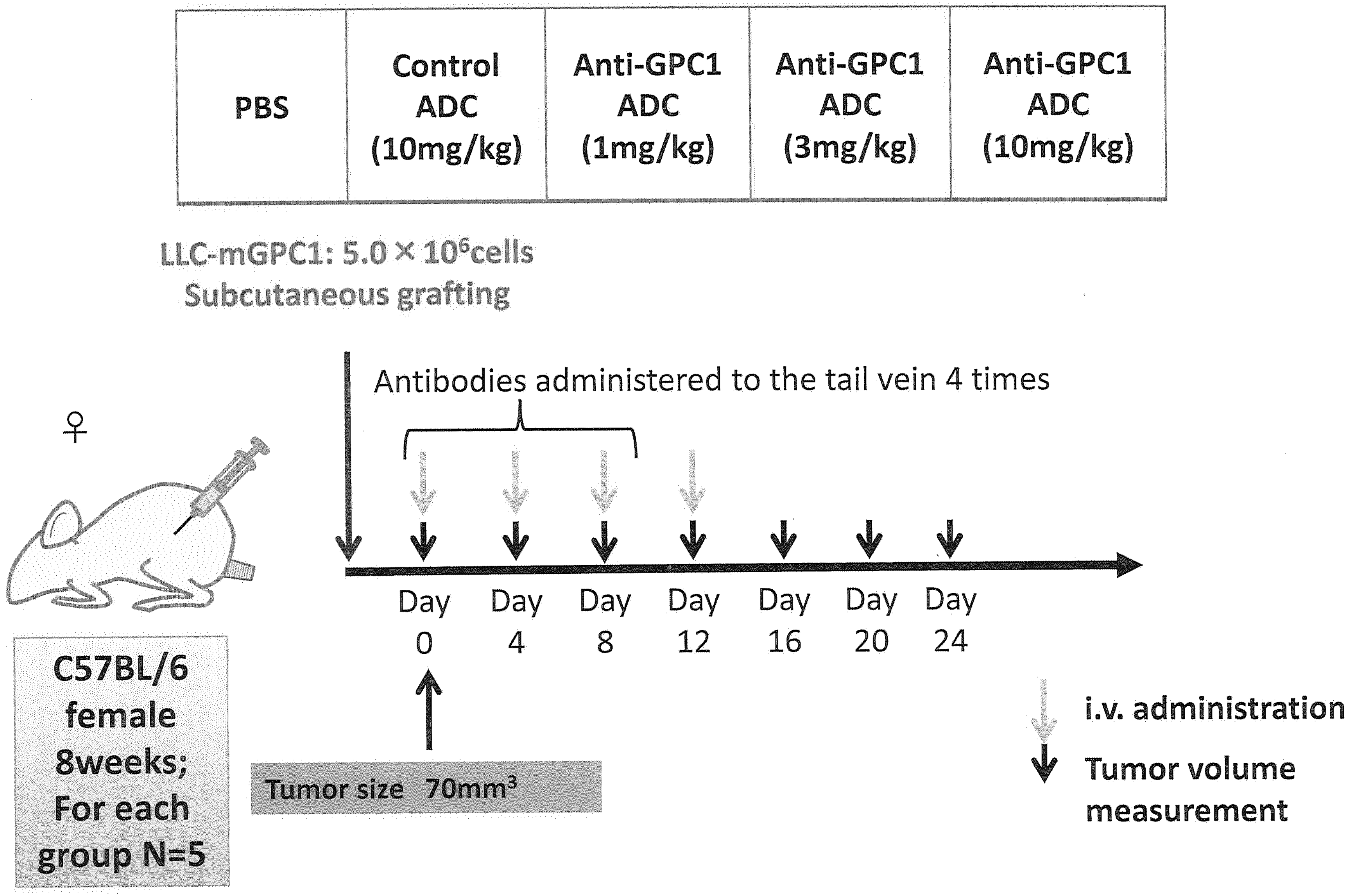
PBS
Control ADC (10mg/kg)
Anti-GPC1 ADC (1mg/kg)
Anti-GPC1 ADC (3mg/kg)
Anti-GPC1 ADC (10mg/kg)
LLC-mGPC1: 5.0 × 10⁶cells
Subcutaneous grafting
Antibodies administered to the tail vein 4 times
♀
Day 0
Day 4
Day 8
Day 12
Day 16
Day 20
Day 24
C57BL/6 female 8weeks; For each group N=5
Tumor size 70mm³
i.v. administration
Tumor volume measurement

Fig.56

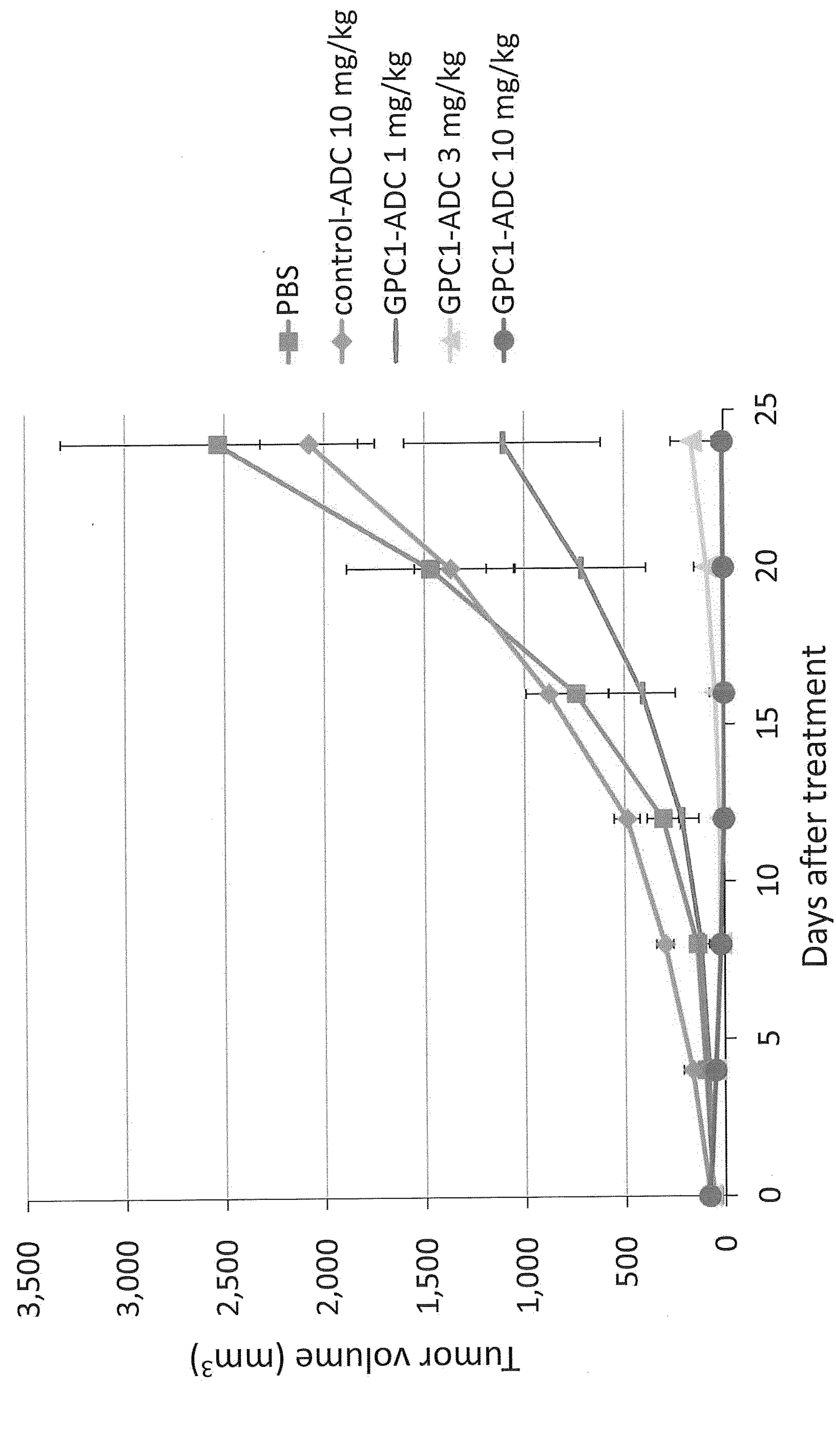
Tumor volume (mm³)
3,500
3,000
2,500
2,000
1,500
1,000
500
0
0
5
10
15
20
25
Days after treatment
PBS
control-ADC 10 mg/kg
GPC1-ADC 1 mg/kg
GPC1-ADC 3 mg/kg
GPC1-ADC 10 mg/kg

Fig.57

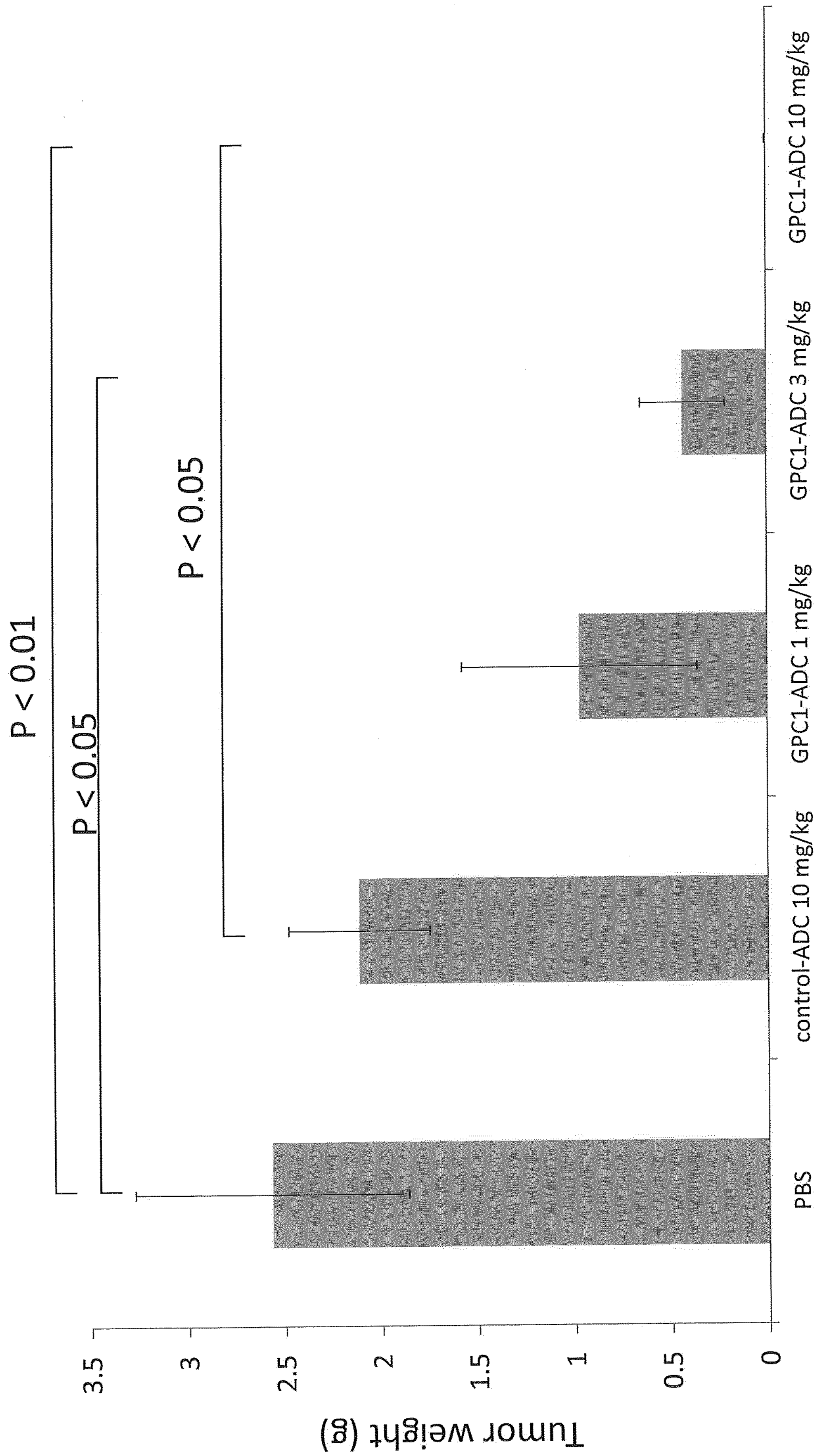
P < 0.01
P < 0.05
P < 0.05
Tumor weight (g)
3.5
3
2.5
2
1.5
1
0.5
0
PBS
control-ADC 10 mg/kg
GPC1-ADC 1 mg/kg
GPC1-ADC 3 mg/kg
GPC1-ADC 10 mg/kg

Fig.58

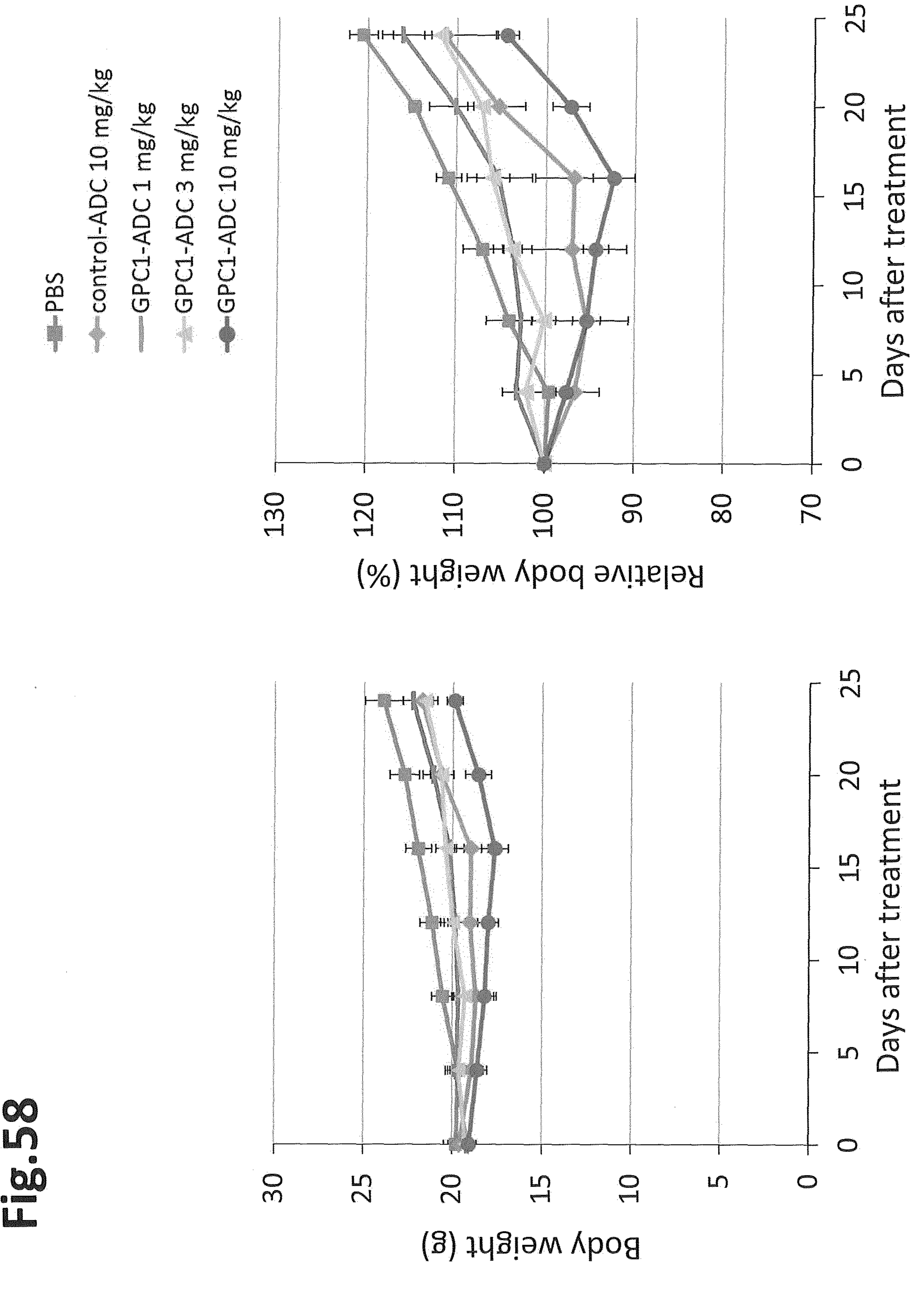
PBS
control-ADC 10 mg/kg
GPC1-ADC 1 mg/kg
GPC1-ADC 3 mg/kg
GPC1-ADC 10 mg/kg
Body weight (g)
Days after treatment
Relative body weight (%)
Days after treatment

## INTERNATIONAL SEARCH REPORT

International application No.
PCT/JP2018/017291

A. CLASSIFICATION OF SUBJECT MATTER
Int.Cl. C07K16/28(2006.01)i, A01H1/00(2006.01)i, A61K39/395(2006.01)i, A61K49/00(2006.01)i, A61P35/00(2006.01)i, C07K16/46(2006.01)i, C12N5/10(2006.01)i, G01N33/574(2006.01)i, C12N15/13(2006.01)n, C12P21/08(2006.01)n

According to International Patent Classification (IPC) or to both national classification and IPC

B. FIELDS SEARCHED

Minimum documentation searched (classification system followed by classification symbols)
Int.Cl. C07K16/00-16/46, C12N15/00-15/90, A01H1/00, A61K39/395, A61K49/00, A61P35/00, C12N5/10, G01N33/574, C12P21/08

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII), CAplus/REGISTRY/MEDLINE/EMBASE/BIOSIS/WPIDS (STN), UniProt/GeneSeq

C. DOCUMENTS CONSIDERED TO BE RELEVANT

| Category* | Citation of document, with indication, where appropriate, of the relevant passages | Relevant to claim No. |
|---|---|---|
| X<br>Y | WO 2015/098112 A1 (NATIONAL INSTITUTE OF BIOMEDICAL INNOVATION) 02 July 2015, claims, paragraphs [0205]-[0209], examples 8-13, fig. 11, 12, 16B & US 2017/0066836 A1, claims, paragraphs [0245]-[0248], examples 8-13, fig. 11, 12, 16B | 1-4, 8-9, 11-33<br>1-33 |
| Y | WO 2016/168885 A1 (MINOMIC INTERNATIONAL LTD.) 27 October 2016, example 4 & EP 3285805 A1 & KR 10-2017-0138530 A & CN 107847593 A | 1-33 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 July 2018 (19.07.2018) | 31 July 2018 (31.07.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No.
PCT/JP2018/017291

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | WO 2016/208754 A1 (KEIO GIJUKU) 29 December 2016, example 7 (Family: none) | 34-36<br>34-37 |
| Y | US 2014/0302060 A1 (BEG, Amer A. et al.) 09 October 2014, examples 1, 2 & WO 2013/067198 A1 | 34-37 |
| Y | JP 2011-513399 A (THE UNIVERSITY OF MIAMI) 28 April 2011, examples & US 2011/0250229 A1, examples & WO 2009/114085 A2 & EP 2257301 A2 & KR 10-2011-0009095 A & CN 102014937 A | 34-37 |
| Y | JP 2009-542592 A (MERCK PATENT GMBH) 03 December 2009, example 6 & US 2008/0025947 A1, example 6 & WO 2008/003473 A2 & EP 2038417 A2 | 34-37 |
| P, X | MATSUZAKI, Satoko et al., "Anti-glypican-1 antibody-drug conjugate exhibits potent preclinical antitumor activity against glypican-1 positive uterine cervical cancer", Int. J. Cancer, 2018, vol. 142, pp. 1056-1066, published online 21 October 2017 | 1-33 |

Form PCT/ISA/210 (continuation of second sheet) (January 2015)

INTERNATIONAL SEARCH REPORT

International application No.
PCT/JP2018/017291

Claim 15 discloses, in (t), an "antibody comprising amino acid sequences of heavy chain CDR1, heavy chain CDR2, and heavy chain CDR3 as shown in SEQ ID NOs: 125, 126, and 127, respectively, and amino acid sequences of light chain CDR1, light chain CDR2, and light chain CDR3 as shown in SEQ ID NOs: 128, 129, and 130, respectively. However, considering paragraph [0238] and the sequence list of the present specification, SEQ ID Nos: 125-130 are not amino acid sequences of CDR, and thus the antibody indicated in (t) cannot be clearly understood.

Thus, claim 15, and claims 16-23, and 33 referring to claim 15 do not comply with the requirements for clarity under PCT Article 6.

Accordingly, a search for the antibody indicated in (t) of claim 15 was carried out by considering SEQ ID NOs: 125-130 to be a typographical error of SEQ ID NOs: 119-124, on the basis of paragraph [0238] and the sequence list of the present specification.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015098112 A
- US 4816567 A
- JP 2006241026 A
- WO 2016168885 A
- JP 2017090054 A

### Non-patent literature cited in the description


- **DAVID G et al.** *J Cell Biol.,* December 1990, vol. 111 (6), 3165-76
- **HAECKER U et al.** *Nat Rev Mol Cell Biol.,* July 2005, vol. 6 (7), 530-41
- **AIKAWA T et al.** *J Clin Invest.,* January 2008, vol. 118 (1), 89-99
- **MATSUDA K et al.** *Cancer Res.,* 15 July 2001, vol. 61 (14), 5562-9
- **BATZER et al.** *Nucleic Acid Res.,* 1991, vol. 19, 5081
- **OHTSUKA et al.** *J. Biol. Chem.,* 1985, vol. 260, 2605-2608
- **ROSSOLINI et al.** *Mol. Cell. Probes,* 1994, vol. 8, 91-98
- **MARK et al.** *Proc Natl Acad Sci USA.,* September 1984, vol. 81 (18), 5662-5666
- **ZOLLER et al.** *Nucleic Acids Res.,* 25 October 1982, vol. 10 (20), 6487-6500
- **WANG et al.** *Science,* 29 June 1984, vol. 224 (4656), 1431-1433
- **AUSUBEL et al.** Current Protocols in Molecular Biology. Wiley Interscience Publishers, 1995
- Current Protocols in Molecular Biology. Molecular Cloning
- DNA Cloning 1: Core Techniques, A Practical Approach. Oxford University Press, 1995
- **SAMBROOK et al.** Molecular Cloning: A Laboratory Manual. Cold Spring Harbor Laboratory Press, 2001, vol. 1, 7.42-7.45
- **ALTSCHUL et al.** *J. Mol. Biol.,* 1990, vol. 215, 403-410
- **PEARSON ; LIPMAN.** *Proc. Natl. Acad. Sci., USA,* 1988, vol. 85, 2444-2448
- **SMITH ; WATERMAN.** *J. Mol. Biol.,* 1981, vol. 147, 195-197
- **NEEDLEMAN ; WUNSCH.** *J. Mol. Biol.,* 1970, vol. 48, 443-453
- Tanpakushitsu Jikken Handobukku [Protein experiment handbook. Yodosha, 2003, 86-91
- **KOHLER G ; MILSTEIN C.** *Nature,* 07 August 1975, vol. 256 (5517), 495-497
- **CLACKSON et al.** *Nature,* 15 August 1991, vol. 352 (6336), 624-628
- **MARKS et al.** *J Mol Biol.,* 05 December 1991, vol. 222 (3), 581-597
- Tanpakushitsu Jikken Handobukku [Protein experiment handbook. Yodosha, 2003, 92-96
- **REITER et al.** *Protein Eng.,* May 1994, vol. 7 (5), 697-704
- **ROGUSKA et al.** *Proc Natl Acad Sci USA.,* 01 February 1994, vol. 91 (3), 969-973
- **ALMAGRO et al.** *Front Biosci.,* 01 January 2008, vol. 13, 1619-1633
- **OZAKI et al.** *Blood,* 01 June 1999, vol. 93 (11), 3922-3930
- **ROGUSKA et al.** *Proc Natl Acad Sci U S A.,* 01 February 1994, vol. 91 (3), 969-973
- **DAMSCHRODER et al.** *Mol Immunol.,* April 2007, vol. 44 (11), 3049-3060
- **RIECHMANN et al.** *Nature,* 24 March 1988, vol. 332 (6162), 323-327
- **LONBERG et al.** *Int Rev Immunol.,* 1995, vol. 13 (1), 65-93
- **VAUGHAN et al.** *Nat Biotechnol.,* March 1996, vol. 14 (3), 309-314
- **FOOTE et al.** *J Mol Biol.,* 20 March 1992, vol. 224 (2), 487-499
- **REITER et al.** *J Mol Biol.,* 16 July 1999, vol. 290 (3), 685-98
- **WOLFSON W.** *Chem Biol.,* December 2006, vol. 13 (12), 1243-1244
- **WILLY et al.** *Biochemical and Biophysical Research Communications,* 27 April 2007, vol. 356 (1), 124-128
- **KABAT et al.** Sequence of Proteins of Immunological Interest. US Dept. Health and Human Services, 1983
- Sequences of Proteins of Immunological Interest. Public Health Service, National Institutes of Health, 1991
- **CHOTHIA et al.** *J. Mol. Biol.,* 1987, vol. 196, 901-917

- *Proc.Natl.Acad.Sci.USA,* 1989, vol. 86, 9268-9272
- DNA Maikuroarei to Saishin PCR ho'' [Cellular engineering, Extra issue, ''DNA Microarrays and Latest PCR Methods''
- *Nat Genet,* December 2002, vol. 32 (526-532
- Genomu Kaiseki Jikkenho Nakamura Yusuke Labo Manyuaru [Genome analysis experimental method Yusuke Nakamura Lab Manual. Yodosha, 2002
- Kakusan IV Idenshi no Fukusei to Hatsugen [Replication and Expression of Gene of Nucleic Acid IV. **HIRASHIMA ; INOUE.** Shin-seikagaku Jikken Kouza 2 [New Biochemical Experiment Course 2. Tokyo Kagaku Dojin, 1993, 319-347
- Kakusan IV Idenshi no Fukusei to Hatsugen [Replication and Expression of Gene of Nucleic Acid IV]. **HIRASHIMA ; INOUE.** Shin-seikagaku Jikkenn Kouza 2 [New Biochemical Experiment Course 2]. Tokyo Kagaku Dojin, 1993, 319-347
- **MAKOTO KOIZUMI ; EIKO OTSUKA.** *Protein, Nucleic Acid and Enzyme,* 1990, vol. 35, 2191
- **BUZAYAN J M.** *Nature,* 1986, vol. 323, 349
- **KIKUCHI, Y. ; SASAKI, N.** *Nucl. Acids Res,* 1991, vol. 19, 6751
- **YO KIKUCHI.** *Kagaku to Seibutsu [Chemistry and Biology],* 1992, vol. 30, 112
- **HOSSAIN MM ; HOSONO-FUKAO T ; TANG R ; SUGAYA N ; VAN KUPPEVELT TH ; JENNISKENS GJ ; KIMATA K ; ROSEN SD ; UCHIMURA K.** Direct detection of HSulf-1 and HSulf-2 activities on extracellular heparan sulphate and their inhibition by PI-88. *Glycobiology,* 2010, vol. 20 (2), 175-186